# EUROPEAN PATENT APPLICATION

(11) **EP 1 402 900 A1**
(43) Date of publication of application: **31.03.2004**
(21) Application number: 02733431.7
(22) Date of filing: 10.06.2002
(51) Int. Cl.: A61K 45/00, A61K 45/06, A61K 31/4439, A61K 31/444, A61K 31/4545, A61K 31/496, A61K 31/5377, A61P 29/00, A61P 43/00, C07D 417/04

(54) **MEDICINAL COMPOSITIONS**

(30) Priority: 11.06.2001 JP 2001175224; 11.06.2001 JP 2001175273
(71) Applicant: Takeda Chemical Industries, Ltd., Osaka-shi, Osaka 541-0045 (JP)
(72) Inventor: OHKAWA, Shigenori, Takatsuki-shi, Osaka 569-1121 (JP); NARUO, Ken-ichi, Sanda-shi, Hyogo 669-1535 (JP); MORIMOTO, Shigeru, Tondabayashi-shi, Osaka 584-0067 (JP); NAGASE, Yoshinori, Ikeda-shi, Osaka 563-0021 (JP); MIWATASHI, Seiji, Ikeda-shi, Osaka 563-0056 (JP)
(74) Representative: Rickard, Timothy Mark Adrian
(86) International application number: PCT/JP2002/005726
(87) International publication number: WO 2002/100433

(57) **Abstract**

The present invention relates to an agent for the prophylaxis or treatment of pain, an agent for suppressing activation of osteoclast, and an inhibitor of osteoclast formation, which contains a p38 MAP kinase inhibitor and/or a TNF-α production inhibitor.

## Description

### Technical Field

The present invention relates to an agent for the prophylaxis or treatment of pain or an agent for the suppression of activation or inhibition of formation of osteoclast, which contains a p38 MAP kinase inhibitor and/or a TNF-< production inhibitor.

### Background Art

Cytokines such as TNF-α (tumor necrosis factor-α), IL-1 (interleukin-1) and the like are biological substances, which are produced by a variety of cells such as monocyte or macrophage in response to infection and other cellular stress (Koj, A., Biochim. Biophys. Acta, 1317, 84-94 (1996)). Although these cytokines play important roles in the immune response when they are present at an appropriate amount, it is thought that the overproduction is associated with a variety of inflammatory diseases (Dinarello, C.A., Curr. Opin. Immunol., 3, 941-948 (1991)). p38 MAP kinase which was cloned as a homologue of MAP kinase is involved in the control of production of these cytokines and signal transduction system coupled with receptors, and there is a possibility that the inhibition of p38 MAP kinase provides a drug for treating inflammatory diseases (Stein, B., Anderson, D., Annual Report in Medicinal Chemistry, edited by Bristol, J.A., Academic Press, vol.31, pages 289-298, 1996).

As compounds having a p38 MAP kinase inhibitory activity, imidazole derivatives are described in JP-T 7-50317 (WO 93/14081) and oxazole derivatives are described in JP-T 9-505055 (WO 95/13067), respectively.

On the other hand, as thiazole compounds, the following compounds are known:
1) 1,3-thiazole derivatives represented by the formula: 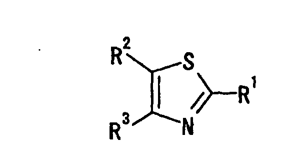 wherein R¹ represents a cycloalkyl group, a cyclic amino group, an amino group optionally having, as substituents, 1 or 2 lower alkyl, phenyl, acetyl or lower alkoxycarbonylacetyl, an alkyl group optionally having, as substituents, hydroxyl, carboxyl or lower alkoxycarbonyl, or a phenyl group optionally having, as substituents, carboxyl, 2-carboxyethenyl or 2-carboxy-1-propenyl, R² represents a pyridyl group optionally having, as substituents, lower alkyl, R³ represents a phenyl group optionally having, as substituents, lower alkoxy, lower alkyl, hydroxyl, halogen or methylenedioxy, or salts thereof, which have analgesic, antipyretic, anti-inflammatory, anti-ulcerative, thromboxane A₂ (TXA₂) synthase-inhibitory, and platelet coagulation-inhibitory activities (JP-A 60-58981),
2) 1,3-thiazole derivatives represented by the formula: 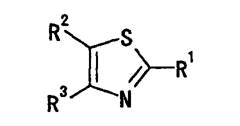 wherein R¹ represents an alkyl group, an alkenyl group, an aryl group, an aralkyl group, a cycloalkyl group, a heterocyclic group employing carbon as an attachment point or an amino group optionally having substituents, R² represents a pyridyl group optionally substituted with alkyl group(s), R³ represents a phenyl group optionally having substituents, or salts thereof, which have analgesic, antipyretic, anti-inflammatory, anti-ulcerative, TXA₂ synthase-inhibitory, and platelet coagulation-inhibitory activities (JP-A 61-10580),
3) 1,3-thiazole derivatives represented by the formula: 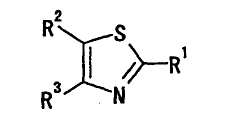 wherein R¹ represents an alkyl group, an alkenyl group, an aryl group, an aralkyl group, a cycloalkyl group, a heterocyclic group employing carbon as an attachment point or an amino group optionally having substituents, R² represents a pyridyl group optionally substituted with alkyl group(s), R³ represents an aryl group optionally having substituents, or salts thereof, which have analgesic, antipyretic, anti-inflammatory, anti-ulcerative, TXA₂ synthase-inhibitory, and platelet coagulation-inhibitory activities (USP 4,612,321),
4) a compound of the formula 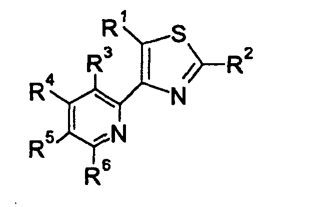 wherein R¹ represents an optionally substituted phenyl, R² represents C₁₋₆ alkyl or (CH₂)ₙAr, n represents 0-2, Ar represents an optionally substituted phenyl, R³ represents a hydrogen or C₁₋₄ alkyl, R⁴ represents a hydrogen, C₁₋₄ alkyl and the like, R⁵ represents a hydrogen or C₁₋₄ alkyl, R⁶ represents a hydrogen, C₁₋₄ alkyl and the like, or a salt thereof, having an inhibitory activity of gastric acid secretion (JP-T 7-503023, WO93/15071),
5) a compound of the formula 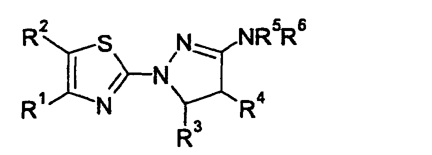 wherein R¹ represents pyridyl and the like, R² represents phenyl and the like, R³ and R⁴ represent a hydrogen or methyl, R⁵ represents methyl and the like, and R⁶ represents a hydrogen, methyl and the like, or a salt thereof, which is an antiinflammatory agent and antiallergic agent (DE-A-3601411),
6) a compound of the formula 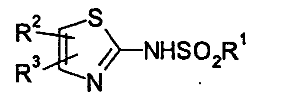 wherein R¹ represents a lower alkyl substituted by halogen, R² represents pyridyl and the like, and R³ represents phenyl and the like, or a salt thereof, having an antiinflammatory, antipyretic, analgesic and antiallergic activity (JP-A-5-70446), and
7) a thiazole compound of the formula 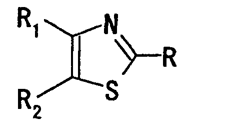 wherein R represents a lower alkyl group; a lower haloalkyl group; a lower hydroxyalkyl group; a lower alkoxy(lower)alkyl group; an aralkyloxy(lower)alkyl group and the like, R¹ represents a cycloalkyl group optionally substituted by lower alkyl group(s) and the like, and R² represents an optionally substituted aryl group and the like, or a pharmaceutically acceptable salt thereof, having a selective inhibitory activity of TNF-α production and/or IFN-γ production (JP-A-11-49762).

WO00/64894 describes that an optionally N-oxidized compound represented by the formula: 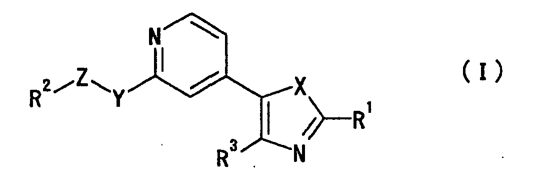 wherein R¹ represents a hydrogen atom, a hydrocarbon group optionally having substituents, a heterocyclic group optionally having substituents, an amino group optionally having substituents or an acyl group,
R² represents an aromatic group optionally having substituents,
R³ represents a hydrogen atom, a pyridyl group optionally having substituents or an aromatic hydrocarbon group optionally having substituents,
X represents an oxygen atom or an optionally oxidized sulfur atom,
Y represents a bond, an oxygen atom, an optionally oxidized sulfur atom or a group represented by the formula: NR⁴ (wherein R⁴ represents a hydrogen atom, a hydrocarbon group optionally having substituents or an acyl group) and
Z represents a bond or a divalent acyclic hydrocarbon group optionally having substituents, or a salt thereof,
has a superior p38 MAP kinase inhibitory activity and TNF-α inhibitory activity and is useful as a prophylactic or therapeutic agent for p38 MAP kinase related diseases and TNF-α related diseases.

WO00/63204 describes that a compound of the formula 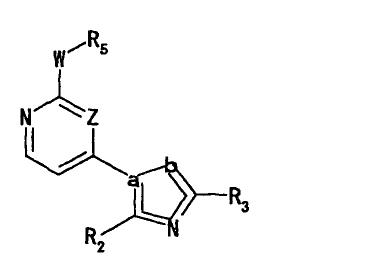 wherein
- a: is N or C;
- b: is CH when a is N, or O when a is C;
- =: denotes a single or a double bond dependent upon whether the azole ring is an imidazole or an oxazole ring;
- Z: is N or CH;
- W: is -NR₆-Y-, -O- or -S-,
where R₆ is a hydrogen atom, C₁₋₄ alkyl group, C₃₋₈ cycloalkyl group, C₃₋₈ cycloalkyl-C₁₋₃ alkyl group, C₆₋₁₈ aryl group, C₃₋₁₈ heteroaryl group, C₇₋₁₉ aralkyl group or C₄₋₁₉ heteroaralkyl group, and -Y- is C₁₋₄ alkylene group or a bond;
- R₂: is phenyl group, optionally substituted by one or more substituents selected from the group consisting of a halogen atom, trifluoromethyl, cyano, amido, thioamido, carboxylate, thiocarboxylate, C₁₋₄ alkoxy, C₁₋₄ alkyl, amino, and mono- or di-C₁₋₄ alkylamino;
- R₃: is a hydrogen atom, a halogen atom, C₁₋₁₀ alkyl group, C₁₋₄ alkenyl group, C₃₋₁₀ cycloalkyl group, C₃₋₁₈ heterocycloalkyl group, C₆₋₁₈ aryl group, C₃₋₁₈ heteroaryl group or -CH=N-NH-C(NH)NH₂, (each of which is optionally substituted by 1 to 4 substituents selected from C₁₋₄ alkyl optionally substituted by hydroxy, halogen atom, halo-substituted-C₁₋₄ alkyl, hydroxy, C₁₋₄ alkoxy, C₁₋₄ alkylthio, carboxy, carbonyl optionally substituted by C₁₋₆ alkyl or C₁₋₆ alkoxy, amino, mono- or di-C₁₋₄ alkylamino and 5 to 7 membered N-heterocyclic group optionally further containing heteroatom(s));
- R₅: is C₆₋₁₈ aryl group, C₃₋₁₈ heteroaryl group or C₃₋₁₂ cycloalkyl group each of which is optionally substituted by 1 to 4 substituents selected from C₁₋₄ alkyl, halogen, halo-substitued-C₁₋₄ alkyl, hydroxy, C₁₋₄ alkoxy, C₁₋₄ alkylthio, amino, mono- or di-C₁₋₄ alkylamino and 5 to 7 membered N-heterocyclic group optionally further containing heteroatom(s), or a salt thereof has a p38 MAP kinase inhibitory activity and is useful as a prophylactic or therapeutic agent of rheumatoid arthritis and the like.

WO01/10865 describes that a 1,3-thiazole compound, which is a compound represented by the formula 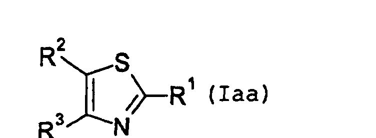 wherein R¹ is a hydrogen atom, a hydrocarbon group optionally having substituents, a heterocyclic group optionally having substituents, an amino group optionally having substituents or an acyl group,
R² is a 4-pyridyl group having substituents free of aromatic group, and
R³ is an aromatic group optionally having substituents, and the like, wherein the 5-position is substituted by a 4-pyridyl group having substituents free of aromatic group, which is other than N-[4-(3,5-dimethylphenyl)-5-(2-hydroxy-4-pyridyl)-1,3-thiazol-2-yl]acetamide and 4-[2-(acetylamino)-4-(3,5-dimethylphenyl)-1,3-thiazol-5-yl]-2-pyridyl acetate, or a salt thereof, and a 1,3-thiazole compound, which is a compound represented by the formula 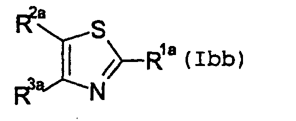 wherein R^{1a} is a hydrogen atom, a hydrocarbon group optionally having substituents, a heterocyclic group optionally having substituents, an amino group optionally having substituents or an acyl group,
R^{2a} is a pyridyl group having a substituent free of an aromatic group at the position next to the nitrogen atom of the pyridyl group, and
R^{3a} is an aromatic group optionally having substituents and the like, wherein the 5-position is substituted by a pyridyl group having substituents free of aromatic group next to the nitrogen atom of the pyridyl group, which is other than N-[4-(3,5-dimethylphenyl)-5-(2-hydroxy-4-pyridyl)-1,3-thiazol-2-yl]acetamide and 4-[2-(acetylamino)-4-(3,5-dimethylphenyl)-1,3-thiazol-5-yl]-2-pyridyl acetate, and a salt thereof have superior p38MAP kinase inhibitory action or TNF-α inhibitory action, and are useful as prophylactic or therapeutic agents of p38 MAP kinase related diseases or TNF-α related diseases.

### Disclosure of the Invention

The present invention aims at providing a prophylactic or therapeutic agent of pain or an activation suppressant or formation inhibitor of osteoclast, which contains a p38 MAP kinase inhibitor and/or a TNF-α production inhibitor.

In view of the above-mentioned object, the present inventors have conducted intensive studies and found that a p38 MAP kinase inhibitor and/or a TNF-α production inhibitor used as a prophylactic or therapeutic agent of diseases such as rheumatism, arthritis and the like unexpectedly has/have a superior prophylactic or therapeutic effect on pain, suppress(es) activation of osteoclast and inhibit(s) formation of osteoclast. Based on this finding, the present inventors have further studied and completed the present invention.

Accordingly, the present invention provides
[1] an agent for the prophylaxis or treatment of pain and/or suppression of activation and/or inhibition of formation of osteoclast, which contains a p38 MAP kinase inhibitor and/or a TNF-α production inhibitor,
[2] the agent of [1] for the prophylaxis or treatment of pain, which contains a p38 MAP kinase inhibitor and/or a TNF-α production inhibitor,
[3] the agent of [1] for the suppression of activation and/or inhibition of formation of osteoclast, which contains a p38 MAP kinase inhibitor and/or a TNF-α production inhibitor,
[4] the agent of [1], wherein the p38 MAP kinase inhibitor and/or the TNF-α production inhibitor are/is a 1,3-thiazole compound substituted at the 5-position by a pyridyl group optionally having substituents, or a salt thereof or a prodrug thereof,
[5] the agent of [1], wherein the p38 MAP kinase inhibitor and/or the TNF-α production inhibitor are/is a compound represented by the formula: 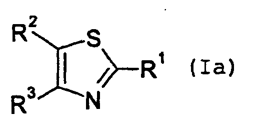 wherein
   - R¹: represents a hydrogen atom, a hydrocarbon group optionally having substituents, a heterocyclic group optionally having substituents, an amino group optionally having substituents or an acyl group;
   - R²: represents a pyridyl group optionally having substituents; and
   - R³: represents an aromatic group optionally having substituents,
   a salt thereof or a prodrug thereof,
[6] the agent of [1], wherein the p38 MAP kinase inhibitor and/or the TNF-α production inhibitor are/is an optionally N-oxidized compound represented by the formula: 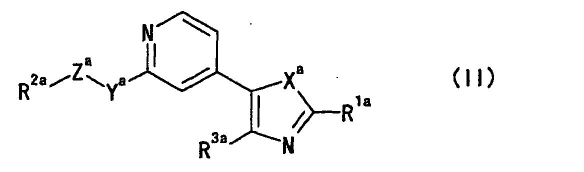 wherein
   - R^{1a}: represents a hydrogen atom, a hydrocarbon group optionally having substituents, a heterocyclic group optionally having substituents, an amino group optionally having substituents or an acyl group;
   - R^{2a}: represents an aromatic group optionally having substituents;
   - R^{3a}: represents a hydrogen atom, a pyridyl group optionally having substituents or an aromatic hydrocarbon group optionally having substituents;
   - X^{a}: represents an oxygen atom or an optionally oxidized sulfur atom;
   - Y^{a}: represents a bond, an oxygen atom, an optionally oxidized sulfur atom or a group represented by the formula: NR^{4a} (wherein R^{4a} represents a hydrogen atom, a hydrocarbon group optionally having substituents or an acyl group); and
   - Z^{a}: represents a bond or a divalent acyclic hydrocarbon group optionally having substituents,
   or a salt thereof, or a prodrug thereof,
[7] the agent of [1], wherein the p38 MAP kinase inhibitor and/or the TNF-α production inhibitor are/is a compound represented by the formula 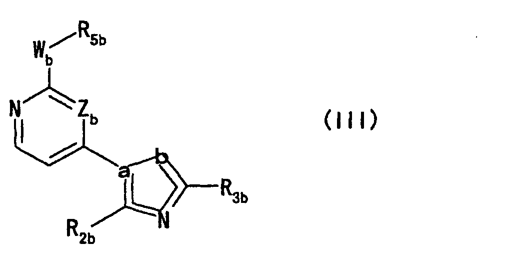 wherein
   - a: is N or C;
   - b: is CH when a is N, or O when a is C;
   - =: denotes a single or a double bond dependent upon whether the azole ring is an imidazole ring or an oxazole ring;
   - Z_{b}: is N or CH;
   - W_{b}: is -NR_{6b}-Y_{b}-, -O- or -S-,
   where R_{6b} is a hydrogen atom, C₁₋₄ alkyl group, C₃₋₈ cycloalkyl group, C₃₋₈ cycloalkyl-C₁₋₃ alkyl group, C₆₋₁₈ aryl group, C₃₋₁₈ heteroaryl group, C₇₋₁₉ aralkyl group or C₄₋₁₉ heteroaralkyl group, and -Y_{b}- is C₁₋₄ alkylene group or a bond;
   - R_{2b}: is phenyl group, optionally substituted by one or more substituents selected from the group consisting of a halogen atom, trifluoromethyl, cyano, amido, thioamido, carboxylate, thiocarboxylate, C₁₋₄ alkoxy, C₁₋₄ alkyl, amino, and mono- or di-C₁₋₄ alkylamino;
   - R_{3b}: is a hydrogen atom, a halogen atom, C₁₋₁₀ alkyl group, C₂₋₄ alkenyl group, C₃₋₁₀ cycloalkyl group, C₃₋₁₈ heterocycloalkyl group, C₆₋₁₈ aryl group, C₃₋₁₈ heteroaryl group or -CH=N-NH-C(NH)NH₂ (wherein C₁₋₁₀ alkyl group, C₂₋₄ alkenyl group, C₃₋₁₀ cycloalkyl group, C₃₋₁₈ heterocycloalkyl group, C₆₋₁₈ aryl group, C₃₋₁₈ heteroaryl group and -CH=N-NH-C(NH)NH₂ are each optionally substituted by 1 to 4 substituents selected from the group consisting of C₁₋₄ alkyl optionally substituted by hydroxy, halogen atom, halo-substituted-C₁₋₄ alkyl, hydroxy, C₁₋₄ alkoxy, C₁₋₄ alkylthio, carboxy, carbonyl optionally substituted by C₁₋₆ alkyl or C₁₋₆ alkoxy, amino, mono- or di-C₁₋₄ alkylamino and 5- to 7- membered N-heterocyclic group optionally further containing heteroatom(s)); and
   - R_{5b}: is C₆₋₁₈ aryl group, C₃₋₁₈ heteroaryl group or C₃₋₁₂ cycloalkyl group each of which is optionally substituted by 1 to 4 substituents selected from the group consisting of C₁₋₄ alkyl, halogen, halo-substitued-C₁₋₄ alkyl, hydroxy, C₁₋₄ alkoxy, C₁₋₄ alkylthio, amino, mono - or di-C₁₋₄ alkylamino and 5- to 7-membered N-heterocyclic group optionally further containing heteroatom(s),
   or a salt thereof or a prodrug thereof,
[8] the agent of [1], wherein the p38 MAP kinase inhibitor and/or the TNF-α production inhibitor is a 1,3-thiazole compound (IV) substituted at the 5-position by a 4-pyridyl group having substituents free of aromatic group, or a salt thereof or a prodrug thereof,
[9] the agent of [8], wherein the 1,3-thiazole compound is a compound represented by the formula 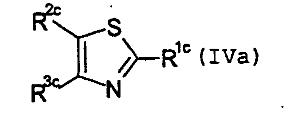 wherein
   - R^{1c}: is a hydrogen atom, a hydrocarbon group optionally having substituents, a heterocyclic group optionally having substituents, an amino group optionally having substituents or an acyl group;
   - R^{2c}: is a 4-pyridyl group having substituents free of aromatic group; and
   - R^{3c}: is an aromatic group optionally having substituents, or a salt thereof,
[10] the agent of [1], wherein the p38 MAP kinase inhibitor and/or the TNF-α production inhibitor is a 1,3-thiazole compound (V) substituted at the 5-position by a pyridyl group having substituents free of aromatic group at a position next to a nitrogen atom of the pyridyl group, or a salt thereof, or a prodrug thereof,
[11] the agent of [10], wherein the 1,3-thiazole compound is a compound represented by the formula 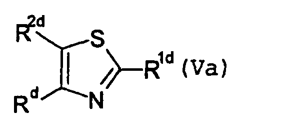 wherein
   - R^{1d}: is a hydrogen atom, a hydrocarbon group optionally having substituents, a heterocyclic group optionally having substituents, an amino group optionally having substituents or an acyl group;
   - R^{2d}: is a pyridyl group having substituents free of aromatic group at a position next to a nitrogen atom of the pyridyl group; and
   - R^{3d}: is an aromatic group optionally having substituents, or a salt thereof,
[12] the agent of [1], wherein the p38 MAP kinase inhibitor and/or the TNF-α production inhibitor are/is a 1,3-thiazole compound (VI) substituted at the 5-position by a 4-pyridyl group having substituents free of aromatic group at a position next to a nitrogen atom of the 4-pyridyl group, or a salt thereof or a prodrug thereof,
[13] the agent of [1], wherein the p38 MAP kinase inhibitor and/or the TNF-α production inhibitor are/is
   N-[5-(2-benzoylamino-4-pyridyl)-4-(3,5-dimethylphenyl)-1,3-thiazol-2-yl]acetamide,
   N-[5-(2-benzylamino-4-pyridyl)-4-(3,5-dimethylphenyl)-1,3-thiazol-2-yl]acetamide,
   N-[4-[4-(4-methoxyphenyl)-2-methyl-1,3-thiazol-5-yl]-2-pyridyl]benzamide,
   N-[4-[2-(4-fluorophenyl)-4-(3-methylphenyl)-1,3-thiazol-5-yl]-2-pyridyl]phenylacetamide,
   N-[4-[2-ethyl-4-(3-methylphenyl)-1,3-thiazol-5-yl]-2-pyridyl]phenylacetamide,
   N-[4-[4-(3-methylphenyl)-2-propyl-1,3-thiazol-5-yl]-2-pyridyl]phenylacetamide,
   N-[4-[2-butyl-4-(3-methylphenyl)-1,3-thiazol-5-yl]-2-pyridyl]phenylacetamide,
   N-[4-[4-(3-methylphenyl)-2-(4-methylthiophenyl)-1,3-thiazol-5-yl]-2-pyridyl]phenylacetamide,
   N-[4-[2-ethyl-4-(3-methylphenyl)-1,3-thiazol-5-yl]-2-pyridyl]benzamide,
   N-[4-[2-ethyl-4-(3-methylphenyl)-1,3-thiazol-5-yl]-2-pyridyl]-3-phenylpropionamide,
   N-[4-[2-ethyl-4-(3-methylphenyl)-1,3-thiazol-5-yl]-2-pyridyl]-3-(4-methoxyphenyl)propionamide,
   N-[4-[2-ethyl-4-(3-methylphenyl)-1,3-thiazol-5-yl]-2-pyridyl]-4-phenylbutyramide,
   N-[4-[4-(3-methylphenyl)-2-propyl-1,3-thiazol-5-yl]-2-pyridyl]benzamide,
   N-[4-[4-(3-methylphenyl)-2-propyl-1,3-thiazol-5-yl]-2-pyridyl]-3-phenylpropionamide,
   N-[4-[2-butyl-4-(3-methylphenyl)-1,3-thiazol-5-yl]-2-pyridyl]benzamide,
   N-[4-[2-butyl-4-(3-methylphenyl)-1,3-thiazol-5-yl]-2-pyridyl]-3-phenylpropionamide,
   N-[4-[2-(4-fluorophenyl)-4-(3-methylphenyl)-1,3-thiazol-5-yl]-2-pyridyl]benzamide,
   N-[4-[2-(4-fluorophenyl)-4-(3-methylphenyl)-1,3-thiazol-5-yl]-2-pyridyl]-3-phenylpropionamide,
   N-[4-[4-(3-methylphenyl)-2-(4-methylthiophenyl)-1,3-thiazol-5-yl]-2-pyridyl]benzamide,
   N-[4-[4-(3-methylphenyl)-2-(4-methylthiophenyl)-1,3-thiazol-5-yl]-2-pyridyl]-3-phenylpropionamide,
   N-benzyl-N-[4-[2-ethyl-4-(3-methylphenyl)-1,3-thiazol-5-yl]-2-pyridyl]amine,
   N-[4-[2-ethyl-4-(3-methylphenyl)-1,3-thiazol-5-yl]-2-pyridyl]-N-(2-phenylethyl)amine,
   N-[4-[2-ethyl-4-(3-rnethylphenyl)-1,3-thiazol-5-yl]-2-pyridyl]-N-(3-phenylpropyl)amine,
   N-benzyl-N-[4-[4-(3-methylphenyl)-2-propyl-1,3-thiazol-5-yl]-2-pyridyl]amine,
   N-[4-[4-(3-methylphenyl)-2-propyl-1,3-thiazol-5-yl]-2-pyridyl]-N-(2-phenylethyl)amine,
   N-[4-[4-(3-methylphenyl)-2-propyl-1,3-thiazol-5-yl]-2-pyridyl]-N-(3-phenylpropyl)amine,
   N-benzyl-N-[4-[2-butyl-4-(3-methylphenyl)-1,3-thiazol-5-yl]-2-pyridyl]amine,
   N-[4-[2-butyl-4-(3-methylphenyl)-1,3-thiazol-5-yl]-2-pyridyl]-N-(2-phenylethyl)amine,
   N-[4-[2-butyl-4-(3-methylphenyl)-1,3-thiazol-5-yl]-2-pyridyl]-N-(3-phenylpropyl)amine,
   N-benzyl-N-[4-[4-(3-methylphenyl)-2-(4-methylthiophenyl)-1,3-thiazol-5-yl]-2-pyridyl]amine,
   N-[4-[4-(3-methylphenyl)-2-(4-methylthiophenyl)-1,3-thiazol-5-yl]-2-pyridyl]-N-(2-phenylethyl)amine,
   N-[4-[4-(3-methylphenyl)-2-(4-methylthiophenyl)-1,3-thiazol-5-yl]-2-pyridyl]-N-(3-phenylpropyl)amine,
   N-[4-[4-(3-methylphenyl)-2-(4-methylsulfonylphenyl)-1,3-thiazol-5-yl]-2-pyridyl]benzamide,
   N-[4-[4-(3-methylphenyl)-2-(4-methylsulfonylphenyl)-1,3-thiazol-5-yl]-2-pyridyl]phenylacetamide,
   N-[4-[4-(3-methylphenyl)-2-(4-methylsulfonylphenyl)-1,3-thiazol-5-yl]-2-pyridyl]-3-phenylpropionamide,
   N-benzyl-N-[4-[4-(3-methylphenyl)-2-(4-methylsulfonylphenyl)-1,3-thiazol-5-yl]-2-pyridyl]amine,
   N-[4-[4-(3-methylphenyl)-2-(4-methylsulfonylphenyl)-1,3-thiazol-5-yl]-2-pyridyl]-N-(3-phenylpropyl)amine,
   N-[4-[4-(3-methylphenyl)-2-(4-methylsulfonylphenyl)-1,3-thiazol-5-yl]-2-pyridyl]-N-(2-phenylethyl)amine,
   N-(4-fluorobenzyl)-N-[4-[4-(3-methylphenyl)-2-(4-methylsulfonylphenyl)-1,3-thiazol-5-yl]-2-pyridyl]amine,
   (S)-N-[4-(3-methylphenyl)-5-(2-(1-phenylethylamino)-4-pyridyl)-1,3-thiazol-2-yl]nicotinamide,
   (R)-N-[4-(3-methylphenyl)-5-(2-(1-phenylethylamino)-4-pyridyl)-1,3-thiazol-2-yl]nicotinamide,
   (S)-N-[4-(3-methylphenyl)-5-(2-(1-phenylethylamino)-4-pyridyl)-1,3-thiazol-2-yl]-2-methylnicotinamide,
   (R)-N-[4-(3-methylphenyl)-5-(2-(1-phenylethylamino)-4-pyridyl)-1,3-thiazol-2-yl]-2-methylnicotinamide,
   (S)-N-[4-(3-methylphenyl)-5-(2-(1-phenylethylamino)-4-pyridyl)-1,3-thiazol-2-yl]-2-chloronicotinamide,
   (R)-N-[4-(3-methylphenyl)-5-(2-(1-phenylethylamino)-4-pyridyl)-1,3-thiazol-2-yl]-2-chloronicotinamide,
   (S)-N-[4-(3-methylphenyl)-5-(2-(1-phenylethylamino)-4-pyridyl)-1,3-thiazol-2-yl]-2-methoxynicotinamide,
   (R)-N-[4-(3-methylphenyl)-5-(2-(1-phenylethylamino)-4-pyridyl)-1,3-thiazol-2-yl]-2-methoxynicotinamide,
   N-[5-(2-benzylamino-4-pyridyl)-4-(3-methylphenyl)-1,3-thiazol-2-yl]nicotinamide,
   N-[5-(2-benzylamino-4-pyridyl)-4-(3-methylphenyl)-1,3-thiazol-2-yl]-2-methoxynicotinamide,
   N-[5-(2-benzylamino-4-pyridyl)-4-(3-methylphenyl)-1,3-thiazol-2-yl]-2-chloronicotinamide,
   N-[5-(2-benzylamino-4-pyridyl)-4-(3-methylphenyl)-1,3-thiazol-2-yl]-2-methylnicotinamide,
   N-[5-(2-benzoylamino-4-pyridyl)-4-(3-methylphenyl)-1,3-thiazol-2-yl]nicotinamide,
   N-[5-(2-benzoylamino-4-pyridyl)-4-(3-methylphenyl)-1,3-thiazol-2-yl]-2-methylnicotinamide,
   N-[5-(2-benzoylamino-4-pyridyl)-4-(3-methylphenyl)-1,3-thiazol-2-yl]-2-chloronicotinamide,
   N-[5-(2-benzoylamino-4-pyridyl)-4-(3-methylphenyl)-1,3-thiazol-2-yl]-2-methoxynicotinamide,
   (S)-N-(1-phenylethyl)-4-[2-ethyl-4-(3-methylphenyl)-1,3-thiazol-5-yl]-2-pyridylamine,
   (R)-N-(1-phenylethyl)-4-[2-ethyl-4-(3-methylphenyl)-1,3-thiazol-5-yl]-2-pyridylamine,
   (S)-N-(1-phenylethyl)-4-[4-(3-methylphenyl)-2-propyl-1,3-thiazol-5-yl]-2-pyridylamine,
   (R)-N-(1-phenylethyl)-4-[4-(3-methylphenyl)-2-propyl-1,3-thiazol-5-yl]-2-pyridylamine,
   (S)-N-(1-phenylethyl)-4-[2-butyl-4-(3-methylphenyl)-1,3-thiazol-5-yl]-2-pyridylamine,
   (R)-N-(1-phenylethyl)-4-[2-butyl-4-(3-methylphenyl)-1,3-thiazol-5-yl]-2-pyridylamine,
   (S)-N-(1-phenylethyl)-4-[4-(3-methylphenyl)-2-(4-methylthiophenyl)-1,3-thiazol-5-yl]-2-pyridylamine,
   (R)-N-(1-phenylethyl)-4-[4-(3-methylphenyl)-2-(4-methylthiophenyl)-1,3-thiazol-5-yl]-2-pyridylamine,
   (S)-N-(1-phenylethyl)-4-[4-(3-methylphenyl)-2-(4-methylsulfonylphenyl)-1,3-thiazol-5-yl]-2-pyridylamine,
   (R)-N-(1-phenylethyl)-4-[4-(3-methylphenyl)-2-(4-methylsulfonylphenyl)-1,3-thiazol-5-yl]-2-pyridylamine,
   (S)-N-(1-phenylethyl)-4-[2-(4-fluorophenyl)-4-(3-methylphenyl)-1,3-thiazol-5-yl]-2-pyridylamine,
   (R)-N-(1-phenylethyl)-4-[2-(4-fluorophenyl)-4-(3-methylphenyl)-1,3-thiazol-5-yl]-2-pyridylamine, or a salt thereof,
[14] the agent of [3], which is an agent for the prophylaxis or treatment of (1) postmenopausal or senile primary osteoporosis, (2) secondary osteoporosis caused by inflammation, blood system malignant disease, endocrine disorder or administration of pharmaceutical agent, (3) bone or joint tissue destruction or deforming associated with bone metastasis of tumor or rheumatism, (4) Paget's disease or (5) hypercalcemia,
[15] a method for the prophylaxis or treatment of pain, which comprises administering an effective amount of p38 MAP kinase inhibitor and/or the TNF-α production inhibitor to a mammal,
[16] a method for the suppression of activation and/or inhibition of formation of osteoclast, which comprises administering an effective amount of p38 MAP kinase inhibitor and/or the TNF-α production inhibitor to a mammal,
[17] a method for the prophylaxis or treatment of (1) postmenopausal or senile primary osteoporosis, (2) secondary osteoporosis caused by inflammation, blood system malignant disease, endocrine disorder or administration of pharmaceutical agent, (3) bone or joint tissue destruction or deforming associated with bone metastasis of tumor or rheumatism, (4) Paget's disease or (5) hypercalcemia, which comprises administering an effective amount of p38 MAP kinase inhibitor and/or the TNF-α production inhibitor to a mammal,
[18] use of a p38 MAP kinase inhibitor and/or the TNF-α production inhibitor for the production of an agent for the prophylaxis or treatment of pain,
[19] use of a p38 MAP kinase inhibitor and/or the TNF-α production inhibitor for the production of an agent for the suppression of activation and/or inhibition of formation of osteoclast, and
[20] use of a p38 MAP kinase inhibitor and/or the TNF-α production inhibitor for the production of an agent for the prophylaxis or treatment of (1) postmenopausal or senile primary osteoporosis, (2) secondary osteoporosis caused by inflammation, blood system malignant disease, endocrine disorder or administration of pharmaceutical agent, (3) bone or joint tissue destruction or deforming associated with bone metastasis of tumor or rheumatism, (4) Paget's disease or (5) hypercalcemia,
[21] a method for reducing a P450 inhibitory action of a compound containing a pyridyl group or a salt thereof, which comprises introducing a substituent into the α-position of a nitrogen atom of the pyridyl group of the compound or a salt thereof,
[22] a method for reducing a P450 inhibitory action of a compound containing a pyridyl group and an aromatic hydrocarbon group, or a salt thereof, which comprises introducing a polar group into the aromatic hydrocarbon group of the compound or a salt thereof,
[23] the method of [22], further comprising introducing a substituent into the α-position of a nitrogen atom of the pyridyl group,
[24] the method of [21] or [22], wherein the P450 is CYP2C9, CYP2D6 or CYP3A4,
[25] the method of [21] or [23], wherein the substituent is 1 to 3 selected from
   (i) halogen atom,
   (ii) C₁₋₆ alkyl group, C₂₋₆ alkenyl group, C₂₋₆ alkynyl group, C₃₋₆ cycloalkyl group, C₆₋₁₄ aryl group and C₇₋₁₆ aralkyl group [these groups may have 1 to 5 substituents selected from a group consisting of oxo, halogen atom, C₁₋₃ alkylenedioxy, nitro, cyano, optionally halogenated C₁₋₆ alkyl, optionally halogenated C₂₋₆ alkenyl, carboxy C₂₋₆ alkenyl, optionally halogenated C₂₋₆ alkynyl, optionally halogenated C₃₋₆ cycloalkyl, C₆₋₁₄ aryl, optionally halogenated C₁₋₈ alkoxy, C₁₋₆ alkoxy-carbonyl-C₁₋₆ alkoxy, hydroxy, C₆₋₁₄ aryloxy, C₇₋₁₆ aralkyloxy, mercapto, optionally halogenated C₁₋₆ alkylthio, C₆₋₁₄ arylthio, C₇₋₁₆ aralkylthio, amino, mono-C₁₋₆ alkylamino, mono-C₆₋₁₄ arylamino, di-C₁₋₆ alkylamino, di-C₆₋₁₄ arylamino, formyl, carboxy, C₁₋₆ alkyl-carbonyl, C₃₋₆ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl, carbamoyl, thiocarbamoyl, mono-C₁₋₆ alkyl-carbamoyl, di-C₁₋₆ alkyl-carbamoyl, C₆₋₁₄ aryl-carbamoyl, 5- or 6-membered heterocyclic carbamoyl, C₁₋₆ alkylsulfonyl, C₆₋₁₄ arylsulfonyl, C₁₋₆ alkylsulfinyl, C₆₋₁₄ arylsulfinyl, formylamino, C₁₋₆ alkyl-carbonylamino, C₆₋₁₄ aryl-carbonylamino, C₁₋₆ alkoxy-carbonylamino, C₁₋₆ alkylsulfonylamino, C₆₋₁₄ arylsulfonylamino, C₁₋₆ alkyl-carbonyloxy, C₆₋₁₄ aryl-carbonyloxy, C₁₋₆ alkoxy-carbonyloxy, mono-C₁₋₆ alkyl-carbamoyloxy, di-C₁₋₆ alkyl-carbamoyloxy, C₆₋₁₄ aryl-carbamoyloxy, nicotinoyloxy, 5- to 7-membered saturated cyclic amino containing 1 to 4 of 1 or 2 kinds of hetero atoms selected from nitrogen atom, sulfur atom and oxygen atom, besides one nitrogen atom and carbon atom (this cyclic amino may have substituents selected from the group consisting of C₁₋₆ alkyl, C₆₋₁₄ aryl, C₁₋₆ alkyl-carbonyl, 5- to 10-membered aromatic heterocyclic group and oxo), and 5-to 10-membered aromatic heterocyclic group, containing 1 to 4 of 1 or 2 kinds of hetero atoms selected from nitrogen atom, sulfur atom and oxygen atom, besides carbon atom, sulfo, sulfamoyl, sulfinamoyl and sulfenamoyl (substituent group A)],
   (iii) 5- to 14-membered heterocyclic group containing 1 to 4 of 1 or 2 kinds of hetero atoms selected from nitrogen atom, sulfur atom and oxygen atom, besides carbon atom, which may have 1 to 3 substituents selected from substituent group A,
   (iv) acyl group represented by the formula: -(C=O)-R⁵, -(C=O)-OR⁵, -(C=O)-NR⁵R⁶, -(C=S)-NHR⁵ or -SO₂-R⁷
      wherein R⁵ is (1) hydrogen atom, (2) C₁₋₆ alkyl group, C₂₋₆ alkenyl group, C₂₋₆ alkynyl group, C₃₋₆ cycloalkyl group, C₆₋₁₄ aryl group or C₇₋₁₆ aralkyl group, which may have 1 to 3 substituents selected from substituent group A or (3) 5- to 14-membered heterocyclic group containing 1 to 4 of 1 or 2 kinds of hetero atoms selected from nitrogen atom, sulfur atom and oxygen atom, besides carbon atom, which may have 1 to 3 substituents selected from substituent group A, R⁶ is hydrogen atom or C₁₋₆ alkyl group, and R⁷ is (1) C₁₋₆ alkyl group, C₂₋₆ alkenyl group, C₂₋₆ alkynyl group, C₃₋₆ cycloalkyl group, C₆₋₁₄ aryl group or C₇₋₁₆ aralkyl group, which may have 1 to 3 substituents selected from substituent group A or (3) 5- to 14-membered heterocyclic group containing 1 to 4 of 1 or 2 kinds of hetero atoms selected from nitrogen atom, sulfur atom and oxygen atom, besides carbon atom, which may have 1 to 3 substituents selected from substituent group A,
   (v) amino group (this amino group may have 1 or 2 substituents selected from (1) C₁₋₆ alkyl group, C₂₋₆ alkenyl group, C₂₋₆ alkynyl group, C₃₋₆ cycloalkyl group, C₆₋₁₄ aryl group or C₇₋₁₆ aralkyl group, which may have 1 to 3 substituents selected from substituent group A, (2) 5- to 14-membered heterocyclic group containing 1 to 4 of 1 or 2 kinds of hetero atoms selected from nitrogen atom, sulfur atom and oxygen atom, besides carbon atom, which may have 1 to 3 substituents selected from substituent group A, and (3) acyl group shown by the above-mentioned (iv)),
   (vi) 5- to 7-membered non-aromatic cyclic amino group containing 1 to 4 of 1 or 2 kinds of hetero atoms selected from nitrogen atom, sulfur atom and oxygen atom, besides one nitrogen atom and carbon atom, (this cyclic amino group may have 1 to 3 substituents selected from C₁₋₆ alkyl, C₆₋₁₄ aryl, C₁₋ ₆ alkyl-carbonyl, 5- to 10-membered aromatic heterocyclic group and oxo), and
   (vii) C₁₋₆ alkoxy group, C₆₋₁₄ aryloxy group and C₇₋₁₆ aralkyloxy group, which may have 1 to 3 substituents selected from substituent group A,
[26] the method of [22], wherein the polar group is 1 to 3 selected from (1) halogen atom, (2) hydroxy, (3) amino optionally having 1 or 2 substituents selected from a substituent selected from substituent group A and acyl shown by the above-mentioned (iv), (4) nitro, (5) carboxy, (6) formyl, (7) C₁₋₆ alkoxy optionally having 1 to 3 substituents selected from substituent group A, (8) C₁₋₆ alkoxy-carbonyl optionally having 1 to 3 substituents selected from substituent group A, (9) cyano and (10) C₁₋₆ alkyl or C₆₋₁₄ aryl having 1 to 3 groups from the above-mentioned (1)-(9) as substituents.
   The present invention further relates to
[27] the agent of [1], wherein the p38 MAP kinase inhibitor and/or the TNF-α production inhibitor are/is an optionally N-oxidized compound represented by the formula: 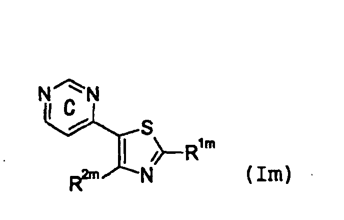 wherein
   - ring C: is a 4-pyrimidinyl group optionally having substituents;
   - R^{1m}: is a hydrogen atom, a hydrocarbon group optionally having substituents, a heterocyclic group optionally having substituents, an amino group optionally having substituents or an acyl group; and
   - R^{2m}: is an aromatic group optionally having substituents,
   or a salt thereof, or a prodrug thereof.
[28] The agent of [27], wherein the compound (Im) is an optionally N-oxidized compound represented by the formula: 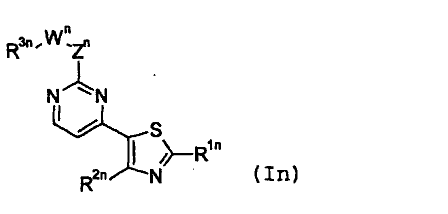 wherein
   - Zⁿ: is a bond, -NR⁴ⁿ- (R⁴ⁿ is a hydrogen atom or a hydrocarbon group optionally having substituents), an oxygen atom or an optionally oxidized sulfur atom;
   - Wⁿ: is a bond or a divalent hydrocarbon group optionally having substituents;
   - R¹ⁿ: is a hydrogen atom, a hydrocarbon group optionally having substituents, a heterocyclic group optionally having substituents, an amino group optionally having substituents or an acyl group;
   - R²ⁿ: is an aromatic group optionally having substituents; and
   - R³ⁿ: is a hydrogen atom, a hydrocarbon group optionally having substituents or a heterocyclic group optionally having substituents,
   or a salt thereof.
[29] The agent of [28], wherein both Wⁿ and Zⁿ are each a bond.
[30] The agent of [27], wherein the compound (Im) is an optionally N-oxidized compound represented by the formula: 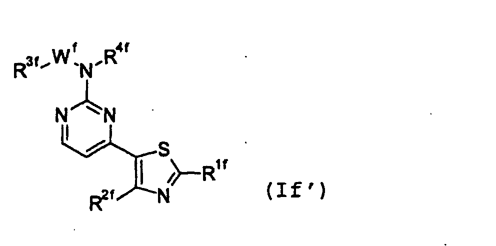 wherein
   - W^{f}: is a bond or a divalent hydrocarbon group optionally having substituents;
   - R^{1f}: is a hydrogen atom, a hydrocarbon group optionally having substituents, a heterocyclic group optionally having substituents, an amino group optionally having substituents or an acyl group;
   - R^{2f}: is an aromatic group optionally having substituents;
   - R^{3f}: is a hydrocarbon group optionally having substituents or a heterocyclic group optionally having substituents; and
   - R^{4f}: is a hydrogen atom or a hydrocarbon group optionally having substituents,
   or a salt thereof.
[31] The agent of [30], wherein the compound (If') is an optionally N-oxidized compound represented by the formula: 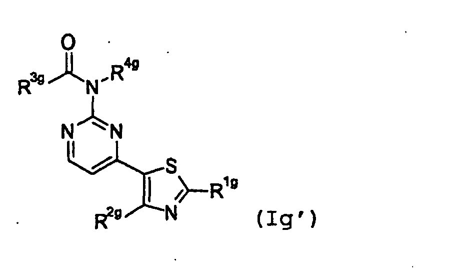 wherein
   - R^{1g}: is a hydrogen atom, a hydrocarbon group optionally having substituents, a heterocyclic group optionally having substituents, an amino group optionally having substituents or an acyl group;
   - R^{2g}: is an aromatic group optionally having substituents;
   - R^{3g}: is a hydrocarbon group optionally having substituents or a heterocyclic group optionally having substituents; and
   - R^{4g}: is a hydrogen atom or a hydrocarbon group optionally having substituents,
   or a salt thereof.
[32] The agent of [30], wherein the compound (If') is an optionally N-oxidized compound represented by the formula: 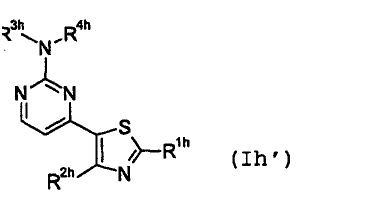 wherein
   - R^{1h}: is a hydrogen atom, a hydrocarbon group optionally having substituents, a heterocyclic group optionally having substituents, an amino group optionally having substituents or an acyl group;
   - R^{2h}: is an aromatic group optionally having substituents;
   - R^{3h}: is a hydrocarbon group optionally having substituents or a heterocyclic group optionally having substituents; and R^{4h} is a hydrogen atom or a hydrocarbon group optionally having substituents,
   or a salt thereof.

While the p38 MAP kinase inhibitor and/or the TNF-α production inhibitor to be used in the present invention are/is not particularly limited as long as the inhibitor(s) has(ve) a p38 MAP kinase inhibitory activity and/or a TNF-α production inhibitory activity, and exemplified by, for example, the following compounds (I)-(VII) and the like.

### [compound (I)]

(1) a 1,3-thiazole compound substituted at the 5-position by a pyridyl group optionally having substituents or a salt thereof,
(2) a 1,3-thiazole compound substituted at the 5-position by a pyridyl group optionally having substituents or a salt thereof, excluding a compound of the formula 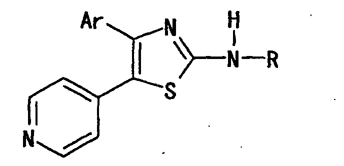 wherein Ar is an unsubstituted or substituted aryl group bonded to a thiazole ring by a carbon atom of an aromatic ring, and R is a hydrogen atom, an acyl group, or a monovalent aromatic group having not more than 10 carbon atoms, which is bonded to a nitrogen atom by a carbon atom of the aromatic ring, and a salt thereof,
(3) the compound of (1) or (2), wherein the 1,3-thiazole compound is a 1,3-thiazole compound substituted at the 4-position by an aromatic group optionally having substituents,
(4) the compound of (1) or (2), wherein the 1,3-thiazole compound is a 1,3-thiazole compound substituted at the 2-position by an aryl group optionally having substituents or an amino group optionally having substituents,
(5) the compound of (1) or (2), wherein the 1,3-thiazole compound is a compound of the formula 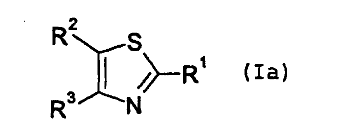 wherein R¹ represents a hydrogen atom, a hydrocarbon group optionally having substituents, a heterocyclic group optionally having substituents, an amino group optionally having substituents or an acyl group;
   R² represents a pyridyl group optionally having substituents; and
   R³ represents an aromatic group optionally having substituents, or a salt thereof,
(6) the compound of (5), wherein R¹ is
   (i) a hydrogen atom,
   (ii) a C₁₋₁₀ alkyl group, a C₂₋₆ alkenyl group, a C₂₋₆ alkynyl group, a C₃₋₆ cycloalkyl group, a C₆₋₁₄ aryl group or a C₇₋₁₆ aralkyl group [these groups may have substituents selected from the group (substituent group A) consisting of oxo, halogen atom, C₁₋₃ alkylenedioxy, nitro, cyano, optionally halogenated C₁₋₆ alkyl, optionally halogenated C₂₋₆ alkenyl, carboxy C₂₋₆ alkenyl, optionally halogenated C₂₋₆ alkynyl, optionally halogenated C₃₋₆ cycloalkyl, C₆₋₁₄ aryl, optionally halogenated C₁₋₈ alkoxy, C₁₋₆ alkoxy-carbonyl-C₁₋₆ alkoxy, hydroxy, C₆₋₁₄ aryloxy, C₇₋₁₆ aralkyloxy, mercapto, optionally halogenated C₁₋₆ alkylthio, C₆₋₁₄ arylthio, C₇₋₁₆ aralkylthio, amino, mono-C₁₋₆ alkylamino, mono-C₆₋₁₄ arylamino, di-C₁₋₆ alkylamino, di-C₆₋₁₄ arylamino, formyl, carboxy, C₁₋₆ alkyl-carbonyl, C₃₋₆ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5 or 6 membered heterocyclic carbonyl, carbamoyl, thiocarbamoyl, mono-C₁₋₆ alkyl-carbamoyl, di-C₁₋₆ alkyl-carbamoyl, C₆₋₁₄ aryl-carbamoyl, 5 or 6 membered heterocyclic carbamoyl, C₁₋ ₆ alkylsulfonyl, C₆₋₁₄ arylsulfonyl, C₁₋₆ alkylsulfinyl, C₆₋₁₄ arylsulfinyl, formylamino, C₁₋₆ alkyl-carbonylamino, C₆₋₁₄ aryl-carbonylamino, C₁₋₆ alkoxy-carbonylamino, C₁₋₆ alkylsulfonylamino, C₆₋₁₄ arylsulfonylamino, C₁₋₆ alkyl-carbonyloxy, C₆₋₁₄ aryl-carbonyloxy, C₁₋₆ alkoxy-carbonyloxy, mono-C₁₋₆ alkyl-carbamoyloxy, di-C₁₋₆ alkyl-carbamoyloxy, C₆₋₁₄ aryl-carbamoyloxy, nicotinoyloxy, 5 to 7 membered saturated cyclic amino optionally having 1 to 4 of one or two kinds of heteroatom(s) selected from a nitrogen atom, a sulfur atom and an oxygen atom in addition to one nitrogen atom and carbon atoms (this cyclic amino may have substituents selected from the group consisting of C₁₋₆ alkyl, C₆₋₁₄ aryl, C₁₋₆ alkyl-carbonyl, 5 to 10 membered aromatic heterocyclic group and oxo), 5 to 10 membered aromatic heterocyclic group containing 1 to 4 of one or two kinds of heteroatom(s) selected from a nitrogen atom, a sulfur atom and an oxygen atom, in addition to carbon atoms, sulfo, sulfamoyl, sulfinamoyl and sulfenamoyl],
   (iii) a monovalent heterocyclic group obtained by removing one arbitrary hydrogen atom from a 5 to 14 membered heterocycle containing 1 to 4 of one or two kinds of heteroatom(s) selected from a nitrogen atom, a sulfur atom and an oxygen atom in addition to carbon atoms optionally having substituents selected from the above-mentioned substituent group A,
   (iv) an acyl group represented by the formula:
      -(C=O)-R⁵, -(C=O)-OR⁵, -(C=O)-NR⁵R⁶, -(C=S)-NHR⁵ or -SO₂-R⁷ wherein R⁵ represents (a) a hydrogen atom, (b) a C₁₋₆ alkyl group, a C₂₋₆ alkenyl group, a C₂₋₆ alkynyl group, a C₃₋₆ cycloalkyl group, a C₆₋₁₄ aryl group or a C₇₋₁₆ aralkyl group as defined in the above (ii) or (c) a heterocyclic group as defined in the above (iii), R⁶ represents a hydrogen atom or a C₁₋₆ alkyl group, R⁷ represents (a) a C₁₋₆ alkyl group, a C₂₋₆ alkenyl group, a C₂₋₆ alkynyl group, a C₃₋₆ cycloalkyl group, a C₆₋₁₄ aryl group or a C₇₋₁₆ aralkyl group as defined in the above (ii), or (b) a heterocyclic group as defined in the above (iii) ,
   (v) an amino group (this amino group may have substituents selected from the group consisting of (a) a C₁₋₆ alkyl group, a C₂₋₆ alkenyl group, a C₂₋₆ alkynyl group, a C₃₋₆ cycloalkyl group, a C₆₋₁₄ aryl group or a C₇₋₁₆ aralkyl group as defined in the above (ii), (b) a heterocyclic group as defined in the above (iii), (c) an acyl group as defined in the above (iv), and (d) a C₁₋₆ alkylidene group optionally having substituents selected from the above substituent group A), or
   (vi) a 5 to 7 membered non-aromatic cyclic amino group optionally containing 1 to 4 of one or two kinds of heteroatom(s) selected from a nitrogen atom, a sulfur atom and an oxygen atom in addition to one nitrogen atom and carbon atoms (this cyclic amino group may have substituents selected from the group consisting of C₁₋₆ alkyl, C₆₋₁₄ aryl, C₁₋₆ alkyl-carbonyl, 5 to 10 membered aromatic heterocyclic group and oxo);
   R² represents a pyridyl group optionally having substituents selected from the above substituent group A; and
   R³ represents (a) a C₆₋₁₄ monocyclic or fused polycyclic aromatic hydrocarbon group optionally having substituents selected from the substituent group A or (b) a monovalent aromatic heterocyclic group obtained by removing one arbitrary hydrogen atom from a 5 to 14 membered aromatic heterocycle containing 1 to 4 of one or two kinds of heteroatom(s) selected from a nitrogen atom, a sulfur atom and an oxygen atom in addition to carbon atoms, said 5 to 14 membered aromatic heterocycle optionally having substituents selected from the substituent group A,
(7) the compound of (5), wherein
   R¹ is (a) a C₆₋₁₄ aryl group (preferably C₆₋₁₀ aryl) optionally having 1 to 5 substituents selected from halogen atom, optionally halogenated C₁₋₆ alkyl, carboxy C₂₋₆ alkenyl, optionally halogenated C₁₋₆ alkoxy, C₁₋₆ alkoxy-carbonyl-C₁₋₆ alkoxy, hydroxy, amino, mono- or di-C₁₋₆ alkylamino, carboxy, C₁₋₆ alkoxy-carbonyl, mono- or di-C₁₋₆ alkyl-carbamoyl, C₆₋₁₄ aryl-carbonylamino, C₁₋₃ alkylenedioxy, C₁₋₆ alkylthio, C₆₋₁₄ arylthio, C₁₋₆ alkylsulfinyl, C₆₋₁₄ arylsulfinyl, C₁₋₆ alkylsulfonyl, C₆₋₁₄ arylsulfonyl and nitro,
   (b) a C₁₋₈ alkyl group optionally having 1 to 5 substituents selected from halogen atom, optionally halogenated C₁₋₆ alkyl, carboxy C₂₋₆ alkenyl, optionally halogenated C₁₋₆ alkoxy, C₁₋₆ alkoxy-carbonyl-C₁₋₆ alkoxy, hydroxy, amino, mono- or di-C₁₋₆ alkylamino, carboxy, C₁₋₆ alkoxy-carbonyl, mono- or di-C₁₋₆ alkyl-carbamoyl and C₆₋₁₄ aryl-carbonylamino,
   (c) a C₃₋₆ cycloalkyl group (e.g., cyclohexyl) optionally having 1 to 5 substituents selected from halogen atom, optionally halogenated C₁₋₆ alkyl, carboxy C₂₋₆ alkenyl, optionally halogenated C₁₋₆ alkoxy, C₁₋₆ alkoxy-carbonyl-C₁₋₆ alkoxy, hydroxy, amino, mono- or di-C₁₋₆ alkylamino, carboxy, C₁₋₆ alkoxy-carbonyl, mono- or di-C₁₋₆ alkyl-carbamoyl and C₆₋₁₄ aryl-carbonylamino,
   (d) a C₇₋₁₆ aralkyl group (e.g., phenyl-C₁₋₆ alkyl group),
   (e) a 5 to 10 membered aromatic heterocyclic group containing 1 to 4 of one or two kinds of heteroatom(s) selected from a nitrogen atom, a sulfur atom and an oxygen atom in addition to carbon atoms (e.g., 5 or 6 membered aromatic heterocyclic group such as pyridyl, thienyl and the like),
   (f) a 5 to 10 membered non-aromatic heterocyclic group containing 1 or 2 of one or two kinds of heteroatom(s) selected from a nitrogen atom, a sulfur atom and an oxygen atom in addition to carbon atoms, said 5 to 10 membered non-aromatic heterocyclic group may have C₆₋₁₄ aryl (e.g., phenyl), C₁₋₆ alkyl-carbonyl or oxo (e.g., 5 or 6 membered non-aromatic cyclic amino group such as piperidino, piperazino and the like),
   (g) an amino group optionally having 1 or 2 substituents selected from the group consisting of the following (1) to (7) [(1) C₁₋₆ alkyl, (2) C₆₋₁₄ aryl, (3) C₇₋₁₆ aralkyl, (4) 5 or 6 membered heterocyclic group containing 1 or 2 heteroatom(s) selected from a nitrogen atom, a sulfur atom and an oxygen atom in addition to carbon atoms (e.g., pyridyl), (5) C₁₋₆ alkyl-carbonyl, C₃₋₆ cycloalkyl-carbonyl, C₆₋₁₄ aryl-carbonyl, C₇₋₁₆ aralkyl-carbonyl, C₁₋₆ alkyl-carbamoyl or 5 or 6 membered heterocyclic carbonyl group, each optionally having 1 to 3 substituents selected from halogen atom, C₁₋₆ alkyl, C₁₋₆ alkoxy, carboxy, C₁₋₆ alkoxy-carbonyl, cyano, tetrazine and the like, (6) C₆₋₁₄ aryl-carbamoyl group optionally having 1 to 3 substituents selected from halogen atom, C₁₋₆ alkyl, C₁₋₆ alkoxy, carboxy, C₁₋₆ alkoxy-carbonyl, cyano, nitro, mono- or di-C₁₋₆ alkylamino and the like and (7) di-C₁₋₆ alkylamino-C₁₋₆ alkylidene], or
   (h) a carboxy group,
(8) the compound of (5), wherein R¹ is a C₆₋₁₄ aryl group optionally having C₁₋₆ alkylsulfonyl,
(9) the compound of (5), wherein R² is a 4-pyridyl group optionally having substituents,
(10) the compound of (5), wherein R³ is a C₆₋₁₀ aryl group optionally having substituents,
(11) the compound of (5), wherein R³ is a phenyl group optionally having substituents,
(12) the compound of (5), wherein R³ is a C₆₋₁₄ aryl group optionally having substituents selected from the group consisting of halogen atom, C₁₋₃ alkylenedioxy, optionally halogenated C₁₋₆ alkyl, carboxy C₂₋₆ alkenyl, optionally halogenated C₁₋₈ alkoxy, carboxy C₁₋₈ alkoxy, hydroxy, C₆₋₁₄ aryloxy, C₁₋₆ alkoxy-carbonyl, C₁₋₆ alkyl-carbonyloxy, mono- or di-C₁₋₆ alkylamino and C₁₋₆ alkoxy-carbonyl-C₁₋₆ alkoxy,
(13) the compound of (5), wherein R³ is a phenyl group optionally having substituents selected from the group consisting of halogen atom and C₁₋₆ alkyl group,
(14) the compound of (5), wherein R¹ is (a) an amino group optionally having 1 or 2 acyl groups represented by the formula: -(C=O)-R⁵ or -(C=O)-NR⁵R⁶ wherein each symbol is as defined above, (b) C₆₋₁₄ aryl group optionally having 1 to 5 substituents selected from C₁₋₆ alkylthio, C₆₋₁₄ arylthio, C₁₋₆ alkylsulfinyl, C₆₋₁₄ arylsulfinyl, C₁₋₆ alkylsulfonyl, C₆₋₁₄ arylsulfonyl and carboxy or (c) C₁₋₆ alkyl group optionally substituted by halogen atom,
   R² is a pyridyl group, and
   R³ is a C₆₋₁₄ aryl group optionally having 1 to 5 substituents selected from halogen atom, optionally halogenated C₁₋₆ alkyl, optionally halogenated C₁₋₆ alkoxy and carboxy,
(15) the compound of (5), wherein R¹ is
   (i) C₁₋₈ alkyl, C₃₋₆ cycloalkyl or C₆₋₁₄ aryl, each optionally having 1 to 5 substituents selected from halogen atom, optionally halogenated C₁₋₆ alkyl, carboxy C₂₋₆ alkenyl, optionally halogenated C₁₋₆ alkoxy, C₁₋₆ alkoxy-carbonyl-C₁₋₆ alkoxy, hydroxy, amino, mono- or di-C₁₋₆ alkylamino, carboxy, C₁₋₆ alkoxy-carbonyl, mono- or di-C₁₋₆ alkyl-carbamoyl and C₆₋₁₄ aryl-carbonylamino,
   (ii) a 5 membered heterocyclic group,
   (iii) an amino group optionally having 1 or 2 substituents selected from (a) C₁₋₆ alkyl, (b) C₆₋₁₄ aryl, (c) C₇₋₁₆ aralkyl, (d) 6 membered heterocyclic group and (e) C₁₋₆ alkyl-carbonyl, C₃₋₆ cycloalkyl-carbonyl, C₆₋₁₄ aryl-carbonyl, C₇₋₁₆ aralkyl-carbonyl, C₁₋₆ alkyl-carbamoyl or 5 or 6 membered heterocyclic carbonyl, each optionally having 1 to 3 substituents selected from halogen atom, C₁₋₆ alkyl, C₁₋₆ alkoxy, carboxy and C₁₋₆ alkoxy-carbonyl, or an amino group optionally having di-C₁₋₆ alkylamino-C₁₋₆ alkylidene,
   (iv) a 5 or 6 membered non-aromatic cyclic amino group optionally substituted by C₁₋₆ alkyl-carbonyl or oxo, or
   (v) a carboxy group;
      R² is a pyridyl group; and
      R³ is a C₆₋₁₀ aryl group optionally having 1 to 3 substituents selected from halogen atom, C₁₋₃ alkylenedioxy, optionally halogenated C₁₋₆ alkyl, carboxy C₂₋₆ alkenyl, optionally halogenated C₁₋₈ alkoxy, hydroxy, C₇₋₁₆ aralkyloxy and C₁₋₆ alkyl-carbonyloxy (two adjacent alkyl groups as substituents may be bonded to form a 5 membered non-aromatic carbon ring),
(16) the compound of (5), wherein R¹ is a C₆₋₁₄ aryl group optionally having C₁₋₆ alkylsulfonyl, R² is a pyridyl group, and R³ is a C₆₋₁₄ aryl group optionally having halogen atom(s),
(17) N-ethyl-[4-(4-methoxyphenyl)-5-(4-pyridyl)-1,3-thiazol-2-yl]amine (Reference Example A 23-269),
   N-propyl-[4-(3,5-dimethylphenyl)-5-(4-pyridyl)-1,3-thiazol-2-yl]amine (Reference Example A 23-276),
   N-butyl-[4-(3,5-dimethylphenyl)-5-(4-pyridyl)-1,3-thiazol-2-yl]amine (Reference Example A 23-280),
   N-benzyl-[4-(3,5-dimethylphenyl)-5-(4-pyridyl)-1,3-thiazol-2-yl]amine (Reference Example A 23-281),
   N-propyl-[4-(4-methoxyphenyl)-5-(4-pyridyl)-1,3-thiazol-2-yl]amine (Reference Example A 23-290),
   N-isopropyl-[4-(4-methoxyphenyl)-5-(4-pyridyl)-1,3-thiazol-2-yl]amine (Reference Example A 23-291),
   N-[4-(4-methoxyphenyl)-5-(4-pyridyl)-1,3-thiazol-2-yl]-N'-phenylurea (Reference Example A 23-296),
   4-[[[4-(4-methoxyphenyl)-5-(4-pyridyl)-1,3-thiazol-2-yl]amino]carbonyl]benzoic acid (Reference Example A 23-299),
   methyl 4-[2-[4-(methylthio)phenyl]-5-(4-pyridyl)-1,3-thiazol-4-yl]phenyl ether (Reference Example A 23-300),
   4-[4-(3,5-dimethylphenyl)-5-(4-pyridyl)-1,3-thiazol-2-yl]phenyl methyl sulfide (Reference Example A 23-302),
   4-[4-(4-methoxyphenyl)-5-(4-pyridyl)-1,3-thiazol-2-yl]phenyl methyl sulfoxide (Reference Example A 23-303),
   4-[4-(3,5-dimethylphenyl)-5-(4-pyridyl)-1,3-thiazol-2-yl]phenyl methyl sulfoxide (Reference Example A 23-305),
   4-[4-(4-methoxyphenyl)-5-(4-pyridyl)-1,3-thiazol-2-yl]phenyl methyl sulfone (Reference Example A 23-306),
   4-[4-(3,5-dimethylphenyl)-5-(4-pyridyl)-1,3-thiazol-2-yl]phenyl methyl sulfone (Reference Example A 23-308),
   4-[4-(4-fluorophenyl)-5-(4-pyridyl)-1,3-thiazol-2-yl]phenyl methyl sulfide (Reference Example A 23-309),
   4-[4-(4-chlorophenyl)-5-(4-pyridyl)-1,3-thiazol-2-yl]phenyl methyl sulfide (Reference Example A 23-310),
   4-[4-(4-fluorophenyl)-5-(4-pyridyl)-1,3-thiazol-2-yl]phenyl methyl sulfoxide (Reference Example A 23-311),
   4-[4-(4-chlorophenyl)-5-(4-pyridyl)-1,3-thiazol-2-yl]phenyl methyl sulfoxide (Reference Example A 23-312),
   4-[4-(4-fluorophenyl)-5-(4-pyridyl)-1,3-thiazol-2-yl]phenyl methyl sulfone (Reference Example A 23-313),
   4-[4-(4-chlorophenyl)-5-(4-pyridyl)-1,3-thiazol-2-yl]phenyl methyl sulfone (Reference Example A 23-314),
   N-[4-(3,5-dimethylphenyl)-5-(4-pyridyl)-1,3-thiazol-2-yl]-N'-phenylurea (Reference Example A 23-315),
   2-hydroxy-N-[4-(4-methoxyphenyl)-5-(4-pyridyl)-1,3-thiazol-2-yl]propionamide (Reference Example A 23-325),
   4-[4-(3,4-dimethylphenyl)-5-(4-pyridyl)-1,3-thiazol-2-yl]phenyl methyl sulfide (Reference Example A 23-326),
   4-[4-(3,4-dimethylphenyl)-5-(4-pyridyl)-1,3-thiazol-2-yl]phenyl methyl sulfoxide (Reference Example A 23-327),
   4-[4-(3,4-dimethylphenyl)-5-(4-pyridyl)-1,3-thiazol-2-yl]phenyl methyl sulfone (Reference Example A 23-328),
   2-hydroxy-N-[4-(4-methoxyphenyl)-5-(4-pyridyl)-1,3-thiazol-2-yl]acetamide (Reference Example A 23-329),
   4-[[[4-(3,5-dimethylphenyl)-5-(4-pyridyl)-1,3-thiazol-2-yl]amino]carbonyl]benzoic acid (Reference Example A 23-337),
   3-[[[4-(3,5-dimethylphenyl)-5-(4-pyridyl)-1,3-thiazol-2-yl]amino]carbonyl]benzoic acid (Reference Example A 23-342),
   4-(4-fluorophenyl)-2-phenyl-5-(4-pyridyl)-1,3-thiazole (Reference Example A 44-1),
   methyl 4-[4-(3-methylphenyl)-5-(4-pyridyl)-1,3-thiazol-2-yl]phenyl sulfide (Reference Example A 44-7),
   methyl 4-[4-(3-methylphenyl)-5-(4-pyridyl)-1,3-thiazol-2-yl]phenyl sulfoxide (Reference Example A 44-8),
   methyl 4-[4-(3-methylphenyl)-5-(4-pyridyl)-1,3-thiazol-2-yl]phenyl sulfone (Reference Example A 44-26), or a salt thereof,

As "acyl group", for example, there are an acyl group represented by the formula:
-(C=O)-R⁵, -(C=O)-OR⁵, -(C=O)-NR⁵R⁶, -(C=S)-NHR⁵ or -SO₂-R⁷ (wherein R⁵ represents a hydrogen atom, a hydrocarbon group optionally having substituents or a heterocyclic group optionally having substituents, R⁶ represents a hydrogen atom or a C₁₋₆ alkyl, R⁷ represents a hydrocarbon group optionally having substituents or a heterocyclic group optionally having substituents) and the like.

In the aforementioned formula, as "hydrocarbon group" of "hydrocarbon group optionally having substituents" represented by R⁵, for example, there are an acyclic or cyclic hydrocarbon group (for example, alkyl, alkenyl, alkynyl, cycloalkyl, aryl, aralkyl and the like) and the like. Among them, acyclic or cyclic hydrocarbon groups having 1 to 16 carbon atom(s) are preferable.

As "alkyl", for example, C₁₋₆ alkyl (for example, methyl, ethyl, propyl, isopropyl, butyl, isobutyl, sec-butyl, tert-butyl, pentyl, hexyl and the like) and the like are preferable.

As "alkenyl", for example, C₂₋₆ alkenyl (for example, vinyl, allyl, isopropenyl, 1-butenyl, 2-butenyl, 3-butenyl, 2-methyl-2-propenyl, 1-methyl-2-propenyl, 2-methyl-1-propenyl and the like) and the like are preferable.

As "alkynyl", for example, C₂₋₆ alkynyl (for example, ethynyl, propargyl, 1-butynyl, 2-butynyl, 3-butynyl, 1-hexynyl and the like) and the like are preferable.

As "cycloalkyl", for example, C₃₋₆ cycloalkyl (for example, cyclopropyl, cyclobutyl, cyclopentyl, cyclohexyl and the like) and the like are preferable.

As "aryl", for example, C₆₋₁₄ aryl (for example, phenyl, 1-naphthyl, 2-naphthyl, 2-biphenylyl, 3-biphenylyl, 4-biphenylyl, 2-anthryl and the like) and the like are preferable.

As "aralkyl", for example, C₇₋₁₆ aralkyl (for example, benzyl, phenethyl, diphenylmethyl, 1-naphthylmethyl, 2-naphthylmethyl, 2,2-diphenylethyl, 3-phenylpropyl, 4-phenylbutyl, 5-phenylpentyl and the like) and the like are preferable.

As "substituents" of "hydrocarbon group optionally having substituents" represented by R⁵, for example, there are oxo, halogen atom (for example, fluorine, chlorine, bromine, iodine and the like), C₁₋₃ alkylenedioxy (for example, methylenedioxy, ethylenedioxy and the like), nitro, cyano, optionally halogenated C₁₋₆ alkyl, optionally halogenated C₂₋₆ alkenyl, carboxy C₂₋₆ alkenyl (for example, 2-carboxyethenyl, 2-carboxy-2-methylethenyl and the like), optionally halogenated C₂₋₆ alkynyl, optionally halogenated C₃₋₆ cycloalkyl, C₆₋₁₄ aryl (for example, phenyl, 1-naphthyl, 2-naphthyl, 2-biphenylyl, 3-biphenylyl, 4-biphenylyl, 2-anthryl and the like), optionally halogenated C₁₋₈ alkoxy, C₁₋₆ alkoxy-carbonyl-C₁₋₆ alkoxy (for example, ethoxycarbonylmethyloxy and the like), hydroxy, C₆₋₁₄ aryloxy (for example, phenyloxy, 1-naphthyloxy, 2-naphthyloxy and the like), C₇₋₁₆ aralkyloxy (for example, benzyloxy, phenethyloxy and the like), mercapto, optionally halogenated C₁₋₆ alkylthio, C₆₋₁₄ arylthio (for example, phenylthio, 1-naphthylthio, 2-naphthylthio and the like), C₇₋₁₆ aralkylthio (for example, benzylthio, phenethylthio and the like), amino, mono-C₁₋₆ alkylamino (for example, methylamino, ethylamino and the like), mono-C₆₋₁₄ arylamino (for example, phenylamino, 1-naphthylamino, 2-naphthylamino and the like), di-C₁₋₆ alkylamino (for example, dimethylamino, diethylamino, ethylmethylamino and the like), di-C₆₋₁₄ arylamino (for example, diphenylamino and the like), formyl, carboxy, carboxy-C₂₋₆ alkenyl, carboxy-C₁₋₆ alkyl, C₁₋₆ alkyl-carbonyl (for example, acetyl, propionyl and the like), C₃₋₆ cycloalkyl-carbonyl (for example, cyclopropylcarbonyl, cyclopentylcarbonyl, cyclohexylcarbonyl and the like), C₁₋₆ alkoxy-carbonyl (for example, methoxycarbonyl, ethoxycarbonyl, propoxycarbonyl, tert-butoxycarbonyl and the like), C₆₋₁₄ aryl-carbonyl (for example, benzoyl, 1-naphthoyl, 2-naphthoyl and the like), C₇₋₁₆ aralkyl-carbonyl (for example, phenylacetyl, 3-phenylpropionyl and the like), C₆₋₁₄ aryloxy-carbonyl (for example, phenoxycarbonyl and the like), C₇₋₁₆ aralkyloxy-carbonyl (for example, benzyloxycarbonyl, phenethyloxycarbonyl and the like), 5 or 6 membered heterocyclic carbonyl (for example, nicotinoyl, isonicotinoyl, thenoyl, furoyl, morpholinocarbonyl, thiomorpholinocarbonyl, piperazin-1-ylcarbonyl, pyrrolidin-1-ylcarbonyl and the like), carbamoyl, thiocarbamoyl, mono-C₁₋₆ alkyl-carbamoyl (for example, methylcarbamoyl, ethylcarbamoyl and the like), di-C₁₋₆ alkyl-carbamoyl (for example, dimethylcarbamoyl, diethylcarbamoyl, ethylmethylcarbamoyl and the like), mono- or di-C₆₋₁₄ aryl-carbamoyl (for example, phenylcarbamoyl, 1-naphthylcarbamoyl, 2-naphthylcarbamoyl and the like), mono- or di-5 or 6 membered heterocyclic carbamoyl (for example, 2-pyridylcarbamoyl, 3-pyridylcarbamoyl, 4-pyridylcarbamoyl, 2-thienylcarbamoyl, 3-thienylcarbamoyl and the like), C₁₋₆ alkylsulfonyl (for example, methylsulfonyl, ethylsulfonyl and the like), C₁₋₆ alkylsulfinyl (for example, methylsulfinyl, ethylsulfinyl and the like), C₆₋₁₄ arylsulfonyl (for example, phenylsulfonyl, 1-naphthylsulfonyl, 2-naphthylsulfonyl and the like), C₆₋₁₄ arylsulfinyl (for example, phenylsulfinyl, 1-naphthylsulfinyl, 2-naphthylsulfinyl and the like), formylamino, C₁₋₆ alkyl-carbonylamino (for example, acetylamino and the like), C₆₋₁₄ aryl-carbonylamino (for example, benzoylamino, naphthoylamino and the like), C₁₋₆ alkoxy-carbonylamino (for example, methoxycarbonylamino, ethoxycarbonylamino, propoxycarbonylamino, butoxycarbonylamino and the like), C₁₋₆ alkylsulfonylamino (for example, methylsulfonylamino, ethylsulfonylamino and the like), C₆₋₁₄ arylsulfonylamino (for example, phenylsulfonylamino, 2-naphthylsulfonylamino, 1-naphthylsulfonylamino and the like), C₁₋₆ alkyl-carbonyloxy (for example, acetoxy, propionyloxy and the like), C₆₋₁₄ aryl-carbonyloxy (for example, benzoyloxy, naphthylcarbonyloxy and the like), C₁₋₆ alkoxy-carbonyloxy (for example, methoxycarbonyloxy, ethoxycarbonyloxy, propoxycarbonyloxy, butoxycarbonyloxy and the like), mono-C₁₋₆ alkyl-carbamoyloxy (for example, methylcarbamoyloxy, ethylcarbamoyloxy and the like), di-C₁₋₆ alkyl-carbamoyloxy (for example, dimethylcarbamoyloxy, diethylcarbamoyloxy and the like), C₆₋₁₄ aryl-carbamoyloxy (for example, phenylcarbamoyloxy, naphthylcarbamoyloxy and the like), nicotinoyloxy, 5 to 7 membered saturated cyclic amino optionally having substituents, 5 to 10 membered aromatic heterocyclic group (for example, 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like), sulfo and the like.

The "hydrocarbon group" may have 1 to 5, preferably 1 to 3 aforementioned substituents at a substitutable position and, when the number of substituents is 2 or more, respective substituents may be the same or different.

As aforementioned "optionally halogenated C₁₋₆ alkyl", for example, there are C₁₋₆ alkyl (for example, methyl, ethyl, propyl, isopropyl, butyl, isobutyl, sec-butyl, tert-butyl, pentyl, hexyl and the like) and the like optionally having 1 to 5, preferably 1 to 3 halogen atom(s) (for example, fluorine, chlorine, bromine, iodine and the like). Examples thereof are methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl and the like.

As the aforementioned "optionally halogenated C₂₋₆ alkenyl", for example, there are C₂₋₆ alkenyl (for example, vinyl, propenyl, isopropenyl, 2-buten-1-yl, 4-penten-1-yl, 5-hexen-1-yl) and the like optionally having 1 to 5, preferably 1 to 3 halogen atom(s) (for example, fluorine, chlorine, bromine, iodine and the like).

As the aforementioned "optionally halogenated C₂₋₆ alkynyl", there are C₂₋₆ alkynyl (for example, 2-butyn-1-yl, 4-pentyn-1-yl, 5-hexyn-1-yl and the like) and the like optionally having 1 to 5, preferably 1 to 3 halogen atom(s) (for example, fluorine, chlorine, bromine, iodine and the like).

As the aforementioned "optionally halogenated C₃₋₆ cycloalkyl", for example, there are C₃₋₆ cycloalkyl (for example, cyclopropyl, cyclobutyl, cyclopentyl, cyclohexyl and the like) and the like optionally having 1 to 5, preferably 1 to 3 halogen atom(s) (for example, fluorine, chlorine, bromine, iodine and the like). Examples thereof are cyclopropyl, cyclobutyl, cyclopentyl, cyclohexyl, 4,4-dichlorocyclohexyl, 2,2,3,3-tetrafluorocyclopentyl, 4-chlorocyclohexyl and the like.

As the aforementioned "optionally halogenated C₁₋₈ alkoxy", for example, there are C₁₋₈ alkoxy (for example, methoxy, ethoxy, propoxy, isopropoxy, butoxy, isobutoxy, sec-butoxy, pentyloxy, hexyloxy and the like) and the like optionally having 1 to 5, preferably 1 to 3 halogen atom(s) (for example, fluorine, chlorine, bromine, iodine and the like). Examples thereof are methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy and the like.

As the aforementioned "optionally halogenated C₁₋₆ alkylthio", for example, there are C₁₋₆ alkylthio (for example, methylthio, ethylthio, propylthio, isopropylthio, butylthio, sec-butylthio, tert-butylthio and the like) and the like optionally having 1 to 5, preferably 1 to 3 halogen atom(s) (for example, fluorine, chlorine, bromine, iodine and the like). Examples thereof are methylthio, difluoromethylthio, trifluoromethylthio, ethylthio, propylthio, isopropylthio, butylthio, 4,4,4-trifluorobutylthio, pentylthio, hexylthio and the like.

As "5 to 7 membered saturated cyclic amino" of the aforementioned "5 to 7 membered saturated cyclic amino optionally having substituents", there are 5 to 7 membered saturated cyclic amino optionally containing 1 to 4 of one or two kinds of heteroatom(s)selected from a nitrogen atom, a sulfur atom and an oxygen atom in addition to one nitrogen atom and carbon atoms and examples thereof are pyrolidin-1-yl, piperidino, piperazin-1-yl, morpholino, thiomorpholino, hexahydroazepin-1-yl and the like.

As "substituents" of the "5 to 7 membered saturated cyclic amino optionally having substituents", for example, there are 1 to 3 C₁₋₆ alkyl (for example, methyl, ethyl, propyl, isopropyl, butyl, isobutyl, sec-butyl, tert-butyl, pentyl, hexyl and the like), C₆₋₁₄ aryl (for example, phenyl, 1-naphthyl, 2-naphthyl, 2-biphenylyl, 3-biphenylyl, 4-biphenylyl, 2-anthryl and the like), C₁₋₆ alkyl-carbonyl (for example, acetyl, propionyl and the like), 5 to 10 membered aromatic heterocyclic group (for example, 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-berizo[b]thienyl, 3-benzo[b]thienyl, 2-benzo[b]furanyl, 3-benzo[b]furanyl and the like), oxo and the like.

As "heterocyclic group" of "heterocyclic group optionally having substituents" represented by R⁵, for example, there is a monovalent group obtained by removing one arbitrary hydrogen atom from a 5 to 14 membered (monocyclic, bicyclic or tricyclic) heterocycle containing 1 to 4 of one or two kinds of heteroatom(s) selected from a nitrogen atom, a sulfur atom and an oxygen atom in addition to carbon atoms, preferably (i) a 5 to 14 membered (preferably 5 to 10 membered) aromatic heterocycle, (ii) a 5 to 10 membered non-aromatic heterocycle or (iii) a 7 to 10 membered bridged heterocycle.

As the aforementioned "5 to 14 membered (preferably 5 to 10 membered) aromatic heterocycle", there are an aromatic heterocycle such as thiophene, benzo[b]thiophene, benzo[b]furan, benzimidazole, benzoxazole, benzothiazole, benzisothiazole, naphtho[2,3-b]thiophene, furan, pyrrole, imidazole, pyrazole, pyridine, pyrazine, pyrimidine, pyridazine, indole, isoindole, 1H-indazole, purine, 4H-quinolizine, isoquinoline, quinoline, phthalazine, naphthyridine, quinoxaline, quinazoline, cinnoline, carbazole, β-carboline, phenanthridine, acridine, phenazine, thiazole, isothiazole, phenothiazine, isoxazole, furazan, phenoxazine and the like, and a ring formed by fusing these rings (preferably monocyclic) with one or more (preferably 1 to 2) aromatic ring(s) (for example, benzene ring and the like).

As the aforementioned "5 to 10 membered non-aromatic heterocycle", for example, there are pyrrolidine, imidazoline, pyrazolidine, pyrazoline, piperidine, piperazine, morpholine, thiomorpholine, dioxazole, oxadiazoline, thiadiazoline, triazoline, thiadiazole, dithiazole and the like.

As the aforementioned "7 to 10 membered bridged heterocycle", for example, there are quinuclidine, 7-azabicyclo[2.2.1]heptane and the like.

The "heterocyclic group" is preferably a 5 to 14 membered (preferably 5 to 10 membered) (monocyclic or bicyclic) heterocyclic group containing preferably 1 to 4 of one or two kinds of heteroatom(s) selected from a nitrogen atom, a sulfur atom and an oxygen atom in addition to carbon atoms. More particularly, examples thereof are an aromatic heterocyclic group such as 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 2-pyrimidinyl, 4-pyrimidinyl, 3-pyrrolyl, 2-imidazolyl, 3-pyridazinyl, 3-isothiazolyl, 3-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like, and a non-aromatic heterocyclic group such as 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino and the like.

Among them, for example, a 5 or 6 membered heterocyclic group containing 1 to 3 heteroatom(s) selected from a nitrogen atom, a sulfur atom and an oxygen atom in addition to carbon atoms is further preferable. More particularly, examples thereof are 2-thienyl, 3-thienyl, 2-pyridyl, 3-pyridyl, 4-pyridyl, 2-furyl, 3-furyl, pyrazinyl, 2-pyrimidinyl, 3-pyrrolyl, 3-pyridazinyl, 3-isothiazolyl, 3-isoxazolyl, 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino and the like.

As "substituents" of "heterocyclic group optionally having substituents", for example, there are the same "substituents" as substituents of "hydrocarbon group optionally having substituents" represented by R⁵.

The "heterocyclic group" may have 1 to 5, preferably 1 to 3 aforementioned substituents at a substitutable position and, when the number of substituents is 2 or more, respective substituents may be the same or different.

As "C₁₋₆ alkyl" represented by R⁶, for example, there are methyl, ethyl, propyl, isopropyl, butyl, isobutyl, sec-butyl, tert-butyl, pentyl, hexyl and the like.

As "hydrocarbon group optionally having substituents" and "heterocyclic group optionally having substituents" represented by R⁷, for example, there are the aforementioned "hydrocarbon group optionally having substituents" and "heterocyclic group optionally having substituents" represented by R⁵, respectively.

As "hydrocarbon group optionally having substituents" represented by R¹, for example, "hydrocarbon group optionally having substituents" represented by R⁵ can be mentioned.

As "heterocyclic group optionally having substituents" represented by R¹, for example, "heterocyclic group optionally having substituents" represented by R⁵ can be mentioned.

As "amino group optionally having substituents" represented by R¹, for example, there are (1) an amino group optionally having 1 or 2 substituents and (2) a cyclic amino group optionally having substituents, and the like.

As "substituents" of "amino group optionally having 1 or 2 substituents" of the aforementioned (1), for example, there are a hydrocarbon group optionally having substituents, a heterocyclic group optionally having substituents, an acyl group, an alkylidene group optionally having substituents, and the like. As these "hydrocarbon group optionally having substituents" and "heterocyclic group optionally having substituents", there are the same "hydrocarbon group optionally having substituents" and "heterocyclic group optionally having substituents" as those represented by R⁵ described above, respectively.

As "alkylidene group" of "alkylidene group optionally having substituents", for example, there are a C₁₋₆ alkylidene group (for example, methylidene, ethylidene, propylidene and the like) and the like. As "substituents" of "alkylidene group optionally having substituents", there are 1 to 5, preferably 1 to 3 same substituents as "substituents" of "hydrocarbon group optionally having substituents" represented by R⁵.

When the number of the aforementioned "substituents" of "amino group optionally having 1 or 2 substituents" is 2, respective substituents may be the same or different.

As "cyclic amino group" of "cyclic amino group optionally having substituents" of the aforementioned (2), there are a 5 to 7 membered non-aromatic cyclic amino group optionally containing 1 to 4 of one or two kinds of heteroatom(s) selected from a nitrogen atom, a sulfur atom and an oxygen atom in addition to one nitrogen atom and carbon atoms. More particularly, examples thereof are pyrrolidin-1-yl, piperidino, piperazin-1-yl, morpholino, thiomorpholino, hexahydroazepin-1-yl, imidazolidin-1-yl, 2,3-dihydro-1H-imidazol-1-yl, tetrahydro-1(2H)-pyrimidinyl, 3,6-dihydro-1(2H)-pyrimidinyl, 3,4-dihydro-1(2H)-pyrimidinyl and the like. As "substituents" of "cyclic amino optionally having substituents", there are 1 to 3 of the same ones as "substituents" of "5 to 7 membered saturated cyclic amino group optionally having substituents" which were described in detail as "substituents" of "hydrocarbon group optionally having substituents" represented by R⁵.

Examples of the 5 to 7 membered non-aromatic cyclic amino group having one oxo, there are 2-oxoimidazolidin-1-yl, 2-oxo-2,3-dihydro-1H-imidazol-1-yl, 2-oxotetrahydro-1(2H)-pyrimidinyl, 2-oxo-3,6-dihydro-1(2H)-pyrimidinyl, 2-oxo-3,4-dihydro-1(2H)-pyrimidinyl, 2-oxopyrrolidin-1-yl, 2-oxopiperidino, 2-oxopiperazin-1-yl, 3-oxopiperazin-1-yl, 2-oxo-2,3,4,5,6,7-hexahydroazepin-1-yl and the like.

As R¹, an amino group optionally having substituents and an aryl group optionally having substituents are preferable. As further preferable example of the "amino group optionally having substituents" is an amino group optionally having 1 or 2 acyl groups represented by the formula:
-(C=O)-R⁵, -(C=O)-OR⁵, -(C=O)-NR⁵R⁶, -(C=S)-NHR⁵ or -SO₂-R⁷ [wherein respective symbols represent the same meanings as described above].

More preferable example is an amino group optionally having 1 or 2 acyl groups represented by the formula:
-C(C=O)-R⁵ or -(C=O)-NR⁵R⁶ [wherein respective symbols represent the same meanings as described above].

As the "aryl group optionally having substituents", for example, there is preferably a C₆₋₁₄ aryl group (preferably a phenyl group and the like) optionally having 1 to 5 substituents selected from C₁₋₆ alkylthio, C₆₋₁₄ arylthio, C₁₋₆ alkylsulfinyl, C₆₋₁₄ arylsulfinyl, C₁₋₆ alkylsulfonyl, C₆₋₁₄ arylsulfonyl and carboxy.

Particularly, as R¹, there are mentioned
(1) C₆₋₁₄ aryl group (preferably C₆₋₁₀ aryl) optionally having 1 to 5 substituents selected from halogen atom, optionally halogenated C₁₋₆ alkyl, carboxy C₂₋₆ alkenyl, optionally halogenated C₁₋₆ alkoxy, C₁₋₆ alkoxy-carbonyl-C₁₋₆ alkoxy, hydroxy, amino, mono- or di-C₁₋₆ alkylamino, carboxy, C₁₋₆ alkoxy-carbonyl, mono- or di-C₁₋₆ alkyl-carbamoyl, C₆₋₁₄ aryl-carbonylamino, C₁₋₃ alkylenedioxy, C₁₋₆ alkylthio, C₆₋₁₄ arylthio, C₁₋₆ alkylsulfinyl, C₆₋₁₄ arylsulfinyl, C₁₋₆ alkylsulfonyl, C₆₋₁₄ arylsulfonyl, nitro and the like,
(2) C₁₋₈ alkyl group optionally having 1 to 5 substituents selected from halogen atom, optionally halogenated C₁₋₆ alkyl, carboxy C₂₋₆ alkenyl, optionally halogenated C₁₋₆ alkoxy, C₁₋₆ alkoxy-carbonyl-C₁₋₆ alkoxy, hydroxy, amino, mono- or di-C₁₋₆ alkylamino, carboxy, C₁₋₆ alkoxy-carbonyl, mono- or di-C₁₋₆ alkyl-carbamoyl and C₆₋₁₄ aryl-carbonylamino,
(3) C₃₋₆ cycloalkyl group (e.g., cyclohexyl) optionally having 1 to 5 substituents selected from halogen atom, optionally halogenated C₁₋₆ alkyl, carboxy C₂₋₆ alkenyl, optionally halogenated C₁₋₆ alkoxy, C₁₋₆ alkoxy-carbonyl-C₁₋₆ alkoxy, hydroxy, amino, mono- or di-C₁₋₆ alkylamino, carboxy, C₁₋₆ alkoxy-carbonyl, mono- or di-C₁₋₆ alkyl-carbamoyl and C₆₋₁₄ aryl-carbonylamino,
(4) C₇₋₁₆ aralkyl group (e.g., phenyl-C₁₋₆ alkyl group),
(5) 5 to 10 membered aromatic heterocyclic group containing 1 to 4 of one or two kinds of heteroatom(s) selected from a nitrogen atom, a sulfur atom and an oxygen atom in addition to carbon atoms (e.g., 5 or 6 membered aromatic heterocyclic group such as pyridyl, thienyl and the like),
(6) 5 to 10 membered non-aromatic heterocyclic group containing 1 or 2 of one or two kinds of heteroatom(s) selected from a nitrogen atom, a sulfur atom and an oxygen atom in addition to carbon atoms, which may have C₆₋₁₄ aryl (e.g., phenyl), C₁₋₆ alkyl-carbonyl or oxo, such as 5 or 6 membered non-aromatic cyclic amino group (e.g., piperidino, piperazino and the like),
(7) amino group optionally having 1 or 2 substituents selected from the group consisting of the following 1) to 7) [1) C₁₋₆ alkyl, 2) C₆₋₁₄ aryl, 3) C₇₋₁₆ aralkyl, 4) a 5 or 6 membered heterocyclic group (e.g., pyridyl) containing 1 or 2 heteroatom(s) selected from a nitrogen atom, a sulfur atom and an oxygen atom in addition to carbon atoms, 5) C₁₋₆ alkyl-carbonyl, C₃₋₆ cycloalkyl-carbonyl, C₆₋₁₄ aryl-carbonyl, C₇₋₁₆ aralkyl-carbonyl, C₁₋₆ alkyl-carbamoyl or 5 or 6 membered heterocyclic carbonyl group, each optionally having 1 to 3 substituents selected from halogen atom, C₁₋₆ alkyl, C₁₋₆ alkoxy, carboxy, C₁₋₆ alkoxy-carbonyl, cyano, tetrazine and the like, 6) C₆₋₁₄ aryl-carbamoyl group optionally having 1 to 3 substituents selected from halogen atom, C₁₋₆ alkyl, C₁₋₆ alkoxy, carboxy, C₁₋₆ alkoxy-carbonyl, cyano, nitro, mono- or di-C₁₋₆ alkylamino and the like, 7) di-C₁₋₆ alkylamino-C₁₋₆ alkylidene], or (8) carboxy group and the like are preferable.

As the "pyridyl group" of the "pyridyl group optionally having substituents" represented by R², 1-, 2-, 3- or 4-pyridyl group is used.

As the "substituents" of the "pyridyl group optionally having substituents" represented by R², for example, those similar to the "substituents" of the "hydrocarbon group optionally having substituents" represented by the aforementioned R⁵ are used.

The "pyridyl group" may have 1 to 5, preferably 1 to 3, substituents such as those mentioned above at substitutable position(s). When the number of substituent is 2 or more, the respective substituents may be the same or different. In addition, the nitrogen atom in the ring of the "pyridyl group" may be N-oxidized.

R² is preferably a pyridyl group optionally having substituents (e.g., 3-pyridyl group, 4-pyridyl group and the like, preferably 4-pyridyl group).

As R², pyridyl group optionally having 1 or 2 substituents selected from the group consisting of C₁₋₆ alkyl (e.g., methyl), hydroxy and C₁₋₆ alkyl-carbonyloxy (e.g., acetyloxy) and the like are preferable.

As the "aromatic group" of "aromatic group optionally having substituents" represented by R³, for example, there are an aromatic hydrocarbon group and an aromatic heterocyclic group.

As the "aromatic hydrocarbon group", examples thereof include a C₆₋₁₄ monocyclic or fused polycyclic (bicyclic or tricyclic) aromatic hydrocarbon group. As examples, there are a C₆₋₁₄ aryl group and the like such as phenyl, 1-naphthyl, 2-naphthyl, 2-biphenylyl, 3-biphenylyl, 4-biphenylyl, 2-anthryl and the like.

As the "aromatic heterocyclic group", there are 5 to 14 membered (preferably 5 to 10 membered)(monocyclic or bicyclic) aromatic heterocyclic groups containing preferably 1 to 4 of one or two kinds of heteroatom(s) selected from a nitrogen atom, a sulfur atom and an oxygen atom in addition to carbon atoms and the like and, more particularly, an aromatic heterocyclic group such as 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 2-pyrimidinyl, 4-pyrimidinyl, 3-pyrrolyl, 2-imidazolyl, 3-pyridazinyl, 3-isothiazolyl, 3-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like.

As the "substituents" of the "aromatic group optionally having substituents", there are 1 to 5, preferably 1 to 3 same substituents as "substituents" of "hydrocarbon group optionally having substituents" represented by the aforementioned R⁵. When the number of substituents is 2 or more, respective substituents may be the same or different. The adjacent two substituents may form a 4 to 7 membered non-aromatic carbon ring. Preferably, it is a 5 membered non-aromatic carbon ring.

R³ is preferably a C₆₋₁₀ aryl group optionally having substituents. More preferably, it is a phenyl group optionally having substituents. The substituent of the C₆₋₁₀ aryl group and phenyl group is preferably 1 to 3 substituents selected from halogen atom, C₁₋₃ alkylenedioxy, optionally halogenated C₁₋₆ alkyl, carboxy C₂₋₆ alkenyl, C₃₋₆ cycloalkyl, optionally halogenated C₁₋₈ alkoxy, hydroxy, C₇₋₁₆ aralkyloxy, C₁₋₆ alkyl-carbonyloxy and carboxy, particularly preferably, is optionally halogenated C₁₋₆ alkyl (e.g., C₁₋₃ alkyl such as methyl, ethyl and the like), optionally halogenated C₁₋₈ alkoxy (e.g., C₁₋₃ alkoxy such as methoxy, ethoxy and the like). The two adjacent alkyl groups as substituents may be bonded to form a 5 membered non-aromatic carbon ring.

The compound (I) preferably does not include a compound of the formula 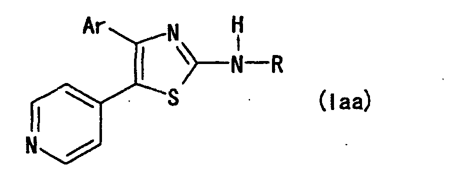 wherein Ar is an unsubstituted or substituted aryl group bonded to a thiazole ring by a carbon atom of the aromatic ring, and R is a hydrogen atom, acyl group, or a monovalent aromatic group having not more than 10 carbon atoms, which is bonded to a nitrogen atom by a carbon atom of the aromatic ring.

As the compound (I), for example, compound (Ia) is preferable.

As compound (Ia), the following compounds of (A)-(B) and the like are preferable.
(A) A compound (Ia) wherein R¹ is (a) an amino group which may have 1 or 2 acyl groups of the formula: -(C=O)-R⁵ or -(C=O)-NR⁵R⁶ wherein each symbol is as defined above or (b) a C₆₋₁₄ aryl group optionally having 1 to 5 substituents selected from C₁₋₆ alkylthio, C₆₋₁₄ arylthio, C₁₋₆ alkylsulfinyl, C₆₋₁₄ arylsulfinyl, C₁₋₆ alkylsulfonyl, C₆₋₁₄ arylsulfonyl and carboxy and the like;
   R² is pyridyl group optionally having 1 to 5, substituents selected from C₁₋₆ alkyl, hydroxy and C₁₋₆ alkyl-carbonyloxy; and
   R³ is a C₆₋₁₄ aryl group optionally having 1 to 5 substituents selected from halogen atom, optionally halogenated C₁₋₆ alkyl, optionally halogenated C₁₋₆ alkoxy and carboxy.
(B) A compound (Ia) wherein R¹ is (i) C₁₋₈ alkyl, C₃₋₆ cycloalkyl or C₆₋₁₄ aryl (preferably C₆₋₁₀ aryl), each optionally having 1 to 5 substituents selected from halogen atom, optionally halogenated C₁₋₆ alkyl, carboxy C₂₋₆ alkenyl, optionally halogenated C₁₋₆ alkoxy, C₁₋₆ alkoxy-carbonyl-C₁₋₆ alkoxy, hydroxy, amino, mono- or di-C₁₋₆ alkylamino, carboxy, C₁₋₆ alkoxy-carbonyl, mono- or di-C₁₋₆ alkyl-carbamoyl and C₆₋₁₄ aryl-carbonylamino,
   (ii) a 5 membered heterocyclic group,
   (iii) an amino group optionally having 1 or 2 substituents selected from (1) C₁₋₆ alkyl, (2) C₆₋₁₄ aryl, (3) C₇₋₁₆ aralkyl, (4) 6 membered heterocyclic group and (5) C₁₋₆ alkyl-carbonyl, C₃₋₆ cycloalkyl-carbonyl, C₆₋₁₄ aryl-carbonyl, C₇₋₁₆ aralkyl-carbonyl, C₁₋₆ alkyl-carbamoyl or 5 or 6 membered heterocyclic carbonyl, each optionally having 1 to 3 substituents selected from halogen atom, C₁₋₆ alkyl, C₁₋₆ alkoxy, carboxy and C₁₋₆ alkoxy-carbonyl, or an amino group optionally having di-C₁₋₆ alkylamino-C₁₋₆ alkylidene,
   (iv) a 5 or 6 membered non-aromatic cyclic amino group optionally substituted by C₁₋₆ alkyl-carbonyl or oxo, or
   (v) a carboxy group;
   R² is a pyridyl group optionally having 1 to 3 substituents selected from C₁₋₆ alkyl, hydroxy and C₁₋₆ alkyl-carbonyloxy;
   R³ is a C₆₋₁₀ aryl group optionally having 1 to 3 substituents selected from halogen atom, C₁₋₃ alkylenedioxy, optionally halogenated C₁₋₆ alkyl, carboxy C₂₋₆ alkenyl, optionally halogenated C₁₋₈ alkoxy, hydroxy, C₇₋₁₆ aralkyloxy and C₁₋₆ alkyl-carbonyloxy (two adjacent alkyl groups as substituents may be bonded to form a 5 membered non-aromatic carbon ring).

Moreover, preferable examples of compound (I) and compound (Ia) include:
[4-(4-methoxyphenyl)-5-(4-pyridyl)-1,3-thiazol-2-yl]amine (Reference Example A 13-14),
[4-phenyl-5-(4-pyridyl)-1,3-thiazol-2-yl]amine (Reference Example A 13-15),
N-methyl [4-(4-methoxyphenyl)-5-(4-pyridyl)-1,3-thiazol-2-yl]amine (Reference Example A 13-16),
N-methyl [4-phenyl-5-(4-pyridyl)-1,3-thiazol-2-yl]amine (Reference Example A 13-47),
N-methyl [4-(4-fluorophenyl)-5-(4-pyridyl)-1,3-thiazol-2-yl]amine (Reference Example A 13-69),
N-methyl [4-(4-chlorophenyl)-5-(4-pyridyl)-1,3-thiazol-2-yl]amine (Reference Example A 13-70),
N-methyl [4-(4-bromophenyl)-5-(4-pyridyl)-1,3-thiazol-2-yl]amine (Reference Example A 13-71),
2-phenyl-N-[4-phenyl-5-(4-pyridyl)-1,3-thiazol-2-yl]acetamide (Reference Example A 23-29),
3-phenyl-N-[4-phenyl-5-(4-pyridyl)-1,3-thiazol-2-yl]propionamide (Reference Example A 23-30),
N-[4-(3-chlorophenyl)-5-(4-pyridyl)-1,3-thiazol-2-yl]acetamide (Reference Example A 23-49),
N-[4-(3-chlorophenyl)-5-(4-pyridyl)-1,3-thiazol-2-yl]propionamide (Reference Example A 23-50),
N-[4-(3-methylphenyl)-5-(4-pyridyl)-1,3-thiazol-2-yl]acetamide (Reference Example A 23-51),
N-[4-(3-methylphenyl)-5-(4-pyridyl)-1,3-thiazol-2-yl]propionamide (Reference Example A 23-52),
[4-(3-chlorophenyl)-5-(4-pyridyl)-1,3-thiazol-2-yl]amine (Reference Example A 23-59),
[4-(3-methylphenyl)-5-(4-pyridyl)-1,3-thiazol-2-yl]amine (Reference Example A 23-60),
[4-(4-chlorophenyl)-5-(4-pyridyl)-1,3-thiazol-2-yl]amine (Reference Example A 23-61),
[4-(4-methylphenyl)-5-(4-pyridyl)-1,3-thiazol-2-yl]amine (Reference Example A 23-62),
N-[4-phenyl-5-(4-pyridyl)-1,3-thiazol-2-yl]acetamide (Reference Example A 23-71),
N-phenyl-[4-(4-methoxyphenyl)-5-(4-pyridyl)-1,3-thiazol-2-yl]amine (Reference Example A 23-80),
N-[4-(4-methoxyphenyl)-5-(4-pyridyl)-1,3-thiazol-2-yl]nicotinamide (Reference Example A 23-101),
N-[4-(4-methoxyphenyl)-5-(4-pyridyl)-1,3-thiazol-2-yl]isonicotinamide (Reference Example A 23-102),
[4-(3,4-dimethylphenyl)-5-(4-pyridyl)-1,3-thiazol-2-yl]amine (Reference Example A 23-125),
N-[4-(3,5-dimethylphenyl)-5-(4-pyridyl)-1,3-thiazol-2-yl]acetamide (Reference Example A 23-128),
[4-(2-naphthyl)-5-(4-pyridyl)-1,3-thiazol-2-yl]amine (Reference Example A 23-144),
N-ethyl-N'-[4-(4-methoxyphenyl)-5-(4-pyridyl)-1,3-thiazol-2-yl]urea (Reference Example A 23-156),
N-[4-(3,5-dimethylphenyl)-5-(4-pyridyl)-1,3-thiazol-2-yl]isonicotinamide (Reference Example A 23-200),
N-ethyl-[4-(4-methoxyphenyl)-5-(4-pyridyl)-1,3-thiazol-2-yl]amine (Reference Example A 23-269),
N-propyl-[4-(3,5-dimethylphenyl)-5-(4-pyridyl)-1,3-thiazol-2-yl]amine (Reference Example A 23-276),
N-butyl-[4-(3,5-dimethylphenyl)-5-(4-pyridyl)-1,3-thiazol-2-yl]amine (Reference Example A 23-280),
N-benzyl-[4-(3,5-dimethylphenyl)-5-(4-pyridyl)-1,3-thiazol-2-yl]amine (Reference Example A 23-281),
N-propyl-[4-(4-methoxyphenyl)-5-(4-pyridyl)-1,3-thiazol-2-yl]amine (Reference Example A 23-290),
N-isopropyl-[4-(4-methoxyphenyl)-5-(4-pyridyl)-1,3-thiazol-2-yl]amine (Reference Example A 23-291),
N-[4-(4-methoxyphenyl)-5-(4-pyridyl)-1,3-thiazol-2-yl]-N'-phenylurea (Reference Example A 23-296),
4-[[[4-(4-methoxyphenyl)-5-(4-pyridyl)-1,3-thiazol-2-yl]amino]carbonyl]benzoic acid (Reference Example A 23-299),
methyl 4-[2-[4-(methylthio)phenyl]-5-(4-pyridyl)-1,3-thiazol-4-yl]phenyl ether (Reference Example A 23-300),
4-[4-(3,5-dimethylphenyl)-5-(4-pyridyl)-1,3-thiazol-2-yl]phenyl methyl sulfide (Reference Example A 23-302),
4-[4-(4-methoxyphenyl)-5-(4-pyridyl)-1,3-thiazol-2-yl]phenyl methyl sulfoxide (Reference Example A 23-303),
4-[4-(3,5-dimethylphenyl)-5-(4-pyridyl)-1,3-thiazol-2-yl]phenyl methyl sulfoxide (Reference Example A 23-305),
4-[4-(4-methoxyphenyl)-5-(4-pyridyl)-1,3-thiazol-2-yl]phenyl methyl sulfone (Reference Example A 23-306),
4-[4-(3,5-dimethylphenyl)-5-(4-pyridyl)-1,3-thiazol-2-yl]phenyl methyl sulfone (Reference Example A 23-308),
4-[4-(4-fluorophenyl)-5-(4-pyridyl)-1,3-thiazol-2-yl]phenyl methyl sulfide (Reference Example A 23-309),
4-[4-(4-chlorophenyl)-5-(4-pyridyl)-1,3-thiazol-2-yl]phenyl methyl sulfide (Reference Example A 23-310),
4-[4-(4-fluorophenyl)-5-(4-pyridyl)-1,3-thiazol-2-yl]phenyl methyl sulfoxide (Reference Example A 23-311),
4-[4-(4-chlorophenyl)-5-(4-pyridyl)-1,3-thiazol-2-yl]phenyl methyl sulfoxide (Reference Example A 23-312),
4-[4-(4-fluorophenyl)-5-(4-pyridyl)-1,3-thiazol-2-yl]phenyl methyl sulfone (Reference Example A 23-313),
4-[4-(4-chlorophenyl)-5-(4-pyridyl)-1,3-thiazol-2-yl]phenyl methyl sulfone (Reference Example A 23-314),
N-[4-(3,5-dimethylphenyl)-5-(4-pyridyl)-1,3-thiazol-2-yl]-N'-phenylurea (Reference Example A 23-315),
2-hydroxy-N-[4-(4-methoxyphenyl)-5-(4-pyridyl)-1,3-thiazol-2-yl]propionamide (Reference Example A 23-325),
4-[4-(3,4-dimethylphenyl)-5-(4-pyridyl)-1,3-thiazol-2-yl]phenyl methyl sulfide (Reference Example A 23-326),
4-[4-(3,4-dimethylphenyl)-5-(4-pyridyl)-1,3-thiazol-2-yl]phenyl methyl sulfoxide (Reference Example A 23-327),
4-[4-(3,4-dimethylphenyl)-5-(4-pyridyl)-1,3-thiazol-2-yl]phenyl methyl sulfone (Reference Example A 23-328),
2-hydroxy-N-[4-(4-methoxyphenyl)-5-(4-pyridyl)-1,3-thiazol-2-yl]acetamide (Reference Example A 23-329),
4-[[[4-(3,5-dimethylphenyl)-5-(4-pyridyl)-1,3-thiazol-2-yl]amino]carbonyl]benzoic acid (Reference Example A 23-337),
3-[[[4-(3,5-dimethylphenyl)-5-(4-pyridyl)-1,3-thiazol-2-yl]amino]carbonyl]benzoic acid (Reference Example A 23-342),
salts thereof and the like.

Preferable examples of compound (I) and compound (Ia) further include 4-(4-fluorophenyl)-2-phenyl-5-(4-pyridyl)-1,3-thiazole (Reference Example A 44-1), methyl 4-[4-(3-methylphenyl)-5-(4-pyridyl)-1,3-thiazol-2-yl]phenyl sulfide (Reference Example A 44-7), methyl 4-[4-(3-methylphenyl)-5-(4-pyridyl)-1,3-thiazol-2-yl]phenyl sulfoxide (Reference Example A 44-8), methyl 4-[4-(3-methylphenyl)-5-(4-pyridyl)-1,3-thiazol-2-yl]phenyl sulfone (Reference Example A 44-26) and the like.

Furthermore, as compound (I) and (Ia),
(S)-N-[4-(3-methylphenyl)-5-(2-(1-phenylethylamino)-4-pyridyl)-1,3-thiazol-2-yl]nicotinamide,
(R)-N-[4-(3-methylphenyl)-5-(2-(1-phenylethylamino)-4-pyridyl)-1,3-thiazol-2-yl]nicotinamide,
(S)-N-[4-(3-methylphenyl)-5-(2-(1-phenylethylamino)-4-pyridyl)-1,3-thiazol-2-yl]-2-methylnicotinamide,
(R)-N-[4-(3-methylphenyl)-5-(2-(1-phenylethylamino)-4-pyridyl)-1,3-thiazol-2-yl]-2-methylnicotinamide,
(S)-N-[4-(3-methylphenyl)-5-(2-(1-phenylethylamino)-4-pyridyl)-1,3-thiazol-2-yl]-2-chloronicotinamide,
(R)-N-[4-(3-methylphenyl)-5-(2-(1-phenylethylamino)-4-pyridyl)-1,3-thiazol-2-yl]-2-chloronicotinamide,
(S)-N-[4-(3-methylphenyl)-5-(2-(1-phenylethylamino)-4-pyridyl)-1,3-thiazol-2-yl]-2-methoxynicotinamide,
(R)-N-[4-(3-methylphenyl)-5-(2-(1-phenylethylamino)-4-pyridyl)-1,3-thiazol-2-yl]-2-methoxynicotinamide,
N-[5-(2-benzylamino-4-pyridyl)-4-(3-methylphenyl)-1,3-thiazol-2-yl]nicotinamide,
N-[5-(2-benzylamino-4-pyridyl)-4-(3-methylphenyl)-1,3-thiazol-2-yl]-2-methoxynicotinamide,
N-[5-(2-benzylamino-4-pyridyl)-4-(3-methylphenyl)-1,3-thiazol-2-yl]-2-chloronicotinamide,
N-[5-(2-benzylamino-4-pyridyl)-4-(3-methylphenyl)-1,3-thiazol-2-yl]-2-methylnicotinamide,
N-[5-(2-benzoylamino-4-pyridyl)-4-(3-methylphenyl)-1,3-thiazol-2-yl]nicotinamide,
N-[5-(2-benzoylamino-4-pyridyl)-4-(3-methylphenyl)-1,3-thiazol-2-yl]-2-methylnicotinamide,
N-[5-(2-benzoylamino-4-pyridyl)-4-(3-methylphenyl)-1,3-thiazol-2-yl]-2-chloronicotinamide,
N-[5-(2-benzoylamino-4-pyridyl)-4-(3-methylphenyl)-1,3-thiazol-2-yl]-2-methoxynicotinamide,
(S)-N-(1-phenylethyl)-4-[2-ethyl-4-(3-methylphenyl)-1,3-thiazol-5-yl]-2-pyridylamine,
(R)-N-(1-phenylethyl)-4-[2-ethyl-4-(3-methylphenyl)-1,3-thiazol-5-yl]-2-pyridylamine,
(S)-N-(1-phenylethyl)-4-[4-(3-methylphenyl)-2-propyl-1,3-thiazol-5-yl]-2-pyridylamine,
(R)-N-(1-phenylethyl)-4-[4-(3-methylphenyl)-2-propyl-1,3-thiazol-5-yl]-2-pyridylamine,
(S)-N-(1-phenylethyl)-4-[2-butyl-4-(3-methylphenyl)-1,3-thiazol-5-yl]-2-pyridylamine,
(R)-N-(1-phenylethyl)-4-[2-butyl-4-(3-methylphenyl)-1,3-thiazol-5-yl]-2-pyridylamine,
(S)-N-(1-phenylethyl)-4-[4-(3-methylphenyl)-2-(4-methylthiophenyl)-1,3-thiazol-5-yl]-2-pyridylamine,
(R)-N-(1-phenylethyl)-4-[4-(3-methylphenyl)-2-(4-methylthiophenyl)-1,3-thiazol-5-yl]-2-pyridylamine,
(S)-N-(1-phenylethyl)-4-[4-(3-methylphenyl)-2-(4-methylsulfonylphenyl)-1,3-thiazol-5-yl]-2-pyridylamine,
(R)-N-(1-phenylethyl)-4-[4-(3-methylphenyl)-2-(4-methylsulfonylphenyl)-1,3-thiazol-5-yl]-2-pyridylamine,
(S)-N-(1-phenylethyl)-4-[2-(4-fluorophenyl)-4-(3-methylphenyl)-1,3-thiazol-5-yl]-2-pyridylamine,
(R)-N-(1-phenylethyl)-4-[2-(4-fluorophenyl)-4-(3-methylphenyl)-1,3-thiazol-5-yl]-2-pyridylamine, salts thereof and the like are preferable.

As the salt of Compounds (I) and (Ia), for example, there are a metal salt, ammonium salt, a salt with an organic base, a salt with an inorganic acid, a salt with an organic acid, a salt with basic or acidic amino acid and the like. As a suitable metal salt, there are alkali metal salt such as sodium salt, potassium salt and the like; alkaline earth metal salt such as calcium salt, magnesium salt, barium salt and the like; aluminum salt and the like. As a suitable example of a salt with an organic base, for example, there are salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like. As a suitable example of a salt with an inorganic acid, for example, there are salts with hydrochloric acid, hydrobromic acid, nitric acid, sulfuric acid, phosphoric acid and the like. As a suitable example of a salt with an organic acid, for example, there are salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like. As a suitable example of a salt with a basic amino acid, for example, there are salts with arginine, lysine, ornithine and the like. As a suitable example of a salt with an acidic amino acid, for example, there are salts with aspartic acid, glutamic acid and the like.

Among them, pharmaceutically acceptable salts are preferable. For example, when a compound has an acidic functional group therein, there are inorganic salts such as alkali metal salts (for example, sodium salt, potassium salt and the like), alkaline earth metal salts (for example, calcium salt, magnesium salt, barium salt and the like), ammonium salts and the like and, when a compound has a basic functional group therein, there are salts with inorganic acids such as hydrochloric acid, hydrobromic acid, nitric acid, sulfuric acid, phosphoric acid and the like, and salts with organic acids such as acetic acid, phthalic acid, fumaric acid, oxalic acid, tartaric acid, maleic acid, citric acid, succinic acid, methanesulfonic acid, p-toluenesulfonic acid and the like.

A process for producing Compound (I) including Compound (Ia) will be described below. Compound (I) can be obtained according to the methods described in WO01/10865, JP-A-60-58981, JP-A-61-10580, JP-T 7-503023, WO 93/15071, DE-A-3601411, JP-A-5-70446 and the like, a method similar to these methods and the like.

When Compound (I) is present as a configurational isomer, diastereomer, conformer or the like, each can be optionally isolated by the above separation and purification means. In addition, Compound (I) is in the form of its racemate, they can be separated into S- and R-forms by any conventional optical resolution.

When Compound (I) includes stereoisomers, both the isomers alone and mixtures of each isomers are included in the scope of the present invention.

In addition, Compound (I) may be hydrated or anhydrous.

Compound (I) may be labeled with an isotope (for example, ³H, ¹⁴C, ³⁵S) or the like.

### [compound (II)]

(1) an optionally N-oxidized compound represented by the formula: 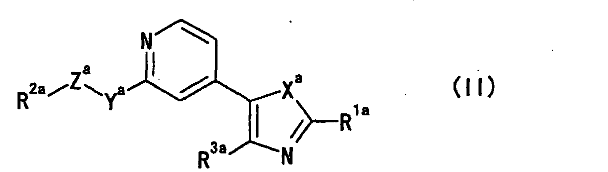 wherein R^{1a} represents a hydrogen atom, a hydrocarbon group optionally having substituents, a heterocyclic group optionally having substituents, an amino group optionally having substituents or an acyl group,
   R^{2a} represents an aromatic group optionally having substituents,
   R^{3a} represents a hydrogen atom, a pyridyl group optionally having substituents or an aromatic hydrocarbon group optionally having substituents,
   X^{a} represents an oxygen atom or an optionally oxidized sulfur atom,
   Y^{a} represents a bond, an oxygen atom, an optionally oxidized sulfur atom or a group represented by the formula: NR^{4a} (wherein R^{4a} represents a hydrogen atom, a hydrocarbon group optionally having substituents or an acyl group) and
   Z^{a} represents a bond or a divalent acyclic hydrocarbon group optionally having substituents, or a salt thereof,
(2) the compound according to (1), wherein Z^{a} is a divalent acyclic hydrocarbon group optionally having substituents,
(3) the compound according to (1), which is a compound represented by the formula: 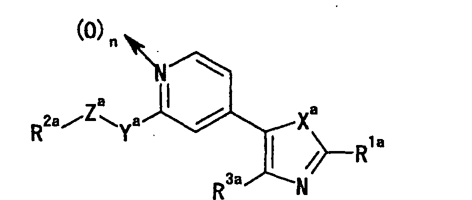 wherein n represents 0 or 1, and other symbols are as defined in (1), or a salt thereof,
(4) the compound according to (1) or (3), wherein R^{1a} represents
   (i) a hydrogen atom,
   (ii) a C₁₋₆ alkyl group, a C₂₋₆ alkenyl group, a C₂₋₆ alkynyl group, a C₃₋₆ cycloalkyl group, a C₆₋₁₄ aryl group or a C₇₋₁₆ aralkyl group [these groups may have substituents selected from the group (substituent group A) consisting of oxo, halogen atom, C₁₋₃ alkylenedioxy, nitro, cyano, optionally halogenated C₁₋₆ alkyl, optionally halogenated C₂₋₆ alkenyl, carboxy C₂₋₆ alkenyl, optionally halogenated C₂₋₆ alkynyl, optionally halogenated C₃₋₆ cycloalkyl, C₆₋₁₄ aryl, optionally halogenated C₁₋₈ alkoxy, C₁₋₆ alkoxy-carbonyl-C₁₋₆ alkoxy, hydroxy, C₆₋₁₄ aryloxy, C₇₋₁₆ aralkyloxy, mercapto, optionally halogenated C₁₋₆ alkylthio, C₆₋₁₄ arylthio,
      C₇₋₁₆ aralkylthio, amino, mono-C₁₋₆ alkylamino, mono-C₆₋₁₄ arylamino, di-C₁₋₆ alkylamino, di-C₆₋₁₄ arylamino, formyl, carboxy, C₁₋₆ alkyl-carbonyl, C₃₋₆ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5 or 6 membered heterocyclic carbonyl, carbamoyl, thiocarbamoyl, mono-C₁₋₆ alkyl-carbamoyl, di-C₁₋₆ alkyl-carbamoyl, C₆₋₁₄ aryl-carbamoyl, 5 or 6 membered heterocyclic carbamoyl, C₁₋₆ alkylsulfonyl, C₆₋₁₄ arylsulfonyl, C₁₋₆ alkylsulfinyl, C₆₋₁₄ arylsulfinyl, formylamino, C₁₋₆ alkyl-carbonylamino, C₆₋₁₄ aryl-carbonylamino, C₁₋₆ alkoxy-carbonylamino, C₁₋₆ alkylsulfonylamino, C₆₋₁₄ arylsulfonylamino, C₁₋₆ alkyl-carbonyloxy, C₆₋₁₄ aryl-carbonyloxy, C₁₋₆ alkoxy-carbonyloxy, mono-C₁₋₆ alkyl-carbamoyloxy, di-C₁₋₆ alkyl-carbamoyloxy, C₆₋₁₄ aryl-carbamoyloxy, nicotinoyloxy, 5 to 7 membered saturated cyclic amino optionally having 1 to 4 heteroatoms of one or two kinds selected from a nitrogen atom, a sulfur atom and an oxygen atom in addition to one nitrogen atom and carbon atoms (this cyclic amino may have substituents selected from the group consisting of C₁₋₆ alkyl, C₆₋₁₄ aryl, C₁₋₆ alkyl-carbonyl, 5 to 10 membered aromatic heterocyclic group and oxo), 5 to 10 membered aromatic heterocyclic group containing 1 to 4 heteroatoms of one or two kinds selected from a nitrogen atom, a sulfur atom and an oxygen atom in addition to carbon atoms, sulfo, sulfamoyl, sulfinamoyl and sulfenamoyl]
   (iii) a 5 to 14 membered heterocyclic group containing 1 to 4 heteroatoms of one or two kinds selected from a nitrogen atom, a sulfur atom and an oxygen atom in addition to carbon atoms optionally having substituents selected from the substituent group A,
   (iv) an acyl group represented by the formula:
      -(C=O)-R^{5a}, -(C=O)-OR^{5a}, -(C=O)-NR^{5a}R^{6a}, -(C=S)-NHR^{5a} or -SO₂-R^{7a}
      (wherein R^{5a} represents (1) a hydrogen atom, (2) a C₁₋₆ alkyl group, an C₂₋₆ alkenyl group, an C₂₋₆ alkynyl group, a C₃₋₆ cycloalkyl group, a C₆₋₁₄ aryl group or a C₇₋₁₆ aralkyl group optionally having substituents selected from the substituent group A or (3) a 5 to 14 membered heterocyclic group containing 1 to 4 heteroatoms of one or two kinds selected from a nitrogen atom, a sulfur atom and an oxygen atom in addition to carbon atoms optionally having substituents selected from the substituent group A, R^{6a} represents a hydrogen atom or a C₁₋₆ alkyl group, R^{7a} represents (1) a C₁₋₆ alkyl group, a C₂₋₆ alkenyl group, a C₂₋₆ alkynyl group, a C₃₋₆ cycloalkyl group, a C₆₋₁₄ aryl group or a C₇₋₁₆ aralkyl group optionally having substituents selected from the substituent group A or (2) a 5 to 14 membered heterocyclic group containing 1 to 4 heteroatoms of one or two kinds selected from a nitrogen atom, a sulfur atom and an oxygen atom in addition to carbon atoms optionally having substituents selected from the substituent group A),
   (v) an amino group (this amino group may have substituents selected from the group consisting of (1) a C₁₋₆ alkyl group, a C₂₋₆ alkenyl group, a C₂₋₆ alkynyl group, a C₃₋₆ cycloalkyl group, a C₆₋₁₄ aryl group or a C₇₋₁₆ aralkyl group optionally having substituents selected from the substituent group A, (2) a 5 to 14 membered heterocyclic group containing 1 to 4 heteroatoms of one or two kinds selected from a nitrogen atom, a sulfur atom and an oxygen atom in addition to carbon atoms optionally having substituents selected from the substituent group A, (3) an acyl group as defined in the (iv), and (4) a C₁₋₆ alkylidene group optionally having substituents selected from the substituent group A), or
   (vi) a 5 to 7 membered non-aromatic cyclic amino group optionally containing 1 to 4 heteroatoms of one or two kinds selected from a nitrogen atom, a sulfur atom and an oxygen atom in addition to one nitrogen atom and carbon atoms (this cyclic amino may have substituents selected from the group consisting of C₁₋₆ alkyl, C₆₋₁₄ aryl, C₁₋₆ alkyl-carbonyl, 5 to 10 membered aromatic heterocyclic group and oxo);
   R^{2a} represents (1) a C₆₋₁₄ monocyclic or fused polycyclic aromatic hydrocarbon group optionally having substituents selected from the substituent group A or (2) a 5 to 14 membered aromatic heterocyclic group containing 1 to 4 heteroatoms of one or two kinds selected from a nitrogen atom, a sulfur atom and an oxygen atom in addition to carbon atoms, optionally having substituents selected from the substituent group A;
   R^{3a} represents (1) a hydrogen atom, (2) a pyridyl group optionally having substituents selected from the substituent group A, or (3) a C₆₋₁₄ monocyclic or fused polycyclic aromatic hydrocarbon group optionally having substituents selected from the substituent group A;
   X^{a} represents O, S, SO or SO₂;
   Y^{a} represents a bond, O, S, SO, SO₂ or a group represented by the formula: NR^{4a} (wherein R^{4a} represents (1) a hydrogen atom, (2) a C₁₋₆ alkyl group, a C₂₋₆ alkenyl group, a C₂₋₆ alkynyl group, a C₃₋₆ cycloalkyl group, a C₆₋₁₄ aryl group or a C₇₋₁₆ aralkyl group optionally having substituents selected from the substituent group A or (3) an acyl group as defined in the (iv)),
   Z^{a} represents a bond, a C₁₋₁₅ alkylene group, a C₂₋₁₆ alkenylene group or a C₂₋₁₆ alkynylene group optionally having substituents selected from the substituent group A,
(5) the compound according to (1), wherein R^{1a} is an amino group optionally having substituents,
(6) the compound according to (1), wherein R^{1a} is (i) a C₁₋₆ alkyl group, (ii) a C₆₋₁₄ aryl group optionally substituted with substituents selected from C₁₋₆ alkylthio, C₁₋₆ alkylsulfonyl and halogen atom, or (iii) an amino group optionally having 1 or 2 acyl groups represented by the formula: -(C=O)-R^{5a}' (wherein R^{5a}' represents (1) a C₁₋₆ alkyl group, (2) a C₆₋₁₄ aryl group or (3) a 5 to 14 membered heterocyclic group containing 1 to 4 heteroatoms of one or two kinds selected from a nitrogen atom, a sulfur atom and an oxygen atom in addition to carbon atoms),
(7) the compound according to (1), wherein R^{1a} is an amino group optionally having 1 or 2 acyl groups represented by -(C=O)-R^{5a}" (wherein R^{5a}" represents (1) a C₆₋₁₄ aryl group or (2) a 5 to 14 membered heterocyclic group containing 1 to 4 heteroatoms of one or two kinds selected from a nitrogen atom, a sulfur atom and an oxygen atom in addition to carbon atoms),
(8) the compound according to (1), wherein R^{2a} is a C₆₋₁₄ aryl group optionally having substituents,
(9) the compound according to (1), wherein R^{2a} is a C₆₋₁₄ aryl group optionally substituted with halogen atom or C₁₋₆ alkoxy, or a 5 to 14 membered aromatic heterocyclic group containing 1 to 4 heteroatoms of one or two kinds selected from a nitrogen atom, a sulfur atom and an oxygen atom in addition to carbon atoms,
(10) the compound according to (1), wherein R^{2a} is a C₆₋₁₄ aryl group, or a 5 to 14 membered heterocyclic group containing 1 to 4 heteroatoms of one or two kinds selected from nitrogen atom, a sulfur atom and an oxygen atom in addition to carbon atoms,
(11) the compound according to (1), wherein R^{3a} is a C₆₋₁₄ aryl group optionally having substituents,
(12) the compound according to (1), wherein R^{3a} is a C₆₋₁₄ aryl group optionally substituted with one or two C₁₋₆ alkyl or C₁₋₆ alkoxy groups,
(13) the compound according to (1), wherein X^{a} is an optionally oxidized sulfur atom,
(14) the compound according to (1), wherein X^{a} is a sulfur atom,
(15) the compound according to (1), wherein Y^{a} is an oxygen atom or a group represented by the formula: NR^{4a} (wherein R^{4a} is as defined in (1)),
(16) the compound according to (1), wherein Y^{a} is an oxygen atom, an optionally oxidized sulfur atom or a group represented by the formula: NR^{4a}' (wherein R^{4a}' represents a C₁₋₆ alkyl group),
(17) the compound according to (1), wherein Y^{a} is O, NH or S,
(18) the compound according to (1), wherein Z^{a} is a lower alkylene group optionally having substituents,
(19) the compound according to (1), wherein Z^{a} is a bond or a C₁₋₆ alkylene group optionally having oxo,
(20) the compound according to (1), wherein R^{1a} is (i) a C₁₋₆ alkyl group, (ii) a C₆₋₁₄ aryl group optionally substituted with C₁₋₆ alkylthio, C₁₋₆ sulfonyl and halogen atom, or (iii) an amino group optionally having 1 or 2 acyl groups represented by the formula: -(C=O)-R^{5a}' (wherein R^{5a}' represents (1) a C₁₋₆ alkyl group, (2) a C₆₋₁₄ aryl group or (3) a 5 to 14 membered heterocyclic group containing 1 to 4 heteroatoms of one or two kinds selected from a nitrogen atom, a sulfur atom and an oxygen atom in addition to carbon atoms;
   R^{2a} is a C₆₋₁₄ aryl group optionally substituted with halogen atom or C₁₋₆ alkoxy, or a 5 to 14 membered aromatic heterocyclic group containing 1 to 4 heteroatoms of one or two kinds selected from a nitrogen atom, a sulfur atom and an oxygen atom in addition to carbon atoms;
   R^{3a} is a C₆₋₁₄ aryl group optionally substituted with 1 or 2 C₁₋₆ alkyl or C₁₋₆ alkoxy groups;
   X^{a} is a sulfur atom;
   Y^{a} is an oxygen atom, an optionally oxidized sulfur atom or a group represented by the formula: NR^{4a}' (wherein R^{4a}' represents a C₁₋₆ alkyl group) ;
   Z^{a} is a C₁₋₆ alkylene group optionally having oxo or C₁₋₆ alkyl or a bond,
(21) the compound according to (1), wherein R^{1a} is an amino group optionally having 1 or 2 acyl groups represented by -(C=O)-R^{5a}" (wherein R^{5a}" represents (1) a C₆₋₁₄ aryl group or (2) a 5 to 14 membered heterocyclic group containing 1 to 4 heteroatoms of one or two kinds selected from a nitrogen atom, a sulfur atom and an oxygen atom in addition to carbon atoms);
   R^{2a} is a C₆₋₁₄ aryl group or a 5 to 14 membered aromatic heterocyclic group containing 1 to 4 heteroatoms of one or two kinds selected from a nitrogen atom, a sulfur atom and an oxygen atom in addition to carbon atoms;
   R^{3a} is a C₆₋₁₄ aryl group optionally substituted with 1 or 2 C₁₋₆ alkyl or C₁₋₆ alkoxy groups;
   X^{a} is a sulfur atom; Y^{a} is O, NH or S; Z^{a} is a bond or a C₁₋₆ alkylene group optionally having oxo,
(22) N-[5-(2-benzoylamino-4-pyridyl)-4-(3,5-dimethylphenyl)-1,3-thiazol-2-yl]acetamide (Reference Example D Compound No.9),
   N-[5-(2-benzylamino-4-pyridyl)-4-(3,5-dimethylphenyl)-1,3-thiazol-2-yl]acetamide (Reference Example D Compound No.10),
   N-[4-[4-(4-methoxyphenyl)-2-methyl-1,3-thiazol-5-yl]-2-pyridyl]benzamide (Reference Example D Compound No.13),
   N-[4-[2-(4-fluorophenyl)-4-(3-methylphenyl)-1,3-thiazol-5-yl]-2-pyridyl]phenylacetamide (Reference Example D Compound No.14),
   N-[4-[2-ethyl-4-(3-methylphenyl)-1,3-thiazol-5-yl]-2-pyridyl]phenylacetamide (Reference Example D Compound No.15-2),
   N-[4-[4-(3-methylphenyl)-2-propyl-1,3-thiazol-5-yl]-2-pyridyl]phenylacetamide (Reference Example D Compound No.15-3),
   N-[4-[2-butyl-4-(3-methylphenyl)-1,3-thiazol-5-yl]-2-pyridyl]phenylacetamide (Reference Example D Compound No.15-4),
   N-[4-[4-(3-methylphenyl)-2-(4-methylthiophenyl)-1,3-thiazol-5-yl]-2-pyridyl]phenylacetamide (Reference Example D Compound No.15-6),
   N-[4-[2-ethyl-4-(3-methylphenyl)-1,3-thiazol-5-yl]-2-pyridyl]benzamide (Reference Example D Compound No.16-1),
   N-[4-[2-ethyl-4-(3-methylphenyl)-1,3-thiazol-5-yl]-2-pyridyl]-3-phenylpropionamide (Reference Example D Compound No.16-2),
   N-[4-[2-ethyl-4-(3-methylphenyl)-1,3-thiazol-5-yl]-2-pyridyl]-3-(4-methoxyphenyl)propionamide (Reference Example D Compound No.16-3),
   N-[4-[2-ethyl-4-(3-methylphenyl)-1,3-thiazol-5-yl]-2-pyridyl]-4-phenylbutyramide (Reference Example D Compound No.16-5),
   N-[4-[4-(3-methylphenyl)-2-propyl-1,3-thiazol-5-yl]-2-pyridyl]benzamide (Reference Example D Compound No.16-7),
   N-[4-[4-(3-methylphenyl)-2-propyl-1,3-thiazol-5-yl]-2-pyridyl]-3-phenylpropionamide (Reference Example D Compound No.16-8),
   N-[4-[2-butyl-4-(3-methylphenyl)-1,3-thiazol-5-yl]-2-pyridyl]benzamide (Reference Example D Compound No.16-9),
   N-[4-[2-butyl-4-(3-methylphenyl)-1,3-thiazol-5-yl]-2-pyridyl]-3-phenylpropionamide (Reference Example D Compound No.16-10),
   N-[4-[2-(4-fluorophenyl)-4-(3-methylphenyl)-1,3-thiazol-5-yl]-2-pyridyl]benzamide (Reference Example D Compound No.16-11),
   N-[4-[2-(4-fluorophenyl)-4-(3-methylphenyl)-1,3-thiazol-5-yl]-2-pyridyl]-3-phenylpropionamide (Reference Example D Compound No.16-12),
   N-[4-[4-(3-methylphenyl)-2-(4-methylthiophenyl)-1,3-thiazol-5-yl]-2-pyridyl]benzamide (Reference Example D Compound No.16-15),
   N-[4-[4-(3-methylphenyl)-2-(4-methylthiophenyl)-1,3-thiazol-5-yl]-2-pyridyl]-3-phenylpropionamide (Reference Example D Compound No.16-16),
   N-benzyl-N-[4-[2-ethyl-4-(3-methylphenyl)-1,3-thiazol-5-yl]-2-pyridyl]amine (Reference Example D Compound No.19-2),
   N-[4-[2-ethyl-4-(3-methylphenyl)-1,3-thiazol-5-yl]-2-pyridyl]-N-(2-phenylethyl)amine (Reference Example D Compound No.19-3),
   N-[4-[2-ethyl-4-(3-methylphenyl)-1,3-thiazol-5-yl]-2-pyridyl]-N-(3-phenylpropyl)amine (Reference Example D Compound No.19-4),
   N-benzyl-N-[4-[4-(3-methylphenyl)-2-propyl-1,3-thiazol-5-yl]-2-pyridyl]amine (Reference Example D Compound No.19-5),
   N-[4-[4-(3-methylphenyl)-2-propyl-1,3-thiazol-5-yl]-2-pyridyl]-N-(2-phenylethyl)amine (Reference Example D Compound No.19-6),
   N-[4-[4-(3-methylphenyl)-2-propyl-1,3-thiazol-5-yl]-2-pyridyl]-N-(3-phenylpropyl)amine (Reference Example D Compound No.19-7),
   N-benzyl-N-[4-[2-butyl-4-(3-methylphenyl)-1,3-thiazol-5-yl]-2-pyridyl]amine (Reference Example D Compound No.19-8),
   N-[4-[2-butyl-4-(3-methylphenyl)-1,3-thiazol-5-yl]-2-pyridyl]-N-(2-phenylethyl)amine (Reference Example D Compound No.19-9),
   N-[4-[2-butyl-4-(3-methylphenyl)-1,3-thiazol-5-yl]-2-pyridyl]-N-(3-phenylpropyl)amine (Reference Example D Compound No.19-10) ,
   N-benzyl-N-[4-[4-(3-methylphenyl)-2-(4-methylthiophenyl)-1,3-thiazol-5-yl]-2-pyridyl]amine (Reference Example D Compound No.19-17),
   N-[4-[4-(3-methylphenyl)-2-(4-methylthiophenyl)-1,3-thiazol-5-yl]-2-pyridyl]-N-(2-phenylethyl)amine (Reference Example D Compound No.19-18),
   N-[4-[4-(3-methylphenyl)-2-(4-methylthiophenyl)-1,3-thiazol-5-yl]-2-pyridyl]-N-(3-phenylpropyl)amine (Reference Example D Compound No.19-19),
   N-[4-[4-(3-methylphenyl)-2-(4-methylsulfonylphenyl)-1,3-thiazol-5-yl]-2-pyridyl]benzamide (Reference Example D Compound No.20),
   N-[4-[4-(3-methylphenyl)-2-(4-methylsulfonylphenyl)-1,3-thiazol-5-yl]-2-pyridyl]phenylacetamide (Reference Example D Compound No.21-1),
   N-[4-[4-(3-methylphenyl)-2-(4-methylsulfonylphenyl)-1,3-thiazol-5-yl]-2-pyridyl]-3-phenylpropionamide (Reference Example D Compound No.21-2),
   N-benzyl-N-[4-[4-(3-methylphenyl)-2-(4-methylsulfonylphenyl)-1,3-thiazol-5-yl]-2-pyridyl]amine (Reference Example D Compound No.21-5),
   N-[4-[4-(3-methylphenyl)-2-(4-methylsulfonylphenyl)-1,3-thiazol-5-yl]-2-pyridyl]-N-(3-phenylpropyl)amine (Reference Example D Compound No.21-6),
   N-[4-(4-(3-methylphenyl)-2-(4-methylsulfonylphenyl)-1,3-thiazol-5-yl]-2-pyridyl]-N-(2-phenylethyl)amine (Reference Example Compound No.25-1),
   N-(4-fluorobenzyl)-N-[4-[4-(3-methylphenyl)-2-(4-methylsulfonylphenyl)-1,3-thiazol-5-yl]-2-pyridyl]amine (Reference Example D Compound No.25-2), or salts thereof,

In the aforementioned formula, R^{1a} represents a hydrogen atom, a hydrocarbon group optionally having substituents, a heterocyclic group optionally having substituents, an amino group optionally having substituents or acyl group.

As "acyl group" represented by R^{1a}, for example, there are an acyl group represented by the formula: -(C=O)-R^{5a}, -(C=O) -OR^{5a}, -(C=O)-NR^{5a}R^{6a}, -(C=S)-NHR^{5a} or -SO₂-R^{7a} (wherein R^{5a} represents a hydrogen atom, a hydrocarbon group optionally having substituents or a heterocyclic group optionally having substituents, R^{6a} represents a hydrogen atom or a C₁₋₆ alkyl, R^{7a} represents a hydrocarbon group optionally having substituents or a heterocyclic group optionally having substituents) and the like.

In the aforementioned formula, as "hydrocarbon group" represented by R^{5a} of "hydrocarbon group optionally having substituents", for example, there are an acyclic or cyclic hydrocarbon group (for example, alkyl, alkenyl, alkynyl, cycloalkyl, aryl, aralkyl and the like) and the like. Among them, C₁₋₁₆ acyclic or cyclic hydrocarbon groups are preferable.

As "alkyl", for example, C₁₋₆ alkyl (for example, methyl, ethyl, propyl, isopropyl, butyl, isobutyl, sec-butyl, tert-butyl, pentyl, hexyl and the like) is preferable and, in particular, C₁₋₃ alkyl (for example, methyl, ethyl, propyl and isopropyl) and the like are preferable.

As "alkenyl", for example, C₂₋₆ alkenyl (for example, vinyl, allyl, isopropenyl, 1-butenyl, 2-butenyl, 3-butenyl, 2-methyl-2-propenyl, 1-methyl-2-propenyl, 2-methyl-1-propenyl and the like) and the like are preferable.

As "alkynyl", for example, C₂₋₆ alkynyl (for example, ethynyl, propargyl, 1-butynyl, 2-butynyl, 3-butynyl, 1-hexynyl and the like) and the like are preferable.

As "cycloalkyl", for example, C₃₋₆ cycloalkyl (for example, cyclopropyl, cyclobutyl, cyclopentyl, cyclohexyl and the like) and the like are preferable.

As "aryl", for example, C₆₋₁₄ aryl (for example, phenyl, 1-naphthyl, 2-naphthyl, 2-biphenylyl, 3-biphenylyl, 4-biphenylyl, 2-anthryl and the like) and the like are preferable.

As "aralkyl", for example, C₇₋₁₆ aralkyl (for example, benzyl, phenethyl, diphenylmethyl, 1-naphthylmethyl, 2-naphthylmethyl, 2,2-diphenylethyl, 3-phenylpropyl, 4-phenylbutyl, 5-phenylpentyl and the like) and the like are preferable.

As "substituents" of "hydrocarbon group optionally having substituents" represented by R^{5a}, for example, there are oxo, halogen atom (for example, fluorine, chlorine, bromine, iodine and the like), C₁₋₃ alkylenedioxy (for example, methylenedioxy, ethylenedioxy and the like), nitro, cyano, optionally halogenated C₁₋₆ alkyl, optionally halogenated C₂₋₆ alkenyl, carboxy C₂₋₆ alkenyl (for example, 2-carboxyethenyl, 2-carboxy-2-methylethenyl and the like), optionally halogenated C₂₋₆ alkynyl, optionally halogenated C₃₋₆ cycloalkyl, C₆₋₁₄ aryl (for example, phenyl, 1-naphthyl, 2-naphthyl, 2-biphenylyl, 3-biphenylyl, 4-biphenylyl, 2-anthryl and the like), optionally halogenated C₁₋₈ alkoxy, C₁₋₆ alkoxy-carbonyl-C₁₋₆ alkoxy (for example, ethoxycarbonylmethyloxy and the like), hydroxy, C₆₋₁₄ aryloxy (for example, phenyloxy, 1-naphthyloxy, 2-naphthyloxy and the like), C₇₋₁₆ aralkyloxy (for example, benzyloxy, phenethyloxy and the like), mercapto, optionally halogenated C₁₋₆ alkylthio, C₆₋₁₄ arylthio (for example, phenylthio, 1-naphthylthio, 2-naphthylthio and the like), C₇₋₁₆ aralkylthio (for example, benzylthio, phenethylthio and the like), amino, mono-C₁₋₆ alkylamino (for example, methylamino, ethylamino and the like), mono-C₆₋₁₄ arylamino (for example, phenylamino, 1-naphthylamino, 2-naphthylamino and the like), di-C₁₋₆ alkylamino (for example, dimethylamino, diethylamino, ethylmethylamino and the like), di-C₆₋₁₄ arylamino (for example, diphenylamino and the like), formyl, carboxy, C₁₋₆ alkyl-carbonyl (for example, acetyl, propionyl and the like), C₃₋₆ cycloalkyl-carbonyl (for example, cyclopropylcarbonyl, cyclopentylcarbonyl, cyclohexylcarbonyl and the like), C₁₋₆ alkoxy-carbonyl (for example, methoxycarbonyl, ethoxycarbonyl, propoxycarbonyl, tert-butoxycarbonyl and the like), C₆₋₁₄ aryl-carbonyl (for example, benzoyl, 1-naphthoyl, 2-naphthoyl and the like), C₇₋₁₆ aralkyl-carbonyl (for example, phenylacetyl, 3-phenylpropionyl and the like), C₆₋₁₄ aryloxy-carbonyl (for example, phenoxycarbonyl and the like), C₇₋₁₆ aralkyloxy-carbonyl (for example, benzyloxycarbonyl, phenethyloxycarbonyl and the like), 5 or 6 membered heterocyclic carbonyl (for example, nicotinoyl, isonicotinoyl, thenoyl, furoyl, morpholinocarbonyl, thiomorpholinocarbonyl, piperazin-1-ylcarbonyl, pyrrolidin-1-ylcarbonyl and the like), carbamoyl, thiocarbamoyl, mono-C₁₋₆ alkyl-carbamoyl (for example, methylcarbamoyl, ethylcarbamoyl and the like), di-C₁₋₆ alkyl-carbamoyl (for example, dimethylcarbamoyl, diethylcarbamoyl, ethylmethylcarbamoyl and the like), C₆₋₁₄ aryl-carbamoyl (for example, phenylcarbamoyl, 1-naphthylcarbamoyl, 2-naphthylcarbamoyl and the like), 5 or 6 membered heterocyclic carbamoyl (for example, 2-pyridylcarbamoyl, 3-pyridylcarbamoyl, 4-pyridylcarbamoyl, 2-thienylcarbamoyl, 3-thienylcarbamoyl and the like), C₁₋₆ alkylsulfonyl (for example, methylsulfonyl, ethylsulfonyl and the like), C₆₋₁₄ arylsulfonyl (for example, phenylsulfonyl, 1-naphthylsulfonyl, 2-naphthylsulfonyl and the like), C₁₋₆ alkylsulfinyl (for example, methylsulfinyl, ethylsulfinyl and the like), C₆₋₁₄ arylsulfinyl (for example, phenylsulfinyl, 1-naphthylsulfinyl, 2-naphthylsulfinyl and the like), formylamino, C₁₋₆ alkyl-carbonylamino (for example, acetylamino and the like), C₆₋₁₄ aryl-carbonylamino (for example, benzoylamino, naphthoylamino and the like), C₁₋₆ alkoxy-carbonylamino (for example, methoxycarbonylamino, ethoxycarbonylamino, propoxycarbonylamino, butoxycarbonylamino and the like), C₁₋₆ alkylsulfonylamino (for example, methylsulfonylamino, ethylsulfonylamino and the like), C₆₋₁₄ arylsulfonylamino (for example, phenylsulfonylamino, 2-naphthylsulfonylamino, 1-naphthylsulfonylamino and the like), C₁₋₆ alkyl-carbonyloxy (for example, acetoxy, propionyloxy and the like), C₆₋₁₄ aryl-carbonyloxy (for example, benzoyloxy, naphthylcarbonyloxy and the like), C₁₋₆ alkoxy-carbonyloxy (for example, methoxycarbonyloxy, ethoxycarbonyloxy, propoxycarbonyloxy, butoxycarbonyloxy and the like), mono-C₁₋₆ alkyl-carbamoyloxy (for example, methylcarbamoyloxy, ethylcarbamoyloxy and the like), di-C₁₋₆ alkyl-carbamoyloxy (for example, dimethylcarbamoyloxy, diethylcarbamoyloxy and the like), C₆₋₁₄ aryl-carbamoyloxy (for example, phenylcarbamoyloxy, naphthylcarbamoyloxy and the like), nicotinoyloxy, 5 to 7 membered saturated cyclic amino optionally having substituents, 5 to 10 membered aromatic heterocyclic group (for example, 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like), sulfo, sulfamoyl, sulfinamoyl, sulfenamoyl and the like.

The "hydrocarbon group" may have 1 to 5, preferably 1 to 3 aforementioned substituents at a substitutable position and, when the number of substituents is 2 or more, respective substituents may be the same or different.

As aforementioned "optionally halogenated C₁₋₆ alkyl", for example, there are C₁₋₆ alkyl (for example, methyl, ethyl, propyl, isopropyl, butyl, isobutyl, sec-butyl, tert-butyl, pentyl, hexyl and the like) and the like optionally having 1 to 5, preferably 1 to 3 halogen atoms (for example, fluorine, chlorine, bromine, iodine and the like). Examples thereof are methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl and the like.

As the aforementioned "optionally halogenated C₂₋₆ alkenyl", for example, there are C₂₋₆ alkenyl (for example, vinyl, propenyl, isopropenyl, 2-buten-1-yl, 4-penten-1-yl, 5-hexen-1-yl) and the like optionally having 1 to 5, preferably 1 to 3 halogen atoms (for example, fluorine, chlorine, bromine, iodine and the like).

As the aforementioned "optionally halogenated C₂₋₆ alkynyl", there are C₂₋₆ alkynyl (for example, 2-butyn-1-yl, 4-pentyn-1-yl, 5-hexyn-1-yl and the like) and the like optionally having 1 to 5, preferably 1 to 3 halogen atoms (for example, fluorine, chlorine, bromine, iodine and the like).

As the aforementioned "optionally halogenated C₃₋₆ cycloalkyl", for example, there are C₃₋₆ cycloalkyl (for example, cyclopropyl, cyclobutyl, cyclopentyl, cyclohexyl and the like) and the like optionally having 1 to 5, preferably 1 to 3 halogen atoms (for example, fluorine, chlorine, bromine, iodine and the like). Examples thereof are cyclopropyl, cyclobutyl, cyclopentyl, cyclohexyl, 4,4-dichlorocyclohexyl, 2,2,3,3-tetrafluorocyclopentyl, 4-chlorocyclohexyl and the like.

As the aforementioned "optionally halogenated C₁₋₈ alkoxy", for example, there are C₁₋₈ alkoxy (for example, methoxy, ethoxy, propoxy, isopropoxy, butoxy, isobutoxy, sec-butoxy, pentyloxy, hexyloxy and the like) and the like optionally having 1 to 5, preferably 1 to 3 halogen atoms (for example, fluorine, chlorine, bromine, iodine and the like). Examples thereof are methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy and the like.

As the aforementioned "optionally halogenated C₁₋₆ alkylthio", for example, there are C₁₋₆ alkylthio (for example, methylthio, ethylthio, propylthio, isopropylthio, butylthio, sec-butylthio, tert-butylthio and the like) and the like optionally having 1 to 5, preferably 1 to 3 halogen atoms (for example, fluorine, chlorine, bromine, iodine and the like). Examples thereof are methylthio, difluoromethylthio, trifluoromethylthio, ethylthio, propylthio, isopropylthio, butylthio, 4,4,4-trifluorobutylthio, pentylthio, hexylthio and the like.

As "5 to 7 membered saturated cyclic amino" of the aforementioned "5 to 7 membered saturated cyclic amino optionally having substituents", there are 5 to 7 membered saturated cyclic amino optionally containing 1 to 4 heteroatoms of one or two kinds selected from a nitrogen atom, a sulfur atom and an oxygen atom in addition to one nitrogen atom and carbon atoms and examples thereof are pyrrolidin-1-yl, piperidino, piperazin-1-yl, morpholino, thiomorpholino, hexahydroazepin-1-yl and the like.

As "substituents" of the "5 to 7 membered saturated cyclic amino optionally having substituents", for example, there are 1 to 3 C₁₋₆ alkyl (for example, methyl, ethyl, propyl, isopropyl, butyl, isobutyl, sec-butyl, tert-butyl, pentyl, hexyl and the like), C₆₋₁₄ aryl (for example, phenyl, 1-naphthyl, 2-naphthyl, 2-biphenylyl, 3-biphenylyl, 4-biphenylyl, 2-anthryl and the like), C₁₋₆ alkyl-carbonyl (for example, acetyl, propionyl and the like), 5 to 10 membered aromatic heterocyclic group (for example, 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like), oxo and the like.

As "heterocyclic group" of "heterocyclic group optionally having substituents" represented by R^{5a}, for example, there is a monovalent group obtained by removing one arbitrary hydrogen atom from a 5 to 14 membered (monocyclic, bicyclic or tricyclic) heterocycle containing 1 to 4 heteroatoms of one or two kinds selected from a nitrogen atom, a sulfur atom and an oxygen atom in addition to carbon atoms, preferably (i) a 5 to 14 membered (preferably 5 to 10 membered, particularly preferably 5 to 6 membered) aromatic heterocycle, (ii) a 5 to 10 membered (preferably 5 to 6 membered) non-aromatic heterocycle or (iii) a 7 to 10 membered bridged heterocycle.

As the aforementioned "5 to 14 membered (preferably 5 to 10 membered) aromatic heterocycle", there are an aromatic heterocycle such as thiophene, benzo[b]thiophene, benzo[b]furan, benzimidazole, benzoxazole, benzothiazole, benzisothiazole, naphtho[2,3-b]thiophene, furan, pyrrole, imidazole, pyrazole, pyridine, pyrazine, pyrimidine, pyridazine, indole, isoindole, 1H-indazole, purine, 4H-quinolizine, isoquinoline, quinoline, phthalazine, naphthyridine, quinoxaline, quinazoline, cinnoline, carbazole, β-carboline, phenanthridine, acridine, phenazine, thiazole, isothiazole, phenothiazine, isoxazole, furazan, phenoxazine and the like, and a ring formed by fusing these rings (preferably monocyclic) with 1 or a plurality (preferably 1 to 2) of aromatic rings (for example, benzene ring and the like).

As the aforementioned "5 to 10 membered non-aromatic heterocycle", for example, there are pyrrolidine, imidazoline, pyrazolidine, pyrazoline, piperidine, piperazine, morpholine, thiomorpholine, dioxazole, oxadiazoline, thiadiazoline, triazoline, thiadiazole, dithiazole and the like.

As the aforementioned "7 to 10 membered bridged heterocycle", for example, there are quinuclidine, 7-azabicyclo[2.2.1]heptane and the like.

The "heterocyclic group" is preferably a 5 to 14 membered (preferably 5 to 10 membered) (monocyclic or bicyclic) heterocyclic group containing preferably 1 to 4 heteroatoms of one or two kinds selected from a nitrogen atom, a sulfur atom and an oxygen atom in addition to carbon atoms. More particularly, examples thereof are an aromatic heterocyclic group such as 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 2-pyrimidinyl, 4-pyrimidinyl, 3-pyrrolyl, 2-imidazolyl, 3-pyridazinyl, 3-isothiazolyl, 3-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like, and a non-aromatic heterocyclic group such as 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino and the like.

Among them, for example, a 5 or 6 membered heterocyclic group containing 1 to 3 heteroatoms selected from a nitrogen atom, a sulfur atom and an oxygen atom in addition to carbon atoms is further preferable. More particularly, examples thereof are 2-thienyl, 3-thienyl, 2-pyridyl, 3-pyridyl, 4-pyridyl, 2-furyl, 3-furyl, pyrazinyl, 2-pyrimidinyl, 3-pyrrolyl, 3-pyridazinyl, 3-isothiazolyl, 3-isoxazolyl, 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino and the like.

As "substituents" of "heterocyclic group optionally having substituents", for example, there are the same "substituents" as substituents of "hydrocarbon group optionally having substituents" represented by R^{5a}.

The "heterocyclic group" may have 1 to 5, preferably 1 to 3 aforementioned substituents at a substitutable position and, when the number of substituents is 2 or more, respective substituents may be the same or different.

As "C₁₋₆ alkyl" represented by R^{6a}, for example, there are methyl, ethyl, propyl, isopropyl, butyl, isobutyl, sec-butyl, tert-butyl, pentyl, hexyl and the like.

As "hydrocarbon group optionally having substituents" and "heterocyclic group optionally having substituents" represented by R^{7a}, for example, there are the aforementioned "hydrocarbon group optionally having substituents" and "heterocyclic group optionally having substituents" represented by R^{5a}, respectively.

As "hydrocarbon group optionally having substituents" and "heterocyclic group optionally having substituents" represented by R^{1a}, for example, there are the aforementioned "hydrocarbon group optionally having substituents" and "heterocyclic group optionally having substituents" represented by R^{5a}, respectively.

As "amino group optionally having substituents" represented by R^{1a}, for example, there are (1) an amino group optionally having 1 or 2 substituents and (2) a cyclic amino group optionally having substituents and the like.

As "substituents" of "amino group optionally having 1 or 2 substituents" of the aforementioned (1), for example, there are a hydrocarbon group optionally having substituents, a heterocyclic group optionally having substituents, an acyl group, an alkylidene group optionally having substituents and the like. As these "hydrocarbon group optionally having substituents" and "heterocyclic group optionally having substituents", there are the same "hydrocarbon group optionally having substituents" and "heterocyclic group optionally having substituents" as those represented by R^{5a} described above, respectively. As the "acyl group", there is the same "acyl group" as that by represented by R^{1a} as described above.

As "alkylidene group" of "alkylidene group optionally having substituents", for example, there are a C₁₋₆ alkylidene group (for example, methylidene, ethylidene, propylidene and the like) and the like. As "substituents" of "alkylidene group optionally having substituents", there are 1 to 5, preferably 1 to 3 same substituents as "substituents" of "hydrocarbon group optionally having substituents" represented by R^{5a}.

When the number of the aforementioned "substituents" of "amino group optionally having 1 or 2 substituents" is 2, respective substituents may be the same or different.

As "cyclic amino group" of "cyclic amino group optionally having substituents" of the aforementioned (2), there are a 5 to 7 membered non-aromatic cyclic amino group optionally containing 1 to 4 heteroatoms of one or two kinds selected from a nitrogen atom, a sulfur atom and an oxygen atom in addition to one nitrogen atom and carbon atoms. More particularly, examples thereof are pyrrolidin-1-yl, piperidino, piperazin-1-yl, morpholino, thiomorpholino, hexahydroazepin-1-yl, imidazolidin-1-yl, 2,3-dihydro-1H-imidazol-1-yl, tetrahydro-1(2H)-pyrimidinyl, 3,6-dihydro-1(2H)-pyrimidinyl, 3,4-dihydro-1(2H)-pyrimidinyl and the like. As "substituents" of "cyclic amino optionally having substituents", there are 1 to 3 of the same ones as "substituents" of "5 to 7 membered saturated cyclic amino group" which were described in detail as "substituents" of "hydrocarbon group optionally having substituents" represented by R^{5a}.

Examples of the 5 to 7 membered non-aromatic cyclic amino group having 1 oxo, there are 2-oxoimidazolidin-1-yl, 2-oxo-2,3-dihydro-1H-imidazol-1-yl, 2-oxotetrahydro-1(2H)-pyrimidinyl, 2-oxo-3,6-dihydro-1(2H)-pyrimidinyl, 2-oxo-3,4-dihydro-1(2H)-pyrimidinyl, 2-oxopyrrolidin-1-yl, 2-oxopiperidino, 2-oxopiperazin-1-yl, 3-oxopiperazin-1-yl, 2-oxo-2,3,4,5,6,7-hexahydroazepin-1-yl and the like.

As R^{1a}, an amino group optionally having substituents, an aryl group optionally having substituents and an alkyl group optionally having substituents and the like are preferable.

As further preferable example of the "amino group optionally having substituents" is an amino group optionally having 1 or 2 acyl groups represented by the formula:
-(C=O)-R^{5a}, -(C=O)-OR^{5a}, -(C=O)-NR^{5a}R^{6a}, -(C=S)-NHR^{5a} or -SO₂-R^{7a} [wherein respective symbols represent the same meanings as described above]. Particularly preferable example is an amino group optionally having 1 or 2 acyl groups represented by the formula: -C(C=O)-R^{5a} or -(C=O)-NR^{5a}R^{6a} [wherein respective symbols represent the same meanings as described above].

As the "aryl group optionally having substituents", for example, there is preferably a C₆₋₁₄ aryl group (preferably a phenyl group and the like) optionally having 1 to 5 substituents selected from C₁₋₆ alkylthio, C₆₋₁₄ arylthio, C₁₋₆ alkylsulfinyl, C₆₋₁₄ arylsulfinyl, C₁₋₆ alkylsulfonyl, C₆₋₁₄ arylsulfonyl and carboxy.

As the "alkyl group optionally having substituents", for example, a C₁₋₆ alkyl group (for example, methyl, ethyl, propyl, isopropyl, butyl, isobutyl, sec-butyl, tert-butyl and the like) optionally substituted with 1 to 3 substituents selected from halogen atom, C₁₋₆ alkoxy, hydroxy, carboxy and C₁₋₆ alkoxy-carbonyl and the like are preferable, and particularly C₁₋₃ alkyl groups such as methyl, ethyl and the like is preferable.

Among them, as R^{1a}, (i) C₁₋₆ alkyl group (for example, C₁₋₄ alkyl group such as methyl, ethyl, propyl, butyl), (ii) a C₆₋₁₄ aryl group (for example, a phenyl group) optionally substituted with substituents selected from C₁₋₆ alkylthio (for example, methylthio), C₁₋₆ alkylsulfonyl (for example, methylsulfonyl) and halogen atom (for example, chlorine atom, fluorine atom) or (iii) an amino group optionally having 1 or 2 acyl groups represented by the formula: -(C=O)-R^{5a}' (wherein R^{5a}' represents (1) a C₁₋₆ alkyl group (for example, C₁₋₃ alkyl group such as methyl), (2) a C₆₋₁₄ aryl group (for example, a phenyl group) or (3) a 5 to 14 membered heterocyclic group containing 1 to 4 heteroatoms of one or two kinds selected from a nitrogen atom, a sulfur atom and an oxygen atom in addition to carbon atoms (for example, a 5 to 6 membered heterocyclic group containing 1 to 2 heteroatoms selected from a nitrogen atom, a sulfur atom and an oxygen atom in addition to carbon atoms such as pyridyl group) are preferable. As R^{5a}' and R^{5a}", a phenyl group or a pyridyl group is suitable.

In the aforementioned formula, R^{2a} represents an aromatic group optionally having substituents.

As "aromatic group" of "aromatic group optionally having substituents" represented by R^{2a}, for example, there are an aromatic hydrocarbon group, an aromatic heterocyclic group and the like.

As the "aromatic hydrocarbon group", examples thereof include a C₆₋₁₄ monocyclic or fused polycyclic (bicyclic or tricyclic) aromatic hydrocarbon group, etc. As examples, there are a C₆₋₁₄ aryl group and the like such as phenyl, 1-naphthyl, 2-naphthyl, 2-biphenylyl, 3-biphenylyl, 4-biphenylyl, 2-anthryl and the like and, further preferably, a C₆₋₁₀ aryl group and the like (for example, phenyl, 1-naphthyl, 2-naphthyl and the like, preferably phenyl and the like).

As the "aromatic heterocyclic group", there is a monovalent group obtained by removing one arbitrary hydrogen atom from 5 to 14 membered (preferably 5 to 10 membered) aromatic heterocycle containing 1 to 4 heteroatoms of one or two kinds selected from nitrogen atom, sulfur atom and oxygen atom in addition to carbon atoms.

As the aforementioned "5 to 14 membered (preferably 5 to 10 membered) aromatic heterocycle", for example, there are an aromatic heterocycle such as thiophene, benzo[b]thiophene, benzo[b]furan, benzimidazole, benzoxazole, benzothiazole, benzisothiazole, naphtho[2,3-b]thiophene, furan, pyrrole, imidazole, pyrazole, pyridine, pyrazine, pyrimidine, pyridazine, indole, isoindole, 1H-indazole, purine, 4H-quinolizine, isoquinoline, quinoline, phthalazine, naphthyridine, quinoxaline, quinazoline, cinnoline, carbazole, β-carboline, phenanthridine, acridine, phenazine, thiazole, isothiazole, phenothiazine, isoxazole, furazan, phenoxazine and the like, and a ring formed by fusing these rings (preferably monocycle) with 1 or a plurality of (preferably 1 or 2) aromatic rings (for example, benzene ring and the like).

As the "aromatic heterocyclic group", there are preferably a 5 to 14 membered (preferably 5 to 10 membered)(monocyclic or bicyclic) aromatic heterocyclic group containing preferably 1 to 4 heteroatoms of one or two kinds selected from a nitrogen atom, a sulfur atom and an oxygen atom in addition to carbon atoms and the like and, more particularly, there are an aromatic heterocyclic group such as 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 2-pyrimidinyl, 4-pyrimidinyl, 3-pyrrolyl, 2-imidazolyl, 3-pyridazinyl, 3-isothiazolyl, 3-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like.

As "substituents" of "aromatic group optionally having substituents", there are 1 to 5, preferably 1 to 3 same substituents as "substituents" of "hydrocarbon group optionally having substituents" represented by R^{5a}. When the number of substituents is 2 or more, respective substituents may be the same or different.

As R^{2a}, (1) a C₆₋₁₄ aryl group optionally having substituents and (2) a 5 to 14 membered aromatic heterocyclic group containing 1 to 4 heteroatoms of one or two kinds selected from a nitrogen atom, a sulfur atom and an oxygen atom in addition to carbon atoms are preferable and, among them, (1) a C₆₋₁₄ aryl group (for example, phenyl group, naphthyl group) optionally substituted with halogen atom (for example, chlorine atom, fluorine atom) or C₁₋₆ alkoxy (for example, methoxy), (2) a 5 to 14 membered aromatic heterocyclic group containing 1 to 4 heteroatoms of one or two kinds selected from a nitrogen atom, a sulfur atom and an oxygen atom in addition to carbon atoms (for example, a 5 to 6 membered aromatic heterocyclic group containing 1 to 2 heteroatoms selected from a nitrogen atom, a sulfur atom and an oxygen atom in addition to carbon atoms such as pyridyl group, thienyl group) and the like are preferable and, in particular, a phenyl group, a pyridyl group and the like are suitable.

In the aforementioned formula, R^{3a} represents a hydrogen atom, a pyridyl group optionally having substituents or an aromatic hydrocarbon group optionally having substituents.

As "substituents" of "pyridyl group optionally having substituents" represented by R^{3a}, there are the same substituents as "substituents" of "hydrocarbon group optionally having substituents" represented by R^{5a}.

The "pyridyl group" may, for example, have 1 to 5, preferably 1 to 3 aforementioned substituents at substitutable positions and, when the number of substituents is 2 or more, respective substituents may be the same or different. In addition, an intracyclic nitrogen atom may be N-oxidized.

As "aromatic hydrocarbon group" of "aromatic hydrocarbon group optionally having substituents" represented by R^{3a}, there is the same aromatic hydrocarbon group as "aromatic hydrocarbon group" of "aromatic group optionally having substituents" represented by R^{2a} and, preferably, there are a C₆₋₁₄ aryl group and the like such as phenyl, 1-naphthyl, 2-naphthyl, 2-biphenylyl, 3-biphenylyl, 4-biphenylyl, 2-anthryl and the like and, further preferably, a C₆₋₁₀ aryl group and the like (for example, phenyl, 1-naphthyl, 2-naphthyl and the like, preferably phenyl and the like) and the like. As "substituents" of "aromatic hydrocarbon group optionally having substituents" represented by R^{3a}, there are the same substituents as substituents of "aromatic group optionally having substituents" represented by R^{2a}.

As R^{3a}, a C₆₋₁₄ aryl group optionally having substituents is preferable and, among them, a C₆₋₁₄ aryl group optionally substituted with 1 or 2 C₁₋₆ alkyl (for example, methyl, ethyl and the like) or C₁₋₆ alkoxy groups (for example, methoxy, ethoxy and the like) is preferable and, in particular, a phenyl group optionally substituted with 1 or 2 C₁₋₆ alkyl or C₁₋₆ alkoxy groups (for example, 3-methoxyphenyl, 2-methylphenyl, 2,4-dimethylphenyl and the like) is suitable.

In the aforementioned formula, X^{a} represents an oxygen atom or an optionally oxidized sulfur atom.

As "optionally oxidized sulfur atom" represented by X^{a}, there are S, SO and SO₂.

As X^{a}, there is preferably an optionally oxidized sulfur atom. Further preferably, it is S.

In the aforementioned formula, Y^{a} represents a bond, an oxygen atom, an optionally oxidized sulfur atom or the formula NR^{4a} (wherein R^{4a} represents a hydrogen atom, a hydrocarbon group optionally having substituents or an acyl group).

As "optionally oxidized sulfur atom" represented by Y^{a}, there are S, SO and SO₂.

As "hydrocarbon group optionally having substituents" represented by R^{4a}, for example, there is the same group as "hydrocarbon group optionally having substituents" represented by R^{5a}. Among them, a C₁₋₆ alkyl group such as methyl, ethyl and the like and, in particular, a C₁₋₃ alkyl group such as methyl and the like is preferable.

As "acyl group" represented by R^{4a}, there is the same group as "acyl group" represented by R^{1a}.

As Y^{a}, an oxygen atom, an optionally oxidized sulfur atom, a group represented by the formula NR^{4a} (wherein R^{4a} represents the same meaning as that described above) and the like are preferable and, among them, an oxygen atom, an optionally oxidized sulfur atom, a group represented by the formula NR^{4a}' (R^{4a}' represents a hydrogen atom or a C₁₋₆ alkyl group) and the like are preferable and, further, an oxygen atom, S, SO₂, NH, N(CH₃) and the like are preferable and, in particular, O or NH is suitable.

In the aforementioned formula, Z^{a} represents a bond or a divalent acyclic hydrocarbon group optionally having substituents.

As "divalent acyclic hydrocarbon group" of "divalent acyclic hydrocarbon group optionally having substituents" represented by Z^{a}, for example, there are a C₁₋₁₅ alkylene group (for example, methylene, ethylene, propylene, butylene, pentamethylene, hexamethylene, heptamethylene, octamethylene and the like, preferably a C₁₋₆ alkylene group and the like), a C₂₋₁₆ alkenylene group (for example, vinylene, propenylene, 1-butenylene, 2-butenylene, 1-pentenylene, 2-pentenylene, 3-pentenylene and the like), a C₂₋₁₆ alkynylene group (ethynylene, propynylene, 1-butynylene, 2-butynylene, 1-pentynylene, 2-pentyriylene, 3-pentynylene and the like) and the like, preferably, a C₁₋₁₅ alkylene group, particularly preferably, a C₁₋₆ alkylene group and the like. As "substituents" of "divalent acyclic hydrocarbon group optionally having substituents" represented by Z^{a}, for example, there are the same substituents as "substituents" of "hydrocarbon group optionally having substituents" represented by R^{5a}.

As Z^{a}, a lower alkylene group optionally having C₁₋₃ alkyl (for example, methyl), oxo and the like (for example, a C₁₋₆ alkylene group such as methylene, ethylene, propylene and the like, in particular, a C₁₋₃ alkylene group) is preferable and, among them, a C₁₋₆ alkylene group optionally having oxo (for example, a C₁₋₃ alkylene group such as methylene, ethylene, propylene, in particular, methylene) is suitable.

More particularly, as Z^{a}, -CH₂-, -(CH₂)₂-, -(CH₂)₃-, -CO-, -CH₂CO-, -(CH₂)₂CO-, -CH(CH₃)- and the like are used and, in particular, -CH₂-, -CO- and the like are suitable.

A nitrogen atom in Compound (II) may be N-oxidized. For example, a nitrogen atom which is a constituent atom of 4-pyridyl group as a substituent at 5-position of a ring represented by the formula: 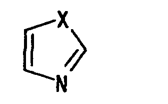 wherein a symbol in the formula represents the same meaning as that described above, may be N-oxidized. As Compound (II), for example, a compound represented by the formula: 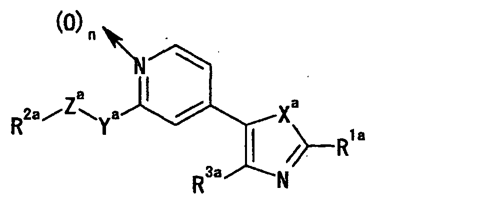 wherein n represents 0 or 1, and other symbols represents the same meanings as those described above, or salts thereof are preferable.

As Compound (II), compounds shown by the following (A) to (F) are preferably used.
(A) Compound (II) wherein R^{1a} is an amino group optionally having substituents, R^{2a} is a C₆₋₁₄ aryl group optionally having substituents, R^{3a} is a C₆₋₁₄ aryl group optionally having substituents, X is a sulfur atom, Y is an oxygen atom or a group represented by the formula NR^{4a} (wherein R^{4a} represents the same meaning as that described above) or (and) Z is a lower alkylene group optionally having substituents.
(B) Compound (II) wherein R^{1a} is (i) a C₁₋₆ alkyl group (for example, a C₁₋₄ alkyl group such as methyl, ethyl, propyl, butyl and the like),
   (ii) a C₆₋₁₄ aryl group (for example, a phenyl group) optionally substituted with substituents selected from C₁₋₆ alkylthio (for example, methylthio), C₁₋₆ alkylsulfonyl (for example, methylsulfonyl) and halogen atom (for example, chlorine atom, fluorine atom), or
   (iii) an amino group optionally having 1 or 2 acyl groups represented by the formula: -(C=O)-R^{5a}' [wherein R^{5a}' represents (1) a C₁₋₆ alkyl group (for example, C₁₋₃ alkyl group such as methyl and the like), (2) a C₆₋₁₄ aryl group (for example, a phenyl group) or (3) a 5 to 14 membered heterocyclic group containing 1 to 4 heteroatoms of one or two kinds selected from a nitrogen atom, a sulfur atom and an oxygen atom in addition to carbon atoms (for example, a 5 to 6 membered heterocyclic group containing 1 to 2 heteroatoms selected from a nitrogen atom, a sulfur atom and an oxygen atom in addition to carbon atoms such as a pyridyl group);
   R^{2a} is a C₆₋₁₄ aryl group (for example, a phenyl group, a naphthyl group) optionally substituted with halogen atom (for example, chlorine atom, fluorine atom) or C₁₋₆ alkoxy (for example, methoxy), or a 5 to 14 membered aromatic heterocyclic group containing 1 to 4 heteroatoms of one or two kinds selected from a nitrogen atom, a sulfur atom and an oxygen atom in addition to carbon atoms (for example, a 5 to 6 membered aromatic heterocyclic group containing 1 to 2 heteroatoms selected from a nitrogen atom, a sulfur atom and an oxygen atom in addition to carbon atoms such as a pyridyl group, a thienyl group and the like);
   R^{3a} is a C₆₋₁₄ aryl group (particularly, a phenyl group) optionally substituted with 1 or 2 C₁₋₆ alkyl (for example, methyl) or C₁₋₆ alkoxy groups (for example, methoxy);
   X^{a} is a sulfur atom;
   Y^{a} is an oxygen atom, an optionally oxidized sulfur atom or a group represented by the formula NR^{4a}' (R^{4a}' is a hydrogen atom or a C₁₋₆ alkyl group) (in particular, an oxygen atom, S, SO₂, NH, N(CH₃) and the like);
   Z^{a} is a C₁₋₆ alkylene group (in particular, a C₁₋₃ alkylene group) optionally having oxo or C₁₋₆ alkyl (for example, C₁₋₃ alkyl such as methyl) or a bond.
(C) Compound (II) wherein R^{1a} is an amino group optionally having 1 or 2 acyl groups represented by the formula -(C=O)-R^{5a}" (wherein R^{5a}" represents (1) a C₆₋₁₄ aryl group (for example, phenyl group) or (2) a 5 to 14 membered heterocyclic group containing 1 to 4 heteroatoms of one or two kinds selected from a nitrogen atom, a sulfur atom and an oxygen atom in addition to carbon atoms (for example, a 5 to 6 membered heterocyclic group containing 1 to 2 heteroatoms selected from a nitrogen atom, a sulfur atom and an oxygen atom in addition to carbon atoms such as a pyridyl group);
   R^{2a} is a C₆₋₁₄ aryl group (for example, a phenyl group) or a 5 to 14 membered aromatic heterocyclic group containing 1 to 4 heteroatoms of one or two kinds selected from a nitrogen atom, a sulfur atom and an oxygen atom in addition to carbon atoms (for example, a 5 to 6 membered aromatic heterocyclic group containing 1 to 2 heteroatoms selected from a nitrogen atom, a sulfur atom and an oxygen atom in addition to carbon atoms such as a pyridyl group);
   R^{3a} is a C₆₋₁₄ aryl group (in particular, a phenyl group) optionally substituted with 1 or 2 C₁₋₆ alkyl (for example, methyl) or C₁₋₆ alkoxy groups (for example, methoxy);
   X^{a} is a sulfur atom;
   Y^{a} is O, NH or S;
   Z^{a} is a bond or a C₁₋₆ alkylene group optionally having oxo (in particular, a C₁₋₃ alkylene group, such as methylene, ethylene and the like).
(D) Compound (II) prepared in Reference Examples D 1-79.
(E) [4-(3,5-dimethylphenyl)-5-(2-phenylmethyloxy-4-pyridyl)-1,3-thiazol-2-yl]amine (Reference Example D Compound No. 1),
   N-[4-[2-benzoylamino-4-(4-methoxyphenyl)-1,3-thiazol-5-yl]-2-pyridyl]benzamide (Reference Example D Compound No. 2),
   N-[4-(4-methoxyphenyl)-5-[2-[(3-pyridylcarbonylamino)]-4-pyridyl]-1,3-thiazol-2-yl]nicotinamide (Reference Example D Compound No. 3),
   N-[4-[2-amino-4-(4-methoxyphenyl)-1,3-thiazol-5-yl]-2-pyridyl]benzamide (Reference Example D Compound No. 4),
   N-[4-[2-amino-4-(3,5-dimethylphenyl)-2,3-thiazol-5-yl]-2-pyridyl]benzamide (Reference Example D Compound No. 5),
   N-[4-[2-amino-4-(3,5-dimethylphenyl)-1,3-thiazol-5-yl]-2-pyridyl]benzylamine (Reference Example D Compound No. 6),
   N-[4-[2-amino-4-(3,5-dimethylphenyl)-1,3-thiazol-5-yl]-2-pyridyl]benzamide hydrochloride (Reference Example D Compound No. 7),
   N-[4-[2-amino-4-(3,5-dimethylphenyl)-1,3-thiazol-5-yl]-2-pyridyl]benzylamine dihydrochloride (Reference Example D Compound No. 8).
(F) N-[5-[2-benzoylamino-4-pyridyl)-4-(3,5-dimethylphenyl)-1,3-thiazol-2-yl]acetamide (Reference Example D Compound No. 9),
   N-[5-(2-benzylamino-4-pyridyl)-4-(3,5-dimethylphenyl)-1,3-thiazol-2-yl]acetamide (Reference Example D Compound No. 10),
   N-[4-[4-(4-methoxyphenyl)-2-methyl-1,3-thiazol-5-yl]-2-pyridyl]benzamide (Reference Example D Compound No. 13),
   N-[4-[2-(4-fluorophenyl)-4-(3-methylphenyl)-1,3-thiazol-5-yl]-2-pyridyl]phenylacetamide (Reference Example D Compound No. 14) ,
   N-[4-[2-ethyl-4-(3-methylphenyl)-1,3-thiazol-5-yl]-2-pyridyl]phenylacetamide (Reference Example D Compound No. 15-2) ,
   N-[4-[4-(3-methylphenyl)-2-propyl-1,3-thiazol-5-yl]-2-pyridyl]phenylacetamide (Reference Example D Compound No. 15-3) ,
   N-[4-[2-butyl-4-(3-methylphenyl)-1,3-thiazol-5-yl]-2-pyridyl]phenylacetamide (Reference Example D Compound No. 15-4),
   N-[4-[4-(3-methylphenyl)-2-(4-methylthiophenyl)-1,3-thiazol-5-yl)-2-pyridyl]phenylacetamide (Reference Example D Compound No. 15-6),
   N-[4-[2-ethyl-4-(3-methylphenyl)-1,3-thiazol-5-yl]-2-pyridyl]benzamide (Reference Example D Compound No. 16-1),
   N-[4-[2-ethyl-4-(3-methylphenyl)-1,3-thiazol-5-yl]-2-pyridyl]-3-phenylpropionamide (Reference Example D Compound No. 16-2),
   N-[4-[2-ethyl-4-(3-methylphenyl)-1,3-thiazol-5-yl]-2-pyridyl]-3-(4-methoxyphenyl)propionamide (Reference Example D Compound No. 16-3),
   N-[4-[2-ethyl-4-(3-methylphenyl)-1,3-thiazol-5-yl]-2-pyridyl]-4-phenylbutyramide (Reference Example D Compound No. 16-5),
   N-[4-[4-(3-methylphenyl)-2-propyl-1,3-thiazol-5-yl]-2-pyridyl]benzamide (Reference Example D Compound No. 16-7),
   N-[4-[4-(3-methylphenyl)-2-propyl-1,3-thiazol-5-yl]-2-pyridyl]-3-phenylpropionamide (Reference Example D Compound No. 16-8) ,
   N-[4-[2-butyl-4-(3-methylphenyl)-1,3-thiazol-5-yl]-2-pyridyl]benzamide (Reference Example D Compound No. 16-9),
   N-[4-[2-butyl-4-(3-methylphenyl)-1,3-thiazol-5-yl]-2-pyridyl]-3-phenylpropionamide (Reference Example D Compound No. 16-10),
   N-[4-[2-(4-fluorophenyl)-4-(3-methylphenyl)-1,3-thiazol-5-yl]-2-pyridyl]benzamide (Reference Example D Compound No. 16-11),
   N-[4-[2-(4-fluorophenyl)-4-(3-methylphenyl)-1,3-thiazol-5-yl]-2-pyridyl]-3-phenylpropionamide (Reference Example D Compound No. 16-12),
   N-[4-[4-(3-methylphenyl)-2-(4-methylthiophenyl)-1,3-thiazol-5-yl]-2-pyridyl]benzamide (Reference Example D Compound No. 16-15) ,
   N-[4-(4-(3-methylphenyl)-2-(4-methylthiophenyl)-1,3-thiazol-5-yl]-2-pyridyl]-3-phenylpropionamide (Reference Example D Compound No. 16-16),
   N-benzyl-N-[4-[2-ethyl-4-(3-methylphenyl)-1,3-thiazol-5-yl]-2-pyridyl]amine (Reference Example D Compound No. 19-2),
   N-[4-[2-ethyl-4-(3-methylphenyl)-1,3-thiazol-5-yl]-2-pyridyl]-N-(2-phenylethyl)amine (Reference Example D Compound No. 19-3),
   N-[4-[2-ethyl-4-(3-methylphenyl)-1,3-thiazol-5-yl]-2-pyridyl]-N-(3-phenylpropyl)amine (Reference Example D Compound No. 19-4),
   N-benzyl-N-[4-[4-(3-methylphenyl)-2-propyl-1,3-thiazol-5-yl]-2-pyridyl]amine (Reference Example D Compound No. 19-5),
   N-[4-[4-(3-methylphenyl)-2-propyl-1,3-thiazol-5-yl]-2-pyridyl]-N-(2-phenylethyl)amine (Reference Example D Compound No. 19-6),
   N-[4-[4-(3-methylphenyl)-2-propyl-1,3-thiazol-5-yl]-2-pyridyl]-N-(3-phenylpropyl)amine (Reference Example D Compound No. 19-7),
   N-benzyl-N-[4-[2-butyl-4-(3-methylphenyl)-1,3-thiazol-5-yl]-2-pyridyl]amine (Reference Example D Compound No. 19-8),
   N-[4-[2-butyl-4-(3-methylphenyl)-1,3-thiazol-5-yl]-2-pyridyl]-N-(2-phenylethyl)amine (Reference Example D Compound No. 19-9),
   N-[4-[2-butyl-4-(3-methylphenyl)-1,3-thiazol-5-yl]-2-pyridyl]-N-(3-phenylpropyl)amine (Reference Example D Compound No. 19-10) ,
   N-benzyl-N-[4-[4-(3-methylphenyl)-2-(4-methylthiophenyl)-1,3-thiazol-5-yl]-2-pyridyl]amine (Reference Example D Compound No. 19-17),
   N-[4-[4-(3-methylphenyl)-2-(4-methylthiophenyl)-1,3-thiazol-5-yl]-2-pyridyl]-N-(2-phenylethyl)amine (Reference Example D Compound No. 19-18),
   N-[4-[4-(3-methylphenyl)-2-(4-methylthiophenyl)-1,3-thiazol-5-yl]-2-pyridyl]-N-(3-phenylpropyl)amine (Reference Example D Compound No. 19-19),
   N-[4-[4-(3-methylphenyl)-2-(4-methylsulfonylphenyl)-1,3-thiazol-5-yl]-2-pyridyl]benzamide (Reference Example D Compound No. 20),
   N-[4-[4-(3-methylphenyl)-2-(4-methylsulfonylphenyl)-1,3-thiazol-5-yl]-2-pyridyl]phenylacetamide (Reference Example D Compound No. 21-1),
   N-[4-[4-(3-methylphenyl)-2-(4-methylsulfonylphenyl)-1,3-thiazol-5-yl]-2-pyridyl]-3-phenylpropionamide (Reference Example D Compound No. 21-2),
   N-benzyl-N-[4-[4-(3-methylphenyl)-2-(4-methylsulfonylphenyl)-1,3-thiazol-5-yl]-2-pyridyl]amine (Reference Example D Compound No. 21-5),
   N-[4-[4-(3-methylphenyl)-2-(4-methylsulfonylphenyl)-1,3-thiazol-5-yl]-2-pyridyl]-N-(3-phenylpropyl)amine (Reference Example D Compound No. 21-6),
   N-[4-[4-(3-methylphenyl)-2-(4-methylsulfonylphenyl)-1,3-thiazol-5-yl]-2-pyridyl]-N-(2-phenylethyl)amine (Reference Example D Compound No. 25-1),
   N-(4-fluorobenzyl)-N-[4-[4-(3-methylphenyl)-2-(4-methylsulfonylphenyl)-1,3-thiazol-5-yl]-2-pyridyl]amine (Reference Example D Compound No. 25-2).

As a salt of Compound (II), for example, there are a metal salt, ammonium salt, a salt with an organic base, a salt with an inorganic acid, a salt with an organic acid, a salt with basic or acidic amino acid and the like. As a suitable metal salt, there are alkali metal salts such as sodium salt, potassium salt and the like; alkaline earth metal salts such as calcium salt, magnesium salt, barium salt and the like; aluminum salt and the like. As a suitable example of a salt with an organic base, for example, there are salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like. As a suitable example of a salt with an inorganic acid, for example, there are salts with hydrochloric acid, hydrobromic acid, nitric acid, sulfuric acid, phosphoric acid and the like. As a suitable example of a salt with an organic acid, for example, there are salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like. As a suitable example of a salt with a basic amino acid, for example, there are salts with arginine, lysine, ornithine and the like. As a suitable example of a salt with an acidic amino acid, for example, there are salts with aspartic acid, glutamic acid and the like.

Among them, pharmaceutically acceptable salts are preferable. For example, when a compound has an acidic functional group therein, there are inorganic salts such as alkali metal salts (for example, sodium salt, potassium salt and the like), alkaline earth metal salts (for example, calcium salt, magnesium salt, barium salt and the like), ammonium salts and the like and, when a compound has a basic functional group therein, there are salts with inorganic acids such as hydrochloric acid, hydrobromic acid, nitric acid, sulfuric acid, phosphoric acid and the like, and salts with organic acids such as acetic acid, phthalic acid, fumaric acid, oxalic acid, tartaric acid, maleic acid, citric acid, succinic acid, methanesulfonic acid, p-toluenesulfonic acid and the like.

The compound (II) and a salt thereof can be produced according to the method described in WO00/64894.

In the above-mentioned formulas, R^{1a}, R^{2a} , R^{3a}, X^{a}, Y^{a} and Z^{a} are each correspond to R¹, R², R³, X, Y and Z, described in WO00/64894.

### [Compound (III)]

A compound represented by the formula 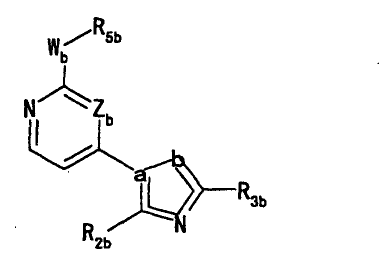 wherein
- a: is N or C;
- b: is CH when a is N, or O when a is C;
- =: denotes a single or a double bond dependent upon whether the azole ring is an imidazole or an oxazole ring;
- Z_{b}: is N or CH;
- W_{b}: is -NR_{6b}-Y_{b}-, -O- or -S-,
where R_{6b} is a hydrogen atom, C₁₋₄ alkyl group, C₃₋₈ cycloalkyl group, C₃₋₈ cycloalkyl-C₁₋₃ alkyl group, C₆₋₁₈ aryl group, C₃₋₁₈ heteroaryl group, C₇₋₁₉ aralkyl group or C₄₋₁₉ heteroaralkyl group, and -Y_{b}- is C₁₋₄ alkylene group or a bond;
- R_{2b}: is phenyl group, optionally substituted by one or more substituents selected from a halogen atom,
trifluoromethyl, cyano, amido, thioamido, carboxylate, thiocarboxylate, C₁₋₄ alkoxy, C₁₋₄ alkyl, amino, and mono- or di-C₁₋₄ alkylamino;
- R_{3b}: is a hydrogen atom, a halogen atom, C₁₋₁₀ alkyl group, C₂₋₄ alkenyl group, C₃₋₁₀ cycloalkyl group, C₃₋₁₈ heterocycloalkyl group, C₆₋₁₈ aryl group, C₃₋₁₈ heteroaryl group or -CH=N-NH-C(NH)NH₂ (wherein C₁₋₁₀ alkyl group, C₂₋₄ alkenyl group, C₃₋₁₀ cycloalkyl group, C₃₋₁₈ heterocycloalkyl group, C₆₋₁₈ aryl group, C₃₋₁₈ heteroaryl group and -CH=N-NH-C(NH)NH₂ are each optionally substituted by 1 to 4 substituents selected from C₁₋₄ alkyl optionally substituted by hydroxy, halogen atom, halo-substituted-C₁₋₄ alkyl, hydroxy, C₁₋₄ alkoxy, C₁₋₄ alkylthio, carboxy, carbonyl optionally substituted by C₁₋₆ alkyl or C₁₋₆ alkoxy, amino, mono- or di-C₁₋₄ alkylamino and 5 to 7 membered N-heterocyclic group optionally further containing heteroatom(s));
- R_{5b}: is C₆₋₁₈ aryl group, C₃₋₁₈ heteroaryl group or C₃₋₁₂ cycloalkyl group each of which is optionally substituted by 1 to 4 substituents selected from C₁₋₄ alkyl, halogen, halo-substitued-C₁₋₄ alkyl, hydroxy, C₁₋₄ alkoxy, C₁₋₄ alkylthio, amino, mono- or di-C₁₋₄ alkylamino and 5 to 7 membered N-heterocyclic group optionally further containing heteroatom(s), or a salt thereof.

The compound (III) and a salt thereof can be produced according to WO00/63204, and specifically, the compounds produced in Examples can be used.

In the above-mentioned formulas, R_{2b}, R_{3b}, R_{5b}, R_{6b}, Z_{b} and W_{b} respectively correspond to R₂, R₃, R₅, R₆, Z and W described in WO00/63204, pages 1-2.

### [compound (IV), (V) and (VI)]

[1] A 1,3-thiazole compound (IV) of which 5-position is substituted with a 4-pyridyl group having a substituent including no aromatic group, or a salt thereof;
[2] A compound as defined in [1] which is a compound represented by the formula: 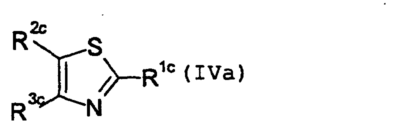 wherein R^{1c} represents a hydrogen atom, a hydrocarbon group optionally having a substituent, a heterocyclic group optionally having a substituent, an amino group optionally having a substituent or an acyl group, R^{2c} represents a 4-pyridyl group having a substituent including no aromatic group, and R^{3c} represents an aromatic group optionally having a substituent, or a salt thereof;
[3] A 1,3-thiazole compound (V) of which 5-position is substituted with a pyridyl group having a substituent including no aromatic group, at a position adjacent to a nitrogen atom of the pyridyl group, or a salt thereof;
[4] A compound represented by the formula: 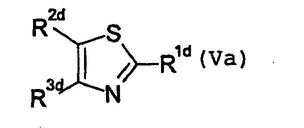 wherein R^{1d} represents a hydrogen atom, a hydrocarbon group optionally having a substituent, a heterocyclic group optionally having a substituent, an amino group optionally having a substituent or an acyl group, R^{2d} represents a pyridyl group having a substituent including no aromatic group, at a position adjacent to a nitrogen atom of the pyridyl group, and R^{3d} represents an aromatic group optionally having a substituent, or a salt thereof;
[5] A 1,3-thiazole compound (VI) of which 5-position is substituted with a 4-pyridyl group having a substituent including no aromatic group, at a position adjacent to a nitrogen atom of the 4-pyridyl group, or a salt thereof;
[6] A compound as defined in any one of [1] to [5] wherein the substituent including no aromatic group is a halogen atom, C₁₋₃ alkylenedioxy, nitro, cyano, C₁₋₆ alkyl which may be halogenated, C₂₋₆ alkenyl which may be halogenated, carboxy C₂₋₆ alkenyl, C₂₋₆ alkynyl which may be halogenated, C₃₋₈ cycloalkyl which may be halogenated, C₃₋₈ cycloalkyl-C₁₋₆ alkyl, C₁₋₈ alkoxy which may be halogenated, C₁₋₆ alkoxy-carbonyl-C₁₋₆ alkoxy, hydroxy, mercapto, C₁₋₆ alkylthio which may be halogenated, amino, mono-C₁₋₆ alkylamino, di-C₁₋₆ alkylamino, C₃₋₈ cycloalkylamino, C₃₋₈ cycloalkyl-C₁₋₆ alkylamino, N-C₃₋₈ cycloalkyl-N-C₁₋₆ alkylamino, formyl, carboxy, carboxy-C₂₋₆ alkenyl, carboxy-C₁₋₆ alkyl, C₁₋₆ alkyl-carbonyl which may be halogenated, C₃₋₈ cycloalkyl-carbonyl optionally substituted by C₁₋₆ alkyl, C₁₋₆ alkoxy-carbonyl, carbamoyl, thiocarbamoyl, mono-C₁₋₆ alkyl-carbamoyl, di-C₁₋₆ alkyl-carbamoyl, C₁₋₆ alkylsulfonyl, C₁₋₆ alkylsulfinyl, formylamino, C₁₋₆ alkyl-carbonylamino, C₃₋₈ cycloalkyl-carbonylamino which may be substituted by C₁₋₆ alkyl, C₁₋₆ alkoxy-carbonylamino, C₁₋₆ alkylsulfonylamino, C₁₋₆ alkyl-carbonyloxy, C₁₋₆ alkoxy-carbonyloxy, mono-C₁₋₆ alkyl-carbamoyloxy, di-C₁₋₆ alkyl-carbamoyloxy, 5- to 7-membered aliphatic heterocyclic group containing 1 to 4 of 1 or 2 kinds of hetero atoms selected from a nitrogen atom, a sulfur atom and an oxygen atom in addition to carbon atoms (this aliphatic heterocyclic group optionally has a substituent selected from C₁₋₆ alkyl, C₁₋₆ alkyl-carbonyl and oxo), sulfo, sulfamoyl, sulfinamoyl, sulfenamoyl or a group obtained by connecting 2 to 3 of these substituents
   (e.g. , (i) C₁₋₆ alkyl, (ii) amino, (iii) C₁₋₆ alkylamino, (iv) C₃₋₈ cycloalkylamino, (v) 5- to 7-membered aliphatic heterocyclic amino containing 1 to 4 of 1 or 2 kinds of hetero atoms selected from a nitrogen atom, a sulfur atom and an oxygen atom in addition to carbon atoms, (vi) C₁₋₆ alkyl-carbonyl amino, (vii) C₃₋₈ cycloalkyl-carbonylamino or (viii) 5- to 7-membered aliphatic heterocyclic-carbonyl amino containing 1 to 4 of 1 or 2 kinds of hetero atoms selected from a nitrogen atom, a sulfur atom and an oxygen atom in addition to carbon atoms, which is substituted, respectively, by a substituent selected from the group consisting of a halogen atom, cyano, hydroxy, C₁₋₆ alkoxy, C₁₋₆ alkylthio, C₁₋₆ alkylsulfinyl, C₁₋₆ alkylsulfonyl, C₃₋₈ cycloalkyl, 5- to 7-membered aliphatic heterocyclic group containing 1 to 4 of 1 or 2 kinds of hetero atoms selected from a nitrogen atom, a sulfur atom and an oxygen atom in addition to carbon atoms, C₁₋₆ alkyl-carbonyl, 5- to 7-membered aliphatic heterocyclic-carbonyl containing 1 to 4 of 1 or 2 kinds of hetero atoms selected from a nitrogen atom, a sulfur atom and an oxygen atom in addition to carbon atoms, C₃₋₈ cycloalkoxy, 5- to 7-membered aliphatic heterocyclic-oxy containing 1 to 4 of 1 or 2 kinds of hetero atoms selected from a nitrogen atom, a sulfur atom and an oxygen atom in addition to carbon atoms, C₁₋₆ alkylamino, C₁₋₆ alkoxy-carbonyl, C₃₋₈ cycloalkoxy-carbonyl, 5- to 7-membered aliphatic heterocyclic-oxycarbonyl containing 1 to 4 of 1 or 2 kinds of hetero atoms selected from a nitrogen atom, a sulfur atom and an oxygen atom in addition to carbon atoms, etc.);
[7] A compound as defined in [2] or [4] wherein
   (1) the hydrocarbon group optionally having a substituent is a C₁₋₆ alkyl group, a C₂₋₆ alkenyl group, a C₂₋₆ alkynyl group, a C₃₋₈ cycloalkyl group, a C₆₋₁₄ aryl group or a C₇₋₁₆ aralkyl group, optionally having a substituent selected from Group A of substituents consisting of oxo, a halogen atom, C₁₋₃ alkylenedioxy, nitro, cyano, C₁₋₆ alkyl which may be halogenated, C₂₋₆ alkenyl which may be halogenated, carboxy C₂₋₆ alkenyl, C₂₋₆ alkynyl which may be halogenated, C₃₋₈ cycloalkyl which may be halogenated, C₃₋₈ cycloalkyl-C₁₋₆ alkyl, C₆₋₁₄ aryl, C₁₋₈ alkoxy which may be halogenated, C₁₋₆ alkoxy-carbonyl-C₁₋₆ alkoxy, hydroxy, C₆₋₁₄ aryloxy, C₇₋₁₆ aralkyloxy, mercapto, C₁₋₆ alkylthio which may be halogenated, C₆₋₁₄ arylthio, C₇₋₁₆ aralkylthio, amino, mono-C₁₋₆ alkylamino, mono-C₆₋₁₄ arylamino, di-C₁₋₆ alkylamino, C₃₋₈ cycloalkylamino, di-C₆-₁₄ arylamino, C₃₋₈ cycloalkyl-C₁₋₆ alkylamino, N-C₃₋₈ cycloalkyl-N-C₁₋₆ alkylamino, formyl, carboxy, C₁₋₆ alkyl-carbonyl which may be halogenated, C₃₋₈ cycloalkyl-carbonyl optionally substituted by C₁₋₆ alkyl, C₁₋₆ alkoxy-carbonyl, C₆₋ ₁₄ aryl-carbonyl, C₇₋₁₆ aralkyl-carbonyl, C₆₋₁₄ aryloxy-carbonyl, C₇₋₁₆ aralkyloxy-carbonyl, 5- to 7-membered heterocyclic carbonyl containing 1 to 4 of 1 or 2 kinds of hetero atoms selected from a nitrogen atom, a sulfur atom and an oxygen atom in addition to carbon atoms, carbamoyl, thiocarbamoyl, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- to 7-membered heterocyclic carbamoyl containing 1 to 4 of 1 or 2 kinds of hetero atoms selected from a nitrogen atom, a sulfur atom and an oxygen atom in addition to carbon atoms, C₁₋₆ alkylsulfonyl, C₆₋₁₄ arylsulfonyl, C₁₋₆ alkylsulfinyl, C₆₋₁₄ arylsulfinyl, formylamino, C₁₋₆ alkyl-carbonylamino, C₃₋₈ cycloalkyl-carbonylamino optionally substituted by C₁₋₆ alkyl, C₆₋₁₄ aryl-carbonylamino, C₁₋₆ alkoxy-carbonylamino, C₁₋₆ alkylsulfonylamino, C₆₋₁₄ arylsulfonylamino, C₁₋₆ alkyl-carbonyloxy, C₆₋₁₄ aryl-carbonyloxy, C₁₋₆ alkoxy-carbonyloxy, mono-C₁₋₆ alkyl-carbamoyloxy, di-C₁₋₆ alkyl-carbamoyloxy, mono-C₆₋₁₄ aryl-carbamoyloxy, di-C₆₋₁₄ aryl-carbamoyloxy, nicotinoyloxy, isonicotinoyloxy, 5- to 10-membered heterocyclic group containing 1 to 4 of 1 or 2 kinds of hetero atoms selected from a nitrogen atom, a sulfur atom and an oxygen atom in addition to carbon atoms (this heterocyclic group optionally has a substituent selected from C₁₋₆ alkyl, C₆₋₁₄ aryl, C₁₋₆ alkyl-carbonyl which may be halogenated, 5- to 10-membered aromatic heterocyclic group containing 1 to 4 of 1 or 2 kinds of hetero atoms selected from a nitrogen atom, a sulfur atom and an oxygen atom in addition to carbon atoms and oxo), sulfo, sulfamoyl, sulfinamoyl, sulfenamoyl and a group formed by connecting 2 to 3 of these substituents (e.g., (i) C₁₋₆ alkyl, (ii) C₆₋₁₄ aryl, (iii) amino, (iv) C₁₋₆ alkylamino, (v) C₃₋₈ cycloalkylamino, (vi) C₆₋₁₄ arylamino, (vii) 5- to 7-membered heterocyclic amino containing 1 to 4 of 1 or 2 kinds of hetero atoms selected from a nitrogen atom, a sulfur atom and an oxygen atom in addition to carbon atoms, (viii) C₁₋₆ alkyl-carbonylamino, (ix) C₃₋₈ cycloalkyl-carbonylamino, (x) C₆₋₁₄ aryl-carbonyl amino or (xi) 5- to 7-membered heterocyclic-carbonyl amino containing 1 to 4 of 1 or 2 kinds of hetero atoms selected from a nitrogen atom, a sulfur atom and an oxygen atom in addition to carbon atoms, which is substituted, respectively, by a substituent selected from the group consisting of a halogen atom, cyano, hydroxy, C₁₋₆ alkoxy, C₆₋₁₄ aryloxy, C₁₋₆ alkylthio, C₆₋₁₄ arylthio, C₁₋₆ alkylsulfinyl, C₆₋₁₄ arylsulfinyl, C₁₋₆ alkylsulfonyl, C₆₋₁₄ arylsulfonyl, C₃₋₈ cycloalkyl, 5- to 7-membered heterocyclic group containing 1 to 4 of 1 or 2 kinds of hetero atoms selected from a nitrogen atom, a sulfur atom and an oxygen atom in addition to carbon atoms, C₆₋₁₄ aryl, C₁₋₆ alkyl-carbonyl, 5- to 7-membered heterocyclic-carbonyl containing 1 to 4 of 1 or 2 kinds of hetero atoms selected from a nitrogen atom, a sulfur atom and an oxygen atom in addition to carbon atoms, C₆₋₁₄ aryl-carbonyl, C₃₋₈ cycloalkoxy, 5- to 7-membered heterocyclic-oxy containing 1 to 4 of 1 or 2 kinds of hetero atoms selected from a nitrogen atom, a sulfur atom and an oxygen atom in addition to carbon atoms, C₁₋₆ alkylamino, C₆₋₁₄ arylamino, C₁₋₆ alkoxy-carbonyl, 5- to 7-membered heterocyclic-oxycarbonyl containing 1 to 4 of 1 or 2 kinds of hetero atoms selected from a nitrogen atom, a sulfur atom and an oxygen atom in addition to carbon atoms, C₆₋₁₄ aryloxycarbonyl, etc.)
   (2) the heterocyclic group optionally having a substituent is a 5- to 14-membered heterocyclic group containing 1 to 4 of 1 or 2 kinds of hetero atoms selected from a nitrogen atom, a sulfur atom and an oxygen atom in addition to carbon atoms, which optionally has a substituent selected from Group A of substituents,
   (3) the acyl group is an acyl group of the formula: - (C=O)-R^{5c}, -(C=O)-OR^{5c}, -(C=O)-NR^{5c}R^{6c}, -(C=S)-NHR^{5c}, -(C=O)-N(OR^{5c})R^{6c}, -(C=S)-NHOR^{5c} or -SO₂-R^{7c} (wherein R^{5c} represents (1) a hydrogen atom, (2) a C₁₋₆ alkyl group, a C₂₋₆ alkenyl group, a C₂₋₆ alkynyl group, a C₃₋₈ cycloalkyl group, a C₆₋₁₄ aryl group or a C₇₋₁₆ aralkyl group, optionally having a substituent selected from Group A of substituents, or (3) a 5- to 14-membered heterocyclic group containing 1 to 4 of 1 or 2 kinds of hetero atoms selected from a nitrogen atom, a sulfur atom and an oxygen atom in addition to carbon atoms, which optionally has a substituent selected from Group A of substituents, R^{6c} represents a hydrogen atom or a C₁₋₆ alkyl group, and R^{7c} represents (1) a C₁₋₆ alkyl group, a C₂₋₆ alkenyl group, a C₂₋₆ alkynyl group, a C₃₋₈ cycloalkyl group, a C₆₋₁₄ aryl group or a C₇₋₁₆ aralkyl group, optionally having a substituent selected from Group A of substituents, or (2) a 5- to 14-membered heterocyclic group containing 1 to 4 of 1 or 2 kinds of hetero atoms selected from a nitrogen atom, a sulfur atom and an oxygen atom in addition to carbon atoms, which optionally has a substituent selected from Group A of substituents),
   (4) the amino group optionally having a substituent is
      (i) an amino group optionally having 1 or 2 substituents selected from the group consisting of (1) a C₁₋₆ alkyl group, a C₂₋₆ alkenyl group, a C₂₋₆ alkynyl group, a C₃₋₈ cycloalkyl group, a C₆₋₁₄ aryl group and a C₇₋₁₆ aralkyl group, optionally having a substituent selected from Group A of substituents, (2) a 5- to 14-membered heterocyclic group containing 1 to 4 of 1 or 2 kinds of hetero atoms selected from a nitrogen atom, a sulfur atom and an oxygen atom in addition to carbon atoms, which optionally have a substituent selected from Group A of substituents, (3) an acyl group of the formula: -(C=O)-R^{5c}, -(C=O)-OR^{5c}, -(C=O)-NR^{5c}R^{6c}, -(C=S)-NHR^{5c}, -(C=O)-N(OR^{5c})R^{6c}, -(C=S)-NHOR^{5c} or -SO₂-R^{7c} (wherein each symbol is as defined above), and (4)a C₁₋₆ alkylidene group optionally having a substituent selected from Group A of substituents, or
      (ii) a 5- to 7-membered non-aromatic cyclic amino group optionally containing 1 to 4 of 1 or 2 kinds of hetero atoms selected from a nitrogen atom, a sulfur atom and an oxygen atom in addition to one nitrogen atom and carbon atoms, which optionally has a substituent selected from the group consisting of C₁₋₆ alkyl, C₆₋₁₄ aryl, C₁₋₆ alkyl-carbonyl which may be halogenated, C₁₋₆ alkoxy-carbonyl, 5- to 10-membered aromatic heterocyclic group containing 1 to 4 of 1 or 2 kinds of hetero atoms selected from a nitrogen atom, a sulfur atom and an oxygen atom in addition to carbon atoms, and oxo,
   (5) the substituent containing no aromatic group is a halogen atom, C₁₋₃ alkylenedioxy, nitro, cyano, C₁₋₆ alkyl which may be halogenated, C₂₋₆ alkenyl which may be halogenated, carboxy C₂₋₆ alkenyl, C₂₋₆ alkynyl which may be halogenated, C₃₋₈ cycloalkyl which may be halogenated, C₃₋₈ cycloalkyl-C₁₋₆ alkyl, C₁₋₈ alkoxy which may be halogenated, C₁₋₆ alkoxy-carbonyl-C₁₋₆ alkoxy, hydroxy, mercapto, C₁₋₆ alkylthio which may be halogenated, amino, mono-C₁₋₆ alkylamino, di-C₁₋₆ alkylamino, C₃₋₈ cycloalkylamino, C₃₋₈ cycloalkyl-C₁₋₆ alkylamino, N-C₃₋₈ cycloalkyl-N-C₁₋₆ alkylamino, formyl, carboxy, carboxy-C₂₋₆ alkenyl, carboxy-C₁₋₆ alkyl, C₁₋₆ alkyl-carbonyl which may be halogenated, C₃₋₈ cycloalkyl-carbonyl optionally substituted by C₁₋₆ alkyl, C₁₋₆ alkoxy-carbonyl, carbamoyl, thiocarbamoyl, mono-C₁₋₆ alkyl-carbamoyl, di-C₁₋₆ alkyl-carbamoyl, C₁₋₆ alkylsulfonyl, C₁₋₆ alkylsulfinyl, formylamino, C₁₋₆ alkyl-carbonylamino, C₃₋₈ cycloalkyl-carbonylamino which may be substituted by C₁₋₆ alkyl, C₁₋₆ alkoxy-carbonylamino, C₁₋₆ alkylsulfonylamino, C₁₋₆ alkyl-carbonyloxy, C₁₋₆ alkoxy-carbonyloxy, mono-C₁₋₆ alkylcarbamoyloxy, di-C₁₋₆ alkyl-carbamoyloxy, 5- to 7-membered aliphatic heterocyclic group containing 1 to 4 of 1 or 2 kinds of hetero atoms selected from a nitrogen atom, a sulfur atom and an oxygen atom in addition to carbon atoms (this aliphatic heterocyclic group optionally has a substituent selected from C₁₋₆ alkyl, C₁₋₆ alkyl-carbonyl and oxo), sulfo, sulfamoyl, sulfinamoyl, sulfenamoyl or a group obtained by connecting 2 to 3 of these substituents (e.g., (i) C₁₋₆ alkyl, (ii) amino, (iii) C₁₋₆ alkylamino, (iv) C₃₋₈ cycloalkylamino, (v) 5- to 7-membered aliphatic heterocyclic amino containing 1 to 4 of 1 or 2 kinds of hetero atoms selected from a nitrogen atom, a sulfur atom and an oxygen atom in addition to carbon atoms, (vi) C₁₋₆ alkyl-carbonyl amino, (vii) C₃₋₈ cycloalkyl-carbonylamino or (viii) 5- to 7-membered aliphatic heterocyclic-carbonyl amino containing 1 to 4 of 1 or 2 kinds of hetero atoms selected from.a nitrogen atom, a sulfur atom and an oxygen atom in addition to carbon atoms, which is substituted, respectively, by a substituent selected from the group consisting of a halogen atom, cyano, hydroxy, C₁₋₆ alkoxy, C₁₋₆ alkylthio, C₁₋₆ alkylsulfinyl, C₁₋₆ alkylsulfonyl, C₃₋₈ cycloalkyl, 5- to 7-membered aliphatic heterocyclic group containing 1 to 4 of 1 or 2 kinds of hetero atoms selected from a nitrogen atom, a sulfur atom and an oxygen atom in addition to carbon atoms, C₁₋₆ alkyl-carbonyl, 5- to 7-membered aliphatic heterocyclic-carbonyl containing 1 to 4 of 1 or 2 kinds of hetero atoms selected from a nitrogen atom, a sulfur atom and an oxygen atom in addition to carbon atoms, C₃₋₈ cycloalkoxy, 5- to 7-membered aliphatic heterocyclic-oxy containing 1 to 4 of 1 or 2 kinds of hetero atoms selected from a nitrogen atom, a sulfur atom and an oxygen atom in addition to carbon atoms, C₁₋₆ alkylamino, C₁₋₆ alkoxy-carbonyl, C₃₋₈ cycloalkoxy-carbonyl, 5-to 7-membered aliphatic heterocyclic-oxycarbonyl containing 1 to 4 of 1 or 2 kinds of hetero atoms selected from a nitrogen atom, a sulfur atom and an oxygen atom in addition to carbon atoms, etc.),
   (6) the aromatic group optionally having a substituent is 1) a C₆₋₁₄ mono-cyclic or fused poly-cyclic aromatic hydrocarbon group optionally having a substituent selected from Group A of substituents, or 2) a 5- to 14-membered aromatic heterocyclic group containing 1 to 4 of 1 or 2 kinds of hetero atoms selected from a nitrogen atom, a sulfur atom and an oxygen atom in addition to carbon atoms;
[8] A compound as defined in [2] or [4] wherein R^{1c} represents (i) a hydrogen atom, (ii) a C₁₋₆ alkyl group optionally substituted by a substituent selected from the group consisting of a halogen atom, C₁₋₆ alkoxy-carbonyl, carboxy, cyano, C₁₋₆ alkylthio, C₁₋₆ alkylsulfinyl, C₁₋₆ alkylsulfonyl, hydroxy, C₁₋₆ alkoxy and C₁₋₆ alkyl-carbonyl, (iii) a C₆₋₁₄ aryl group optionally having a substituent selected from the group consisting of a halogen atom and a group of the formula: -S(O)ₙ-R^{1f} (wherein R^{1f} represents a C₁₋₆ alkyl group, and n represents an integer of 0 to 2), (iv) a C₇₋₁₅ aralkyl group, (v) an amino group optionally having one or two substituents selected from 1)C₁₋₆ alkyl, 2)C₁₋₆ alkyl-carbonyl, 3) 5- to 7-membered heterocyclic-carbonyl containing 1 to 4 hetero atoms selected from the group consisting of a nitrogen atom, an oxygen atom and a sulfur atom, in addition to carbon atoms, optionally substituted with a halogen atom, C₁₋₆ alkyl or C₁₋₆ alkoxy, 4) C₆₋₁₄ aryl-carbamoyl, 5) C₁₋₆ alkyl-carbamoyl which may be halogenated, 6) C₁₋₆ alkoxy-carbonyl which may be halogenated, 7) C₁₋₆ alkoxy-carbamoyl and 8) C₆₋₁₄ aryloxy-carbamoyl, (vi) a 5- to 10-membered heterocyclic group containing 1 to 4 of 1 or 2 kinds of hetero atoms selected from a nitrogen atom, a sulfur atom and an oxygen atom in addition to carbon atoms, optionally substituted by oxo, C₁₋₆ alkyl, C₆₋₁₄ aryl or C₁₋₆ alkoxy-carbonyl, (vii) an acyl group represented by the formula:-(C=O)-R^{5d}' (wherein R^{5d}' represents a hydrogen atom, a C₁₋₆ alkyl group which may be halogenated or a C₆₋₁₄ aryl group which may be halogenated), or (viii) an acyl group represented by the formula:-(C=O)-OR^{5d}" (wherein R^{5d}" represents a hydrogen atom or a C₁₋₆ alkyl group);
[9] A compound as defined in [2] or [4] wherein the substituent having no aromatic group is
   (1) a C₁₋₆ alkyl group (this C₁₋₆ alkyl may be substituted by a halogen atom, cyano, hydroxy, C₃₋₈ cycloalkyl or 5- to 7-membered aliphatic heterocyclic group containing 1 to 4 hetero atoms selected from a nitrogen atom, an oxygen atom and a sulfur atom in addition to carbon atoms),
   (2) a halogen atom,
   (3) an amino group optionally having a substituent selected from the group consisting of the following 1) to 7);
      1) a C₁₋₆ alkyl group (this C₁₋₆ alkyl group may be substituted by a halogen atom, cyano, hydroxy, C₃₋₈ cycloalkyl or 5- to 7-membered aliphatic heterocyclic group having 1 to 4 hetero atoms selected from a nitrogen atom, an oxygen atom and a sulfur atom in addition to carbon atoms),
      2) a C₃₋₈ cycloalkyl group,
      3) a C₁₋₆ alkyl-carbonyl group (this C₁₋₆ alkyl-carbonyl group may be substituted by a halogen atom, cyano, hydroxy, C₃₋₈ cycloalkyl or 5- to 7-membered aliphatic heterocyclic group having 1 to 4 hetero atoms selected from a nitrogen atom, an oxygen atom and a sulfur atom in addition to carbon atoms),
      4) a C₁₋₆ alkoxy-carbonyl group,
      5) a C₃₋₈ cycloalkyl-carbonyl group optionally substituted by C₁₋₆ alkyl,
      6) a 5- to 7-membered saturated heterocyclic group having 1 to 4 hetero atoms selected from a nitrogen atom, an oxygen atom and a sulfur atom in addition to carbon atoms (this saturated heterocyclic group may be substituted by C₁₋₆ alkyl or C₁₋₆ alkyl-carbonyl),
      7) a 5- to 7-membered saturated heterocyclic-carbonyl group having 1 to 4 hetero atoms selected from a nitrogen atom, an oxygen atom and a sulfur atom in addition to carbon atoms (this saturated heterocyclic-carbonyl group may be substituted by C₁₋₆ alkyl or C₁₋₆ alkyl-carbonyl)
   (4) a 5- to 7-membered saturated cyclic amino group optionally further containing 1 to 4 hetero atoms selected from a nitrogen atom, an oxygen atom and a sulfur atom in addition to carbon atoms and one nitrogen atom (this saturated cyclic amino group may be substituted by C₁₋₆ alkyl or C₁₋₆ alkyl-carbonyl),
   (5) a hydroxy group, or
   (6) a C₁₋₆ alkyl-carbonyloxy group.
[10] A compound as defined in [2] or [4] wherein R³ is (1) a C₆₋₁₄ aryl group or (2) a 5- to 14-membered aromatic heterocyclic group preferably containing 1 to 4 of 1 or 2 kinds of hetero atoms selected from a nitrogen atom, a sulfur atom and an oxygen atom in addition to carbon atoms, which optionally has substituents selected from the group consisting of C₁₋₆ alkyl which may be halogenated, C₁₋₆ alkoxy, a halogen atom, carboxyl, C₁₋₆ alkoxy-carbonyl, cyano, C₁₋₆ alkylthio and C₁₋₆ alkylsulfonyl;
[11] A compound as defined in [3] which is a compound of the formula: 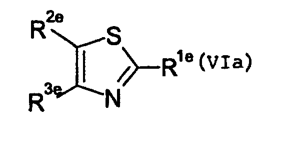 wherein R^{1e} represents (i) a hydrogen atom, (ii) a C₁₋₆ alkyl group optionally substituted by a substituent selected from the group consisting of a halogen atom, C₁₋₆ alkoxy-carbonyl, carboxy, cyano, C₁₋₆ alkylthio, C₁₋₆ alkylsulfinyl, C₁₋₆ alkylsulfonyl, hydroxy, C₁₋₆ alkoxy and C₁₋₆ alkyl-carbonyl, (iii) a C₆₋₁₄ aryl group optionally having a substituent selected from the group consisting of a halogen atom and a group of the formula: -S(O)ₙ-R^{1f} (R^{1f} represents a C₁₋₆ alkyl group, and n represents an integer of 0 to 2), (iv) a C₇₋₁₅ aralkyl group, (v) an amino group optionally having one or two substituents selected from 1) C₁₋₆ alkyl, 2) C₁₋₆ alkyl-carbonyl, 3) C₁₋₆ alkoxy-carbonyl, 4) 5- to 7-membered heterocyclic-carbonyl containing 1 to 4 hetero atoms selected from the group consisting of a nitrogen atom, an oxygen atom and a sulfur atom in addition to carbon atoms, optionally substituted with a halogen atom, C₁₋₆ alkyl or C₁₋₆ alkoxy, 5) C₆₋₁₄ aryl-carbamoyl, 6) C₁₋₆ alkyl-carbamoyl which may be halogenated, 7) C₁₋₆ alkoxy-carbonyl which may be halogenated, 8) C₁₋₆ alkoxy-carbamoyl and 9) C₆₋₁₄ aryloxy-carbamoyl, (vi) a 5- to 10-membered heterocyclic group containing 1 to 4 of 1 or 2 kinds of hetero atoms selected from a nitrogen atom, a sulfur atom and an oxygen atom in addition to carbon atoms, optionally substituted by oxo, C₁₋₆ alkyl, C₆₋₁₄ aryl, C₁₋₆ alkyl-carbonyl or C₁₋₆ alkoxy-carbonyl, (vii) an acyl group represented by the formula: -(C=O)-R^{5d}' (wherein R^{5d}' represents a hydrogen atom, a C₁₋₆ alkyl group which may be halogenated or a C₆₋₁₄ aryl group which may be halogenated), or (viii) an acyl group represented by the formula: -(C=O)-OR^{5d}" (wherein R^{5d}" represents a hydrogen atom or C₁₋₆ alkyl group),
   R^{2e} represents a pyridyl group (preferably 4-pyridyl group which may be N-oxidized) having, at the position adjacent to a nitrogen atom of the pyridyl group, a substituent selected from the group consisting of
   (1) a C₁₋₆ alkyl group (this C₁₋₆ alkyl group may be substituted by a halogen atom, cyano, hydroxy, C₃₋₈ cycloalkyl or a 5- to 7-membered aliphatic heterocyclic group containing hetero atoms selected from a nitrogen atom, an oxygen atom and a sulfur atom in addition to carbon atoms),
   (2) a halogen atom,
   (3) an amino group optionally having a substituent selected from the group consisting of the following 1) to 7);

1) a C₁₋₆ alkyl group (this C₁₋₆ alkyl group may be substituted by a halogen atom, cyano, hydroxy, C₃₋₈ cycloalkyl or a 5- to 7-membered aliphatic heterocyclic group having 1 to 4 hetero atoms selected from a nitrogen atom, an oxygen atom and a sulfur atom in addition to carbon atoms),
2) a C₃₋₈ cycloalkyl group,
3) a C₁₋₆ alkyl-carbonyl group (this C₁₋₆ alkyl-carbonyl group may be substituted by a halogen atom, cyano, hydroxy, C₃₋₈ cycloalkyl or a 5- to 7-membered aliphatic heterocyclic group having 1 to 4 hetero atoms selected from a nitrogen atom, an oxygen atom and a sulfur atom in addition to carbon atoms),
4) a C₁₋₆ alkoxy-carbonyl group,
5) a C₃₋₈ cycloalkyl-carbonyl group optionally substituted by C₁₋₆ alkyl,
6) a 5- to 7-membered aliphatic heterocyclic group having 1 to 4 hetero atoms selected from a nitrogen atom, an oxygen atom and a sulfur atom in addition to carbon atoms (this aliphatic heterocyclic group may be substituted by C₁₋₆ alkyl or C₁₋₆ alkyl-carbonyl),
7) a 5- to 7-membered aliphatic heterocyclic-carbonyl group having 1 to 4 hetero atoms selected from a nitrogen atom, an oxygen atom and a sulfur atom in addition to carbon atoms (this aliphatic heterocyclic-carbonyl group may be substituted by C₁₋₆ alkyl or C₁₋₆ alkyl-carbonyl),
   (4) a 5- to 7-membered saturated cyclic amino group optionally further containing 1 to 4 hetero atoms selected from a nitrogen atom, an oxygen atom and a sulfur atom in addition to carbon atoms and one nitrogen atom (this saturated cyclic amino group may be substituted by C₁₋₆ alkyl or C₁₋₆ alkyl-carbonyl),
   (5) a hydroxy group, and
   (6) a C₁₋₆ alkyl-carbonyloxy group, particularly from the group consisting of (1) to (4) above, and
      R^{3e} represents (1) a C₆₋₁₄ aryl group or (2) a 5- to 14-membered aromatic heterocyclic group containing 1 to 4 of 1 or 2 kinds of hetero atoms selected from a nitrogen atom, a sulfur atom and an oxygen atom in addition to carbon atoms, which optionally has a substituent selected from the group consisting of C₁₋₆ alkyl which may be halogenated, C₁₋₆ alkoxy, a halogen atom, carboxyl, C₁₋₆ alkoxy-carbonyl, cyano, C₁₋₆ alkylthio and C₁₋₆ alkylsulfonyl, or a salt thereof;
[12] A compound as defined in [11] wherein the pyridyl group is a 4-pyridyl group;
[13] A compound as defined in [11] wherein R^{1e} is a C₁₋₆ alkyl group optionally having a substituent selected from the group consisting of a halogen atom, hydroxy, C₁₋₆ alkoxy, C₁₋₆ alkylthio, C₁₋₆ alkylsulfinyl and C₁₋₆ alkylsulfonyl, R^{2e} is a 4-pyridyl group having a C₁₋₆ alkyl-carbonyl-amino group or a C₃₋₈ cycloalkylamino group at the position adjacent to a nitrogen atom of the 4-pyridyl group, R^{3e} is a C₆₋₁₄ aryl group which optionally has a substituent selected from the group consisting of C₁₋₆ alkyl and a halogen atom;
[14] A compound as defined in [11] wherein R^{1e} is a C₁₋₃ alkyl group optionally having a substituent selected from the group consisting of a halogen atom, hydroxy, C₁₋₆ alkoxy, C₁₋₆ alkylthio, C₁₋₆ alkylsulfinyl and C₁₋₆ alkylsulfonyl, R^{2e} is a 4-pyridyl group having a C₁₋₃ alkyl-carbonyl-amino group or a C₃₋₈ cycloalkylamino group at the position adjacent to a nitrogen atom of the 4-pyridyl group, R^{3e} is a phenyl group which optionally has methyl or a chlorine atom;
[15] A compound as defined in [5] which is 5-[2-(tert-butoxycarbonylamino)-4-pyridyl]-2-ethyl-4-(3-methylphenyl)-1,3-thiazole (Reference Example H 3),
   [4-(3-methylphenyl)-5-(2-methyl-4-pyridyl)-1,3-thiazol-2-yl]amine (Reference Example H 7-4),
   2-ethyl-5-(2-fluoro-4-pyridyl)-4-(3-methylphenyl)-1,3-thiazole (Reference Example H 11),
   5-(2-fluoro-4-pyridyl)-4-(3-methylphenyl)-2-[4-(methylthio)phenyl]-1,3-thiazole (Reference Example H 15),
   4-(3-methylphenyl)-5-(2-methyl-4-pyridyl)-2-[4-(methylthio)phenyl]-1,3-thiazole (Reference Example H 16-1),
   4-[2-ethyl-4-(3-methylphenyl)-1,3-thiazol-5-yl]-2-pyridylamine (Reference Example H 22),
   N-[4-[2-ethyl-4-(3-methylphenyl)-1,3-thiazol-5-yl]-2-pyridyl]acetamide (Reference Example H 29-2),
   N-[4-[2-ethyl-4-(3-methylphenyl)-1,3-thiazol-5-yl]-2-pyridyl]propionamide (Reference Example H 29-4),
   N-[4-[4-(3-chlorophenyl)-2-methyl-1,3-thiazol-5-yl]-2-pyridyl]acetamide (Reference Example H 30-1),
   N-[4-[4-(3-chlorophenyl)-2-ethyl-1,3-thiazol-5-yl]-2-pyridyl]acetamide (Reference Example H 30-2),
   N-[4-[4-(3-chlorophenyl)-2-propyl-1,3-thiazol-5-yl]-2-pyridyl]acetamide (Reference Example H 30-3),
   N-[4-[4-(3-chlorophenyl)-2-methyl-1,3-thiazol-5-yl]-2-pyridyl]propionamide (Reference Example H 30-7),
   N-[4-[4-(3-chlorophenyl)-2-ethyl-1,3-thiazol-5-yl]-2-pyridyl]propionamide (Reference Example H 30-8),
   N-[4-[4-(3-chlorophenyl)-2-propyl-1,3-thiazol-5-yl]-2-pyridyl]propionamide (Reference Example H 30-9),
   N-cyclohexyl-4-[2-ethyl-4-(3-methylphenyl)-1,3-thiazol-5-yl]-2-pyridylamine (Reference Example H 36-4),
   N-cyclohexyl-4-[4-(3-methylphenyl)-2-(4-methylsulfonylphenyl)-1,3-thiazol-5-yl]-2-pyridylamine (Reference Example H 36-5),
   N-cyclopentyl-4-[2-ethyl-4-(3-methylphenyl)-1,3-thiazol-5-yl]-2-pyridylamine (Reference Example H 36-6),
   N-cyclopentyl-4-[4-(3-methylphenyl)-2-(4-methylsulfonylphenyl)-1,3-thiazol-5-yl]-2-pyridylamine (Reference Example H 36-7),
   4-[4-(3-chlorophenyl)-2-ethyl-1,3-thiazol-5-yl]-N-cyclohexyl-2-pyridylamine (Reference Example H 36-10),
   4-[4-(3-chlorophenyl)-2-ethyl-1,3-thiazol-5-yl]-N-cyclopentyl-2-pyridylamine (Reference Example H 36-11),
   N-[4-(3-methylphenyl)-5-(2-methyl-4-pyridyl)-1,3-thiazol-2-yl]acetamide (Reference Example H 39),
   N-[4-(3,5-dimethylphenyl)-5-(2-methyl-4-pyridyl)-1,3-thiazol-2-yl]nicotinamide (Reference Example H 42-1),
   6-chloro-N-[4-(3,5-dimethylphenyl)-5-(2-methyl-4-pyridyl)-1,3-thiazol-2-yl]nicotinamide (Reference Example H 44-3) ,
   N-[4-(3,5-dimethylphenyl)-5-(2-methyl-4-pyridyl)-1,3-thiazol-2-yl]-6-methylnicotinamide (Reference Example H 46-3),
   N-[4-(3,5-dimethylphenyl)-5-(2-methyl-4-pyridyl)-1,3-thiazol-2-yl]-6-methoxynicotinamide (Reference Example H 48-3),
   4-(3-methylphenyl)-5-(2-methyl-4-pyridyl)-2-(4-methylsulfinylphenyl)-1,3-thiazole (Reference Example H 54),
   4-(3-methylphenyl)-5-(2-methyl-4-pyridyl)-2-(4-methylsulfonylphenyl)-1,3-thiazole (Reference Example H 57),
   5-(2-fluoro-4-pyridyl)-4-(3-methylphenyl)-2-(4-methylsulfonylphenyl)-1,3-thiazole (Reference Example H 58-4),
   N-[4-[4-(3-chlorophenyl)-2-(4-methylsulfonylphenyl)-1,3-thiazol-5-yl)-2-pyridyl]acetamide (Reference Example H 58-6) ,
   N-[4-[4-(3-chlorophenyl)-2-(4-methylsulfonylphenyl)-1,3-thiazol-5-yl]-2-pyridyl]propionamide (Reference Example H 58-7),
   N-[4-[4-(3-chlorophenyl)-2-(4-methylsulfonylphenyl)-1,3-thiazol-5-yl]-2-pyridyl]pivalamide (Reference Example H 58-8),
   or a salt thereof;
[16] A pro-drug of a compound as claimed in any one as defined in [1] to [5];

In this specification, as the "acyl group", for example, a acyl group represented by the formula: -(C=O)-R^{5c}, -(C=O)-OR^{5c}, -(C=O)-NR^{5c}R^{6c}, -(C=S)-NHR^{5c}, -(C=O)-N(OR^{5c})R^{6c}, -(C=S)-NHOR^{5c} or -SO₂-R^{7c} wherein R^{5c} represents a hydrogen atom, a hydrocarbon group optionally having a substituent or a heterocyclic group optionally having a substituent, R^{6c} represents a hydrogen atom or a C₁₋₆ alkyl group, and R^{7c} represents a hydrocarbon group optionally having a substituent or a heterocyclic group optionally having a substituent, etc. are exemplified.

In the above-described formulae, as the "hydrocarbon group" of the "hydrocarbon group optionally having a substituent" represented by R^{5c}, for example, a chain or cyclic hydrocarbon group (e.g., alkyl, alkenyl, alkynyl, cycloalkyl, aryl, aralkyl and the like), etc. are exemplified. Of them, a chain or cyclic hydrocarbon group having 1 to 16 carbon atoms, etc. are preferable.

As the "alkyl", for example, a C₁₋₆ alkyl group (e.g., methyl, ethyl, propyl, isopropyl, butyl, isobutyl, sec-butyl, tert-butyl, pentyl, hexyl and the like), etc. are preferable.

As the "alkenyl", for example, a C₂₋₆ alkenyl group (e.g., vinyl, allyl, isopropenyl, 1-butenyl, 2-butenyl, 3-butenyl, 2-methyl-2-propenyl, 1-methyl-2-propenyl, 2-methyl-1-propenyl and the like), etc. are preferable.

As the "alkynyl", for example, a C₂₋₆ alkynyl group (e.g., ethynyl, propargyl, 1-butynyl, 2-butynyl, 3-butynyl, 1-hexynyl and the like), etc. are preferable.

As the "cycloalkyl", for example, a C₃₋₈ cycloalkyl group (e.g., cyclopropyl, cyclobutyl, cyclopentyl, cyclohexyl and the like), etc. are preferable.

As the "aryl", for example, a C₆₋₁₄ aryl group (e.g., phenyl, 1-naphthyl, 2-naphthyl, 2-biphenylyl, 3-biphenylyl, 4-biphenylyl, 2-anthryl and the like), etc. are preferable.

As the "aralkyl", for example, a C₇₋₁₆ aralkyl group (e.g., benzyl, phenethyl, diphenylmethyl, 1-naphthylmethyl, 2-naphthylmethyl, 2,2-diphenylethyl, 3-phenylpropyl, 4-phenylbutyl, 5-phenylpentyl and the like), etc. are preferable.

As the "substituent" of the "hydrocarbon group optionally having a substituent" represented by R^{5c}, the substituent selected from Group A of substituents consisting of, for example, oxo, a halogen atom (e.g., fluorine, chlorine, bromine, iodine and the like), C₁₋₃ alkylenedioxy (e.g., methylenedioxy, ethylenedioxy and the like), nitro, cyano, C₁₋₆ alkyl which may be halogenated, C₂₋₆ alkenyl which may be halogenated, carboxy C₂₋₆ alkenyl (e.g., 2-carboxyethenyl, 2-carboxy-2-methylethenyl and the like), C₂₋₆ alkynyl which may be halogenated, C₃₋₈ cycloalkyl which may be halogenated, C₃₋₈ cycloalkyl-C₁₋₆ alkyl, C₆₋₁₄ aryl (e.g., phenyl, 1-naphthyl, 2-naphthyl, 2-biphenylyl, 3-biphenylyl, 4-biphenylyl, 2-anthryl and the like), C₁₋₈ alkoxy which may be halogenated, C₁₋₆ alkoxy-carbonyl-C₁₋₆ alkoxy (e.g., ethoxycarbonylmethyloxy and the like), hydroxy, C₆₋₁₄ aryloxy (e.g., phenyloxy, 1-naphthyloxy, 2-naphthyloxy and the like), C₇₋₁₆ aralkyloxy (e.g., benzyloxy, phenethyloxy and the like), mercapto, C₁₋₆ alkylthio which may be halogenated, C₆₋₁₄ arylthio (e.g., phenylthio, 1-naphthylthio, 2-naphthylthio and the like), C₇₋₁₆ aralkylthio (e.g., benzylthio, phenethylthio and the like), amino, mono-C₁₋₆ alkylamino (e.g. methylamino, ethylamino and the like), mono-C₆₋₁₄ arylamino (e.g., phenylamino, 1-naphthylamino, 2-naphthylamino and the like), di-C₁₋₆ alkylamino (e.g., dimethylamino, diethylamino, ethylmethylamino and the like), C₃₋₈ cycloalkylamino (e.g., cyclopentylamino, cyclohexylamino and the like), di-C₆₋₁₄ arylamino (e.g., diphenylamino and the like), C₃₋₈ cycloalkyl-C₁₋₆ alkylamino (e.g., cyclopentylmethylamino, cyclohexylmethylamino, cyclopentylethylamino, cyclohexylethylamino and the like), N-C₃₋₈ cycloalkyl-N-C₁₋₆ alkylamino (N-cyclopentyl-N-methylamino, N-cyclohexyl-N-methylamino, N-cyclopentyl-N-ethylamino, N-cyclohexyl-N-ethylamino and the like), formyl, carboxy, carboxy-C₂₋₆ alkenyl, carboxy-C₁₋₆ alkyl, C₁₋₆ alkyl-carbonyl which may be halogenated (e.g., acetyl, propionyl, pivaloyl and the like), C₃₋₈ cycloalkyl-carbonyl optionally substituted by C₁₋₆ alkyl such as methyl, ethyl, etc. (e.g., cyclopropylcarbonyl, cyclopentylcarbonyl, cyclohexylcarbonyl, 1-methyl-cyclohexyl-carbonyl and the like), C₁₋₆ alkoxy-carbonyl (e.g., methoxycarbonyl, ethoxycarbonyl, propoxycarbonyl, tert-butoxycarbonyl and the like), C₆₋₁₄ aryl-carbonyl (e.g., benzoyl, 1-naphthoyl, 2-naphthoyl and the like), C₇₋₁₆ aralkyl-carbonyl (e.g., phenylacetyl, 3-phenylpropionyl and the like), C₆₋₁₄ aryloxy-carbonyl (e.g., phenoxycarbonyl and the like), C₇₋₁₆ aralkyloxy-carbonyl (e.g., benzyloxycarbonyl, phenethyloxycarbonyl and the like), 5- to 7-membered heterocyclic carbonyl containing 1 to 4 of 1 or 2 kinds of hetero atoms selected from a nitrogen atom, a sulfur atom and an oxygen atom in addition to carbon atoms (e.g., nicotinoyl, isonicotinoyl, thenoyl, furoyl, morpholinocarbonyl, thiomorpholinocarbonyl, piperazin-1-ylcarbonyl, pyrrolidin-1-ylcarbonyl and the like), carbamoyl, thiocarbamoyl, mono-C₁₋₆ alkyl-carbamoyl (e.g., methylcarbamoyl, ethylcarbamoyl, and the like), di-C₁₋₆ alkyl-carbamoyl (e.g., dimethylcarbamoyl, diethylcarbamoyl, ethylmethylcarbamoyl and the like), mono- or di-C₆₋₁₄ aryl-carbamoyl (e.g., phenylcarbamoyl, 1-naphthylcarbamoyl, 2-naphthylcarbamoyl and the like), mono- or di- 5- to 7-membered heterocyclic carbamoyl containing 1 to 4 of 1 or 2 kinds of hetero atoms selected from a nitrogen atom, a sulfur atom and an oxygen atom in addition to carbon atoms (e.g., 2-pyridylcarbamoyl, 3-pyridylcarbamoyl, 4-pyridylcarbamoyl, 2-thienylcarbamoyl, 3-thienylcarbamoyl and the like), C₁₋₆ alkylsulfonyl (e.g., methylsulfonyl, ethylsulfonyl and the like), C₁₋₆ alkylsulfinyl (e.g., methylsulfinyl, ethylsulfinyl and the like), C₆₋₁₄ arylsulfonyl (e.g., phenylsulfonyl, 1-naphthylsulfonyl, 2-naphthylsulfonyl and the like) , C₆₋₁₄ arylsulfinyl (e.g., phenylsulfinyl, 1-naphthylsulfinyl, 2-naphthylsulfinyl and the like), formylamino, C₁₋₆ alkyl-carbonylamino (e.g., acetylamino, propionylamino, pivaloylamino and the like), C₃₋₈ cycloalkyl-carbonylamino optionally substituted by C₁₋₆ alkyl (e.g., cyclopropylcarbonylamino, cyclopentylcarbonylamino, cyclohexylcarbonylamino and the like), C₆₋₁₄ aryl-carbonylamino (e.g., benzoylamino, naphthoylamino and the like), C₁₋₆ alkoxy-carbonylamino (e.g., methoxycarbonylamino, ethoxycarbonylamino, propoxycarbonylamino, butoxycarbonylamino and the like), C₁₋₆ alkylsulfonylamino (e.g., methylsulfonylamino, ethylsulfonylamino and the like), C₆₋₁₄ arylsulfonylamino (e.g., phenylsulfonylamino, 2-naphthylsulfonylamino, 1-naphthylsulfonylamino and the like), C₁₋₆ alkyl-carbonyloxy (e.g., acetoxy, propionyloxy and the like), C₆₋₁₄ aryl-carbonyloxy (e.g., benzoyloxy, naphthylcarbonyloxy and the like), C₁₋₆ alkoxy-carbonyloxy (e.g., methoxycarbonyloxy, ethoxycarbonyloxy, propoxycarbonyloxy, butoxycarbonyloxy and the like), mono-C₁₋₆ alkyl-carbamoyloxy (e.g., methylcarbamoyloxy, ethylcarbamoyloxy and the like), di-C₁₋₆ alkyl-carbamoyloxy (e.g., dimethylcarbamoyloxy, diethylcarbamoyloxy and the like), mono- or di-C₆₋₁₄ aryl-carbamoyloxy (e.g., phenylcarbamoyloxy, naphthylcarbamoyloxy and the like), nicotinoyloxy, isonicotinoyloxy, 5- to 10-membered heterocyclic group containing 1 to 4 of 1 or 2 kinds of hetero atoms selected from a nitrogen atom, a sulfur atom and an oxygen atom in addition to carbon atoms, which may have a substituent (e.g. 5- to 7-membered aliphatic heterocyclic group, 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like) optionally having a substituent, sulfo, sulfamoyl, sulfinamoyl, sulfenamoyl, a group obtained by connecting two or more (e.g., 2 to 3) of these substituents (e.g., (i) C₁₋₆ alkyl, (ii) C₆₋₁₄ aryl, (iii) amino, (iv) C₁₋₆ alkylamino, (v) C₃₋₈ cycloalkylamino, (vi) C₆₋ ₁₄ arylamino, (vii) 5- to 7-membered heterocyclic amino containing 1 to 4 of 1 or 2 kinds of hetero atoms selected from a nitrogen atom, a sulfur atom and an oxygen atom in addition to carbon atoms, (viii) C₁₋₆ alkyl-carbonyl amino, (ix) C₃₋₈ cycloalkyl-carbonylamino, (x) C₆₋₁₄ aryl-carbonylamino or (xi) 5- to 7-membered heterocyclic-carbonyl amino containing 1 to 4 of 1 or 2 kinds of hetero atoms selected from a nitrogen atom, a sulfur atom and an oxygen atom in addition to carbon atoms, which is optionally substituted, respectively, by a substituent selected from the group consisting of a halogen atom, cyano, hydroxy, C₁₋₆ alkoxy, C₆₋₁₄ aryloxy, C₁₋₆ alkylthio, C₆₋₁₄ arylthio, C₁₋₆ alkylsulfinyl, C₆₋₁₄ arylsulfinyl, C₁₋₆ alkylsulfonyl, C₆₋₁₄ arylsulfonyl, C₃₋₈ cycloalkyl, 5- to 7-membered heterocyclic group containing 1 to 4 of 1 or 2 kinds of hetero atoms selected from a nitrogen atom, a sulfur atom and an oxygen atom in addition to carbon atoms, C₆₋₁₄ aryl, C₁₋₆ alkyl-carbonyl, 5- to 7-membered heterocyclic-carbonyl containing 1 to 4 of 1 or 2 kinds of hetero atoms selected from a nitrogen atom, a sulfur atom and an oxygen atom in addition to carbon atoms, C₆₋₁₄ aryl-carbonyl, C₃₋₈ cycloalkoxy, 5- to 7-membered heterocyclic-oxy containing 1 to 4 of 1 or 2 kinds of hetero atoms selected from a nitrogen atom, a sulfur atom and an oxygen atom in addition to carbon atoms, C₁₋₆ alkylamino, C₆₋₁₄ arylamino, C₁₋₆ alkoxy-carbonyl, C₃₋₈ cycloalkoxy-carbonyl, 5-to 7-membered heterocyclic-oxycarbonyl containing 1 to 4 of 1 or 2 kinds of hetero atoms selected from a nitrogen atom, a sulfur atom and an oxygen atom in addition to carbon atoms, C₆₋₁₄ aryloxycarbonyl, etc.) and the like, and the like can be mentioned.

The above-mentioned "hydrocarbon group" may have, for example, the 1 to 5, preferably 1 to 3 above-mentioned substituents at substitutable positions, and when the number of the substituent is 2 or more, they may be the same or different.

As the above-mentioned "C₁₋₆ alkyl which may be halogenated", for example, C₁₋₆ alkyl (e.g., methyl, ethyl, propyl, isopropyl, butyl, isobutyl, sec-butyl, tert-butyl, pentyl, hexyl and the like) which may have 1 to 5, preferably 1 to 3 halogen atoms (e.g., fluorine, chlorine, bromine, iodine and the like), etc. are exemplified. As specific examples thereof, methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl 5,5,5-trifluoropentyl, hexyl, 6,6,6-trifluorohexyl, etc. are exemplified.

As the above-mentioned "C₂₋₆ alkenyl which may be halogenated", for example, C₂₋₆ alkenyl (e.g., vinyl, propenyl, isopropenyl, 2-buten-1-yl, 4-penten-1-yl, 5-hexen-1-yl and the like) which may have 1 to 5, preferably 1 to 3 halogen atoms (e.g., fluorine, chlorine, bromine, iodine and the like), etc. are exemplified.

As the above-mentioned "C₂₋₆ alkynyl which may be halogenated", for example, C₂₋₆ alkynyl (e.g., 2-butyn-1-yl, 4-pentyn-1-yl, 5-hexyn-1-yl and the like) which may have 1 to 5, preferably 1 to 3 halogen atoms (e.g., fluorine, chlorine, bromine, iodine and the like), etc. are exemplified.

As the above-mentioned "C₃₋₈ cycloalkyl which may be halogenated", for example, C₃₋₈ cycloalkyl (e.g., cyclopropyl, cyclobutyl, cyclopentyl, cyclohexyl and the like) which may have 1 to 5, preferably 1 to 3 halogen atoms (e.g., fluorine, chlorine, bromine, iodine and the like), etc. are exemplified. As specific examples thereof, cyclopropyl, cyclobutyl, cyclopentyl, cyclohexyl, 4,4-dichlorocyclohexyl, 2,2,3,3-tetrafluorocyclopentyl, 4-chlorocyclohexyl, etc. are exemplified.

As the above-mentioned "C₁₋₈ alkoxy which may be halogenated", for example, C₁₋₈ alkoxy (e.g., methoxy, ethoxy, propoxy, isopropoxy, butoxy, isobutoxy, sec-butoxy, pentyloxy, hexyloxy and the like) which may have 1 to 5, preferably 1 to 3 halogen atoms (e.g., fluorine, chlorine, bromine, iodine and the like), etc. are exemplified. As specific examples thereof, methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy, etc. are exemplified.

As the above-mentioned "C₁₋₆ alkylthio which may be halogenated", for example, C₁₋₆ alkylthio (e.g., methylthio, ethylthio, propylthio, isopropylthio, butylthio, sec-butylthio, tert-butylthio and the like) which may have 1 to 5 preferably 1 to 3 halogen atoms (e.g., fluorine, chlorine, bromine, iodine and the like), etc. are exemplified. As specific examples thereof, methylthio, difluoromethylthio, trifluoromethylthio, ethylthio, propylthio, isopropylthio, butylthio, 4,4,4-trifluorobutylthio, pentylthio, hexylthio, etc. are exemplified.

As the "5- to 7-membered aliphatic heterocyclic group" of the above-mentioned "5- to 7-membered aliphatic heterocyclic group optionally having a substituent", for example, 5- to 7-membered aliphatic heterocyclic group containing 1 to 4 of 1 or 2 kinds of hetero atoms selected from a nitrogen atom, a sulfur atom and an oxygen atom in addition to carbon atoms are exemplified, and as specific examples thereof, 1-pyrrolidinyl, 2-pyrrolidinyl, 3-pyrrolidinyl, piperidino, 2-piperidyl, 3-piperidyl, 4-piperidyl, 1-piperazinyl, 2-piperazinyl, 3-piperazinyl, 4-piperazinyl, morpholino, 2-morpholinyl, 3-morpholinyl, thiomorpholino, 2-thiomorpholinyl, 3-thiomorpholinyl, hexahydroazepin-1-yl, etc. are exemplified.

As the "substituent" of the above-mentioned "5- to 7-membered aliphatic heterocyclic group optionally having a substituent", for example, 1 to 3 of C₁₋₆ alkyl (e.g., methyl, ethyl, propyl, isopropyl, butyl, isobutyl, sec-butyl, tert-butyl, pentyl, hexyl and the like), C₆₋₁₄ aryl (e.g., phenyl, 1-naphthyl, 2-naphthyl, 2-biphenylyl, 3-biphenylyl, 4-biphenylyl, 2-anthryl and the like), C₁₋₆ alkyl-carbonyl (e.g., acetyl, propionyl and the like) which may be halogenated, C₁₋₆ alkoxy-carbonyl (e.g., methoxycarbonyl, ethoxycarbonyl, propoxycarbonyl), 5- to 10-membered aromatic heterocyclic group (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like), oxo, etc. are exemplified.

As the "heterocyclic group" of the "heterocyclic group optionally having a substituent" represented by R⁵, for example, a 5- to 14-membered (monocyclic, bicyclic or tricyclic) heterocyclic ring which contains 1 to 4 of 1 or 2 kinds of hetero atoms selected from a nitrogen atom, a sulfur atom and an oxygen atom in addition to carbon atoms are exemplified, preferably, mono-valent groups obtained by removing any one hydrogen atom from (i) a 5- to 14-membered (preferably, 5- to 10-membered) aromatic heterocyclic ring, (ii) a 5- to 10-membered non-aromatic heterocyclic ring or (iii) a 7- to 10-membered bridged heterocyclic ring, etc. are exemplified.

As the above-mentioned "5- to 14-membered (preferably, 5- to 10-membered) aromatic heterocyclic ring", for example, an aromatic heterocyclic ring such as thiophene, benzo[b]thiophene, benzo[b]furan, benzimidazole, benzoxazole, benzothiazole, benzisothiazole, naphtho[2,3-b]thiophene, furan, pyrrole, imidazole, pyrazole, pyridine, pyrazine, pyrimidine, pyridazine, indole, isoindole, 1H-indazole, purine, 4H-quinolidine, isoquinoline, quinoline, phthalazine, naphthylidine; quinoxaline, quinazoline, cinnoline, carbazole, β-carboline, phenanthridine, acridine, phenazine, thiazole, isothiazole, phenothiazine, isoxazole, furazane, phenoxazine and the like, or rings formed by fusing these rings (preferably, monocyclic ring) with 1 or plural (preferably, 1 or 2) aromatic rings (for example, benzene ring and the like), etc. are exemplified.

As the above-mentioned "5- to 10-membered non-aromatic heterocyclic ring", for example, pyrrolidine, imidazoline, pyrazolidine, pyrazoline, piperidine, piperazine, morpholine, thiomorpholine, dioxazole, oxadiazoline, thiadiazoline, triazoline, thiadiazole, dithiazole, etc. are exemplified.

As the above-mentioned "7- to 10-membered bridged heterocyclic ring", for example, quinuclidine, 7-azabicyclo [2.2.1] heptane, etc. are exemplified.

The above-mentioned "heterocyclic group" is preferably a 5- to 14-membered (preferably, 5- to 10-membered) (monocyclic or bicyclic) heterocyclic group which contains preferably 1 to 4 of 1 or 2 kinds of hetero atoms selected from a nitrogen atom, a sulfur atom and an oxygen atom in addition to carbon atoms. Specifically, an aromatic heterocyclic group such as, for example, 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pirazinyl, 2-pyrimidinyl, 4-pyrimidinyl, 3-pyrrolyl, 2-imidazolyl, 3-pyridazinyl, 3-isothiazolyl, 3-isooxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like, and aliphatic heterocyclic groups such as, for example, 1-pyrrolidinyl, 2-pyrrolidinyl, 3-pyrrolidinyl, 3-oxazolidinyl, 1-imidazolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholin0 and the like, etc. are exemplified.

Of them, for example, a 5- or 6-membered heterocyclic group containing 1 to 3 hetero atoms selected from a nitrogen atom, a sulfur atom and an oxygen atom in addition to carbon atoms are further preferable. Specifically, 2-thienyl, 3-thienyl, 2-pyridyl, 3-pyridyl, 4-pyridyl, 2-furyl, 3-furyl, pirazinyl, 2-pyrimidinyl, 3-pyrrolyl, 3-pyridazinyl, 3-isothiazolyl, 3-isooxazolyl, 1-pyrrolidinyl, 2-pyrrolidinyl, 3-pyrrolidinyl, 3-oxazolidinyl, 1-imidazolidinyl, 2-imidazolidinyl, 4-imidazolidinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino, etc. are exemplified.

As the "substituent" of the above-mentioned "heterocyclic group optionally having a substituent", for example, the same moieties as for the "substituent" of the above-mentioned "hydrocarbon group optionally having a substituent" represented by R^{5c}, etc. are exemplified.

The above-mentioned "heterocyclic group" may have, for example, 1 to 5, preferably 1 to 3 of the above-mentioned substituents at substitutable positions, and when the number of the substituent is 2 or more, they may be the same or different.

As the "C₁₋₆ alkyl group" represented by R^{6c}, for example, methyl, ethyl, propyl, isopropyl, butyl, isobutyl, sec-butyl, tert-butyl, pentyl, hexyl, etc. are exemplified.

As the "hydrocarbon group optionally having a substituent" and "heterocyclic group optionally having a substituent" represented by R^{7c}, for example, the above-mentioned "hydrocarbon group optionally having a substituent" and "heterocyclic group optionally having a substituent". represented by R^{5c} are exemplified, respectively.

As the "hydrocarbon group optionally having a substituent" represented by R^{1c} and R^{1d}, for example, the "hydrocarbon group optionally having a substituent" represented by R^{5c} is exemplified.

As the "heterocyclic group optionally having a substituent" represented by R^{1c} and R^{1d}, for example, the "heterocyclic group optionally having a substituent" represented by R^{5c} is exemplified.

As the "amino group optionally having a substituent" represented by R^{1c} and R^{1d}, (1) an amino group optionally having 1 or 2 substituents and (2) a cyclic amino group optionally having a substituent are exemplified.

As the "substituent" of the above-mentioned "(1) amino group optionally having 1 or 2 substituents", for example, a hydrocarbon group optionally having a substituent, a heterocyclic group optionally having a substituent, an acyl group, an alkylidene group optionally having a substituent, etc. are exemplified. As the "hydrocarbon group optionally having a substituent" and "heterocyclic group optionally having a substituent", for example, the same moieties as for the above-mentioned "hydrocarbon group optionally having a substituent" and "heterocyclic group optionally having a substituent" represented by R⁵ are exemplified, respectively.

As the "alkylidene group" of the above-mentioned "alkylidene group optionally having a substituent", for example, C₁₋₆ alkylidene (e.g., methylidene, ethylidene, propylidene and the like), etc. are exemplified. As the "substituent" of the above-mentioned "alkylidene group optionally having a substituent", for example, 1 to 5, preferably 1 to 3 of the same moieties as for the "substituent" of the above-mentioned "hydrocarbon group optionally having a substituent" represented by R^{5c} are exemplified.

When the number of the "substituent" of the above-mentioned "amino group optionally having 1 or 2 substituents" is two, these substituents may be the same or different.

As the "cyclic amino group" of the above-mentioned "(2) cyclic amino group optionally having a substituent", a 5- to 7-membered non-aromatic cyclic amino group (saturated cyclic amino group) which may contain 1 to 4 of 1 or 2 kinds of hetero atoms selected from a nitrogen atom, a sulfur atom and an oxygen atom in addition to one nitrogen atom and carbon atoms are exemplified, and as specific examples thereof, 1-pyrrolidinyl, piperidino, 1-piperazinyl, morpholino, thiomorpholino, hexahydroazepin-1-yl, imidazolidin-1-yl, 2,3-dihydro-1(1H)-imidazolyl, tetrahydro-1(2H)-pyrimidinyl, 3,6-dihydro-1(2H)-pyrimidinyl, 3,4-dihydro-1(2H)-pyrimidinyl, etc. are exemplified. As the "substituent" of the "cyclic amino group optionally having a substituent", for example, 1 to 3 of the same moieties as for the "substituent" of the "5- to 7-membered aliphatic heterocyclic group optionally having a substituent" described in detail as the "substituent" of the "hydrocarbon group optionally having a substituent" represented by R^{5c}.

As specific examples of the 5- to 7-membered non-aromatic cyclic amino group having one oxo, for example, 2-oxoimidazolidin-1-yl, 2-oxo-2,3-dihydro-1H-imidazol-1-yl, 2-oxoteterahydro-1(2H)-pyrimidinyl, 2-oxo-3,6-dihydro-1(2H)-pyrimidinyl, 2-oxo-3,4-dihydro-1(2H)-pyrimidinyl, 2-oxopyrrolidin-1-yl, 2-oxopiperidino, 2-oxopiperazin-1-yl, 3-oxopiperazin-1-yl, 2-oxo-2,3,4,5,6,7-hexahydroazepin-1-yl, etc. are exemplified.

R^{1c} or R^{1d} is preferably an alkyl group optionally having a substituent, an aryl group optionally having a substituent, an amino group optionally having a substituent, a heterocyclic group optionally having a substituent, an acyl group represented by the formula: -(C=O)-R^{5c} (wherein R^{5c} is as defined above), an acyl group represented by the formula: -(C=O)-OR^{5c} (wherein R^{5c} is as defined above), or the like.

As the "alkyl group optionally having a substituent", for example, a C₁₋₆ alkyl group (preferably, methyl, ethyl, propyl, butyl and the like) optionally having 1 to 5 substituents selected from a halogen atom, carboxy, hydroxy, C₁₋₆ alkoxy, C₁₋₆ alkoxy-carbonyl, C₁₋₆ alkylthio, C₁₋₆ alkylsulfinyl, C₁₋₆ alkylsulfonyl, and the like, etc. are preferably exemplified.

As the above-mentioned "aryl group optionally having a substituent", for example, a C₆₋₁₄ aryl group (preferably, phenyl and the like) optionally having 1 to 5 substituents selected from a halogen atom, C₁₋₆ alkylthio, C₆₋₁₄ arylthio, C₁₋₆ alkylsulfinyl, C₆₋₁₄ arylsulfinyl, C₁₋₆ alkylsulfonyl, C₆₋₁₄ arylsulfonyl, carboxy and the like, etc. are preferably exemplified.

As the above-mentioned "amino group optionally having a substituent", an amino group optionally having 1 or 2 acyl represented by the formula: -(C=O)-R^{5c}, -(C=O)-OR^{5c}, -(C=O)-NR^{5c}R^{6c}, -(C=S)-NHR^{5c}, -(C=O)-N(OR^{5c})R^{6c}, -(C=S)-NHOR^{5c} or -SO₂-R^{7c} (wherein each symbol is as defined above), etc. are preferably exemplified.

Further preferably, R^{1c} is an amino group optionally having 1 or 2 acyls represented by the formula: -(C=O)-R^{5c} or -(C=O)-NR^{5c}R^{6c} (wherein each symbol is as defined above), etc. are exemplified.

As the "heterocyclic group" of the "heterocyclic group optionally having a substituent", for example, a 5- to 14-membered (monocyclic, bicyclic or tricyclic) heterocyclic group which contain 1 to 4 of 1 or 2 kinds of hetero atoms selected from a nitrogen atom, a sulfur atom and an oxygen atom in addition to carbon atoms are used, and of them, a 5- to 7-membered aromatic heterocyclic group, a 5- to 10-membered non-aromatic heterocyclic group, etc. are preferable.

As the "substituent" of the "heterocyclic group optionally having a substituent", for example, an oxo group, a C₁₋₆ alkyl group (e.g., methyl, ethyl, etc.), a C₆₋₁₄ aryl group (e.g., phenyl, etc.), a C₁₋₆ alkyl-carbonyl group (e.g., acetyl, etc.), a C₁₋₆ alkoxy-carbonyl group (e.g., methoxycarbonyl, ethoxycarbonyl, etc.) and the like are used, and the number of substituents is 1 to 3.

As R^{5c} of the "acyl group represented by the formula:- (C=O)-R^{5c}", a hydrogen atom, a hydrocarbon group optionally having a substituent and an aromatic heterocyclic group optionally having a substituent are preferable, and particularly, (1) a hydrogen atom, (2) a C₁₋₆ alkyl group which may be halogenated (e.g., methyl, ethyl, propyl, trifluoromethyl, etc.), (3) a C₆₋₁₄ aryl group which may be halogenated (e.g., phenyl, naphthyl, fluorophenyl, chlorophenyl, etc.), (4)a 5- to 7-membered aromatic heterocyclic group (e.g., pyridyl, thienyl, pyrrolyl, furyl, pyridazinyl, pyrimidinyl, etc.) which may be substituted by a halogen atom (e.g., fluorine, chlorine, bromine, etc.), optionally halogenated C₁₋₆ alkyl (e.g., methyl, ethyl, propyl, trifluoromethyl, etc.), C₁₋₆ alkoxy group (e.g., methoxy, ethoxy, propoxy, butoxy, etc.), and the like are preferable.

As R^{5c} of the "acyl group represented by the formula:- (C=O)-OR^{5c}", a hydrogen atom and a hydrocarbon group optionally substituted are preferable, and particularly, a hydrogen atom and a C₁₋₆ alkyl group (e.g., methyl, ethyl, propyl, etc.), and the like are preferable.

As R^{1c} or R^{1d}, (i) a hydrogen atom, (ii) a C₁₋₆ alkyl group optionally substituted by a substituent selected from the group consisting of a halogen atom, C₁₋₆ alkoxy-carbonyl, carboxy, cyano, C₁₋₆ alkylthio, C₁₋₆ alkylsulfinyl, C₁₋₆ alkylsulfonyl, hydroxy, C₁₋₆ alkoxy and C₁₋₆ alkyl-carbonyl, (iii) a C₆₋₁₄ aryl group optionally having a substituent selected from the group consisting of a halogen atom and a group of the formula: -S(O)ₙ-R^{1f} (R^{1f} represents a C₁₋₆ alkyl group, and n represents an integer of 0 to 2), (iv) a C₇₋₁₅ aralkyl group, (v) an amino group optionally having one or two substituents selected from (1) C₁₋₆ alkyl, (2) C₁₋₆ alkyl-carbonyl, (3)5- to 7-membered heterocyclic-carbonyl containing 1 to 4 hetero atoms selected from the group consisting of a nitrogen atom, an oxygen atom and a sulfur atom in addition to carbon atoms, optionally substituted with a halogen atom, C₁₋₆ alkyl or C₁₋₆ alkoxy, (4) C₆₋₁₄ aryl-carbamoyl, (5) C₁₋₆ alkyl-carbamoyl which may be halogenated, (6) C₁₋₆ alkoxy-carbonyl which may be halogenated, (7) C₁₋₆ alkoxy-carbamoyl and (8) C₆₋₁₄ aryloxy-carbamoyl, (vi) a 5- to 10-membered heterocyclic group containing 1 to 4 of 1 or 2 kinds of hetero atoms selected from a nitrogen atom, a sulfur atom and an oxygen atom in addition to carbon atoms, optionally substituted by oxo, C₁₋₆ alkyl, C₆₋₁₄ aryl, C₁₋₆ alkoxy-carbonyl or C₁₋₆ alkyl-carbonyl, (vii) an acyl group represented by the formula:-(C=O)-R^{5d} (wherein R^{5d} represents a hydrogen atom, a C₁₋₆ alkyl group which may be halogenated or a C₆₋₁₄ aryl group which may be halogenated), or (viii) an acyl group represented by the formula:-(C=O)-OR^{5d} (wherein R^{5d} represents a hydrogen atom or a C₁₋₆ alkyl group), and the like are suitable.

As the C₁₋₆ alkyl group represented by R^{1c} or R^{1d}, for example, methyl, ethyl, propyl, isopropyl, butyl, isobutyl, sec-butyl, tert-butyl, pentyl, hexyl, etc. are used, and particularly, C₁₋₄ alkyl groups such as methyl, ethyl, propyl, butyl and the like are preferable.

As the halogen atom which is a substituent of the C₁₋₆ alkyl group represented by R^{1c} or R^{1d}, for example, a fluorine atom, a chlorine atom, a bromine atom and an iodine atom and the like are preferable.

As the C₁₋₆ alkoxy-carbonyl which is a substituent of the C₁₋₆ alkyl group represented by R^{1c} or R^{1d}, for example, methoxycarbonyl, ethoxycarbonyl and the like are preferable.

As the C₁₋₆ alkylthio which is a substituent of the C₁₋₆ alkyl group represented by R^{1c} or R^{1d}, for example, methylthio ethylthio and the like are preferable.

As the C₁₋₆ alkylsulfinyl which is a substituent of the C₁₋₆ alkyl group represented by R^{1c} or R^{1d}, for example, methylsulfinyl, ethylsulfinyl and the like are preferable.

As the C₁₋₆ alkylsulfonyl which is a substituent of the C₁₋₆ alkyl group represented by R^{1c} or R^{1d}, for example, methylsulfonyl, ethylsulfonyl and the like are preferable.

As the C₁₋₆ alkoxy which is a substituent of the C₁₋₆ alkyl group represented by R^{1c} or R^{1d}, for example, methoxy, ethoxy, propoxy and the like are preferable.

As the C₁₋₆-alkyl-carbonyl which is a substituent of the C₁₋₆ alkyl group represented by R^{1c} or R^{1d}, for example, acetyl, propionyl and the like are preferable.

As the C₁₋₆ alkyl group represented by R^{1f}, for example, methyl, ethyl, propyl, isopropyl, butyl, isobutyl, sec-butyl, tert-butyl, pentyl, hexyl, etc. are used, and of them, C₁₋₄ alkyl groups such as methyl, ethyl, propyl, butyl and the like are preferable, and methyl is particularly preferable.

As the C₆₋₁₄ aryl group represented by R^{1c} or R^{1d}, for example, phenyl, naphthyl, etc. are preferable, and of them, phenyl is particularly preferable.

As the halogen atom which is a substituent of the C₆₋₁₄ aryl group represented by R^{1c} or R^{1d}, a fluorine atom, a chlorine atom, a bromine atom and an iodine atom are used.

As the C₇₋₁₅ aralkyl group represented by R^{1c} or R^{1d}, for example, phenyl-C₁₋₃ alkyl groups such as benzyl, phenylethyl, phenylpropyl and the like are preferable.

As the C₁₋₆ alkyl group which is a substituent of an amino group represented by R^{1c} or R^{1d}, for example, methyl, ethyl, propyl, isopropyl, butyl, isobutyl, sec-butyl, tert-butyl, pentyl, hexyl, etc. are used, and of them, C₁₋₃ alkyl groups such as methyl, ethyl, propyl and the like are preferable, particularly, methyl is preferable.

As the C₁₋₆ alkyl-carbonyl which is a substituent of an amino group represented by R^{1c} or R^{1d}, for example, a C₁₋₃ alkyl-carbonyl group such as acetyl, propionyl and the like are preferable.

As the "5- to 7-membered heterocyclic-carbonyl containing 1 to 4 hetero atoms selected from the group consisting of a nitrogen atom, an oxygen atom and a sulfur atom in addition to carbon atoms" which is a substituent of an amino group represented by R^{1c} or R^{1d}, for example, a 5- to 7-membered heterocyclic (e.g., furyl, thienyl, pyrrolyl, pyridyl, pyrimidinyl, pyridazinyl and the like)-carbonyl group containing 1 or 2 hetero atoms selected from the group consisting of a nitrogen atom, an oxygen atom and a sulfur atom, etc. are preferable. As the substituent of the heterocyclic group of this heterocyclic-carbonyl group, a halogen atom such as a chlorine atom and the like, C₁₋₆ alkyl group such as methyl, ethyl and the like, and C₁₋₆ alkoxy such as methoxy, ethoxy and the like are preferable.

As the C₆₋₁₄ aryl-carbamoyl which is a substituent of an amino group represented by R^{1c} or R^{1d}, for example, phenyl-carbamoyl, etc. are preferable.

As the C₁₋₆ alkyl-carbamoyl which may be halogenated which is a substituent of an amino group represented by R^{1c} or R^{1d}, for example, methylcarbamoyl, ethylcarbamoyl, propylcarbamoyl optionally substituted by a halogen atom (e.g., chlorine atom) and the like are preferable.

As the C₁₋₆ alkoxy-carbonyl which may be halogenated which is a substituent of an amino group represented by R^{1c} or R^{1d}, for example, methoxycarbonyl, ethoxycarbonyl, propoxycarbonyl optionally substituted by halogen atoms (e.g. a chlorine atom) and the like are preferable.

As the C₁₋₆ alkoxy-carbamoyl which is a substituent of an amino group represented by R^{1c} or R^{1d}, for example, methoxycarbamoyl, ethoxycarbamoyl, propoxycarbamoyl and the like are preferable.

As the C₆₋₁₄ aryloxy-carbamoyl which is a substituent of an amino group represented by R^{1c} or R^{1d}, phenyloxy-carbamoyl and the like are preferable.

As the 5- to 10-membered non-aromatic heterocyclic group represented by R^{1c} or R^{1d}, for example, 1-pyrrolidinyl, 2-pyrrolidinyl, 3-pyrrolidinyl, 3-oxazolidinyl, 1-imidazolidinyl, 2-imidazolidinyl, 4-imidazolidinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino and the like, are used, and of them, 4-piperidyl, 1-piperazinyl, 3-oxazolidinyl 1-imidazolidinyl and the like are preferable.

As the 5- to 10-membered non-aromatic heterocyclic group optionally substituted by oxo, C₁₋₆ alkyl (preferably, methyl, ethyl), C₆₋₁₄ aryl (preferably, phenyl), C₁₋₆ alkyl-carbonyl (preferably, acetyl) or C₁₋₆ alkoxy-carbonyl (preferably, methoxycarbonyl, ethoxycarbonyl) represented by R^{1c} or R^{1d}, 1-methyl-4-piperidyl, 4-methyl-1-piperazinyl, 2-oxo-3-oxazolidinyl, 2-oxo-1-imidazolidinyl, 2-oxo-3-phenyl-1-imidazolidinyl and the like are preferable.

As R^{5d} of the formula: -(C=O)-R^{5d} represented by R^{1c} or R^{1d}, a hydrogen atom, a C₁₋₆ alkyl group which may be halogenated by a fluorine atom, a chlorine atom and the like (e.g., methyl, ethyl, trifluoromethyl, etc.), a C₆₋₁₄ aryl group which may be halogenated by a fluorine atom, a chlorine atom and the like (e.g., phenyl, naphthyl, fluorophenyl, chlorophenyl, etc.) are preferable.

As R^{5d} of the formula: -(C=O)-OR^{5d} represented by R^{1c} or R^{1d}, a hydrogen atom and a C₁₋₃ alkyl group (methyl, ethyl, etc.) are preferable.

As the "substituent containing no aromatic group" carried by a 4-pyridyl group substituted at the 5-position of a compound (Ia), the "substituent containing no aromatic group" substituted at the position adjacent to a nitrogen atom of a pyridyl group substituted at the 5-position of a compound (Ib) the "substituent containing no aromatic group" substituted at the position adjacent to a nitrogen atom of a 4-pyridyl group substituted at the 5-position of a compound (Ic), the "substituent containing no aromatic group" of the "4-pyridyl group having a substituent containing no aromatic group" represented by R^{2c}, and "the substituent containing no aromatic group" of the "pyridyl group having at the position adjacent to a nitrogen atom of the pyridyl group a substituent containing no aromatic group" represented by R^{2d}, for example, a halogen atom (e.g., fluorine, chlorine, bromine, iodine and the like), C₁₋₃ alkylenedioxy (e.g., methylenedioxy, ethylenedioxy and the like), nitro, cyano, C₁₋₆ alkyl which may be halogenated, C₂₋₆ alkenyl which may be halogenated, carboxy C₂₋₆ alkenyl (e.g., 2-carboxyethenyl, 2-carboxy-2-methylethenyl and the like), C₂₋₆ alkynyl which may be halogenated, C₃₋₈ cycloalkyl which may be halogenated, C₃₋₈ cycloalkyl-C₁₋₆ alkyl, C₁₋₈ alkoxy which may be halogenated, C₁₋₆ alkoxy-carbonyl-C₁₋₆ alkoxy (e.g., ethoxycarbonylmethyloxy and the like), hydroxy, mercapto, C₁₋₆ alkylthio which may be halogenated, amino, mono-C₁₋₆ alkylamino (e.g., methylamino, ethylamino and the like), di-C₁₋₆ alkylamino (e.g., dimethylamino, diethylamino, ethylmethylamino and the like), C₃₋₈ cycloalkylamino (e.g., cyclopentylamino, cyclohexylamino and the like), C₃₋₈ cycloalkyl-C₁₋₆ alkylamino (e.g., cyclopentylmethylamino, cyclohexylmethylamino, cyclopentylethylamino, cyclohexylethylamino and the like), N-C₃₋₈ cycloalkyl-N-C₁₋₆ alkylamino (e.g., N-cyclopentyl-N-methylamino, N-cyclohexyl-N-methylamino, N-cyclopentyl-N-ethylamino, N-cyclohexyl-N-ethylamino and the like), formyl, carboxy, carboxy-C₂₋₆ alkenyl, carboxy-C₁₋₆ alkyl, C₁₋₆ alkyl-carbonyl which may be halogenated (e.g., acetyl, propionyl, 2,2,2-trifluoroacetyl, 3,3,3-trifluoropropionyl, 2,2-difluoropropionyl and the like), C₃₋₈ cycloalkyl-carbonyl optionally substituted by C₁₋₆ alkyl (e.g., cyclopropylcarbonyl, cyclopentylcarbonyl, cyclohexylcarbonyl, 1-methyl-cyclohexyl-carbonyl and the like), C₁₋₆ alkoxy-carbonyl (e.g., methoxycarbonyl, ethoxycarbonyl, propoxycarbonyl, tert-butoxycarbonyl and the like), carbamoyl, thiocarbamoyl, mono-C₁₋₆ alkyl-carbamoyl (e.g., methylcarbamoyl, ethylcarbamoyl, and the like), di-C₁₋₆ alkyl-carbamoyl (e.g., dimethylcarbamoyl, diethylcarbamoyl, ethylmethylcarbamoyl and the like), C₁₋₆ alkylsulfonyl (e.g., methylsulfonyl, ethylsulfonyl and the like), C₁₋₆ alkylsulfinyl (e.g., methylsulfinyl, ethylsulfinyl and the like), formylamino, C₁₋₆ alkyl-carbonylamino (e.g., acetylamino, propionylamino, pivaloylamino and the like), C₃₋₈ cycloalkyl-carbonylamino optionally substituted by C₁₋₆ alkyl (e.g., cyclopropylcarbonylamino, cyclopentylcarbonylamino, cyclohexylcarbonylamino, 1-methyl-cyclohexylcarbonylamino and the like), C₁₋₆ alkoxy-carbonylamino (e.g., methoxycarbonylamino, ethoxycarbonylamino, propoxycarbonylamino, butoxycarbonylamino and the like), C₁₋₆ alkylsulfonylamino (e.g., methylsulfonylamino, ethylsulfonylamino and the like), C₁₋₆ alkyl-carbonyloxy (e.g., acetoxy, propionyloxy and the like), C₁₋₆ alkoxy-carbonyloxy (e.g., methoxycarbonyloxy, ethoxycarbonyloxy, propoxycarbonyloxy, butoxycarbonyloxy and the like), mono-C₁₋₆ alkylcarbamoyloxy (e.g., methylcarbamoyloxy, ethylcarbamoyloxy and the like), di-C₁₋₆ alkyl-carbamoyloxy (e.g., dimethylcarbamoyloxy, diethylcarbamoyloxy and the like), 5- to 7-membered aliphatic heterocyclic group optionally having a substituent, sulfo, sulfamoyl, sulfinamoyl, sulfenamoyl, a group formed by connecting 2 or more (e.g., 2 to 3) of these substituents (e.g., (i) C₁₋₆ alkyl, (ii) amino, (iii) C₁₋₆ alkylamino, (iv) C₃₋₈ cycloalkylamino, (v) 5- to 7-membered aliphatic heterocyclic amino containing 1 to 4 of 1 or 2 kinds of hetero atoms selected from a nitrogen atom, a sulfur atom and an oxygen atom in addition to carbon atoms, (vi) C₁₋₆ alkyl-carbonyl amino, (vii) C₃₋₈ cycloalkyl-carbonylamino or (viii) 5- to 7-membered aliphatic heterocyclic-carbonyl amino containing 1 to 4 of 1 or 2 kinds of hetero atoms selected from a nitrogen atom, a sulfur atom and an oxygen atom in addition to carbon atoms, which is optionally substituted, respectively, by a substituent selected from the group consisting of a halogen atom, cyano, hydroxy, C₁₋₆ alkoxy, C₁₋₆ alkylthio, C₁₋₆ alkylsulfinyl, C₁₋₆ alkylsulfonyl, C₃₋₈ cycloalkyl, 5- to 7-membered aliphatic heterocyclic group containing 1 to 4 of 1 or 2 kinds of hetero atoms selected from a nitrogen atom, a sulfur atom and an oxygen atom in addition to carbon atoms, C₁₋₆ alkyl-carbonyl, 5- to 7-membered aliphatic heterocyclic-carbonyl containing 1 to 4 of 1 or 2 kinds of hetero atoms selected from a nitrogen atom, a sulfur atom and an oxygen atom in addition to carbon atoms, C₃₋₈ cycloalkoxy, 5- to 7-membered aliphatic heterocyclic-oxy containing 1 to 4 of 1 or 2 kinds of hetero atoms selected from a nitrogen atom, a sulfur atom and an oxygen atom in addition to carbon atoms, C₁₋₆ alkylamino, C₁₋₆ alkoxy-carbonyl, C₃₋₈ cycloalkoxy-carbonyl, 5- to 7-membered aliphatic heterocyclic-oxycarbonyl containing 1 to 4 of 1 or 2 kinds of hetero atoms selected from a nitrogen atom, a sulfur atom and an oxygen atom in addition to carbon atoms, etc.) and the like are exemplified.

As the above-mentioned "C₁₋₆ alkyl which may be halogenated", for example, C₁₋₆ alkyl (e.g., methyl, ethyl, propyl, isopropyl, butyl, isobutyl, sec-butyl, tert-butyl, pentyl, hexyl and the like) which may have 1 to 5, preferably 1 to 3 halogen atoms (e.g., fluorine, chlorine, bromine, iodine and the like), etc. are exemplified. As specific examples thereof, methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl, etc. are exemplified.

As the above-mentioned "C₂₋₆ alkenyl which may be halogenated", for example, C₂₋₆ alkenyl (e.g., vinyl, propenyl, isopropenyl, 2-buten-1-yl, 4-penten-1-yl, 5-hexen-1-yl and the like) which may have 1 to 5, preferably 1 to 3 halogen atoms (e.g., fluorine, chlorine, bromine, iodine and the like), etc. are exemplified.

As the above-mentioned "C₂₋₆ alkynyl which may be halogenated", for example, C₂₋₆ alkynyl (e.g., 2-butyn-1-yl, 4-pentyn-1-yl, 5-hexyn-1-yl and the like) which may have 1 to 5, preferably 1 to 3 halogen atoms (e.g., fluorine, chlorine, bromine, iodine and the like), etc. are exemplified.

As the above-mentioned "C₃₋₈ cycloalkyl which may be halogenated", for example, C₃₋₈ cycloalkyl (e.g., cyclopropyl, cyclobutyl, cyclopentyl, cyclohexyl and the like) which may have 1 to 5, preferably 1 to 3 halogen atoms (e.g., fluorine, chlorine, bromine, iodine and the like), etc. are exemplified. As specific examples thereof, cyclopropyl, cyclobutyl, cyclopentyl, cyclohexyl, 4,4-dichlorocyclohexyl, 2,2,3,3-tetrafluorocyclopentyl, 4-chlorocyclohexyl, etc. are exemplified.

As the above-mentioned "C₁₋₈ alkoxy which may be halogenated", for example, C₁₋₈ alkoxy (e.g., methoxy, ethoxy, propoxy, isopropoxy, butoxy, isobutoxy, sec-butoxy, pentyloxy, hexyloxy and the like) which may have 1 to 5, preferably 1 to 3 halogen atoms (e.g., fluorine, chlorine, bromine, iodine and the like), etc. are exemplified. As specific examples thereof, methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy, etc. are exemplified.

As the above-mentioned "C₁₋₆ alkylthio which may be halogenated", for example, C₁₋₆ alkylthio (e.g., methylthio, ethylthio, propylthio, isopropylthio, butylthio, sec-butylthio, tert-butylthio and the like) which may have 1 to 5, preferably 1 to 3 halogen atoms (e.g., fluorine, chlorine, bromine, iodine and the like), etc. are exemplified. As specific examples thereof, methylthio, difluoromethylthio, trifluoromethylthio, ethylthio, propylthio, isopropylthio, butylthio, 4,4,4-trifluorobutylthio, pentylthio, hexylthio, etc. are exemplified.

As the "5- to 7-membered aliphatic heterocyclic group" of the above-mentioned "5- to 7-membered aliphatic heterocyclic group optionally having a substituent", for example, 5- to 7-membered aliphatic heterocyclic group containing 1 to 4 of 1 or 2 kinds of hetero atoms selected from a nitrogen atom, a sulfur atom and an oxygen atom in addition to carbon atoms are exemplified, and as specific examples thereof, 1-pyrrolidinyl, 2-pyrrolidinyl, 3-pyrrolidinyl, piperidino, 2-piperidyl, 3-piperidyl, 4-piperidyl, 1-piperazinyl, 2-piperazinyl, 3-piperazinyl, 4-piperazinyl, morpholino, 2-morpholinyl, 3-morpholinyl, thiomorpholino, 2-thiomorpholinyl, 3-thiomorpholinyl, hexahydroazepin-1-yl, etc. are exemplified.

As the "substituent" of the above-mentioned "5- to 7-membered aliphatic heterocyclic group optionally having a substituent", for example, 1 to 3 of C₁₋₆ alkyl (e.g., methyl, ethyl, propyl, isopropyl, butyl, isobutyl, sec-butyl, tert-butyl, pentyl, hexyl and the like), C₁₋₆ alkyl-carbonyl (e.g., acetyl, propionyl and the like), oxo, etc. are exemplified.

Provided that, when
(i) R^{1c} or R^{1d} is an acetylamino group, and R^{3c} or R^{3d} is a 3,5-dimethylphenyl group,
(ii) R^{1c} or R^{1d} is a C₁₋₆ alkyl-carbonylamino group, and R^{3c} or R^{3d} is a C₆₋₁₄ aryl group substituted by a C₁₋₆ alkyl group, or
(iii) R^{1c} or R^{1d} is an amino group optionally having a substituent, and R^{3c} or R^{3d} is an aromatic hydrocarbon group optionally having a substituent,
   there are exemplified, as the "substituent containing no aromatic group", a halogen atom (e.g., fluorine, chlorine, bromine, iodine and the like), C₁₋₃ alkylenedioxy (e.g., methylenedioxy, ethylenedioxy and the like), nitro, cyano, C₁₋₆ alkyl which may be halogenated, C₂₋₆ alkenyl which may be halogenated, carboxy C₂₋₆ alkenyl (e.g., 2-carboxyethenyl, 2-carboxy-2-methylethenyl and the like), C₂₋₆ alkynyl which may be halogenated, C₃₋₈ cycloalkyl which may be halogenated, C₃₋₈ cycloalkyl-C₁₋₆ alkyl, C₁₋₈ alkoxy which may be halogenated, C₁₋₆ alkoxy-carbonyl-C₁₋₆ alkoxy (e.g., ethoxycarbonylmethyloxy and the like), mercapto, C₁₋₆ alkylthio which may be halogenated, amino, mono-C₁₋₆ alkylamino (e.g., methylamino, ethylamino and the like), di-C₁₋₆ alkylamino (e.g., dimethylamino, diethylamino, ethylmethylamino and the like), C₃₋₈ cycloalkylamino (e.g., cyclopentylamino, cyclohexylamino and the like), C₃₋₈ cycloalkyl-C₁₋₆ alkylamino (e.g., cyclopentylmethylamino, cyclohexylmethylamino, cyclopentylethylamino, cyclohexylethylamino and the like), N-C₃₋₈ cycloalkyl-N-C₁₋₆ alkylamino (e.g., N-cyclopentyl-N-methylamino, N-cyclohexyl-N-methylamino, N-cyclopentyl-N-ethylamino, N-cyclohexyl-N-ethylamino and the like), formyl, carboxy, carboxy-C₂₋₆ alkenyl, carboxy-C₁₋₆ alkyl, C₁₋₆ alkyl-carbonyl (e.g., acetyl, propionyl and the like), C₃₋₈ cycloalkyl-carbonyl optionally substituted by C₁₋₆ alkyl (e.g., cyclopropylcarbonyl, cyclopentylcarbonyl, cyclohexylcarbonyl, 1-methyl-cyclohexyl-carbonyl and the like), C₁₋₆ alkoxy-carbonyl (e.g., methoxycarbonyl, ethoxycarbonyl, propoxycarbonyl, tert-butoxycarbonyl and the like), carbamoyl, thiocarbamoyl, mono-C₁₋₆ alkyl-carbamoyl (e.g., methylcarbamoyl, ethylcarbamoyl, and the like), di-C₁₋₆ alkyl-carbamoyl (e.g., dimethylcarbamoyl, diethylcarbamoyl, ethylmethylcarbamoyl and the like), C₁₋₆ alkylsulfonyl (e.g., methylsulfonyl, ethylsulfonyl and the like), C₁₋₆ alkylsulfinyl (e.g., methylsulfinyl, ethylsulfinyl and the like), formylamino, C₁₋₆ alkyl-carbonylamino (e.g., acetylamino, propionylamino, pivaloylamino and the like), C₃₋₈ cycloalkyl-carbonylamino optionally substituted by C₁₋₆ alkyl (e.g., cyclopropylcarbonylamino, cyclopentylcarbonylamino, cyclohexylcarbonylamino and the like), C₁₋₆ alkoxy-carbonylamino (e.g., methoxycarbonylamino, ethoxycarbonylamino, propoxycarbonylamino, butoxycarbonylamino and the like), C₁₋₆ alkylsulfonylamino (e.g., methylsulfonylamino, ethylsulfonylamino and the like), C₁₋₆ alkoxy-carbonyloxy (e.g., methoxycarbonyloxy, ethoxycarbonyloxy, propoxycarbonyloxy, butoxycarbonyloxy and the like), mono-C₁₋₆ alkyl-carbamoyloxy (e.g., methylcarbamoyloxy, ethylcarbamoyloxy and the like), di-C₁₋₆ alkyl-carbamoyloxy (e.g., dimethylcarbamoyloxy, diethylcarbamoyloxy and the like), 5- to 7-membered aliphatic heterocyclic group optionally having a substituent (preferably, 5- to 7-membered aliphatic heterocyclic group having 1 to 4 hetero atoms selected from a nitrogen atom, an oxygen atom and a sulfur atom in addition to carbon atoms), sulfo, sulfamoyl, sulfinamoyl, sulfenamoyl and the like are exemplified. Further, a group obtained by connecting two or more (e.g., 2 or 3) (e.g., (i) C₁₋₆ alkyl, (ii) amino, (iii) C₁₋₆ alkylamino, (iv) C₃₋₈ cycloalkylamino, (v) 5- to 7-membered aliphatic heterocyclic amino containing 1 to 4 of 1 or 2 kinds of hetero atoms selected from a nitrogen atom, a sulfur atom and an oxygen atom in addition to carbon atoms, (vi) C₁₋₆ alkyl-carbonyl amino, (vii) C₃₋₈ cycloalkyl-carbonylamino or (viii) 5- to 7-membered aliphatic heterocyclic-carbonyl amino containing 1 to 4 of 1 or 2 kinds of hetero atoms selected from a nitrogen atom, a sulfur atom and an oxygen atom in addition to carbon atoms, which is substituted, respectively, by a substituent selected from the group consisting of a halogen atom, cyano, hydroxy, C₁₋₆ alkoxy, C₁₋₆ alkylthio, C₁₋₆ alkylsulfinyl, C₁₋₆ alkylsulfonyl, C₃₋₈ cycloalkyl, 5- to 7-membered aliphatic heterocyclic group containing 1 to 4 of 1 or 2 kinds of hetero atoms selected from a nitrogen atom, a sulfur atom and an oxygen atom in addition to carbon atoms, C₁₋₆ alkyl-carbonyl, 5- to 7-membered aliphatic heterocyclic-carbonyl containing 1 to 4 of 1 or 2 kinds of hetero atoms selected from a nitrogen atom, a sulfur atom and an oxygen atom in addition to carbon atoms, C₃₋₈ cycloalkoxy, 5- to 7-membered aliphatic heterocyclic-oxy containing 1 to 4 of 1 or 2 kinds of hetero atoms selected from a nitrogen atom, a sulfur atom and an oxygen atom in addition to carbon atoms, C₁₋₆ alkylamino, C₁₋₆ alkoxy-carbonyl, C₃₋₈ cycloalkoxy-carbonyl, 5- to 7-membered aliphatic heterocyclic-oxycarbonyl containing 1 to 4 of 1 or 2 kinds of hetero atoms selected from a nitrogen atom, a sulfur atom and an oxygen atom in addition to carbon atoms, etc.), etc. can also be used as a substituent.
   Among these substituents, specifically, 1 to 3, preferably 1 or 2 substituents selected from the following (1) to (6), particularly (1) to (4) are preferably used.
   (1) a C₁₋₆ alkyl group (e.g., methyl, ethyl, propyl, isopropyl, butyl, isobutyl, sec-butyl, tert-butyl, pentyl, hexyl and the like, preferably a C₁₋₄ alkyl group such as methyl, ethyl, propyl, butyl and the like): this C₁₋₆ alkyl group may be substituted by a halogen atom, cyano, hydroxy, C₃₋₈ cycloalkyl or a 5- to 7-membered aliphatic heterocyclic group containing 1 to 4 hetero atoms selected from a nitrogen atom, an oxygen atom and a sulfur atom in addition to carbon atoms (e.g., 1-pyrrolidinyl, 2-pyrrolidinyl, 3-pyrrolidinyl, piperidino, 2-piperidyl, 3-piperidyl, 4-piperidyl, 1-piperazinyl, 2-piperazinyl, 3-piperazinyl, 4-piperazinyl, morpholino, 2-morpholinyl, 3-morpholinyl, thiomorpholino, 2-thiomorpholinyl, 3-thiomorpholinyl, hexahydroazepin-1-yl and the like),
   (2) a halogen atom (e.g., fluorine atom, chlorine atom, bromine atom, iodine atom),
   (3) an amino group optionally having a substituent selected from the group consisting of the following 1) to 7):
      1) a C₁₋₆ alkyl group (e.g., methyl, ethyl, propyl, isopropyl, butyl, isobutyl, sec-butyl, tert-butyl, pentyl, hexyl and the like, preferably C₁₋₃ alkyl groups such as methyl, ethyl, propyl and the like), this C₁₋₆ alkyl group may be substituted by a halogen atom, cyano, hydroxy, C₃₋₈ cycloalkyl or 5- to 7-membered aliphatic heterocyclic group containing 1 to 4 hetero atoms selected from a nitrogen atom, an oxygen atom and a sulfur atom in addition to carbon atoms (e.g., 1-pyrrolidinyl, 2-pyrrolidinyl, 3-pyrrolidinyl, piperidino, 2-piperidyl, 3-piperidyl, 4-piperidyl, 1-piperazinyl, 2-piperazinyl, 3-piperazinyl, 4-piperazinyl, morpholino, 2-morpholinyl, 3-morpholinyl, thiomorpholino, 2-thiomorpholinyl, 3-thiomorpholinyl, hexahydroazepin-1-yl and the like),
      2) a C₃₋₈ cycloalkyl group (e.g., cyclopropyl, cyclobutyl, cyclopentyl, cyclohexyl and the like),
      3) a C₁₋₆ alkyl-carbonyl group (e.g., acetyl, propionyl, butyryl, valeryl, isovaleryl, 2-methylpropionyl, pivaloyl and the like), this C₁₋₆ alkyl-carbonyl group may be substituted by a halogen atom, cyano, hydroxy, C₃₋₈ cycloalkyl or 5- to 7-membered aliphatic heterocyclic group containing 1 to 4 hetero atoms selected from a nitrogen atom, an oxygen atom and a sulfur atom in addition to carbon atoms (e.g., 1-pyrrolidinyl, 2-pyrrolidinyl, 3-pyrrolidinyl, piperidino, 2-piperidyl, 3-piperidyl, 4-piperidyl, 1-piperazinyl, 2-piperazinyl, 3-piperazinyl, 4-piperazinyl, morpholino, 2-morpholinyl, 3-morpholinyl, thiomorpholino, 2-thiomorpholinyl, 3-thiomorpholinyl, hexahydroazepin-1-yl and the like),
      4) a C₁₋₆ alkoxy-carbonyl group (e.g., methoxycarbonyl, ethoxycarbonyl, propoxycarbonyl, butoxycarbonyl, isobutoxycarbonyl, sec-butoxycarbonyl, tert-butoxycarbonyl and the like),
      5) a C₃₋₈ cycloalkyl-carbonyl group optionally substituted by C₁₋₆ alkyl such as methyl, ethyl (e.g., cyclopropylcarbonyl, cyclobutylcarbonyl, cyclopentylcarbonyl, cyclohexylcarbonyl, 1-methyl-cyclohexylcarbonyl and the like),
      6) a 5- to 7-membered aliphatic heterocyclic group having 1 to 4 hetero atoms selected from a nitrogen atom, an oxygen atom and a sulfur atom in addition to carbon atoms (e.g., 1-pyrrolidinyl, 2-pyrrolidinyl, 3-pyrrolidinyl, piperidino, 2-piperidyl, 3-piperidyl, 4-piperidyl, 1-piperazinyl, 2-piperazinyl, 3-piperazinyl, 4-piperazinyl, morpholino, 2-morpholinyl, 3-morpholinyl, thiomorpholino, 2-thiomorpholinyl, 3-thiomorpholinyl, hexahydroazepin-1-yl, etc.). This aliphatic heterocyclic group may be substituted by C₁₋₆ alkyl (e.g., methyl, ethyl) and the like.
      7) a 5- to 7-membered aliphatic heterocyclic (e.g., 1-pyrrolidinyl, 2-pyrrolidinyl, 3-pyrrolidinyl, piperidino, 2-piperidyl, 3-piperidyl, 4-piperidyl, 1-piperazinyl, 2-piperazinyl, 3-piperazinyl, 4-piperazinyl, morpholino,.2-morpholinyl, 3-morpholinyl, thiomorpholino, 2-thiomorpholinyl, 3-thiomorpholinyl, hexahydroazepin-1-yl, etc.)-carbonyl group having 1 to 4 hetero atoms selected from a nitrogen atom, an oxygen atom and a sulfur atom in addition to carbon atoms. This aliphatic heterocyclic-carbonyl group may be substituted by C₁₋₆ alkyl (e.g., methyl, ethyl) and the like.
   (4) a 5- to 7-membered saturated cyclic amino group which may contain further 1 to 4 hetero atoms selected from a nitrogen atom, an oxygen atom and a sulfur atom in addition to carbon atoms and one nitrogen atom (e.g., 1-pyrrolidinyl, piperidino, morpholino, 1-piperazinyl and the like). This saturated cyclic amino group may be substituted with C₁₋₆ alkyl (e.g., methyl, ethyl) and the like.
   (5) a hydroxyl group,
   (6) a C₁₋₆ alkyl-carbonyloxy group (e.g., acetyloxy, propionyloxy, butyryloxy and the like).

The "pyridyl group" represented by R^{2c} and R^{2d} may have, for example, 1 to 5, preferably 1 to 3 of the above-mentioned substituents at substitutable positions, and when the number of substituents is 2 or more, they may be the same or different. Further, an endocyclic nitrogen atom of the "pyridyl group" may be N-oxidized.

As the "pyridyl group" of the "pyridyl group having at the position adjacent to a nitrogen atom of the pyridyl group a substituent including no aromatic group" represented by R^{2d}, 1-, 2-, 3- and 4-pyridyl group are exemplified, and 4-pyridyl group is particularly preferable.

As the "aromatic group" of the "aromatic group optionally having a substituent," represented by R^{3c} and R^{3d}, an aromatic hydrocarbon group, an aromatic heterocyclic group, etc. are exemplified.

As the "aromatic hydrocarbon group", for example, a monocyclic or fused polycyclic (bicyclic or tricyclic) aromatic hydrocarbon group having 6 to 14 carbon atoms are exemplified. As specific examples thereof, for example, C₆₋₁₄ aryl such as phenyl, 1-naphthyl, 2-naphthyl, 2-biphenylyl, 3-biphenylyl, 4-biphenylyl, 2-anthryl and the like, etc. are exemplified.

As the "aromatic heterocyclic group", for example, a 5-to 14-membered (preferably, 5- to 10-membered) (monocyclic or bicyclic) aromatic heterocyclic groups preferably containing 1 to 4 of 1 or 2 kinds of hetero atoms selected from a nitrogen atom, a sulfur atom and an oxygen atom in addition to carbon atoms, are exemplified. Specifically, an aromatic heterocyclic group such as, for example, 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pirazinyl, 2-pyrimidinyl, 4-pyrimidinyl, 3-pyrrolyl, 2-imidazolyl, 3-pyridazinyl, 3-isothiazolyl, 3-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like, are exemplified.

As the "substituent" of the above-mentioned "aromatic group optionally having a substituent", for example, 1 to 5, preferably 1 to 3 of the same moieties as for the "substituent" of the above-mentioned "hydrocarbon group optionally having a substituent" represented by R^{5c} are exemplified. When the number of the substituents is two or more, these substituents may be the same or different. Further, adjacent two substituents may form a 4- to 7-membered non-aromatic carbon ring. Preferable is a 5- or 6-membered non-aromatic carbon ring.

R^{3c} and R^{3d} preferably represent a C₆₋₁₄ aryl group, or a 5- to 14-membered aromatic heterocyclic group preferably containing 1 to 4 of 1 or 2 kinds of hetero atoms selected from a nitrogen atom, a sulfur atom and an oxygen atom in addition to carbon atoms. More preferably, they represent a phenyl group optionally having a substituent or a thienyl group optionally having a substituent, and a phenyl group optionally having a substituent is particularly preferable.

As the substituent on the C₆₋₁₀ aryl group, a phenyl group, a 5- to 14-membered aromatic heterocyclic group and a thienyl group, preferable are 1 to 3 substituents selected from a halogen atom, C₁₋₃ alkylenedioxy, C₁₋₆ alkyl which may be halogenated, carboxy C₂₋₆ alkenyl, C₃₋₈ cycloalkyl, C₁₋₈ alkoxy which may be halogenated, hydroxy, C₇₋₁₆ aralkyloxy, C₁₋ ₆ alkyl-carbonyloxy, carboxy, C₁₋₆ alkoxy-carbonyl, cyano, C₁₋₆ alkylthio, C₁₋₆ alkylsulfonyl and particularly, a halogen atom, C₁₋₆ alkyl which may be halogenated (e.g., C₁₋₃ alkyl such as methyl, ethyl, propyl and the like), C₁₋₈ alkoxy which may be halogenated (e.g., C₁₋₃ alkoxy such as methoxy, ethoxy and the like), carboxy, C₁₋₆ alkoxy-carbonyl, cyano, C₁₋₆ alkylthio (e.g., methylthio, ethylthio), C₁₋₆ alkylsulfonyl (e.g., methylsulfonyl, ethylsulfonyl), etc. are preferable. Further, two alkyl groups adjacent as substituents may form a 5-membered non-aromatic carbocyclic ring.

As the compound (Va), specifically, compounds represented by the following formula are preferably used: 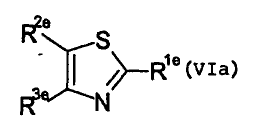 wherein R^{1e} represents (i) a hydrogen atom, (ii) a C₁₋₆ alkyl group optionally substituted by a substituent selected from the group consisting of a halogen atom, C₁₋₆ alkoxy-carbonyl, carboxy, cyano, C₁₋₆ alkylthio, C₁₋₆ alkylsulfinyl, C₁₋₆ alkylsulfonyl, hydroxy, C₁₋₆ alkoxy and C₁₋₆ alkyl-carbonyl, (iii) a C₆₋₁₄ aryl group optionally having a substituent selected from the group consisting of a halogen atom and a group represented by the formula: -S(O)ₙ-R^{1f} (R^{1f} represents a C₁₋₆ alkyl group, and n represents an integer of 0 to 2), (iv) a C₇₋₁₅ aralkyl group, (v) an amino group optionally having one or two substituents selected from 1) C₁₋₆ alkyl, 2) C₁₋₆ alkyl-carbonyl, 3) C₁₋₆ alkoxy-carbonyl, 4) 5- to 7-membered heterocyclic-carbonyl containing 1 to 4 hetero atoms selected from the group consisting of a nitrogen atom, an oxygen atom and a sulfur atom, in addition to carbon atoms, optionally substituted with a halogen atom, C₁₋₆ alkyl group or C₁₋₆ alkoxy, 5) C₆₋₁₄ aryl-carbamoyl, 6) C₁₋₆ alkyl-carbamoyl which may be halogenated, 7) C₁₋₆ alkoxy-carbonyl which may be halogenated, 8) C₁₋₆ alkoxy-carbamoyl and 9) C₆₋₁₄ aryloxy-carbamoyl, (vi) a 5- to 10-membered heterocyclic group containing 1 to 4 of 1 or 2 kinds of hetero atoms selected from a nitrogen atom, a sulfur atom and an oxygen atom in addition to carbon atoms, optionally substituted by oxo, C₁₋₆ alkyl, C₆₋₁₄ aryl, C₁₋₆ alkyl-carbonyl or C₁₋₆ alkoxy-carbonyl, (vii) an acyl group represented by the formula: -(C=O)-R^{5d} (wherein R^{5d} represents a hydrogen atom, a C₁₋₆ alkyl group which may be halogenated or a C₆₋₁₄ aryl group which may be halogenated), or (viii) an acyl group represented by the formula: -(C=O)-OR^{5d} (wherein R^{5d} represents a hydrogen atom or a C₁₋₆ alkyl group) ,
R^{2e} represents a pyridyl group (preferably, 4-pyridyl group and this pyridyl group may be N-oxidized.) having at the position adjacent to a nitrogen atom of the pyridyl group a substituent selected from the group (particularly, consisting of (1) to (4)) consisting of
(1) a C₁₋₆ alkyl group (this C₁₋₆ alkyl group may be substituted by a halogen atom, cyano, hydroxy, C₃₋₈ cycloalkyl or a 5- to 7-membered aliphatic heterocyclic group containing 1 to 4 hetero atoms selected from a nitrogen atom, an oxygen atom and a sulfur atom in addition to carbon atoms),
(2) a halogen atom,
(3) an amino group optionally having a substituent selected from the group consisting of the following 1) to 7);
   1) a C₁₋₆ alkyl group (this C₁₋₆ alkyl group may be substituted by halogen atoms, cyano, hydroxy, C₃₋₈ cycloalkyl or 5- to 7-membered aliphatic heterocyclic group having 1 to 4 hetero atoms selected from a nitrogen atom, an oxygen atom and a sulfur atom in addition to carbon atoms),
   2) a C₃₋₈ cycloalkyl group,
   3) a C₁₋₆ alkyl-carbonyl group (this C₁₋₆ alkyl-carbonyl group may be substituted by halogen atoms, cyano, hydroxy, C₃₋₈ cycloalkyl or 5- to 7-membered aliphatic heterocyclic group having 1 to 4 hetero atoms selected from a nitrogen atom, an oxygen atom and a sulfur atom in addition to carbon atoms),
   4) a C₁₋₆ alkoxy-carbonyl group,
   5) a C₃₋₈ cycloalkyl-carbonyl group optionally substituted by C₁₋₆ alkyl,
   6) a 5- to 7-membered aliphatic heterocyclic group having 1 to 4 hetero atoms selected from a nitrogen atom, an oxygen atom and a sulfur atom in addition to carbon atoms (this aliphatic heterocyclic group may be substituted by C₁₋₆ alkyl or C₁₋₆ alkyl-carbonyl) ,
   7) a 5- to 7-membered aliphatic heterocyclic-carbonyl group having 1 to 4 hetero atoms selected from a nitrogen atom, an oxygen atom and a sulfur atom in addition to carbon atoms (this aliphatic heterocyclic-carbonyl group may be substituted by C₁₋₆ alkyl or C₁₋₆ alkyl-carbonyl),
(4) a 5- to 7-membered saturated cyclic amino group optionally further containing 1 to 4 hetero atoms selected from a nitrogen atom, an oxygen atom and a sulfur atom in addition to carbon atoms and one nitrogen atom (this saturated cyclic amino group may be substituted by C₁₋₆ alkyl or C₁₋₆ alkyl-carbonyl),
(5) a hydroxy group, and
(6) a C₁₋₆ alkyl-carbonyloxy group, and
   R^{3e} represents (1) a C₆₋₁₄ aryl group or (2) a 5- to 14-membered aromatic heterocyclic group preferably containing 1 to 4 of 1 or 2 kinds of hetero atoms selected from a nitrogen atom, a sulfur atom and an oxygen atom in addition to carbon atoms, which optionally has a substituent selected from the group consisting of C₁₋₆ alkyl which may be halogenated, C₁₋₆ alkoxy, a halogen atom, carboxyl, C₁₋₆ alkoxy-carbonyl, cyano, C₁₋₆ alkylthio and C₁₋₆ alkylsulfonyl.

As the C₁₋₆ alkyl group represented by R^{1e}, for example, methyl, ethyl, propyl, isopropyl, butyl, isobutyl, sec-butyl, tert-butyl, pentyl, hexyl, etc. are used, and particularly, a C₁₋₄ alkyl group such as methyl, ethyl, propyl, butyl and the like are preferable, and a C₁₋₃ alkyl group such as methyl, ethyl, propyl, etc. are particularly preferable.

As the halogen atom which is a substituent of the C₁₋₆ alkyl group represented by R^{1e}, a fluorine atom, a chlorine atom, a bromine atom and an iodine atom and the like are used.

As the C₁₋₆ alkoxy-carbonyl which is a substituent of the C₁₋₆ alkyl group represented by R^{1e}, for example, methoxycarbonyl, ethoxycarbonyl and the like are preferable.

As the C₁₋₆ alkylthio which is a substituent of the C₁₋₆ alkyl group represented by R^{1e}, for example, methylthio, ethylthio and the like are preferable.

As the C₁₋₆ alkylsulfinyl which is a substituent of the C₁₋₆ alkyl group represented by R^{1e}, for example, methylsulfinyl, ethylsulfinyl and the like are preferable.

As the C₁₋₆ alkylsulfonyl which is a substituent of the C₁₋₆ alkyl group represented by R^{1e}, for example, methylsulfonyl, ethylsulfonyl and the like are preferable.

As the C₁₋₆ alkoxy which is a substituent of the C₁₋₆ alkyl group represented by R^{1e}, for example, methoxy, ethoxy, propoxy and the like are preferable.

As the C₁₋₆ alkyl-carbonyl which is a substituent of the C₁₋₆ alkyl group represented by R^{1e}, for example, acetyl, propionyl and the like are preferable.

As the C₁₋₆ alkyl group represented by R^{1f}, for example; methyl, ethyl, propyl, isopropyl, butyl, isobutyl, sec-butyl, tert-butyl, pentyl, hexyl, etc. are used, and particularly, a C₁₋₃ alkyl group such as methyl, ethyl, propyl and the like are preferable, particularly, methyl is preferable.

As the C₆₋₁₄ aryl group represented by R^{1e}, for example, phenyl, naphthyl, etc. are preferable, and of them, phenyl is particularly preferable.

As the halogen atom which is a substituent of the C₆₋₁₄ aryl group represented by R^{1e}, a fluorine atom, a chlorine atom, a bromine atom and an iodine atom are used.

As the C₇₋₁₅ aralkyl group represented by R^{1e}, for example, a phenyl-C₁₋₃ alkyl group such as benzyl, phenylethyl, phenylpropyl and the like are preferable.

As the C₁₋₆ alkyl group which is a substituent of an amino group represented by R^{1e}, for example, methyl, ethyl, propyl, isopropyl, butyl, isobutyl, sec-butyl, tert-butyl, pentyl, hexyl, etc. are used, and particularly, a C₁₋₃ alkyl group such as methyl, ethyl, propyl and the like are preferable, particularly, methyl is preferable.

As the C₁₋₆ alkyl-carbonyl which is a substituent of an amino group represented by R^{1e}, for example, a C₁₋₃ alkyl-carbonyl group such as acetyl, propionyl and the like are preferable.

As the "5- to 7-membered heterocyclic-carbonyl containing 1 to 4 hetero atoms selected from the group consisting of a nitrogen atom, an oxygen atom and a sulfur atom, in addition to carbon atoms" which is a substituent of an amino group represented by R^{1e}, for example, a 5- to 7-membered heterocyclic (e.g., pyridyl and the like)-carbonyl group containing 1 or 2 hetero atoms selected from the group consisting of a nitrogen atom, an oxygen atom and a sulfur atom, etc. are preferable. As the substituent of the heterocyclic group of this heterocyclic-carbonyl group, a halogen atom such as a chlorine atom, a C₁₋₆ alkyl group such as methyl, ethyl and the like, and a C₁₋₆ alkoxy group such as methoxy, ethoxy and the like are preferable.

As the C₆₋₁₄ aryl-carbamoyl which is a substituent of an amino group represented by R^{1e}, for example, phenyl-carbamoyl, etc. are preferable.

As the C₁₋₆ alkyl-carbamoyl which may be halogenated which is a substituent of an amino group represented by R^{1e}, for example, methylcarbamoyl, ethylcarbamoyl, propylcarbamoyl optionally substituted by a halogen atom (e.g., chlorine atom) and the like are preferable.

As the C₁₋₆ alkoxy-carbonyl which may be halogenated which is a substituent of an amino group represented by R^{1e}, for example, methoxycarbamoyl, ethoxycarbamoyl, propoxycarbamoyl optionally substituted by halogen atoms (e.g., chlorine atom) and the like are preferable.

As the C₁₋₆ alkoxy-carbamoyl which is a substituent of an amino group represented by R^{1e}, for example, methoxycarbamoyl, ethoxycarbamoyl, propoxycarbamoyl and the like are preferable.

As the C₆₋₁₄ aryloxy-carbamoyl which is a substituent of an amino group represented by R^{1e}, for example, phenyloxycarbamoyl and the like are preferable.

As the 5- to 10-membered non-aromatic heterocyclic group represented by R^{1e}, for example, 1-pyrrolidinyl, 2-pyrrolidinyl, 3-pyrrolidinyl, 3-oxazolidinyl, 1-imidazolidinyl, 2-imidazolidinyl, 4-imidazolidinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino and the like, are used, and of them, 4-piperidyl, 1-piperazinyl, 3-oxazolidinyl, 1-imidazolidinyl and the like are preferable.

As the 5- to 10-membered non-aromatic heterocyclic group optionally substituted by oxo, C₁₋₆ alkyl (preferably, methyl, ethyl), C₆₋₁₄ aryl (preferably, phenyl), C₁₋₆ alkyl-carbonyl (preferably, acetyl, propionyl) or C₁₋₆ alkoxy-carbonyl (preferably, methoxycarbonyl, ethoxycarbonyl, tert-butoxycarbonyl) represented by R^{1e}, 1-methyl-4-piperidyl, 4-methyl-1-piperazinyl, 2-oxo-3-oxazolidinyl, 2-oxo-1-imidazolidinyl, 2-oxo-3-phenyl-1-imidazolidinyl and the like are preferable.

As R^{5d} of the formula: -(C=O)-R^{5d} represented by R^{1e}, a hydrogen atom, a C₁₋₆ alkyl group which may be halogenated (methyl, ethyl, trifluoromethyl and the like), a C₆₋₁₄ alkyl group which may be halogenated (phenyl, naphthyl, fluorophenyl, chlorophenyl and the like), etc. are preferable.

As R^{5d} of the formula -(C=O)-OR^{5d} represented by R^{1e},, a hydrogen atom, a C₁₋₃ alkyl group (methyl, ethyl, etc.), etc. are preferable.

As R^{1e}, a C₁₋₆ alkyl group (e.g., methyl, ethyl, propyl, isopropyl, butyl, isobutyl, sec-butyl, tert-butyl, pentyl, hexyl, etc.) is preferable, particularly, a C₁₋₃ alkyl group such as methyl, ethyl, propyl, etc. is preferable.

As specific examples of a substituent of the pyridyl group represented by R^{2e}, 1 to 3, preferably 1 or 2 substituents selected from the following (1) to (6), particularly (1) to (4) are used.
(1) a C₁₋₆ alkyl group (e.g., methyl, ethyl, propyl, isopropyl, butyl, isobutyl, sec-butyl, tert-butyl, pentyl, hexyl and the like, preferably a C₁₋₄ alkyl group such as methyl, ethyl, propyl, butyl and the like): this C₁₋₆ alkyl group may be substituted by a halogen atom, cyano, hydroxy, C₃₋₈ cycloalkyl or 5- to 7-membered aliphatic heterocyclic group containing hetero atoms selected from a nitrogen atom, an oxygen atom and a sulfur atom in addition to carbon atoms (e.g., 1-pyrrolidinyl, 2-pyrrolidinyl, 3-pyrrolidinyl, piperidino, 2-piperidyl, 3-piperidyl, 4-piperidyl, 1-piperazinyl, 2-piperazinyl, 3-piperazinyl, 4-piperazinyl, morpholino, 2-morpholinyl, 3-morpholinyl, thiomorpholino, 2-thiomorpholinyl, 3-thiomorpholinyl, hexahydroazepin-1-yl and the like),
(2) a halogen atom (e.g., fluorine atom, chlorine atom, bromine atom, iodine atom),
(3) an amino group optionally having a substituent selected from the group consisting of the following 1) to 6):
   1) a C₁₋₆ alkyl group (e.g., methyl, ethyl, propyl, isopropyl, butyl, isobutyl, sec-butyl, tert-butyl, pentyl, hexyl and the like, preferably a C₁₋₃ alkyl group such as methyl, ethyl, propyl and the like): this C₁₋₆ alkyl group may be substituted by a halogen atom, cyano, hydroxy, C₃₋₈ cycloalkyl or 5- to 7-membered aliphatic heterocyclic group containing 1 to 4 hetero atoms selected from a nitrogen atom, an oxygen atom and a sulfur atom in addition to carbon atoms (e.g., 1-pyrrolidinyl, 2-pyrrolidinyl, 3-pyrrolidinyl, piperidino, 2-piperidyl, 3-piperidyl, 4-piperidyl, 1-piperazinyl, 2-piperazinyl, 3-piperazinyl, 4-piperazinyl, morpholino, 2-morpholinyl, 3-morpholinyl, thiomorpholino, 2-thiomorpholinyl, 3-thiomorpholinyl, hexahydroazepin-1-yl and the like),
   2) a C₃₋₈ cycloalkyl group (e.g., cyclopropyl, cyclobutyl, cyclopentyl, cyclohexyl and the like),
   3) a C₁₋₆ alkyl-carbonyl group (e.g., acetyl, propionyl, butyryl, valeryl, isovaleryl, 2-methylbutyryl, pivaloyl and the like): this C₁₋₆ alkyl-carbonyl group may be substituted by a halogen atom, cyano, hydroxy, C₃₋₈ cycloalkyl or 5- to 7-membered aliphatic heterocyclic group containing 1 to 4 hetero atoms selected from a nitrogen atom, an oxygen atom and a sulfur atom in addition to carbon atoms (e.g., 1-pyrrolidinyl, 2-pyrrolidinyl, 3-pyrrolidinyl, piperidino, 2-piperidyl, 3-piperidyl, 4-piperidyl, 1-piperazinyl, 2-piperazinyl, 3-piperazinyl, 4-piperazinyl, morpholino, 2-morpholinyl, 3-morpholinyl, thiomorpholino, 2-thiomorpholinyl, 3-thiomorpholinyl, hexahydroazepin-1-yl and the like),
   4) a C₁₋₆ alkoxy-carbonyl group (e.g., methoxycarbonyl, ethoxycarbonyl, propoxycarbonyl, butoxycarbonyl, isobutoxycarbonyl, sec-butoxycarbonyl, tert-butoxycarbonyl and the like),
   5) a C₃₋₈ cycloalkyl-carbonyl group optionally substituted by C₁₋₆ alkyl such as methyl, ethyl (e.g., cyclopropylcarbonyl, cyclobutylcarbonyl, cyclopentylcarbonyl, cyclohexylcarbonyl, 1-methyl-cyclohexylcarbonyl and the like),
   6) a 5- to 7-membered aliphatic heterocyclic group having 1 to 4 hetero atoms selected from a nitrogen atom, an oxygen atom and a sulfur atom in addition to carbon atoms (e.g., 1-pyrrolidinyl, 2-pyrrolidinyl, 3-pyrrolidinyl, piperidino, 2-piperidyl, 3-piperidyl, 4-piperidyl, 1-piperazinyl, 2-piperazinyl, 3-piperazinyl, 4-piperazinyl, morpholino, 2-morpholinyl, 3-morpholinyl, thiomorpholino, 2-thiomorpholinyl, 3-thiomorpholinyl, hexahydroazepin-1-yl and the like). This aliphatic heterocyclic group may be substituted by C₁₋₆ alkyl (e.g., methyl, ethyl) or C₁₋₆ alkyl-carbonyl (e.g., acetyl, propionyl),
   7) a 5- to 7-membered aliphatic heterocyclic (e.g., 1-pyrrolidinyl, 2-pyrrolidinyl, 3-pyrrolidinyl, piperidino, 2-piperidyl, 3-piperidyl, 4-piperidyl, 1-piperazinyl, 2-piperazinyl, 3-piperazinyl, 4-piperazinyl, morpholino, 2-morpholinyl, 3-morpholinyl, thiomorpholino, 2-thiomorpholinyl, 3-thiomorpholinyl, hexahydroazepin-1-yl and the like)-carbonyl group having 1 to 4 hetero atoms selected from a nitrogen atom, an oxygen atom and a sulfur atom in addition to carbon atoms. This aliphatic heterocyclic-carbonyl group may be substituted by C₁₋₆ alkyl (e.g., methyl, ethyl) or C₁₋₆ alkyl-carbonyl (e.g., acetyl, propionyl),
(4) a 5- or 7-membered saturated cyclic amino group which may further contain 1 to 4 hetero atoms selected from a nitrogen atom, an oxygen atom and a sulfur atom in addition to carbon atoms and one nitrogen atom (e.g., 1-pyrrolidinyl, piperidino, morpholino, 1-piperazinyl and the like). This saturated cyclic amino group may be substituted with C₁₋₆ alkyl (e.g., methyl, ethyl), and the like.
(5) a hydroxyl group,
(6) a C₁₋₆ alkyl-carbonyloxy group (e.g., acetyloxy, propionyloxy, butyryloxy and the like).

Of them, as a substituent of the pyridyl group represented by R^{2b}, for example, a C₁₋₆ alkyl-carbonylamino group (e.g., acetylamino, propionylamino, butyrylamino, valerylamino, isovalerylamino, 2-methylbutyrylamino, pivaloylamino and the like) is preferable, and particularly, a C₁₋₃ alkyl-carbonylamino group such as acetylamino, propionylamino, etc. is preferable.

As the C₆₋₁₄ aryl group represented by R^{3e}, for example, phenyl, naphthyl, etc. are preferable, and of them, phenyl is particularly preferable.

As the 5- to 14-membered aromatic heterocyclic group preferably containing 1 to 4 of 1 or 2 kinds of hetero atoms selected from a nitrogen atom, a sulfur atom and an oxygen atom in addition to carbon atoms, represented by R^{3e}, for example, a 5- or 6-membered aromatic heterocyclic group preferably containing 1 to 4 of 1 or 2 kinds of hetero atoms selected from a nitrogen atom, a sulfur atom and an oxygen atom in addition to carbon atoms, such as thienyl and the like, are preferable.

As the C₁₋₆ alkyl group which may be halogenated, which is a substituent of the C₆₋₁₄ aryl group or the aromatic heterocyclic group, for example, methyl, ethyl, propyl, isopropyl, butyl, isobutyl, sec-butyl, tert-butyl, pentyl, hexyl, etc. which may be substituted by a halogen atom (e.g., fluorine, chlorine and the like) are used, and particularly, a C₁₋₃ alkyl group which may be halogenated such as methyl, ethyl, propyl, trifluoromethyl and the like are preferable.

As the C₁₋₆ alkoxy which is a substituent of the C₆₋₁₄ aryl group or the aromatic heterocyclic group, for example, methoxy, ethoxy, propoxy, etc. are used, and of them, methoxy is particularly preferable.

As the halogen atom which is a substituent of the C₆₋₁₄ aryl group or the aromatic heterocyclic group, a fluorine atom, a chlorine atom, a bromine atom and an iodine atom are used, and of them, a fluorine atom and a chlorine atom, etc. are preferable.

As the C₁₋₆ alkoxy-carbonyl which is a substituent of the C₆₋₁₄ aryl group or the aromatic heterocyclic group, for example, methoxycarbonyl, ethoxycarbonyl and the like are preferable.

As the C₁₋₆ alkylthio which is a substituent of the C₆₋₁₄ aryl group for example, methylthio, ethylthio and the like are preferable.

As the C₁₋₆ alkylsulfonyl which is a substituent of the C₆₋₁₄ aryl group, for example, methylsulfonyl, ethylsulfonyl and the like are preferable.

As R^{3e}, a C₆₋₁₄ aryl group optionally having a substituent selected from the group consisting of C₁₋₆ alkyl and a halogen atom is preferable, and a phenyl group optionally substituted by methyl or a chlorine atom is more preferable.

As the compound (VIa), for example,
(1) the compound (VIa) wherein R^{1e} is a C₁₋₆ alkyl group optionally having a substituent selected from the group consisting of a halogen atom, hydroxy, C₁₋₆ alkoxy, C₁₋₆ alkylthio, C₁₋₆ alkylsulfinyl and C₁₋₆ alkylsulfonyl, R^{2e} is a C₁₋₆ alkyl-carbonylamino group or a C₃₋₈ cycloalkylamino group, R^{3e} is a C₆₋₁₄ aryl group optionally having a substituent selected from the group consisting of C₁₋₆ alkyl and a halogen atom,
(2) the compound (VIa) wherein R^{1e} is a C₁₋₃ alkyl group optionally having a substituent selected from the group consisting of a halogen atom, hydroxy, C₁₋₆ alkoxy, C₁₋₆ alkylthio, C₁₋₆ alkylsulfinyl and C₁₋₆ alkylsulfonyl, R^{2e} is a C₁₋₃ alkyl-carbonylamino group or a C₃₋₈ cycloalkylamino group, R^{3e} is a phenyl group optionally having a substituent selected from the group consisting of methyl or a chlorine atom, and the like are preferable.

As preferable specific examples of the compound (VIa), compounds produced in Reference Examples H 1 to 113 described later are exemplified, and of them, 5-[2-(tert-butoxycarbonylamino)-4-pyridyl]-2-ethyl-4-(3-methylphenyl)-1,3-thiazol (Reference Example H 3), [4-(3-methylphenyl)-5-(2-methyl-4-pyridyl)-1,3-thiazol-2-yl]amine (Reference Example H 7-4), 2-ethyl-5-(2-fluoro-4-pyridyl)-4-(3-methylphenyl)-1,3-thiazole (Reference Example H 11), 5-(2-fluoro-4-pyridyl)-4-(3-methylphenyl)-2-[4-(methylthio)phenyl]-1,3-thiazole (Reference Example H 15), 4-(3-methylphenyl)-5-(2-methyl-4-pyridyl)-2-[4-(methylthio)phenyl]-1,3-thiazole (Reference Example H 16-1), 4-[2-ethyl-4-(3-methylphenyl)-1,3-thiazol-5-yl]-2-pyridylamine (Reference Example H 22), N-[4-[2-ethyl-4-(3-methylphenyl)-1,3-thiazol-5-yl]-2-pyridyl)acetamide (Reference Example H 29-2), N-[4-[2-ethyl-4-(3-methylphenyl)-1,3-thiazol-5-yl]-2-pyridyl]propionamide (Reference Example H 29-4), N-[4-[4-(3-chlorophenyl)-2-methyl-1,3-thiazol-5-yl]-2-pyridyl]acetamide (Reference Example H 30-1), N-[4-[4-(3-chlorophenyl)-2-ethyl-1,3-thiazol-5-yl]-2-pyridyl]acetamide (Reference Example H 30-2), N-[4-[4-(3-chlorophenyl)-2-propyl-1,3-thiazol-5-yl]-2-pyridyl]acetamide (Reference Example H 30-3), N-[4-[4-(3-chlorophenyl)-2-methyl-1,3-thiazol-5-yl]-2-pyridyl]propionamide (Reference Example H 30-7), N-[4-[4-(3-chlorophenyl)-2-ethyl-1,3-thiazol-5-yl]-2-pyridyl]propionamide (Reference Example H 30-8), N-[4-[4-(3-chlorophenyl)-2-propyl-1,3-thiazol-5-yl]-2-pyridyl]propionamide (Reference Example H 30-9), N-cyclohexyl-4-[2-ethyl-4-(3-methylphenyl)-1,3-thiazol-5-yl]-2-pyridylamine (Reference Example H 36-4), N-cyclohexyl-4-[4-(3-methylphenyl)-2-(4-methylsulfonylphenyl)-1,3-thiazol-5-yl]-2-pyridylamine (Reference Example H 36-5), N-cyclopentyl-4-[2-ethyl-4-(3-methylphenyl)-1,3-thiazol-5-yl]-2-pyridylamine (Reference Example H 36-6), N-cyclopentyl-4-[4-(3-methylphenyl)-2-(4-methylsulfonylphenyl)-1,3-thiazol-5-yl]-2-pyridylamine (Reference Example H 36-7), 4-[4-(3-chlorophenyl)-2-ethyl-1,3-thiazol-5-yl]-N-cyclohexyl-2-pyridylamine (Reference Example H 36-10), 4-[4-(3-chlorophenyl)-2-ethyl-1,3-thiazol-5-yl]-N-cyclopentyl-2-pyridylamine (Reference Example H 36-11), N-[4-(3-methylphenyl)-5-(2-methyl-4-pyridyl)-1,3-thiazol-2-yl]acetamide (Reference Example H 39), N-[4-(3,5-dimethylphenyl)-5-(2-methyl-4-pyridyl)-1,3-thiazol-2-yl]nicotinamide (Reference Example H 42-1), 6-chloro-N-[4-(3,5-dimethylphenyl)-5-(2-methyl-4-pyridyl)-1,3-thiazol-2-yl]nicotinamide (Reference Example H 44-3), N-[4-(3,5-dimethylphenyl)-5-(2-methyl-4-pyridyl)-1,3-thiazol-2-yl]-6-methylnicotinamide (Reference Example H 46-3), N-[4-(3,5-dimethylphenyl)-5-(2-methyl-4-pyridyl)-1,3-thiazol-2-yl]-6-methoxynicotinamide (Reference Example H 48-3), 4-(3-methylphenyl)-5-(2-methyl-4-pyridyl)-2-(4-methylsulfinylphenyl)-1,3-thiazole (Reference Example H 54), 4-(3-methylphenyl)-5-(2-methyl-4-pyridyl)-2-(4-methylsulfonylphenyl)-1,3-thiazole (Reference Example H 57), 5-(2-fluoro-4-pyridyl)-4-(3-methylphenyl)-2-(4-methylsulfonylphenyl)-1,3-thiazole (Reference Example H 58-4), N-[4-[4-(3-chlorophenyl)-2-(4-methylsulfonylphenyl)-1,3-thiazol-5-yl]-2-pyridyl]acetamide (Reference Example H 58-6), N-[4-[4-(3-chlorophenyl)-2-(4-methylsulfonylphenyl)-1,3-thiazol-5-yl]-2-pyridyl]propionamide (Reference Example H 58-7), N-[4-(4-(3-chlorophenyl)-2-(4-methylsulfonylphenyl)-1,3-thiazol-5-yl]-2-pyridyl]pivalamide (Reference Example H 58-8) and the like are preferable.

The compounds (IV), (V) and (VI) of the present invention do not include N-[4-(3,5-dimethylphenyl)-5-(2-hydroxy-4-pyridyl)-1,3-thiazol-2-yl]acetamide and 4-[2-(acetylamino)-4-(3,5-dimethylphenyl)-1,3-thiazol-2-yl]-2-pyridyl acetate.

As a salt of the compound (IV), (V) or (VI) of the present invention, for example, metal salts, ammonium salts, salts with organic bases, salts with inorganic acids, salts with organic acids, salts with basic or acidic amino acids, etc. are exemplified. As suitable examples of metal salts, for example, alkali metal salts such as sodium salts, potassium salts and the like; alkaline earth metal salts such as potassium salts, magnesium salts, barium salts and the like; aluminum salts; etc. are exemplified. As suitable examples of salts with organic bases, for example, salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc. are exemplified. As suitable examples of salts with inorganic acids, for example, salts with hydrochloric acid, hydrobromic acid, nitric acid, sulfuric acid, phosphoric acid, etc. are exemplified. As suitable examples of salts with organic acids, for example, salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, etc. are exemplified. As suitable examples of salts with basic amino acids, for example, salts with arginine, lysine, ornithine, etc. are exemplified, and as suitable examples of salts with acidic amino acids, for example, salts with aspartic acid, glutamic acid, etc. are exemplified.

Of them, pharmaceutically acceptable salts are preferable. When an acidic functional group is contained in a compound, for example, inorganic salts such as alkali metal salts (e.g., sodium salts, potassium salts and the like), alkaline earth metal salts (e.g., calcium salts, magnesium salts, barium salts and the like), and ammonium salts, etc. are exemplified, and when a basic functional group is contained in a compound, for example, salts with inorganic acids such as hydrochloric acid, hydrobromic acid, nitric acid, sulfuric acid, phosphoric acid, etc. or salts with organic acids such as acetic acid, phthalic acid, fumaric acid, oxalic acid, tartaric acid, maleic acid, citric acid, succinic acid, methanesulfonic acid; p-toluenesulfonic acid, etc. are preferable.

A method for producing the compound (IV), (V), (VI) or a salt thereof of the present invention will be illustrated below. Hereinafter, the compound (I') means a compound including compounds (IV), (V), (VI), (Va) and (VIa).

The compound (I) of the present invention is obtained by a method represented by the following reaction formula 1 or methods according to this method, and additionally, obtained, for example, by methods described in JP-A No. 60-58981, JP-A No. 61-10580, JP-T No. 7-503023, WO 93/15071, DE-A-3601411, JP-A No. 5-70446 and methods according to them.

Symbols of compounds in the following reaction formulae 1 to 2 are as defined above. Compounds in the reaction formulae also include salts, and as this salt, for example, the same salts as for the compound (IV) are exemplified.

### Reaction Formula 1

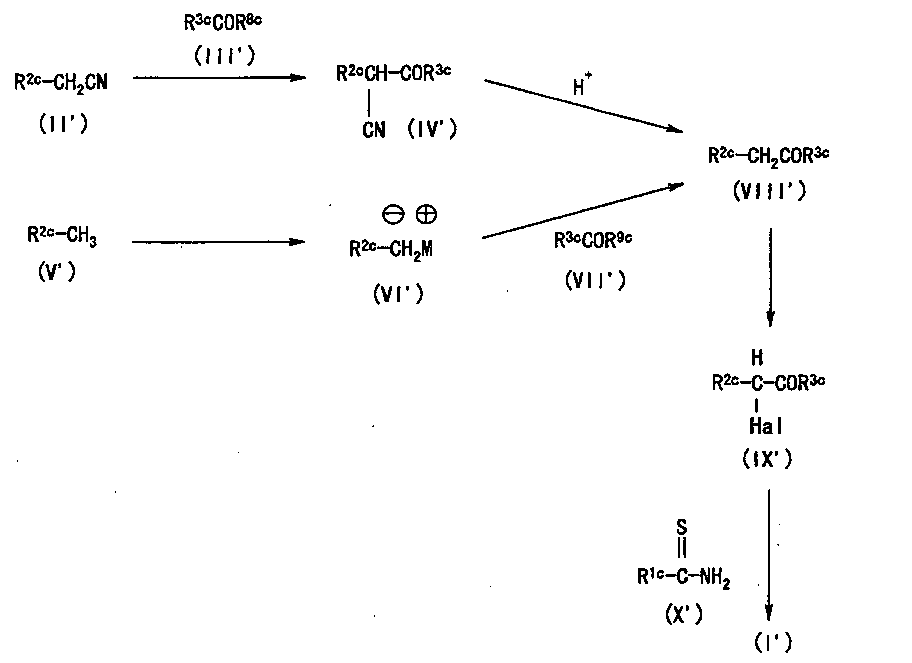

When compounds (II'), (III'), (V'), (VII'), (XI'), (XIII') and (XIV') are commercially available, they may be used without any treatment, and also can be produced by methods known per se or methods according to them.

The compound (IV') is obtained by condensing a compound (II') with a compound (III') in the presence of a base.

In the compound (III'), R^{8c} represents, for example, (1) a C₁₋₆ alkoxy (e.g., methoxy, ethoxy and the like), (2) a di-C₁₋₆ alkylamino (e.g., dimethylamino, diethylamino and the like) , (3) an N-C₆₋₁₀ aryl-N-C₁₋₆ alkylamino (e.g., N-phenyl-N-methylamino and the like), (4) a 3- to 7-membered saturated cyclic amino optionally substituted with a C₆₋₁₀ aryl and (or) C₁₋₆ alkyl (e.g., pyrrolidin-1-yl, morpholino, methylaziridin-1-yl, and the like), etc.

The amount of the compound (III') used is about 0.5 to about 3.0 mol, preferably about 0.8 to about 2.0 mol per mol of the compound (II').

The amount of a base used is about 1.0 to about 30 mol, preferably about 1.0 to about 10 mol per mol of the compound (II').

As the "base", for example, basic salts such as sodium carbonate, potassium carbonate, cesium carbonate and the like, inorganic bases such as sodium hydroxide, potassium hydroxide and the like, aromatic amines such as pyridine, lutidine and the like, tertiary amines such triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, alkali metal hydrides such as sodium hydride, potassium hydride and the like, metal amides such as sodium amide, lithium diisopropylamide, lithium hexamethyldisilazide and the like, metal alkoxides such as sodium methoxide, sodium ethoxide, potassium tert-butoxide and the like, etc. are exemplified.

The present reaction is advantageously conducted in the absence or presence of a solvent inactive to the reaction. This solvent is not particularly restricted providing the reaction can progress, and for example, halogenated hydrocarbons, aliphatic hydrocarbons, aromatic hydrocarbons, ethers, amides, alcohols, water or mixtures of two or more of them, etc. are used.

The reaction temperature is usually from about -5°C to about 200°C, preferably from about 5°C to about 150°C. The reaction time is usually from about 5 minutes to about 72 hours, preferably from about 0.5 to about 30 hours.

The product can be used in the following reaction as the reaction solution itself or as a crude product, and can also be isolated from the reaction mixture according to an ordinary method, and can be easily purified by separation means such as recrystallization, distillation, chromatography and the like.

A compound (VIII') is obtained by treating a compound (IV') with an acid.

The amount of an acid used is about 1.0 to about 100 mol, preferably about 1.0 to about 30 mol per mol of the compound (IV').

As the "acid", for example, mineral acids such as hydrochloric acid, hydrobromic acid, sulfuric acid, etc. are used.

The present reaction is conducted in the presence of a solvent inactive to the reaction. The solvent is not particularly restricted providing the reaction can progress, and for example, water, mixtures of water with amides, mixtures of water with alcohols, etc. are used.

The reaction temperature is usually from about 20°C to about 200°C, preferably from about 60°C to about 150°C. The reaction time is usually from about 30 minutes to about 72 hours, preferably from about 1 hour to about 30 hours.

The product can be used in the following reaction as the reaction solution itself or as a crude product, and can also be isolated from the reaction mixture according to an ordinary method, and can be easily purified by separation means such as recrystallization, distillation, chromatography and the like.

A compound (VIII') can also be obtained by condensing a compound (VII') with a compound (VI') obtained by treating a compound (V') with a base.

In the compound (VI'), M represents, for example, an alkali metal such as lithium, sodium, potassium and the like.

In the compound (VII'), as R⁹, for example, the same moieties as for R⁸ are exemplified.

The amount of a base used is about 1.0 to about 30 mol, preferably about 1.0 to about 10 mol per mol of the compound (V').

As the "base", for example, metal amides such as sodium amide, lithium diisopropylamide, lithium hexamethyldisilazide and the like, and alkyl metal compounds such as alkyllithium and the like are used.

The present reaction is advantageously conducted in the absence or presence of a solvent inactive to the reaction. This solvent is not particularly restricted providing the reaction can progress, and for example, aliphatic hydrocarbons, aromatic hydrocarbons, ethers, or mixtures of two or more of them, etc. are used.

The reaction temperature is usually from about -78°C to about 60°C, preferably from about -78°C to about 20°C. The reaction time is usually from about 5 minutes to about 24 hours, preferably from about 0.5 to about 3 hours.

The product can be used in the following reaction as the reaction solution itself or as a crude product, and can also be isolated from the reaction mixture according to an ordinary method, and can be easily purified by separation means such as recrystallization, distillation, chromatography and the like.

A compound (IX') is obtained by treating a compound (VIII') with halogens. This reaction is conducted in the presence of a base or basic salt, if necessary.

In the compound (IX'), Hal represents halogens.

The amount of halogens used is about 1.0 to about 5.0 mol, preferably about 1.0 to about 2.0 mol per mol of the compound (VIII').

As the "halogens", bromine, chlorine, iodine, etc. are exemplified.

The amount of a base used is about 1.0 to about 10.0 mol, preferably about 1.0 to about 3.0 mol per mol of the compound (VIII').

As the "base", for example, aromatic amines such as pyridine, lutidine and the like, tertiary amines such triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, etc. are exemplified.

The amount of a basic salt used is about 1.0 to about 10.0 mol, preferably about 1.0 to about 3.0 mol per mol of the compound (VIII').

As the "basic salt", for example, sodium carbonate, potassium carbonate, cesium carbonate, sodium hydrogen carbonate, sodium acetate, potassium acetate, etc. are exemplified.

The present reaction is advantageously conducted in the absence or presence of a solvent inactive to the reaction. This solvent is not particularly restricted providing the reaction can progress, and for example, ethers, aromatic hydrocarbons, aliphatic hydrocarbons, amides, halogenated hydrocarbons, nitriles, sulfoxides, organic acids, aromatic amines, or mixtures of two or more of them, etc. are used.

The reaction temperature is usually from about -20°C to about 150°C, preferably from about 0°C to about 100°C. The reaction time is usually from about 5 minutes to about 24 hours, preferably from about 10 minutes to about 5 hours.

The product can be used in the following reaction as the reaction solution itself or as a crude product, and can also be isolated from the reaction mixture according to an ordinary method, and can be easily purified by separation means such as recrystallization, distillation, chromatography and the like.

A compound (I') can be obtained by condensing a compound (IX') with a compound (X'). The present reaction is conducted in the presence of a base, if necessary.

When the compound (X') is commercially available, it may be used without any treatment, and also can be obtained by methods known per se or methods according to them, further, can be obtained by a method of the following reaction formula 2, etc.

The amount of the compound (X') used is about 0.5 to about 3.0 mol, preferably about 0.8 to about 2.0 mol per mol of the compound (IX').

The amount of a base used is about 1.0 to about 30 mol, preferably about 1.0 to about 10 mol per mol of the compound (IX').

As the "base", for example, basic salts such as sodium carbonate, potassium carbonate, cesium carbonate, sodium hydrogen carbonate and the like, aromatic amines such as pyridine, lutidine and the like, tertiary amines such triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, etc. are exemplified.

The present reaction is advantageously conducted in the absence or presence of a solvent inactive to the reaction. This solvent is not particularly restricted providing the reaction can progress, and for example, halogenated hydrocarbons, aliphatic hydrocarbons, aromatic hydrocarbons, ethers, amides, alcohols, nitriles, or mixtures of two or more of them, etc. are used.

The reaction temperature is from about -5°C to about 200°C, preferably from about 5°C to about 150°C. The reaction time is usually from about 5 minutes to about 72 hours, preferably from about 0.5 to about 30 hours.

The product can be used in the following reaction as the reaction solution itself or as a crude product, and can also be isolated from the reaction mixture according to an ordinary method, and can be easily purified by separation means such as recrystallization, distillation, chromatography and the like.

### Reaction Formula 2

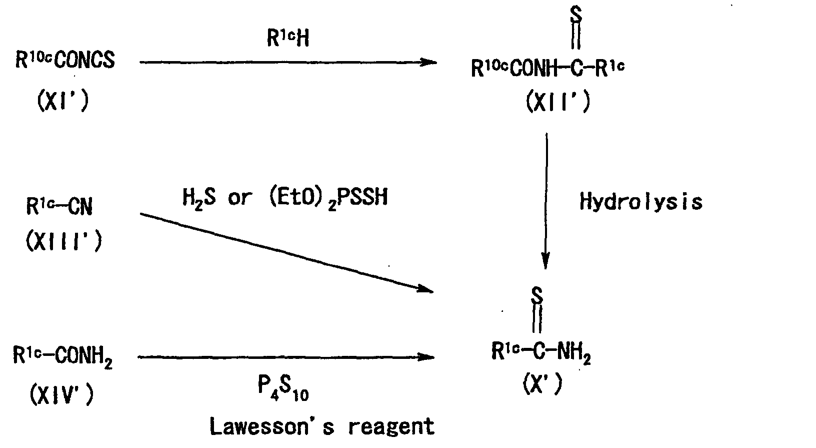

A compound (XII') can be obtained by condensing a compound (XI') with amines represented by R^{1c}H.

In the compound (XI'), R^{10c} represents an aromatic hydrocarbon group or alkoxy. As the "aromatic hydrocarbon group", a phenyl group optionally having a substituent, etc. are listed. As the "alkoxy", for example, C₁₋₆ alkoxys such as methoxy, ethoxy, propoxy, isopropoxy, butoxy and the like, etc. are exemplified.

The amount of the "amines" used is about 1.0 to about 30 mol, preferably about 1.0 to about 10 mol per mol of the compound (XI').

The present reaction is advantageously conducted in the absence or presence of a solvent inactive to the reaction. This solvent is not particularly restricted providing the reaction can progress, and for example, halogenated hydrocarbons, aliphatic hydrocarbons, aromatic hydrocarbons, ethers, amides, alcohols, nitriles, ketones, or mixtures of two or more of them, etc. are used.

The reaction temperature is from about -5°C to about 200°C, preferably from about 5°C to about 120°C. The reaction time is usually from about 5 minutes to about 72 hours, preferably from about 0.5 to about 30 hours.

The product can be used in the following reaction as the reaction solution itself or as a crude product, and can also be isolated from the reaction mixture according to an ordinary method, and can be easily purified by separation means such as recrystallization, distillation, chromatography and the like.

A compound (X') can be obtained by hydrolyzing the compound (XII') with an acid or base.

The amount of the acid or base used is about 0.1 to about 50 mol, preferably about 1 to about 20 mol, respectively, per mole of the compound (XII').

As the "acid", for example, mineral acids such as hydrochloric acid, hydrobromic acid, sulfuric acid and the like, Lewis acids such as boron trichloride, boron tribromide and the like, co-use of Lewis acids with thiols or sulfides, and organic acids such as trifluoroacetic acid, p-toluenesulfonic acid and the like, etc. are used.

As the "base", for example, metal hydroxides such as sodium hydroxide, potassium hydroxide, barium hydroxide and the like, basic salts such as sodium carbonate, potassium carbonate and the like, metal alkoxides such as sodium methoxide, sodium ethoxide, potassium tert-butoxide and the like, organic bases such as triethylamine, imidazole, formamidine and the like, etc. are used.

The present reaction is advantageously conducted in the absence or presence of a solvent inactive to the reaction. This solvent is not particularly restricted providing the reaction can progress, and for example, alcohols, ethers, aromatic hydrocarbons, aliphatic hydrocarbons, halogenated hydrocarbons, sulfoxides, water or mixtures of two or more of them, etc. are used.

The reaction time is usually from about 10 minutes to about 50 hours, preferably from about 30 minutes to about 12 hours. The reaction temperature is usually from about 0°C to about 200°C, preferably from about 20°C to about 120°C.

A compound (X') can also be obtained by treating a compound (XIII') with hydrogen sulfide in the presence of a base.

The amount of hydrogen sulfide used is about 1 to about 30 mol per mol of the compound (XIII').

The amount of a base used is about 1.0 to about 30 mol, preferably about 1,0 to about 10 mol per mol of the compound (XIII').

As the "base", for example, aromatic amines such as pyridine, lutidine and the like, tertiary amines such triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, etc. are used.

The present reaction is advantageously conducted in the absence or presence of a solvent inactive to the reaction. This solvent is not particularly restricted providing the reaction can progress, and for example, halogenated hydrocarbons, aliphatic hydrocarbons, aromatic hydrocarbons, ethers, aromatic amines, or mixtures of two or more of them, etc. are used.

The present reaction is effected under normal pressure or positive pressure. The reaction temperature is usually from about -20°C to about 80°C, preferably from about -10°C to about 30°C. The reaction time is usually from about 5 minutes to about 72 hours, preferably from about 0.5 to about 30 hours.

The product can be used in the following reaction as the reaction solution itself or as a crude product, and can also be isolated from the reaction mixture according to an ordinary method, and can be easily purified by separation means such as recrystallization, distillation, chromatography and the like.

A compound (X') can also be obtained by treating a compound (XIII') with dithiophosphoric acid 0,0-diethyl ester in the presence of an acid.

The amount of dithiophosphoric acid 0,0-diethyl ester used is about 0.9 to about 2 mol per mole of the compound (XIII').

The amount of an acid used is about 3.0 to about 30 mol, preferably about 3.0 to about 10 mol per mol of the compound (XIII').

As the "acid", for example, hydrogen halides such as hydrogen chloride, hydrogen bromide and the like, and mineral acids such as hydrochloric acid, hydrobromic acid and the like, etc. are exemplified.

The present reaction is advantageously conducted in the absence or presence of a solvent inactive to the reaction. This solvent is not particularly restricted providing the reaction can progress, and for example, halogenated hydrocarbons, alcohols, amides, ethers, esters, water, or mixtures of two or more of them, etc. are used.

The reaction temperature is usually from about 0°C to about 80°C, preferably from about 0°C to about 30°C. The reaction time is usually from about 5 minutes to about 72 hours, preferably from about 0.5 hours to about 30 hours.

The product can be used in the following reaction as the reaction solution itself or as a crude product, and can also be isolated from the reaction mixture according to an ordinary method, and can be easily purified by separation means such as recrystallization, distillation, chromatography and the like.

A compound (X') can also be obtained by treating a compound (XIV') with phosphorus pentasulfide or a Lawesson's reagent.

The amount of the phosphorus pentasulfide or Lawesson's reagent used is about 0.5 to about 10 mol, preferably about 0.5 to about 3 mol per mole of the compound (XIV').

The present reaction is advantageously conducted in the absence or presence of a solvent inactive to the reaction. This solvent is not particularly restricted providing the reaction can progress, and for example, ethers, aromatic hydrocarbons, aliphatic hydrocarbons, halogenated hydrocarbons, or mixtures of two or more of them, etc. are used.

The reaction time is usually from about 10 minutes to about 50 hours, preferably from about 30 minutes to about 12 hours. The reaction temperature is usually from about 0°C to about 150°C, preferably from about 20°C to about 120°C.

The product (X') can be used in the following reaction as the reaction solution itself or as a crude product, and can also be isolated from the reaction mixture according to an ordinary method, and can be easily purified by separation means such as recrystallization, distillation, chromatography and the like.

When the compound (I') is an acylamino compound, the corresponding amine can be subjected to an acylation reaction known per se to obtain the intended substance.

For example, of compounds (I'), that in which a substituent at the 2-position of a thiazole ring is acylamino optionally having a substituent is obtained by reacting the corresponding 2-thiazolamine and acylating agent, if necessary, in the presence of a base or acid.

The amount of the acylating agent used is about 1.0 to about 5.0 mol, preferably about 1.0 to about 2.0 mol per mol of the corresponding 2-thiazolamine.

As the "acylating agent", for example, carboxylic acids corresponding to the acyl group of the intended substance, or reactive derivatives thereof (e.g., acid halide, acid anhydride, ester and the like), etc. are exemplified.

The amount of a base or acid used is about 0.8 to about 5.0 mol, preferably about 1.0 to about 2.0 mol per mol of the corresponding 2-thiazolamine.

As the "base", for example, triethylamine, pyridine, 4-dimethylaminopyridine, etc. are exemplified.

As the "acid", for example, methanesulfonic acid, p-toluenesulfonic acid, camphor-sulfonic acid, etc. are exemplified.

The present reaction is advantageously conducted in the absence or presence of a solvent inactive to the reaction. This solvent is not particularly restricted providing the reaction can progress, and for example, ethers, aromatic hydrocarbons, aliphatic hydrocarbons, amides, halogenated hydrocarbons, nitriles, sulfoxides, aromatic amines, or mixtures of two or more of them, etc. are used.

The reaction temperature is from about -20°C to about 150°C, preferably from about 0°C to about 100°C. The reaction time is usually from about 5 minutes to about 24 hours, preferably from about 10 minutes to about 5 hours.

The product can be used in the following reaction as the reaction solution itself or as a crude product, and can also be isolated from the reaction mixture according to an ordinary method, and can be easily purified by separation means such as recrystallization, distillation, chromatography and the like.

Further, of compounds (I'), that in which a substituent at the 5-position of a thiazole ring is acylaminopyridyl optionally having a substituent is obtained by reacting the corresponding 5-(2-aminopyridyl)thiazole and acylating agent, if necessary in the presence of a base or acid.

The amount of the acylation agent used is from about 1.0 to about 5.0 mol, preferably from about 1.0 to about 2.0 mol per mol of the corresponding 5-(2-aminopyridyl)thiazole.

As the "acylating agent", for example, carboxylic acids corresponding to the acyl group of the intended substance, or reactive derivatives thereof (e.g., acid halide, acid anhydride, ester and the like), etc. are exemplified.

The amount of a base or acid used is from about 0.8 to about 5.0 mol, preferably from about 1.0 to about 2.0 mol per mol of the corresponding 5-(2-aminopyridyl)thiazole.

As the "base", for example, triethylamine, pyridine, 4-dimethylaminopyridine, etc. are exemplified.

As the "acid", for example, methanesulfonic acid, p-toluenesulfonic acid, camphor-sulfonic acid, etc. are exemplified.

The present reaction is advantageously conducted in the absence or presence of a solvent inactive to the reaction. This solvent is not particularly restricted providing the reaction can progress, and for example, ethers, aromatic hydrocarbons, aliphatic hydrocarbons, amides, halogenated hydrocarbons, nitriles, sulfoxides, aromatic amines, or mixtures of two or more of them, etc. are used.

The reaction temperature is from about -20°C to about 150°C, preferably from about 0°C to about 100°C. The reaction time is usually from about 5 minutes to about 24 hours, preferably from about 10 minutes to about 5 hours.

The product can be used in the following reaction as the reaction solution itself or as a crude product, and can also be isolated from the reaction mixture according to an ordinary method, and can be easily purified by separation means such as recrystallization, distillation, chromatography and the like.

Of compounds (I'), that in which a substituent at the 5-position of a thiazole ring is alkylaminopyridyl optionally having a substituent is obtained by reducing the corresponding acylaminopyridine with a reducing agent.

The amount of the reducing agent used is from about 1.0 to about 5.0 mol, preferably from about 1.0 to about 3.0 mol per mol of the corresponding acylaminopyridine.

As the "reducing agent", for example, metal hydrides such as aluminum hydride, diisobutylaluminum hydride and the like, metal hydrogen complex compounds such as lithium aluminum hydride, sodium boron hydride and the like, borane complexes such as borane tetrahydrofuran complex, borane dimethylsulfide complex and the like, alkyl boranes such as thexyl borane, dicyamyl borane and the like, etc. are exemplified.

In the present reaction, an acid is also added together with a reducing agent, if necessary.

The amount of an acid used is from about 0.8 to about 5.0 mol, preferably from about 1.0 to about 3.0 mol per mol of the corresponding acylaminopyridine.

As the "acid", for example, Lewis acids such as aluminum chloride and the like, are exemplified.

The present reaction is advantageously conducted in the absence or presence of a solvent inactive to the reaction. This solvent is not particularly restricted providing the reaction can progress, and for example, ethers, aromatic hydrocarbons, aliphatic hydrocarbons, halogenated hydrocarbons, or mixtures of two or more of them, etc. are used.

The reaction temperature is from about -78°C to about 150°C, preferably from about 0°C to about 100°C. The reaction time is usually from about 5 minutes to about 24 hours, preferably from about 10 minutes to about 5 hours.

The product can be used in the following reaction as the reaction solution itself or as a crude product, and can also be isolated from the reaction mixture according to an ordinary method, and can be easily purified by separation means such as recrystallization, distillation, chromatography and the like.

Of compounds (I'), that in which a substituent at the 5-position of a thiazole ring is alkylaminopyridyl optionally having a substituent is obtained by condensing the corresponding 5-(2-halogenopyridyl)thiazole with amines.

The amount of the amine used is from about 1.0 to about 100.0 mol, preferably from about 1.0 to about 20.0 mol per mol of the corresponding 5-(2-halogenopyridyl)thiazole.

As the halogen of the "5-(2-halogenopyridyl)thiazole", fluorine, chlorine, bromine, iodine, etc. are exemplified.

As the "amines", for example, aliphatic amines and cyclic amines corresponding to the intended alkylamine, etc. are exemplified.

The present reaction is conducted, if necessary in the presence of a base or basic salt.

The amount of the base used is from about 1.0 to about 10.0 mol, preferably from about 1.0 to about 3.0 mol per mol of the corresponding 5-(2-halogenopyridyl)thiazole.

As the "base", for example, aromatic amines such as pyridine, lutidine and the like, tertiary amines such triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, etc. are used.

The amount of the basic salt used is from about 1.0 to about 10.0 mol, preferably from about 1.0 to about 3.0 mol per mol of the corresponding 5-(2-halogenopyridyl)thiazole.

As the "basic salt", for example, sodium carbonate, potassium carbonate, cesium carbonate, sodium hydrogen carbonate, sodium acetate, potassium acetate, etc. are used.

The present reaction is advantageously conducted in the absence or presence of a solvent inactive to the reaction. This solvent is not particularly restricted providing the reaction can progress, and for example, ethers, aromatic hydrocarbons, aliphatic hydrocarbons, amides, halogenated hydrocarbons, nitriles, water or mixtures of two or more of them, etc. are used.

The reaction temperature is from about 0°C to about 300°C, preferably from about 20°C to about 200°C. The reaction time is usually from about 5 minutes to about 48 hours, preferably from about 10 minutes to about 15 hours.

The product can be used in the following reaction as the reaction solution itself or as a crude product, and can also be isolated from the reaction mixture according to an ordinary method, and can be easily purified by separation means such as recrystallization, distillation, chromatography and the like.

When the compound (I') is an N-oxide, it is obtained by treating the corresponding pyridyl compound with an organic peracid.

The amount of the organic peracid used is from about 0.8 to about 10 mol, preferably from about 1.0 to about 3.0 mol per mol of the corresponding pyridyl compound.

As the above-mentioned "organic peracid", for example, peracetic acid, pertrifluoroacetic acid, m-chloroperbenzoic acid, etc. are exemplified.

The present reaction is advantageously conducted in the absence or presence of a solvent inactive to the reaction. This solvent is not particularly restricted providing the reaction can progress, and for example, halogenated hydrocarbons, aliphatic hydrocarbons, aromatic hydrocarbons, organic acids, ethers, amides, sulfoxides, alcohols, nitriles, ketones or mixtures of two or more of them, etc. are used.

The reaction temperature is from about -20°C to about 130°C, preferably from about 0°C to about 100°C. The reaction time is usually from about 5 minutes to about 72 hours, preferably from about 0.5 to about 12 hours.

Further, an N-oxide can also be obtained by treating the corresponding pyridyl compound with hydrogen peroxide or alkyl hydroperoxide, if necessary in the presence of a base, acid or metal oxide.

The amount of the hydrogen peroxide or alkyl hydroperoxide used is from about 0.8 to about 10 mol, preferably from about 1.0 to about 3.0 mol per mol of the corresponding pyridyl compound.

As the above-mentioned "alkyl hydroperoxide", for example, tert-butyl hydroperoxide, cumene hydroperoxide, etc. are exemplified.

The amount of the base, acid or metal oxide used is from about 0.1 to about 30 mol, preferably from about 0.8 to about 5 mol per mol of the corresponding pyridyl compound.

As the above-mentioned "base", for example, inorganic bases such as sodium hydroxide, potassium hydroxide and the like, basic salts such as sodium carbonate, potassium carbonate and the like, etc. are exemplified.

As the above-mentioned "acid", for example, mineral acids such as hydrochloric acid, sulfuric acid, perchloric acid and the like, Lewis acids such as boron trifluoride, aluminum chloride, titanium tetrachloride and the like, organic acids such as formic acid, acetic acid and the like, etc. are exemplified.

As the above-mentioned "metal oxide", for example, vanadium oxide (V₂O₅), osmium tetraoxide (OsO₄), tungsten oxide (WO₃), molybdenum oxide (MoO₃), selenium dioxide (SeO₂), chromium oxide (CrO₃), etc. are exemplified.

The present reaction is advantageously conducted in the absence or presence of a solvent inactive to the reaction. This solvent is not particularly restricted providing the reaction can progress, and for example, halogenated hydrocarbons, aliphatic hydrocarbons, aromatic hydrocarbons, organic acids, ethers, amides, sulfoxides, alcohols, nitriles, ketones or mixtures of two or more of them, etc. are used.

The reaction temperature is from about -20°C to about 130°C, preferably from about 0°C to about 100°C. The reaction time is usually from about 5 minutes to about 72 hours, preferably from about 0.5 to about 12 hours.

The product can be used in the following reaction as the reaction solution itself or as a crude product, and can also be isolated from the reaction mixture according to an ordinary method, and can be easily purified by separation means such as recrystallization, distillation, chromatography and the like.

When the compound (I') is an S-oxide, it is obtained by treating the corresponding sulfide with a peroxide.

The amount of the peroxide used is from about 0.8 to about 10 mol, preferably from about 1.0 to about 3.0 mol per mol of the corresponding sulfide.

As the above-mentioned "peracid", for example, peracetic acid, pertrifluoroacetic acid, m-chloroperbenzoic acid, potassium persulfate, meta-periodic acid, etc. are exemplified.

The present reaction is advantageously conducted in the absence or presence of a solvent inactive to the reaction. This solvent is not particularly restricted providing the reaction can progress, and for example, halogenated hydrocarbons, aliphatic hydrocarbons, aromatic hydrocarbons, organic acids, ethers, amides, sulfoxides, alcohols, nitriles, ketones or mixtures of two or more of them, etc. are used.

The reaction temperature is from about -20°C to about 130°C, preferably from about 0°C to about 100°C. The reaction time is usually from about 5 minutes to about 72 hours, preferably from about 0.5 to about 12 hours.

Further, an S-oxide can also be obtained by treating the corresponding sulfide with hydrogen peroxide or alkyl hydroperoxide, if necessary in the presence of a base, acid or metal oxide.

The amount of the hydrogen peroxide or alkyl hydroperoxide used is from about 0.8 to about 10 mol, preferably from about 1.0 to about 3.0 mol per mol of the corresponding sulfide.

As the above-mentioned "alkyl hydroperoxide", for example, tert-butyl hydroperoxide, cumene hydroperoxide, etc. are exemplified.

The amount of the "base, acid or metal oxide" used is from about 0.1 to about 30 mol, preferably from about 0.8 to about 5 mol per mol of the corresponding sulfide.

As the above-mentioned "base", for example, inorganic bases such as sodium hydroxide, potassium hydroxide and the like, basic salts such as sodium carbonate, potassium carbonate and the like, etc. are exemplified.

As the above-mentioned "acid", for example, mineral acids such as hydrochloric acid, sulfuric acid, perchloric acid and the like, Lewis acids such as boron trifluoride, aluminum chloride, titanium tetrachloride and the like, organic acids such as formic acid, acetic acid and the like, etc. are exemplified.

As the above-mentioned "metal oxide", for example, vanadium oxide (V₂O₅), osmium tetraoxide (OsO₄), tungsten oxide (WO₃), molybdenum oxide (MoO₃), selenium dioxide (SeO₂), chromium oxide (CrO₃), etc. are exemplified.

The present reaction is advantageously conducted in the absence or presence of a solvent inactive to the reaction. This solvent is not particularly restricted providing the reaction can progress, and for example, halogenated hydrocarbons, aliphatic hydrocarbons, aromatic hydrocarbons, organic acids, ethers, amides, sulfoxides, alcohols, nitriles, ketones or mixtures of two or more of them, etc. are used.

The reaction temperature is from about -20°C to about 130°C, preferably from about 0°C to about 100°C. The reaction time is usually from about 5 minutes to about 72 hours, preferably from about 0.5 to about 12 hours.

The product can be used in the following reaction as the reaction solution itself or as a crude product, and can also be isolated from the reaction mixture according to an ordinary method, and can be easily purified by separation means such as recrystallization, distillation, chromatography and the like.

In the above-mentioned reactions, when a starting material has amino, carboxy, hydroxy as a substituent, a protective group as generally used may be introduced into these groups by peptide chemistry and the like, and the intended compound can be obtained by removing the protective group after the reaction, if necessary.

As the protective group for amino, for example, formyl or, C₁₋₆ alkyl-carbonyls (e.g., acetyl, propionyl and the like), phenylcarbonyl, C₁₋₆ alkoxy-carbonyls (e.g., methoxycarbony, ethoxycarbonyl and the like), phenyloxycarbonyl, C₇₋₁₀ aralkyloxy-carbonyls (e.g., benzyloxycarbonyl and the like), trityl, phthaloyl, each optionally having a substituent, etc. are used. As these substituents, halogen atoms (e.g., fluorine, chlorine, bromine, iodine and the like), C₁₋₆ alkyl-carbonyls (e.g., acetyl, propionyl, valeryl and the like), nitro, etc. are used, and the number of the substituent is 1 to 3.

As the protective group for carboxy, for example, C₁₋₆ alkyls (e.g., methyl, ethyl, propyl, isopropyl, butyl, tert-butyl and the like), phenyl, trityl, silyl, each optionally having a substituent, etc. are used. As these substituents, halogen atoms (e.g., fluorine, chlorine, bromine, iodine and the like), formyl, C₁₋₆ alkyl-carbonyls (e.g., acetyl, propionyl, butylcarbonyl and the like), nitro, C₁₋₆ alkyls (e.g., methyl, ethyl, tert-butyl and the like), C₆₋₁₀ aryls (e.g., phenyl, naphthyl and the like), etc. are used, and the number of the substituent is 1 to 3.

As the protective group for hydroxy, for example, C₁₋₆ alkyls (e.g., methyl, ethyl, propyl, isopropyl, butyl, tert-butyl and the like), phenyl, C₇₋₁₁ aralkyls (e.g., benzyl and the like), formyl, C₁₋₆ alkyl-carbonyls (e.g., acetyl, propionyl and the like), phenyloxycarbonyl, C₇₋₁₁ aralkyloxy-carbonyls (e.g., benzyloxycarbonyl and the like), tetrahydropyranyl, tetrahydrofuranyl, and silyl, each optionally having a substituent, and so on are used. As these substituents, halogen atoms (e.g., fluorine, chlorine, bromine, iodine and the like), C₁₋₆ alkyls (e.g., methyl, ethyl, tert-butyl and the like), C₇₋₁₁ aralkyls (e.g., benzyl and the like), C₆₋₁₀ aryls (e.g., phenyl, naphthyl and the like), nitro, etc. are used, and the number of the substituent is 1 to 4.

For removing a protective group, method known per se or methods according to them are used, and for example, methods for treating with an acid, a base, ultraviolet ray, hydrazine, phenylhydrazine, sodium N-methyldithiocarbamate, tetrabutylammonium fluoride, palladium acetate and the like, or reducing methods are used.

In any case, further if necessary, the compound (I) can be synthesized by using known de-protection reactions, acylation reactions, alkylation reactions, hydrogenation reactions, oxidation reactions, reduction reactions, carbon chain extension reactions, substituent interchange reactions, each alone or in combination of two or more of them. As these reactions, for example, methods described in Shinjikkenkagakukoza 14, vol. 15, 1977 (Maruzen Press), etc. are adopted.

As the above-mentioned "alcohols", for example, methanol, ethanol, propanol, isopropanol, tert-butanol, etc. are exemplified.

As the above-mentioned "ethers", for example, diethyl ether, diisopropyl ether, diphenyl ether, tetrahydrofuran, dioxane, 1,2-dimethoxyethane, etc. are exemplified.

As the above-mentioned "halogenated hydrocarbons", for example, dichloromethane, chloroform, 1,2-dichloroethane, carbon tetrachloride, etc. are exemplified.

As the above-mentioned "aliphatic hydrocarbons", for example, hexane, pentane, cyclohexane, etc. are exemplified.

As the above-mentioned "aromatic hydrocarbons", for example, benzene, toluene, xylene, chlorobenzene, etc. are exemplified.

As the above-mentioned "aromatic amines", for example, pyridine, lutidine, quinoline, etc. are exemplified.

As the above-mentioned "amides", for example, N,N-dimethylformamide, N,N-dimethylacetamide, hexamethylphosphoric triamide, etc. are exemplified.

As the above-mentioned "ketones", for example, acetone, methyl ethyl ketone, etc. are exemplified.

As the above-mentioned "sulfoxides", for example, dimethylsulfoxide, etc. are exemplified.

As the above-mentioned "nitriles", for example, acetonitrile, propionitrile, etc. are exemplified.

As the above-mentioned "organic acids", for example, acetic acid, propionic acid, trifluoroacetic acid, etc. are exemplified.

As the above-mentioned "esters", for example, methyl acetate, ethyl acetate, amyl acetate, ethyl propionate, etc. are exemplified.

When the intended substance is obtained in the free form by the above-mentioned reaction, it may be converted into a salt according to an ordinary method, while when obtained in the form of a salt, it can also be converted into a free form or other salt according to an ordinary method. Thus obtained compound (I') can be isolated and purified from a reaction solution by known means, for example, rolling, concentration, solvent extraction, fractionation, crystallization, recrystallization, chromatography and the like.

When the compound (I') is present as a configuration isomer, diastereomer, conformer or the like, if necessary, each can be isolated by the above-mentioned separation and purification means. When the compound (I') is a racemate, it can be separated into an S form and R form by a usual optical resolution.

When a stereoisomer is present in the compound (I'), this isomer alone and mixtures thereof are also included in the present invention.

Further, the compound (I') may be a hydrate or non-hydrate.

The compound (I') may be labeled with an isotope (e.g., ³H, ¹⁴C, ³⁵S), etc.

### [compound (VII)]

[1] An optionally N-oxidized compound represented by the formula: 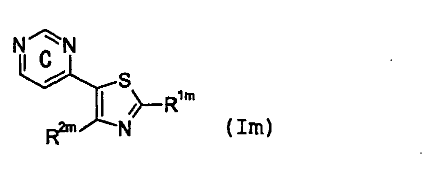 wherein
   ring C is a 4-pyrimidinyl group optionally having substituents,
   R^{1m} is a hydrogen atom, a hydrocarbon group optionally having substituents, a heterocyclic group optionally having substituents, an amino group optionally having substituents or an acyl group, and
   R^{2m} is an aromatic group optionally having substituents, or a salt thereof.
[2] The compound of the above-mentioned [1], wherein the compound (Im) is an optionally N-oxidized compound represented by the formula: 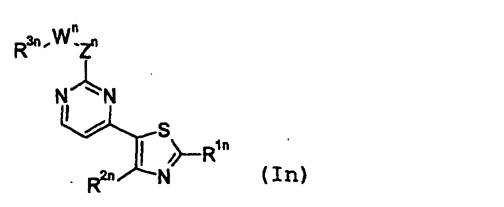 wherein
   Zⁿ is a bond, -NR⁴ⁿ- (R⁴ⁿ is a hydrogen atom or a hydrocarbon group optionally having substituents), an oxygen atom or an optionally oxidized sulfur atom,
   Wⁿ is a bond or a divalent hydrocarbon group optionally having substituents,
   R¹ⁿ is a hydrogen atom, a hydrocarbon group optionally having substituents, a heterocyclic group optionally having substituents, an amino group optionally having substituents or an acyl group,
   R²ⁿ is an aromatic group optionally having substituents, and
   R³ⁿ is a hydrogen atom, a hydrocarbon group optionally having substituents or a heterocyclic group optionally having substituents.
[3] The compound of the above-mentioned [2], wherein both Wⁿ and Zⁿ are each a bond.
[4] The compound of the above-mentioned [1], wherein the compound (Im) is an optionally N-oxidized compound represented by the formula: 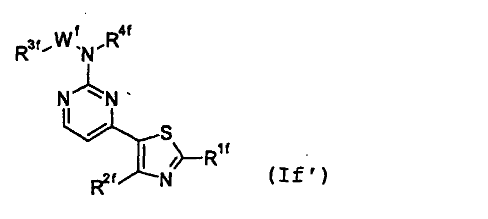 wherein
   W^{f} is a bond or a divalent hydrocarbon group optionally having substituents,
   R^{1f} is a hydrogen atom, a hydrocarbon group optionally having substituents, a heterocyclic group optionally having substituents, an amino group optionally having substituents or an acyl group,
   R^{2f} is an aromatic group optionally having substituents,
   R^{3f} is a hydrocarbon group optionally having substituents or a heterocyclic group optionally having substituents, and
   R^{4f} is a hydrogen atom or a hydrocarbon group optionally having substituents.
[5] The compound of the above-mentioned [4], wherein the compound (If') is an optionally N-oxidized compound represented by the formula: 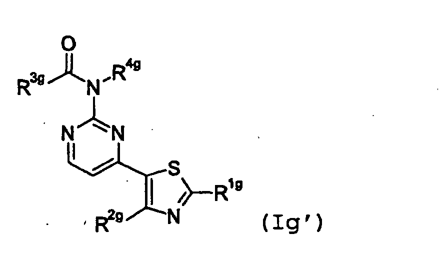 wherein
   R^{1g} is a hydrogen atom, a hydrocarbon group optionally having substituents, a heterocyclic group optionally having substituents, an amino group optionally having substituents or an acyl group,
   R^{2g} is an aromatic group optionally having substituents,
   R^{3g} is a hydrocarbon group optionally having substituents or a heterocyclic group optionally having substituents, and
   R^{4g} is a hydrogen atom or a hydrocarbon group optionally having substituents.
[6] The compound of the above-mentioned [4], wherein th4compound (If') is an optionally N-oxidized compound represented by the formula: 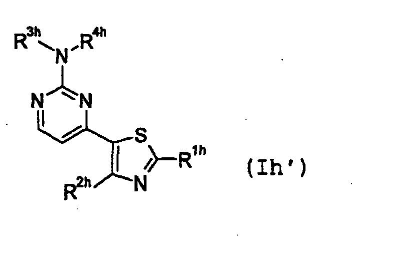 wherein
   R^{1h} is a hydrogen atom, a hydrocarbon group optionally having substituents, a heterocyclic group optionally having substituents, an amino group optionally having substituents or an acyl group,
   R^{2h} is an aromatic group optionally having substituents,
   R^{3h} is a hydrocarbon group optionally having substituents or a heterocyclic group optionally having substituents, and
   R^{4h} is a hydrogen atom or a hydrocarbon group optionally having substituents.
[7] A prodrug of the compound of [1].

As the "hydrocarbon group" of the "hydrocarbon group optionally having substituents" in the compounds represented by the formulae (Im), (In), (If'), (Ig') and (Ih'), for example, an acyclic or cyclic hydrocarbon group (e.g., alkyl, alkenyl, alkynyl, cycloalkyl, aryl, aralkyl etc.) and the like can be mentioned. Of these, an acyclic or cyclic hydrocarbon group having 1 to 16 carbon atoms and the like are preferable.

As the "alkyl", for example, C₁₋₆ alkyl (e.g., methyl, ethyl, propyl, isopropyl, butyl, isobutyl, sec-butyl, tert-butyl, pentyl, hexyl etc.) and the like are preferable.

As the "alkenyl", for example, C₂₋₆ alkenyl (e.g., vinyl, allyl, isopropenyl, 1-butenyl, 2-butenyl, 3-butenyl, 2-methyl-2-propenyl, 1-methyl-2-propenyl, 2-methyl-1-propenyl etc.) and the like are preferable.

As the "alkynyl", for example, C₂₋₆ alkynyl (e.g., ethynyl, propargyl, 1-butynyl, 2-butynyl, 3-butynyl, 1-hexynyl etc.) and the like are preferable.

As the "cycloalkyl", for example, C₃₋₈ cycloalkyl (e.g., cyclopropyl, cyclobutyl, cyclopentyl, cyclohexyl, cycloheptyl, cyclooctyl etc.) and the like are preferable.

As the "aryl", for example, C₆₋₁₄ aryl (e.g., phenyl, 1-naphthyl, 2-naphthyl, 2-biphenylyl, 3-biphenylyl, 4-biphenylyl, 2-anthryl etc.) and the like are preferable.

As the "aralkyl", for example, C₇₋₁₆ aralkyl (e.g., benzyl, phenethyl, diphenylmethyl, 1-naphthylmethyl, 2-naphthylmethyl, 2,2-diphenylethyl, 3-phenylpropyl, 4-phenylbutyl, 5-phenylpentyl etc.) and the like are preferable.

As the "substituent" of the "hydrocarbon group optionally having substituents", for example, oxo, halogen atom (e.g., fluorine, chlorine, bromine, iodine etc.), C₁₋₃ alkylenedioxy (e.g., methylenedioxy, ethylenedioxy etc.), nitro, cyano, optionally halogenated C₁₋₆ alkyl, optionally halogenated C₂₋₆ alkenyl, carboxy C₂₋₆ alkenyl (e.g., 2-carboxyethenyl, 2-carboxy-2-methylethenyl etc.), optionally halogenated C₂₋₆ alkynyl, optionally halogenated C₃₋₈ cycloalkyl, C₆₋₁₄ aryl (e.g., phenyl, 1-naphthyl, 2-naphthyl, 2-biphenylyl, 3-biphenylyl, 4-biphenylyl, 2-anthryl etc.), optionally halogenated C₁₋₈ alkoxy, C₁₋₆ alkoxy-carbonyl-C₁₋₆ alkoxy (e.g., ethoxycarbonylmethyloxy etc.), hydroxy, C₆₋₁₄ aryloxy (e.g., phenyloxy, I-naphthyloxy, 2-naphthyloxy etc.), C₇₋₁₆ aralkyloxy (e.g., benzyloxy, phenethyloxy etc.), mercapto, optionally halogenated C₁₋₆ alkylthio, C₆₋₁₄ arylthio (e.g., phenylthio, 1-naphthylthio, 2-naphthylthio etc.), C₇₋₁₆ aralkylthio (e.g., benzylthio, phenethylthio etc.), amino, mono-C₁₋₆ alkylamino (e.g., methylamino, ethylamino etc.), mono-C₆₋₁₄ arylamino (e.g., phenylamino, 1-naphthylamino, 2-naphthylamino etc.), di-C₁₋₆ alkylamino (e.g., dimethylamino, diethylamino, ethylmethylamino etc.), C₃₋₈ cycloalkylamino (e.g., cyclopentylamino, cyclohexylamino etc.), di-C₆₋₁₄ arylamino (e.g., diphenylamino etc.), formyl, carboxy, carboxy-C₁₋₆ alkyl (e.g., carboxymethyl, carboxyethyl etc.), C₁₋₆ alkyl-carbonyl (e.g., acetyl, propionyl, pivaloyl etc.), C₃₋₈ cycloalkyl-carbonyl (e.g:, cyclopropylcarbonyl, cyclopentylcarbonyl, cyclohexylcarbonyl etc.), C₁₋₆ alkoxy-carbonyl (e.g., methoxycarbonyl, ethoxycarbonyl, propoxycarbonyl, tert-butoxycarbonyl etc.), C₆₋₁₄ aryl-carbonyl (e.g., benzoyl, 1-naphthoyl, 2-naphthoyl etc.), C₇₋₁₆ aralkyl-carbonyl (e.g., phenylacetyl, 3-phenylpropionyl etc.), C₆₋₁₄ aryloxy-carbonyl (e.g., phenoxycarbonyl etc.), C₇₋₁₆ aralkyloxy-carbonyl (e.g., benzyloxycarbonyl, phenethyloxycarbonyl etc.), 5- or 6-membered heterocyclic carbonyl (e.g., nicotinoyl, isonicotinoyl, thenoyl, furoyl, morpholinocarbonyl, thiomorpholinocarbonyl, piperazin-1-ylcarbonyl, pyrrolidin-1-ylcarbonyl etc.), carbamoyl, thiocarbamoyl, mono-C₁₋₆ alkyl-carbamoyl (e.g., methylcarbamoyl, ethylcarbamoyl etc.), di-C₁₋₆ alkyl-carbamoyl (e.g., dimethylcarbamoyl, diethylcarbamoyl, ethylmethylcarbamoyl etc.), mono- or di-C₆₋₁₄ aryl-carbamoyl (e.g., phenylcarbamoyl, 1-naphthylcarbamoyl, 2-naphthylcarbamoyl etc.), mono- or di-5- or 6-membered heterocyclic carbamoyl (e.g., 2-pyridylcarbamoyl, 3-pyridylcarbamoyl, 4-pyridylcarbamoyl, 2-thienylcarbamoyl, 3-thienylcarbamoyl etc.), C₁₋₆ alkylsulfonyl (e.g., methylsulfonyl, ethylsulfonyl etc.), C₁₋₆ alkylsulfinyl (e.g., methylsulfinyl, ethylsulfinyl etc.), C₆₋₁₄ arylsulfonyl (e.g., phenylsulfonyl, 1-naphthylsulfonyl, 2-naphthylsulfonyl etc.), C₆₋₁₄ arylsulfinyl (e.g., phenylsulfinyl, 1-naphthylsulfinyl, 2-naphthylsulfinyl etc.), formylamino, C₁₋₆ alkyl-carbonylamino (e.g., acetylamino, propionylamino, pivaloylamino etc.), C₃₋₈ cycloalkyl-carbonylamino (e.g., cyclopentylcarbonylamino, cyclohexylcarbonylamino etc.), C₆₋₁₄ aryl-carbonylamino (e.g., benzoylamino, naphthoylamino etc.), C₁₋₆ alkoxy-carbonylamino (e.g., methoxycarbonylamino, ethoxycarbonylamino, propoxycarbonylamino, butoxycarbonylamino etc.), C₁₋₆ alkylsulfonylamino (e.g., methylsulfonylamino, ethylsulfonylamino etc.), C₆₋₁₄ arylsulfonylamino (e.g., phenylsulfonylamino, 2-naphthylsulfonylamino, 1-naphthylsulfonylamino etc.), C₁₋₆ alkyl-carbonyloxy (e.g., acetoxy, propionyloxy etc.), C₆₋₁₄ aryl-carbonyloxy (e.g., benzoyloxy, naphthylcarbonyloxy etc.), C₁₋₆ alkoxy-carbonyloxy (e.g., methoxycarbonyloxy, ethoxycarbonyloxy, propoxycarbonyloxy, butoxycarbonyloxy etc.), mono-C₁₋₆ alkyl-carbamoyloxy (e.g., methylcarbamoyloxy, ethylcarbamoyloxy etc.), di-C₁₋₆ alkyl-carbamoyloxy (e.g., dimethylcarbamoyloxy, diethylcarbamoyloxy etc.), mono- or di-C₆₋₁₄ aryl-carbamoyloxy (e.g., phenylcarbamoyloxy, naphthylcarbamoyloxy etc.), nicotinoyloxy, isonicotinoyloxy, 5- to 7-membered saturated cyclic amino optionally having substituents, 5- to 10-membered aromatic heterocyclic group (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl etc.), sulfo, sulfamoyl, sulfinamoyl, sulfenamoyl, a group wherein 2 or more (e.g., 2-3) of these substituents are bonded and the like can be mentioned.

The "hydrocarbon group" may have, for example, 1 to 5, preferably 1 to 3, of the above-mentioned substituents at substitutable positions, and when the number of substituents is 2 or more, the respective substituents may be the same or different.

As the aforementioned "optionally halogenated C₁₋₆ alkyl", for example, C₁₋₆ alkyl (e.g., methyl, ethyl, propyl, isopropyl, butyl, isobutyl, sec-butyl, tert-butyl, pentyl, hexyl etc.) optionally having 1 to 5, preferably 1 to 3, halogen atoms (e.g., fluorine, chlorine, bromine, iodine etc.) and the like can be mentioned. As specific examples, methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl and the like can be mentioned.

As the aforementioned "optionally halogenated C₂₋₆ alkenyl", for example, C₂₋₆ alkenyl (e.g., vinyl, propenyl, isopropenyl, 2-buten-1-yl, 4-penten-1-yl, 5-hexen-1-yl etc.) optionally having 1 to 5, preferably 1 to 3, halogen atoms (e.g., fluorine, chlorine, bromine, iodine etc.) and the like can be mentioned. As specific examples, vinyl, propenyl, 3,3,3-trifluoropropenyl, 2-buten-1-yl, 4,4,4-trifluoro-2-buten-1-yl, 4-penten-1-yl, 5-hexen-1-yl and the like can be mentioned.

As the aforementioned "optionally halogenated C₂₋₆ alkynyl", for example, C₂₋₆ alkynyl (e.g., 2-butyn-1-yl, 4-pentyn-1-yl, 5-hexyn-1-yl etc.) optionally having 1 to 5, preferably 1 to 3, halogen atoms (e.g., fluorine, chlorine, bromine, iodine etc.) and the like can be mentioned. As specific examples, propargyl, 2-butyn-1-yl, 4,4,4-trifluoro-2-butyn-1-yl, 4-pentyn-1-yl, 5,5,5-trifluoro-4-pentyn-1-yl, 5-hexyn-1-yl and the like can be mentioned.

As the aforementioned "optionally halogenated C₃₋₈ cycloalkyl", for example, C₃₋₈ cycloalkyl (e.g., cyclopropyl, cyclobutyl, cyclopentyl, cyclohexyl, cycloheptyl, cyclooctyl etc.) optionally having 1 to 5, preferably 1 to 3, halogen atoms (e.g., fluorine, chlorine, bromine, iodine etc.) and the like can be mentioned. As specific examples, cyclopropyl, cyclobutyl, cyclopentyl, cyclohexyl, cycloheptyl, cyclooctyl, 4,4-dichlorocyclohexyl, 2,2,3,3-tetrafluorocyclopentyl, 4-chlorocyclohexyl and the like can be mentioned.

As the aforementioned "optionally halogenated C₁₋₈ alkoxy", for example, C₁₋₈ alkoxy (e.g., methoxy, ethoxy, propoxy, isopropoxy, butoxy, isobutoxy, sec-butoxy, pentyloxy, hexyloxy etc.) optionally having 1 to 5, preferably 1 to 3, halogen atoms (e.g., fluorine, chlorine, bromine, iodine etc.) and the like can be mentioned. As specific examples, for example, methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy and the like can be mentioned.

As the aforementioned "optionally halogenated C₁₋₆ alkylthio", for example, C₁₋₆ alkylthio (e.g., methylthio, ethylthio, propylthio, isopropylthio, butylthio, sec-butylthio, tert-butylthio etc.) optionally having 1 to 5, preferably 1 to 3, halogen atoms (e.g., fluorine, chlorine, bromine, iodine etc.) and the like can be mentioned. As specific examples, methylthio, difluoromethylthio, trifluoromethylthio, ethylthio, propylthio, isopropylthio, butylthio, 4,4,4-trifluorobutylthio, pentylthio, hexylthio and the like can be mentioned.

As the "5- to 7-membered saturated cyclic amino" of the aforementioned "5- to 7-membered saturated cyclic amino optionally having substituents", for example, a 5- to 7-membered saturated cyclic amino optionally containing, besides one nitrogen atom and carbon atom(s), 1 or 2 kinds of 1 to 4 hetero atoms selected from nitrogen atom, sulfur atom and oxygen atom can be mentioned. As specific examples, pyrrolidin-1-yl, piperidino, piperazin-1-yl, morpholino, thiomorpholino, hexahydroazepin-1-yl and the like can be mentioned.

As the "substituent" of the "5- to 7-membered saturated cyclic amino optionally having substituents", for example, 1 to 3 substituents from C₁₋₆ alkyl (e.g., methyl, ethyl, propyl, isopropyl, butyl, isobutyl, sec-butyl, tert-butyl, pentyl, hexyl etc.), C₆₋₁₄ aryl (e.g., phenyl, 1-naphthyl, 2-naphthyl, 2-biphenylyl, 3-biphenylyl, 4-biphenylyl, 2-anthryl etc.), C₁₋₆ alkyl-carbonyl (e.g., acetyl, propionyl, pivaloyl etc.), 5- to 10-membered aromatic heterocyclic group (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl etc.), oxo and the like can be mentioned.

The "divalent hydrocarbon group" of the "divalent hydrocarbon group optionally having substituents" in the compounds represented by the formulae (In) and (If') refers to a divalent group derived from the "hydrocarbon group" of the aforementioned "hydrocarbon group optionally having substituents", and, for example, a divalent group derived from alkylene, alkenylene, alkynylene or cycloalkane, a divalent group derived from cycloalkene, a divalent group derived from aromatic hydrocarbon ring and the like can be mentioned.

As the "alkylene", for example, C₁₋₁₅ alkylene group (e.g., methylene, ethylene, trimethylene, tetramethylene, pentamethylene, hexamethylene, heptamethylene, octamethylene and the like, preferably C₁₋₆ alkylene etc.) and the like can be mentioned.

As the "alkenylene", for example, C₂₋₁₆ alkenylene group (e.g., vinylene, propenylene, 1-butenylene, 2-butenylene, 1-pentenylene, 2-pentenylene, 3-pentenylene etc.) and the like can be mentioned.

As the "alkynylene", for example, C₂₋₁₆ alkynylene group (ethynylene, propynylene, 1-butynylene, 2-butynylene, 1-pentynylene, 2-pentynylene, 3-pentynylene etc.) can be mentioned.

As the "cycloalkane", for example, C₃₋₇ cycloalkane such as cyclopropane, cyclobutane, cyclopentane, cyclohexane, cycloheptene, cyclooctane and the like, and the like can be mentioned.

As the "cycloalkene", for example, C₃₋₈ cycloalkene such as cyclopropene, cyclobutene, cyclopentene, cyclohexene, cycloheptene, cyclooctene and the like, and the like can be mentioned.

As the "aromatic hydrocarbon ring", a hydrocarbon ring having 6 to 14 carbon atoms such as benzene ring, naphthalene ring and the like, and the like can be mentioned.

The divalent group derived from "cycloalkane", "cycloalkene" or "aromatic hydrocarbon ring" refers to a divalent group obtained by removing two hydrogen atoms from one carbon atom of, or removing one hydrogen atom from each of two different carbon atoms of "cycloalkane", "cycloalkene" or "aromatic hydrocarbon ring", and the like. Specifically, for example, 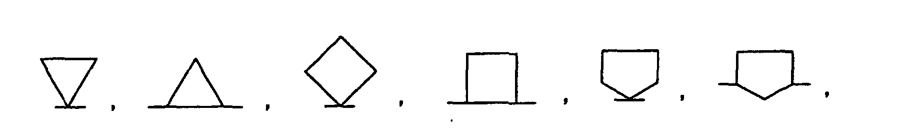 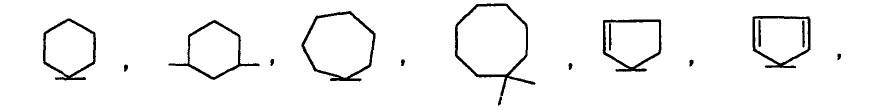 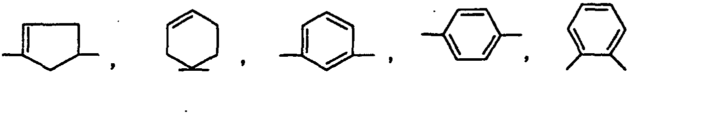 and the like are used, preferably, 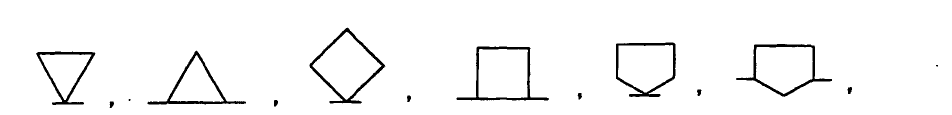 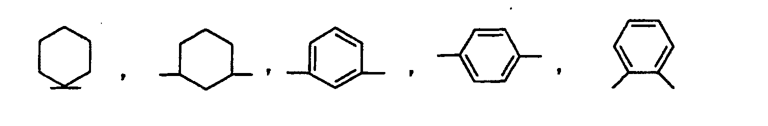 and the like are used, and more preferably, 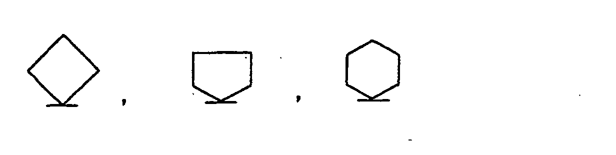 and the like are widely used.

As the "substituent" of the "divalent hydrocarbon group", those similar to the "substituent" of the aforementioned "hydrocarbon group optionally having substituents" can be mentioned.

The "divalent hydrocarbon group" may have, for example, 1 to 4, preferably 1 to 3, of the above-mentioned substituents at substitutable positions, and when the number of substituents is 2 or more, the respective substituents may be the same or different.

As the divalent hydrocarbon group optionally having substituents, C₁₋₁₅ alkylene group optionally substituted by oxo group, and the like are preferable. Particularly, C₁₋₆ alkylene optionally substituted by oxo group, and the like are preferable.

As the "heterocyclic group" of the "heterocyclic group optionally having substituents" in the compounds represented by the formulae (Im), (In), (If'), (Ig') and (Ih'), for example, a monovalent group obtained by removing optional one hydrogen atom from a 5- to 14-membered (monocyclic, bicyclic or tricyclic) heterocycle containing, besides carbon atom(s), 1 or 2 kinds of 1 to 4 hetero atoms selected from nitrogen atom, sulfur atom and oxygen atom, preferably (i) a 5- to 14-membered (preferably 5- to 10-membered) aromatic heterocycle, (ii) a 5- to 10-membered non-aromatic heterocycle or (iii) a 7- to 10-membered bridged heterocycle, and the like can be mentioned.

As the above-mentioned "5 to 14-membered (preferably 5- to 10-membered) aromatic heterocycle", for example, an aromatic heterocycle such as thiophene, benzo[b]thiophene, benzo[b]furan, benzimidazole, benzoxazole, benzothiazole, benzisothiazole, naphtho[2,3-b]thiophene, furan, pyrrole, imidazole, pyrazole, pyridine, pyrazine, pyrimidine, pyridazine, indole, isoindole, 1H-indazole, purine, 4H-quinolizine, isoquinoline, quinoline, phthalazine, naphthyridine, quinoxaline, quinazoline, cinnoline, carbazole, β-carboline, phenanthridine, acridine, phenazine, thiazole, isothiazole, phenothiazine, isoxazole, furazan, phenoxazine and the like, a ring formed by condensation of these rings (preferably monocycle) with one or plural (preferably 1 or 2) aromatic rings (e.g., benzene ring etc.) and the like can be mentioned.

As the above-mentioned "5- to 10-membered non-aromatic heterocycle", for example, pyrrolidine, imidazoline, pyrazolidine, pyrazoline, piperidine, piperazine, morpholine, thiomorpholine, dioxazole, oxadiazoline, thiadiazoline, triazoline, thiadiazole, dithiazole and the like can be mentioned.

As the above-mentioned "7 to 10-membered crosslinked heterocycle", for example, quinuclidine, 7-azabicyclo[2.2.1]heptane and the like can be mentioned.

The preferable "heterocyclic group" is a 5 to 14-membered (preferably 5- to 10-membered) (monocyclic or bicyclic) heterocyclic group preferably containing, besides carbon atom(s), 1 or 2 kinds of 1 to 4 hetero atoms selected from nitrogen atom, sulfur atom and oxygen atom. Specific examples include aromatic heterocyclic groups such as 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 2-pyrimidinyl, 4-pyrimidinyl, 3-pyrrolyl, 2-imidazolyl, 3-pyridazinyl, 3-isothiazolyl, 3-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like, non-aromatic heterocyclic groups such as 1-pyrrolizinyl, 2-pyrrolizinyl, 3-pyrrolizinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino and the like, and the like.

Of these, for example, a 5- or 6-membered heterocyclic group containing, besides carbon atom(s), 1 to 3 hetero atoms selected from nitrogen atom, sulfur atom and oxygen atom, and the like are more preferable. Specifically, 2-thienyl, 3-thienyl, 2-pyridyl, 3-pyridyl, 4-pyridyl, 2-furyl, 3-furyl, pyrazinyl, 2-pyrimidinyl, 3-pyrrolyl, 3-pyridazinyl, 3-isothiazolyl, 3-isoxazolyl, 1-pyrrolizinyl, 2-pyrrolizinyl, 3-pyrrolizinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino and the like can be mentioned.

As the "substituent" of the "heterocyclic group optionally having substituents", those similar to the "substituent" of the aforementioned "hydrocarbon group optionally having substituents" can be mentioned.

The "heterocyclic group" may have, for example, 1 to 5, preferably 1 to 3, of the above-mentioned substituents at substitutable positions, and when the number of substituents is 2 or more, the respective substituents may be the same or different.

As the "acyl group" in the compounds represented by the formulae (Im), (In), (If'), (Ig'), and (Ih'), for example, an acyl group represented by the formula: -(C=O)-R⁷, -(C=O)-OR⁷, -(C=O)-NR⁷R⁸, -(C=S)-NHR⁷ or -SO₂-R⁹ wherein R⁷ is a hydrogen atom, a hydrocarbon group optionally having substituents or a heterocyclic group optionally having substituents, R⁸ is a hydrogen atom or C₁₋₆ alkyl group, and R⁹ is a hydrocarbon group optionally having substituents or a heterocyclic group optionally having substituents, and the like can be mentioned.

As the "hydrocarbon group optionally having substituents" and "heterocyclic group optionally having substituents", those similar to the aforementioned can be used.

As the "C₁₋₆ alkyl group", methyl, ethyl, propyl, isopropyl, butyl, isobutyl, sec-butyl, tert-butyl, pentyl, hexyl and the like can be mentioned.

As the "amino group optionally having substituents" in the compounds represented by the formulae (Im), (In), (If'), (Ig'), and (Ih'), (1) an amino group optionally having 1 or 2 substituents and (2) a cyclic amino group optionally having substituents can be mentioned.

As the "substituent" of the "amino group optionally having 1 or 2 substituents" of the above-mentioned (1), for example, a hydrocarbon group optionally having substituents, a heterocyclic group optionally having substituents, an acyl group, an alkylidene group optionally having substituents and the like can be mentioned. As these "hydrocarbon group optionally having substituents", "heterocyclic group optionally having substituents" and "acyl group", those similar to the aforementioned can be respectively used.

As the "alkylidene group" of the "alkylidene group optionally having substituents", for example, C₁₋₆ alkylidene (e.g., methylidene, ethylidene, propylidene etc.) and the like can be mentioned. As the "substituent" of the "alkylidene group optionally having substituents", those similar to the "substituent" of the aforementioned "hydrocarbon group optionally having substituents" can be mentioned. The "alkylidene group" can be substituted by 1 to 5, preferably 1 to 3, of these substituents.

When the number of "substituents" of the above-mentioned "amino group optionally having 1 or 2 substituents" is 2, the respective substituents may be the same or different.

As the "cyclic amino group" of the "cyclic amino group optionally having substituents" of the above-mentioned (2), a 5- to 7-membered non-aromatic cyclic amino group optionally containing, besides one nitrogen atom and carbon atom(s), 1 or 2 kinds of 1 to 4 hetero atoms selected from nitrogen atom, sulfur atom and oxygen atom can be mentioned. As specific examples, pyrrolidin-1-yl, piperidino, piperazin-1-yl, morpholino, thiomorpholino, hexahydroazepin-1-yl, imidazolidin-1-yl, 2,3-dihydro-1H-imidazol-1-yl, tetrahydro-1(2H)-pyrimidinyl, 3,6-dihydro-1(2H)-pyrimidinyl, 3,4-dihydro-1(2H)-pyrimidinyl and the like can be mentioned.

As the "substituent" of the "cyclic amino group optionally having substituents", for example, those similar to the "substituent" of the "5- to 7-membered saturated cyclic amino optionally having substituents" explained in detail as the "substituent" of the aforementioned "hydrocarbon group optionally having substituents", and the like can be mentioned, wherein the "cyclic amino group" is preferably substituted by 1 to 3 of these substituents.

As specific examples of a 5- to 7-membered non-aromatic cyclic amino group having one oxo, 2-oxoimidazolidin-1-yl, 2-oxo-2,3-dihydro-1H-imidazol-1-yl, 2-oxotetrahydro-1(2H)-pyrimidinyl, 2-oxo-3,6-dihydro-1(2H)-pyrimidinyl, 2-oxo-3,4-dihydro-1(2H)-pyrimidinyl, 2-oxopyrrolidin-1-yl, 2-oxopiperidino, 2-oxopiperazin-1-yl, 3-oxopiperazin-1-yl, 2-oxo-2,3,4,5,6,7-hexahydroazepin-1-yl and the like can be mentioned.

In the present invention, as the "aromatic group" of the "aromatic group optionally having substituents in the compounds represented by the formulae (Im), (In), (If'), (Ig'), and (Ih')", for example, aromatic hydrocarbon group, aromatic heterocyclic group and the like can be mentioned.

As the "aromatic hydrocarbon group", for example, a monocyclic or fused polycyclic (di- or tri-cyclic) aromatic hydrocarbon group having 6 to 14 carbon atoms and the like can be mentioned. As specific examples, C₆₋₁₄ aryl such as phenyl, 1-naphthyl, 2-naphthyl, 2-biphenylyl, 3-biphenylyl, 4-biphenylyl, 2-anthryl and the like, and the like, preferably C₆₋₁₀ aryl (e.g., phenyl, 1-naphthyl, 2-naphthyl and the like, preferably phenyl etc.), and the like can be mentioned.

As the "aromatic heterocyclic group", a monovalent group obtained by removing one optional hydrogen atom from a 5- to 14-membered (preferably 5- to 10-membered) aromatic heterocycle containing, besides carbon atom(s), 1 or 2 kinds of 1 to 4 hetero atoms selected from nitrogen atom, sulfur atom and oxygen atom, and the like can be mentioned.

As the "5- to 14-membered (preferably 5- to 10-membered) aromatic heterocycle", for example, aromatic heterocycle such as thiophene, benzo[b]thiophene, benzo[b]furan, benzimidazole, benzoxazole, benzothiazole, benzisothiazole, naphtho[2,3-b]thiophene, furan, pyrrole, imidazole, pyrazole, pyridine, pyrazine, pyrimidine, pyridazine, indole, isoindole, 1H-indazole, purine, 4H-quinolizine, isoquinoline, quinoline, phthalazine, naphthyridine, quinoxaline, quinazoline, cinnoline, carbazole, β-carboline, phenanthridine, acridine, phenazine, thiazole, isothiazole, phenothiazine, isoxazole, furazan, phenoxazine and the like, a ring formed by condensation of these rings (preferably monocycle) with one or plural (preferably 1 or 2) aromatic rings (e.g., benzene ring etc.), and the like can be mentioned.

As the preferable "aromatic heterocyclic group", a 5- to 14-membered (preferably 5- to 10-membered) (monocyclic or bicyclic) aromatic heterocyclic group preferably containing, besides carbon atom(s), 1 or 2 kinds of 1 to 4 hetero atoms selected from nitrogen atom, sulfur atom and oxygen atom, and the like, specifically, an aromatic heterocyclic group such as 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 2-pyrimidinyl, 4-pyrimidinyl, 3-pyrrolyl, 2-imidazolyl, 3-pyridazinyl, 3-isothiazolyl, 3-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like can be mentioned.

As the "substituent" of the "aromatic group optionally having substituents", 1 to 5, preferably 1 to 3, of those similar to the "substituent" of the aforementioned "hydrocarbon group optionally having substituents" can be mentioned. When the number of the substituents is 2 or more, the respective substituents may be the same or different.

As the "substituent" of the "4-pyrimidinyl group optionally having substituents" for ring C, for example, a group represented by the formula: -Zⁿ-Wⁿ-R³ⁿ wherein the symbols in the formula are as defined above as well as halogen atom (e.g., fluorine, chlorine, bromine, iodine etc.), C₁₋₃ alkylenedioxy (e.g., methylenedioxy, ethylenedioxy etc.), nitro, cyano, optionally halogenated C₁₋₆ alkyl, optionally halogenated C₂₋₆ alkenyl, carboxy C₂₋₆ alkenyl (e.g., 2-carboxyethenyl, 2-carboxy-2-methylethenyl etc.), optionally halogenated C₂₋₆ alkynyl, optionally halogenated C₃₋₈ cycloalkyl, C₆₋₁₄ aryl (e.g., phenyl, 1-naphthyl, 2-naphthyl, 2-biphenylyl, 3-biphenylyl, 4-biphenylyl, 2-anthryl etc.), optionally halogenated C₁₋₈ alkoxy, C₁₋₆ alkoxy-carbonyl-C₁₋₆ alkoxy (e.g., ethoxycarbonylmethyloxy etc.), hydroxy, C₆₋₁₄ aryloxy (e.g., phenyloxy, 1-naphthyloxy, 2-naphthyloxy etc.), C₇₋₁₆ aralkyloxy (e.g., benzyloxy, phenethyloxy etc.), mercapto, optionally halogenated C₁₋₆ alkylthio, C₆₋₁₄ arylthio (e.g., phenylthio, 1-naphthylthio, 2-naphthylthio etc.), C₇₋₁₆ aralkylthio (e.g., benzylthio, phenethylthio etc.), amino, mono-C₁₋₆ alkylamino (e.g., methylamino, ethylamino etc.), mono-C₆₋₁₄ arylamino (e.g., phenylamino, 1-naphthylamino, 2-naphthylamino etc.), di-C₁₋₆ alkylamino (e.g., dimethylamino, diethylamino, ethylmethylamino etc.), C₃₋₈ cycloalkylamino (e.g., cyclopentylamino, cyclohexylamino etc.), di-C₆₋₁₄ arylamino (e.g., diphenylamino etc.), formyl, carboxy, carboxy-C₁₋₆ alkyl (e.g., carboxymethyl, carboxyethyl etc.), C₁₋₆ alkyl-carbonyl (e.g., acetyl, propionyl, pivaloyl etc.), C₃₋₈ cycloalkyl-carbonyl (e.g., cyclopropylcarbonyl, cyclopentylcarbonyl, cyclohexylcarbonyl etc.), C₁₋₆ alkoxy-carbonyl (e.g., methoxycarbonyl, ethoxycarbonyl, propoxycarbonyl, tert-butoxycarbonyl etc.), C₆₋₁₄ aryl-carbonyl (e.g., benzoyl, 1-naphthoyl, 2-naphthoyl etc.), C₇₋₁₆ aralkyl-carbonyl (e.g., phenylacetyl, 3-phenylpropionyl etc.), C₆₋₁₄ aryloxy-carbonyl (e.g., phenoxycarbonyl etc.), C₇₋₁₆ aralkyloxy-carbonyl (e.g., benzyloxycarbonyl, phenethyloxycarbonyl etc.), 5 or 6-membered heterocyclic-carbonyl (e.g., nicotinoyl, isonicotinoyl, thenoyl, furoyl, morpholinocarbonyl, thiomorpholinocarbonyl, piperazin-1-ylcarbonyl, pyrrolidin-1-ylcarbonyl etc.), carbamoyl, thiocarbamoyl, mono-C₁₋₆ alkyl-carbamoyl (e.g., methylcarbamoyl, ethylcarbamoyl etc.), di-C₁₋₆ alkyl-carbamoyl (e.g., dimethylcarbamoyl, diethylcarbamoyl, ethylmethylcarbamoyl etc.), mono- or di-C₆₋₁₄aryl-carbamoyl (e.g., phenylcarbamoyl, 1-naphthylcarbamoyl, 2-naphthylcarbamoyl etc.), mono- or di-5 or 6-membered heterocyclic carbamoyl (e.g., 2-pyridylcarbamoyl, 3-pyridylcarbamoyl, 4-pyridylcarbamoyl, 2-thienylcarbamoyl, 3-thienylcarbamoyl etc.), C₁₋₆ alkylsulfonyl (e.g., methylsulfonyl, ethylsulfonyl etc.), C₁₋₆ alkylsulfinyl (e.g., methylsulfinyl, ethylsulfinyl etc.), C₆₋₁₄ arylsulfonyl (e.g., phenylsulfonyl, 1-naphthylsulfonyl, 2-naphthylsulfonyl etc.), C₆₋₁₄ arylsulfinyl (e.g., phenylsulfinyl, 1-naphthylsulfinyl, 2-naphthylsulfinyl etc.), formylamino, C₁₋₆ alkyl-carbonylamino (e.g., acetylamino, propionylamino, pivaloylamino etc.), C₃₋₈ cycloalkyl-carbonylamino (e.g., cyclopentylcarbonylamino, cyclohexylcarbonylamino etc.), C₆₋₁₄ aryl-carbonylamino (e.g., benzoylamino, naphthoylamino etc.), C₁₋₆ alkoxy-carbonylamino (e.g., methoxycarbonylamino, ethoxycarbonylamino, propoxycarbonylamino, butoxycarbonylamino etc.), C₁₋ ₆alkylsulfonylamino (e.g., methylsulfonylamino, ethylsulfonylamino etc.), C₆₋₁₄ arylsulfonylamino (e.g., phenylsulfonylamino, 2-naphthylsulfonylamino, 1-naphthylsulfonylamino etc.), C₁₋₆ alkyl-carbonyloxy (e.g., acetoxy, propionyloxy etc.), C₆₋₁₄ aryl-carbonyloxy (e.g., benzoyloxy, naphthylcarbonyloxy etc.), C₁₋₆ alkoxy-carbonyloxy (e.g., methoxycarbonyloxy, ethoxycarbonyloxy, propoxycarbonyloxy, butoxycarbonyloxy etc.), mono-C₁₋₆ alkyl-carbamoyloxy (e.g., methylcarbamoyloxy, ethylcarbamoyloxy etc.), di-C₁₋₆ alkyl-carbamoyloxy (e.g., dimethylcarbamoyloxy, diethylcarbamoyloxy etc.), mono- or di-C₆₋₁₄ aryl-carbamoyloxy (e.g., phenylcarbamoyloxy, naphthylcarbamoyloxy etc.), nicotinoyloxy, isonicotinoyloxy, optionally having substituents 5- to 7-membered saturated cyclic amino, 5- to 10-membered aromatic heterocyclic group (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl etc.), sulfo, sulfamoyl, sulfinamoyl, sulfenamoyl and a group linked with or 2 or more of these substituents (e.g., 2-3) can be mentioned, with particular preference given to a group represented by the formula: -Zⁿ-Wⁿ-R³ⁿ.

As the salt of Compound (Im), (In), (If'), (Ig') and (Ih'), for example, a metal salt, an ammonium salt, a salt with an organic base, a salt with an inorganic acid, a salt with an organic acid, a salt with a basic or acidic amino acid and the like can be mentioned. As examples of suitable metal salt, alkali metal salts such as sodium salt, potassium salt and the like; alkaline earth metal salts such as calcium salt, magnesium salt, barium salt and the like; aluminum salt and the like can be mentioned. As a suitable example of a salt with an organic base, for example, salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine etc., and the like can be mentioned. As a suitable example of the salt with an inorganic acid, for example, salts with hydrochloric acid, hydrobromic acid, nitric acid, sulfuric acid, phosphoric acid etc., and the like can be mentioned. As a suitable example of the salt with an organic acid, for example, salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid etc., and the like can be mentioned. As a suitable example of the salt with a basic amino acid, for example, salts with arginine, lysine, ornithine etc., and the like can be mentioned. As a suitable example of the salt with an acidic amino acid, for example, salts with aspartic acid, glutamic acid etc., and the like can be mentioned.

Of these, pharmaceutically acceptable salts are preferable. For example, when a compound has an acidic functional group therein, inorganic salts such as alkali metal salts (e.g., sodium salt, potassium salt and the like), alkaline earth metal salts (e.g., calcium salt, magnesium salt, barium salt and the like), and the like, ammonium salts and the like can be mentioned, and when a compound has a basic functional group therein, salts with inorganic acids such as hydrochloric acid, hydrobromic acid, nitric acid, sulfuric acid, phosphoric acid and the like, and salts with organic acids such as acetic acid, phthalic acid, fumaric acid, oxalic acid, tartaric acid, maleic acid, citric acid, succinic acid, methanesulfonic acid, p-toluenesulfonic acid and the like, and the like can be mentioned.

The production methods of Compound (Im) (including (In), (If'), (Ig'), (In')), or a salt thereof of the present invention are explained in the following.

Compound (Im) can be obtained by the methods described in JP-A-60-58981, 61-10580, 5-70446, 7-503023, DE-A-3601411, WO 93/15071, WO 00/64894 and the like or a method analogous thereto and the like, as well as a method shown in the following Reaction Schemes 1, 2 and 3 or a method analogous thereto and the like. Here, the production method of Compound (Im) is briefly described.

Compound (Im) or a salt thereof can be produced by a method characterized by reacting a compound represented by the formula: 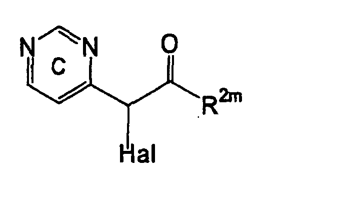 wherein
ring C is a 4-pyrimidinyl group optionally having substituents,
Hal is a halogen, and
R^{2m} is an aromatic group optionally having substituents, or a salt thereof with a compound represented by the formula: R^{1m}CSNH₂ wherein R^{1m} is a hydrogen atom, a hydrocarbon group optionally having substituents, a heterocyclic group optionally having substituents, an amino group optionally having substituents or an acyl group [as regards compound R^{1m}CSNH₂, refer to compound (VII) appearing below in the present specification] or a salt thereof (see Reaction Schemes 1, 2, 3 and 4 below for the detail).

Respective symbols in the compounds in Reaction Schemes 1, 2, 3 and 4 are as defined above. The compounds in Reaction Schemes may form a salt, and as the salt, for example, those similar to the salt of Compound (I), and the like can be mentioned. For Compound (II), (III), (IV), (X), (XI), (XV), (XVI), (XVIII) and (XIX), commercially available compounds can be used, or can be produced according to a method known *per se* or a method analogous thereto.

### Reaction Scheme 1

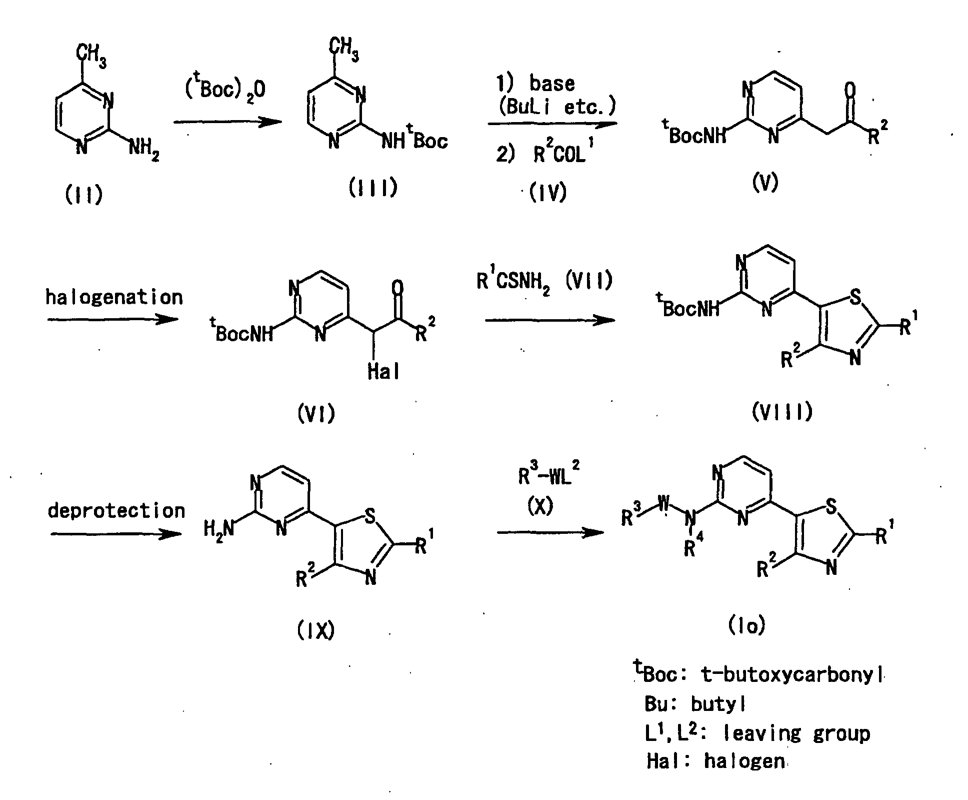

In the following, L¹, L² and L³ (Reaction Scheme 2) each denote a leaving group. The "leaving group" denoted by L¹, L² and L³ is, for example (1) C₁₋₆ alkoxy (e.g., methoxy, ethoxy etc.), (2) di-C₁₋₆ alkylamino (e.g., dimethylamino, diethylamino etc.), (3) N-C₆₋₁₀ aryl-N-C₁₋₆ alkylamino (e.g., N-phenyl-N-methylamino etc.), (4) 3 to 7-membered cyclic amino (e.g., pyrrolidino, morpholino, methylaziridin-1-yl etc.) optionally substituted by C₆₋₁₀ aryl and/or C₁₋₆ alkyl, (5) N-C₁₋₆ alkyl-N-C₁₋ ₆ alkoxyamino (N-methoxy-N-methylamino etc.) and the like, (6) hydroxy, (7) halogen atom (e.g., fluorine, chlorine, bromine, iodine etc.), (8) optionally halogenated C₁₋₅ alkylsulfonyloxy (e.g., methanesulfonyloxy, ethanesulfonyloxy, trifluoromethanesulfonyloxy etc.), (9) C₆₋₁₀ arylsulfonyloxy optionally having substituents, (10) optionally halogenated C₁₋₅ alkylsulfonyl (e.g., methanesulfonyl, ethanesulfonyl, trifluoromethanesulfonyl etc.), (11) C₆₋₁₀ arylsulfonyl optionally having substituents and the like can be mentioned.

As the "C₆₋₁₀ arylsulfonyloxy optionally having substituents", for example, C₆₋₁₀ arylsulfonyloxy (e.g., phenylsulfonyloxy, naphthylsulfonyloxy etc.) optionally having 1 to 3 substituents selected from C₁₋₆ alkyl, C₁₋₆ alkoxy and nitro, and the like can be mentioned. As specific examples, benzenesulfonyloxy, m-nitrobenzenesulfonyloxy, p-toluenesulfonyloxy and the like can be mentioned.

As the "C₆₋₁₀ arylsulfonyl optionally having substituents", for example, C₆₋₁₀ arylsulfonyl (e.g., phenylsulfonyl, naphthylsulfonyl etc.) optionally having 1 to 3 substituents selected from C₁₋₆ alkyl, C₁₋₆ alkoxy and nitro, and the like can be mentioned. As specific examples, benzenesulfonyl, m-nitrobenzenesulfonyl, p-toluenesulfonyl and the like can be mentioned.

Compound (III) is obtained by protecting Compound (II) with di-t-butyl dicarbonate.

The amount of di-t-butyl dicarbonate to be used is about 0.8 to about 5 moles, preferably about 1 to about 1.5 moles, per 1 mole of Compound (II).

It is advantageous to carry out this reaction without a solvent or in the presence of an inert solvent for the reaction. The solvent is not particularly limited as long as the reaction proceeds, but, for example, aromatic hydrocarbons, ethers, alcohols, esters or a mixture of two or more of them and the like are used.

The reaction temperature is usually about 0 to about 100°C, preferably about 0 to about 60°C. The reaction time is usually about 5 minutes to about 48 hours, preferably about 1 hour to about 24 hours.

Although the product can be used as a reaction solution itself or as a crude product in the next reaction, it can be isolated from the reaction mixture by a conventional method, and can be easily purified by a separating methods such as recrystallization, distillation, chromatography and the like.

To obtain Compound (V), Compound (III) is treated with a base, followed by condensing with Compound (IV).

The amount of base to be used is about 0.8 to about 5 moles, preferably about 2 to about 2.5 moles, per 1 mole of Compound (III).

As the "base", for example, alkyl lithiums such as n-butyl lithium and the like, and metal amides such as sodium amide, lithium diisopropylamide, lithium hexamethyldisilazide and the like are used.

It is advantageous to carry out this reaction without a solvent or in the presence of an inert solvent for the reaction. The solvent is not particularly limited as long as the reaction proceeds, but, for example, aliphatic hydrocarbons, aromatic hydrocarbons, ethers or a mixture of two or more of them and the like are used.

The reaction temperature is usually about -78 to about 60°C, preferably about -78 to about 20°C. The reaction time is usually about 5 minutes to about 24 hours, preferably about 0.5 hour to about 3 hours.

Although the product can be used as a reaction solution itself or as a crude product in the next reaction, it can be isolated from the reaction mixture by a conventional method, and can be easily purified by a separating methods such as recrystallization, distillation, chromatography and the like.

Compound (VI) can be obtained by treating Compound (V) with a halogen or metal halide. Where desired, this reaction is carried out in the presence of a base or a basic salt.

As the "halogen", chlorine, bromine, iodine and the like can be mentioned.

As the "metal halide", copper halides such as copper(II) bromide, copper(II) chloride and the like can be mentioned.

Accordingly, in Compound (VI), Hal is halogen such as chlorine, bromine, iodine and the like.

The amount of halogen or metal halide to be used is about 1 to about 5 moles, preferably about 1 to about 2 moles, per 1 mole of compound (V).

The amount of a base to be used is about 1 to about 10 moles, preferably about 1 to about 3 moles, per 1 mole of Compound (V).

As the "base", for example, metal hydroxides such as sodium hydroxide, potassium hydroxide, lithium hydroxide and the like, basic salts such as sodium carbonate, potassium carbonate, cesium carbonate, sodium hydrogen carbonate, sodium acetate and the like, aromatic amines such as pyridine, lutidine and the like, tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, and the like can be mentioned.

It is advantageous to carry out this reaction without a solvent or in the presence of an inert solvent for the reaction. The solvent is not particularly limited as long as the reaction proceeds, but, for example, ethers, esters, aromatic hydrocarbons, aliphatic hydrocarbons, amides, halogenated hydrocarbons, nitriles, sulfoxides, organic acids, aromatic amines or a mixture of two or more of them and the like are used.

The reaction temperature is about -20 to about 150°C, preferably about 0 to about 100°C. The reaction time is usually about 5 minutes to about 24 hours, preferably about 10 minutes to about 5 hours.

Although the product can be used as a reaction solution itself or as a crude product in the next reaction, it can be isolated from the reaction mixture by a conventional method, and can be easily purified by a separating methods such as recrystallization, distillation, chromatography and the like.

Compound (VIII) can be obtained by condensing Compound (VI) with Compound (VII). This reaction is performed optionally in the presence of a base.

When Compound (VII) is commercially available, it can be used as it is, or is obtained by a method known *per se* or a method according to a known method, or further by a method shown by the following Reaction Scheme 4.

The amount of Compound (VII) to be used is about 0.5 to about 3 moles, preferably about 0.8 to about 2 moles, per 1 mole of Compound (VI).

The amount of a base to be used is about 1 to about 30 moles, preferably about 1 to about 10 moles, per 1 mole of Compound (VI).

As the "base", for example, basic salts such as sodium carbonate, potassium carbonate, cesium carbonate, sodium hydrogen carbonate, sodium acetate and the like, aromatic amines such as pyridine, lutidine and the like, tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, and the like can be mentioned.

It is advantageous to carry out this reaction without a solvent or in the presence of an inert solvent for the reaction. The solvent is not particularly limited as long as the reaction proceeds, but, for example, halogenated hydrocarbons, aliphatic hydrocarbons, aromatic hydrocarbons, ethers, amides, alcohols, nitriles or a mixture of two or more of them and the like are used.

The reaction temperature is about -5 to about 200°C, preferably about 5 to about 150°C. The reaction time is usually about 5 minutes to about 72 hours, preferably about 0.5 to about 30 hours.

Although the product can be used as a reaction solution itself or as a crude product in the next reaction, it can be isolated from the reaction mixture by a conventional method, and can be easily purified by a separating methods such as recrystallization, distillation, chromatography and the like.

Compound (IX) is obtained by deprotecting Compound (VIII) using an acid or a base.

The amount of an acid or a base to be used is about 0.1 to about 50 moles, preferably about 1 to about 20 moles, per 1 mole of Compound (VIII).

As the "acid", for example, mineral acids such as hydrochloric acid, hydrobromic acid, sulfuric acid and the like, Lewis acids such as boron trichloride, boron tribromide and the like, the use of Lewis acid together with thiols or sulfides, organic acids such as trifluoroacetic acid, p-toluenesulfonic acid and the like, and the like are used.

As the "base", for example, metal hydroxides such as sodium hydroxide, potassium hydroxide, barium hydroxide and the like, basic salts such as sodium carbonate, potassium carbonate and the like, metal alkoxides such as sodium methoxide, sodium ethoxide, potassium tert-butoxide and the like, organic bases such as triethylamine, imidazole, formamidine and the like, and the like are used.

It is advantageous to carry out this reaction without a solvent or in the presence of an inert solvent for the reaction. The solvent is not particularly limited as long as the reaction proceeds, but, for example, alcohols, ethers, aromatic hydrocarbons, aliphatic hydrocarbons, halogenated hydrocarbons, sulfoxides, water or a mixture of two or more of them and the like are used.

The reaction time is usually about 10 minutes to about 50 hours, preferably about 30 minutes to about 12 hours. The reaction temperature is usually about 0 to about 200°C, preferably about 20 to about 120°C.

Compound (Io) can be obtained by condensing Compound (IX) with Compound (X) optionally in the presence of a base.

The amount of Compound (XVIII) to be used is about 0.8 to about 5 moles, preferably about 1 to about 3 moles, per 1 mole of Compound (XVII).

The amount of the base to be used is about 0.1 to about 5 moles, preferably about 0.8 to about 2.5 moles, per 1 mole of Compound (XVII).

As the "base", for example, basic salts such as sodium carbonate, potassium carbonate, cesium carbonate, sodium acetate and the like, metal hydroxides such as sodium hydroxide, potassium hydroxide and the like, aromatic amines such as pyridine, lutidine and the like, tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, alkali metal hydrides such as sodium hydride, potassium hydride and the like, metal amides such as sodium amide, lithium diisopropylamide, lithium hexamethyldisilazide and the like, metal alkoxides such as sodium methoxide, sodium ethoxide, potassium tert-butoxide and the like, and the like can be mentioned.

It is advantageous to carry out this reaction without a solvent or in the presence of an inert solvent for the reaction. The solvent is not particularly limited as long as the reaction proceeds, but, for example, aliphatic hydrocarbons, aromatic hydrocarbons, ethers, amides or a mixture of two or more of them and the like are used.

The reaction temperature is usually about -78 to about 100°C, preferably about -78 to about 70°C. The reaction time is usually about 5 minutes to about 24 hours, preferably about 0.5 to about 20 hours.

Although the product can be used as a reaction solution itself or as a crude product in the next reaction, it can be isolated from the reaction mixture by a conventional method, and can be easily purified by a separating methods such as recrystallization, distillation, chromatography and the like. Thereafter, compounds wherein R⁴ is other than hydrogen atom can be synthesized by performing alkylation or acylation and the like, if desired.

### Reaction Scheme 2

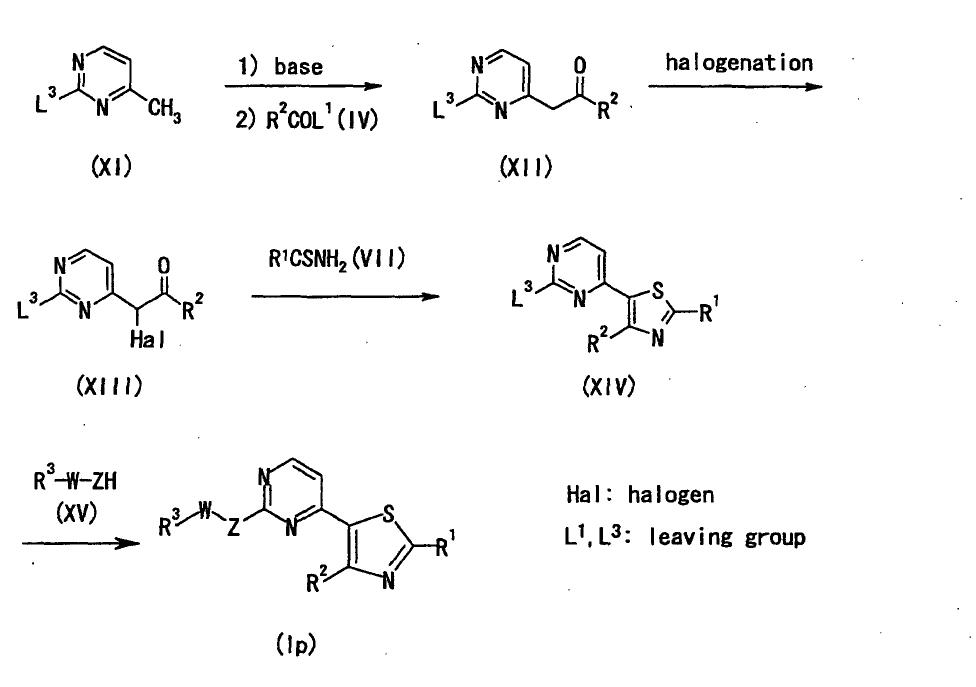

Compound (XII) can be obtained by treating Compound (XI) with a base and condensing Compound (IV).

The amount of the base to be used is about 0.8 to about 3 moles, preferably about 1 to about 1.2 moles, per 1 mole of compound (XI).

As the "base", for example, metal amides such as sodium amide, lithium diisopropylamide, lithium hexamethyldisilazide and the like are used.

It is advantageous to carry out this reaction without a solvent or in the presence of an inert solvent for the reaction. The solvent is not particularly limited as long as the reaction proceeds, but, for example, aliphatic hydrocarbons, aromatic hydrocarbons, ethers or a mixture of two or more of them and the like are used.

The reaction temperature is usually about -78 to about 60°C, preferably about -78 to about 20°C. The reaction time is usually about 5 minutes to about 24 hours, preferably about 0.5 to about 3 hours.

Although the product can be used as a reaction solution itself or as a crude product in the next reaction, it can be isolated from the reaction mixture by a conventional method, and can be easily purified by a separating methods such as recrystallization, distillation, chromatography and the like.

Compound (XIII) can be obtained by treating Compound (XII) with a halogen or metal halides. Where desired, this reaction is carried out in the presence of a base or a basic salt.

As the "halogen", chlorine, bromine, iodine and the like can be mentioned.

As the "metal halide", copper halides such as copper(II) bromide, copper(II) chloride and the like can be mentioned.

Accordingly, in compound (XIII), Hal methods halogen such as chlorine, bromine, iodine and the like.

The amount of halogen or metal halide to be used is about 1 to about 5 moles, preferably about 1 to about 2 moles, per 1 mole of compound (XIII).

The amount of the base to be used is about 1 to about 10 moles, preferably about 1 to about 3 moles, per 1 mole of Compound (XII).

As the "base", for example, metal hydroxides such as sodium hydroxide, potassium hydroxide, lithium hydroxide, barium hydroxide and the like, basic salts such as sodium carbonate, potassium carbonate, cesium carbonate, sodium hydrogen carbonate, sodium acetate and the like, aromatic amines such as pyridine, lutidine and the like, tertiary amines such as a triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, and the like can be mentioned.

It is advantageous to carry out this reaction without a solvent or in the presence of an inert solvent for the reaction. The solvent is not particularly limited as long as the reaction proceeds, but, for example, ethers, esters, aromatic hydrocarbons, aliphatic hydrocarbons, amides, halogenated hydrocarbons, nitriles, sulfoxides, organic acids, aromatic amines or a mixture of two or more of them and the like are used.

The reaction temperature is about -20 to about 150°C, preferably about 0 to about 100°C. The reaction time is usually about 5 minutes to about 24 hours, preferably about 10 minutes to about 5 hours.

Although the product can be used as a reaction solution itself or as a crude product in the next reaction, it can be isolated from the reaction mixture by a conventional method, and can be easily purified by a separating methods such as recrystallization, distillation, chromatography and the like.

Compound (XIV) can be obtained by condensing Compound (XIII) with Compound (VII). Where desired, this reaction is carried out in the presence of a base.

The amount of Compound (VII) to be used is about 0.5 to about 3 moles, preferably about 0.8 to about 2 moles, per 1 mole of Compound (XIII).

The amount of the base to be used is about 1 to about 30 moles, preferably about 1 to about 10 moles, per 1 mole of compound (XIII).

As the "base", for example, basic salts such as sodium carbonate, potassium carbonate, cesium carbonate, sodium hydrogen carbonate, sodium acetate and the like, aromatic amines such as pyridine, lutidine and the like, tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, and the like can be mentioned.

It is advantageous to carry out this reaction without a solvent or in the presence of an inert solvent for the reaction. The solvent is not particularly limited as long as the reaction proceeds, but, for example, halogenated hydrocarbons, aliphatic hydrocarbons, aromatic hydrocarbons, ethers, amides, alcohols, nitriles or a mixture of two or more of them and the like are used.

The reaction temperature is about -5 to about 200°C, preferably about 5 to about 150°C. The reaction time is usually about 5 minutes to about 72 hours, preferably about 0.5 hour to about 30 hours.

Although the product can be used as a reaction solution itself or as a crude product in the next reaction, it can be isolated from the reaction mixture by a conventional method, and can be easily purified by a separating methods such as recrystallization, distillation, chromatography and the like.

Compound (Ip) can be obtained by condensing Compound (XIV) with Compound (XV).

Where desired, this reaction is carried out in the presence of a base.

The amount of Compound (XV) to be used is about 1 to about 100 moles, preferably about 1 to about 30 moles, per 1 mole of Compound (XIV).

As the "base", for example, basic salts such as sodium carbonate, potassium carbonate, cesium carbonate, sodium hydrogen carbonate, sodium acetate and the like, aromatic amines such as pyridine, lutidine and the like, tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, alkali metal hydrides such as sodium hydride, potassium hydride and the like, metal amides such as sodium amide, lithium diisopropylamide, lithium hexamethyldisilazide and the like, metal alkoxides such as sodium methoxide, sodium ethoxide, potassium tert-butoxide and the like, and the like can be mentioned.

It is advantageous to carry out this reaction without a solvent or in the presence of an inert solvent for the reaction. The solvent is not particularly limited as long as the reaction proceeds, but, for example, halogenated hydrocarbons, aliphatic hydrocarbons, aromatic hydrocarbons, ethers, sulfoxides, alcohols, nitriles, ketones or a mixture of two or more of them and the like are used.

The reaction temperature is about -5 to about 200°C, preferably about 5 to about 120°C. The reaction time is usually about 5 minutes to about 72 hours, preferably about 0.5 hour to about 30 hours.

Although the product can be used as a reaction solution itself or as a crude product in the next reaction, it can be isolated from the reaction mixture by a conventional method, and can be easily purified by a separating methods such as recrystallization, distillation, chromatography and the like.

### Reaction Scheme 3

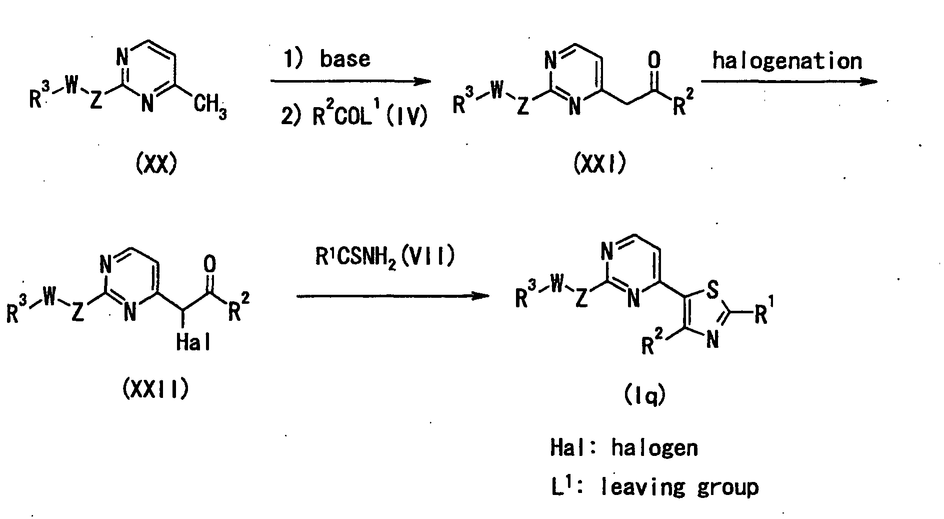

Compound (XXI) can be obtained by treating Compound (XX) with a base and condensing Compound (IV).

The amount of the base to be used is about 0.8 to about 3 moles, preferably about 1 to about 1.2 moles, per 1 mole of Compound (XX).

As the "base", for example, metal amides such as sodium amide, lithium diisopropylamide, lithium hexamethyldisilazide and the like are used.

It is advantageous to carry out this reaction without a solvent or in the presence of an inert solvent for the reaction. The solvent is not particularly limited as long as the reaction proceeds, but, for example, aliphatic hydrocarbons, aromatic hydrocarbons, ethers or a mixture of two or more of them and the like are used.

The reaction temperature is usually about -78 to about 60°C, preferably about -78 to about 20°C. The reaction time is usually about 5 minutes to about 24 hours, preferably about 0.5 to about 3 hours.

Although the product can be used as a reaction solution itself or as a crude product in the next reaction, it can be isolated from the reaction mixture by a conventional method, and can be easily purified by a separating methods such as recrystallization, distillation, chromatography and the like.

Compound (XXII) can be obtained by treating Compound (XXI) with halogen or metal halide. Where desired, this reaction is carried out in the presence of a base or a basic salt.

As the "halogen", chlorine, bromine, iodine and the like can be mentioned.

As the "metal halide", copper halide such as copper(II) bromide, copper(II) chloride and the like can be mentioned.

Accordingly, Hal in Compound (XXII) methods halogen such as chlorine, bromine, iodine and the like.

The amount of the halogen or metal halide to be used is about 1 to about 5 moles, preferably about 1 to about 2 moles, per 1 mole of Compound (XXI).

The amount of the base to be used is about 1 to about 10 moles, preferably about 1 to about 3 moles, per 1 mole of Compound (XXI).

As the "base", for example, metal hydroxides such as sodium hydroxide, potassium hydroxide, lithium hydroxide, barium hydroxide and the like, basic salts such as sodium carbonate, potassium carbonate, cesium carbonate, sodium hydrogen carbonate, sodium acetate and the like, aromatic amines such as pyridine, lutidine and the like, tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, and the like can be mentioned.

It is advantageous to carry out this reaction without a solvent or in the presence of an inert solvent for the reaction. The solvent is not particularly limited as long as the reaction proceeds, but, for example, ethers, esters, aromatic hydrocarbons, aliphatic hydrocarbons, amides, halogenated hydrocarbons, nitriles, sulfoxides, organic acids, aromatic amines or a mixture of two or more of them and the like are used.

The reaction temperature is about -20 to about 150°C, preferably about 0 to about 100°C. The reaction time is usually about 5 minutes to about 24 hours, preferably about 10 minutes to about 5 hours.

Although the product can be used as a reaction solution itself or as a crude product in the next reaction, it can be isolated from the reaction mixture by a conventional method, and can be easily purified by a separating methods such as recrystallization, distillation, chromatography and the like.

Compound (Iq) can be obtained by condensing Compound (XXII) with Compound (VII). Where desired, this reaction is carried out in the presence of a base.

The amount of Compound (VII) to be used is about 0.5 to about 3 moles, preferably about 0.8 to about 2 moles, per 1 mole of Compound (XXII).

The amount of the base to be used is about 1 to about 30 moles, preferably about 1 to about 10 moles, per 1 mole of Compound (XXII).

As the "base", for example, basic salts such as sodium carbonate, potassium carbonate, cesium carbonate, sodium hydrogen carbonate, sodium acetate and the like, aromatic amines such as pyridine, lutidine and the like, tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, and the like can be mentioned.

It is advantageous to carry out this reaction without a solvent or in the presence of an inert solvent for the reaction. The solvent is not particularly limited as long as the reaction proceeds, but, for example, halogenated hydrocarbons, aliphatic hydrocarbons, aromatic hydrocarbons, ethers, amides, alcohols, nitriles or a mixture of two or more of them and the like are used.

The reaction temperature is about -5 to about 200°C, preferably about 5 to about 150°C. The reaction time is usually about 5 minutes to about 72 hours, preferably about 0.5 hour to about 30 hours.

Although the product can be used as a reaction solution itself or as a crude product in the next reaction, it can be isolated from the reaction mixture by a conventional method, and can be easily purified by a separating methods such as recrystallization, distillation, chromatography and the like.

### Reaction Scheme 4

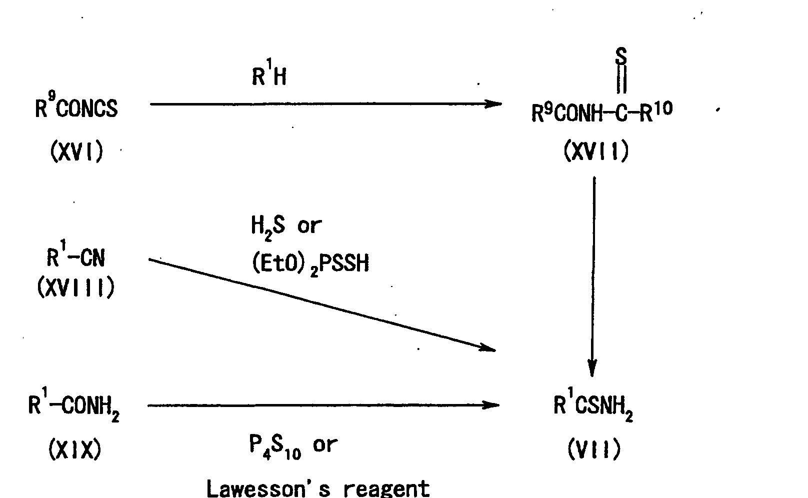

wherein R¹⁰ is an amino group optionally having substituents, and other symbols are as defined above.

Compound (XVII) can be obtained by condensing Compound (XVI) with amines represented by the formula: R¹⁰H (e.g., 1-propylamine, 1-butylamine, pyrrolidine, piperidine, piperazine, 4-methylpiperazine, 4-phenylpiperidine and the like, preferably, pyrrolidine, piperidine, piperazine, 4-methylpiperazine etc.).

In Compound (XVII), R⁹ is an aromatic hydrocarbon group or alkoxy. As the "aromatic hydrocarbon group", phenyl group optionally having substituents and the like can be mentioned. As the "alkoxy", for example, C₁₋₆ alkoxy such as methoxy, ethoxy, propoxy, isopropoxy, butoxy and the like, and the like can be mentioned.

The amount of the "amines" to be used is about 1.0 to about 30 moles, preferably about 1.0 to about 10 moles, per 1 mole of Compound (XVI).

It is advantageous to carry out this reaction without a solvent or in the presence of an inert solvent for the reaction. The solvent is not particularly limited as long as the reaction proceeds, but, for example, halogenated hydrocarbons, aliphatic hydrocarbons, aromatic hydrocarbons, ethers, amides, alcohols, nitriles, ketones or a mixture of two or more of them and the like are used.

The reaction temperature is about -5 to about 200°C, preferably about 5 to about 120°C. The reaction time is usually about 5 minutes to about 72 hours, preferably about 0.5 to about 30 hours.

Although the product can be used as a reaction solution itself or as a crude product in the next reaction, it can be isolated from the reaction mixture by a conventional methods, and can be easily purified by a separating methods such as recrystallization, distillation, chromatography and the like.

Compound (VII) is obtained by hydrolyzing Compound (XVII) using an acid or a base.

The amount of acid or base to be used is about 0.1 to about 50 moles, preferably about 1 to about 20 moles, per 1 mole of Compound (XVII), respectively.

As the "acid", for example, mineral acids such as hydrochloric acid, hydrobromic acid, sulfuric acid and the like, Lewis acids such as boron trichloride, boron tribromide and the like, the use of Lewis acid together with thiols or sulfides, organic acids such as trifluoroacetic acid, p-toluenesulfonic acid and the like, and the like are used.

As the "base", for example, metal hydroxides such as sodium hydroxide, potassium hydroxide, barium hydroxide and the like, basic salts such as sodium carbonate, potassium carbonate and the like, metal alkoxides such as sodium methoxide, sodium ethoxide, potassium tert-butoxide and the like, organic bases such as triethylamine, imidazole, formamidine and the like, and the like are used.

It is advantageous to carry out this reaction without a solvent or in the presence of an inert solvent for the reaction. The solvent is not particularly limited as long as the reaction proceeds, but, for example, alcohols, ethers, aromatic hydrocarbons, aliphatic hydrocarbons, halogenated hydrocarbons, sulfoxides, water or a mixture of two or more of them and the like are used.

The reaction time is usually about 10 minutes to about 50 hours, preferably about 30 minutes to about 12 hours. The reaction temperature is usually about 0 to about 200°C, preferably about 20 to about 120°C.

Compound (VII) can be also obtained by treating Compound (XVIII) with hydrogen sulfide in the presence of a base.

The amount of hydrogen sulfide to be used is about 1 to about 30 moles, per 1 mole of Compound (XVIII).

The amount of base to be used is about 1.0 to about 30 moles, preferably about 1.0 to about 10 moles, per 1 mole of Compound (XVIII).

As the "base", for example, aromatic amines such as pyridine, lutidine and the like, tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, and the like can be mentioned.

It is advantageous to carry out this reaction without a solvent or in the presence of an inert solvent for the reaction. The solvent is not particularly limited as long as the reaction proceeds, but, for example, halogenated hydrocarbons, aliphatic hydrocarbons, aromatic hydrocarbons, ethers, aromatic amines or a mixture of two or more of them and the like are used.

This reaction is performed under atmospheric pressure or under a pressurized condition. The reaction temperature is usually about -20 to about 80°C, preferably about -10 to about 30°C. The reaction time is usually about 5 minutes to about 72 hours, preferably about 0.5 hour to about 30 hours.

Although the product can be used as a reaction solution itself or as a crude product in the next reaction, it can be isolated from the reaction mixture by a conventional methods, and can be easily purified by a separating methods such as recrystallization, distillation, chromatography and the like.

Compound (VII) can be also obtained by treating compound (XVIII) with O,O-diethyl dithiophosphate in the presence of an acid.

The amount of O,O-diethyl dithiophosphate to be used is about 0.9 to about 2 moles, relative to 1 mole of Compound (XVIII).

The amount of acid to be used is about 3.0 to about 30 moles, preferably about 3.0 to about 10 moles, per 1 mole of Compound (XVIII).

As the acid, for example, hydrogen halides such as hydrogen chloride, hydrogen bromide and the like, mineral acids such as hydrochloric acid, hydrobromic acid and the like, and the like are used.

It is advantageous to carry out this reaction without a solvent or in the presence of an inert solvent for the reaction. The solvent is not particularly limited as long as the reaction proceeds, but, for example, halogenated hydrocarbons, alcohols, amides, ethers, esters, water or a mixture of two or more of them and the like are used.

The reaction temperature is generally about 0 to about 80°C, preferably about 0 to about 30°C. The reaction time is generally about 5 minutes to about 72 hours, preferably about 0.5 hour to about 30 hours.

Although the product can be used as a reaction solution itself or as a crude product in the next reaction, it can be isolated from the reaction mixture by a conventional methods, and can be easily purified by a separating methods such as recrystallization, distillation, chromatography and the like.

Compound (VII) can also be obtained by treating Compound (XIX) with phosphorus pentasulfide or Lawesson's reagent.

The amount of the phosphorus pentasulfide or Lawesson's reagent to be used is about 0.5 to about 10 moles, preferably about 0.5 to about 3 moles, per 1 mole of Compound (XIX).

It is advantageous to carry out this reaction without a solvent or in the presence of an inert solvent for the reaction. The solvent is not particularly limited as long as the reaction proceeds, but, for example, ethers, aromatic hydrocarbons, aliphatic hydrocarbons, halogenated hydrocarbons or a mixture of two or more of them and the like are used.

The reaction time is usually 10 minutes to about 50 hours, preferably about 30 minutes to about 12 hours. The reaction temperature is usually about 0 to about 150°C, preferably about 20 to about 120°C.

Although the product (VII) can be used as a reaction solution itself or as a crude product in the next reaction, it can be isolated from the reaction mixture by a conventional methods, and can be easily purified by a separating methods such as recrystallization, distillation, chromatography and the like.

When Compound (Im) is an acylamino compound, the objective compound can be also obtained by subjecting the corresponding amine compound to an acylating reaction known *per se.*

Of Compound (Im), for example, a compound wherein R¹ is acylamino group optionally having substituents is obtained by reacting the corresponding 2-thiazolamine and an acylating agent optionally in the presence of a base or an acid.

The amount of the acylating agent to be used is about 1 to about 5 moles, preferably about 1 to about 2 moles, per 1 mole of the corresponding 2-thiazolamine.

As the "acylating agent", for example, carboxylic acids corresponding to an objective acyl group or a reactive derivative thereof (e.g., acid halide, acid anhydride, ester and the like) and the like can be mentioned.

The amount of the base or acid to be used is about 0.8 to about 5 moles, preferable about 1 to about 2 moles, per 1 mole of the corresponding 2-thiazolamine.

As the "base", for example, triethylamine, pyridine, 4-dimethylaminopyridine and the like can be mentioned.

As the "acid", for example, methanesulfonic acid, p-toluenesulfonic acid, camphorsulfonic acid and the like can be mentioned.

It is advantageous to carry out this reaction without a solvent or in the presence of an inert solvent for the reaction. The solvent is not particularly limited as long as the reaction proceeds, but, for example, ethers, aromatic hydrocarbons, aliphatic hydrocarbons, amides, halogenated hydrocarbons, nitriles, sulfoxides, aromatic amines or a mixture of two or more of them and the like are used.

The reaction temperature is about -20 to about 150°C, preferably about 0 to about 100°C. The reaction time is usually 5 minutes to about 24 hours, preferably about 10 minutes to about 5 hours.

Although the product can be used as a reaction solution itself or as a crude product in the next reaction, it can be isolated from the reaction mixture by a conventional methods, and can be easily purified by a separating methods such as recrystallization, distillation, chromatography and the like.

When Compound (Im) is an N-oxide compound, it is obtained by treating the corresponding pyrimidine compound with an organic peroxy acid.

The amount of the organic peroxy acid to be used is about 0.8 to about 10 moles, preferable about 1.0 to about 3.0 moles, per 1 mole of the corresponding pyrimidine compound.

As the "organic peroxy acid", for example, peracetic acid, trifluoroperacetic acid, m-chloroperbenzoic acid and the like can be mentioned.

It is advantageous to carry out this reaction without a solvent or in the presence of an inert solvent for the reaction. The solvent is not particularly limited as long as the reaction proceeds, but, for example, halogenated hydrocarbons, aliphatic hydrocarbons, aromatic hydrocarbons, organic acids, ethers, amides, sulfoxides, alcohols, nitriles, ketones or a mixture of two or more of them and the like are used.

The reaction temperature is about -20°C to about 130°C, preferably about 0 to about 100°C. The reaction time is usually 5 minutes to about 72 hours, preferably about 0.5 hour to about 12 hours.

Alternatively, the N-oxide compound is also obtained by treating the corresponding pyrimidine compound with hydrogen peroxide or alkyl hydroperoxide in the presence of a base, an acid or a metal oxide, if desired.

The amount of the hydrogen peroxide or alkyl hydroperoxide to be used is about 0.8 to about 10 moles, preferably about 1.0 to about 3.0 moles, per 1 mole of the corresponding pyrimidine compound.

As the "alkyl hydroperoxide", for example, tert-butyl hydroperoxide, cumene hydroperoxide and the like can be mentioned.

The amount of the base, acid or metal oxide to be used is about 0.1 to about 30 moles, preferably 0.8 to about 5 moles, per 1 mole of the corresponding pyrimidine compound.

As the "base", for example, inorganic bases such as sodium hydroxide, potassium hydroxide and the like, basic salts such as sodium carbonate, potassium carbonate and the like, and the like can be mentioned.

As the "acid", for example, mineral acids such as hydrochloric acid, sulfuric acid, perchloric acid and the like, Lewis acids such as boron trifluoride, aluminum chloride, titanium tetrachloride and the like, organic acids such as formic acid, acetic acid and the like, and the like can be mentioned.

As the "metal oxide", for example, vanadium oxide (e.g., V₂O₅ etc.), osmium tetroxide (OsO₄), tungsten oxide (e.g. , WO₃ etc.), molybdenum oxide (e.g., MoO₃ etc.), selenium dioxide (SeO₂), chromium oxide (e.g., CrO₃ etc.) and the like can be mentioned.

It is advantageous to carry out this reaction without a solvent or in the presence of an inert solvent for the reaction. The solvent is not particularly limited as long as the reaction proceeds, but, for example, halogenated hydrocarbons, aliphatic hydrocarbons, aromatic hydrocarbons, organic acids, ethers, amides, sulfoxides, alcohols, nitriles, ketones or a mixture of two or more of them and the like are used.

The reaction temperature is about -20°C to about 130°C, preferably about 0°C to about 100°C. The reaction time is usually 5 minutes to about 72 hours, preferably about 0.5 hour to about 12 hours.

Although the product can be used as a reaction solution itself or as a crude product in the next reaction, it can be a mixture isolated by a conventional method, and can be easily purified by a separating methods such as recrystallization, distillation, chromatography and the like.

When compound (Im) is an S-oxide compound, it can be obtained by treating the corresponding sulfide compound with peroxide.

The amount of peroxide to be used is about 0.8 to about 10 moles, preferably about 1.0 to about 3.0 moles, relative to 1 mole of the corresponding sulfide compound.

As the "peroxide", for example, peracetic acid, trifluoroperacetic acid, m-chloroperbenzoic acid, potassium persulfate, metaperiodic acid and the like can be mentioned.

It is advantageous to carry out this reaction without a solvent or in the presence of an inert solvent for the reaction. The solvent is not particularly limited as long as the reaction proceeds, but, for example, halogenated hydrocarbons, aliphatic hydrocarbons, aromatic hydrocarbons, organic acids, ethers, amides, sulfoxides, alcohols, nitriles, ketones or a mixture of two or more of them and the like are used.

The reaction temperature is about -20°C to about 130°C, preferably about 0°C to about 100°C. The reaction time is usually about 5 minutes to about 72 hours, preferably about 0.5 hour to about 12 hours.

In addition, an S-oxide compound can be obtained by treating the corresponding sulfide compound with hydrogen peroxide or alkyl hydroperoxide in the presence of a base, acid and/or metal oxide, if desired.

The amount of the hydrogen peroxide or alkyl hydroperoxide to be used is about 0.8 to about 10 moles, preferably about 1.0 to about 3.0 moles, per 1 mole of the corresponding sulfide compound.

As the "alkyl hydroperoxide", for example, tert-butyl hydroperoxide, cumene hydroperoxide and the like can be mentioned.

The amount of the "base, acid or metal oxide" to be used is about 0.1 to about 30 moles, preferably about 0.8 to about 5 moles, per 1 mole of the corresponding sulfide compound.

As the "base", for example, inorganic bases such as sodium hydroxide, potassium hydroxide and the like, basic salts such as sodium carbonate, potassium carbonate and the like, and the like can be mentioned.

As the "acid", for example, mineral acids such as hydrochloric acid, sulfuric acid, perchloric acid and the like, Lewis acids such as boron trifluoride, aluminum chloride, titanium tetrachloride and the like, organic acids such as formic acid, acetic acid and the like, and the like can be mentioned.

As the "metal oxide", for example, vanadium oxide (e.g., V₂O₅ etc.), osmium tetroxide (OsO₄), tungsten oxide (e.g., WO₃ etc.), molybdenum oxide (e.g., MoO₃ etc.), selenium dioxide (SeO₂), chromium oxide (e.g., CrO₃ etc.) and the like can be mentioned.

It is advantageous to carry out this reaction without a solvent or in the presence of an inert solvent for the reaction. The solvent is not particularly limited as long as the reaction proceeds, but, for example, halogenated hydrocarbons, aliphatic hydrocarbons, aromatic hydrocarbons, organic acids, ethers, amides, sulfoxides, alcohols, nitriles, ketones or a mixture of two or more of them and the like are used.

The reaction temperature is about -20°C to about 130°C, preferably about 0°C to about 100°C. The reaction time is usually about 5 minutes to about 72 hours, preferably about 0.5 to about 12 hours.

Although the product can be used as a reaction solution itself or as a crude product in the next reaction, it can be a mixture isolated by a conventional methods, and can be easily purified by a separating methods such as recrystallization, distillation, chromatography and the like.

In the respective reactions mentioned above, when starting compounds have amino, carboxy, hydroxy as substituents, a protecting groups which are generally used in the peptide chemistry or the like may be introduced into these groups and, after reaction, a desired compound can be obtained by removing protecting groups if needed.

As a protecting group for amino, for example, formyl or C₁₋₆ alkyl-carbonyl (e.g., acetyl, propionyl and the like), phenylcarbonyl, C₁₋₆ alkoxy-carbonyl (e.g., methoxycarbonyl, ethoxycarbonyl and the like), phenyloxycarbonyl, C₇₋₁₀ aralkyloxy-carbonyl (e.g., benzyloxycarbonyl and the like), trityl, phthaloyl and the like, which may have substituents are used. As these substituents, halogen atom(s) (e.g., fluorine, chlorine, bromine, iodine and the like), C₁₋₆ alkyl-carbonyl (e.g., acetyl, propionyl, valeryl and the like), nitro and the like are used and the number of substituents is 1 to 3.

As a protecting group for carboxy, for example, C₁₋₆ alkyl (e.g., methyl, ethyl, propyl, isopropyl, butyl, tert-butyl and the like), phenyl, trityl, silyl and the like, which may have substituents, are used. As these substituents, halogen atom(s) (e.g., fluorine, chlorine, bromine, iodine and the like), formyl, C₁₋₆ alkyl-carbonyl (e.g., acetyl, propionyl, butylcarbonyl and the like), nitro, C₁₋₆ alkyl (e.g., methyl, ethyl, tert-butyl and the like), C₆₋₁₀ aryl (e.g., phenyl, naphthyl and the like) and the like are used and the number of substituents is 1 to 3.

As a protecting group for hydroxy, for example, C₁₋₆ alkyl (e.g., methyl, ethyl, propyl, isopropyl, butyl, tert-butyl and the like), phenyl, C₇₋₁₁ aralkyl (e.g., benzyl and the like), formyl, C₁₋₆ alkyl-carbonyl (e.g., acetyl, propionyl and the like), phenyloxycarbonyl, C₇₋₁₁ aralkyloxy-carbonyl (e.g., benzyloxycarbonyl and the like), tetrahydropyranyl, tetrahydrofuranyl, silyl and the like, which may have substituents, are used. As these substituents, halogen atom(s) (e.g., fluorine, chlorine, bromine, iodine and the like), C₁₋₆ alkyl (e.g., methyl, ethyl, tert-butyl and the like), C₇₋₁₁ aralkyl (e.g., benzyl and the like), C₆₋₁₀ aryl (e.g., phenyl, naphthyl and the like), nitro and the like are used, wherein the number of substituents is 1 to 4.

In addition, as a method of removing a protecting group, a method known *per se* or a method according to such method is used, and, for example, method by treating with an acid, a base, ultraviolet rays, hydrazine, phenylhydrazine, sodium N-methyldithiocarbamate, tetrabutylammonium fluoride, palladium acetate and the like or a method of reduction is used.

In any case, Compound (I) can be synthesized by optionally applying further known deprotection, acylation, alkylation, hydrogenation, oxidation, reduction, carbon chain extension and substituent exchange reactions alone or a combination of two or more of them. As these reactions, those described in, for example, Shinjikkenkagakukoza 14, vol.15, 1977 (Maruzen Press) and the like are adopted.

As the above "alcohols", for example, methanol, ethanol, propanol, isopropanol, tert-butanol and the like can be mentioned.

As the above "ethers", for example, diethyl ether, diisopropyl ether, diphenyl ether, tetrahydrofuran, dioxane, 1,2-dimethoxyethane and the like can be mentioned.

As the above "halogenated hydrocarbons", for example, dichloromethane, chloroform, 1,2-dichloroethane, carbon tetrachloride and the like can be mentioned.

As the above "aliphatic hydrocarbons", for example, hexane, pentane, cyclohexane and the like can be mentioned.

As the above "aromatic hydrocarbons", for example, benzene, toluene, xylene, chlorobenzene and the like can be mentioned.

As the above "aromatic amines", for example, pyridine, lutidine, quinoline and the like can be mentioned.

As the above "amides", for example, N,N-dimethylformamide, N,N-dimethylacetamide, hexamethylphosphoric triamide and the like can be mentioned.

As the above "ketones", for example, acetone, methyl ethyl ketone and the like can be mentioned.

As the above "sulfoxides", for example, dimethyl sulfoxide and the like can be mentioned.

As the above "nitriles", for example, acetonitrile, propionitrile and the like can be mentioned.

As the above "organic acids", for example, acetic acid, propionic acid, trifluoroacetic acid and the like can be mentioned.

As the above "esters", for example, methyl acetate, ethyl acetate, amyl acetate, methyl propionate and the like can be mentioned.

When a desired product is obtained in a free form by the above reaction, it may be converted into a salt according to conventional methods or, when a desired product is obtained as a salt, it can be converted into a free form or another salt according to conventional methods. Compound (Im) thus obtained can be isolated and purified from the reaction solution by the known methods, for example, trans-solvation, concentration, solvent extraction, fractional distillation, crystallization, recrystallization, chromatography and the like.

When Compound (Im) is present as a configurational isomer (stereoisomer), diastereomer, conformer or the like, each can be optionally isolated by the above separation and purification methods. In addition, when Compound (Im) is in the form of its racemate, they can be separated into S- and R-forms by any conventional optical resolution.

When Compound (Im) includes stereoisomers, both the isomers alone and mixtures of each isomers are included in the scope of the present invention.

In addition, Compound (I) may be a hydrate or non-hydrate. Compound (I) may be labeled with an isotope (e.g., ³H, ¹⁴C, ³⁵S and the like) or the like.

A prodrug of Compounds (Ia) , (II), (III), (Iva), (Va), (VIa), (Im), (In), (If'), (Ig') or (Ih') above (hereinafter abbreviated as Compound (A) refers to a compound which is converted to Compound (A) as a result of a reaction with an enzyme, gastric acid etc. under physiological conditions in vivo, thus a compound that undergoes enzymatic oxidation, reduction, hydrolysis etc. to convert into Compound (A) and a compound that undergoes hydrolysis and the like by gastric acid etc. to convert into Compound (A). As a prodrug for Compound (A), a compound obtained by subjecting an amino group in Compound (A) to an acylation, alkylation or phosphorylation (e.g., a compound obtained by subjecting an amino group in Compound (A) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation, tert-butylation, etc.); a compound obtained by subjecting a hydroxy group in Compound (A) to an acylation, alkylation, phosphorylation and boration (e.g., a compound obtained by subjecting a hydroxy group in Compound (A) to an acetylation, palmitoylation, propanoylation, pivaloylation, succinylation, fumarylation, alanylation, dimethylaminomethylcarbonylation, etc.); a compound obtained by subjecting a carboxyl group in Compound (A) to an esterification or amidation (e.g., a compound obtained by subjecting a carboxyl group in Compound (A) to an ethylesterification, phenylesterification, carboxymethylesterification, dimethylaminomethylesterification, pivaloyloxymethylesterification, ethoxycarbonyloxyethylesterification, phthalidylesterification, (5-methyl-2-oxo-1,3-dioxolen-4-yl)methylesterification, cyclohexyloxycarbonylethylesterification and methylamidation, etc.) and the like can be mentioned. Any of these compounds can be produced from Compound (A) by a method known *per se*.

A prodrug for Compound (A) may also be one which is converted to Compound (A) under physiological conditions as described in "IYAKUHIN no KAIHATSU (Development of Pharmaceuticals)", Vol. 7, Design of Molecules, p. 163-198, Published by HIROKAWA SHOTEN (1990).

In addition, as the p38 MAP kinase inhibitor and/or the TNF-α production inhibitor to be used in the present invention, the compounds described in WO98/57966, WO98/56377, WO98/25619, WO98/07425, WO98/06715, US5739143, WO97/35855, WO97/33883, WO97/32583, WO97/25048, WO97/25046, WO96/10143, WO96/21654, WO95/07922, WO2000/09525,, WO99/17776, WO99/01131, WO98/28292, WO97/25047, WO97/25045, US5658903, WO96/21452, WO99/18942, US5756499, US5864036, US6046208, US5716955, US5811549, US5670527, US5969184, WO2000/31072, WO2000/31063, WO2000/20402, WO2000/18738, WO2000/17175, WO2000/12497, WO2000/12074, WO2000/07991, WO2000/07980 WO2000/02561, US6096711, WO99/64400, WO99/61440, WO99/59959, WO99/58523, WO99/58502, WO99/57101, WO99/32111, WO99/32110, WO99/26657, WO99/20624 WO99/18942, WO99/15164, WO99/00357, WO98/52940, WO98/52937, WO98/52558, WO98/06715, WO97/22256, WO96/21452, WO2000/43366, WO2000/42003, WO2000/42002, WO2000/41698, WO2000/41505, WO2000/40243, WO2000/34303, WO2000/25791, WO2000/17204, WO2000/10563, US6080546, WO99/61426, WO99/32463, WO99/32121, WO99/17776, WO98/28292, WO98/27098, WO98/25619, WO98/20868, WO97/35855, WO97/32583, WO97/25048, WO97/25047, WO97/25046, WO97/25045, US5658903, WO96/40143, WO96/21654, WO2000/55153, WO2000/55120, WO2000/26209, US6046208, US5756499, US5864036, JP-A-2000-86657, WO99/59960, WO99/21859, WO99/03837, WO99/01449, WO99/01136, WO/, WO99/01130, US5905089, WO98/57966, WO98/52941, WO98/47899, WO98/07425, WO97/33883, WO2000/42213, WO99/58128, WO2000/04025, WO2000/40235, WO2000/31106, WO97/46228, WO2000/59904, WO2000/42003, WO2000/42002, WO2000/41698, WO2000/10563, WO99/61426, WO99/32463, US6002008, WO98/43960, WO98/27098, WO97/35856, WO97/35855, WO96/22985, JP-A-61-145167 and the like, and the like can be used.

Of the p38 MAP kinase inhibitor and/or the TNF-α production inhibitor to be used in the present invention, a compound containing a pyridyl group or a salt thereof, wherein a substituent has been introduced into a position of nitrogen atom of the pyridyl group, or a compound containing a pyridyl group and an aromatic hydrocarbon group, or a salt thereof, wherein a polar group has been introduced into the aromatic hydrocarbon group can be preferably used, because P450 (e.g., CYP3A4) inhibitory action and the like is reduced, which in turn reduces side effects such as liver toxicity and the like, thereby enabling combined use with other drugs.

As the pyridyl group of the "compound containing a pyridyl group" "compound containing a pyridyl group and an aromatic hydrocarbon group", any of 1-pyridyl group, 2-pyridyl group, 3-pyridyl group and 4-pyridyl group can be used. Of these, 4-pyridyl group is preferable. As the aromatic hydrocarbon group of the "compound containing a pyridyl group and an aromatic hydrocarbon group", for example, a monocyclic or fused polycyclic (di- or tricyclic) aromatic hydrocarbon group having 6 to 14 carbon atoms and the like can be mentioned. Concrete examples thereof include C₆₋₁₄ aryl such as phenyl, 1-naphthyl, 2-naphthyl, 2-biphenylyl, 3-biphenylyl, 4-biphenylyl, 2-anthryl and the like, with preference given to phenyl.

As the "compound containing a pyridyl group" or "compound containing a pyridyl group and an aromatic hydrocarbon group", the above-mentioned compounds (I)-(VI) and the like are used.

As the substituent that can be introduced into the α-position of the pyridyl group, for example, those similar to the "substituent" of the above-mentioned "pyridyl group optionally having substituents" represented by R² and the like can be mentioned. Concretely, 1 to 3 of the following substituents can be introduced.
(i) halogen atom,
(ii) C₁₋₆ alkyl group, C₂₋₆ alkenyl group, C₂₋₆ alkynyl group, C₃₋₆ cycloalkyl group, C₆₋₁₄ aryl group and C₇₋₁₆ aralkyl group [these groups may have 1 to 5 substituents selected from a group consisting of oxo, halogen atom, C₁₋₃ alkylenedioxy, nitro, cyano, optionally halogenated C₁₋₆ alkyl, optionally halogenated C₂₋₆ alkenyl, carboxy C₂₋₆ alkenyl, optionally halogenated C₂₋₆ alkynyl, optionally halogenated C₃₋₆ cycloalkyl, C₆₋₁₄ aryl, optionally halogenated C₁₋₈ alkoxy, C₁₋₆ alkoxy-carbonyl-C₁₋₆ alkoxy, hydroxy, C₆₋₁₄ aryloxy, C₇₋₁₆ aralkyloxy, mercapto, optionally halogenated C₁₋₆ alkylthio, C₆₋₁₄ arylthio, C₇₋₁₆ aralkylthio, amino, mono-C₁₋₆ alkylamino, mono-C₆₋₁₄ arylamino, di-C₁₋₆ alkylamino, di-C₆₋₁₄ arylamino, formyl, carboxy, C₁₋₆ alkyl-carbonyl, C₃₋₆ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5 or 6-membered heterocyclic carbonyl, carbamoyl, thiocarbamoyl, mono-C₁₋₆ alkyl-carbamoyl, di-C₁₋₆ alkyl-carbamoyl, C₆₋₁₄ aryl-carbamoyl, 5- or 6-membered heterocyclic carbamoyl, C₁₋₆ alkylsulfonyl, C₆₋₁₄ arylsulfonyl, C₁₋₆ alkylsulfinyl, C₆₋₁₄ arylsulfinyl, formylamino, C₁₋₆ alkyl-carbonylamino, C₆₋₁₄ aryl-carbonylamino, C₁₋₆ alkoxy-carbonylamino, C₁₋₆ alkylsulfonylamino, C₆₋₁₄ arylsulfonylamino, C₁₋₆ alkyl-carbonyloxy, C₆₋₁₄ aryl-carbonyloxy, C₁₋₆ alkoxy-carbonyloxy, mono-C₁₋₆ alkyl-carbamoyloxy, di-C₁₋₆ alkyl-carbamoyloxy, C₆₋₁₄ aryl-carbamoyloxy, nicotinoyloxy, 5- to 7-membered saturated cyclic amino containing, besides one nitrogen atom and carbon atom, 1 or 2 kinds of 1 to 4 hetero atoms selected from nitrogen atom, sulfur atom and oxygen atom (this cyclic amino may have substituents selected from the group consisting of C₁₋₆ alkyl, C₆₋₁₄ aryl, C₁₋₆ alkyl-carbonyl, 5- to 10-membered aromatic heterocyclic group and oxo), and 5- to 10-membered aromatic heterocyclic group, containing, besides carbon atom, 1 or 2 kinds of 1 to 4 hetero atoms selected from nitrogen atom, sulfur atom and oxygen atom, sulfo, sulfamoyl, sulfinamoyl and sulfenamoyl (substituent group A)],
(iii) 5- to 14-membered heterocyclic group containing, besides carbon atom, 1 or 2 kinds of 1 to 4 hetero atoms selected from nitrogen atom, sulfur atom and oxygen atom, which may have 1 to 3 substituents selected from substituent group A,
(iv) acyl group represented by the formula: -(C=O)-R⁵, -(C=O)-OR⁵, -(C=O)-NR⁵R⁶, -(C=S)-NHR⁵ or -SO₂-R⁷ wherein R⁵ is (1) hydrogen atom, (2) C₁₋₆ alkyl group, C₂₋₆ alkenyl group, C₂₋₆ alkynyl group, C₃₋₆ cycloalkyl group, C₆₋₁₄ aryl group or C₇₋₁₆ aralkyl group, each of which may have 1 to 3 substituents selected from substituent group A, or (3) 5- to 14-membered heterocyclic group containing, besides carbon atom, 1 or 2 kinds of 1 to 4 hetero atoms selected from nitrogen atom, sulfur atom and oxygen atom, which may have 1 to 3 substituents selected from substituent group A, R⁶ is hydrogen atom or C₁₋₆ alkyl group, and R⁷ is (1) C₁₋₆ alkyl group which may have 1 to 3 substituents selected from substituent group A, C₂₋₆ alkenyl group, C₂₋₆ alkynyl group, C₃₋₆ cycloalkyl group, C₆₋ ₁₄ aryl group or C₇₋₁₆ aralkyl group, or (3) 5- to 14-membered heterocyclic group containing, besides carbon atom, 1 or 2 kinds of 1 to 4 hetero atoms selected from nitrogen atom, sulfur atom and oxygen atom, which may have 1 to 3 substituents selected from substituent group A,
(v) amino group (this amino group may have 1 or 2 substituents selected from (1) C₁₋₆ alkyl group which may have 1 to 3 substituents selected from substituent group A, C₂₋₆ alkenyl group, C₂₋₆ alkynyl group, C₃₋₆ cycloalkyl group, C₆₋₁₄ aryl group and C₇₋₁₆ aralkyl group, (2) 5- to 14-membered heterocyclic group containing, besides carbon atom, 1 or 2 kinds of 1 to 4 hetero atoms selected from nitrogen atom, sulfur atom and oxygen atom, which may have 1 to 3 substituents selected from substituent group A, and (3) acyl group shown by the above-mentioned (iv)),
(vi) 5- to 7-membered non-aromatic cyclic amino group containing, besides one nitrogen atom and carbon atom, 1 or 2 kinds of 1 to 4 hetero atoms selected from nitrogen atom, sulfur atom and oxygen atom (this cyclic amino group may have 1 to 3 substituents selected from C₁₋₆ alkyl, C₆₋₁₄ aryl, C₁₋₆ alkyl-carbonyl, 5- to 10-membered aromatic heterocyclic group and oxo), and
(vii) C₁₋₆ alkoxy group, C₆₋₁₄ aryloxy group and C₇₋₁₆ aralkyloxy group, which may have 1 to 3 substituents selected from substituent group A.

Of these, the following substituents are preferable. (i) halogen atom, (ii) C₁₋₆ alkyl group, (iii) amino group (this amino group may have 1 or 2 substituents selected from (1) C₁₋₆ alkyl group, C₂₋₆ alkenyl group, C₂₋₆ alkynyl group, C₃₋₆ cycloalkyl group, C₆₋₁₄ aryl group and C₇₋₁₆ aralkyl group, which may have 1 to 3 substituents selected from substituent group A, (2) 5- to 14-membered heterocyclic group containing, besides carbon atom, 1 or 2 kinds of 1 to 4 hetero atoms selected from nitrogen atom, sulfur atom and oxygen atom, which may have 1 to 3 substituents selected from substituent group A, and (3) acyl group shown by the formula: -(C=O)-R⁵, -(C=O)-OR⁵, -(C=O)-NR⁵R⁶ wherein R⁵ is (1) hydrogen atom, (2) C₁₋₆ alkyl group, C₂₋₆ alkenyl group, C₂₋₆ alkynyl group, C₃₋₆ cycloalkyl group, C₆₋₁₄ aryl group or C₇₋₁₆ aralkyl group, each of which may have 1 to 3 substituents selected from substituent group A, or (3) 5- to 14-membered heterocyclic group containing, besides carbon atom, 1 or 2 kinds of 1 to 4 hetero atoms selected from nitrogen atom, sulfur atom and oxygen atom, which may have 1 to 3 substituents selected from substituent group A, and R⁶ is hydrogen atom or C₁₋₆ alkyl group) and (iv) 5- to 7-membered non-aromatic cyclic amino group containing, besides one nitrogen atom and carbon atom, 1 or 2 kinds of 1 to 4 hetero atoms selected from nitrogen atom, sulfur atom and oxygen atom (this cyclic amino group may have 1 to 3 substituents selected from C₁₋₆ alkyl, C₆₋₁₄ aryl, C₁₋₆ alkyl-carbonyl, 5- to 10-membered aromatic heterocyclic group and oxo).

As the polar group that can be introduced into the aromatic hydrocarbon group of the "compound containing a pyridyl group and an aromatic hydrocarbon group or a salt thereof", for example, 1 to 3 selected from (1) halogen atom, (2) hydroxy, (3) amino optionally having 1 or 2 substituents selected from substituents selected from substituent group A and acyl shown by the above-mentioned (iv), (4)nitro, (5) carboxy, (6) formyl, (7) C₁₋₆ alkoxy optionally having 1 to 3 substituents selected from substituent group A, (8) C₁₋₆ alkoxy-carbonyl optionally having 1 to 3 substituents selected from substituent group A, (9)cyano and (10) C₁₋₆ alkyl and C₆₋₁₄ aryl optionally having 1 to 3 selected from these polar groups (groups shown in the above-mentioned (1)-(9)) as substituents are mentioned. Of these, C₁₋₆ alkyl and C₆₋₁₄ aryl optionally having 1 to 3 substituents selected from (1) carboxy, (2) hydroxy, (3) carboxy and hydroxy, and the like are preferable.

As P450, CYP1A1, CYP1A2, CYP2A1, CYP2A2, CYP2A4, CYP2A5, CYP2A6, CYP2B1, CYP2B2, CYP2B4, CYP2B5, CYP2B6, CYP2B9, CYP2C2, CYP2C3, CYP2C4, CYP2C5, CYP2C6, CYP2C7, CYP2C8, CYP2C9, CYP2C11, CYP2C12, CYP2C14, CYP2C19, CYP2C29, CYP2D1, CYP2D2, CYP2D6, CYP2D9, CYP2E1, CYP2F1, CYP2F2, CYP2G1, CYP3A1, CYP3A2, CYP3A3, CYP3A4, CYP3A6, CYP3A7, CYP4A1, CYP4B1 and the like can be mentioned, with preference given to CYP2C9, CYP2D6 and CYP3A4.

In the present specification, the above-mentioned p38 MAP kinase inhibitor and/or the TNF-α production inhibitor are sometimes collectively abbreviated as the compound of the present invention.

The compound of the present invention has superior p38MAP kinase inhibitory action, TNF-α inhibitory action (TNF-α production inhibitory action, TNF-α activity inhibitory action), phosphodiesterase IV (PDE IV) inhibitory action and the like, is superior in (oral) absorbability, (metabolism) stability and the like, and shows low toxicity and fewer side effects. Therefore, the compound can be used as a safe pharmaceutical product.

A pharmaceutical composition containing the compound of the present invention can be used for a mammal (e.g., mouse, rat, hamster, rabbit, cat, dog, bovine, sheep, monkey, human etc.) as a prophylactic or therapeutic agent of various pains shown in the following.
cancer pain, acute pain due to inflammation, pain associated with chronic inflammation, postoperative pain (pain of incision, deep pain, visceral pain, postoperative chronic pain and the like), muscular pain (muscular pain associated with chronic pain disease, stiff neck and the like), arthritic pain, tooth pain, temporomandibular joint pain, headache (migrain, tension-type headache, headache due to fever, headache caused by hypertension), visceral pain (cardiac pain, anginal pain, abdominal pain, kidney pain, ureteral pain, bladder pain, pain in the field of obstetrics and gynecology (intermenstrual pain, menstrual cramps, labor pain)), nerve pain (ruptured disc, radicular pain, postherpetic neuralgia, trigeminal neuralgia), reflex sympathetic dystrophy, complex regional pain syndrome and the like.

A pharmaceutical composition containing the compound of the present invention can be also used for a mammal (e.g., mouse, rat, hamster, rabbit, cat, dog, bovine, sheep, monkey, human etc.) as an agent for the suppression of osteoclast activation and inhibitor of osteoclast formation.

Osteoclast is a cell multinucleated by differentiation and fusion of hematopoietic cells, which has bone matrix decomposability and plays a role of resorbing bone from osteoclast that newly forms bone in the bone metabolism. The maintenance of bone mass and form depends on the balance of the formation and resorption by the both cells. When osteoclast is activated to promote resorption of the bone, this balance is broken, and a decrease in the bone mass and morphological destruction and deformation occur. In addition, since osteoclast is involved in the adjustment of blood calcium concentration via resorption of bone, which is a calcium storage organ, extreme activation of osteoclast results in an increase in the blood calcium concentration.

The compound of the present invention suppresses the activation of osteoclast and can inhibit the formation of osteoclast. Thus, the compound can be used as an agent for the prophylaxis or treatment of, for example, (1) postmonopausal or senile primary osteoporosis, (2) secondary osteoporosis caused by inflammation (rheumatism and the like), blood system malignant disease (malignant lymphoma, leukemia and the like), endocrine disorder (thyroid hyperfunction, diabetes and the like) or administration of pharmaceutical agent such as adrenal cortex hormone and the like, (3) bone or joint tissue destruction or deformation associated with bone metastasis of tumor or rheumatism, (4) Paget's disease or (5) hypercalcemia and the like.

Of the compounds of the present invention, a compound having both effects of the prophylaxis or treatment of pain and the suppression of activation and/or inhibition of formation of osteoclast is useful because it alleviates pain such as arthritic pain and the like, and simultaneously prevents or treats diseases related to osteoclast, such as destruction and deformation of bone or joint tissue and the like.

The pharmaceutical composition of the present invention containing the compound of the present invention shows low toxicity, and can be safely administered orally or parenterally (e.g., topical, rectal, intravenous administration etc.) as a pharmaceutical preparation of the compound of the present invention as it is or after admixing with a pharmacologically acceptable carrier to give, for example, tablet (including sugar-coated tablet and film-coated tablet), powder, granule, capsules (including soft capsules), liquid, injection, suppository, sustained-release preparation and the like, according to a methods known per se used for the general production method for pharmaceutical preparations.

The content of the Compound of the present invention in a pharmaceutical composition of the present invention is about 0.01 to about 100% by weight relative to the whole preparation.

As the pharmacologically acceptable carrier which may be used for preparing a pharmaceutical composition of the present invention, the conventional various organic or inorganic carriers as a pharmaceutical material, for example, excipient, lubricant, binder and disintegrating agent in solid preparations, or solvent, solubilizing agent, suspending agent, isotonizing agent, buffer and soothing agent in liquid preparations, and the like can be mentioned. Further, if needed, additives such as the conventional preservative, antioxidant, colorant, sweetening agent, adsorbing agent, wetting agent and the like can be appropriately used at an appropriate amount.

As the excipient, for example, lactose, saccharose, D-mannitol, starch, corn starch, crystalline cellulose, light silicic acid anhydride and the like can be mentioned.

As the lubricant, for example, magnesium stearate, calcium stearate, talc, colloidal silica and the like can be mentioned.

As the binder, for example, crystalline cellulose, saccharose, D-mannitol, dextrin, hydroxypropylcellulose, hydroxypropylmethylcellulose, polyvinylpyrrolidone, starch, sucrose, gelatin, methylcellulose, sodium carboxymethylcellulose and the like can be mentioned.

As the disintegrating agent, for example, starch, carboxymethyl cellulose, calcium carboxymethyl cellulose, sodium carboxymethyl starch, L-hydroxypropylcellulose and the like can be mentioned.

As the solvent, for example, water for injection, alcohol, propylene glycol, macrogol, sesame oil, corn oil, olive oil and the like can be mentioned.

As the solubilizing agent, for example, polyethylene glycol, propylene glycol, D-mannitol, benzyl benzoate, ethanol, tris-aminomethane, cholesterol, triethanolamine, sodium carbonate, sodium citrate and the like can be mentioned.

As the suspending agent, for example, surfactants such as stearyl triethenolamine, sodium lauryl sulfate, lauryl aminopropionate, lecithin, benzalkonium chloride, benzethonium chloride, glyceryl monostearate and the like; hydrophilic polymers such as polyvinyl alcohol, polyvinylpyrrolidone, sodium carboxymethyl cellulose, methylcellulose, hydroxymethylcellulose, hydroxyethylcellulose, hydroxypropylcellulose and the like, and the like can be mentioned.

As the isotonizing agent, for example, glucose, D-sorbitol, sodium chloride, glycerin, D-mannitol and the like can be mentioned.

As the buffer, for example, buffering solutions such as phosphate, acetate, carbonate, citrate and the like, and the like can be mentioned.

As the soothing agent, for example, benzyl alcohol and the like can be mentioned.

As the preservative, for example, p-hydroxybenzoates, chlorobutanol, benzyl alcohol, phenethyl alcohol, dehydroacetic acid, sorbic acid and the like can be mentioned.

As the antioxidant, for example, sulfites, ascorbic acid, α-tocopherol and the like can be mentioned.

While the content of additive such as carrier and the like in the pharmaceutical composition of the present invention varies depending on the form of the preparation, it is generally about 1 to 99.99 wt%, preferably about 10 to 90 wt%, relative to the entire preparation.

While the dose of the pharmaceutical composition of the present invention varies depending on the administration subject, administration route, disease, symptoms and the like, it is, for example, about 0.01 to about 30 mg/kg body weight, preferably about 0.1 to about 20 mg/kg body weight, more preferably about 1 to about 20 mg/kg body weight, in the amount of an active ingredient [the compound of the present invention] per one day, which is orally administered to patients with pain (body weight about 60 kg) once a day or several times a day in divided doses. For example, moreover, it is orally administered to patients with primary osteoporosis (body weight about 60 kg) in a daily dose of about 0.01 to about 30 mg/kg body weight, preferably about 0.1 to about 20 mg/kg body weight, more preferably about 1 to about 20 mg/kg body weight, in the amount of an active ingredient [the compound of the present invention], once a day or several times a day in divided doses.

As the drugs that can be used in combination with the compound of the present invention (hereinafter the drug is sometimes abbreviated as a concomitant drug) includes, for example, the following.
(1) non-steroidal antiinflammatory drugs (NSAIDs)
   (i) classical NSAIDs
      alcofenac, aceclofenac, sulindac, tolmetin, etodolac, fenoprofen, thiaprofenic acid, meclofenamic acid, meloxicam, tenoxicam, lornoxicam, nabumeton, acetaminophen, phenacetin, ethenzamide, sulpyrine, antipyrine, migrenin, aspirin, mefenamic acid, flufenamic acid, diclofenac sodium, loxoprofen sodium, phenylbutazone, indomethacin, ibuprofen, ketoprofen, naproxen, oxaprozin, flurbiprofen, fenbufen, pranoprofen, floctafenine, piroxicam, epirizole, tiaramide hydrochloride, zaltoprofen, gabexate mesylate, camostat mesylate, ulinastatin, colchicine, probenecid, sulfinpyrazone, benzbromarone, allopurinol, sodium aurothiomalate, hyaluronate sodium, sodium salicylate, morphine hydrochloride, salicylic acid, atropine, scopolamine, morphine, pethidine, levorphanol, oxymorphone or a salt thereof and the like.
   (ii) cyclooxygenase inhibitor (COX-1 selective inhibitor, COX-2 selective inhibitor and the like)
      salicylic acid derivatives (e.g., celecoxib, Rofecoxib, aspirin), MK-663, valdecoxib, SC-57666, tiracoxib, S-2474, diclofenac, indomethacin, loxoprofen and the like.
   (iii) drug concurrently having COX inhibitory activity and 5-lipoxygenase inhibitory activity
      ML-3000, p54 (COX inhibitor & 5-lipoxygenase inhibitor) and the like.
   (iv) nitric oxide-releasing NSAIDs
(2) disease-modifying anti-rheumatic drugs (DMARDs)
   (i) gold preparation
      Auranofin and the like.
   (ii) penicillamine
      D-penicillamine
   (iii) sulfasalazine
   (iv) antimalarial drug
      chloroquine and the like.
   (v) pyrimidine synthesis inhibitor
      leflunomide and the like.
   (vi) prograf
(3) anti-cytokine drug
   (I) protein drug
      (i) TNF inhibitor
         etanercept, infliximab, D2E7, CDP-571, PASSTNF-α, soluble TNF-α receptor, TNF-α binding protein, anti-TNF-α antibody and the like.
      (ii) interleukin-1 inhibitor
         anakinra (interleukin-1 receptor antagonist), soluble interleukin-1 receptor and the like.
      (iii) interleukin-6 inhibitor
         MRA (anti-interleukin-6 receptor antibody), anti-interleukin-6 antibody and the like.
      (iv) interleukin-10 drug
         interleukin-10 and the like.
      (v) interleukin-12 inhibitor
         anti-interleukin-12 antibody and the like.
      (vi) drug concurrently having interferon-α and -γ inhibitory activity and TNF-α inhibitory activity (polyclonal antibody) AGT-1
   (II) non-protein drug
      (i) MAP kinase inhibitor
         PD-98059 and the like.
      (ii) gene modulator
         SP-100030, inhibitor of molecule involved in signal transduction, such as NF-κ, NF-κB, IKK-1, IKK-2, AP-1 and the like
      (iii) cytokine production inhibitor
         T-614, SR-31747, sonatimod and the like.
      (iv) TNF-α converting enzyme inhibitor
      (v) interleukin-1β converting enzyme inhibitor
         HMR3480/VX-740 and the like.
      (vi) interleukin-6 antagonist
         SANT-7 and the like.
      (vii) interleukin-8 inhibitor
         IL-8 antagonist, CXCR1 & CXCR2 antagonist and the like.
      (viii) chemokine antagonist
         MCP-1 antagonist and the like.
      (ix) interleukin-2 receptor antagonist
         denileukin diftitox and the like.
      (x) therapeutic vaccines
         TNF-α vaccine and the like.
      (xi) gene therapy drug
         gene therapy drugs aiming at promoting the expression of gene having an anti-inflammatory action such as interleukin-4, interleukin-10, soluble interleukin-1 receptor, soluble TNF-α receptor and the like.
      (xii) antisense compound
         ISIS-104838 and the like.
(4) immunomodulator (immunosuppressant)
   (i) T cell differentiation modulator
      ethyl 6,7-dimethoxy-4-(3,4-dimethoxyphenyl)-2-(1,2,4-triazol-1-ylmethyl)quinoline-3-carboxylate (JP-A-7-118266)
   (ii) others
      methotrexate, cyclophosphamide, MX-68, atiprimod dihydrochloride, BMS-188667, CKD-461, rimexolone, cyclosporine, tacrolimus, gusperimus, azathiopurine, antilymphocyte serum, freeze-dried sulfonated normal immunoglobulin, erythropoietin, colony stimulating factor, interleukin, interferon and the like.
(5) steroid
   dexamethasone, hexestrol, methimazole, betamethasone, triamcinolone, triamcinolone acetonide, fluocinonide, fluocinolone acetonide, prednisolone, methylprednisolone, cortisone acetate, hydrocortisone, fluorometholone, beclomethasone dipropionate, estriol and the like.
(6) c-Jun N terminal kinase (JNK) inhibitor
   compounds described in WO00/35906, WO00/35909, WO00/35921, WO00/64872 or WO00/75118 and the like.
(7) angiotensin converting enzyme inhibitor
   enalapril, captopril, ramipril, lisinopril, cilazapril, perindopril and the like.
(8) angiotensin II receptor antagonist
   candesartan cilexetil (TCV-116), valsartan, irbesartan, olmesartan, eprosartan and the like.
(9) diuretic drug
   hydrochlorothiazide, spironolactone, furosemide, indapamide, bendrofluazide, cyclopenthiazide and the like.
(10) cardiotonic drug
   digoxin, dobutamine and the like.
(11) β receptor antagonist
   carvedilol, metoprolol, atenolol and the like.
(12) Ca sensitizer
   MCC-135 and the like.
(13) Ca channel antagonist
   nifedipine, diltiazem, verapamil and the like.
(14) anti-platelet drug, anticoagulator
   heparin, aspirin, warfarin and the like.
(15) HMG-CoA reductase inhibitor
   atorvastatin, simvastatin and the like.
(16) contraceptive
   (i) sex hormone or derivatives thereof
      gestagen or a derivative thereof (progesterone, 17α-hydroxy progesterone, medroxyprogesterone, medroxyprogesterone acetate, norethisterone, norethisterone enanthate, norethindrone, norethindrone acetate, norethynodrel, levonorgestrel, norgestrel, ethynodiol diacetate, desogestrel, norgestimate, gestodene, progestin, etonogestrel, drospirenone, dienogest, trimegestone, nestorone, chlormadinone acetate, mifepristone, nomegestrol acetate, Org-30659, TX-525, EMM-310525) or a combination of a gestagen or a derivative thereof and an estrogen or a derivative thereof (estradiol, estradiol benzoate, estradiol cypionate, estradiol dipropionate, estradiol enanthate, estradiol hexahydrobenzoate, estradiol phenylpropionate, estradiol undecylate, estradiol valerate, estrone, ethinylestradiol, mestranol) and the like.
   (ii) antiestrogen
      ormeloxifene, mifepristone, Org-33628 and the like.
   (iii) spermatocide
      ucarcide and the like.
(17) others
   (i) T cell inhibitors
      IR-501 (T cell receptor peptide) and the like.
   (ii) inosine monophosphate dehydrogenase (IMPDH) inhibitor mycophenolate mofetil, VX-497 and the like.
   (iii) adhesion molecule inhibitor
      ISIS-2302, selectin inhibitor, ELAM-1, VCAM-1, ICAM-1 and the like.
   (iv) thalidomide
   (v) cathepsin inhibitor
   (vi) matrix metalloprotease (MMPs) inhibitor
      BB-3644, CGS-27023A, Bay-12-9566, KB-R7785, L-758354, POL-641 and the like.
   (vii) glucose-6-phosphate dehydrogenase inhibitor
      CBF-BS2 and the like.
   (viii) hydroorotate dehydrogenase (DHODH) inhibitor
   (ix) phosphodiesterase IV (PDE IV) inhibitor CG-1088 and the like.
   (x) phospholipase A₂ inhibitor
   (xi) iNOS inhibitor
      NOX-200 and the like.
   (xii) microtubule stimulating drug
      paclitaxel and the like.
   (xiii) microtubule inhibitor
      reumacon and the like.
   (xiv) MHC class II antagonist
      ZD-2315 and the like.
   (xv) prostacyclin agonist
      iloprost and the like.
   (xvi) CD4 antagonist
      4162W94, keliximab and the like.
   (xvii) CD23 antagonist
   (xviii) LTB4 receptor antagonist
      CGS-25019C and the like.
   (xix) 5-lipoxygenase inhibitor
      zileuton and the like.
   (xx) cholinesterase inhibitor galanthamine and the like.
   (xxi) tyrosine kinase inhibitor
      YT-146 and the like.
   (xxii) cathepsin B inhibitor
   (xxiii) adenosine deaminase inhibitor
      pentostatin and the like.
   (xxiv) osteogenesis stimulator
      (2R,4S)-(-)-N-[4-(diethoxyphosphorylmethyl)phenyl]-1,2,4,5-tetrahydro-4-methyl-7,8-methylenedioxy-5-oxo-3-benzothiepin-2-carboxamide or a salt thereof (JP-A-8-231659) and the like.
   (xxv) dipeptidylpeptidase inhibitor
      TMC-2A and the like.
   (xxvi) TRK-530, TOK-8801
   (xxvii) collagen agonist
      AI-200 and the like.
   (xxviii) capsaicin cream
   (xxix) hyaluronic acid derivative
      synvisc (hylan G-F 20), orthovisc and the like.
   (xxx) glucosamine sulfate
   (xxxi) amiprilose

Other concomitant drugs besides the above-mentioned include, for example, antibacterial agent, antifungal agent, antiprotozoal agent, antibiotic, antitussive and expectorant drug, sedative, anesthetic, antiulcer drug, antiarrhythmic agent, hypotensive diuretic drug, anticoagulant, tranquilizer, antipsychotic, antitumor drug, hypolipidemic drug, muscle relaxant, anticonvulsant, antidepressant, antiallergic drug, cardiac, antiarrhythmic agent, vasodilator, vasoconstrictor, hypotensive diuretic drug, antidiabetic drug, antinarcotic, vitamin, vitamin derivative, antiasthmatic, therapeutic agent for pollakisuria/anischuria, therapeutic agent for atopic dermatitis, therapeutic agent for allergic rhinitis, hypertensor, endotoxin-antagonist or -antibody, signal transduction inhibitor, inhibitor of inflammatory mediator activity, antibody to inhibit inflammatory mediator activity, inhibitor of anti-inflammatory mediator activity, antibody to inhibit anti-inflammatory mediator activity and the like. Specific examples thereof include the following.
(1) antibacterial agent
   1) sulfa drug
      sulfamethizole, sulfisoxazole, sulfamonomethoxine, sulfamethizole, salazosulfapyridine, silver sulfadiazine and the like.
   2) quinoline antibacterial agent
      nalidixic acid, pipemidic acid trihydrate, enoxacin, norfloxacin, ofloxacin, tosufloxacin tosilate, ciprofloxacin hydrochloride, lomefloxacin hydrochloride, sparfloxacin, fleroxacin and the like.
   3) antiphthisic
      isoniazid, ethambutol (ethambutol hydrochloride), p-aminosalicylic acid (calcium p-aminosalicylate), pyrazinamide, ethionamide, protionamide, rifampicin, streptomycin sulfate, kanamycin sulfate, cycloserine and the like.
   4) antiacidfast bacterium drug
      diaphenylsulfone, rifampicin and the like.
   5) antiviral drug
      idoxuridine, acyclovir, vidarabine, gancyclovir and the like.
   6) anti-HIV agent
      zidovudine, didanosine, zalcitabine, indinavir sulfate ethanolate, ritonavir and the like.
   7) antispirochetele
   8) antibiotic
      tetracycline hydrochloride, ampicillin, piperacillin, gentamicin, dibekacin, kanendomycin, lividomycin, tobramycin, amikacin, fradiomycin, sisomicin, tetracycline, oxytetracycline, rolitetracycline, doxycycline, ampicillin, piperacillin, ticarcillin, cephalothin, cephapirin, cephaloridine, cefaclor, cephalexin, cefroxadine, cefadroxil, cefamandole, cefotoam, cefuroxime, cefotiam, cefotiam hexetil, cefuroxime axetil, cefdinir, cefditoren pivoxil, ceftazidime, cefpiramide, cefsulodin, cefmenoxime, cefpodoxime proxetil, cefpirome, cefozopran, cefepime, cefsulodin, cefmenoxime, cefmetazole, cefminox, cefoxitin, cefbuperazone, latamoxef, flomoxef, cefazolin, cefotaxime, cefoperazone, ceftizoxime, moxalactam, thienamycin, sulfazecin, aztreonam or a salt thereof, griseofulvin, lankacidin-group [Journal of Antibiotics (J. Antibiotics), 38, 877-885(1985)], azole compound [2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone, fluconazole, itraconazole] and the like.
(2) antifungal agent
   1) polyethylene antibiotic (e.g., amphotericin B, nystatin, trichomycin)
   2) griseofulvin, pyrrolnitrin and the like.
   3) cytosine metabolism antagonist (e.g., flucytosine)
   4) imidazole derivative (e.g., econazole, clotrimazole, miconazole nitrate, bifonazole, croconazole)
   5) triazole derivative (e.g. fluconazole, itraconazole)
   6) thiocarbamic acid derivative (e.g. trinaphthol)
(3) antiprotozoal agent
   metronidazole, tinidazole, diethylcarbamazine citrate, quinine hydrochloride, quinine sulfate and the like.
(4) antitussive and expectorant drug
   ephedrine hydrochloride, noscapine hydrochloride, codeine phosphate, dihydrocodeine phosphate, isoproterenol hydrochloride, ephedrine hydrochloride, methylephedrine hydrochloride, noscapine hydrochloride, alloclamide, chlophedianol, picoperidamine, cloperastine, protokylol, isoproterenol, salbutamol, terbutaline, oxymetebanol, morphine hydrochloride, dextromethorfan hydrobromide, oxycodone hydrochloride, dimemorphan phosphate, tipepidine hibenzate, pentoxyverine citrate, clofedanol hydrochloride, benzonatate, guaifenesin, bromhexine hydrochloride, ambroxol hydrochloride, acetylcysteine, ethyl cysteine hydrochloride, carbocysteine and the like.
(5) sedative
   chlorpromazine hydrochloride, atropine sulfate, phenobarbital, barbital, amobarbital, pentobarbital, thiopental sodium, thiamylal sodium, nitrazepam, estazolam, flurazepam, haloxazolam, triazolam, flunitrazepam, bromovalerylurea, chloral hydrate, triclofos sodium and the like.
(6) anesthetic
   (6-1) local anesthetic
      cocaine hydrochloride, procaine hydrochloride, lidocaine, dibucaine hydrochloride, tetracaine hydrochloride, mepivacaine hydrochloride, bupivacaine hydrochloride, oxybuprocaine hydrochloride, ethyl aminobenzoate, oxethazaine) and the like.
   (6-2) general anesthetic
      1) inhalation anesthetic (e.g., ether, halothane, nitrous oxide, isoflurane, enflurane),
      2) intravenous anesthetic (e.g., ketamine hydrochloride, droperidol, thiopental sodium, thiamylal sodium, pentobarbital) and the like.
(7) antiulcer drug
   histidine hydrochloride, lansoprazole, metoclopramide, pirenzepine, cimetidine, ranitidine, famotidine, urogastrone, oxethazaine, proglumide, omeprazole, sucralfate, sulpiride, cetraxate, gefarnate, aldioxa, teprenone, prostaglandin and the like.
(8) antiarrhythmic agent
   1) Na channel blocker (e.g., quinidine, procainamide, disopyramide, ajmaline, lidocaine, mexiletine, phenytoin),
   2) β-blocker (e.g., propranolol, alprenolol, bufetolol hydrochloride, oxprenolol, atenolol, acebutolol, metoprolol, bisoprolol, pindolol, carteolol, arotinolol,
   3) K channel blocker (e.g., amiodarone),
   4) Ca channel blocker (e.g., verapamil, diltiazem) and the like.
(9) hypotensive diuretic drug
   hexamethonium bromide, clonidine hydrochloride, hydrochlorothiazide, trichlormethiazide, furosemide, ethacrynic acid, bumetanide, mefruside, azosemide, spironolactone, potassium canrenoate, triamterene, amiloride, acetazolamide, D-mannitol, isosorbide, aminophylline and the like.
(10) anticoagulant
   heparin sodium, sodium citrate, activated protein C, tissue factor pathway inhibitor, antithrombin III, dalteparin sodium, warfarin potassium, argatroban, gabexate, sodium citrate, ozagrel sodium, ethyl icosapentate, beraprost sodium, alprostadil, ticlopidine hydrochloride, pentoxifylline, dipyridamole, tisokinase, urokinase, streptokinase and the like.
(11) tranquilizer
   diazepam, lorazepam, oxazepam, chlordiazepoxide, medazepam, oxazolam, cloxazolam, clotiazepam, bromazepam, etizolam, fludiazepam, hydroxyzine and the like.
(12) antipsychotic
   chlorpromazine hydrochloride, prochlorperazine, trifluoperazine, thioridazine hydrochloride, perphenazine maleate, fluphenazine enanthate, prochlorperazine maleate, levomepromazine maleate, promethazine hydrochloride, haloperidol, bromperidol, spiperone, reserpine, clocapramine hydrochloride, sulpiride, zotepine and the like.
(13) antitumor drug
   6-O-(N-chloroacetylcarbamoyl)fumagillol, bleomycin, methotrexate, actinomycin D, mitomycin C, daunorubicin, adriamycin, neocarzinostatin, cytosine arabinoside, fluorouracil, tetrahydrofuryl-5-fluorouracil, picibanil, lentinan, levamisole, bestatin, azimexon, glycyrrhizin, doxorubicin hydrochloride, aclarubicin hydrochloride, bleomycin hydrochloride, peplomycin sulfate, vincristine sulfate, vinblastine sulfate, irinotecan hydrochloride, cyclophosphamide, melphalan, busulfan, thiotepa, procarbazine hydrochloride, cisplatin, azathioprine, mercaptopurine, tegafur, carmofur, cytarabine, methyltestosterone, testosterone propionate, testosterone enanthate, mepitiostane, fosfestrol, chlormadinone acetate, leuprorelin acetate, buserelin acetate and the like.
(14) antihypolipidemic drug
   clofibrate, ethyl 2-chloro-3-[4-(2-methyl-2-phenylpropoxy)-phenyl]propionate [Chemical and Pharmaceutical Bulletin (Chem. Pharm. Bull), 38, 2792-2796 (1990)], pravastatin, simvastatin, probucol, bezafibrate, clinofibrate, nicomol, cholestyramine, dextran sulfate sodium and the like.
(15) muscle relaxant
   pridinol, tubocurarine, pancuronium, tolperisone hydrochloride, chlorphenesin carbamate, baclofen, chlormezanone, mephenesin, chlorzoxazone, eperisone, tizanidine and the like.
(16) anticonvulsant
   phenytoin, ethosuximide, acetazolamide, chlordiazepoxide, trimethadione, carbamazepine, phenobarbital, primidone, sulthiame, sodium valproate, clonazepam, diazepam, nitrazepam and the like.
(17) antidepressant
   imipramine, clomipramine, noxiptiline, phenelzine, amitriptyline hydrochloride, nortriptyline hydrochloride, amoxapine, mianserin hydrochloride, maprotiline hydrochloride, sulpiride, fluvoxamine maleate, trazodone hydrochloride and the like.
(18) antiallergic drug
   diphenhydramine, chlorpheniramine, tripelennamine, metodilamine, clemizole, diphenylpyraline, methoxyphenamine, sodium cromoglicate, tranilast, repirinast, amlexanox, ibudilast, ketotifen, terfenadine, mequitazine, azelastine hydrochloride, epinastine, ozagrel hydrochloride, pranlukast hydrate, seratrodast and the like.
(19) cardiac
   trans-π-oxocamphor, terephyllol, aminophylline, etilefrine, dopamine, dobutamine, denopamine, aminophylline, bencirin, amrinone, pimobendan, ubidecarenone, digitoxin, digoxin, methyldigoxin, lanatoside C, G-strophanthin and the like.
(20) vasodilator
   oxyfedrine, diltiazem, tolazoline, hexobendine, bamethan, clonidine, methyldopa, guanabenz and the like.
(21) vasoconstrictor
   dopamine, dobutamine denopamine and the like.
(22) hypotensive diuretic drug
   hexamethonium bromide, pentolinium, mecamylamine, ecarazine, clonidine, diltiazem, nifedipine and the like.
(23) antidiabetic drug
   tolbutamide, chlorpropamide, acetohexamide, glibenclamide, tolazamide, acarbose, epalrestat, troglitazone, glucagon, glymidine, glipizide, phenformin, buformin, metformin and the like.
(24) antinarcotic
   levallorphan, nalorphine, naloxone or a salt thereof and the like.
(25) fat-soluble vitamin
   1) vitamin A: vitamin A₁, vitamin A₂ and retinol palmitate
   2) vitamin D: vitamin D₁, D₂, D₃, D₄ and D₅
   3) vitamin E: α-tocopherol, β-tocopherol, γ-tocopherol, δ-tocopherol, dl-α-tocopherol nicotinate
   4) vitamin K: vitamin K₁, K₂, K₃ and K₄
   5) folic acid (vitamin M) and the like.
(26) vitamin derivative
   various derivatives of vitamins, for example, vitamin D₃ derivatives such as 5,6-trans-cholecalciferol, 2,5-hydroxycholecalciferol, 1-α-hydroxycholecalciferol and the like, vitamin D₂ derivatives such as 5,6-trans-ergocalciferol and the like.
(27) antiasthmatic
   isoprenaline hydrochloride, salbutamol sulfate, procaterol hydrochloride, terbutaline sulfate, trimetoquinol hydrochloride, tulobuterol hydrochloride, orciprenaline sulfate, fenoterol hydrobromide, ephedrine hydrochloride, ipratropium bromide, oxitropium bromide, flutropium bromide, theophylline, aminophylline, sodium cromoglicate, tranilast, repirinast, amlexanox, ibudilast, ketotifen, terfenadine, mequitazine, azelastine, epinastine, ozagrel hydrochloride, pranlkast hydrate, seratrodast, dexamethasone, prednisolone, hydrocortisone, hydrocortisone sodium succinate, beclometasone dipropionate and the like.
(28) therapeutic agent for pollakisuria/anischuria flavoxate hydrochloride and the like.
(29) therapeutic agent for atopic dermatitis
   sodium cromoglicate and the like.
(30) therapeutic agent for allergic rhinitis
   sodium cromoglicate, chlorpheniramine maleate, alimemazine tartrate, clemastine fumarate, homochlorcyclizine hydrochloride, terfenadine, mequitazine and the like.
(31) hypertensive drug
   dopamine, dobutamine, denopamine, digitoxin, digoxin, methyldigoxin, lanatoside C, G-strophanthin and the like.
(32) Others
   hydroycam, diacerein, megestrol acetate, nicergoline, prostaglandins and the like.

By combining the compound of the present invention and a concomitant drug, a superior effect such as
(1) the dose can be reduces as compared to single administration of the compound of the present invention or a combination drug,
(2) the drug to be combined with the compound of the present invention can be selected according to the condition of patients (mild case, severe case and the like),
(3) the period of treatment can be set longer by selecting a combination drug having different action and mechanism from the compound of the present invention,
(4) a sustained treatment effect can be designed by selecting a combination drug having different action and mechanism from the compound of the present invention,
(5) a synergistic effect can be afforded by a combined use of the compound of the present invention and a combination drug, and the like, can be achieved.

As regards the use of the combination agent of the present invention, the administration time of the compound of the present invention and the concomitant drug is not restricted, and the compound of the present invention or the concomitant drug can be administered to an administration subject simultaneously, or may be administered at different times. In addition, the combination agent can be used after synovectomy, after treatment with Prosorba column, after mononuclear cell therapy, and the like. The dosage of the concomitant drug may be determined according to the dose clinically used, and can be appropriately selected depending on an administration subject, administration route, disease, combination and the like.

The administration mode of the compound of the present invention and the concomitant drug of the present invention is not particularly restricted, and it is sufficient that the compound of the present invention and the concomitant drug are combined in administration. Examples of such administration mode include the following methods: (1) The compound of the present invention and the concomitant drug are simultaneously produced to give a single preparation which is administered. (2) The compound of the present invention and the concomitant drug are separately produced to give two kinds of preparations which are administered simultaneously by the same administration route. (3) The compound of the present invention and the concomitant drug are separately produced to give two kinds of preparations which are administered by the same administration route only at the different times. (4) The compound of the present invention and the concomitant drug are separately produced to give two kinds of preparations which are administered simultaneously by the different administration routes. (5) The compound of the present invention and the concomitant drug are separately produced to give two kinds of preparations which are administered by the different administration routes only at different times (for example, the compound of the present invention and the concomitant drug are administered in this order, or in the reverse order).

A combination agent of the present invention has low toxicity, and for example, the compound of the present invention or (and) the above-mentioned concomitant drug can be mixed, according to a method known per se, with a pharmacologically acceptable carrier to give pharmaceutical compositions, for example, tablets (including a sugar-coated tablet, film-coated tablet), powders, granules, capsules (including a soft capsule), solutions, injections, suppositories, sustained release agents and the like which can be safely administered orally or parenterally (e.g., local, rectum, vein, and the like). An injection can be administered by intravenous, intramuscular, subcutaneous or intraorgan route, or directly to the lesion.

As the pharmacologically acceptable carrier which may be used for preparing a preparation of a combination agent of the present invention, there are the various conventional organic or inorganic carriers as pharmaceutical materials, for example, excipient, lubricant, binder and disintegrating agent in solid preparations, or solvent, solubilizing agent, suspending agent, isotonizing agent, buffer and soothing agent in liquid preparations. Further, if needed, additives such as the conventional preservative, antioxidant, colorant, sweetening agent, adsorbing agent, wetting agent and the like can be appropriately used in an appropriate amount.

As the excipient, for example, there are lactose, sucrose, D-mannitol, starch, corn starch, microcrystalline cellulose, light anhydrous silicic acid and the like.

As the lubricant, for example, there are magnesium stearate, calcium stearate, talc, colloidal silica and the like.

As the binder, for example, there are microcrystalline cellulose, sucrose, D-mannitol, dextrin, hydroxypropylcellulose, hydroxypropylmethylcellulose, polyvinylpyrrolidone, starch, saccharose, gelatin, methylcellulose, sodium carboxymethylcellulose and the like.

As the disintegrating agent, for example, there are starch, carboxymethylcellulose, calcium carboxymethylcellulose, sodium carboxymethylstarch, L-hydroxypropylcellulose and the like.

As the solvent, for example, there are water for injection, alcohol, propylene glycol, macrogol, sesame oil, corn oil, olive oil and the like.

As the solubilizing agent, for example, there are polyethylene glycol, propylene glycol, D-mannitol, benzyl benzoate, ethanol, tris-aminomethane, cholesterol, triethanolamine, sodium carbonate, sodium citrate and the like.

As the suspending agent, for example, there are surfactants such as stearyl triethenolamine, sodium lauryl sulfate, lauryl aminopropionate, lecithin, benzalkonium chloride, benzethonium chloride, glyceryl monostearate and the like; hydrophilic polymers such as polyvinyl alcohol, polyvinylpyrrolidone, sodium carboxymethylcellulose, methylcellulose, hydroxymethylcellulose, hydroxyethylcellulose, hydroxypropylcellulose and the like.

As the isotonizing agent, for example, there are glucose, D-sorbitol, sodium chloride, glycerin, D-mannitol and the like.

As the buffer, for example, there are buffering solutions such as phosphate, acetate, carbonate, citrate and the like.

As the soothing agent, for example, there are benzyl alcohol and the like.

As the preservative, for example, there are p-hydroxybenzoates, chlorobutanol, benzyl alcohol, phenethyl alcohol, dehydroacetic acid, sorbic acid and the like.

As the antioxidant, for example, there are sulfites, ascorbic acid, α-tocopherol and the like. The compounding ratio of the compound of the present invention to the concomitant drug in the combination agent of the present invention can be appropriately selected depending on an administration subject, administration route, diseases and the like.

For example, the content of the compound of the present invention in the combination agent of the present invention differs depending on the form of a preparation, and usually from about 0.01 to 100% by weight, preferably from about 0.1 to 50% by weight, further preferably from about 0.5 to 20% by weight, based on the preparation.

The content of the concomitant drug in the combination agent of the present invention differs depending on the form of a preparation, and usually from about 0.01 to 100% by weight, preferably from about 0.1 to 50% by weight, further preferably from about 0.5 to 20% by weight, based on the preparation.
The content of additives such as a carrier and the like in the combination agent of the present invention differs depending on the form of a preparation, and usually from about 1 to 99.99% by weight, preferably from about 10 to 90% by weight, based on the preparation.

In the case when the compound of the present invention and the combination drug are separately prepared respectively, the same contents may be adopted.

These preparations can be produced by a method known per se usually used in a preparation process.

For example, the compound of the present invention and the concomitant drug can be made into an aqueous injection together with a dispersing agent (e.g., Tween 80 (manufactured by Atlas Powder, US), HCO 60 (manufactured by Nikko Chemicals), polyethylene glycol, carboxymethylcellulose, sodium alginate, hydroxypropylmethylcellulose, dextrin and the like), a stabilizer (e.g., ascorbic acid, sodium pyrosulfite, and the like), a surfactant (e.g., Polysorbate 80, macrogol and the like), a solubilizer (e.g., glycerin, ethanol and the like), a buffer (e.g., phosphoric acid and alkali metal salt thereof, citric acid and alkali metal salt thereof, and the like), an isotonizing agent (e.g., sodium chloride, potassium chloride, mannitol, sorbitol, glucose and the like), a pH regulator (e.g., hydrochloric acid, sodium hydroxide and the like), a preservative (e.g., ethyl p-hydroxybenzoate, benzoic acid, methylparaben, propylparaben, benzyl alcohol and the like), a dissolving agent (e.g., conc. glycerin, meglumine and the like), a dissolution aid (e.g., propylene glycol, sucrose and the like), a soothing agent (e.g., glucose, benzyl alcohol and the like), and the like, or can be dissolved, suspended or emulsified in a vegetable oil such as olive oil, sesame oil, cotton seed oil, corn oil and the like or a dissolution aid such as propylene glycol and molded into an oily injection.

In the case of a preparation for oral administration, an excipient (e.g., lactose, sucrose, starch and the like), a disintegrating agent (e.g., starch, calcium carbonate and the like), a binder (e.g., starch, acacia, carboxymethylcellulose, polyvinylpyrrolidone, hydroxpropylcellulose and the like), a lubricant (e.g., talc, magnesium stearate, polyethylene glycol 6000 and the like) and the like, for example, can be added to the compound of the present invention or the combination drug, according to a method known per se, and the mixture can be compression-molded, then if desirable, the molded product can be coated by a method known per se for the purpose of masking of taste, enteric property or durability, to obtain a preparation for oral administration. As this coating agent, for example, hydroxypropylmethylcellulose, ethylcellulose, hydroxymethylcellulose, hydroxypropylcellulose, polyoxyethylene glycol, Tween 80, Pluronic F68, cellulose acetate phthalate, hydroxypropylmethylcellulose phthalate, hydroxymethylcellulose acetate succinate, Eudragit (methacrylic acid·acrylic acid copolymer, manufactured by Rohm, DE), pigment (e.g., iron oxide red, titanium dioxide, et.) and the like can be used. The preparation for oral administration may be any of a quick release preparation and a sustained release preparation.

For example, in the case of a suppository, the compound of the present invention and the combination drug can be made into an oily or aqueous solid, semisolid or liquid suppository according to a method known per se. As the oily substrate used in the above-mentioned composition, for example, glycerides of higher fatty acids [e.g., cacao butter, Witepsols (manufactured by Dynamite Novel, DE), etc.], intermediate grade fatty acids [e.g., Miglyols (manufactured by Dynamite Nobel, DE), etc.], or vegetable oils (e.g., sesame oil, soy bean oil, cotton seed oil and the like), and the like are listed. Further, as the aqueous substrate, for example, polyethylene glycols, propylene glycol are listed, and as the aqueous gel substrate, for example, natural gums, cellulose derivatives, vinyl polymers, acrylic acid polymers and the like are listed.

As the above-mentioned sustained release agent, sustained release microcapsules and the like are listed.

For obtaining a sustained release microcapsule, a method known per se can be adopted, and for example, it is preferably molded into a sustained release preparation shown in the following [2] before administration.

A compound of the present invention is preferably molded into an oral administration preparation such as a solid preparation (e.g., powder, granule, tablet, capsule) and the like, or molded into a rectal administration preparation such as a suppository. Particularly, an oral administration preparation is preferable.

The concomitant drug can be made into the above-mentioned drug form depending on the kind of the drug.

[1] An injection of the compound of the present invention or the concomitant drug, and preparation thereof, [2] a sustained release preparation or quick release preparation of the compound of the present invention or the concomitant drug, and preparation thereof, [3] a sublingual, buccal or intraoral quick integrating agent of the compound of the present invention or the concomitant drug, and preparation thereof, will be described below specifically.

### [1] Injection and preparation thereof

An injection prepared by dissolving the compound of the present invention or the concomitant drug into water is preferable. This injection may be allowed to contain a benzoate and/or salicylate.

The injection is obtained by dissolving the compound of the present invention or the concomitant drug, and if desirable, a benzoate and/or salicylate, into water.

As the above-mentioned salts of benzoic acid and salicylic acid, for example, salts of alkali metals such as sodium, potassium and the like, salts of alkaline earth metals such as calcium, magnesium and the like, ammonium salts, meglumine salts, organic acid salts such as tromethamol and the like, etc. are listed.

The concentration of the compound of the present invention or the concomitant drug in an injection is from 0.5 to 50 w/v%, preferably from about 3 to 20 w/v%. The concentration of a benzoate salt or/and salicylate salt is from 0.5 to 50 w/v%, preferably from 3 to 20 w/v%.

Into a preparation of the present invention, additives usually used in an injection, for example, a stabilizer (ascorbic acid, sodium pyrosulfite, and the like), a surfactant (Polysorbate 80, macrogol and the like), a solubilizer (glycerin, ethanol and the like), a buffer (phosphoric acid and alkali metal salt thereof, citric acid and alkali metal salt thereof, and the like), an isotonizing agent (sodium chloride, potassium chloride, and the like), a dispersing agent (hydroxypropylmethylcellulose, dextrin), a pH regulator (hydrochloric acid, sodium hydroxide and the like), a preservative (ethyl p-hydroxybenzoate, benzoic acid and the like), a dissolving agent (conc. glycerin, meglumine and the like), a dissolution aid (propylene glycol, sucrose and the like), a soothing agent (glucose, benzyl alcohol and the like), and the like, can be appropriately compounded. These additives are generally compounded in a proportion usually used in an injection.

It is advantageous that pH of an injection is controlled from 2 to 12, preferably from 2.5 to 8.0 by addition of a pH regulator.

An injection is obtained by dissolving the compound of the present invention or the concomitant drug and if desirable, a benzoate and/or a salicylate, and if necessary, the above-mentioned additives into water. These may be dissolved in any order, and can be appropriately dissolved in the same manner as in a conventional method of producing an injection.

An aqueous solution for injection may be advantageously be heated, alternatively, for example, filter sterilization, high pressure heat sterilization and the like can be conducted in the same manner as for a usual injection, to provide an injection.

It may be advantageous that an aqueous solution for injection is subjected to high pressure heat sterilization at 100 to 121°C for 5 to 30 minutes.

Further, a preparation endowed with an antibacterial property of a solution may also be produced so that it can be used as a preparation which is divided and administered multiple times.

### [2] Sustained release preparation or quick release preparation, and preparation thereof

A sustained release preparation is preferable which is obtained, if desirable, by coating a nucleus containing the compound of the present invention or the concomitant drug with a film agent such as a water-insoluble substance, swellable polymer and the like. For example, a sustained release preparation for oral administration for a single administration per day type is preferable.

As the water-insoluble substance used in a film agent, there are listed, for example, cellulose ethers such as ethylcellulose, butylcellulose ad the like, cellulose esters such as cellulose stearate, cellulose propionate and the like, polyvinyl esters such as polyvinyl acetate, polyvinyl butyrate and the like, acrylic acid/methacrylic acid copolymers, methyl methacrylate copolymers, ethoxyethyl methacrylate/cinnamoethyl methacrylate/aminoalkyl methacrylate copolymers, polyacrylic acid, polymethacrylic acid, methacrylic acid alkylamide copolymers, poly(methyl methacrylate), polymethacrylate, polymethacrylamide, aminoalkyl methacrylate copolymers, poly(methacrylic anhydride), glycidyl methacrylate copolymer, particularly, acrylic acid-based polymers such as Eudragits (Rohm Pharma) such as Eudragit RS-100, RL-100, RS-30D, RL-30D, RL-PO, RS-PO (ethyl acrylate·methyl methacrylate·trimethyl chloride methacrylate·ammoniumethyl copolymer), Eudragit NE-30D (methyl methacrylate·ethyl acrylate copolymer), and the like, hardened oils such as hardened castor oil (e.g., Lovery wax (Freunt) and the like), waxes such as carnauba wax, fatty acid glycerin ester, paraffin and the like, polyglycerin fatty esters, and the like.

As the swellable polymer, polymers having an acidic dissociating group and showing pH dependent swelling are preferable, and polymers manifesting slight swelling in acidic regions such as in the stomach and greater swelling in neutral regions such as in the small intestine and the large intestine are preferable.

As such a polymer having an acidic dissociating group and showing pH dependent swelling, cross-linkable polyacrylic acid copolymers such as, for example, Carbomer 934P, 940, 941, 974P, 980, 1342 and the like, polycarbophil, calcium polycarbophil (last two are manufactured by BF Goodrich), Hibiswako 103, 104, 105, 304 (all are manufactured by Wako Pure Chemical Co., Ltd.), and the like, are listed.

The film agent used in a sustained release preparation may further contain a hydrophilic substance.

As the hydrophilic substance, for example, polysaccharides which may contain a sulfate group such as pullulan, dextrin, alkali metal alginate and the like, polysaccharides having a hydroxyalkyl group or carboxyalkyl group such as hydroxypropylcellulose, hydroxypropylmethylcellulose, carboxymethylcellulose sodium and the like, methylcellulose, polyvinylpyrrolidone, polyvinyl alcohol, polyethylene glycol and the like.

The content of a water-insoluble substance in the film agent of a sustained release preparation is from about 30 to 90% (w/w), preferably from about 35 to 80% (w/w), further preferably from about 40 to 75% (w/w), the content of a swellable polymer is from about 3 to 30% (w/w), preferably from about 3 to 15% (w/w). The film agent may further contain a hydrophilic substance, and in which case, the content of a hydrophilic substance in the film agent is about 50% (w/w) or less, preferably about 5 to 40% (w/w), further preferably from about 5 to 35% (w/w). This % (w/w) indicates % by weight based on a film agent composition which is obtained by removing a solvent (e.g., water, lower alcohols such as methanol, ethanol and the like) from a film agent solution.

The sustained release preparation is produced by preparing a nucleus containing a drug as exemplified below, then, coating the resulting nucleus with a film agent solution prepared by heat-solving a water-insoluble substance, swellable polymer and the like or by dissolving or dispersing it in a solvent.

### I. Preparation of nucleus containing drug

The form of nucleus containing a drug to be coated with a film agent (hereinafter, sometimes simply referred to as nucleus) is not particularly restricted, and preferably, the nucleus is formed into particles such as a granule or fine particle.

When the nucleus is composed of granules or fine particles, the average particle size thereof is preferably from about 150 to 2000 µm, further preferably, from about 500 to 1400 µm.

Preparation of the nucleus can be effected by a usual production method. For example, a suitable excipient, binding agent, integrating agent, lubricant, stabilizer and the like are mixed into a drug, and the mixture is subjected to a wet extrusion granulating method, fluidized bed granulating method or the like, to prepare a nucleus.

The content of drugs in a nucleus is from about 0.5 to 95% (w/w), preferably from about 5.0 to 80% (w/w), further preferably from about 30 to 70% (w/w).

As the excipient contained in the nucleus, for example, saccharides such as sucrose, lactose, mannitol, glucose and the like, starch, crystalline cellulose, calcium phosphate, corn starch and the like are used. Among them, crystalline cellulose and corn starch are preferable.

As the bonder, for example, polyvinyl alcohol, hydroxypropyl cellulose, polyethylene glycol, polyvinyl pyrrolidone, Pluronic F68, gum Arabic, gelatin, starch and the like are used. As the disintegrating agent, for example, carboxymethylcellulose calcium (ECG505), crosscarmelose sodium (Ac-Di-Sol), crosslinked polyvinylpyrrolidone (Crospovidone), lower substituted hydroxypropylcellulose (L-HPC) and the like are used. Among them, hydroxypropylcellulose, polyvinylpyrrolidone, lower substituted hydroxypropylcellulose are preferable. As the lubricant and coagulation inhibitor, for example, talc, magnesium stearate and inorganic salts thereof are used, and as the lubricant, polyethylene glycol and the like are used. As the stabilizer, acids such as tartaric acid, citric acid, succinic acid, fumaric acid, maleic acid and the like, are used.

A nucleus can also be prepared by, in addition to the above-mentioned, for example, a rolling granulation method in which a drug or a mixture of a drug with an excipient, lubricant and the like is added portionwise onto an inert carrier particle which is the core of the nucleus while spraying a binder dissolved in a suitable solvent such as water, lower alcohol (e.g., methanol, ethanol and the like) and the like, a pan coating method, a fluidized bed coating method or a melt granulating method. As the inert carrier particle, for example, those made of sucrose, lactose, starch, crystalline cellulose, waxes can be used, and the average particle size thereof is preferably from about 100 µm to 1500 µm.

For separating a drug and a film agent contained in a nucleus, the surface of the nucleus may be coated with a protective agent. As the protective agent, for example, the above-mentioned hydrophilic substances, water-insoluble substances and the like are used. As the protective agent, preferably polyethylene glycol, and polysaccharides having a hydroxyalkyl group or carboxyalkyl group are used, more preferably, hydroxypropylmethylcellulose and hydroxypropylcellulose are use. The protective agent may contain, as a stabilizer, acids such as tartaric acid, citric acid, succinic acid, fumaric acid, maleic acid and the like, and lubricants such as talc and the like. When the protective agent is used, the coating amount is from about 1 to 15% (w/w), preferably from about 1 to 10% (w/w), further preferably from about 2 to 8% (w/w), based on the nucleus.

The protective agent can be coated by a usual coating method, and specifically, the protective agent can be coated, for example, by a fluidized bed coating method, pan coating method and the like.

### II. Coating of nucleus with film agent

A nucleus obtained in the above-mentioned step I is coated with a film agent solution obtained by heat-solving the above-mentioned water-insoluble substance and pH-dependent swellable polymer, and a hydrophilic substance, or by dissolving or dispersing them in a solvent, to give a sustained release preparation.

As the method for coating a nucleus with a film agent solution, for example, a spray coating method and the like are listed.

The composition ratio of a water-insoluble substance, swellable polymer and hydrophilic substance in a film agent solution is appropriately selected so that the contents of these components in a coated film are the above-mentioned contents, respectively.

The coating amount of a film agent is from about 1 to 90% (w/w), preferably from about 5 to 50% (w/w), further preferably from about 5 to 35% (w/w), based on a nucleus (not including coating amount of protective agent).

As the solvent in a film agent solution, water or an organic solvent can be used alone or in admixture thereof. In the case of use in admixture, the mixing ratio of water to an organic solvent (water/organic solvent: by weight) can be varied in the range from 1 to 100%, and preferably from 1 to about 30%. The organic solvent is not particularly restricted providing it dissolves a water-insoluble substance, and for example, lower alcohols such as methyl alcohol, ethyl alcohol, isopropyl alcohol, n-butyl alcohol and the like, lower alkanone such as acetone and the like, acetonitrile, chloroform, methylene chloride and the like are used. Among them, lower alcohols are preferable, and ethyl alcohol and isopropyl alcohol are particularly preferable. Water, and a mixture of water with an organic solvent are preferably used as a solvent for a film agent. In this case, if necessary, an acid such as tartaric acid, citric acid, succinic acid, fumaric acid, maleic acid and the like may also be added into a film agent solution for stabilizing the film agent solution. An operation of coating by spray coating can be effected by a usual coating method, and specifically, it can be effected by spray-coating a film agent solution onto a nucleus by a fluidized bed coating method, pan coating method and the like. In this case, if necessary, talc, titanium oxide, magnesium stearate, calcium stearate, light anhydrous silicic acid and the like may also be added as a lubricant, and glycerin fatty ester, hardened castor oil, triethyl citrate, cetyl alcohol, stearyl alcohol and the like may also be added as a plasticizer.

After coating with a film agent, if necessary, an antistatic agent such as talc and the like may be mixed.

The quick release preparation may be liquid (solution, suspension, emulsion and the like) or solid (particle, pill, tablet and the like). Oral agents and parenteral agents such as an injection and the like are used, and oral agents are preferable.

The quick release preparation, usually, may contain, in addition to an active component drug, also carriers, additives and excipients conventionally used in the production field (hereinafter, sometimes abbreviated as excipient). The preparation excipient used is not particularly restricted providing it is an excipient ordinarily used as a preparation excipient. For example, as the excipient for an oral solid preparation, lactose, starch, corn starch, crystalline cellulose (Acevil PH101, manufactured by Asahi Chemical Industry Co., Ltd., and the like), powder sugar, granulated sugar, mannitol, light anhydrous silicic acid, magnesium carbonate, calcium carbonate, L-cysteine and the like are listed, and preferably, corn starch and mannitol and the like are listed. These excipients can be used alone or in combination of two or more. The content of the excipient is, for example, from about 4.5 to 99.4 w/w%, preferably from about 20 to 98.5 w/w%, further preferably from about 30 to 97 w/w%, based on the total amount of the quick release preparation.

The content of a drug in the quick release preparation can be appropriately selected in the range from about 0.5 to 95%, preferably from about 1 to 60% based on the total amount of the quick release preparation.

When the quick release preparation is an oral solid preparation, it usually contains, in addition to the above-mentioned components, also an integrating agent. As this integrating agent, there are used, for example, carboxymethylcellulose calcium (ECG-505, manufactured by Gotoku Yakuhin), crosscarmelose sodium (for example, Actisol, manufactured by Asahi Chemical Industry Co., Ltd.), crosspovidone (for example, Colicone CL, manufactured by BASF), lower substitution hydroxypropylcellulose (manufactured by Shin-Etsu Chemical Co., Ltd.), carboxymethylstarch (manufactured by Matsutani Kagaku K.K.), carboxymethylstarch sodium (Exprotab, manufactured by Kimura Sangyo), partially pregelatinized starch (PCS, manufactured by Asahi Chemical Industry Co., Ltd.), and the like are used, and for example, those which disintegrate a granule by adsorbing water in contact with water, causing swelling, or making a channel between an effective ingredient constituting the nucleus and an excipient, can be used. These disintegrating agents can be used alone or in combination of two or more. The amount of the disintegrating agent used is appropriately selected depending on the kind and compounding amount of a drug used, design of releasing property, and the like, and for example, from about 0.05 to 30 w/w%, preferably from about 0.5 to 15 w/w%, based on the total amount of the quick releasing agent.

When the quick release preparation is an oral solid preparation, it may further contain, in addition to the above-mentioned composition, if desired, additives conventional in solid preparations. As such an additive, there are used, for example, a binder (e.g., sucrose, gelatin, gum Arabic powder, methylcellulose, hydroxypropylcellulose, hydroxypropylmethylcellulose, carboxylmethylcellulose, polyvinylpyrrolidone, pullulan, dextrin and the like), a lubricant (e.g., polyethylene glycol, magnesium stearate, talc, light anhydrous silicic acid (e.g., aerosil (Nippon Aerosil)), a surfactant (e.g., anionic surfactants such as sodium alkylsulfate and the like, nonionic surfactants such as polyoxyethylene fatty acid ester and polyoxyethylene sorbitan fatty acid ester, polyoxyethylene castor oil derivatives and the like), a coloring agent (e.g., tar coloring matter, caramel, iron oxide red, titanium oxide, riboflavins), if necessary, an appetizing agent (e.g., sweetening agent, aroma and the like), an adsorbent, preservative, wetting agent, antistatic agent, and the like, Further, as the stabilizer, an organic acid such as tartaric acid, citric acid, succinic acid, fumaric acid and the like may also be added.

As the above-mentioned binder, hydroxypropylcellulose, polyethylene glycol and polyvinylpyrrolidone and the like are preferably used.

The quick releasing preparation can be prepared by, based on a usual technology of producing preparations, mixing the above-mentioned components, and if necessary, further kneading the mixture, and molding it. The above-mentioned mixing is conducted by generally used methods, for example, mixing, kneading and the like. Specifically, when a quick release preparation is formed, for example, into a particle, it can be prepared, according to the same means as in the above-mentioned method for preparing a nucleus of a sustained release preparation, by mixing the components using a vertical granulator, universal kneader (manufactured by Hata Tekkosho), fluidized bed granulator FD-5S (manufactured by Pulek), and the like, then, subjecting the mixture to a wet extrusion granulation method, fluidized bed granulation method and the like.

Thus obtained quick releasing preparation and sustained releasing preparation may be themselves made into products or made into products appropriately together with preparation excipients and the like, separately, by an ordinary method, then, may be administered simultaneously or may be administered in combination at any administration interval, or they may be themselves made into one oral preparation (e.g., granule, fine particle, tablet, capsule and the like) or made into one oral preparation together with preparation excipients and the like. It may also be permissible that they are made into granules or fine particles, and filled in the same capsule to be used as a preparation for oral administration.

### [3] Sublingual, buccal or intraoral quick disintegrating agent and preparation thereof

Sublingual, buccal or intraoral quick disintegrating agents may be a solid preparation such as tablet and the like, or may be an oral mucosa membrane patch (film).

As the sublingual, buccal or intraoral quick disintegrating agent, a preparation containing the compound of the present invention or the concomitant drug and an excipient is preferable. It may contain also auxiliary agents such as a lubricant, isotonizing agent, hydrophilic carrier, water-dispersible polymer, stabilizer and the like. Further, for easy absorption and increase in *in vivo* use efficiency, β-cyclodextrin or β-cyclodextrin derivatives (e.g., hydroxypropyl-β-cyclodextrin and the like) and the like may also be contained.

As the above-mentioned excipient, lactose, sucrose, D-mannitol, starch, crystalline cellulose, light anhydrous silicic acid and the like are listed. As the lubricant, magnesium stearate, calcium stearate, talc, colloidal silica and the like are listed, and particularly, magnesium stearate and colloidal silica are preferable. As the isotonizing agent, sodium chloride, glucose, fructose, mannitol, sorbitol, lactose, saccharose, glycerin, urea and the like are listed, and particularly, mannitol is preferable. As the hydrophilic carrier, swellable hydrophilic carriers such as crystalline cellulose, ethylcellulose, crosslinkable polyvinylpyrrolidone, light anhydrous silicic acid, silicic acid, dicalcium phosphate, calcium carbonate and the like are listed, and particularly, crystalline cellulose (e.g., fine crystalline cellulose and the like) is preferable. As the water-dispersible polymer, gums (e.g., gum tragacanth, acacia gum, cyamoposis gum), alginates (e.g., sodium alginate), cellulose derivatives (e.g., methylcellulose, carboxymethylcellulose, hydroxymethylcellulose, hydroxypropylcellulose, hydroxypropylmethylcellulose), gelatin, water-soluble starch, polyacrylic acids (e.g., Carbomer), polymethacrylic acid, polyvinyl alcohol, polyethylene glycol, polyvinylpyrrolidone, polycarbofil, ascorbate palmitates and the like are listed, and hydroxypropylmethylcellulose, polyacrylic acid, alginate, gelatin, carboxymethylcellulose, polyvinylpyrrolidone, polyethylene glycol and the like are preferable. Particularly, hydroxypropylmethylcellulose is preferable. As the stabilizer, cysteine, thiosorbitol, tartaric acid, citric acid, sodium carbonate, ascorbic acid, glycine, sodium sulfite and the like are listed, and particularly, citric acid and ascorbic acid are preferable.

The sublingual, buccal or intraoral quick disintegrating agent can be produced by mixing the compound of the present invention or the concomitant drug and an excipient by a method known per se. Further, is desirable, auxiliary agents such as a lubricant, isotonizing agent, hydrophilic carrier, water-dispersible polymer, stabilizer, coloring agent, sweetening agent, preservative and the like may be mixed. The sublingual, buccal or intraoral quick disintegrating agent is obtained by mixing the above-mentioned components simultaneously or at a time interval, then subjecting the mixture to tablet-making molding under pressure. For obtaining suitable hardness, it may also be permissible that the materials are moistened by using a solvent such as water, alcohol and the like if desired before and after the tablet making process, and after the molding, the materials are dried, to obtain a product.

In the case of molding into a mucosa membrane patch (film), the compound of the present invention or the concomitant drug and the above-mentioned water-dispersible polymer (preferably, hydroxypropylcellulose, hydroxypropylmethylcellulose), excipient and the like are dissolved in a solvent such as water and the like, and the resulted solution is cast, to give a film. Further, additives such as a plasticizer, stabilizer, antioxidant, preservative, coloring agent, buffer, sweetening agent and the like may also be added. For imparting suitable elasticity to the film, glycols such as polyethylene glycol, propylene glycol and the like may be contained, or for enhancing adhesion of the film to an intraoral mucosa membrane lining, a bio-adhesive polymer (e.g., polycarbofil, carbopol) may also be contained. In the casting, a solution is poured on the non-adhesive surface, spread to uniform thickness (preferably, about 10 to 1000 micron) by an application tool such as a doctor blade and the like, then, the solution is dried to form a film. It may be advantageous that thus formed film is dried at room temperature or under heat, and cut into given area.

As the preferable intraoral quick disintegrating agent, there are listed solid quick scattering dose agents composed of a network body comprising the compound of the present invention or the concomitant drug, and a water-soluble or water-diffusible carrier which is inert to the compound of the present invention or combination drug, are listed. This network body is obtained by sublimating a solvent from the solid composition constituted of a solution prepared by dissolving the compound of the present invention or the concomitant drug in a suitable solvent.

It is preferable that the composition of an intraoral quick disintegrating agent contains a matrix forming agent and a secondary component, in addition to the compound of the present invention or the concomitant drug.

Examples of the matrix forming agent include animal proteins or vegetable proteins such as gelatins, dextrins and, soybean, wheat and psyllium seed protein and the like; rubber substances such as gum Arabic, guar gum, agar, xanthan gum and the like; polysaccharides; alginic acids; carboxymethylcelluloses; carageenans; dextrans; pectins; synthetic polymers such as polyvinylpyrrolidone and the like; substances derived from a gelatin-gum Arabic complex, and the like. Further, saccharides such as mannitol, dextrose, lactose, galactose, trehalose and the like; cyclic saccharides such as cyclodextrin and the like; inorganic salts such as sodium phosphate, sodium chloride and aluminum silicate and the like; amino acids having 2 to 12 carbon atoms such as glycine, L-alanine, L-aspartic acid, L-glutamic acid, L-hydroxyproline, L-isoleucine, L-leucine, L-phenylalanine and the like, are contained.

One or more of the matrix forming agents can be introduced in a solution or suspension before solidification. Such as matrix forming agent may be present in addition to a surfactant, or may be present while a surfactant being excluded. The matrix forming agent aids to maintain the compound of the present invention or the concomitant drug in the solution or suspension in diffused condition, in addition to formation of the matrix.

The composition may contain secondary components such as a preservative, antioxidant, surfactant, thickening agent, coloring agent, pH controlling agent, flavoring agent, sweetening agent, food taste masking agent and the like. As the suitable coloring agent, there are listed red, black and yellow iron oxides, and FD & C dyes such as FD & C Blue 2, FD & C Red 40 and the like manufactured by Elis and Eberald. Examples of the suitable flavoring agent include mint, raspberry, licorice, orange, lemon, grape fruit, caramel, vanilla, cherry, grape flavor and combinations thereof. Examples of the suitable pH controlling agent include citric acid, tartaric acid, phosphoric acid, hydrochloric acid and maleic acid. Examples of the suitable sweetening agent include aspartame, acesulfame K and thaumatin and the like. Examples of the suitable food taste masking agent include sodium bicarbonate, ion exchange resin, cyclodextrin-containing compounds, adsorbent substances and microcapsulated apomorphine.

The preparation contains the compound of the present invention or the concomitant drug in an amount usually from about 0.1 to 50% by weight, preferably from about 0.1 to 30% by weight, and preferable are preparations (such as the above-mentioned sublingual agent, buccal and the like) which can dissolve 90% or more the compound of the present invention or the concomitant drug (into water) within the time range of about 1 to 60 minutes, preferably of about 1 to 16 minutes, more preferably of about 2 to 5 minutes, and intraoral quick disintegrating preparations which are disintegrated within the range of 1 to 60 seconds, preferably of 1 to 30 seconds, further preferably of 1 to 10 seconds after place in an oral cavity.

The content of the above-mentioned excipient in the whole preparation is from about 10 to 99% by weight, preferably from about 30 to 90% by weight. The content of β-cyclodextrin or β-cyclodextrin derivative in the whole preparation is from 0 to about 30% by weight. The content of the lubricant in the whole preparation is from about 0.01 to 10% by weight, preferably from about 1 to 5% by weight. The content of the isotonizing agent in the whole preparation is from about 0.1 to 90% by weight, preferably, from about 10 to 70% by weight. The content of the hydrophilic carrier agent in the whole preparation is from about 0.1 to 50% by weight, preferably, from about 10 to 30% by weight. The content of the water-dispersible polymer in the whole preparation is from about 0.1 to 30% by weight, preferably, from about 10 to 25% by weight. The content of the stabilizer in the whole preparation is from about 0.1 to 10% by weight, preferably, from about 1 to 5% by weight. The above-mentioned preparation may further contain additives such as a coloring agent, sweetening agent, preservative and the like, if necessary.

The dosage of a combination agent of the present invention differs depending on the kind of a compound (I), age, body weight, condition, drug form, administration method, administration period and the like, and for example, for one sepsis patient (adult, body weight: about 60 kg), the combination agent is administered intravenously, at a dose of about 0.01 to 1000 mg/kg/day, preferably about 0.01 to 100 mg/kg/day, more preferably about 0.1 to 100 mg/kg/day, particularly about 0.1 to 50 mg/kg/day, especially about 1.5 to 30 mg/kg/day, in terms of the compound of the present invention or the concomitant drug, respectively, once or divided several times in a day. Of course, since the dose as described above varies depending on various conditions, amounts smaller than the above-mentioned dosage may sometimes be sufficient, further, amounts over that range sometimes have to be administered.

The amount of the concomitant drug can be set at any value unless side effects are problematical. The daily dosage in terms of the combination drug differs depending on the severity, age, sex, body weight, sensitivity difference of the subject, administration period, interval, and nature, pharmacology, kind of the pharmaceutical preparation, kind of effective ingredient, and the like, and not particularly restricted, and the amount of a drug is, in the case of oral administration for example, usually from about 0.001 to 2000 mg, preferably from about 0.01 to 500 mg, further preferably from about 0.1 to 100 mg, per 1 kg of a mammal and this is usually administered once to 4-times divided in a day.

In administration of a medicine of the present invention, the compound of the present invention may be administered after administration of the concomitant drug or the concomitant drug may be administered after administration of the compound of the present invention, though they may be administered simultaneously. When administered at a time interval, the interval differs depending on the effective ingredient, drug form and administration method, and for example, when the concomitant drug is administered first, a method in which the compound of the present invention is administered within time range of from 1 minute to 3 days, preferably from 10 minutes to 1 day, more preferably from 15 minutes to 1 hour after administration of the concomitant drug is exemplified. When the compound of the present invention is administered first, a method in which the concomitant drug is administered within time range of from 1 minute to 1 day, preferably from 10 minutes to 6 hours, more preferably from 15 minutes to 1 hour after administration of the compound of the present invention is exemplified.

In a preferable administration method, for example, the concomitant drug which has been formed into an oral administration preparation is administered orally at a daily dose of about 0.001 to 200 mg/kg, and 15 minutes after, the compound of the present invention which has been formed into an oral administration preparation is administered orally at a daily dose of about 0.005 to 100 mg/kg.

### Best mode of the invention

The present invention is explained in detail by way of the following Reference Example, Examples, Preparation Examples and Test Examples but these are mere examples and do not limit the present invention and can be varied without departing the scope of the present invention.

"Room temperature" in the following Reference Example and Examples indicates normally about 10°C to about 35°C. "%" indicates percentage by weight unless otherwise indicated, provided that yield represents mol/mol%.

Abbreviations used elsewhere indicate the following meanings:
s: singlet
d: doublet
t: triplet
q: quartet
dd: double doublet
ddd: double double doublet
dt: double triplet
br: broad
J: coupling constant
Hz: Hertz
CDCl₃: deuterated chloroform
¹H-NMR: proton nuclear magnetic resonance
Me: methyl

### Reference Example A 1

### 1-(4-methoxyphenyl)-2-(3-pyridyl)ethanone

A solution of diisopropylamine (33.2 mL) in anhydrous tetrahydrofuran (300 mL) was cooled to -78°C and a 1.6 M n-butyllithium/hexane solution (148 mL) was added dropwise with stirring. After completion of dropwise addition, the mixture was stirred for 10 min at the same temperature, and then β-picoline (20 g) was added dropwise. The temperature was raised to -10-0°C, and after stirring for 20 min, a solution of ethyl p-anisate (19.4 g) in anhydrous tetrahydrofuran (40 mL) was added dropwise. After completion of dropwise addition, the mixture was stirred at room temperature for 1 h, and water (100 mL) was added. The organic solvent was evaporated under reduced pressure and an oily product was extracted with ethyl acetate. The extract was washed with water, and after drying, the solvent was evaporated. The remaining crude crystals were recrystallized from ethyl acetate-isopropyl ether to give the title compound (20.8 g, yield 85%).
m.p.: 71-72°C.

### Reference Example A 2:

In accordance with the above-mentioned Reference Example A 1 and respectively using, instead of ethyl p-anisate, ethyl benzoate, ethyl 3,4-dimethoxybenzoate, ethyl 3,4,5-trimethoxybenzoate, ethyl 4-(methoxymethoxy)benzoate, ethyl 4-fluorobenzoate, ethyl 4-ethylbenzoate, ethyl 3,4-methylenedioxybenzoate, methyl 5-indanylcarboxylate, methyl 5,6,7,8-tetrahydro-2-naphthoate, methyl 1,4-benzodioxane-6-carboxylate and methyl 2-naphthoate, the following Reference Example A compounds 2-1 to 2-11 were synthesized.
Reference Example compound A 2-1: 1-phenyl-2-(3-pyridyl)ethanone m.p.: 44.5-45.5°C.
Reference Example A compound 2-2: 1-(3,4-dimethoxyphenyl)-2-(3-pyridyl)ethanone m.p.: 114-115°C.
Reference Example A compound 2-3: 2-(3-pyridyl)-1-(3,4,5-trimethoxyphenyl)ethanone m.p.: 104-105°C.
Reference Example A compound 2-4: 1-(4-methoxymethoxyphenyl)-2-(3-pyridyl)ethanone m.p.: 43-44°C.
Reference Example A compound 2-5: 1-(4-fluorophenyl)-2-(3-pyridyl)ethanone oil.
Reference Example A compound 2-6: 1-(4-ethylphenyl)-2-(3-pyridyl)ethanone m.p.: 80-81°C.
Reference Example A compound 2-7: 1-(3,4-methylenedioxyphenyl)-2-(3-pyridyl)ethanone m.p.: 98-99°C.
Reference Example A compound 2-8: 1-(5-indanyl)-2-(3-pyridyl)ethanone m.p.: 55-56°C.
Reference Example A compound 2-9: 2-(3-pyridyl)-1-(5,6,7,8-tetrahydro-2-naphthyl)ethanone m.p.: 65-66°C.
Reference Example A compound 2-10: 1-(1,4-benzodioxan-6-yl)-2-(3-pyridyl)ethanone m.p.: 89-90°C.
Reference Example A compound 2-11: 1-(2-naphthyl)-2-(3-pyridyl)ethanone m.p.: 69-70°C.

### Reference Example A 3

In accordance with the above-mentioned Reference Example A 2 and respectively using α-picoline, γ-picoline and 3,5-lutidine instead of β-picoline, the following Reference Example A compounds 3-1 to 3-3 were synthesized.
Reference Example A compound 3-1: 1-phenyl-2-(2-pyridyl)ethanone m.p.: 59-60°C.
Reference Example A compound 3-2: 1-(4-methoxyphenyl)-2-(2-pyridyl)ethanone m.p.: 77-78°C.
Reference Example A compound 3-3: 1-phenyl-2-(4-pyridyl)ethanone m.p.: 109-110°C.

### Reference Example A 4

### 1-(4-methoxyphenyl)-2-(4-pyridyl)ethanone

A solution of diisopropylamine (33.2 mL) in anhydrous tetrahydrofuran (300 mL) was cooled to -78°C and 1.6 M n-butyllithium-hexane solution (148 mL) was added dropwise with stirring. After completion of dropwise addition, the mixture was stirred for 10 min at the same temperature, then γ-picoline (20 g) was added dropwise. The temperature was raised to -10-0°C, and after stirring for 20 min, a solution of ethyl p-anisate (19.4 g) in anhydrous tetrahydrofuran (40 mL) was added dropwise. After completion of dropwise addition, the mixture was stirred at room temperature for 1 h, and water (100 mL) was added. The organic solvent was evaporated under reduced pressure and an oily product was extracted with ethyl acetate. The extract was washed with water, and after drying, the solvent was evaporated. The remaining crude crystals were recrystallized from ethyl acetate-isopropyl ether to give the title compound (16.2 g, yield 66 %).
m.p.: 103-104°C.

### Reference Example A 5

### 2-(5-methyl-3-pyridyl)-1-phenylethanone

A solution of diisopropylamine (20.2 mL) in anhydrous tetrahydrofuran (180 mL) was cooled to -78°C, and a 1.6 M n-butyllithium-hexane solution (90 mL) was added dropwise with stirring. After completion of dropwise addition, the mixture was stirred for 10 min at the same temperature, and then 3,5-lutidine (14 g) was added dropwise. The temperature was raised to -10-0°C, and after stirring for 20 min, a solution of ethyl benzoate (9.8 g) in anhydrous tetrahydrofuran (20 mL) was added dropwise. After completion of dropwise addition, the mixture was stirred at room temperature for 1 h, and water (100 mL) was added. The organic solvent was evaporated under reduced pressure and an oily product was extracted with ethyl acetate. The extract was washed with water, and after drying, the solvent was evaporated. The remaining crude crystals were recrystallized from ethyl acetate-isopropyl ether to give the title compound (10 g, yield 70%).
m.p.: 53-54°C.

### Reference Example A 6

### 2-bromo-1-(4-methoxyphenyl)-2-(3-pyridyl)ethanone hydrobromide

1-(4-Methoxyphenyl)-2-(3-pyridyl)ethanone (6.9 g) was dissolved in acetic acid (36 mL), bromine (1.7 mL) was added, and the mixture was stirred at 80°C for 3 h. The reaction mixture was cooled with iced water and the precipitated crude crystals were collected by filtration. The crude crystals were recrystallized from ethanol-ethyl ether to give the title compound (10 g, yield 89%).
m.p.: 188-195°C.

### Reference Example A 7

In accordance with the above-mentioned Reference Example A 6, 1-phenyl-2-(3-pyridyl)ethanone, 1-(3,4-dimethoxyphenyl)-2-(3-pyridyl)ethanone, 2-(3-pyridyl)-1-(3,4,5-trimethoxyphenyl) ethanone, 1-(4-methoxymethoxyphenyl)-2-(3-pyridyl)ethanone, 1-(4-fluorophenyl)-2-(3-pyridyl)ethanone, 1-phenyl-2-(2-pyridyl)ethanone, 1-(4-methoxyphenyl)-2-(2-pyridyl)ethanone, 1-phenyl-2-(4-pyridyl)ethanone, 1-(4-methoxyphenyl)-2-(4-pyridyl)ethanone, 2-(5-methyl-3-pyridyl)-1-phenylethanone, 1-(4-ethylphenyl)-2-(3-pyridyl)ethanone, 1-(3,4-methylenedioxyphenyl)-2-(3-pyridyl)ethanone, 1-(5-indanyl)-2-(3-pyridyl)ethanone, 2-(3-pyridyl)-1-(5,6,7,8-tetrahydro-2-naphthyl)ethanone, 1-(1,4-benzodioxan-6-yl)-2-(3-pyridyl)ethanone, 1-(2-naphthyl)-2-(3-pyridyl)ethanone and 1-(4-methoxyphenyl)-2-(2-pyridyl)ethanone were respectively used instead of 1-(4-methoxyphenyl)-2-(3-pyridyl)ethanone, the following Reference Example A compounds 7-1 to 7-17 were synthesized.
Reference Example A compound 7-1: 2-bromo-1-phenyl-2-(3-pyridyl)ethanonehydrobromide m.p.: 208-215°C.
Reference Example A compound 7-2: 2-bromo-1-(3,4-dimethoxyphenyl)-2-(3-pyridyl)ethanonehydrobromide m.p.: 191-193°C.
Reference Example A compound 7-3: 2-bromo-2-(3-pyridyl)-1-(3,4,5-trimethoxyphenyl)ethanone hydrobromide m.p.: 184-186°C.
Reference Example A compound 7-4: 2-bromo-1-(4-hydroxyphenyl)-2-(3-pyridyl)ethanone hydrobromide Used in the next reaction without purification.
Reference Example A compound 7-5: 2-bromo-1-(4-fluorophenyl)-2-(3-pyridyl)ethanone hydrobromide m.p.: 189-191°C.
Reference Example A compound 7-6: 2-bromo-1-phenyl-2-(2-pyridyl)ethanone hydrobromide m.p.: 180-181°C.
Reference Example A compound 7-7: 2-bromo-1-(4-methoxyphenyl)-2-(2-pyridyl)ethanone hydrobromide m.p.: 170-171°C.
Reference Example A compound 7-8: 2-bromo-1-phenyl-2-(4-pyridyl)ethanone hydrobromide m.p.: 230-232°C.
Reference Example A compound 7-9: 2-bromo-1-(4-methoxyphenyl)-2-(4-pyridyl)ethanone hydrobromide m.p.: 207-209°C.
Reference Example A compound 7-10: 2-bromo-2-(5-methyl-3-pyridyl)-1-phenylethanone hydrobromide m.p.: 189-193°C.
Reference Example A compound 7-11: 2-bromo-1-(4-ethylphenyl)-2-(3-pyridyl)ethanone hydrobromide m.p.: 145-146°C.
Reference Example A compound 7-12: 2-bromo-1-(3,4-methylenedioxyphenyl)-2-(3-pyridyl)ethanone hydrobromide m.p.: 174-175°C.
Reference Example A compound 7-13: 2-bromo-1-(5-indanyl)-2-(3-pyridyl)ethanone hydrobromide m.p.: 177-178°C.
Reference Example A compound 7-14: 2-bromo-2-(3-pyridyl)-1-(5,6,7,8-tetrahydro-2-naphthyl)ethanone hydrobromide m.p.: 160-162°C.
Reference Example A compound 7-15: 1-(1,4-benzodioxan-6-yl)-2-bromo-2-(3-pyridyl)ethanone hydrobromide oil.
Reference Example A compound 7-16: 2-bromo-1-(2-naphthyl)-2-(3-pyridyl)ethanone hydrobromide m.p.: 197-199°C.
Reference Example A compound 7-17: 2-bromo-1-(4-methoxyphenyl)-2-(2-pyridyl)ethanone hydrobromide m.p.: 170-171°C.

### Reference Example A 8

### [4-(4-methoxyphenyl)-5-(3-pyridyl)-1,3-thiazol-2-yl]amine

To a suspension of thiourea (0.52 g) in acetonitrile (40 mL) was added 2-bromo-1-(4-methoxyphenyl)-2-(3-pyridyl)ethanone hydrobromide (2.5 g) and triethylamine (0.95 mL) was slowly added dropwise with stirring. After completion of dropwise addition, the mixture was stirred at a refluxing temperature for 3 h, and after allowing to cool, the precipitated crystals were collected by filtration. The crystals were washed successively with saturated sodium hydrogencarbonate solution, water, ethanol and ethyl ether and dried. The obtained crude crystals were recrystallized from tetrahydrofuran to give the title compound (1.5 g, yield 90%).
m.p.: 265-266°C.

### Reference Example A 9

### N-methyl [4-(4-methoxyphenyl)-5-(3-pyridyl)-1,3-thiazol-2-yl]amine

To a suspension of N-methylthiourea (0.24 g) in acetonitrile (18 mL) was added 2-bromo-1-(4-methoxyphenyl)-2-(3-pyridyl)ethanone hydrobromide (1.0 g) and triethylamine (0.4 mL) was slowly added dropwise with stirring. After completion of dropwise addition, the mixture was stirred at a refluxing temperature for 3 h, and the solvent was evaporated. To the residue was added saturated aqueous sodium hydrogencarbonate and the mixture was extracted with ethyl acetate, and the extract was washed with water and dried, and the solvent was evaporated. The remaining crude crystals were recrystallized from ethyl acetate-isopropyl ether to give the title compound (0.65 g, yield 85%).
m.p.: 158-159°C.

### Reference Example A 10

### N-[4-(4-methoxyphenyl)-5-(3-pyridyl)-1,3-thiazol-2-yl]acetamide

Using [(4-methoxyphenyl)-5-(3-pyridyl)-1,3-thiazol-2-yl]amine as a starting compound and according to a method similar to Reference Example A 23-128 to be mentioned below, the title compound was obtained (yield 82%).
m.p.: 208-210°C.

### Reference Example A 11

### 2-(4-acetylpiperazin-1-yl)-4-(4-methoxyphenyl)-5-(3-pyridyl)-1,3-thiazole

In a solution of 1-piperazinecarbothioamide (0.39 g) in acetonitrile (15 mL) was suspended 2-bromo-1-(4-methoxyphenyl)-2-(3-pyridyl)ethanone hydrobromide (1.0 g) and triethylamine (0.4 mL) was slowly added dropwise with stirring. After completion of dropwise addition, the mixture was stirred at a refluxing temperature for 3 h, and the solvent was evaporated. To the residue was added saturated aqueous sodium hydrogencarbonate and the mixture was extracted with ethyl acetate, and the extract was washed with water and dried, and the solvent was evaporated. The residue was dissolved in pyridine (2 mL) and cooled with ice. Acetyl chloride (0.3 mL) was added, and the mixture was left standing at room temperature for 1 h. The reaction mixture was poured into iced water, and the resulting product was extracted with ethyl acetate. The extract was washed with water, and after drying, the solvent was evaporated. The residue was purified by silica gel column chromatography (ethyl acetate-methanol=9:1) to give the title compound (0.30 g, yield 28%).
oil

### Reference Example A 12

### [4-(4-methoxyphenyl)-5-(3-pyridyl)-1,3-thiazol-2-yl]amine hydrochloride

[4-(4-Methoxyphenyl)-5-(3-pyridyl)-1,3-thiazol-2-yl]amine (200 mg) was dissolved in 1% hydrochloric acid-methanol (3.2 mL) and the solvent was evaporated. The obtained crude crystals were recrystallized from methanol-ethyl acetate to give the title compound (180 mg, yield 80%).
m.p. : 145-150°C.

The chemical structural formulas of the compounds obtained in Reference Example s A8 to 12 are shown in the following Table 1.

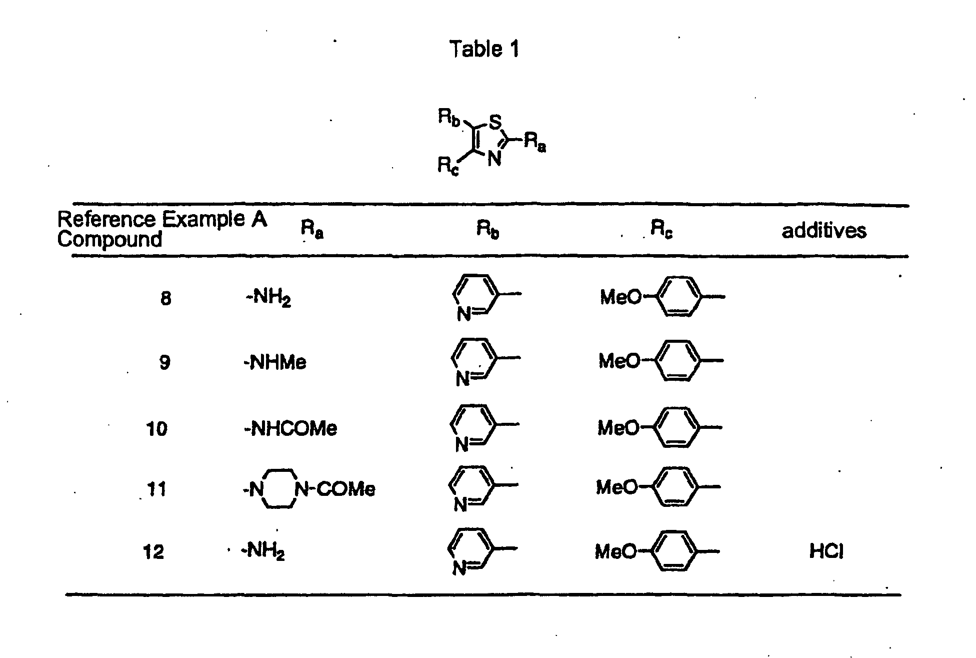

### Reference Example A 13

Reference Example A compounds 13-1 to 13-102 shown in the following Tables 2-7 were synthesized in accordance with the methods described in Reference Example A 8-12, JP-A-61-10580 and USP 4,612,321.

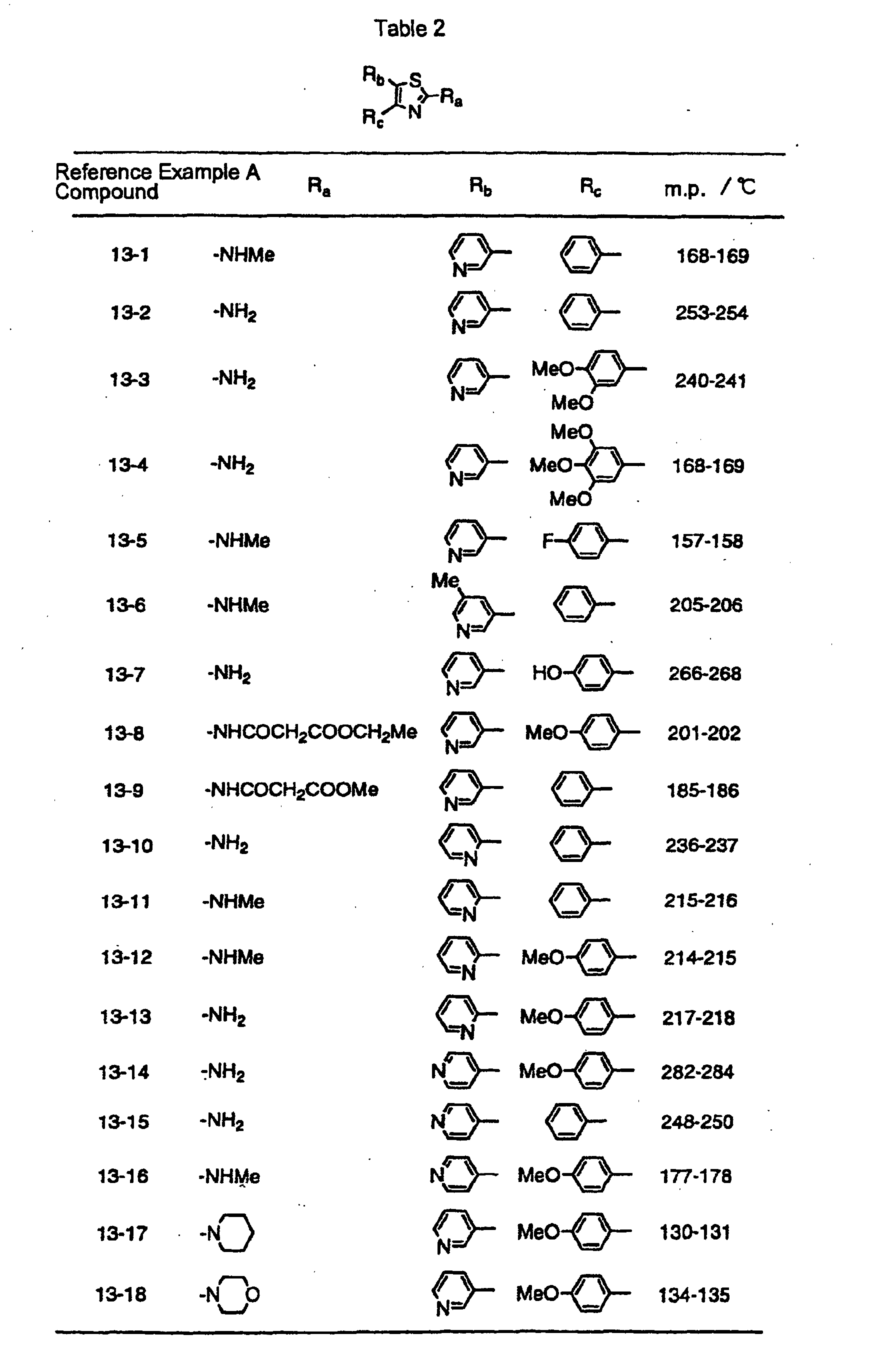

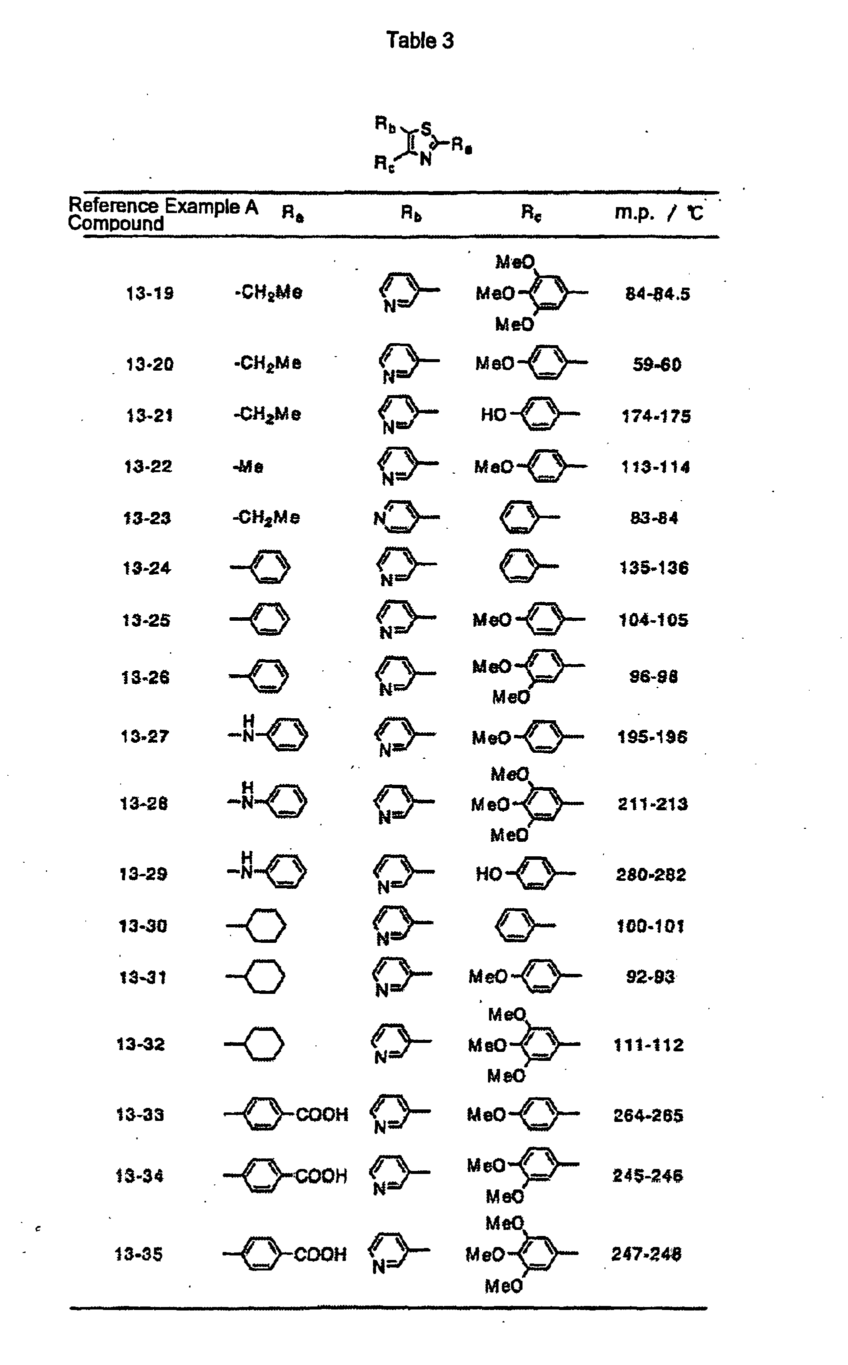

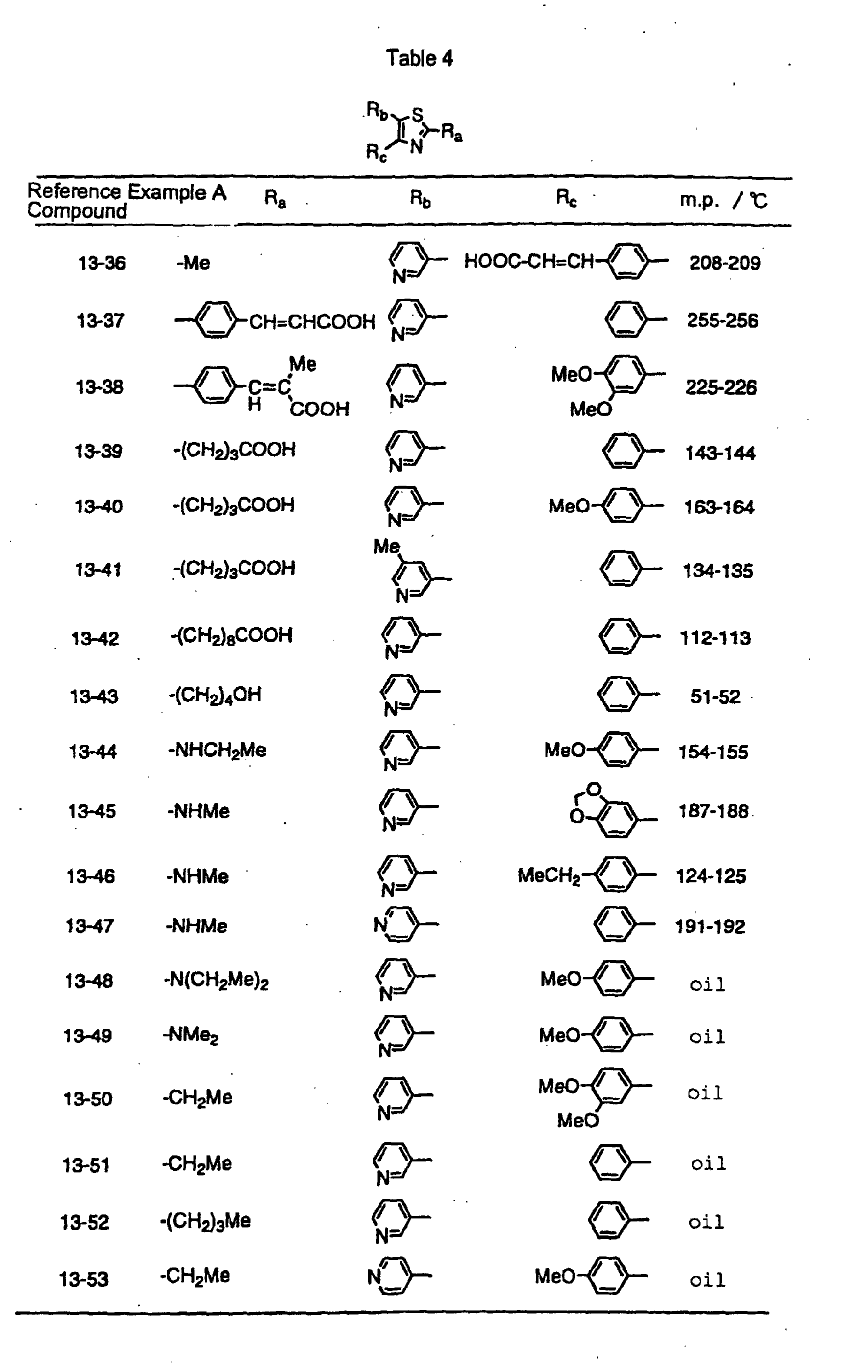

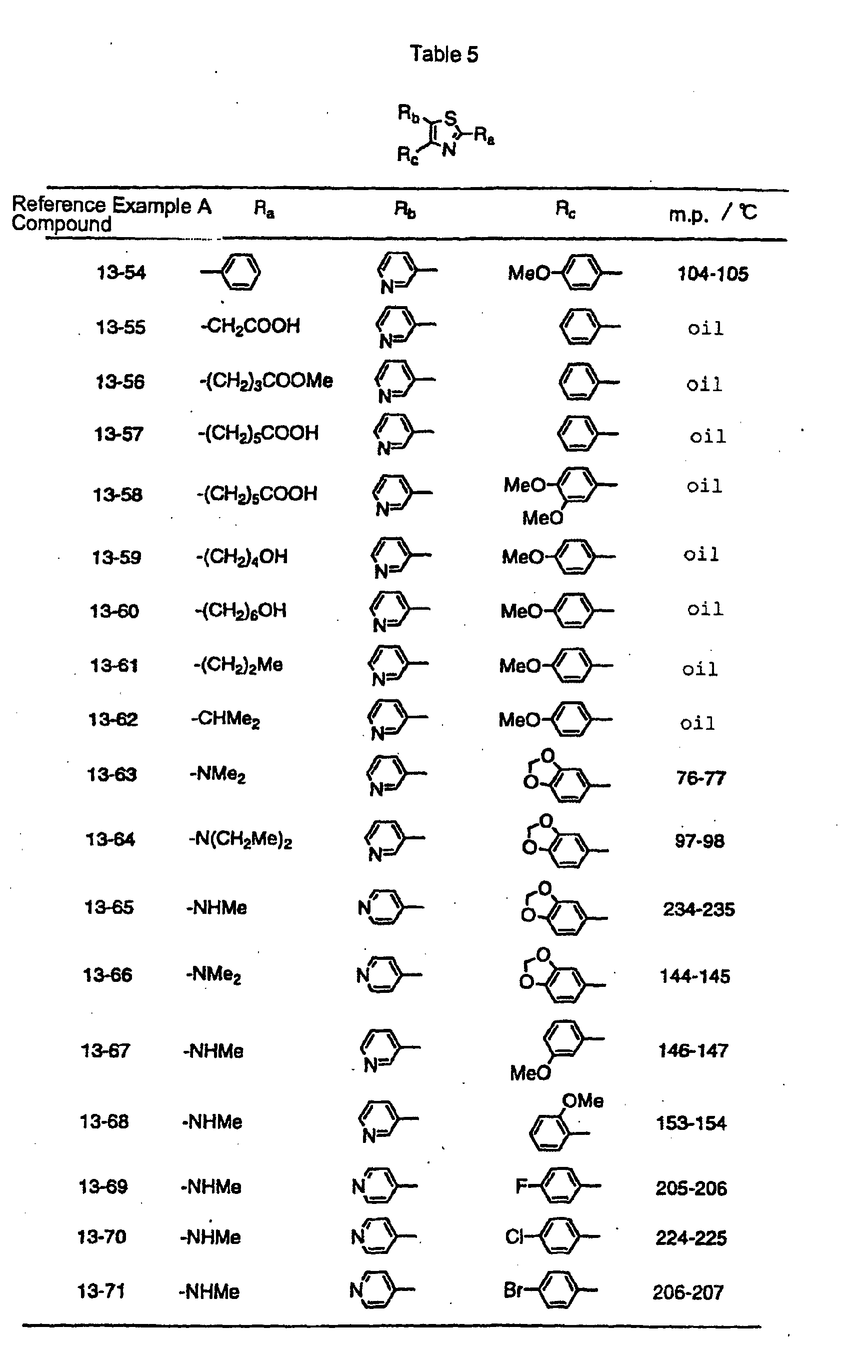

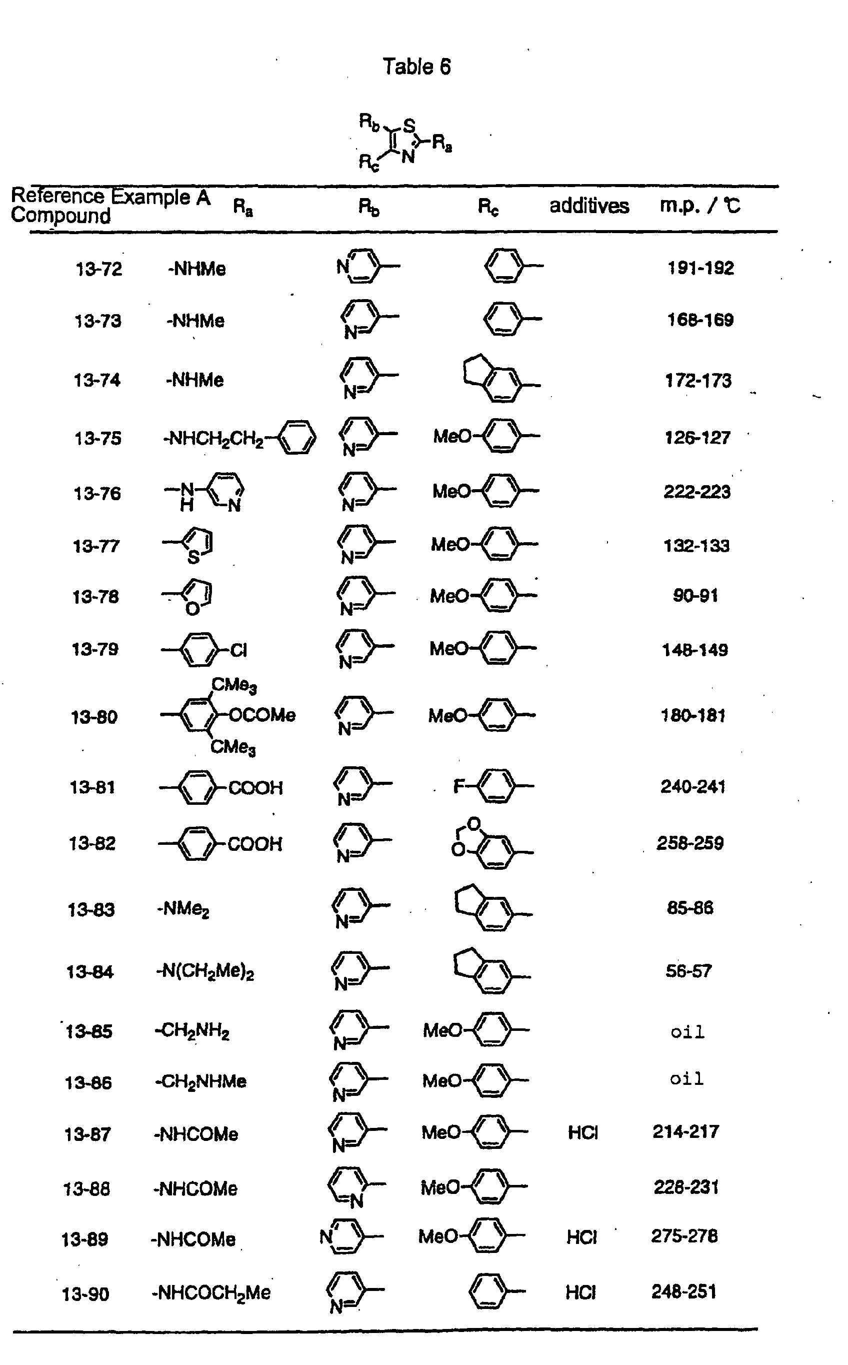

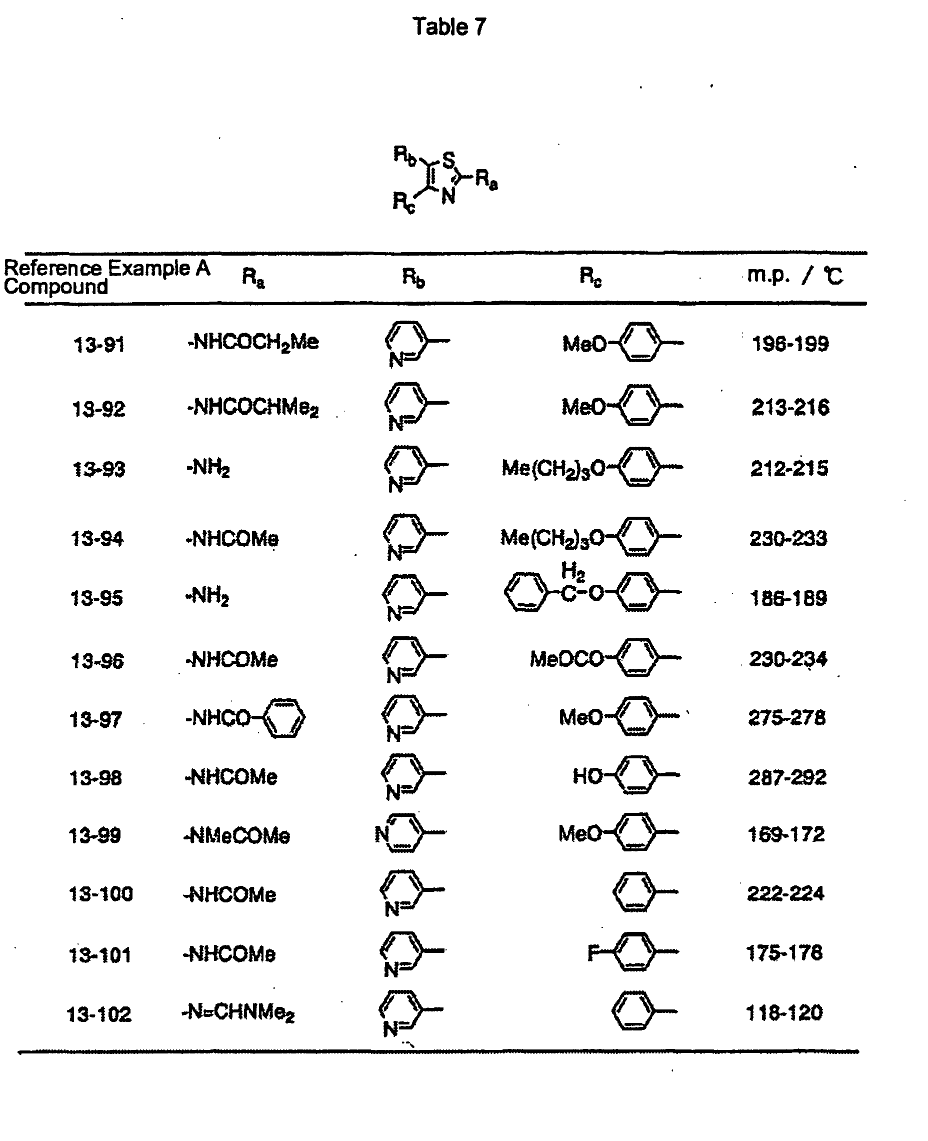

### Reference Example A 14

### N-(4-chlorobenzoyl)propyleneimine

A solution of propyleneimine (12.3 mL) in tetrahydrofuran (160 mL) was added to 1N aqueous sodium hydroxide solution. To this mixture was added dropwise 4-chlorobenzoyl chloride (25 g) at 0°C. After completion of dropwise addition, the mixture was stirred for further 30 min. The reaction mixture was extracted with ethyl acetate. The extract was dried, and the solvent was evaporated to give the title compound (24.9 g, yield 89%).
oil
¹H-NMR (CDCl₃) δ: 1.39 (3H, d, J= 5.5 Hz), 2.15 (1H, d, J= 2.9 Hz), 2.51-2.66 (2H, m), 7.39-7.47 (2H, m), 7.93-8.01 (2H, m).

### Reference Example A 15

In accordance with Reference Example A 14, 3-chlorobenzoyl chloride, 2-chlorobenzoyl chloride, 2-methylbenzoyl chloride, 3-methylbenzoyl chloride, 4-methylbenzoyl chloride, 2-methoxybenzoyl chloride, 3-methoxybenzoyl chloride, 4-ethylbenzoyl chloride, 4-(1-methylethyl)benzoyl chloride, 4-(1,1-dimethylethyl)benzoyl chloride, 4-propylbenzoyl chloride, 4-butylbenzoyl chloride, 4-hexylbenzoyl chloride, 4-trifluoromethoxybenzoyl chloride, 4-trifluoromethylbenzoyl chloride, 3,4-dimethoxybenzoyl chloride, 3,4-dimethylbenzoyl chloride, 3,5-dimethylbenzoyl chloride, 3,4-methylenedioxybenzoyl chloride, 2-naphthoyl chloride, 4-fluorobenzoyl chloride and 3-cyclopentyloxy-4-methoxybenzoyl chloride were respectively used instead of 4-chlorobenzoyl chloride, the following Reference Example A compounds 15-1 to 15-22 were synthesized.
Reference Example A compound 15-1: N-(3-chlorobenzoyl)-propyleneimine
   oil
   ¹H-NMR (CDCl₃) δ: 1.40 (3H, d, J= 5.1 Hz), 2.17 (1H, d, J= 3.3 Hz), 2.53-2.68 (2H, m), 7.40 (1H, dd, J= 8.1, 7.7 Hz), 7.53 (1H, ddd, J= 8.1, 2.2, 1.5 Hz), 7.90 (1H, dt, J= 7.7, 1.5 Hz), 8.00 (1H, dd, J= 2.2, 1.5 Hz).
Reference Example A compound 15-2: N-(2-chlorobenzoyl)-propyleneimine
   oil
   ¹H-NMR (CDCl₃) δ: 1.30 (3H, d, J= 5.1 Hz), 2.12 (1H, d, J= 3.3 Hz), 2.53 (1H, d, J= 5.5 Hz), 2.56-2.68 (1H, m), 7.28-7.48 (3H, m), 7.75-7.81 (1H, m).
Reference Example A compound 15-3: N-(2-methylbenzoyl)-propyleneimine
   oil
   ¹H-NMR (CDCl₃) δ: 1.30 (3H, d, J= 5.5 Hz), 2.08 (1H, d, J= 3.3 Hz), 2.43-2.57 (5H, m), 7.20-7.31 (2H, m), 7.33-7.43 (1H, m), 7.89 (1H, d, J= 7.7 Hz).
Reference Example A compound 15-4: N-(3-methylbenzoyl)-propyleneimine
   oil
   ¹H-NMR (CDCl₃) δ: 1.39 (3H, d, J= 5.5 Hz), 2.14 (1H, d, J= 3.3 Hz), 2.41 (3H, s), 2.51-2.66 (2H, m), 7.32-7.39 (2H, m), 7.79-7.87 (2H, m).
Reference Example A compound 15-5: N-(4-methylbenzoyl)-propyleneimine
   oil
   ¹H-NMR (CDCl₃) δ: 1.39 (3H, d, J= 5.5 Hz), 2.12 (1H, d, J= 2.9 Hz), 2.42 (3H, s), 2.50-2.62 (2H, m), 7.25 (2H, d, J= 8.1 Hz), 7.92 (2H, d, J= 8.1 Hz).
Reference Example A compound 15-6: N-(2-methoxybenzoyl)-propyleneimine
   oil
   ¹H-NMR (CDCl₃) δ: 1.30 (3H, d, J= 5.5 Hz), 2.10 (1H, d, J= 3.3 Hz), 2.50 (1H, d, J= 5.9Hz), 2.53-2.65 (1H, m), 3.90 (3H, s), 6.95-7.05 (2H, m), 7.41-7.52 (1H, m), 7.81-7.88 (1H, m).
Reference Example A compound 15-7: N-(3-methoxybenzoyl)-propyleneimine
   oil
   ¹H-NMR (CDCl₃) δ: 1.40 (3H, d, J= 5.9 Hz), 2.14 (1H, d, J= 2.9 Hz), 2.52-2.65 (2H, m), 3.86 (3H, s), 7.10 (1H, ddd, J= 8.4, 2.6, 1.1 Hz), 7.37 (1H, dd, J= 8.4, 7.3 Hz), 7.55 (1H, dd, J= 2.6, 1.5 Hz), 7.63 (1H, ddd, J= 7.3, 1.5, 1.1 Hz).
Reference Example A compound 15-8: N-(4-ethylbenzoyl)-propyleneimine
   oil
   ¹H-NMR (CDCl₃) δ: 1.27 (3H, t, J= 7.6 Hz), 1.39 (3H, d, J= 5.5 Hz), 2.13 (1H, d, J= 3.3 Hz), 2.50-2.61 (2H, m), 2.71 (2H, q, J= 7.6 Hz), 7.28 (2H, d, J= 7.7 Hz), 7.95 (2H, d, J= 7.7 Hz).
Reference Example A compound 15-9: N-[4-(1-methylethyl)-benzoyl]propyleneimine
   oil
   ¹H-NMR (CDCl₃) δ: 1.28 (6H, d, J= 7.0 Hz) , 1.40 (3H, d, J= 5.5 Hz), 2.13 (1H, d, J= 3.3 Hz), 2.50-2.64 (2H, m), 2.90-3.05 (1H, m), 7.31 (2H, d, J= 8.2 Hz), 7.96 (2H, d, J= 8.2 Hz).
Reference Example A compound 15-10: N-[4-(1,1-dimethylethyl)-benzoyl]propyleneimine
   A solution of propyleneimine (11 mL, 0.14 mol) in tetrahydrofuran (160 mL) was added to 2N aqueous sodium hydroxide solution (70 mL). To this mixture was added dropwise 4-(1,1-dimethylethyl)benzoyl chloride (25 g, 0.13 mol) at 0°C. After completion of dropwise addition, the mixture was stirred further for 30 min. The reaction mixture was extracted with ethyl acetate. The extract was dried, and the solvent was evaporated to give the title compound (27 g, 0.13 mol, yield 99%).
   oil
   ¹H-NMR (CDCl₃) δ: 1.35 (9H, s), 1.41 (3H, d, J= 5.5 Hz), 2.12 (1H, d, J= 2.9 Hz), 2.51-2.64 (2H, m), 7.47 (2H, d, J= 8.8 Hz), 7.96 (2H, d, J= 8.8 Hz).
Reference Example A compound 15-11: N-(4-propylbenzoyl)-propyleneimine
   oil
   ¹H-NMR (CDCl₃) δ: 0.96 (3H, t, J= 7.3 Hz), 1.39 (3H, d, J= 5.5 Hz), 1.57-1.75 (2H, m), 2.12 (1H, d, J= 3.3 Hz), 2.50-2.59 (2H, m), 2.65 (2H, t, J= 7.7 Hz), 7.26 (2H, d, J= 8.1 Hz), 7.94 (2H, d, J= 8.1 Hz).
Reference Example A compound 15-12: N-(4-butylbenzoyl)-propyleneimine
   oil
   ¹H-NMR (CDCl₃) δ: 0.94 (3H, t, J= 7.1 Hz), 1.26-1.47 (5H, m), 1.54-1.73 (2H, m), 2.12 (1H, d, J= 2.9 Hz), 2.51-2.62 (2H, m), 2.67 (2H, t, J= 7.7 Hz), 7.26 (2H, d, J= 8.1 Hz), 7.94 (2H, d, J= 8.1 Hz).
Reference Example A compound 15-13: N-(4-hexylbenzoyl)-propyleneimine
   oil
   ¹H-NMR (CDCl₃) δ: 0.89 (3H, t, J= 6.6 Hz), 1.24-1.38 (6H, m), 1.39 (3H, d, J= 5.5 Hz), 1.56-1.68 (2H, m), 2.12 (1H, d, J= 3.3 Hz), 2.51-2.61 (2H, m), 2.66 (2H, t, J= 7.7 Hz), 7.26 (2H, d, J= 8.1 Hz), 7.94 (2H, d, J= 8.1 Hz).
Reference Example A compound 15-14: N-(4-trifluoromethoxybenzoyl)propyleneimine
   oil
   ¹H-NMR (CDCl₃) δ: 1.40 (3H, d, J= 5.5 Hz), 2.16 (1H, d, J= 3.3 Hz), 2.53-2.68 (2H, m), 7.29 (2H, d, J= 9.0 Hz), 8.08 (2H, d, J= 9.0 Hz).
Reference Example A compound 15-15: N-(4-trifluoromethylbenzoyl)propyleneimine
   oil
   ¹H-NMR (CDCl₃) δ:1.40 (3H, d, J= 5.5 Hz), 2.19 (1H, d, J= 3.7 Hz), 2.54-2.70 (2H, m), 7.73 (2H, d, J= 8.0 Hz), 8.13 (2H, d, J= 8.0 Hz).
Reference Example A compound 15-16: N-(3,4-dimethoxybenzoyl)-propyleneimine
   oil
   ¹H-NMR (CDCl₃) δ: 1.41 (3H, d, J= 5.5 Hz), 2.12 (1H, d, J= 3.3 Hz), 2.51-2.63 (2H, m), 3.94 (3H, s), 3.95 (3H, s), 6.92 (1H, d, J= 8.5 Hz), 7.56 (1H, d, J= 2.2 Hz), 7.69 (1H, dd, J= 8.5, 2.2 Hz).
Reference Example A compound 15-17: N-(3,4-dimethylbenzoyl)-propyleneimine
   oil
   ¹H-NMR (CDCl₃) δ: 1.39 (3H, d, J= 5.5 Hz), 2.12 (1H, d, J= 3.3 Hz), 2.32 (6H, s), 2.49-2.61 (2H, m), 7.21 (1H, d, J= 7.7 Hz) , 7.77 (1H, dd, J= 7.7, 1.8 Hz), 7.80 (1H, d, J= 1.8 Hz).
Reference Example A compound 15-18: N-(3,5-dimethylbenzoyl)-propyleneimine
   3,5-Dimethylbenzoic acid (25 g, 0.17 mol) and dimethylformamide (0.1 mL) were added to thionyl chloride (50 mL) at 0°C successively. The mixture was refluxed under heating for 2 h. The excess thionyl chloride was evaporated under reduced pressure and to the residue was added toluene (50 mL). Toluene was evaporated under reduced pressure to give oily 3,5-dimethylbenzoyl chloride. A solution of propyleneimine (14 mL, 0.18 mol) in tetrahydrofuran (160 mL) was added to 1N aqueous sodium hydroxide solution (180 mL). 3,5-Dimethylbenzoyl chloride was added dropwise to this mixture at 0°C. After completion of dropwise addition, the mixture was stirred further for 30 min. The reaction mixture was extracted with ethyl acetate. The extract was dried, and the solvent was evaporated to give the title compound (31 g, 0.16 mol, yield 99%).
   oil
   ¹H-NMR (CDCl₃) δ: 1.39 (3H, d, J= 5.5 Hz), 2.13 (1H, d, J= 3.7 Hz), 2.37 (6H, s) , 2.47-2.62 (2H, m), 7.19 (1H, s), 7.64 (2H, s).
Reference Example A compound 15-19: N-(3,4-methylenedioxybenzoyl)propyleneimine
   oil
   ¹H-NMR (CDCl₃) δ: 1.38 (3H, d, J= 4.9 Hz), 2.11 (1H, d, J= 3.1 Hz), 2.48-2.64 (2H, m), 6.05 (2H, s), 6.86 (1H, d, J= 8.2 Hz), 7.48 (1H, d, J= 1.7 Hz), 7.65 (1H, dd, J= 8.2, 1.7 Hz).
Reference Example A compound 15-20: N-(2-naphthoyl)-propyleneimine
   oil
   ¹H-NMR (CDCl₃) δ: 1.44 (3H, d, J= 5.5 Hz), 2.22 (1H, d, J= 3.3 Hz), 2.57-2.84 (2H, m), 7.50-7.65 (2H, m), 7.85-8.00 (3H, m), 8.06 (1H, dd, J= 8.6, 1.5 Hz), 8.59 (1H, s).
Reference Example A compound 15-21: N-(4-fluorobenzoyl)-propyleneimine
   oil
   ¹H-NMR (CDCl₃) δ: 1.39 (3H, d, J= 5.2 Hz), 2.14-2.15 (1H, m), 2.52-2.63 (2H, m), 7.08-7.19 (2H, m), 8.00-8.10 (2H, m).
Reference Example compound A 15-22: N-(3-cyclopentyloxy-4-methoxybenzoyl)propyleneimine
   oil
   ¹H-NMR (CDCl₃) δ: 1.40 (3H, d, J= 5.1 Hz), 1.54-1.68 (2H, m), 1.73-2.06 (6H, m), 2.11 (1H, d, J= 3.3 Hz), 2.51-2.63 (2H, m), 3.91 (3H, s), 4.79-4.90 (1H, m), 6.90 (1H, d, J= 8.4 Hz), 7.55 (1H, d, J= 1.8 Hz), 7.65 (1H, dd, J= 8.4, 1.8 Hz).

### Reference Example A 16

### 1-(2-chlorophenyl)-2-(4-pyridyl)ethanone

A solution of diisopropylamine (15 mL) in anhydrous tetrahydrofuran (100 mL) was cooled at -50°C and 1.6 M n-butyllithium/hexane solution (69 mL) was added dropwise with stirring. After completion of dropwise addition, the mixture was stirred for 10 min and a solution of γ-picoline (20 g) in anhydrous tetrahydrofuran (10 mL) was added dropwise at -30°C. The mixture was stirred for 1 h and a solution of N-(2-chlorobenzoyl)propyleneimine (20 g) in anhydrous tetrahydrofuran (10 mL) was added dropwise at -10°C. After completion of dropwise addition, the mixture was stirred for at room temperature for 2 h. To the reaction mixture was added water (100 mL) and the mixture was extracted with ethyl acetate. The extract was washed with water, and after drying, the solvent was evaporated. The residue was purified by silica gel column chromatography (hexane-ethyl acetate=1:1) to give the title compound (16 g, yield 71%).
oil
¹H-NMR (CDCl₃) δ: 4.28 (2H, s), 7.20 (2H, d, J= 6.2 Hz) , 7.28-7.39 (1H, m), 7.41-7.48 (3H, m), 8.56 (2H, d, J= 6.2 Hz).

### Reference Example A 17

In accordance with Reference Example A 16, N-(3-chlorobenzoyl)propyleneimine, N-(4-chlorobenzoyl)-propyleneimine, N-(2-methylbenzoyl)propyleneimine, N-(3-methylbenzoyl)propyleneimine, N-(4-methylbenzoyl)-propyleneimine, N-(2-methoxybenzoyl)propyleneimine, N-(3-methoxybenzoyl)propyleneimine, N-(4-ethylbenzoyl)-propyleneimine, N-[4-(1-methylethyl)benzoyl]propyleneimine, N-[4-(1,1-dimethylethyl)benzoyl]propyleneimine, N-(4-propylbenzoyl)propyleneimine, N-(4-butylbenzoyl)propyleneimine, N-(4-hexylbenzoyl)propyleneimine, N-(4-trifluoromethoxybenzoyl)propyleneimine, N-(4-trifluoromethylbenzoyl)propyleneimine, N-(3,4-dimethoxybenzoyl)propyleneimine, N-(3,4-dimethylbenzoyl)-propyleneimine, N-(3,5-dimethylbenzoyl)propyleneimine, N-(3,4-methylenedioxybenzoyl)propyleneimine, N-(2-naphthoyl)-propyleneimine and N-(3-cyclopentyloxy-4-methoxybenzoyl)-propyleneimine, instead of N-(2-chlorobenzoyl)propyleneimine, the following Reference Example A compounds 17-1 to 17-21 were synthesized.
Reference Example A compound 17-1: 1-(3-chlorophenyl)-2-(4-pyridyl)ethanone
   m.p.: 79-80°C.
Reference Example A compound 17-2: 1-(4-chlorophenyl)-2-(4-pyridyl)ethanone
   m.p.: 93-94°C.
Reference Example A compound 17-3: 1-(2-methylphenyl)-2-(4-pyridyl)ethanone
   oil
   ¹H-NMR (CDCl₃) δ: 2.48 (3H, s), 4.23 (2H, s), 7.19 (2H, d, J= 6.2 Hz), 7.24-7.47 (3H, m), 7.73 (1H, d, J= 7.7 Hz), 8.56 (2H, d, J= 6.2 Hz).
Reference Example A compound 17-4: 1-(3-methylphenyl)-2-(4-pyridyl)ethanone
   m.p.: 115-116°C.
Reference Example A compound 17-5: 1-(4-methylphenyl)-2-(4-pyridyl)ethanone
   m.p.: 110-111°C.
Reference Example A compound 17-6: 1-(2-methoxyphenyl)-2-(4-pyridyl)ethanone
   oil
   ¹H-NMR (CDCl₃) δ: 3.92 (3H, s) , 4.30 (2H, s), 6.95-7.07 (2H, m), 7.17 (2H, d, J= 5.9 Hz), 7.50 (1H, ddd, J= 8.4, 7.3, 1.8 Hz), 7.73 (1H, dd, J= 7.7, 1.8 Hz), 8.53 (2H, d, J= 5.9 Hz).
Reference Example A compound 17-7: 1-(3-methoxyphenyl)-2-(4-pyridyl)ethanone
   oil
   ¹H-NMR (CDCl₃) δ: 3.86 (3H, s), 4.28 (2H, s), 7.14 (1H, ddd, J= 8.1, 2.6, 1.1 Hz), 7.20 (2H, d, J= 6.2 Hz), 7.36 (1H, dd, J= 8.1, 7.7 Hz), 7.51 (1H, dd, J= 2.6, 1.5 Hz), 7.58 (1H, ddd, J= 7.7, 1.5, 1.1 Hz), 8.57 (2H, d, J= 6.2 Hz).
Reference Example A compound 17-8: 1-(4-ethylphenyl)-2-(4-pyridyl)ethanone
   m.p.: 87-89°C.
Reference Example A compound 17-9: 1-[4-(1-methylethyl)phenyl]-2-(4-pyridyl)ethanone
   m.p.: 86-88°C.
Reference Example A compound 17-10: 1-[4-(1,1-dimethylethyl)-phenyl]-2-(4-pyridyl)ethanone
   A solution of diisopropylamine (15 mL, 0.11 mol) in anhydrous tetrahydrofuran (100 mL) was cooled to -50°C, 1.6 M n-butyllithium-hexane solution (69 mL, 0.11 mol) was added dropwise with stirring. After completion of dropwise addition, the mixture was stirred for 10 min, and then a solution of γ-picoline (9.3 g, 0.10 mol) in anhydrous tetrahydrofuran (10 mL) was added dropwise at -30°C. The mixture was stirred for 1 h, a solution of N-[4-(1,1-dimethylethyl)benzoyl]-propyleneimine (22 g, 0.10 mol) in anhydrous tetrahydrofuran (10 mL) was added dropwise at -30°C. After completion of dropwise addition, the temperature of the mixture was increased gradually to room temperature and the mixture was stirred for 2 h. To the reaction mixture was added water (100 mL), the mixture was extracted with ethyl acetate. The extract was washed with water, and after drying, the solvent was evaporated. The residue was purified by silica gel column chromatography (hexane-ethyl acetate, 1:1) and recrystallized from diisopropyl ether-hexane to give the title compound (11 g, yield 43%).
   m.p.: 75-76°C.
Reference Example A compound 17-11: 1-(4-propylphenyl)-2-(4-pyridyl)ethanone
   m.p.: 71-72°C.
Reference Example A compound 17-12: 1-(4-butylphenyl)-2-(4-pyridyl)ethanone
   m.p.: 41-43°C.
Reference Example A compound 17-13: 1-(4-hexylphenyl)-2-(4-pyridyl)ethanone
   m.p.: 57-58°C.
Reference Example A compound 17-14: 2-(4-pyridyl)-1-(4-trifluoromethoxyphenyl)ethanone
   m.p.: 65-66°C.
Reference Example A compound 17-15: 2-(4-pyridyl)-1-(4-trifluoromethylphenyl)ethanone
   m.p.: 94-95°C.
Reference Example A compound 17-16: 1-(3,4-dimethoxyphenyl)-2-(4-pyridyl)ethanone
   m.p.: 110-111°C.
Reference Example A compound 17-17: 1-(3,4-dimethylphenyl)-2-(4-pyridyl)ethanone
   m.p.: 81-83°C.
Reference Example A compound 17-18 1-(3,5-dimethylphenyl)-2-(4-pyridyl)ethanone
   A solution of diisopropylamine (15 mL, 0.11 mol) in anhydrous tetrahydrofuran (100 mL) was cooled to -50°C, 1.6 M n-butyllithium-hexane solution (69 mL, 0.11 mol) was added dropwise with stirring. After completion of dropwise addition, the mixture was stirred for 10 min, and a solution of γ-picoline (9.3 g, 0.10 mol) in anhydrous tetrahydrofuran (10 mL) was added dropwise at -30°C. The mixture was stirred for 1 h, a solution of N-(3,5-dimethylbenzoyl)propyleneimine (19 g, 0.10 mol) in anhydrous tetrahydrofuran (10 mL) was added dropwise at -30°C. After completion of dropwise addition, the temperature of the mixture was gradually raised to room temperature and the mixture was stirred for 2 h. To the reaction mixture was added water (100 mL) and the mixture was extracted with ethyl acetate. The extract was washed with water, and after drying, the solvent was evaporated. The residue was crystallized from diisopropyl ether-hexane to give the title compound (13 g, yield 58%).
   m.p.: 90-91°C.
Reference Example A compound 17-19: 1-(3,4-methylenedioxyphenyl)-2-(4-pyridyl)ethanone
   m.p.: 126-127°C.
Reference Example A compound 17-20: 1-(2-naphthyl)-2-(4-pyridyl)ethanone
   m.p.: 114-115°C.
Reference Example A compound 17-21: 1-(3-cyclopentyloxy-4-methoxyphenyl)-2-(4-pyridyl)ethanone
   m.p.: 87-89°C.

### Reference Example A 18

In accordance with Reference Example A 17, the following Reference Example A compound 18-1-18-9 were synthesized using γ-picoline instead of β-picoline.
Reference Example A compound 18-1: 1-(2-chlorophenyl)-2-(3-pyridyl)ethanone
   oil
   ¹H-NMR (CDCl₃) δ: 4.28 (2H, s), 7.18-7.49 (5H, m), 7.59-7.67 (1H, m), 8.47-8.56 (2H, m).
Reference Example A compound 18-2: 1-(3-chlorophenyl)-2-(3-pyridyl)ethanone
   oil
   ¹H-NMR (CDCl₃) δ: 4.29 (2H, s), 7.25-7.34 (1H, m), 7.44 (1H, t, J= 7.7 Hz), 7.54-7.63 (2H, m), 7.90 (1H, dt, J= 7.7, 1.5 Hz), 8.00 (1H, dd, J= 1.8, 1.5 Hz), 8.49-8.57 (2H, m).
Reference Example A compound 18-3: 1-(4-chlorophenyl)-2-(3-pyridyl)ethanone
   ¹H-NMR (CDCl₃) δ: 4.27 (2H, s), 7.24-7.31 (1H, m), 7.47 (2H, d, J= 8.8 Hz), 7.55-7.63 (1H, m), 7.96 (2H, d, J= 8.8 Hz), 8.46-8.53 (2H, m).
Reference Example A compound 18-4: 1-(2-methylphenyl)-2-(3-pyridyl)ethanone
   oil
   ¹H-NMR (CDCl₃) δ: 2.47 (3H, s), 4.23 (2H, s), 7.18-7.47 (5H, m), 7.73 (1H, d, J= 7.7 Hz), 8.47-8.56 (2H, m).
Reference Example A compound 18-5: 1-(3-methylphenyl)-2-(3-pyridyl)ethanone
   oil
   ¹H-NMR (CDCl₃) δ: 2.43 (3H, s), 4.29 (2H, s), 7.17-7.36 (1H, m), 7.36-7.46 (2H, m), 7.58-7.65 (1H, m), 7.78-7.86 (2H, m), 8.50- 8.56 (2H, m).
Reference Example A compound 18-6: 1-(4-methylphenyl)-2-(3-pyridyl)ethanone
   m.p. : 72-74°C.
Reference Example A compound 18-7: 1-(3-methoxyphenyl)-2-(3-pyridyl)ethanone
   oil
   ¹H-NMR (CDCl₃) δ: 3.86 (3H, s), 4.29 (2H, s), 7.14 (1H, ddd, J= 8.1, 2.6, 1.8 Hz), 7.28 (1H, dd, J= 7.3, 4.8 Hz), 7.40 (1H, dd, J= 8.1, 7.7 Hz), 7.53 (1H, dd, J= 2.6, 1.8.Hz), 7.58-7.65 (2H, m), 8.50-8.55 (2H, m).
Reference Example A compound 18-8: 1-[4-(1,1-dimethylethyl)phenyl]-2-(3-pyridyl)ethanone
   oil
   ¹H-NMR (CDCl₃) δ: 1.34 (9H, s), 4.28 (2H, s), 7.22-7.31 (1H, m), 7.50 (2H, d, J= 8.4 Hz), 7.56-7.65 (1H, m), 7.96 (2H, d, J= 8.4 Hz), 8.48-8.55 (2H, m).
Reference Example A compound 18-9: 1-(3,5-dimethylphenyl)-2-(3-pyridyl)ethanone
   oil
   ¹H-NMR (CDCl₃) δ: 2.38 (6H, s), 4.27 (2H, s), 7.24-7.30 (2H, m), 7.58-7.63 (3H, m), 8.50-8.52 (2H, m).

### Reference Example A 19

In accordance with Reference Example A 1, the following Reference Example A compound 19 was synthesized using ethyl 4-dimethylaminobenzoate instead of ethyl p-anisate.
Reference Example A compound 19: 1-(4-dimethylaminophenyl)-2-(4-pyridyl)ethanone
m.p.: 189-192°C.

### Reference Example A 20

### 1-(4-fluorophenyl)-2-(4-pyridyl) ethanone

A solution of diisopropylamine (29 mL) in anhydrous tetrahydrofuran (300 mL) was cooled to -78°C, and 1.6 M n-butyllithium/hexane solution (140 mL) was added dropwise with stirring. After completion of dropwise addition, the mixture was stirred for 10 min, and then a solution of γ-picoline (21 g) in anhydrous tetrahydrofuran (50 mL) was added. The reaction mixture was stirred at -10°C for 30 min. The reaction solution was cooled to -78°C and a solution of N-(4-fluorobenzoyl)propyleneimine (36 g) in anhydrous tetrahydrofuran (50 mL) was added dropwise. After completion of dropwise addition, the mixture was stirred at room temperature for 3 h. To the reaction mixture was added water (100 mL) and extracted with ethyl acetate. The extract was washed with water, and after drying, the solvent was evaporated. The residue was crystallized from diisopropyl ether to give the title compound (28 g, yield 66%).
m.p.: 90-91°C.

### Reference Example A 21

### 4-(methylthio)thiobenzamide

4-Methylthiobenzonitrile (12 g) was dissolved in a solution (130 mL) of 4N hydrogen chloride in ethyl acetate. To this solution was added O,O-diethyl dithiophosphate (15 mL) and the mixture was stirred at room temperature for 22 h. To the reaction mixture was added water (100 mL), and the mixture was extracted with ethyl acetate. The insoluble material was filtered off and the filtrate was washed with saturated brine, dried and the solvent was evaporated. The residue was recrystallized from ethyl acetate to give the title compound (10 g, yield 67%).
m.p.: 176-178°C.

### Reference Example A 22

In accordance with Reference Example A 6 and respectively using 1-(2-chlorophenyl)-2-(3-pyridyl)ethanone, 1-(3-chlorophenyl)-2-(3-pyridyl)ethanone, 1-(4-chlorophenyl)-2-(3-pyridyl)ethanone, 1-(2-methylphenyl)-2-(3-pyridyl)ethanone, 1-(3-methylphenyl)-2-(3-pyridyl)ethanone, 1-(4-methylphenyl)-2-(3-pyridyl)ethanone, 1-(3-methoxyphenyl)-2-(3-pyridyl)ethanone, 1-[4-(1,1-dimethylethyl)phenyl]-2-(3-pyridyl)ethanone, 1-(3,5-dimethylphenyl)-2-(3-pyridyl)ethanone, 1-(2-chlorophenyl)-2-(4-pyridyl)ethanone, 1-(3-chlorophenyl)-2-(4-pyridyl)ethanone, 1-(4-chlorophenyl)-2-(4-pyridyl)ethanone, 1-(2-methylphenyl)-2-(4-pyridyl)ethanone, 1-(3-methylphenyl)-2-(4-pyridyl)ethanone, 1-(4-methylphenyl)-2-(4-pyridyl)ethanone, 1-(2-methoxyphenyl)-2-(4-pyridyl)ethanone, 1-(3-methoxyphenyl)-2-(4-pyridyl)ethanone, 1-(4-ethylphenyl)-2-(4-pyridyl)ethanone, 1-[4-(1-methylethyl)phenyl]-2-(4-pyridyl)ethanone, 1-[4-(1,1-dimethylethyl)phenyl]-2-(4-pyridyl)ethanone, 1-(4-propylphenyl)-2-(4-pyridyl)ethanone, 1-(4-butylphenyl)-2-(4-pyridyl)ethanone, 1-(4-hexylphenyl)-2-(4-pyridyl)ethanone, 2-(4-pyridyl)-1-(4-trifluoromethoxyphenyl)ethanone, 2-(4-pyridyl)-1-(4-trifluoromethylphenyl)ethanone, 1-(4-dimethylaminophenyl)-2-(4-pyridyl)ethanone hydrobromide, 1-(3,4-dimethoxyphenyl)-2-(4-pyridyl)ethanone, 1-(3,4-dimethylphenyl)-2-(4-pyridyl)ethanone, 1-(3,5-dimethylphenyl)-2-(4-pyridyl)ethanone, 1-(3,4-methylenedioxyphenyl)-2-(4-pyridyl)ethanone, 1-(2-naphthyl)-2-(4-pyridyl)ethanone, 1-(4-fluorophenyl)-2-(4-pyridyl)ethanone and 1-(3-cyclopentyloxy-4-methoxyphenyl)-2-(4-pyridyl)ethanone instead of 1-(4-methoxyphenyl)-2-(3-pyridyl)ethanone, the following Reference Example A compounds 22-1 to 22-33 were synthesized.
Reference Example A compound 22-1: 2-bromo-1-(2-chlorophenyl)-2-(3-pyridyl)ethanone hydrobromide
   m.p.: 88-90°C.
Reference Example A compound 22-2: 2-bromo-1-(3-chlorophenyl)-2-(3-pyridyl)ethanone hydrobromide
   m.p.: 164-166°C
Reference Example A compound 22-3: 2-bromo-1-(4-chlorophenyl)-2-(3-pyridyl)ethanone hydrobromide
   Used in the next reaction without purification.
Reference Example A compound 22-4: 2-bromo-1-(2-methylphenyl)-2-(3-pyridyl)ethanone hydrobromide
   Used in the next reaction without purification.
Reference Example A compound 22-5: 2-bromo-1-(3-methylphenyl)-2-(3-pyridyl)ethanone hydrobromide
   Used in the next reaction without purification.
Reference Example A compound 22-6: 2-bromo-1-(4-methylphenyl)-2-(3-pyridyl)ethanone hydrobromide
   m.p.: 96-98°C.
Reference Example A compound 22-7: 2-bromo-1-(3-methoxyphenyl)-2-(3-pyridyl)ethanone hydrobromide
   Used in the next reaction without purification.
Reference Example A compound 22-8: 2-bromo-1-[4-(1,1-dimethylethyl)phenyl]-2-(3-pyridyl)ethanone hydrobromide
   m.p.: 190-194°C.
Reference Example A compound 22-9: 2-bromo-1-(3,5-dimethylphenyl)-2-(3-pyridyl)ethanone hydrobromide
   m.p.: 195-197°C.
Reference Example A compound 22-10: 2-bromo-1-(2-chlorophenyl)-2-(4-pyridyl)ethanone hydrobromide
   m.p.: 157-159°C.
Reference Example A compound 22-11: 2-bromo-1-(3-chlorophenyl)-2-(4-pyridyl)ethanone hydrobromide
   m.p.: 178-181°C.
Reference Example A compound 22-12: 2-bromo-1-(4-chlorophenyl)-2-(4-pyridyl)ethanone hydrobromide
   m.p.: 189-193°C.
Reference Example A compound 22-13: 2-bromo-1-(2-methylphenyl)-2-(4-pyridyl)ethanone hydrobromide
   m.p.: 183-186°C.
Reference Example A compound 22-14: 2-bromo-1-(3-methylphenyl)-2-(4-pyridyl)ethanone hydrobromide
   Used in the next reaction without purification.
Reference Example A compound 22-15: 2-bromo-1-(4-methylphenyl)-2-(4-pyridyl)ethanone hydrobromide
   m.p.: 111-113°C.
Reference Example A compound 22-16: 2-bromo-1-(2-methoxyphenyl)-2-(4-pyridyl)ethanone hydrobromide
   m.p.: 168-171°C.
Reference Example A compound 22-17: 2-bromo-1-(3-methoxyphenyl)-2-(4-pyridyl)ethanone hydrobromide
   Used in the next reaction without purification.
Reference Example A compound 22-18: 2-bromo-1-(4-ethylphenyl)-2-(4-pyridyl)ethanone hydrobromide
   m.p.: 170-173°C.
Reference Example A compound 22-19: 2-bromo-1-[4-(1-methylethyl)phenyl]-2-(4-pyridyl)ethanone hydrobromide
   m.p.: 185-188°C.
Reference Example A compound 22-20: 2-bromo-1-[4-(1,1-dimethylethyl)phenyl]-2-(4-pyridyl)ethanone hydrobromide
   1-[4-(1,1-Dimethylethyl)phenyl]-2-(4-pyridyl)ethanone (10 g, 39 mmol) was dissolved in acetic acid (40 mL) and bromine (2.0 mL, 39 mmol) was added. The mixture was stirred at 80°C for 3 h. The reaction mixture was cooled with iced water and the precipitated crude crystals were collected by filtration. The crude crystals were washed with ethyl acetate to give the title compound (9.6 g, yield 81%).
   m.p.: 209-212°C.
Reference Example A compound 22-21: 2-bromo-1-(4-propylphenyl)-2-(4-pyridyl)ethanone hydrobromide
   m.p.: 167-170°C.
Reference Example A compound 22-22: 2-bromo-1-(4-butylphenyl)-2-(4-pyridyl)ethanone hydrobromide
   m.p.: 158-161°C.
Reference Example A compound 22-23: 2-bromo-1-(4-hexylphenyl)-2-(4-pyridyl)ethanone hydrobromide
   m.p.: 153-155°C.
Reference Example A compound 22-24: 2-bromo-2-(4-pyridyl)-1-(4-trifluoromethoxyphenyl)ethanone hydrobromide
   Used in the next reaction without purification.
Reference Example A compound 22-25: 2-bromo-2-(4-pyridyl)-1-(4-trifluoromethylphenyl)ethanone hydrobromide
   m.p.: 190-194°C.
Reference Example A compound 22-26: 2-bromo-1-(4-dimethylaminophenyl)-2-(4-pyridyl)ethanone dihydrobromide
   m.p.: 163-167°C.
Reference Example A compound 22-27: 2-bromo-1-(3,4-dimethoxyphenyl)-2-(4-pyridyl)ethanone hydrobromide
   m.p.: 174-175°C.
Reference Example A compound 22-28: 2-bromo-1-(3,4-dimethylphenyl)-2-(4-pyridyl)ethanone hydrobromide
   m.p.: 196-199°C.
Reference Example A compound 22-29: 2-bromo-1-(3,5-dimethylphenyl)-2-(4-pyridyl)ethanone hydrobromide
1-(3,5-Dimethylphenyl)-2-(4-pyridyl)ethanone (7.0 g, 31 mmol) was dissolved in acetic acid (35 mL) and bromine (1.6 mL, 31 mmol) was added. The mixture was stirred at 80°C for 3 h. Ethyl acetate was added to the residue and the precipitated crude crystals were collected by filtration. The crude crystals were washed with ethyl acetate to give the title compound (16 g, yield 96%).
   m.p.: 216-219°C.
Reference Example A compound 22-30: 2-bromo-1-(3,4-methylenedioxyphenyl)-2-(4-pyridyl)ethanone hydrobromide
   m.p.: 211-214°C.
Reference Example A compound 22-31: 2-bromo-1-(2-naphthyl)-2-(4-pyridyl)ethanone hydrobromide
   m.p.: 149-152°C.
Reference Example A compound 22-32: 2-bromo-1-(4-fluorophenyl)-2-(4-pyridyl)ethanone hydrobromide
   m.p.: 185-189°C.
Reference Example A compound 22-33: 2-bromo-1-(3-cyclopentyloxy-4-methoxyphenyl)-2-(4-pyridyl)ethanone hydrobromide
   m.p.: 168-170°C.

### Reference Example A 23

In accordance with the method described in Reference Example s A 8-12, JP-A-61-10580 and USP 4,612,321, Reference Example A compounds 23-1 to 23-294 and 23-295 to 23-349 shown in the following Tables 8 to 31 were synthesized.

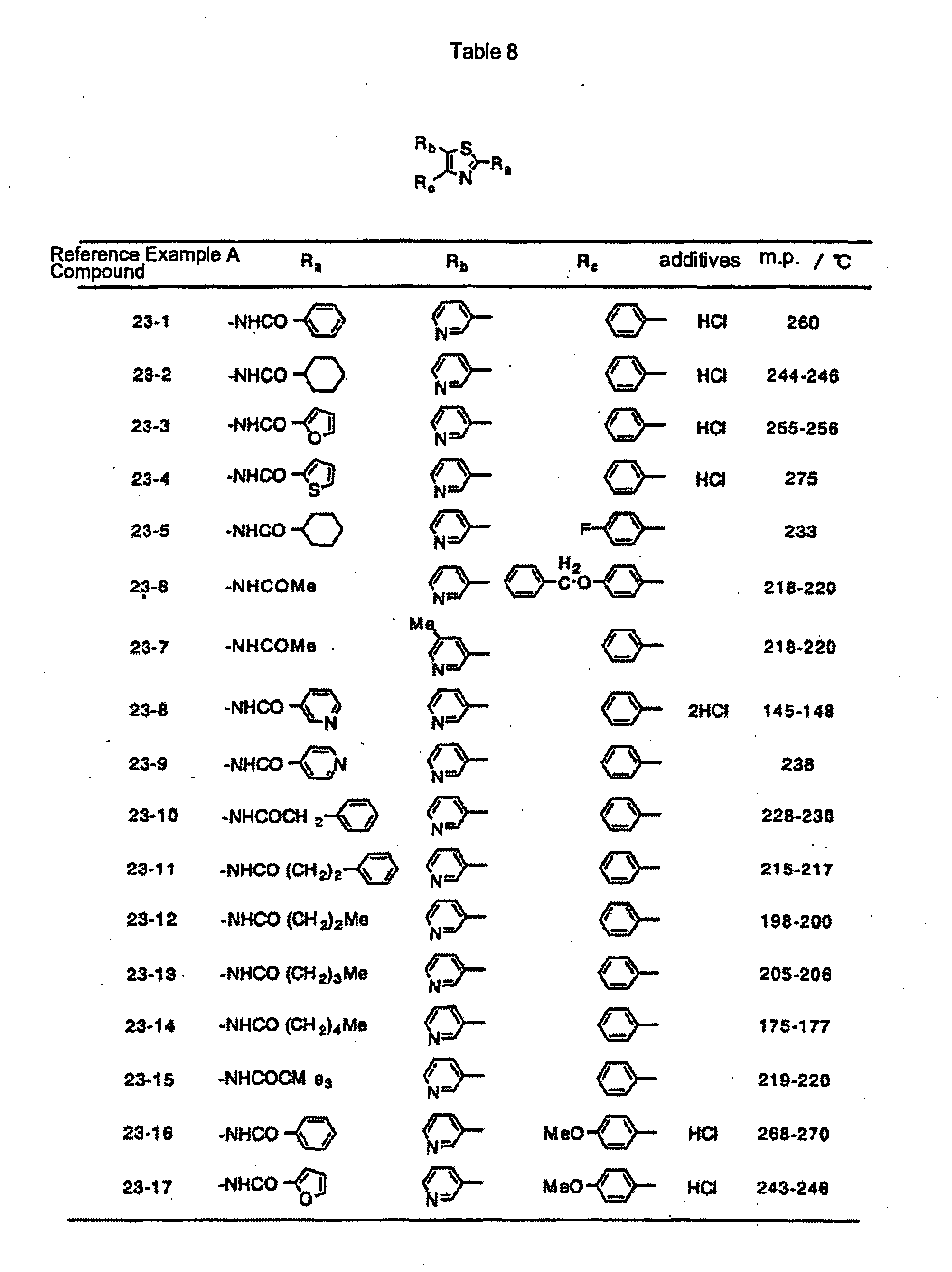

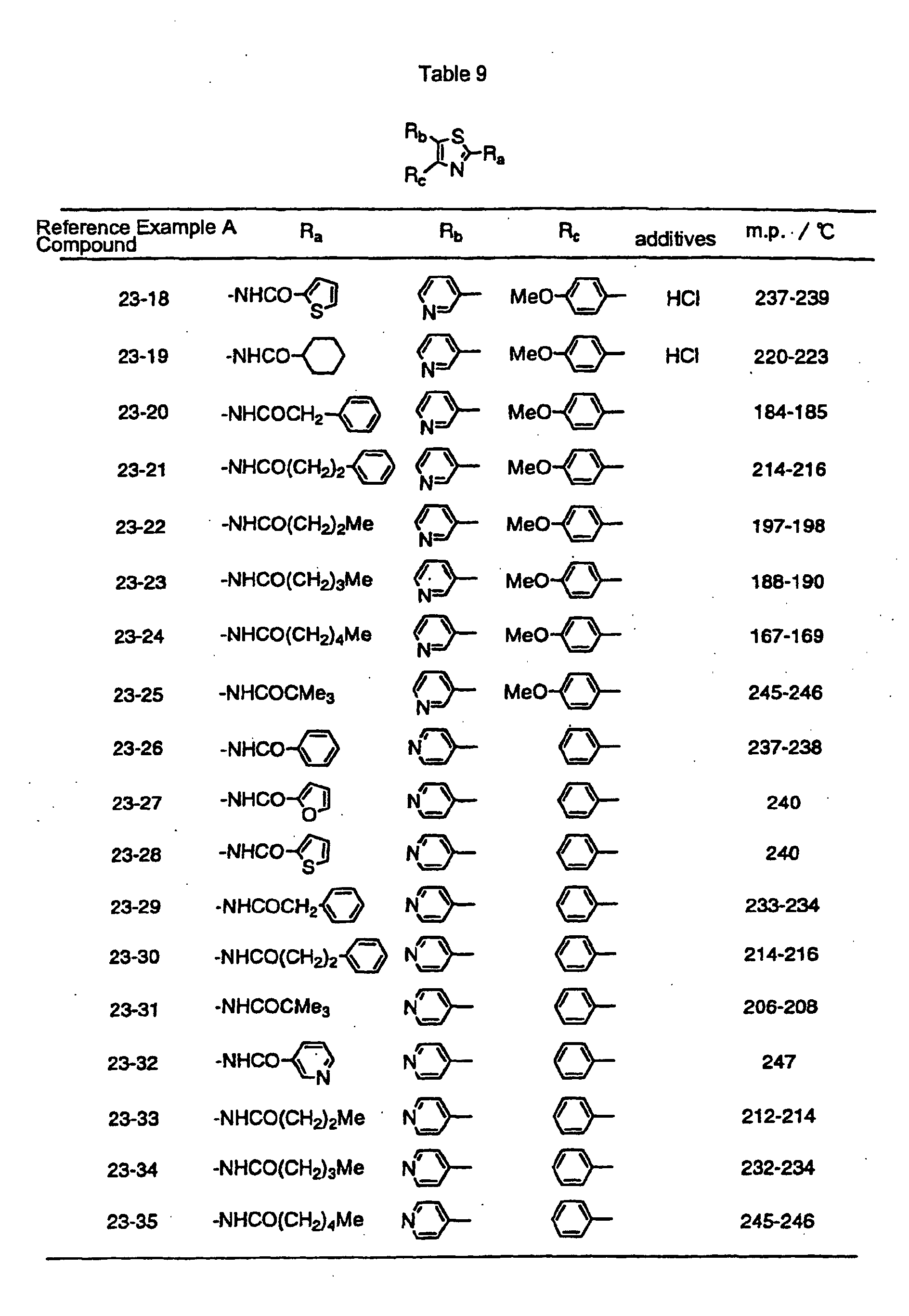

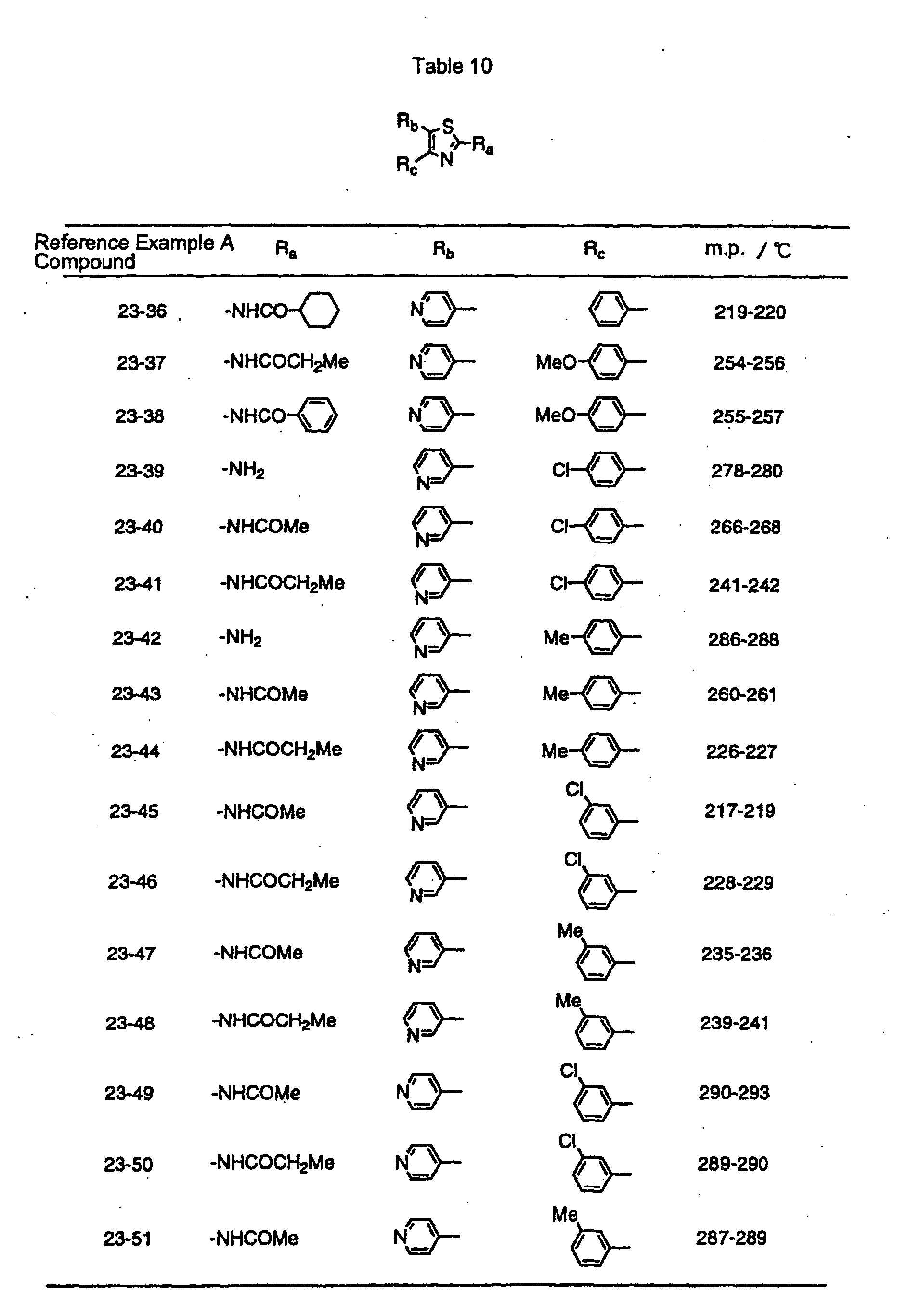

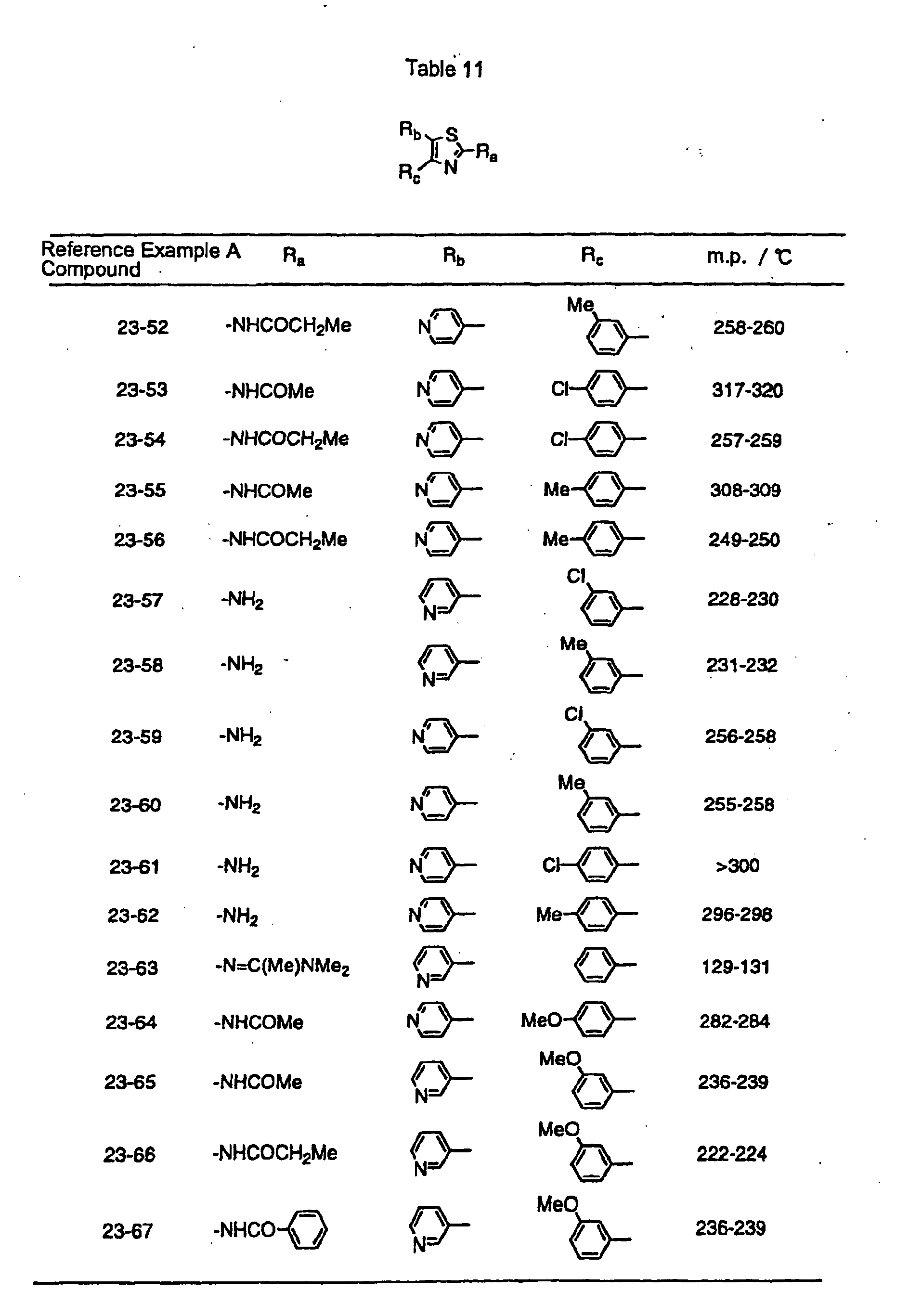

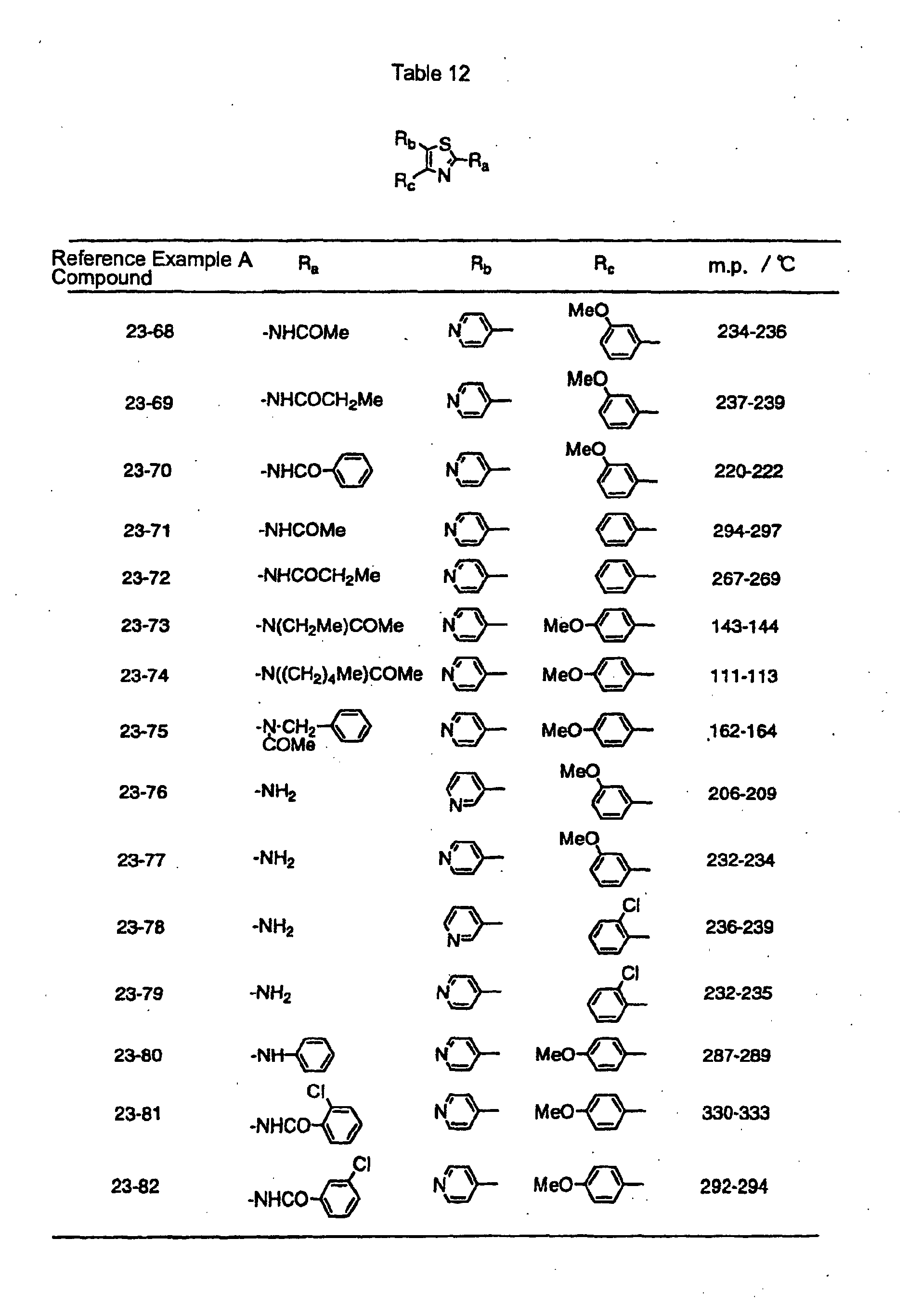

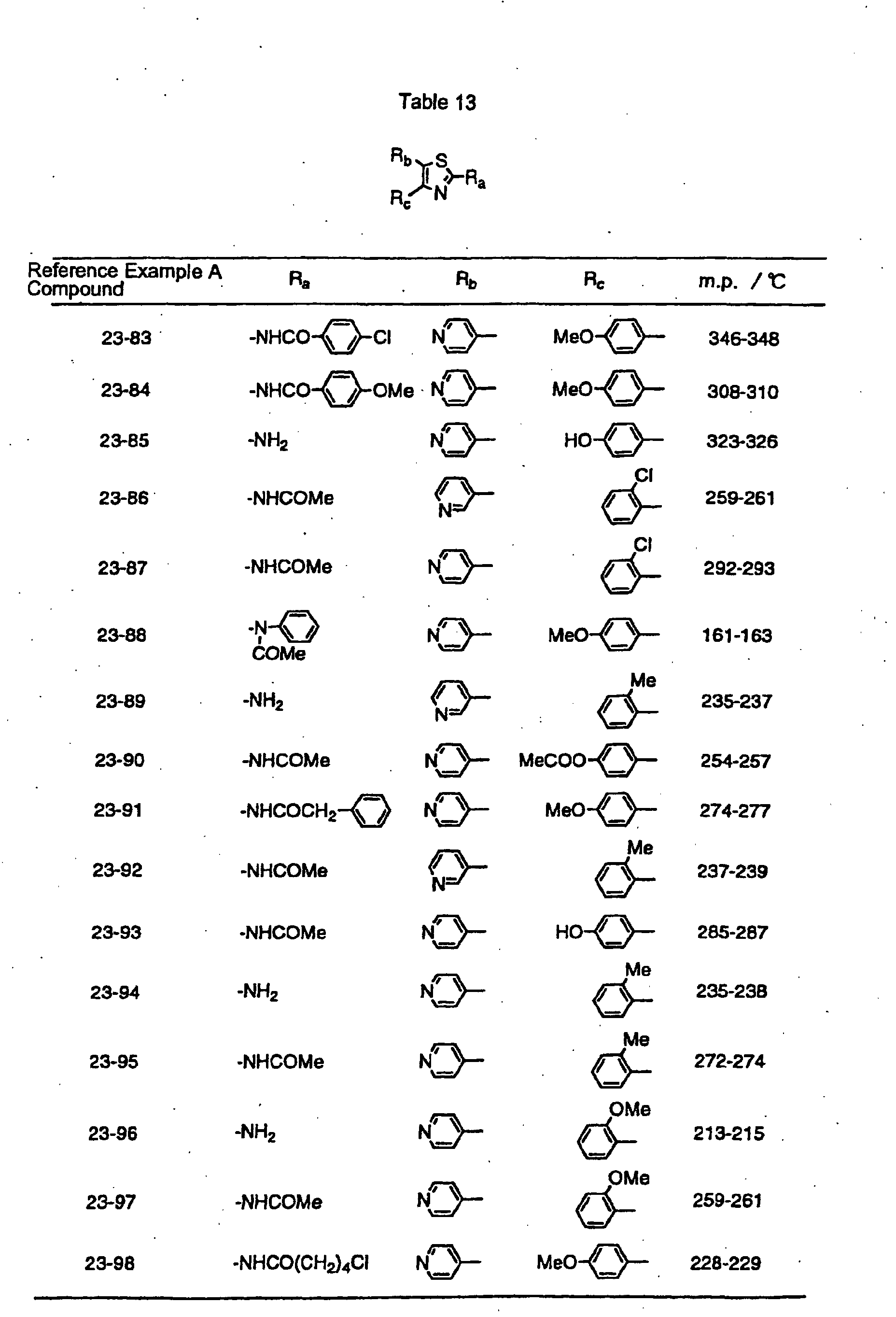

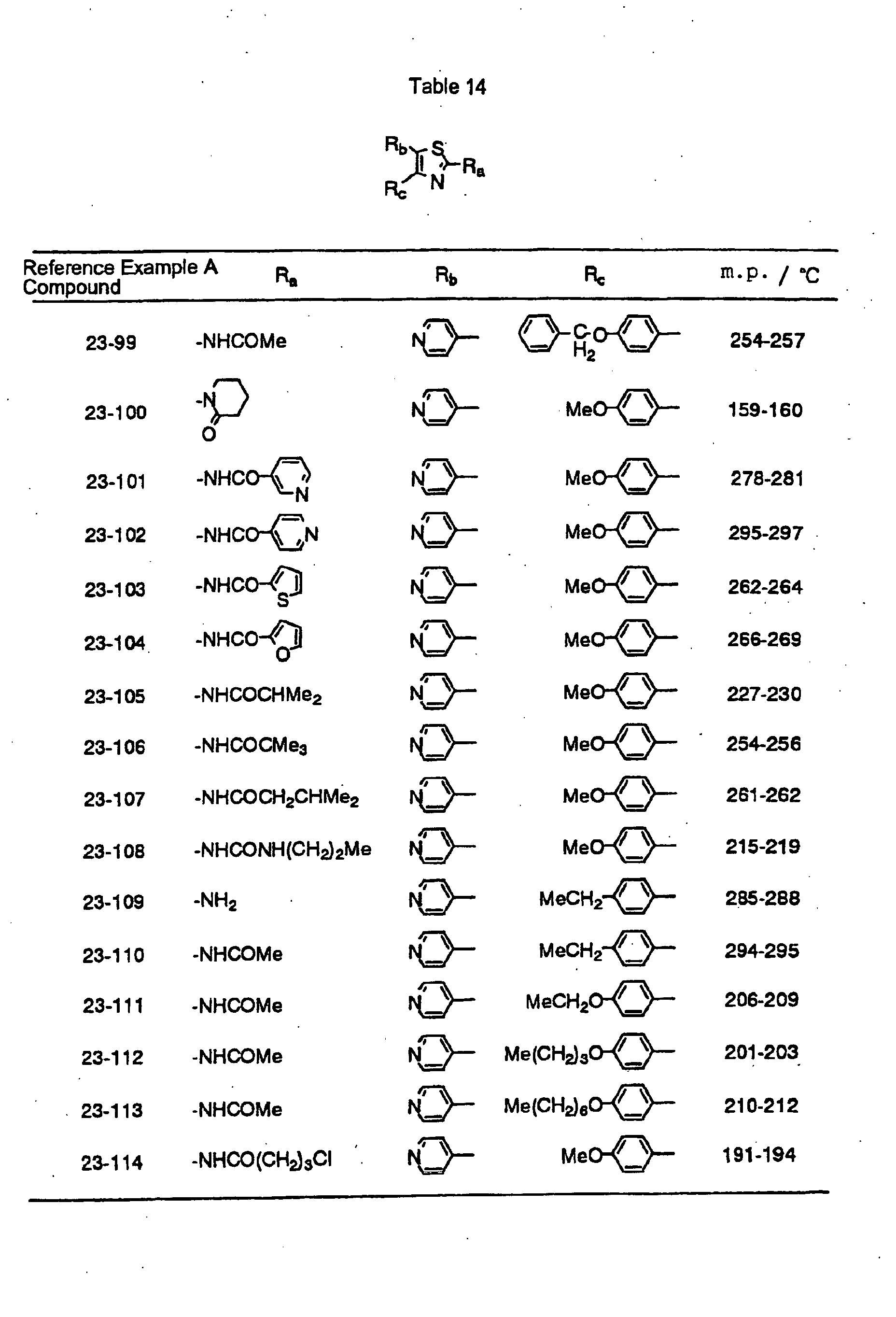

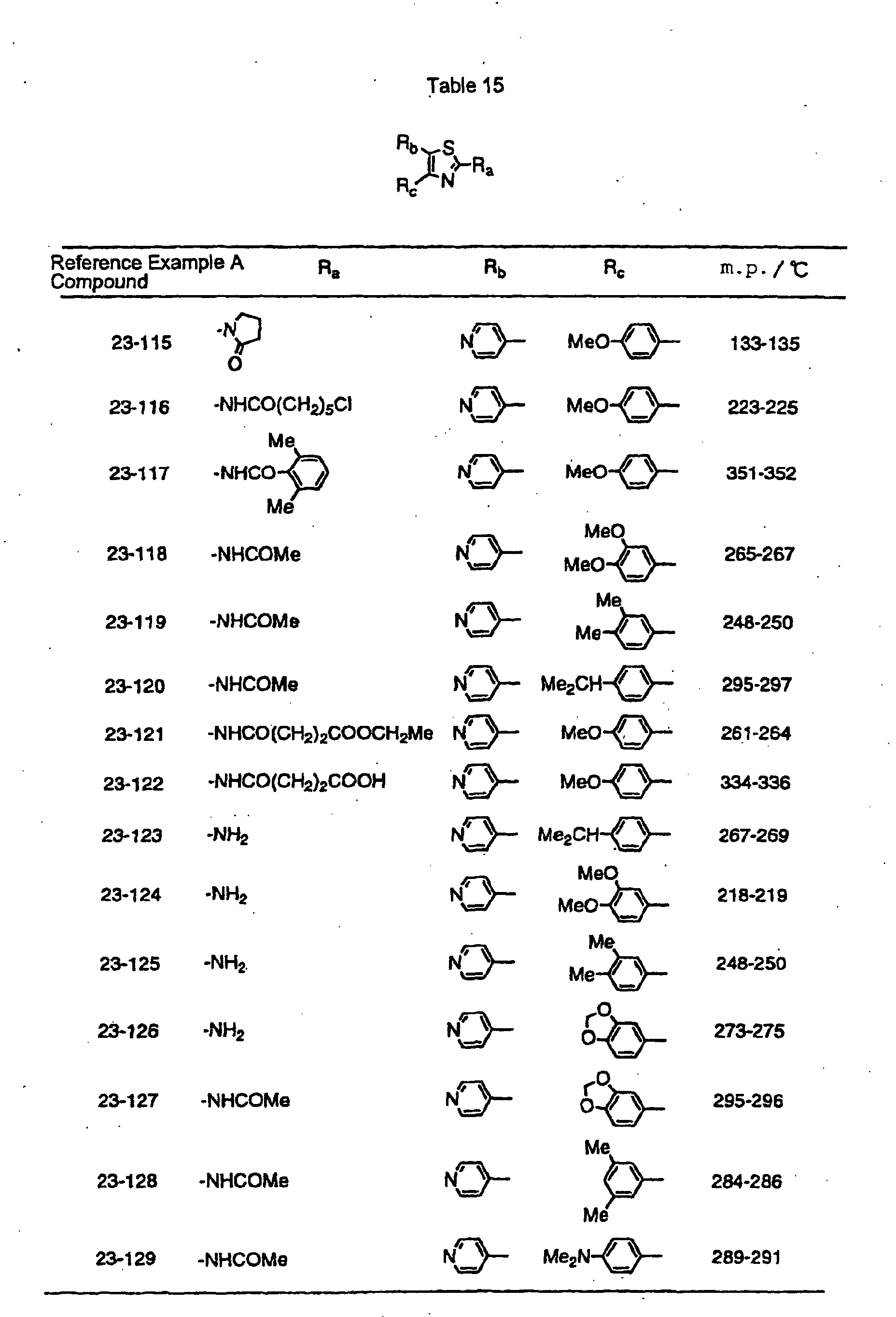

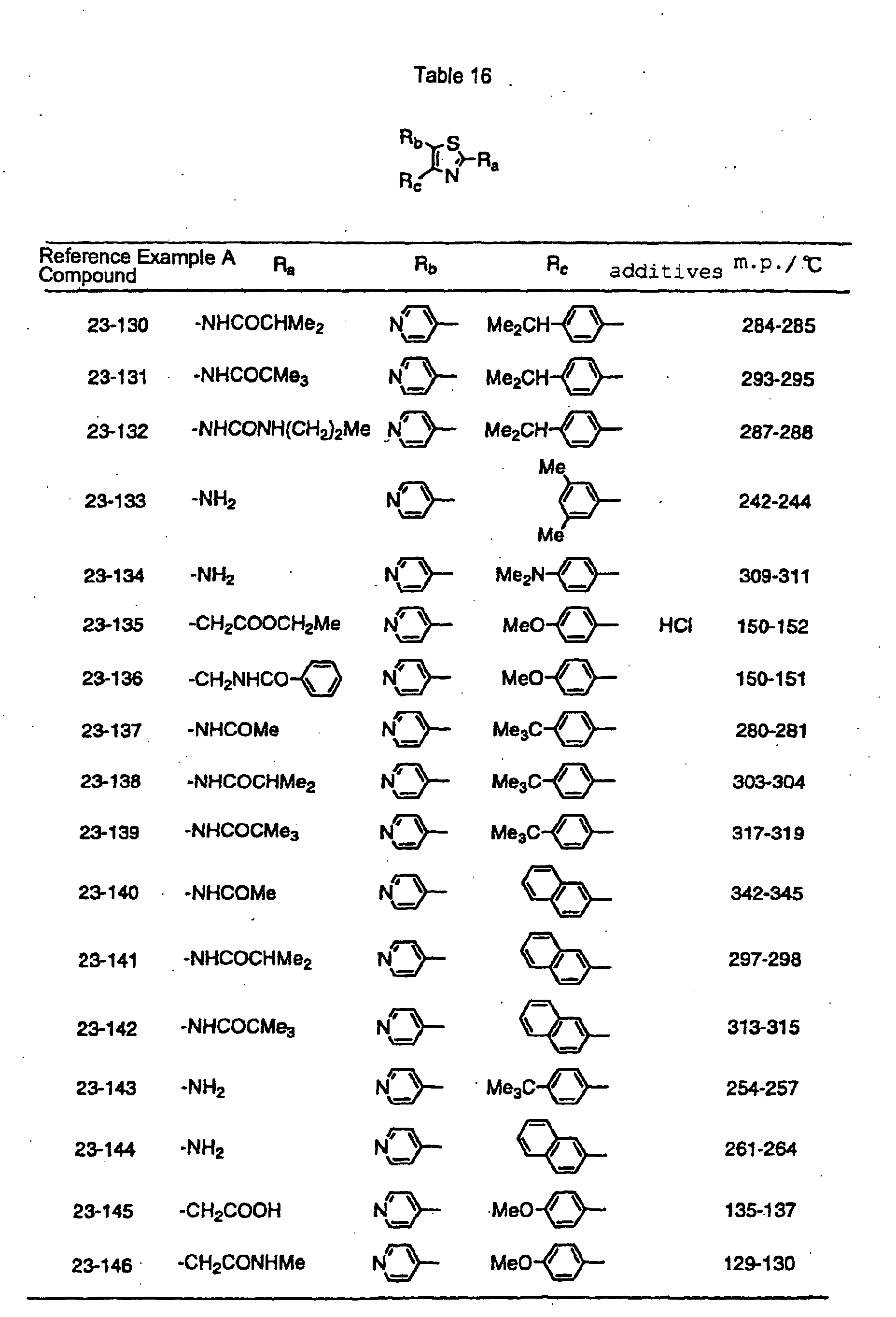

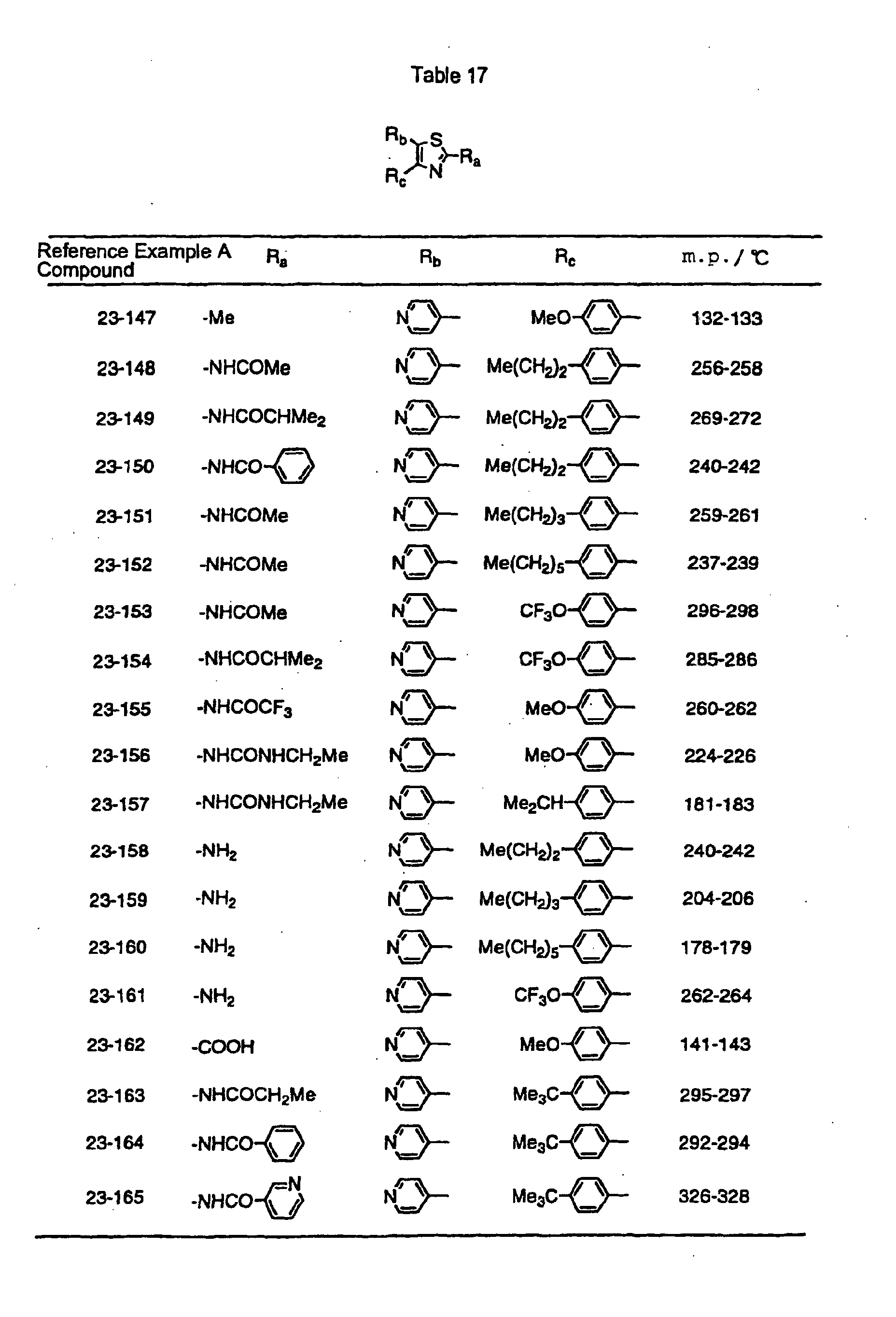

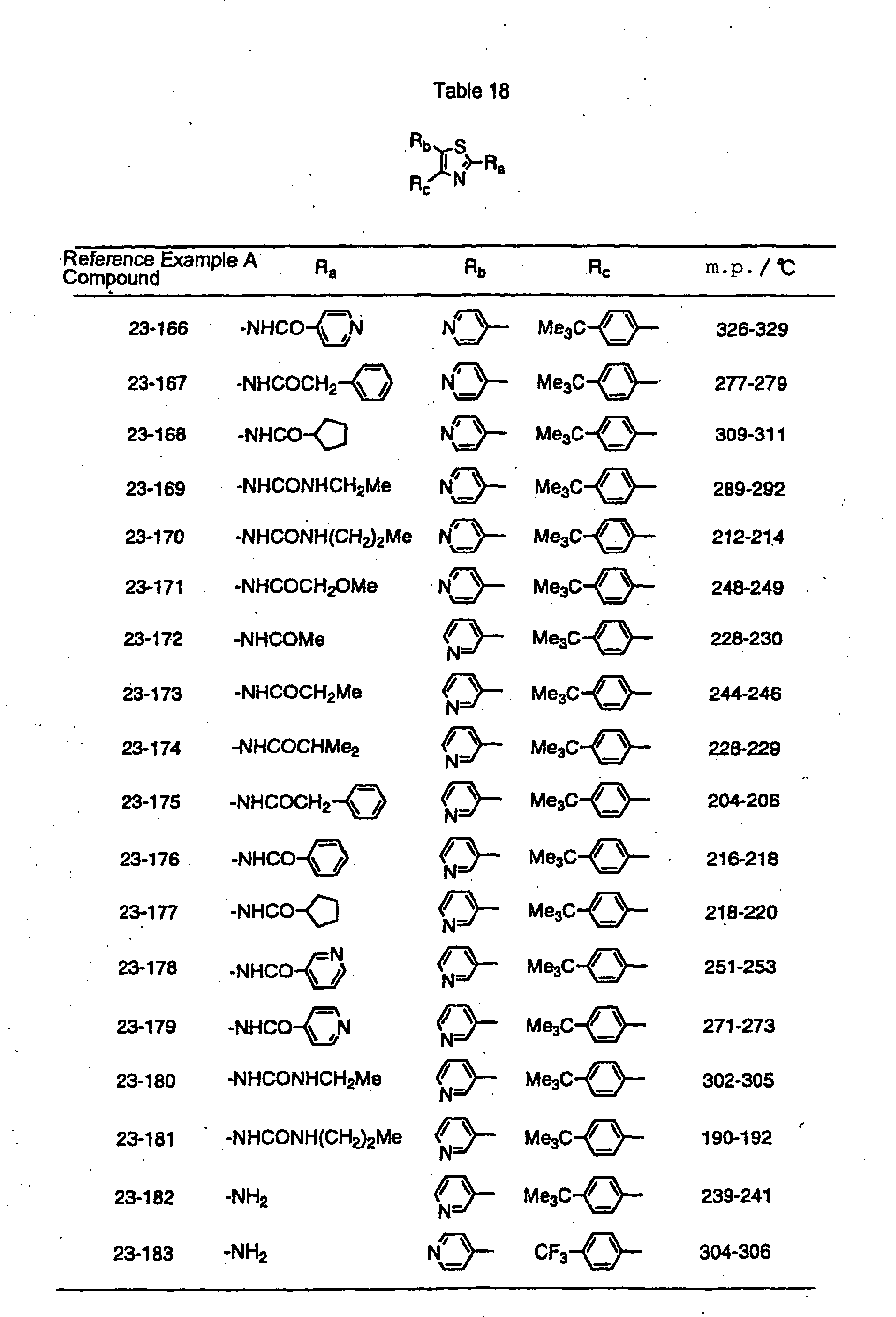

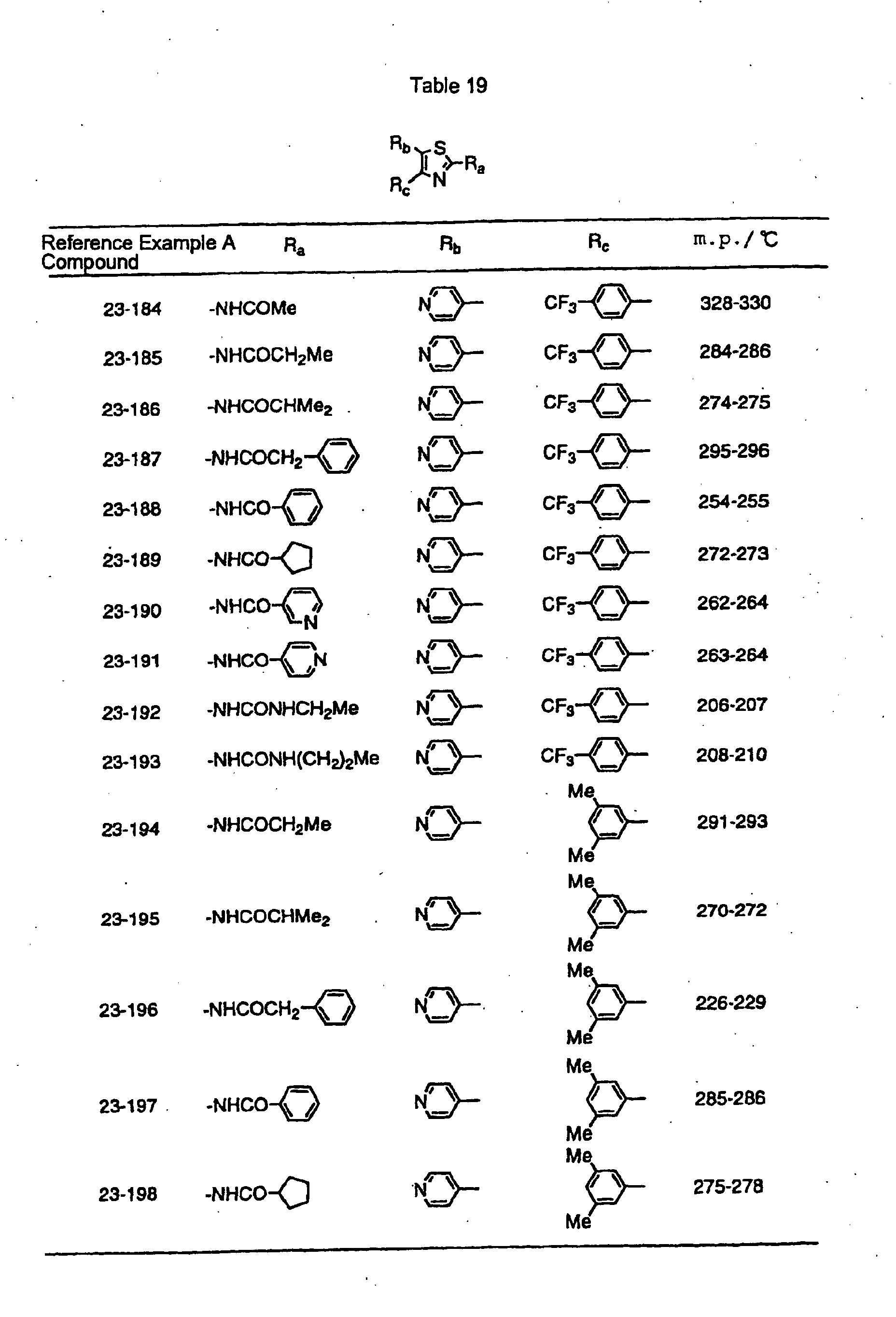

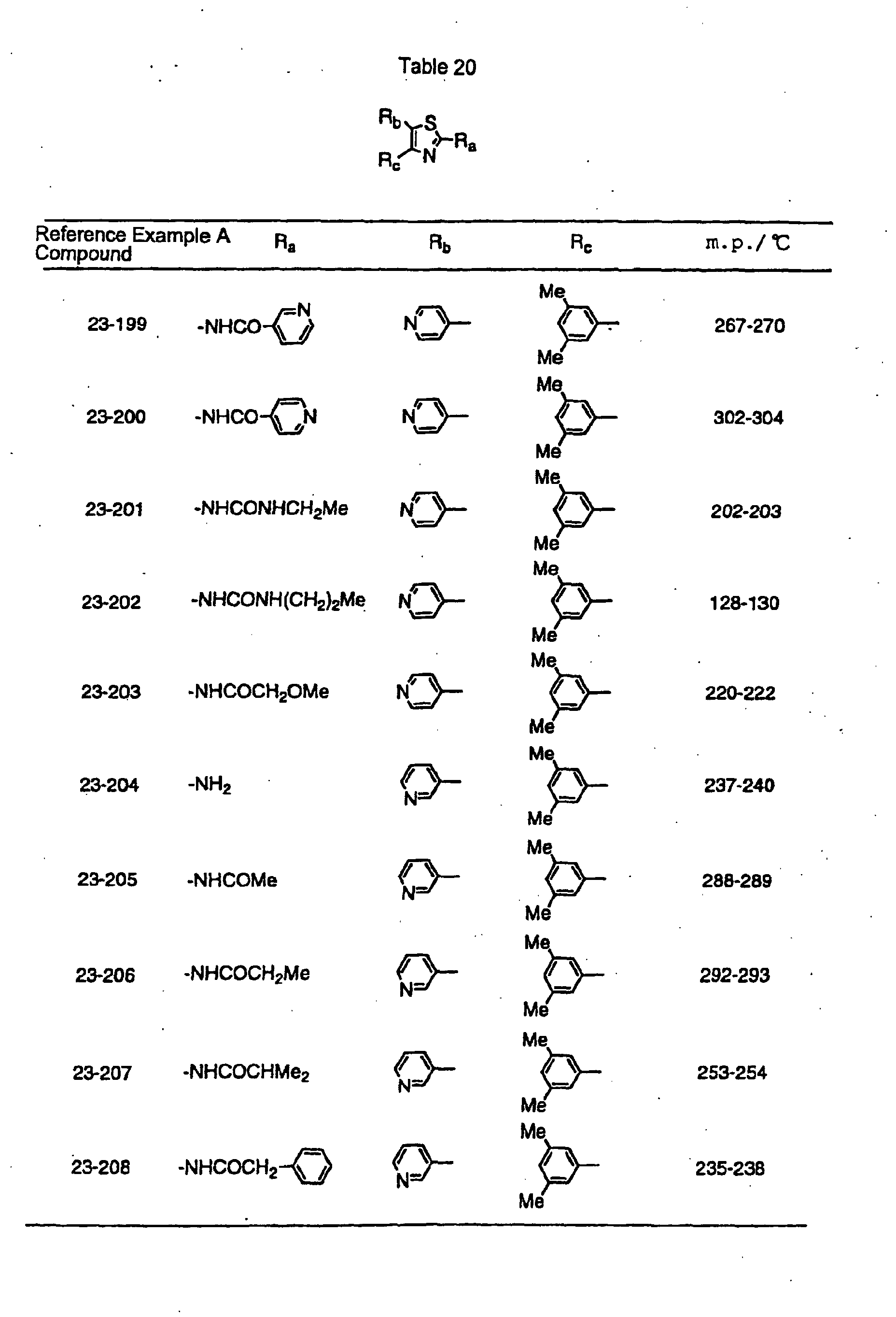

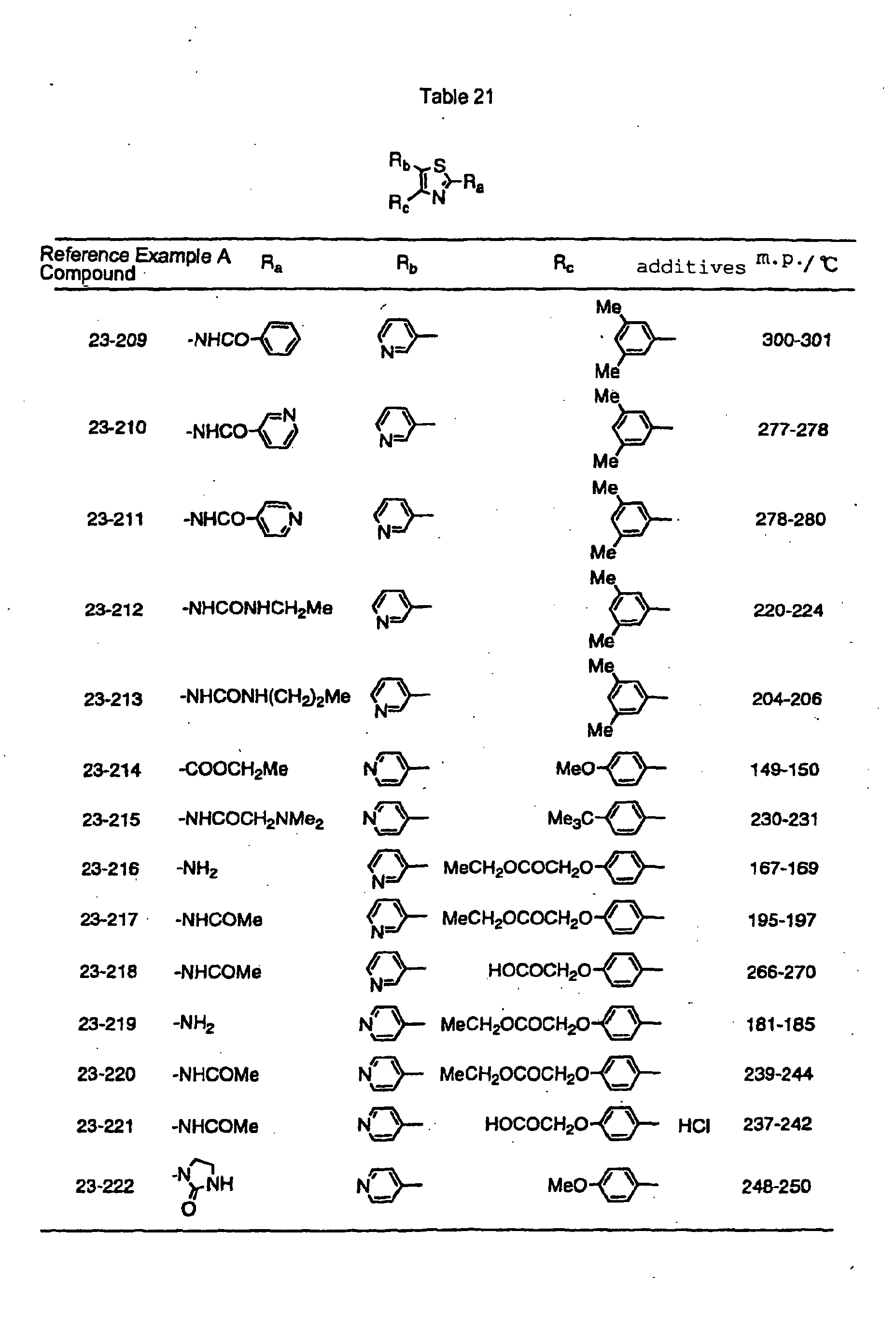

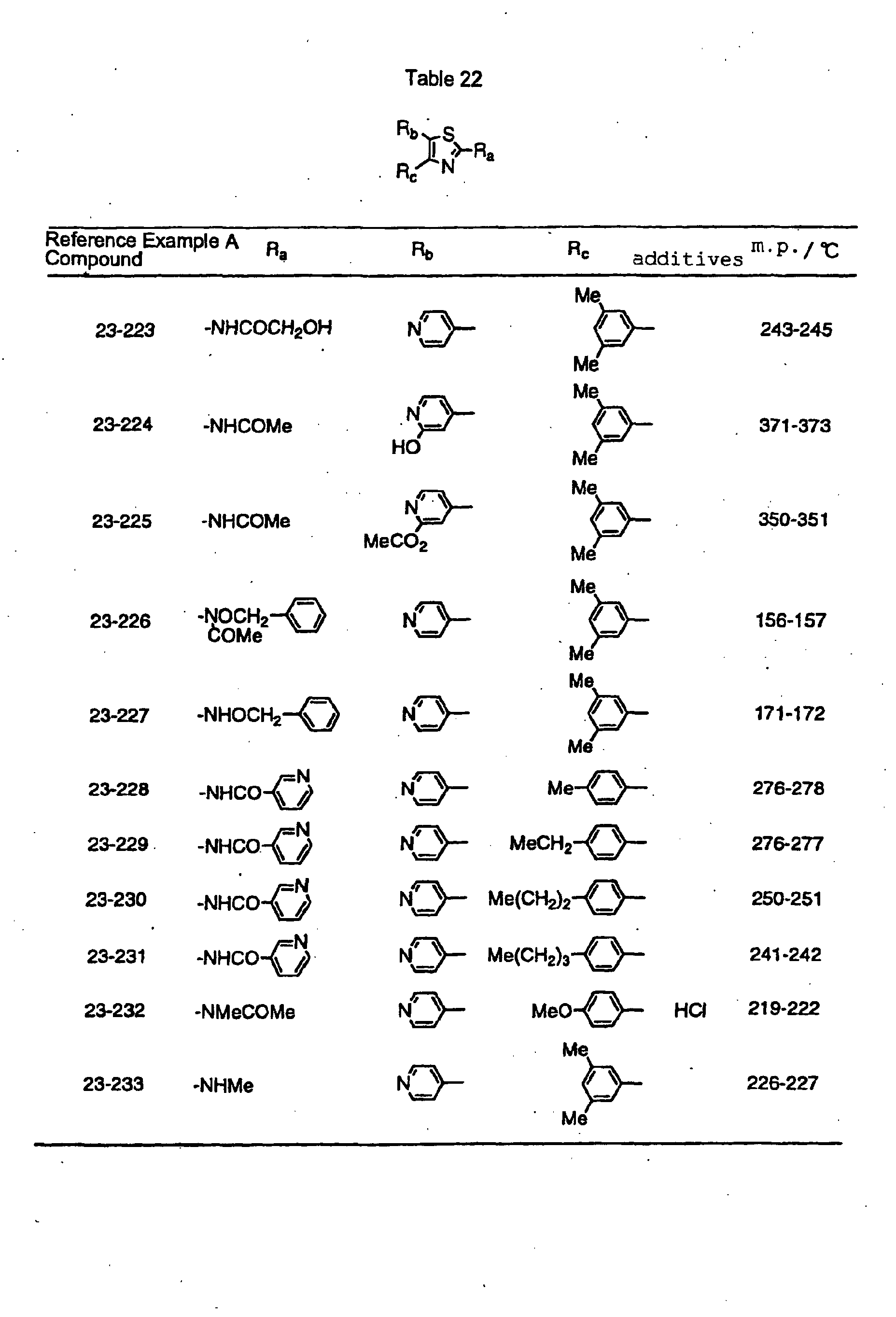

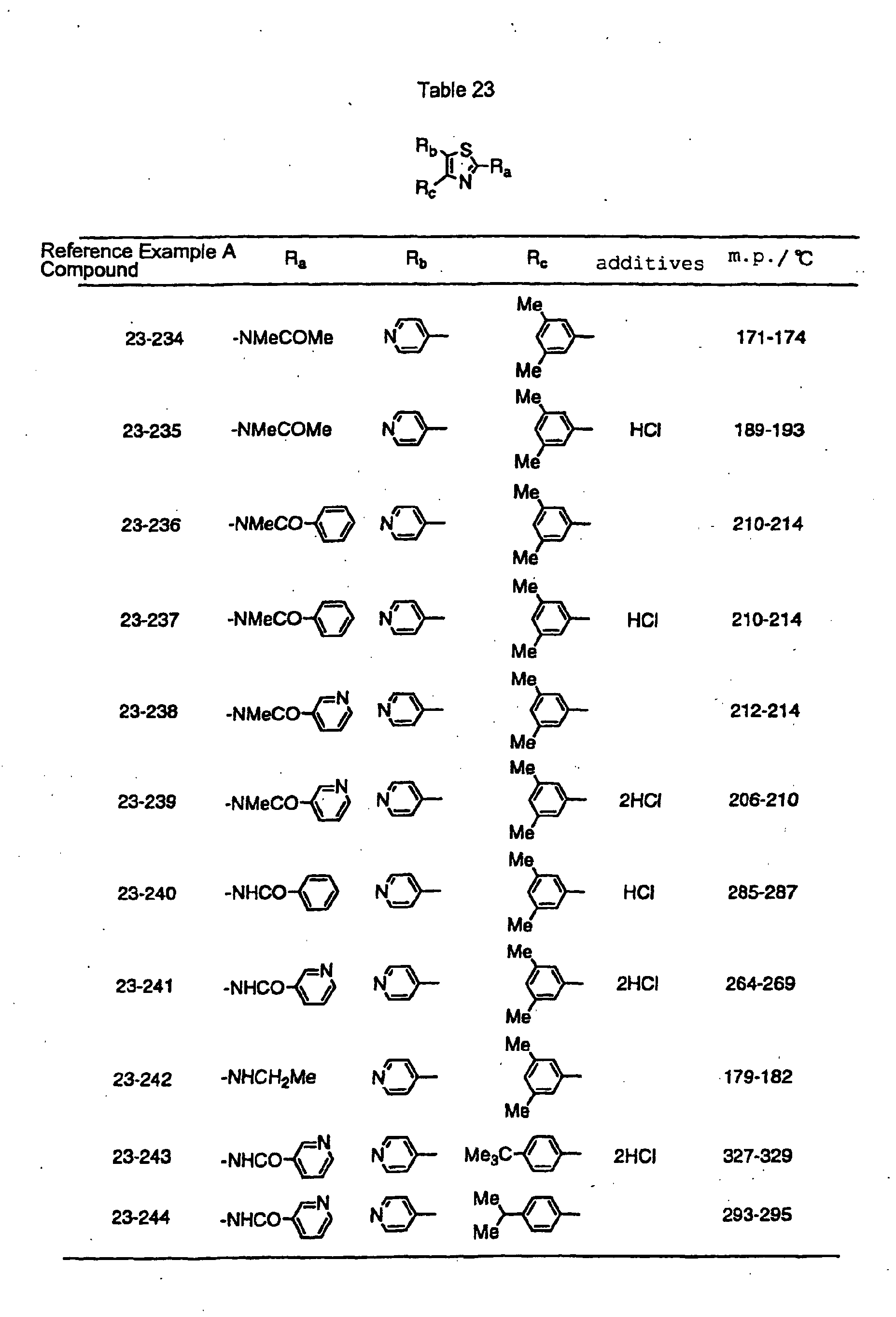

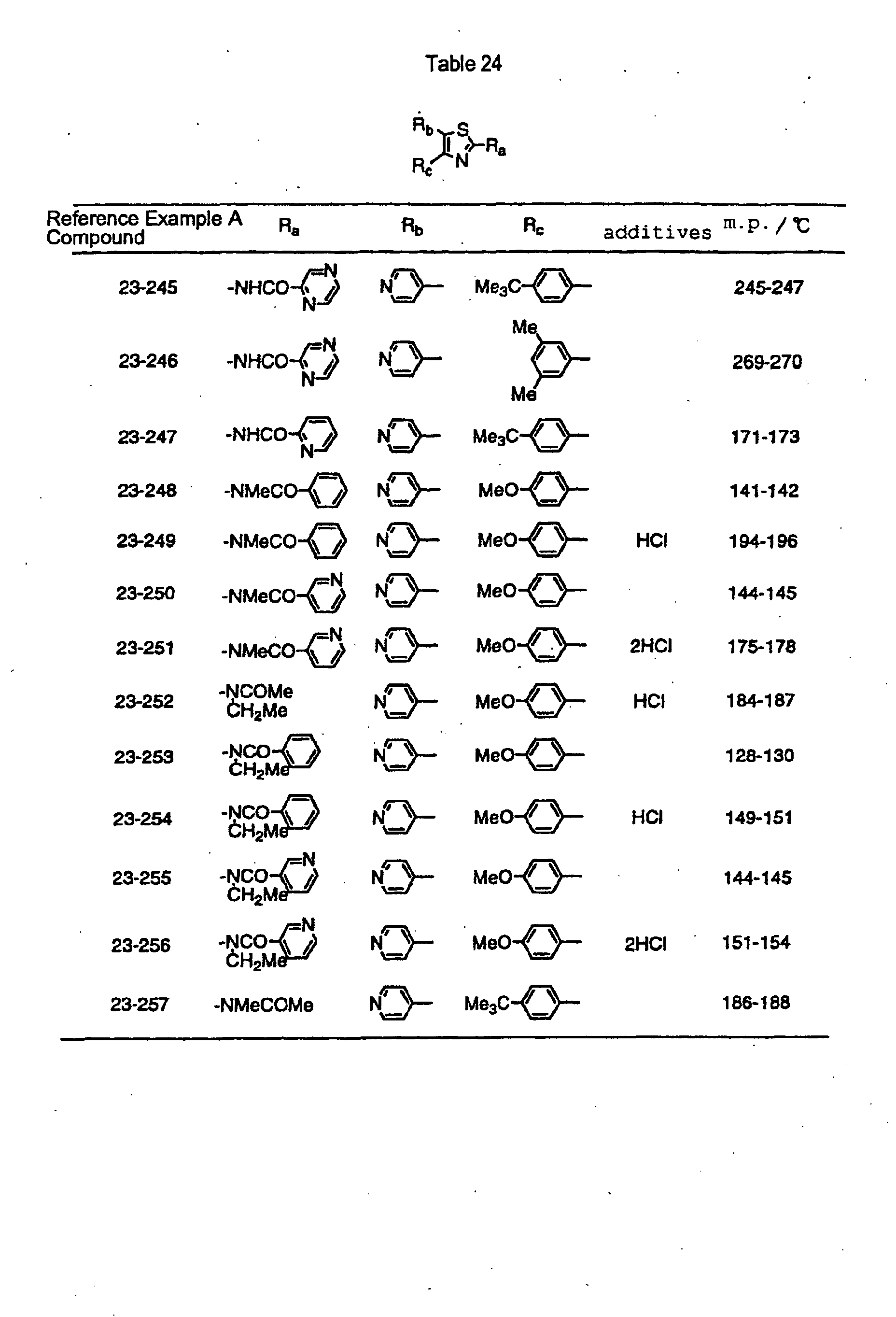

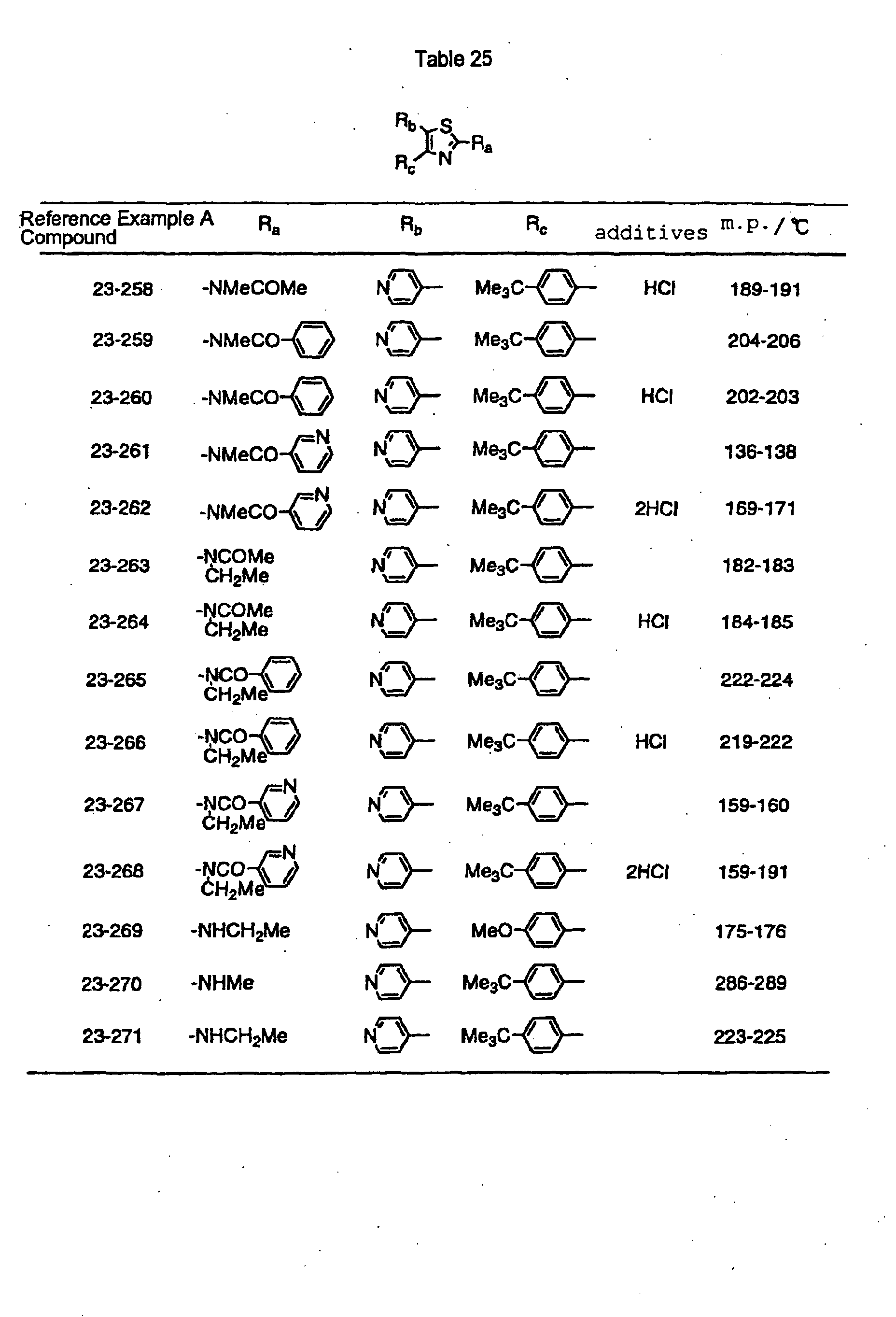

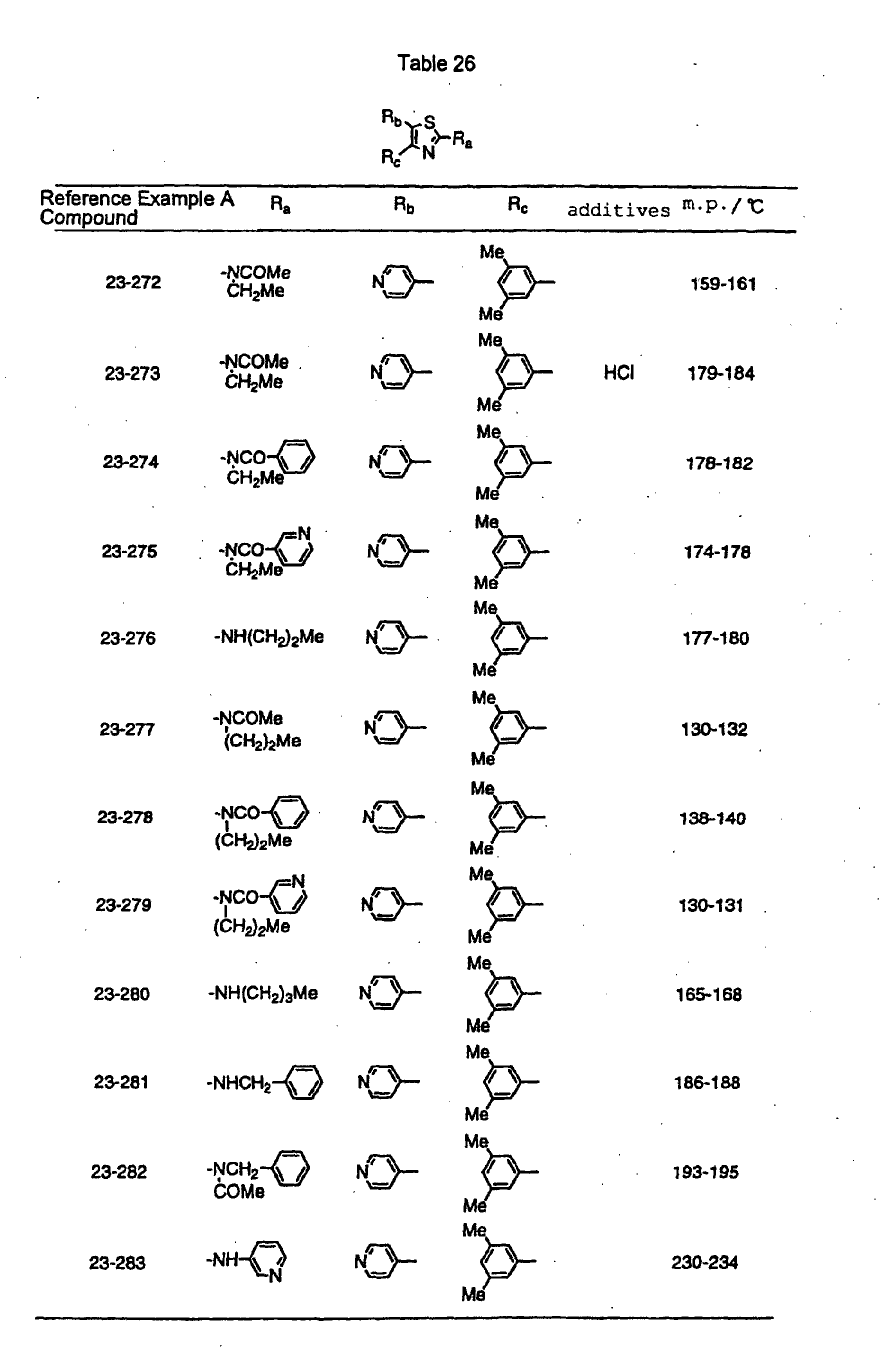

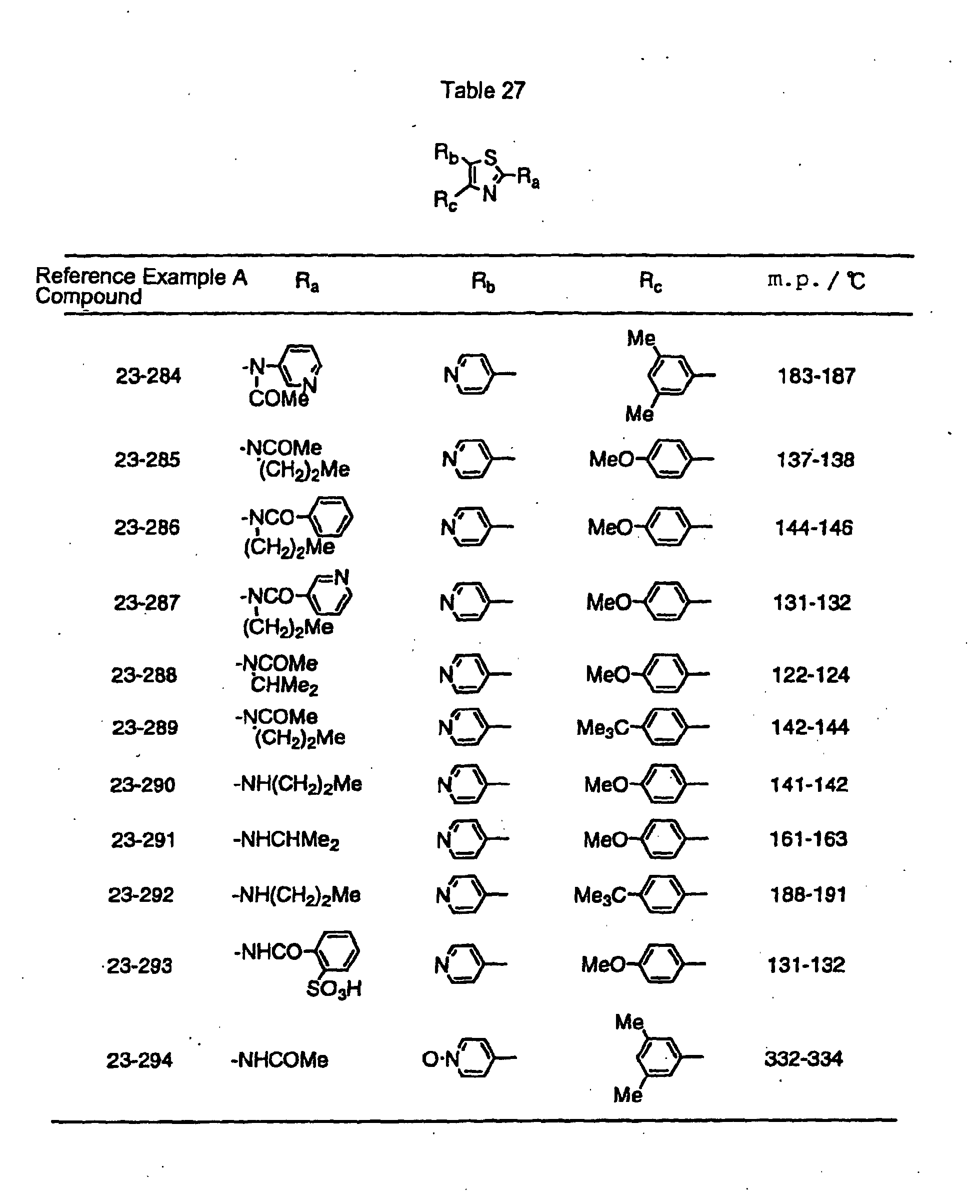

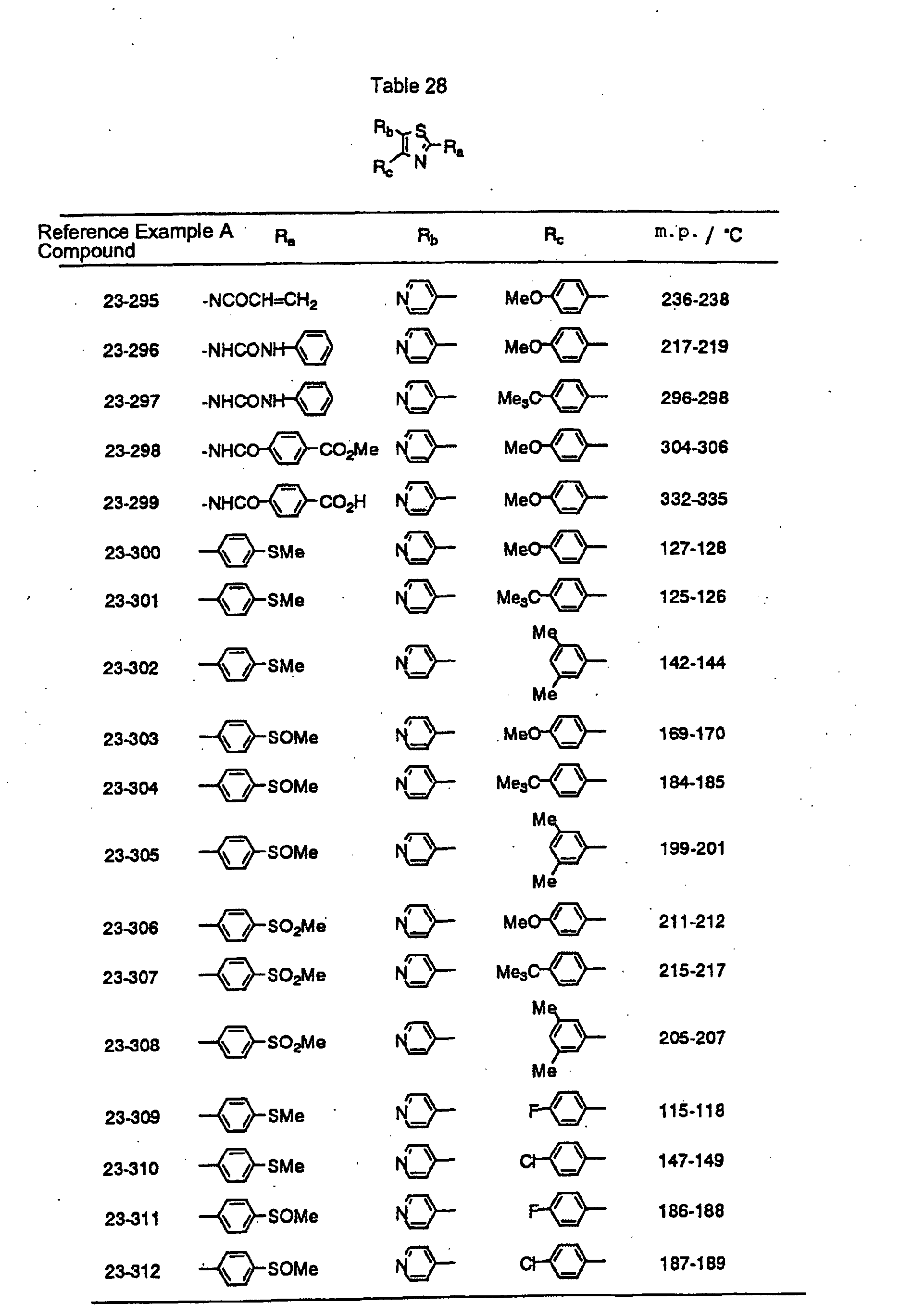

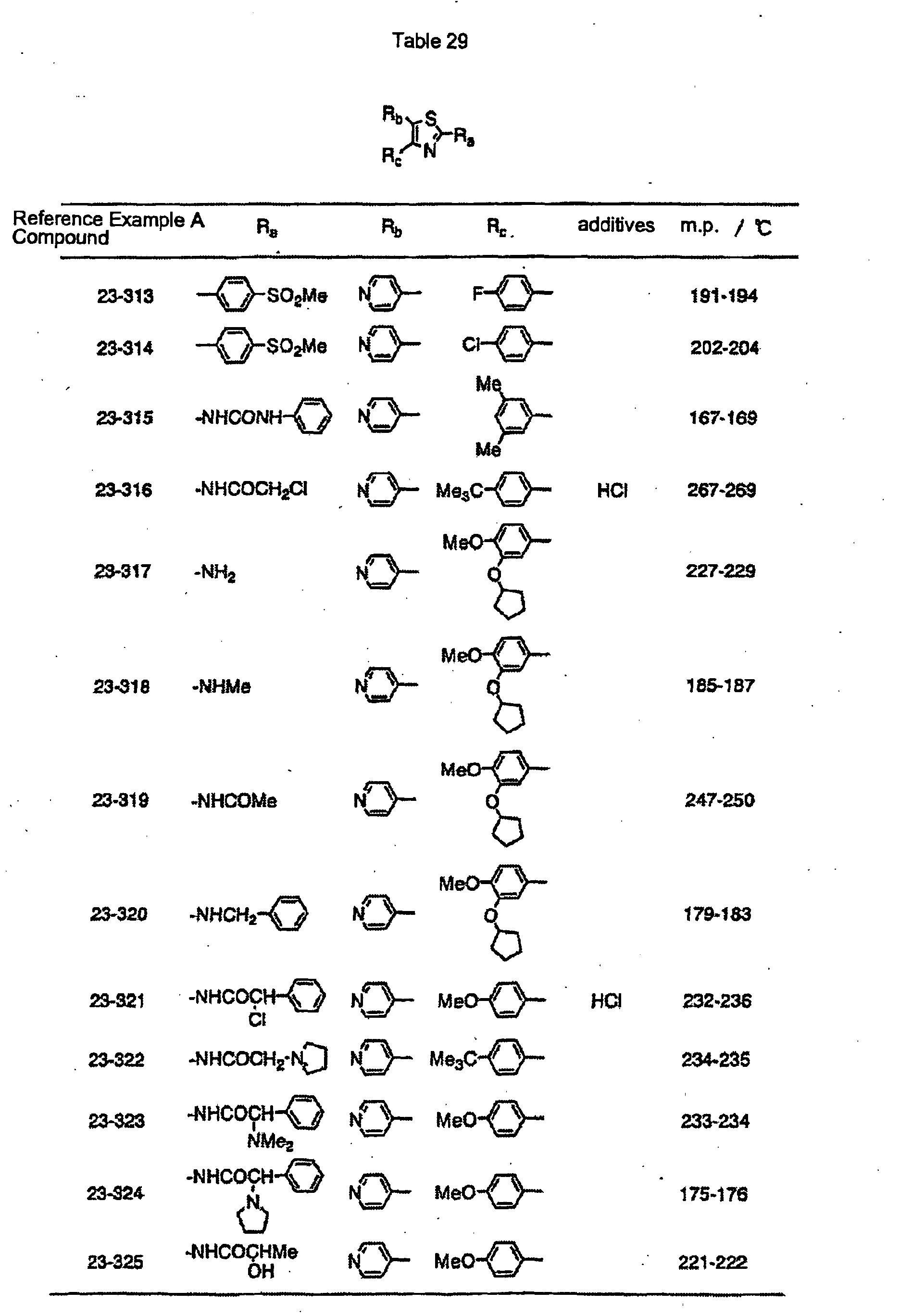

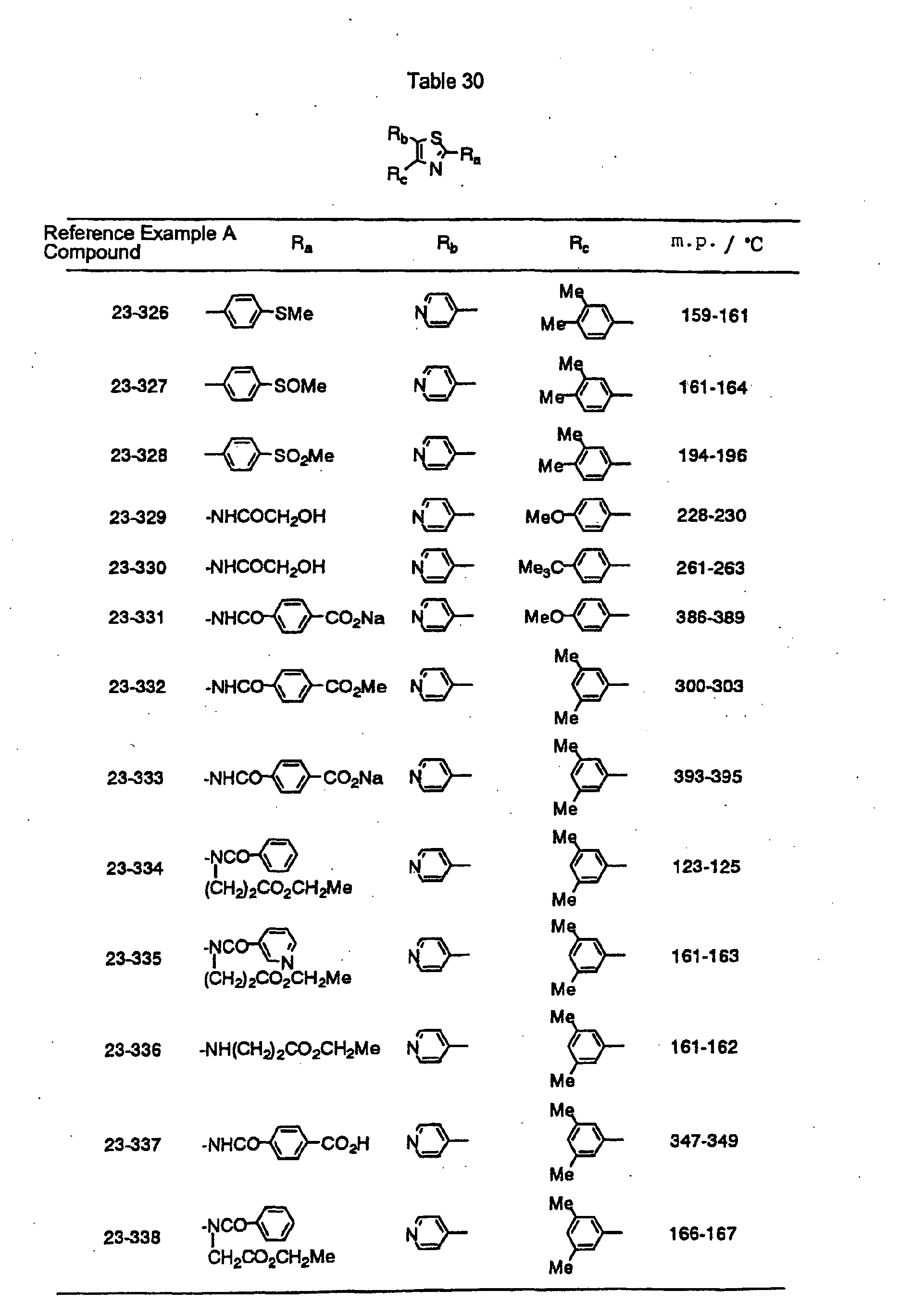

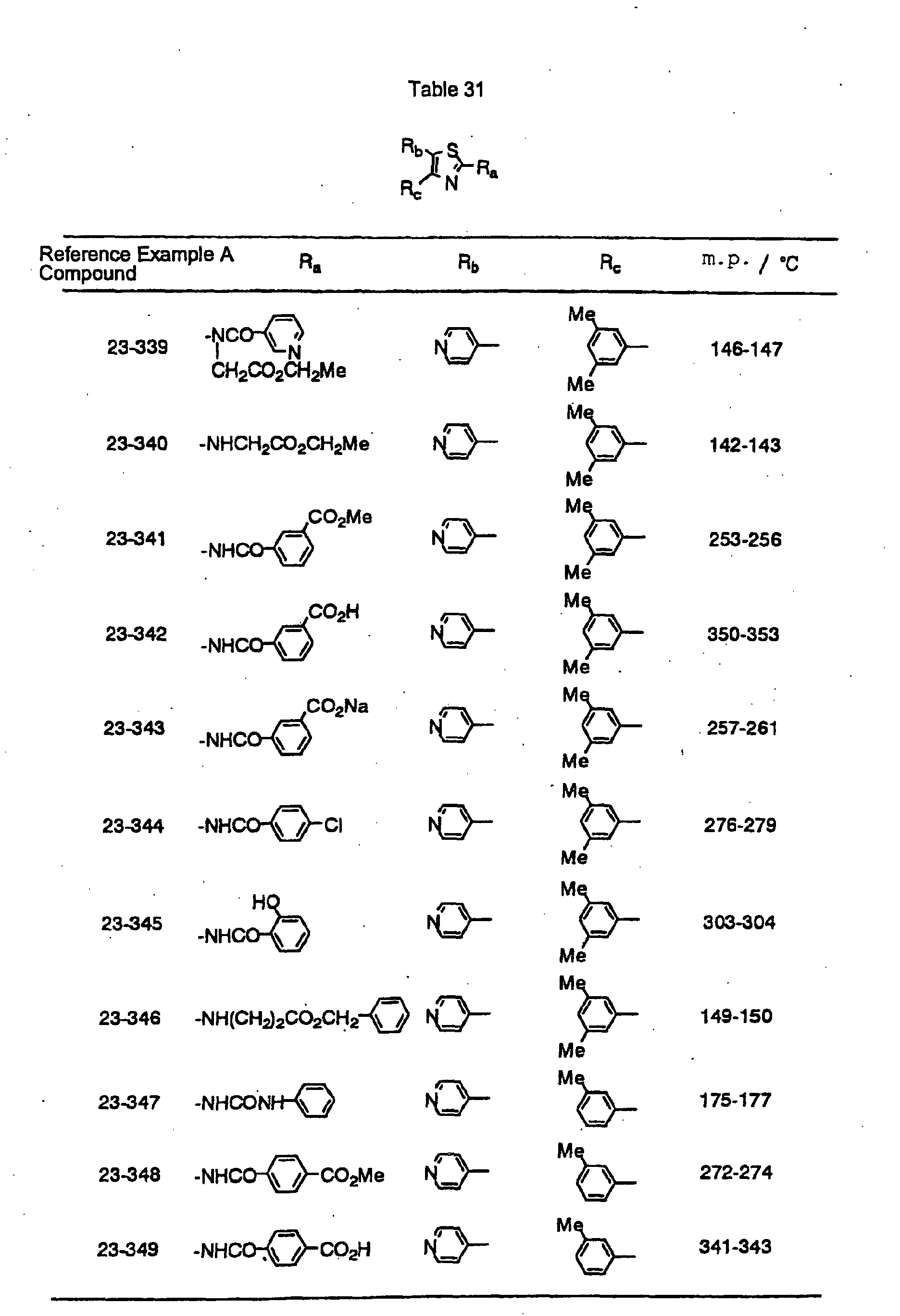

### Reference Example A 23-128

### N-[4-(3,5-dimethylphenyl)-5-(4-pyridyl)-1,3-thiazol-2-yl]acetamide

To a solution of [4-(3,5-dimethylphenyl)-5-(4-pyridyl)-1,3-thiazol-2-yl]amine (0.50 g, 1.78 mmol) and 4-dimethylaminopyridine (0.06 g, 0.51 mmol) in N,N-dimethylacetamide (5 mL) was added acetyl chloride (0.21 g, 2.67 mmol) and the mixture was stirred at 80°C for 14 h. To the reaction mixture was poured aqueous sodium hydrogencarbonate. The precipitated solid was collected by filtration. The obtained solid was washed with water and dried. The crude crystals were recrystallized from ethanol to give the title compound (0.17 g, yield 29%).
m.p.: 284-286°C.

### Reference Example A 23-133

### [4-(3,5-dimethylphenyl)-5-(4-pyridyl)-1,3-thiazol-2-yl]amine

To a solution of 2-bromo-1-(3,5-dimethylphenyl)-2-(4-pyridyl)ethanone hydrobromide (5.0 g, 13 mmol) and thiourea (1.0 g, 14 mmol) in acetonitrile (60 mL) was added dropwise triethylamine (1.9 ml, 14 mmol) and the mixture was stirred at room temperature for 3 h. The solvent was concentrated under reduced pressure and a saturated aqueous sodium hydrogencarbonate solution was added to the residue: The mixture was extracted with ethyl acetate. The organic layer was washed with water and the solvent was evaporated. The obtained crude crystals were recrystallized from ethyl acetate to give the title compound (2.0 g, 7.2 mmol, yield 55%).
m.p.: 242-244°C.

### Reference Example A 23-137

### N-[4-[4-(1,1-dimethylethyl)phenyl]-5-(4-pyridyl)-1,3-thiazol-2-yl]acetamide

To a solution of [4-[4-(1,1-dimethylethyl)phenyl]-5-(4-pyridyl)-1,3-thiazol-2-yl]amine (0.40 g, 1.29 mmol) and 4-dimethylaminopyridine (0.05 g, 0.39 mmol) in N,N-dimethylacetamide (4 mL) was added acetyl chloride (0.15 g, 1.94 mmol) and the mixture was stirred at 80°C for 14 h. To the reaction mixture was poured aqueous sodium hydrogencarbonate and the precipitated solid was collected by filtration. The obtained solid was washed with water and dried. Crude crystals were recrystallized from ethanol to give the title compound (0.23 g, yield 50%).
m.p.: 280-281°C.

### Reference Example A 23-143

### [4-[4-(1,1-dimethylethyl)phenyl]-5-(4-pyridyl)-1,3-thiazol-2-yl]amine

To a solution of 2-bromo-1-[4-(1,1-dimethylethyl)-phenyl]-2-(4-pyridyl)ethanone hydrobromide (5.0 g, 12 mmol) and thiourea (0.95 g, 13 mmol) in acetonitrile (60 mL) was added dropwise triethylamine (1.8 ml, 13 mmol) and the mixture was refluxed for 3 h. The solvent was evaporated under reduced pressure and saturated aqueous sodium hydrogencarbonate solution was added to the residue. The precipitated solid was collected by filtration. The obtained crude crystal was recrystallized from ethanol to give the title compound (2.6 g, 8.4 mmol, yield 69%).
m.p.: 254-257°C.

### Reference Example A 23-164

### N-[4-[4-(1,1-dimethylethyl)phenyl]-5-(4-pyridyl)-1,3-thiazol-2-yl]benzamide

To a solution of [4-[4-(1,1-Dimethylethyl)phenyl]-5-(4-pyridyl)-1,3-thiazol-2-yl]amine (0.50 g, 1.62 mmol) and 4-dimethylaminopyridine (0.05 g, 0.39 mmol) in N,N-dimethylacetamide (5 mL) was added benzoyl chloride (0.15 g, 1.94 mmol), and the mixture was stirred at 80°C for 14 h. To the reaction mixture was poured an aqueous sodium hydrogencarbonate and the precipitated solid was collected by filtration. The obtained solid was washed with water and dried. The crude crystals were recrystallized from ethanol to give the title compound (0.44 g, yield 66%).
m.p.: 292-294°C.

### Reference Example A 23-165

### N-[4-[4-(1,1-dimethylethyl)phenyl]-5-(4-pyridyl)-1,3-thiazol-2-yl]nicotinamide

To a solution of [4-[4-(1,1-dimethylethyl)phenyl]-5-(4-pyridyl)-1,3-thiazol-2-yl]amine (0.50 g, 1.62 mmol) and 4-dimethylaminopyridine (0.06 g, 0.49 mmol) in N,N-dimethylacetamide (5 mL) was added nicotinoyl chloride hydrochloride (0.43 g, 2.42 mmol) and the mixture was stirred at 70°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.49 g, yield 73%).
m.p.: 326-328°C.

### Reference Example A 23-168

### N-[4-[4-(1,1-dimethylethyl)phenyl]-5-(4-pyridyl)-1,3-thiazol-2-yl]cyclopentanecarboxamide

To a solution of [4-[4-(1,1-dimethylethyl)phenyl]-5-(4-pyridyl)-1,3-thiazol-2-yl]amine (0.50 g, 1.62 mmol) and 4-dimethylaminopyridine (0.06 g, 0.49 mmol) in N,N-dimethylacetamide (5 mL) was added cyclopentanecarbonyl chloride (0.32 g, 2.42 mmol) and the mixture was stirred at 70°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.43 g, yield 66%).
m.p.: 309-311°C.

### Reference Example A 23-194

### N-[4-(3,5-dimethylphenyl)-5-(4-pyridyl)-1,3-thiazol-2-yl]propionamide

To a solution of [4-(3,5-dimethylphenyl)-5-(4-pyridyl)-1,3-thiazol-2-yl]amine (0.51 g, 1.8 mmol) and 4-dimethylaminopyridine (0.06 g, 0.52 mmol) in N,N-dimethylacetamide (20 mL) was added propionyl chloride (0.18 g, 1.96 mmol) and the mixture was stirred at 80°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.41 g, yield 67%).
m.p.: 291-293°C.

### Reference Example A 23-195

### N-[4-(3,5-dimethylphenyl)-5-(4-pyridyl)-1,3-thiazol-2-yl]-2-methylpropionamide

To a solution of [4-(3,5-dimethylphenyl)-5-(4-pyridyl)-1,3-thiazol-2-yl]amine (0.50 g, 1.8 mmol) and 4-dimethylaminopyridine (0.06 g, 0.53 mmol) in N,N-dimethylacetamide (20 mL) was added 2-methylpropionyl chloride (0.20 g, 1.91 mmol) and the mixture was stirred at 80°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.52 g, yield 83%).
m.p.: 270-272°C.

### Reference Example A 23-196

### N-[4-(3,5-dimethylphenyl)-5-(4-pyridyl)-1,3-thiazol-2-yl]-2-phenylacetamide

To a solution of [4-(3,5-dimethylphenyl)-5-(4-pyridyl)-1,3-thiazol-2-yl]amine (0.51 g, 1.8 mmol) and 4-dimethylaminopyridine (0.06 g, 0.52 mmol) in N,N-dimethylacetamide (15 mL) was added 2-phenylacetyl chloride (0.32 g, 2.0 mmol) and the mixture was stirred at 80°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.33 g, yield 46%).
m.p.: 226-229°C.

### Reference Example A 23-197

### N-[4-(3,5-dimethylphenyl)-5-(4-pyridyl)-1,3-thiazol-2-yl]benzamide

To a solution of [4-(3,5-dimethylphenyl)-5-(4-pyridyl)-1,3-thiazol-2-yl]amine (0.51 g, 1.8 mmol) and 4-dimethylaminopyridine (0.06 g, 0.52 mmol) in N,N-dimethylacetamide (20 mL) was added benzoyl chloride (0.30 g, 2.15 mmol) and the mixture was stirred at 80°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.18 g, yield 26%).
m.p.: 285-286°C.

### Reference Example A 23-198

### N-[4-(3,5-dimethylphenyl)-5-(4-pyridyl)-1,3-thiazol-2-yl]cyclopentanecarboxamide

To a solution of [4-(3,5-dimethylphenyl)-5-(4-pyridyl)-1,3-thiazol-2-yl]amine (0.51 g, 1.8 mmol) and 4-dimethylaminopyridine (0.07 g, 0.56 mmol) in N,N-dimethylacetamide (10 mL) was added cyclopentanecarbonyl chloride (0.33 g, 2.47 mmol) and the mixture was stirred at 70°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.41 g, yield 59%).
m.p.: 275-278°C.

### Reference Example A 23-199

### N-[4-(3,5-dimethylphenyl)-5-(4-pyridyl)-1,3-thiazol-2-yl]nicotinamide

To a solution of [4-(3,5-dimethylphenyl)-5-(4-pyridyl)-1,3-thiazol-2-yl]amine (0.52 g, 1.9 mmol) and 4-dimethylaminopyridine (0.07 g, 0.56 mmol) in N,N-dimethylacetamide (10 mL) was added nicotinoyl chloride hydrochloride (0.51 g, 2.86 mmol) and the mixture was stirred at 80°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.44 g, yield 61%).
m.p.: 267-270°C.

### Reference Example A 23-200

### N-[4-(3,5-dimethylphenyl)-5-(4-pyridyl)-1,3-thiazol-2-yl]isonicotinamide

To a solution of [4-(3,5-dimethylphenyl)-5-(4-pyridyl)-1,3-thiazol-2-yl]amine (0.51 g, 1.8 mmol) and 4-dimethylaminopyridine (0.07 g, 0.56 mmol) in N,N-dimethylacetamide (10 mL) was added isonicotinoyl chloride hydrochloride (0.48 g, 2.72 mmol) and the mixture was stirred at 80°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.22 g, yield 32%).
m.p.: 302-304°C.

### Reference Example A 23-201

### N-[4-(3,5-dimethylphenyl)-5-(4-pyridyl)-1,3-thiazol-2-yl]-N'-ethylurea

To a solution of [4-(3,5-dimethylphenyl)-5-(4-pyridyl)-1,3-thiazol-2-yl]amine (0.51 g, 1.8 mmol) in N,N-dimethylacetamide (10 mL) was added ethyl isocyanate (0.20 g, 2.8 mmol) and the mixture was stirred at 80°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.27 g, yield 42%).
m.p.: 202-203°C.

### Reference Example A 23-202

### N-[4-(3,5-dimethylphenyl)-5-(4-pyridyl)-1,3-thiazol-2-yl]-N'-propylurea

To a solution of [4-(3,5-dimethylphenyl)-5-(4-pyridyl)-1,3-thiazol-2-yl]amine (0.51 g, 1.8 mmol) in N,N-dimethylacetamide (15 mL) was added propyl isocyanate (0.23 g, 2.67 mmol) and the mixture was stirred at 80°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.23 g, yield 33%).
m.p.: 128-130°C.

### Reference Example A 23-246

### N-[4-(3,5-dimethylphenyl)-5-(4-pyridyl)-1,3-thiazol-2-yl]pyrazinecarboxamide

To a solution of [4-(3,5-dimethylphenyl)-5-(4-pyridyl)-1,3-thiazol-2-yl]amine (0.50 g, 1.8 mmol) and 4-dimethylaminopyridine (0.06 g, 0.53 mmol) in N,N-dimethylacetamide (5 mL) was added pyrazinecarbonyl chloride (0.44 g, 2.7 mmol) and the mixture was stirred at 70°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.41 g, yield 59%).
m.p.: 269-270°C.

### Reference Example A 24

### 1-bromo-3-ethylbenzene

To a 50% aqueous sulfuric acid solution (43.6 g) of 3-ethylaniline (10.0 g, 82.5mmol) was added dropwise at 0°C an aqueous solution (16.5 mL) of sodium nitrite (6.83 g, 99.0 mmol) over 30 min. The obtained reaction mixture was stirred at 0°C for 45 min. This diazonium salt solution was added by small portions to a 48% hydrobromic acid solution (82.5 mL) of copper(I) bromide (12.4 g, 86.6 mmol) being gently refluxed under heating. After the addition, the reaction mixture was refluxed under heating for 30 min. The reaction mixture was cooled to room temperature and extracted with ether. The extract was washed successively with 1N aqueous sodium hydroxide solution and saturated brine, filtrated, dried and concentrated. The residue was purified by silica gel column chromatography (hexane-ethyl acetate = 20:1) to give the title compound (6.13 g, yield 40%).
oil
¹H-NMR (CDCl₃) δ: 1.23 (3H, t, J= 7.5 Hz), 2.63 (2H, q, J= 7.5 Hz), 7.11-7.20 (2H, m), 7.28-7.38 (2H, m).

### Reference Example A 25

In accordance with Reference Example A 24, the following Reference Example compound A 25 was synthesized using 3-(1-methylethyl)aniline instead of 3-ethylaniline.
Reference Example compound 25: 1-bromo-3-(1-methylethyl)benzene
oil
¹H-NMR (CDCl₃) δ: 1.24 (6H, d, J= 7.0 Hz), 2.77-2.99 (1H, m), 7.03-7.16 (2H, m), 7.27-7.34 (1H, m), 7.37 (1H, s).

### Reference Example A 26

### 3-ethylbenzoic acid

A solution (45 mL) of 1-bromo-3-ethylbenzene (5.1 g, 28 mmol) in tetrahydrofuran was added dropwise to a mixture (5.0 mL) of magnesium turnings (0.74 g, 31 mmol) and tetrahydrofuran under an argon atmosphere, and the mixture was stirred as it was for 30 min. The reaction mixture was added to the crushed dry ice and the mixture was stirred as it was for 1 h. 1N Hydrochloric acid was added to the reaction mixture and the mixture was extracted with ethyl acetate. The extract was dried, filtrated and concentrated. The residue was purified by silica gel column chromatography (hexane-ethyl acetate = 5:1) to give the title compound (3.87 g, yield 93%). oil
¹H-NMR (CDCl₃) δ: 1.28 (3H, t, J= 7.5 Hz), 2.73 (2H, q, J= 7.5 Hz), 7.34-7.50 (2H, m), 7.92-7.98 (2H, m).

### Reference Example A 27

In accordance with Reference Example 26, the following Reference Example A compounds 27-1 and 27-2 were synthesized using 1-bromo-3-(1-methylethyl)benzene or 1-bromo-4-fluoro-3-methylbenzene instead of 1-bromo-3-ethylbenzene.
Reference Example A compound 27-1: 3-(1-methylethyl)benzoic acid
   oil
   ¹H-NMR (CDCl₃) δ: 1.29 (6H, d, J= 7.0 Hz), 2.98-3.06 (1H, m), 7.38-7.54 (2H, m), 7.90-8.02 (2H, m).
Reference Example A compound 27-2: 4-fluoro-3-methylbenzoic acid
   m.p.: 165-167°C.

### Reference Example A 28

### 3-ethylbenzoyl chloride

3-Ethylbenzoic acid (9.40 g, 62.6 mmol) was added slowly to thionyl chloride (45 mL) at 0°C, and N,N-dimethylformamide (3 drops) was added dropwise. The obtained reaction mixture was refluxed under heating as it was for 2 h. The reaction mixture was concentrated and used without purification in the next reaction.

### Reference Example A 29

In accordance with Reference Example A 28, the following Reference Example A compounds 29-1 to 29-3 were synthesized using 3-(1-methylethyl)benzoic acid, 4-fluoro-3-methylbenzoic acid or 4-cyclohexylbenzoic acid instead of 3-ethylbenzoic acid.
Reference Example A compound 29-1: 3-(1-methylethyl)benzoyl chloride
   Used in the next reaction without purification. Reference Example A compound 29-2: 4-fluoro-3-methylbenzoyl chloride
   Used in the next reaction without purification. Reference Example A compound 29-3: 4-cyclohexylbenzoyl chloride
   Used in the next reaction without purification.

### Reference Example A 30

In accordance with Reference Example A 14, the following Reference Example A compounds 30-1 to 30-7 were synthesized respectively using 3-trifluoromethylbenzoyl chloride, 3,5-dichlorobenzoyl chloride, 3-ethylbenzoyl chloride, 3-(1-methylethyl)benzoyl chloride, 4-fluoro-3-methylbenzoyl chloride, 4-cyclohexylbenzoyl chloride and 3-fluorobenzoyl chloride instead of 4-chlorobenzoyl chloride.
Reference Example A compound 30-1: N-(3-trifluoromethylbenzoyl)propyleneimine
   oil
   ¹H-NMR (CDCl₃) δ: 1.42 (3H, d, J= 5.5 Hz), 2.20 (1H, d, J= 3.3 Hz), 2.56-2.67 (2H, m), 7.61 (1H, t, J= 7.7 Hz), 7.81 (1H, d, J= 7.7 Hz), 8.21 (1H, d, J= 7.7 Hz), 8.30 (1H, s).
Reference Example A compound 30-2: N-(3,5-dichlorobenzoyl)-propyleneimine
   oil
   ¹H-NMR (CDCl₃) δ: 1.40 (3H, d, J= 5.1 Hz), 2.19 (1H, d, J= 3.3 Hz), 2.57 (1H, t, J= 5.5 Hz), 2.57-2.70 (1H, m), 7.54 (1H, t, J= 1.8 Hz), 7.88 (2H, d, J= 1.8 Hz).
Reference Example A compound 30-3: N-(3-ethylbenzoyl)-propyleneimine
   oil
   ¹H-NMR (CDCl₃) δ: 1.27 (3H, t, J= 7.5 Hz), 1.40 (3H, d, J= 5.5 Hz), 2.14 (1H, d, J= 2.9 Hz), 2.52-2.61 (2H, m), 2.71 (2H, q, J= 7.5 Hz), 7.32-7.41 (2H, m), 7.81-7.89 (2H, m).
Reference Example A compound 30-4: N-[3-(1-methylethyl)benzoyl]propyleneimine
   oil
   ¹H-NMR (CDCl₃) δ: 1.29 (6H, d, J= 7.0 Hz), 1.40 (3H, d, J= 5.9 Hz), 2.14 (1H, d, J= 3.7 Hz), 2.51-2.64 (2H, m), 2.87-3.10 (1H, m), 7.33-7.46 (2H, m), 7.84 (1H, dt, J= 7.0, 1.8 Hz), 7.91 (1H, s).
Reference Example A compound 30-5: N-(4-fluoro-3-methylbenzoyl)propyleneimine
   oil
   ¹H-NMR (CDCl₃) δ: 1.39 (3H, d, J= 5.4 Hz), 2.14 (1H, d, J= 3.4 Hz), 2.33 (s, 3H), 2.51-2.61 (2H, m), 7.06 (1H, t, J= 8.8 Hz), 7.81-7.90 (2H, m).
Reference Example A compound 30-6: N-(4-cyclohexylbenzoyl)-propyleneimine
   oil
   ¹H-NMR (CDCl₃) δ: 1.22-1.54 (7H, m), 1.67-1.89 (6H, m), 2.12 (1H, d, J= 3.2 Hz), 2.52-2.60 (3H, m), 7.28 (2H, d, J= 8.3 Hz), 7.95 (2H, d, J= 8.3 Hz).
Reference Example A compound 30-7: N-(3-fluorobenzoyl)-propyleneimine
   oil
   ¹H-NMR (CDCl₃) δ: 1.40 (3H, d, J= 5.5 Hz), 2.16 (1H, d, J= 3.3 Hz), 2.52-2.68 (2H, m), 7.25 (1H, ddd, J= 8.4, 2.6, 1.1 Hz), 7.43 (1H, ddd, J= 8.1, 7.7, 5.5 Hz), 7.69 (1H, ddd, J= 8.1, 2.6, 1.5 Hz), 7.81 (1H, ddd, J= 7.7, 1.5, 1.1 Hz).

### Reference Example A 31

In accordance with Reference Example A 16, the following Reference Example A compounds 31-1 to 31-7 were synthesized respectively using N-(3-trifluoromethylbenzoyl)propyleneimine, N-(3,5-dichlorobenzoyl)propyleneimine, N-(3-ethylbenzoyl)-propyleneimine, N-[3-(1-methylethyl)benzoyl]propyleneimine, N-(4-fluoro-3-methylbenzoyl)propyleneimine, N-(4-cyclohexylbenzoyl)propyleneimine and N-(3-fluorobenzoyl)-propyleneimine instead of N-(2-chlorobenzoyl)propyleneimine.
Reference Example A compound 31-1: 2-(4-pyridyl)-1-(3-trifluoromethylphenyl)ethanone
   oil
   ¹H-NMR (CDCl₃) δ: 4.33 (2H, s), 7.21 (2H, d, J= 6.0 Hz), 7.65 (1H, dd, J= 8.4, 7.7 Hz), 7.87 (1H, d, J= 7.7 Hz), 8.18 (1H, d, J= 8.4 Hz), 8.26 (1H, s), 8.59 (2H, d, J= 6.0 Hz).
Reference Example A compound 31-2: 1-(3,5-dichlorophenyl)-2-(4-pyridyl)ethanone
   m.p.: 163-164°C.
Reference Example A compound 31-3: 1-(3-ethylphenyl)-2-(4-pyridyl)ethanone
   m.p.: 102-103°C.
Reference Example A compound 31-4: 1-[3-(1-methylethyl)phenyl]-2-(4-pyridyl)ethanone
   m.p.: 50-52°C.
Reference Example A compound 31-5: 1-(4-fluoro-3-methylphenyl)-2-(4-pyridyl)ethanone
   m.p.: 86-88°C.
Reference Example A compound 31-6: 1-(4-cyclohexylphenyl)-2-(4-pyridyl)ethanone
   oil
   ¹H-NMR (CDCl₃) δ: 1.32-1.52 (5H, m), 1.77-1.89 (5H, m), 2.58 (1H, m), 4.26 (2H, s), 7.20 (2H, d, J= 6.3 Hz), 7.32 (2H, d, J= 8.4 Hz), 7.93 (2H, d, J= 8.4 Hz), 8.56 (2H, d, J= 6.3 Hz).
Reference Example A compound 31-7: 1-(3-fluorophenyl)-2-(4-pyridyl)ethanone
   amorphous powder
   ¹H-NMR (CDCl₃) δ: 4.28 (2H, s), 7.20 (2H, d, J= 6.2 Hz), 7.33 (1H, ddd, J= 8.1, 2.6, 1.1 Hz), 7.49 (1H, ddd, J= 8.1, 7.7, 5.5 Hz), 7.68 (1H, ddd, J= 9.5, 2.6, 1.5 Hz), 7.79 (1H, ddd, J= 7.7, 1.5, 1.1 Hz), 8.58 (2H, d, J= 6.2 Hz).

### Reference Example A 32

In accordance with Reference Example A 17, the following Reference Example A compounds 32-1 to 32-4 were synthesized using 2,4-lutidine or γ-collidine instead of γ-picoline. Reference Example A compound 32-1: 1-(3-methylphenyl)-2-(2-methyl-4-pyridyl)ethanone
m.p.: 56-57°C.
Reference Example A compound 32-2: 1-(3,5-dimethylphenyl)-2-(2-methyl-4-pyridyl)ethanone
   oil
   ¹H-NMR (CDCl₃) δ: 2.38 (6H, s), 2.54 (3H, s), 4.21 (2H, s), 6.98-7.10 (1H, m), 7.01 (1H, m), 7.06 (1H, s), 7.23 (1H, s), 7.60 (2H, s), 8.42-8.45 (1H, m).
Reference Example A compound 32-3: 2-(2,6-dimethyl-4-pyridyl)-1- (3-methylphenyl) ethanone
   m.p.: 46-48°C.
Reference Example A compound 32-4: 1-(3,5-dimethylphenyl)-2-(2,6-dimethyl-4-pyridyl)ethanone
   m.p.: 135-136°C.

### Reference Example A 33

### 2-(2-tert-butoxycarbonylamino-4-pyridyl)-1-(4-methoxyphenyl)ethanone

A solution of 2-tert-butoxycarbonylamino-4-methylpyridine (20 g, 97 mmol) in anhydrous tetrahydrofuran (300 mL) was cooled to -78°C and 1.6 M n-butyllithium/hexane solution (140 mL, 0.23 mol) was added dropwise with stirring. After completion of the dropwise addition, the mixture was stirred at room temperature for 30 min and cooled to -78°C. A solution of N-(4-methoxybenzoyl)propyleneimine (25 g, 0.13 mol) in anhydrous tetrahydrofuran (50 mL) was added dropwise. After completion of the dropwise addition, the mixture was stirred at room temperature for 2 h. To the reaction mixture were added water (100 mL) and isopropyl ether (300 mL), and the obtained crude crystals were collected by filtration. The crude crystals were recrystallized from tetrahydrofuran-hexane to give the title compound (23 g, yield 69%).
m.p.: 187-190°C.

### Reference Example A 34

In accordance with Reference Example A 33, the following Reference Example A compound 34-1 and 34-2 were synthesized respectively using N-(3-methylbenzoyl)propyleneimine and N-(3,5-dimethylbenzoyl)propyleneimine instead of N-(4-methoxybenzoyl)propyleneimine.
Reference Example A compound 34-1: 2-(2-tert-butoxycarbonylamino-4-pyridyl)-1-(3-methylphenyl)ethanone
   m.p.: 144-146°C.
Reference Example A compound 34-2: 2-(2-tert-butoxycarbonylamino-4-pyridyl)-1-(3,5-dimethylphenyl)ethanone
   m.p.: 133-136°C.

### Reference Example A 35

### 2-fluoro-4-methylpyridine

Synthesized in accordance with the method described in Journal of Medicinal Chemistry, vol. 33, pp. 1667-1675 (1990). b.p.: 82-86°C (10 kPa).

### Reference Example A 36

### 2-(2-fluoro-4-pyridyl)-1-(3-methylphenyl)ethanone

A solution of diisopropylamine (44 mL, 0.31 mol) in anhydrous tetrahydrofuran (300 mL) was cooled to -78°C under an argon atmosphere, and 1.6 M n-butyllithium/hexane solution (190 mL, 0.31 mol) was added dropwise with stirring. After completion of the dropwise addition, the mixture was stirred for 10 min, and a solution of 2-fluoro-4-methylpyridine (34.5 g, 0.31 mol) in anhydrous tetrahydrofuran (30 mL) was added. The reaction mixture was stirred at -10°C for 30 min. The reaction solution was cooled to -78°C and a solution of N-(3-methylbenzoyl)propyleneimine (52 g, 0.30 mol) in anhydrous tetrahydrofuran (30 mL) was added dropwise. After completion of dropwise addition, the mixture was stirred at room temperature for 2 h. To the reaction mixture was added water (100 mL), and the mixture was extracted with ethyl acetate. The extract was washed with water, dried and the solvent was evaporated. The residue was recrystallized from isopropyl ether to give the title compound (35 g, yield 52%).
m.p.: 66-67°C.

### Reference Example A 37

In accordance with Reference Example A 36, the following Reference Example A compound 37 was synthesized using N-(3-methoxybenzoyl)propyleneimine instead of N-(3-methylbenzoyl)propyleneimine.
Reference Example A compound 37: 2-(2-fluoro-4-pyridyl)-1-(3-methoxyphenyl)ethanone
   oil
   ¹H-NMR (CDCl₃) δ: 3.86 (3H, s), 4.31 (2H, s), 6.86 (1H, s), 7.03-7.19 (2H, m), 7.31-7.59 (3H, m), 8.18 (1H, d, J= 5.6 Hz).

### Reference Example A 38

In accordance with Reference Example A 21, the following Reference Example compounds 38-1 to 3.8-21 were synthesized respectively using 2-methylbenzonitrile, 3-methylbenzonitrile, 4-methylbenzonitrile, 2-chlorobenzonitrile, 3-chlorobenzonitrile, 4-chlorobenzonitrile, 3-methoxybenzonitrile, 4-methoxybenzonitrile, 2-fluorobenzonitrile, 3-fluorobenzonitrile, 4-fluorobenzonitrile, 4-nitrobenzonitrile, piperonylonitrile, 3-methoxycarbonylbenzonitrile, 4-methoxycarbonylbenzonitrile, butyronitrile, isobutyronitrile, valeronitrile, hexanenitrile, 3-phenylpropionitrile and 4-phenylbutyronitrile instead of 4-methylthiobenzonitrile.
Reference Example A compound 38-1: 2-methyl(thiobenzamide)
   oil
   ¹H-NMR (CDCl₃) δ: 2.37 (3H, s), 6.88 (1H, br s), 7.06-7.23 (3H, m), 7.24-7.31 (1H, m), 7.88 (1H, br s).
Reference Example A compound 38-2: 3-methyl(thiobenzamide)
   m.p.: 88-89°C.
Reference Example A compound 38-3: 4-methyl(thiobenzamide)
   m.p.: 172-174°C.
Reference Example A compound 38-4: 2-chlorothiobenzamide
   m.p.: 58-59°C.
Reference Example A compound 38-5: 3-chlorothiobenzamide
   m.p.: 114-115°C.
Reference Example A compound 38-6: 4-chlorothiobenzamide
   m.p.: 130-131°C.
Reference Example A compound 38-7: 3-methoxythiobenzamide
   oil
   ¹H-NMR (CDCl₃) δ: 3.86 (3H, s), 7.02-7.08 (1H, m), 7.31-7.36 (3H, m), 7.46-7.49 (1H, m), 7.76 (1H, br s).
Reference Example A compound 38-8: 4-methoxythiobenzamide
   m.p.: 148-149°C.
Reference Example A compound 38-9: 2-fluorothiobenzamide
   m.p.: 113-114°C.
Reference Example A compound 38-10: 3-fluorothiobenzamide
   m.p.: 151-152°C.
Reference Example A compound 38-11: 4-fluorothiobenzamide
   m.p.: 156-157°C.
Reference Example A compound 38-12: 4-nitrothiobenzamide
   m.p.: 159-160°C.
Reference Example A compound 38-13: thiopiperonylamide
   m.p.: 188-189°C.
Reference Example A compound 38-14: 3-methoxycarbonyl-thiobenzamide
   m.p.: 140-141°C.
Reference Example A compound 38-15: 4-methoxycarbonylthiobenzamide
   m.p.: 191-192°C.
Reference Example A compound 38-16: thiobutylamide
   oil
   ¹H-NMR (CDCl₃) δ: 0.99 (3H, t, J= 7.6 Hz), 1.72-1.93 (2H, m), 2.64 (2H, t, J= 7.6 Hz), 7.02 (1H, br s), 7.77 (1H, br s).
Reference Example compound A 38-17: thioisobutylamide
   oil
   ¹H-NMR (CDCl₃) δ: 1.28 (6H, d, J= 5.8 Hz), 2.79-2.96 (1H, m), 6.99 (1H, br s), 7.71 (1H, br s).
Reference Example A compound 38-18: thiovaleramide
   oil
   ¹H-NMR (CDCl₃) δ: 0.94 (3H, t, J= 7.3 Hz), 1.31-1.49 (2H, m), 1.68-1.83 (2H, m), 2.67 (2H, t, J= 7.7 Hz), 6.92 (1H, br s), 7.73 (1H, br s).
Reference Example A compound 38-19: hexanethioamide
   oil
   ¹H-NMR (CDCl₃) δ: 0.90 (3H, t, J= 6.9 Hz), 1.22-1.45 (4H, m), 1.70-1.84 (2H, m), 2.66 (2H, t, J= 7.5 Hz), 7.05 (1H, br s), 7.91 (1H, br s).
Reference Example A compound 38-20: 3-phenyl(thiopropionamide) m.p.: 83-84°C.
Reference Example A compound 38-21: 4-phenyl(thiobutylamide)
   m.p.: 60-61°C.

### Reference Example A 39

In accordance with Reference Example A 6, the following Reference Example A compounds 39-1 to 39-13 were synthesized respectively using 2-(4-pyridyl)-1-(3-trifluoromethylphenyl)-ethanone, 1-(3,5-dichlorophenyl)-2-(4-pyridyl)ethanone, 1-(3-ethylphenyl)-2-(4-pyridyl)ethanone, 1-[3-(1-methylethyl)-phenyl]-2-(4-pyridyl)ethanone, 1-(4-fluoro-3-methylphenyl)-2-(4-pyridyl)ethanone, 1-(4-cyclohexylphenyl)-2-(4-pyridyl)-ethanone, 1-(3-fluorophenyl)-2-(4-pyridyl)ethanone, 2-(2-fluoro-4-pyridyl)-1-(3-methylphenyl)ethanone, 2-(2-fluoro-4-pyridyl)-1-(3-methoxyphenyl)ethanone, 1-(3-methylphenyl)-2-(2-methyl-4-pyridyl)ethanone, 1-(3,5-dimethylphenyl)-2-(2-methyl-4-pyridyl)ethanone, 2-(2,6-dimethyl-4-pyridyl)-1-(3-methylphenyl)ethanone and 1-(3,5-dimethylphenyl)-2-(2,6-dimethyl-4-pyridyl)ethanone instead of 1-(4-methoxyphenyl)-2-(3-pyridyl)ethanone.
Reference Example A compound 39-1: 2-bromo-2-(4-pyridyl)-1-(3-trifluoromethylphenyl)ethanone hydrobromide
   Used in the next reaction without purification.
Reference Example A compound 39-2: 2-bromo-1-(3,5-dichlorophenyl)-2-(4-pyridyl)ethanone hydrobromide
   m.p.: 253-254°C
Reference Example A compound 39-3: 2-bromo-1-(3-ethylphenyl)-2-(4-pyridyl)ethanone hydrobromide
   m.p.: 146-148°C.
Reference Example A compound 39-4: 2-bromo-1-[3-(1-methylethyl)phenyl]-2-(4-pyridyl)ethanone hydrobromide
   m.p.: 143-144°C.
Reference Example A compound 39-5: 2-bromo-1-(4-fluoro-3-methylphenyl)-2-(4-pyridyl)ethanone hydrobromide
   m.p.: 211-214°C.
Reference Example A compound 39-6: 2-bromo-1-(4-cyclohexylphenyl)-2-(4-pyridyl)ethanone hydrobromide
   m.p.: 189-191°C.
Reference Example A compound 39-7: 2-bromo-1-(3-fluorophenyl)-2-(4-pyridyl)ethanone hydrobromide
   m.p.: 191-194°C.
Reference Example A compound 39-8: 2-bromo-2-(2-fluoro-4-pyridyl)-1-(3-methylphenyl)ethanone hydrobromide
   Used in the next reaction without purification.
Reference Example A compound 39-9: 2-bromo-2-(2-fluoro-4-pyridyl)-1-(3-methoxyphenyl)ethanone hydrobromide
   Used in the next reaction without purification.
Reference Example A compound 39-10: 2-bromo-1-(3-methylphenyl)-2-(2-methyl-4-pyridyl)ethanone hydrobromide
   m.p.: 144-146°C.
Reference Example A compound 39-11: 2-bromo-1-(3,5-dimethylphenyl)-2-(2-methyl-4-pyridyl)ethanone hydrobromide Used in the next reaction without purification.
Reference Example A compound 39-12: 2-bromo-2-(2,6-dimethyl-4-pyridyl)-1-(3-methylphenyl)ethanone hydrobromide
   Used in the next reaction without purification.
Reference Example A compound 39-13: 2-bromo-1-(3,5-dimethylphenyl)-2-(2,6-dimethyl-4-pyridyl)ethanone hydrobromide
   m.p.: 208-212°C.

### Reference Example A 40

### 2-bromo-2-(2-tert-butoxycarbonylamino-4-pyridyl)-1-(4-methoxyphenyl)ethanone hydrobromide

To a solution of 2-(2-tert-butoxycarbonylamino-4-pyridyl)-1-(4-methoxyphenyl)ethanone (0.36 g, 1.1 mmol) in acetic acid (5 mL) was added bromine (0.058 mL, 1.1 mmol) and the mixture was stirred at room temperature for 1 h. The reaction mixture was concentrated and the residue was washed with isopropyl ether to give the title compound (0.44 g, yield 82%).
amorphous powder
¹H-NMR (CDCl₃) δ: 1.55 (6H, s), 3.92 (3H, s), 6.35 (1H, s), 6.99-7.03 (2H, m), 7.66 (1H, dd, J= 6.6, 1.8 Hz), 8.02-8.07 (2H, m), 8.20 (1H, d, J= 6.6 Hz), 8.70 (2H, d, J= 1.8 Hz), 11.02 (1H, br s).

### Reference Example A 41

In accordance with Reference Example A 40, the following Reference Example A compounds 41-1 and 41-2 were synthesized respectively using 2-(2-tert-butoxycarbonylamino-4-pyridyl)-1-(3-methylphenyl)ethanone and 2-(2-tert-butoxycarbonylamino-4-pyridyl)-1-(3,5-dimethylphenyl)ethanone instead of 2-(2-tert-butoxycarbonylamino-4-pyridyl)-1-(4-methoxyphenyl)ethanone. Reference Example A compound 41-1: 2-bromo-2-(2-tert-butoxycarbonylamino-4-pyridyl)-1-(3-methylphenyl)ethanone hydrobromide

Used in the next reaction without purification. Reference Example A compound 41-2: 2-bromo-2-(2-tert-butoxycarbonylamino-4-pyridyl)-1-(3,5-dimethylphenyl)ethanone hydrobromide

Used in the next reaction without purification.

### Reference Example A 42

### ethyl (4-phenyl-1-piperazinyl)carbothioylcarbamate

1-Phenylpiperazine (10 g, 62 mmol) was added to a solution of ethyl isothiocyanatoformate (8.1 g, 62 mmol) in acetone (30 mL) and the mixture was refluxed under heating for 1 h. The reaction mixture was concentrated and the crude crystals were recrystallized from ethyl acetate to give the title compound (13 g, yield 73%).
m.p.: 134-135°C.

### Reference Example A 43

### 4-phenyl-1-piperazinecarbothioamide

Ethyl (4-phenyl-1-piperazinyl)carbothioylcarbamate (13 g, 44 mmol) was added to conc. hydrochloric acid (44 mL) and the mixture was stirred at 80°C for 2 h. The reaction mixture was made basic with 8N aqueous sodium hydroxide solution and the crystals were collected by filtration. The crystals were washed with water and dried to give the title compound (6.1 g, yield 63%).
m.p.: 178-179°C.

### Reference Example A 44

In accordance with the methods described in Reference Examples A 8 to 12, Reference Example A 44-1, JP-A-61-10580 and USP 4,612,321, Reference Example compounds A 44-1 to 44-129 shown in the following Tables 32-42 were synthesized.

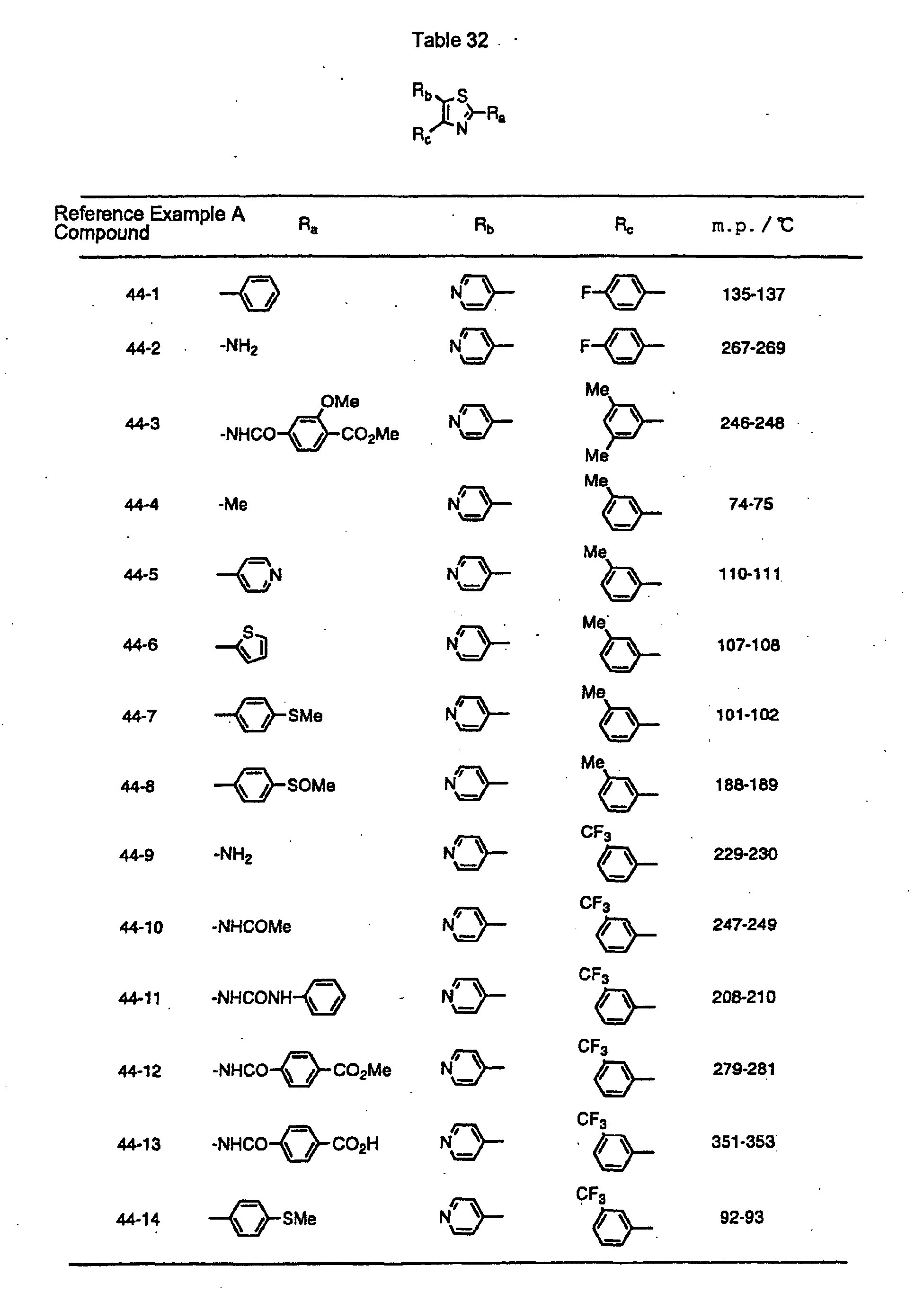

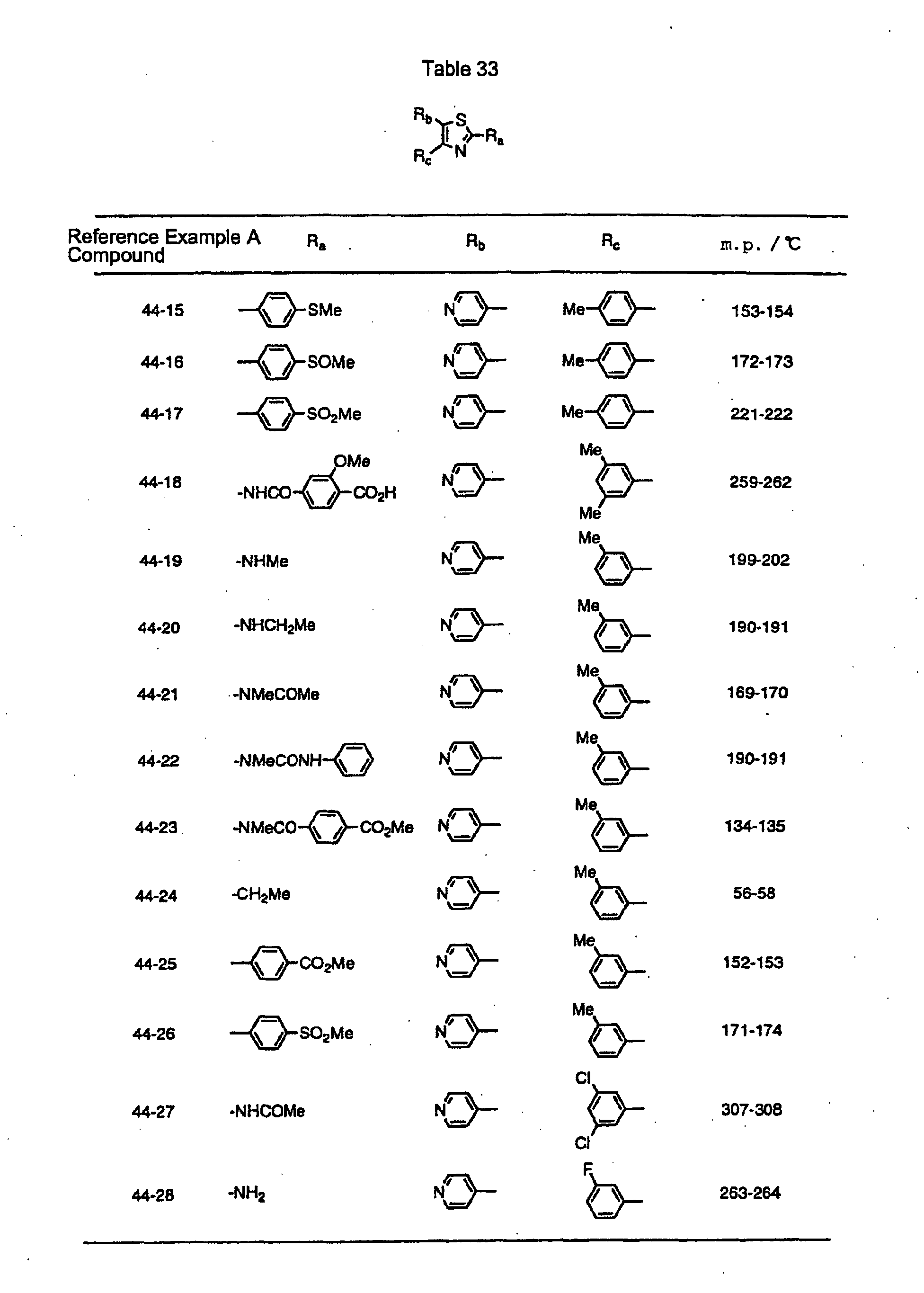

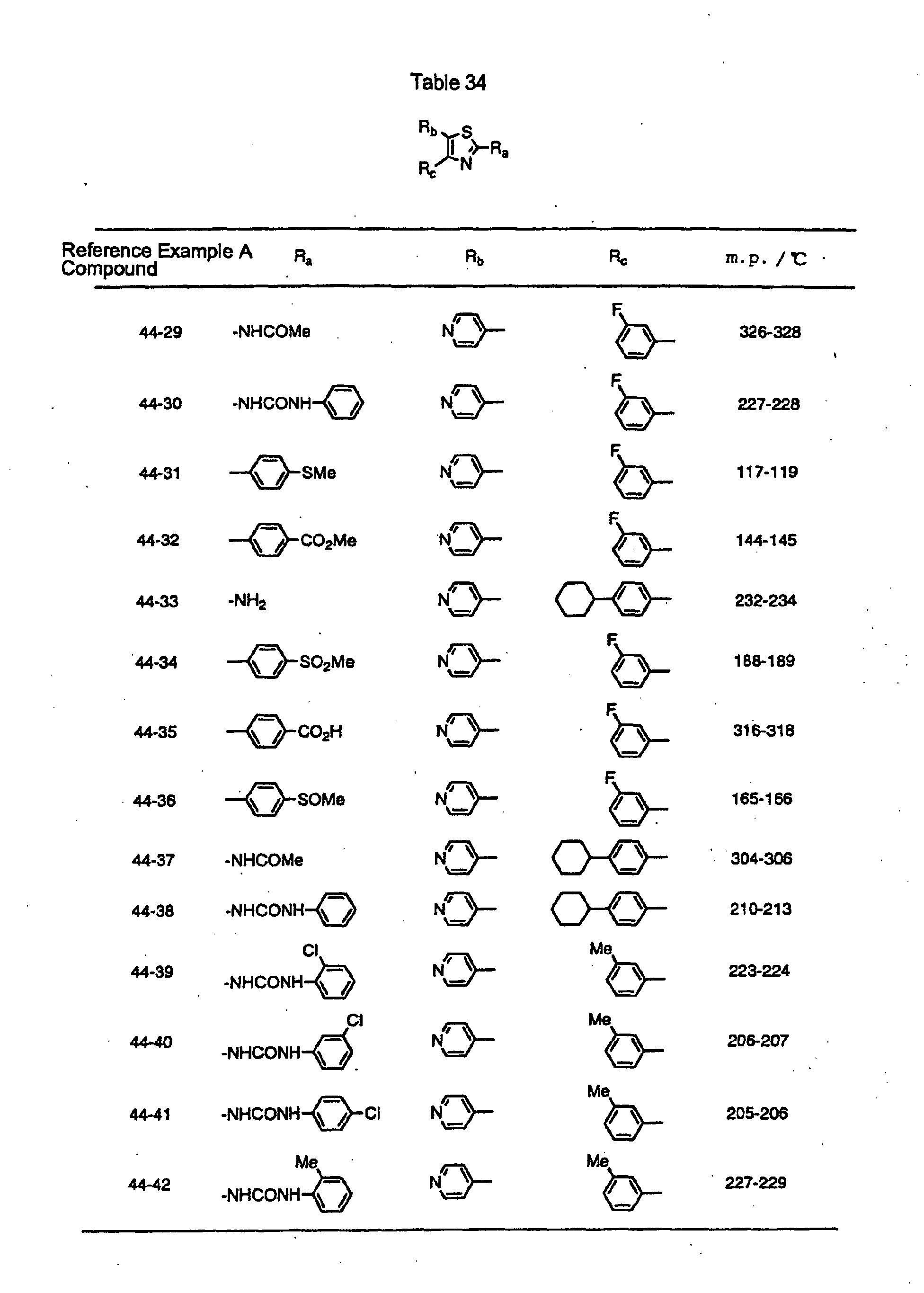

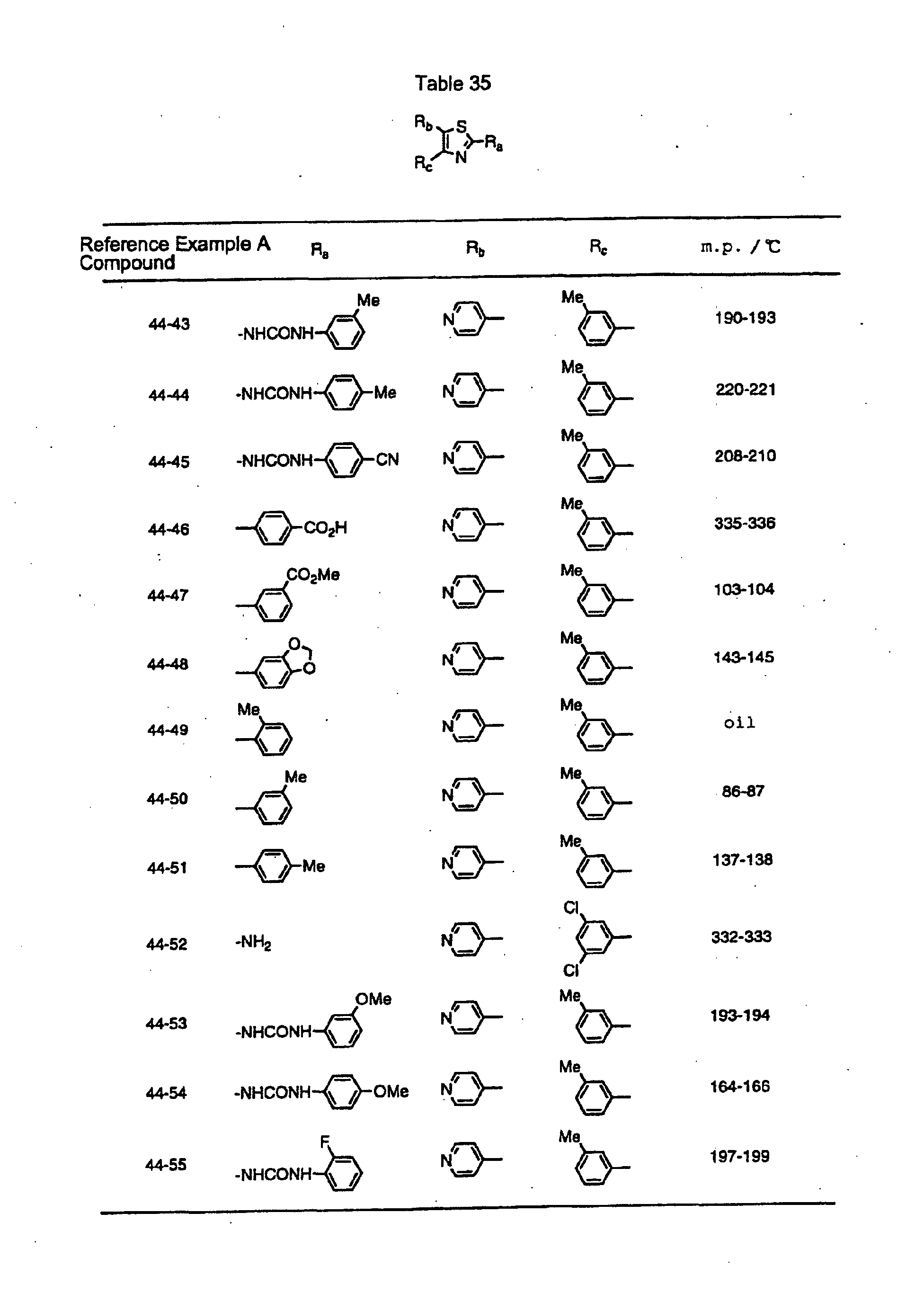

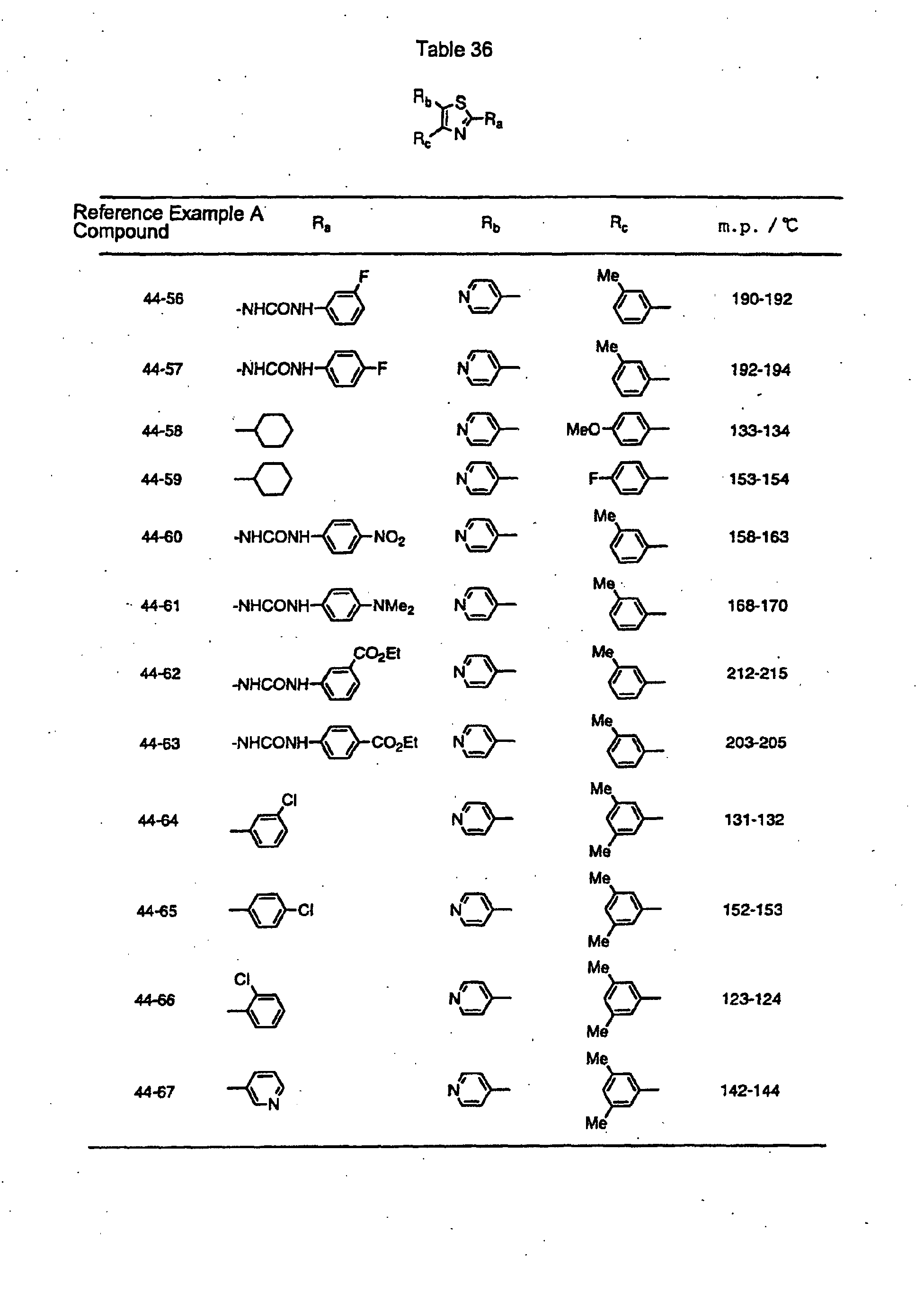

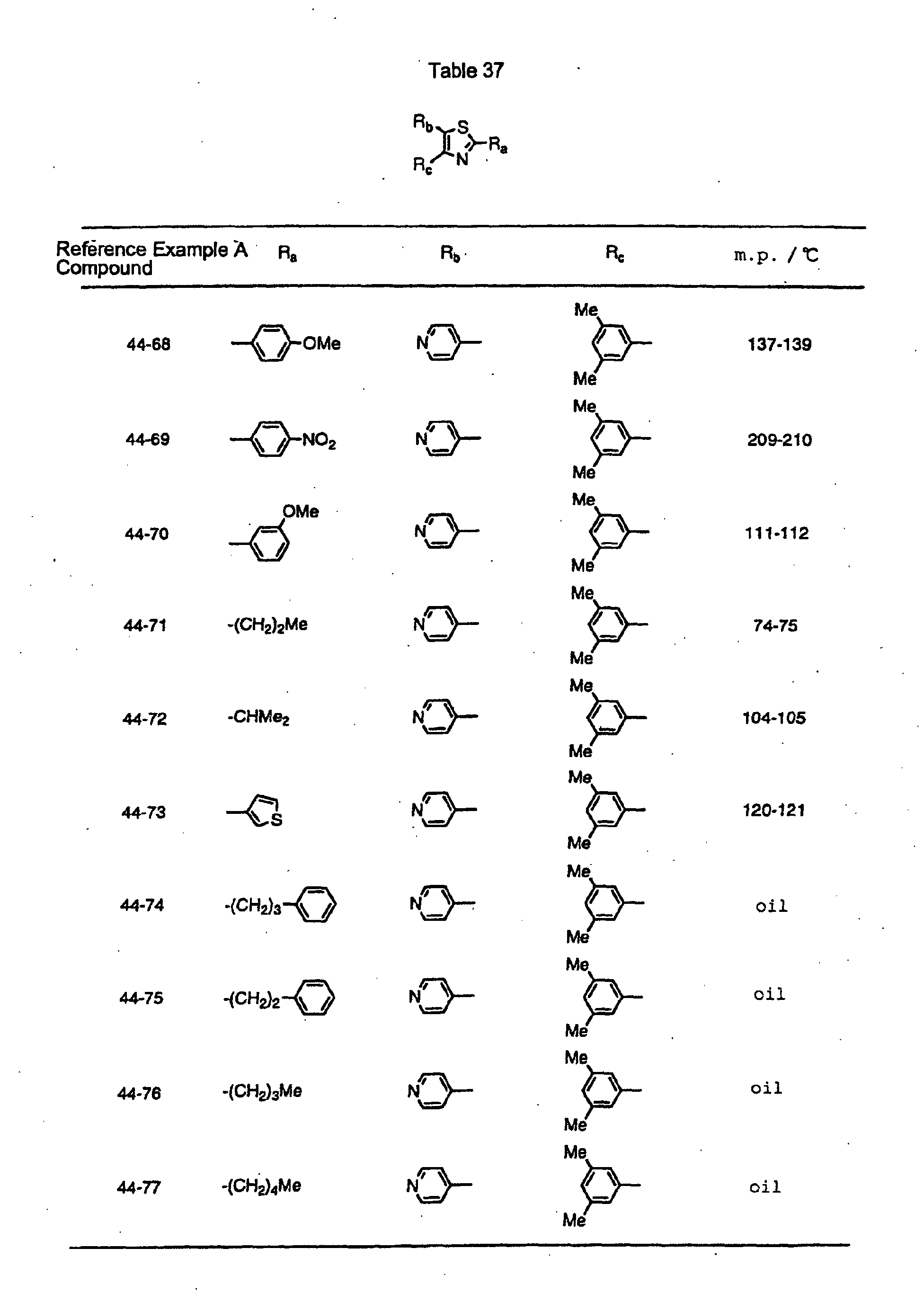

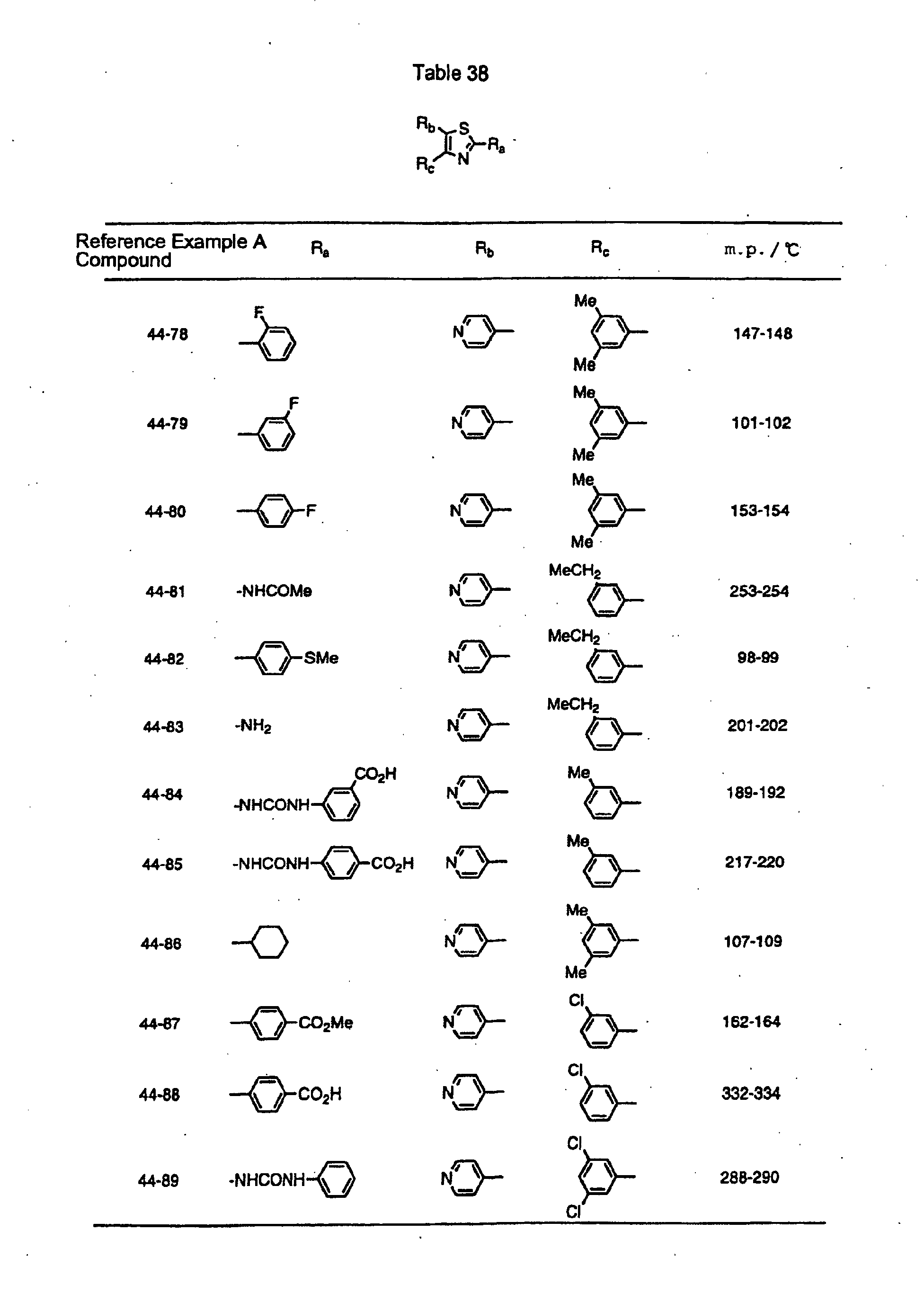

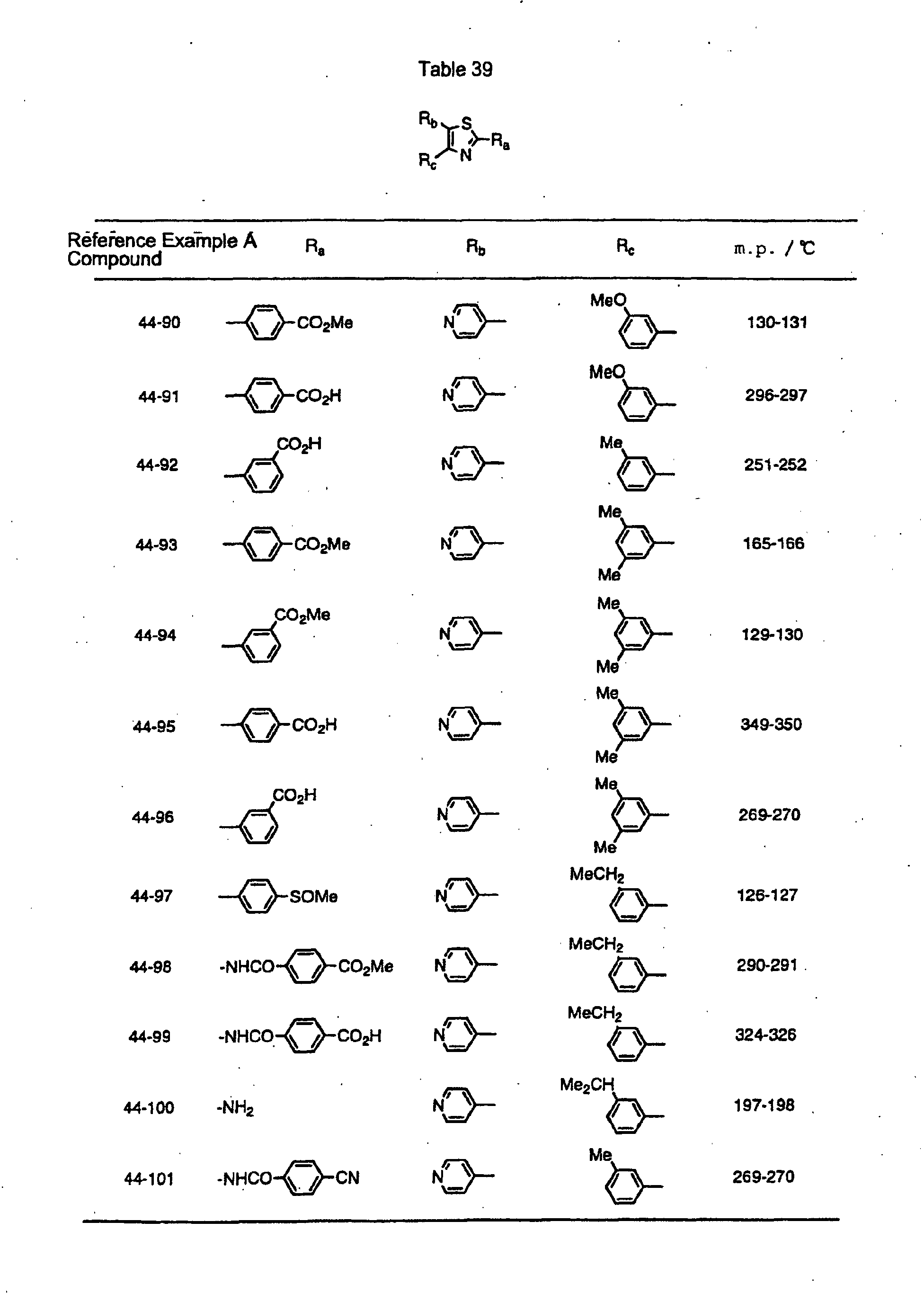

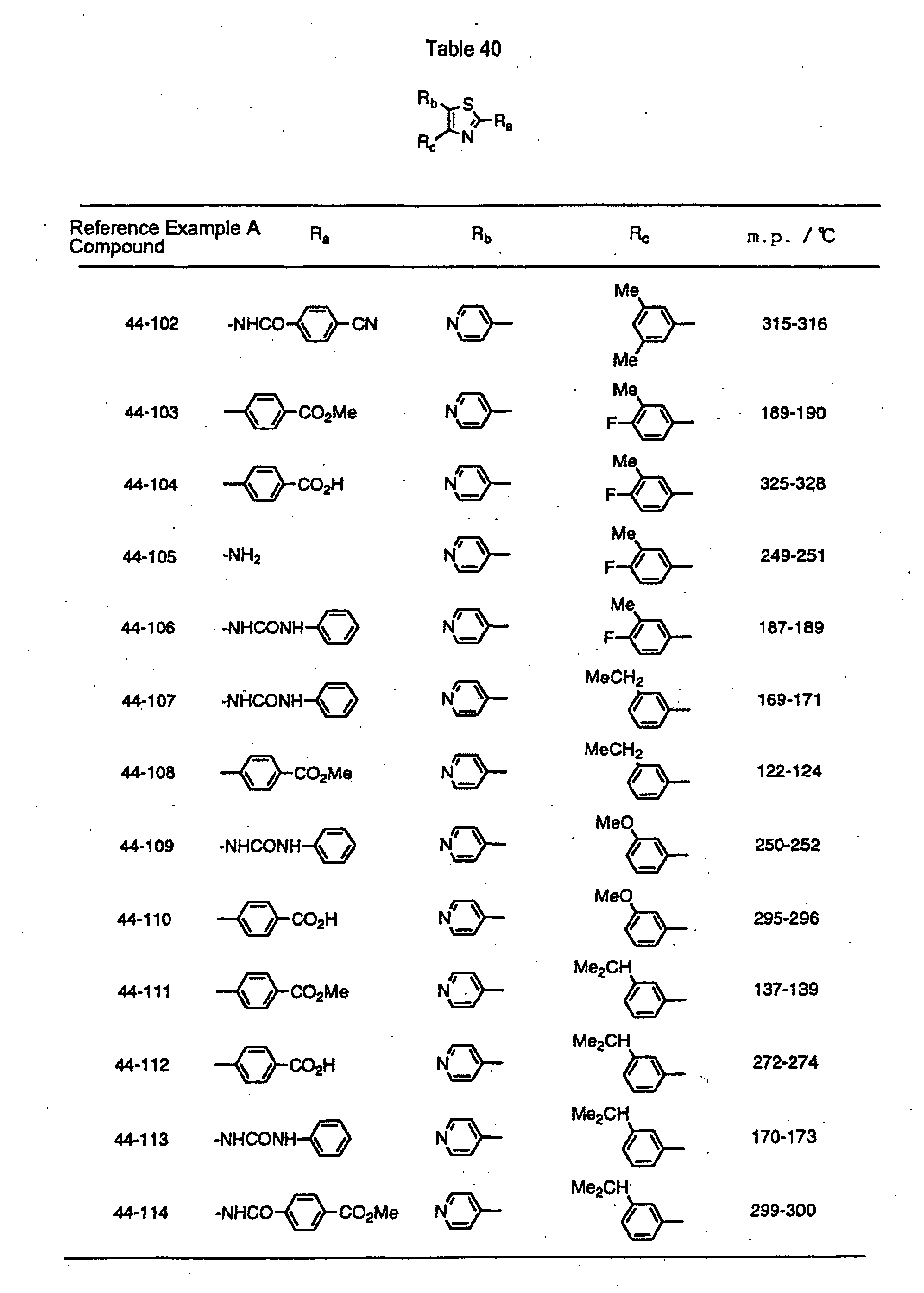

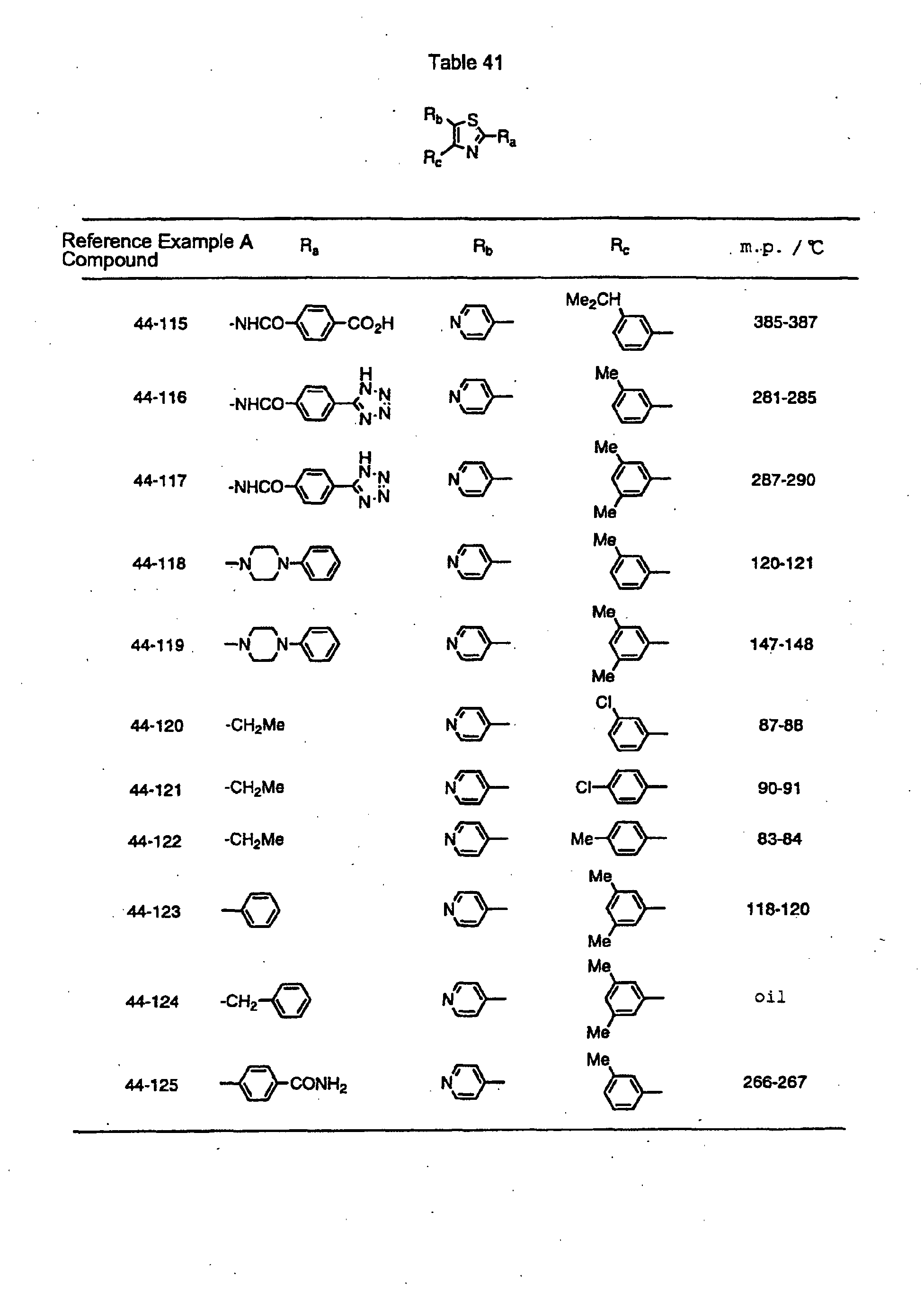

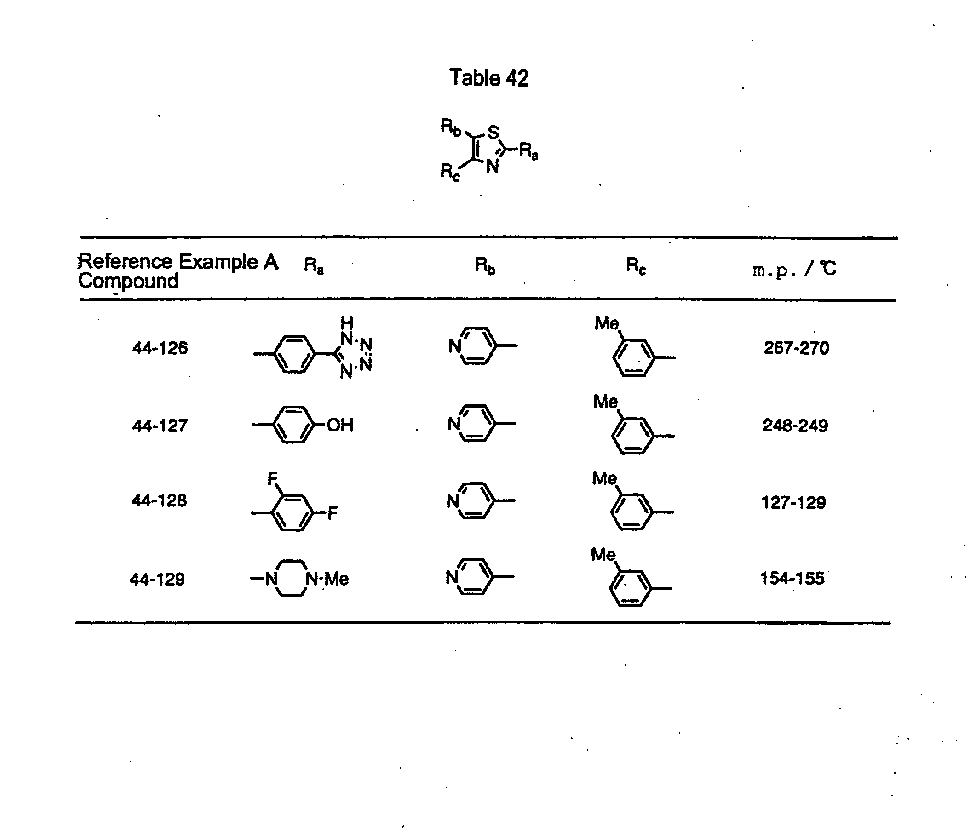

### Reference Example A 44-1

### 4-(4-fluorophenyl)-2-phenyl-5-(4-pyridyl)-1,3-thiazole

A solution of 2-bromo-1-(4-fluorophenyl)-2-(4-pyridyl)ethanone hydrobromide (1.6 g, 4.1 mmol) and thiobenzamide (0.57 g, 4.2 mmol) in N,N-dimethylformamide (5 mL) was stirred at room temperature for 14 h. To the reaction mixture was poured aqueous sodium hydrogencarbonate solution and the precipitated solid was collected by filtration. The obtained solid was washed with water and dried. The crude crystals were recrystallized from ethyl acetate to give the title compound (0.27 g, yield 19%).
m.p.: 135-137°C.

The proton nuclear magnetic resonance spectrum of the aforementioned Reference Example A 44 is shown in the following Table 43.

**Table 43**

| Reference Example A Compound No. | Proton Nuclear Magnetic Resonance Spectrum |
|---|---|
| 44-49 | ¹H-NMR (CDCl₃) δ : 2.34 (3H, s), 2.70 (3H, s), 7.14-7.38 (8H, m), 7.46 (1H, s), 7.81 (1H, ddd, J= 6.6, 1.8, 1.1 Hz), 8.56 (2H, d, J= 6.0 Hz). |
| 44-74 | ¹H-NMR (CDCl₃) δ : 2.04-2.26 (8H, m), 2.79 (2H, t, J= 7.5 Hz), 3.08 (2H, t, J= 7.6 Hz), 6.97 (1H, s), 7.08 (2H, s), 7.17-7.35 (7H, m), 8.50 (2H, dd, J= 4.6, 1.8 Hz). |
| 44-75 | ¹H-NMR (CDCl₃) δ : 2.27 (6H, s), 3.13-3.23 (2H, m), 3.31-3.41 (2H, m), 6.98 (1H, s), 7.08 (2H, s), 7.19 (2H, dd, J= 4.5, 1.7 Hz), 7.24-7.37 (5H, m), 8.50 (2H, dd, J= 4.5, 1.7 Hz). |
| 44-76 | ¹H-NMR (CDCl₃) δ : 0.98 (3H, t, J= 7.3 Hz), 1.43-1.55 (2H, m), 1.76-1.88 (2H, m), 2.26 (6H, m), 3.05 (2H, t, J= 7.7 Hz), 6.97 (1H, s), 7.08 (2H, s), 7.21 (2H, dd, J= 4.6, 1.8 Hz), 8.50 (2H, dd, J= 4.6, 1.8 Hz). |
| 44-77 | ¹H-NMR (CDCl₃) δ : 0.90-0.97 (3H, m), 1.38-1.49 (4H, m), 1.78-1.89 (2H, m), 2.26 (6H, s), 3.04 (2H, t, J= 7.9 Hz), 6.97 (1H, s), 7.08 (2H, s), 7.21 (2H, dd, J= 4.5, 1.8 Hz), 8.50 (2H, dd, J= 4.5, 1.8 Hz). |
| 44-124 | ¹H-NMR (CDCl₃) δ : 2.27 (6H, s), 4.38 (2H, s), 6.99 (1H, s), 7.10 (2H, s), 7.16 (2H, dd, J= 4.9, 1.6 Hz), 7.34-7.41 (5H, m), 8.47 (2H, dd, J=4.9, 1.6 Hz). |

### Reference Example A 45

In accordance with Reference Example A 21, the following Reference Example A compound 45 was synthesized using pivalonitrile instead of 4-methylthiobenzonitrile. Reference Example A compound 45: thiopivaloamide
m.p.: 117-119°C.

### Reference Example A 46

In accordance with the methods described in Reference Example s A 8 to 12, Reference Example A 44-1, JP-A-61-10580 and USP 4,612,321, Reference Example A compounds 46-1 to 46-7 shown in the following Table 44 were synthesized.

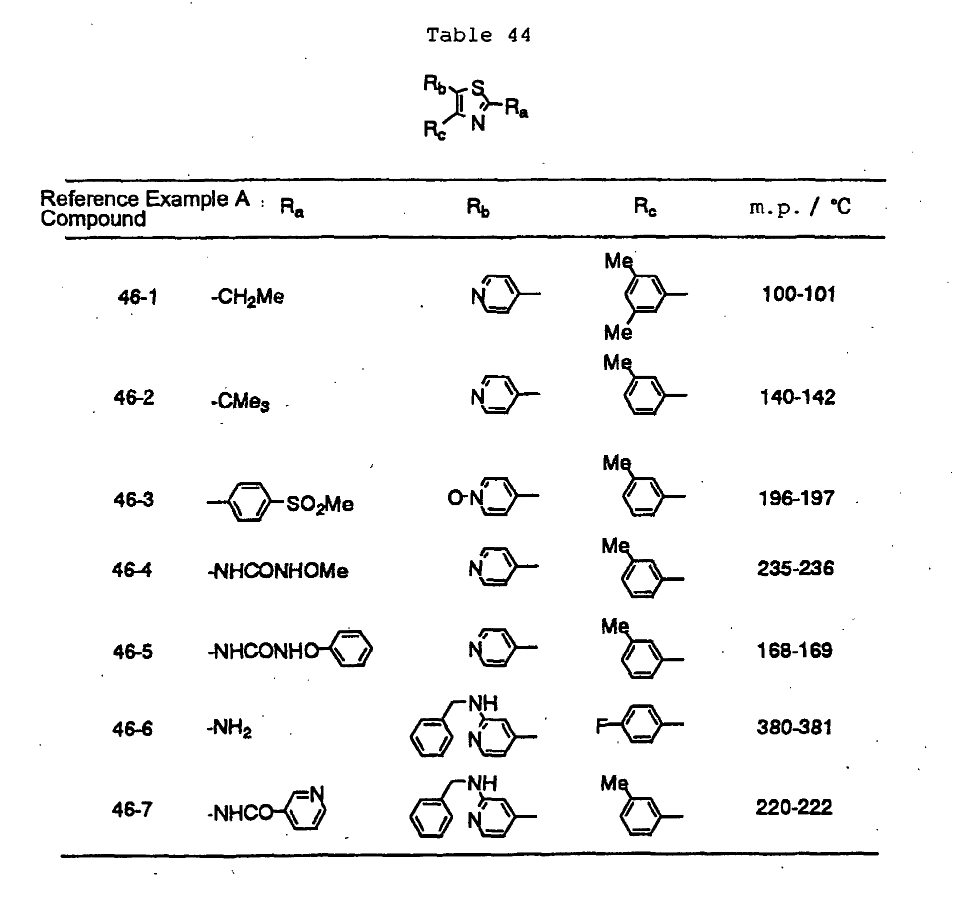

### Reference Example B 1

| | |
|---|---|
| (1) Reference Example A compound 23-313 | 10.0 mg |
| (2) lactose | 60.0 mg |
| (3) cornstarch | 35.0 mg |
| (4) gelatin | 3.0 mg |
| (5) magnesium stearate | 2.0 mg |

A mixture of Reference Example A compound 23-313 (10.0 mg), lactose (60.0 mg) and cornstarch (35.0 mg) is granulated using 10% aqueous gelatin solution (0.03 ml, 3.0 mg as gelatin) and passing through a 1 mm mesh sieve. The granules are dried at 40°C and passed through the sieve again. The granules thus obtained are mixed with magnesium stearate (2.0 mg) and compressed. The obtained core tablet is coated with sugar coating made of an aqueous suspension of sucrose, titanium dioxide, talc and gum arabic. The coated tablet is polished with bee wax to give a coated tablet.

### Reference Example B 2

| | |
|---|---|
| (1) Reference Example A compound 23-313 | 10.0 mg |
| (2) lactose | 70.0 mg |
| (3) cornstarch | 50.0 mg |
| (4) soluble starch | 7.0 mg |
| (5) magnesium stearate | 3.0 mg |

Reference Example A compound 23-313 (10.0 mg) and magnesium stearate (3.0 mg) are granulated using an aqueous solution (0.07 ml) of soluble starch (7.0 mg as soluble starch), dried and mixed with lactose (70.0 mg) and cornstarch (50.0 mg). The mixture is compressed to give tablets.

### Reference Example B 3

| | |
|---|---|
| (1) Reference Example A compound 23-313 | 5.0 mg |
| (2) sodium chloride | 20.0 mg |
| (3) distilled water | to total 2 ml |

Reference Example A compound 23-313 (5.0 mg) and sodium chloride (20.0 mg) are dissolved in distilled water and water is added to make the total amount 2.0 ml. The solution is filtrated and aseptically filled in a 2 ml ampoule. The ampoule is sterilized and sealed to give a solution for injection.

### Reference Example B 4

| | |
|---|---|
| (1) Reference Example A compound 23-331 | 10.0 mg |
| (2) lactose | 60.0 mg |
| (3) cornstarch | 35.0 mg |
| (4) gelatin | 3.0 mg |
| (5) magnesium stearate | 2.0 mg |

A mixture of Reference Example A compound 23-331 (10.0 mg), lactose (60.0 mg) and cornstarch (35.0 mg) is granulated using 10% aqueous gelatin solution (0.03 ml, 3.0 mg as gelatin) and passing through a 1 mm mesh sieve. The granules are dried at 40°C and passed through the sieve again. The granules thus obtained are mixed with magnesium stearate (2.0 mg) and compressed. The obtained core tablet is coated with sugar coating made of an aqueous suspension of sucrose, titanium dioxide, talc and gum arabic. The coated tablet is polished with bee wax to give a coated tablet.

### Reference Example B 5

| | |
|---|---|
| (1) Reference Example A compound 23-331 | 10.0 mg |
| (2) lactose | 70.0 mg |
| (3) cornstarch | 50.0 mg |
| (4) soluble starch | 7.0 mg |
| (5) magnesium stearate | 3.0 mg |

Reference Example A compound 23-331 (10.0 mg) and magnesium stearate (3.0 mg) are granulated using an aqueous solution (0.07 ml) of soluble starch (7.0 mg as soluble starch), dried and mixed with lactose (70.0 mg) and cornstarch (50.0 mg). The mixture is compressed to give tablets.

### Reference Example B 6

| | |
|---|---|
| (1) Reference Example A compound 23-331 | 5.0 mg |
| (2) sodium chloride | 20.0 mg |

| | |
|---|---|
| (3) distilled water | to total 2 ml |

Reference Example A compound 23-331 (5.0 mg) and sodium chloride (20.0 mg) are dissolved in distilled water and water is added to make the total 2.0 ml. The solution is aseptically filtered and filled into a 2 ml ampoule. The ampoule is sterilized and sealed to give a solution for injection.

### Reference Example C 1:

The genetic manipulations described below were according to a method described in the book (Maniatis et al., Molecular Cloning, Cold Spring Harbor Laboratory, 1989) or methods described in the protocols attached to the reagents.

### (1) Cloning of human p38 MAP kinase gene and preparation of recombinant baculovirus

Cloning of human p38 MAP kinase gene was performed by a PCR method using a primer set P38-U: 5'-ACCACTCGAGATGGACTACAAGGACGACGATGACAAGTCTCAGGAGAGGCCCACGTTCTACC -3' [SEQ ID NO:1] and PAG-L: 5'-ACCCGGTACCACCAGGTGCTCAGGACTCCATCTCT-3' [SEQ ID NO:2] made by the use of kidney cDNA (Toyobo, QUICK-Clone cDNA) as a template and referring to the base sequence of p38 MAP kinase gene reported by Han et al. (Science 265 (5173), 808-811 (1994)).

A PCR reaction was performed by a Hot Start method using AmpliWax PCR Gem 100 (Takara Shuzo). As the lower mixed solution, 2 µL 10×LA PCR Buffer, 3 µL 2.5 mM dNTP solution, each 2.5 µL of 12.5 µM primer solutions, and 10 µL sterile distilled water were mixed. As the upper mixed solution, 1 µL human cardiac cDNA (1 ng/mL) as a template, 3 µL 10×LA PCR Buffer, 1 µL 2.5 mM dNTP solution, 0.5 µL TaKaRa LA Taq DNA polymerase (Takara Shuzo), and 24.5 µL sterile distilled water were mixed. One AmpliWax PCR Gem 100 (Takara Shuzo) was added to the prepared lower mixed solution and the mixture was treated at 70°C for 5 min and for 5 min in an ice and, thereafter, the upper mixed solution was added to prepare a reaction solution for PCR. A tube containing the reaction solution was set at a thermal cycler (Perkin Elmer), which was treated at 95°C for 2 min. Further, after repeating 35 times a cycle of 15 seconds at 95°C and 2 minutes at 68°C, treatment was performed at 72°C for 8 minutes. The resulting PCR product was subjected to agarose gel (1%) electrophoresis, 1.1 kb DNA fragment containing p38 MAP kinase gene was recovered from the gel and, thereafter, which was inserted into pT7Blue-T vector (Takara Shuzo) to make the plasmid pHP38.

The 4.8 kb XhoI-KpnI fragment of the plasmid pFASTBAC1 (CIBCOBRL) and the 1.1 kb XhoI-Kpn fragment of the above plasmid pHP38 were ligated to make the plasmid pFBHP38.

The plasmid pFBHP38 and BAC-TO-BAC Baculovirus Expression System (GIBCOBRL) were used to prepare the recombinant baculovirus virusstock BAC-HP38.

### (2) Cloning of human MKK3 gene and preparation of recombinant baculovirus

Cloning of human MKK3 gene was performed by a PCR method using a primer set MKK-U: 5'-ACAAGAATTCATAACATATGGCTCATCATCATCATCATCATTCCAAGCCACCCGCACCCAA-3' [SEQ ID NO:3] and MKK-L: 5'-TCCCGTCTAGACTATGAGTCTTCTCCCAGGAT-3' [SEQ ID NO:4] made by the use of kidney cDNA (Toyobo, QUICK-Clone cDNA) as a template and referring to the base sequence of MKK3 gene reported by Derijard, B. et al., Science 267 (5198), 682-685 (1995).

A PCR reaction was performed by a Hot Start method using AmpliWax PCR Gem 100 (Takara Shuzo). As the lower mixed solution, 2 µL 10×LA PCR Buffer, 3 µL 2.5 mM dNTP solution, each 2.5 µL of 12.5 µM primer solutions, and 10 µL sterile distilled water were mixed. As the upper mixed solution, 1 µL human kidney cDNA (1 ng/mL) as a template, 3 µL 10×LA PCR Buffer, 1 µL 2.5 mM dNTP solution, 0.5 µL TaKaRa LA Taq DNA polymerase (Takara Shuzo) and 24.5 µL sterile distilled water were mixed. One AmpliWax PCR Gem 100 (Takara Shuzo) was added to the prepared lower mixed solution and the mixture was treated at 70°C for 5 minutes and for 5 minutes in an ice and, thereafter, the upper mixed solution was added to prepare a reaction solution for PCR. A tube containing the reaction solution was set at a thermal cycler (Perkin Elmer), which was treated at 95°C for 2 minutes. Further, after repeating 35 times a cycle of 15 seconds at 95°C and 2 minutes at 68°C, treatment was performed at 72°C for 8 minutes. The resulting PCR product was subjected to agarose gel (1%) electrophoresis, 1.0 kb DNA fragment containing MKK3 gene was recovered from the gel and, thereafter, which was inserted into pT7Blue-T vector (Takara Shuzo) to make the plasmid pHMKK3.

In order to mutate MKK3 into a constitutive active form (from Ser to Glu at 189 position, from Thr to Glu at position 193), a primer set SER-U: 5'-GGCTACTTGGTGGACGAGGTGGCCAAGGAGATGGATGCCGGCTGC-3' [SEQ ID NO:5] and SER-L: 5'-GCAGCCGGCATCCATCTCCTTGGCCACCTCGTCCACCAAGTAGCC-3' [SEQ ID NO:6] was used to introduce a mutation by QuikChange Site-Directed Mutagenesis Kit (Stratagene), to obtain pcaMKK3.

4.8 kb EcoRI-XbaI fragment of the plasmid pFASTBAC1 (CIBCOBRL) and the 1.0 kb EcoRI-XbaI fragment of the above plasmid pcaMKK 3 were ligated to make the plasmid pFBcaMKK3.

The plasmid pFBcaMKK3 and BAC-TO-BAC Baculovirus Expression System (GIBCOBRL) were used to prepare the recombinant baculovirus virusstock BAC-caMKK3.

### (3) Preparation of active form p38 MAP kinase

The Sf-21 cells were seeded on 100 mL Sf-900II SFM medium (GIBCOBRL) to 1×10⁶ cells/mL and cultured at 27°C for 24 hours. After each 0.2 mL of the virusstock BAC-HP38 and BAC-caMKK3 of recombinant baculovirus were added, the culturing was further performed for 48 hours. After the cells were separated from the culturing solution by centrifugation (3000 rpm, 10 min), the cells were washed twice with PBS. After the cells were suspended in 10 ml Lysis buffer (25 mM HEPES (pH 7.5), 1% Triton X, 130 mM NaCl, 1 mM EDTA, 1 mM DTT, 25 mM β-glycerophosphate, 20 mM leupeptin, 1 mM APMSF, 1 mM Sodium orthovanadate), the cells were lysed by treating twice in a homogenizer (POLYTRON) at 20000 rpm for 2 minutes. From the supernatant obtained by centrifugation (40000 rpm, 45 minutes), active form p38 MAP kinase was purified using Anti-FLAG M2 Affinity Gel (Eastman Chemical).

### (4) Measurement of the enzyme inhibitory activity

2.5 µL of a test compound dissolved in DMSO was added to 37.5 µL reaction solution (25 mM HEPES (pH 7.5), 10 mM Magnesium Acetate) containing 260 ng active form p38 MAP kinase and 1 µg Myelin Basic Protein, which was maintained at 30°C for 5 minutes. The reaction was initiated by adding 10 µL ATP solution (2.5 µM ATP, 0.1 µCi [g-³²P]ATP). After the reaction was performed at 30°C for 60 minutes, the reaction was stopped by adding 50 µL 20% TCA solution. After the reaction solution was allowed to stand at 0°C for 20 minutes, an acid insoluble fraction was transferred to GF/C filter (Packard Japan) using Cell Harvester (Packard Japan) and washed with 250 mM H₃PO₄. After drying at 45°C for 60 minutes, 40 µL Microscint 0 (Packard Japan) was added and the radioactivity was measured with a TopCount (Packard Japan). The concentration (IC₅₀ value) of the test compound necessary for inhibiting uptake of ³²P into an acid insoluble fraction by 50% was calculated with PRISM 2.01 (Graphpad Software). The results are shown in Table 45.

**Table 45**

| Reference Example A Compound No. | IC₅₀ (µM) |
|---|---|
| 13-14 | 0.086 |
| 13-15 | 0.081 |
| 13-16 | 0.060 |
| 13-70 | 0.026 |
| 13-74 | 0.63 |

### Reference Example C 2

### Measurement of inhibiting activity of TNF-α production

After THP-1 cells which had been cultured on PRMI 1640 medium (manufactured by Life Technologies, Inc.) containing 1% inactivated bovine fetal serum (manufactured by Life Technologies, Inc., U.S.A.) and 10 mM HEPES (pH 7.5) seeded on a 96-well plate to 1×10⁵ cells/well, 1 µL test compound dissolved in DMSO was added. After incubation at 37°C for 1 hour in a CO₂ incubator, LPS (Wako Pure Chemicals) was added to the final concentration 5 µg/mL. After cultured at 37°C for 4 hours in a CO₂ incubator, the supernatant was obtained by centrifugation. The concentration of TNF-α in the supernatant was measured by ELISA (R&D Systems, Quantikine Kit). The concentration (IC₅₀ value) of the test compound necessary for inhibiting TNF-α production by 50% was calculated using PRIMS 2.01 (Graphpad Software). The results are shown in Table 46.

**Table 46**

| Reference Example A Compound No. | IC₅₀ (µM) |
|---|---|
| 13-16 | 0.14 |
| 13-70 | 0.18 |
| 23-60 | 0.046 |

From the above results, it can be seen that Compound (I) has an excellent inhibitory activity against p38 MAP kinase and TNF-α production.

The following Reference Example D can be produced according to Examples of WO00/64894.
**Reference Example D 1:** [4-(3,5-dimethylphenyl)-5-(2-phenylmethyloxy-4-pyridyl)-1,3-thiazol-2-yl]amine
**Reference Example D 2:** N-[4-[2-benzoylamino-4-(4-methoxyphenyl)-1,3-thiazol-5-yl]-2-pyridyl]benzamide
**Reference Example D 3:** N-[4-(4-methoxypheny)-5-[2-[(3-pyridylcarbonylamino)]-4-pyridyl]-1,3-thiazol-2-yl]nicotinamide
**Reference Example D 4:** N-[4-[2-amino-4-(4-methoxyphenyl)-1,3-thiazol-5-yl]-2-pyridyl]benzamide
**Reference Example D 5:** N-[4-[2-amino-4-(3,5-dimethylphenyl)-1,3-thiazol-5-yl]-2-pyridyl]benzamide
**Reference Example D 6:** N-[4-[2-amino-4-(3,5-dimethylphenyl)-1,3-thiazol-5-yl]-2-pyridyl]benzylamine
**Reference Example D 7:** N-[4-[2-amino-4-(3,5-dimethylphenyl)-1,3-thiazol-5-yl]-2-pyridyl]benzamide hydrochloride
**Reference Example D 8:** N-[4-[2-amino-4-(3,5-dimethylphenyl)-1,3-thiazol-5-yl]-2-pyridyl]benzylamine dihydrochloride
   The structures of the compounds obtained in Reference Examples D 1 to 6 are shown below:
   Reference Example D 1 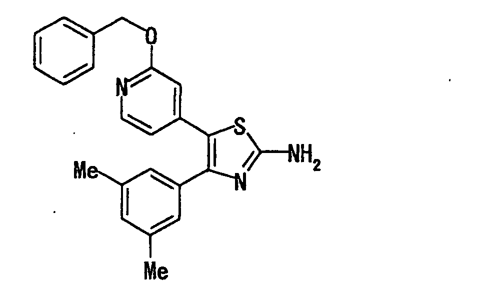
   Reference Example D 2 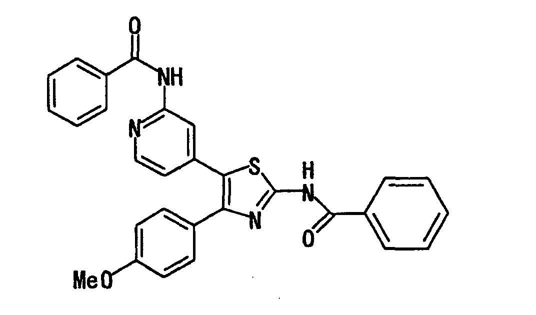
   Reference Example D 3 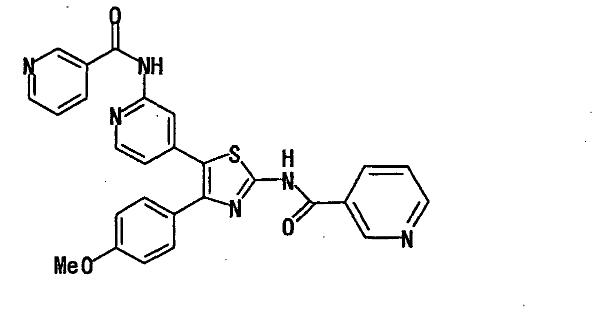
   Reference Example D 4 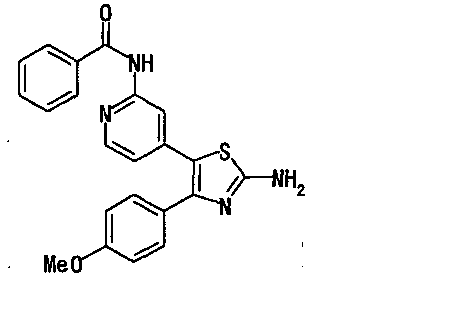
   Reference Example D 5 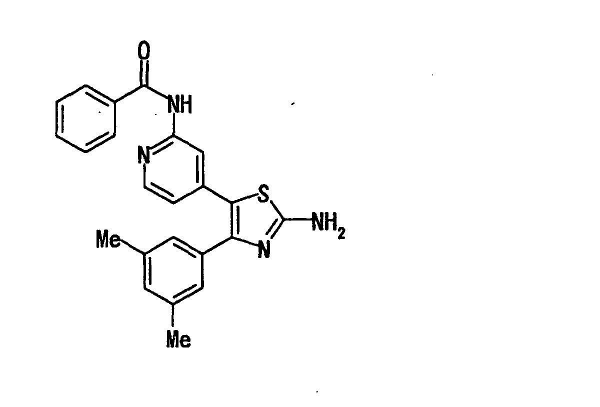
   Reference Example D 6 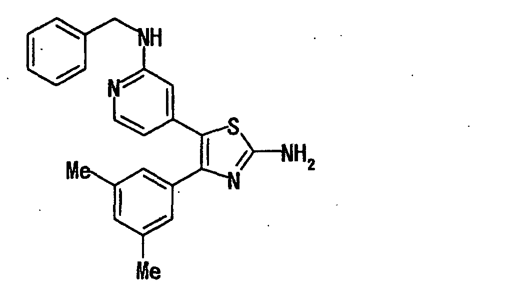
**Reference Example D 9:** N-[5-[2-benzoylamino-4-pyridyl)-4-(3,5-dimethylphenyl)-1,3-thiazol-2-yl]acetamide
**Reference Example D 10:** N-[5-(2-benzylamino-4-pyridyl)-4-(3,5-dimethylphenyl)-1,3-thiazol-2-yl]acetamide
**Reference Example D 11:** N-[4-[4-(4-methoxyphenyl)-2-methylamino-1,3-thiazol-5-yl]-2-pyridyl]benzamide
**Reference Example D 12:** N-[4-[2-amino-4-(3-methylphenyl)-1,3-thiazol-5-yl]-2-pyridyl]benzamide
**Reference Example D 13:** N-[4-[4-(4-methoxyphenyl)-2-methyl-1,3-thiazol-5-yl]-2-pyridyl]benzamide
**Reference Example D 14:** N-[4-[2-[(4-fluorophenyl)-4-(3-methylphenyl)-1,3-thiazol-5-yl]-2-pyridyl]phenylacetamide
**Reference Example D 15:**
   Reference Example D compound 15-1: N-[4-[4-(4-methoxyphenyl)-2-methyl-1,3-thiazol-5-yl]-2-pyridyl]phenylacetamide
   Reference Example D compound 15-2: N-[4-[2-ethyl-4-(3-methylphenyl)-1,3-thiazol-5-yl]-2-pyridyl]phenylacetamide
   Reference Example D compound 15-3: N-[4-[4-(3-methylphenyl)-2-propyl-1,3-thiazol-5-yl]-2-pyridyl]phenylacetamide
   Reference Example D compound 15-4: N-[4-[2-butyl-4-(3-methylphenyl)-1,3-thiazol-5-yl]-2-pyridyl]phenylacetamide
   Reference Example D compound 15-5: N-[4-[2-(2-chlorophenyl)-4-(3-methylphenyl)-1,3-thiazol-5-yl]-2-pyridyl]phenylacetamide
   Reference Example D compound 15-6: N-[4-[4-(3-methylphenyl)-2-(4-methylthiophenyl)-1,3-thiazol-5-yl]-2-pyridyl]phenylacetamide
   **Reference Example D 16:**
      Reference Example D compound 16-1: N-[4-[2-ethyl-4-(3-methylphenyl)-1,3-thiazol-5-yl]-2-pyridyl]benzamide
      Reference Example D compound 16-2: N-[4-[2-ethyl-4-(3-methylphenyl)-1,3-thiazol-5-yl]-2-pyridyl]-3-phenylpropionamide
      Reference Example D compound 16-3: N-[4-[2-ethyl-4-(3-methylphenyl)-1,3-thiazol-5-yl]-2-pyridyl]-3-(4-methoxyphenyl)propionamide
      Reference Example D compound 16-4: N-[4-[2-ethyl-4-(3-methylphenyl)-1,3-thiazol-5-yl]-2-pyridyl]-3-(4-fluorophenyl)propionamide
      Reference Example D compound 16-5: N-[4-[2-ethyl-4-(3-methylphenyl)-1,3-thiazol-5-yl]-2-pyridyl]-4-phenylbutyramide
      Reference Example D compound 16-6: N-[4-[2-ethyl-4-(3-methylphenyl)-1,3-thiazol-5-yl]-2-pyridyl]-5-phenylvaleramide
      Reference Example D compound 16-7: N-[4-[4-(3-methylphenyl)-2-propyl-1,3-thiazol-5-yl]-2-pyridyl]benzamide
      Reference Example D compound 16-8: N-[4-[4-(3-methylphenyl)-2-propyl-1,3-thiazol-5-yl]-2-pyridyl]-3-phenylpropionamide
      Reference Example D compound 16-9: N-[4-[2-butyl-4-(3-methylphenyl)-1,3-thiazol-5-yl]-2-pyridyl]benzamide
      Reference Example D compound 16-10: N-[4-[2-butyl-4-(3-methylphenyl)-1,3-thiazol-5-yl]-2-pyridyl]-3-phenylpropionamide
      Reference Example D compound 16-11: N-[4-[2-(4-fluorophenyl)-4-(3-methylphenyl)-1,3-thiazol-5-yl]-2-pyridyl]benzamide
      Reference Example D compound 16-12: N-[4-[2-(4-fluorophenyl)-4-(3-methylphenyl)-1,3-thiazol-5-yl]-2-pyridyl]-3-phenylpropionamide
      Reference Example D compound 16-13: N-[4-[2-(2-chlorophenyl)-4-(3-methylphenyl)-1,3-thiazol-5-yl]-2-pyridyl]benzamide Reference Example D compound 16-14: N-[4-[2-(2-chlorophenyl)-4-(3-methylphenyl)-1,3-thiazol-5-yl]-2-pyridyl]-3-phenylpropionamide
      Reference Example D compound 16-15: N-[4-[4-(3-methylphenyl)-2-(4-methylthiophenyl)-1,3-thiazol-5-yl]-2-pyridyl]benzamide
      Reference Example D compound 16-16: N-[4-[4-(3-methylphenyl)-2-(4-methylthiophenyl)-1,3-thiazol-5-yl]-2-pyridyl]-3-phenylpropionamide
      Reference Example D compound 16-17: N-[4-[4-(3-methylphenyl)-2-(4-methylthiophenyl)-1,3-thiazol-5-yl]-2-pyridyl]-2-thiophenecarboxamide
      Reference Example D compound 16-18: N-[4-[4-(3-methylphenyl)-2-(4-methylthiophenyl)-1,3-thiazol-5-yl]-2-pyridyl]-2-naphthamide
   **Reference Example D 17:** N-[4-[2-ethyl-4-(3-methylphenyl)-1,3-thiazol-5-yl]-2-pyridyl]-N-methylphenylacetamide
   **Reference Example D 18:** N-[4-[2-ethyl-4-(3-methylphenyl)-1,3-thiazol-5-yl]-2-pyridyl]-N-methyl-3-phenylpropionamide
   **Reference Example D 19:**
      Reference Example D compound 19-1: N-benzyl-N-[4-[4-(4-methoxyphenyl)-2-methyl-1,3-thiazol-5-yl]-2-pyridyl]amine
      Reference Example D compound 19-2: N-benzyl-N-[4-[2-ethyl-4-(3-methylphenyl)-1,3-thiazol-5-yl]-2-pyridyl]amine
      Reference Example D compound 19-3: N-[4-[2-ethyl-4-(3-methylphenyl)-1,3-thiazol-5-yl]-2-pyridyl]-N-(2-phenylethyl)amine
      Reference Example D compound 19-4: N-[4-[2-ethyl-4-(3-methylphenyl)-1,3-thiazol-5-yl]-2-pyridyl]-N-(3-phenylpropyl)amine
      Reference Example D compound 19-5: N-benzyl-N-[4-[4-(3-methylphenyl)-2-propyl-1,3-thiazol-5-yl]-2-pyridyl]amine
      Reference Example D compound 19-6: N-[4-[4-(3-methylphenyl)-2-propyl-1,3-thiazol-5-yl]-2-pyridyl]-N-(2-phenylethyl)amine
      Reference Example D compound 19-7: N-[4-[4-(3-methylphenyl)-2-propyl-1,3-thiazol-5-yl]-2-pyridyl]-N-(3-phenylpropyl)amine
      Reference Example D compound 19-8: N-benzyl-N-[4-[2-butyl-4-(3-methylphenyl)-1,3-thiazol-5-yl]-2-pyridyl]amine
      Reference Example D compound 19-9: N-[4-[2-butyl-4-(3-methylphenyl)-1,3-thiazol-5-yl]-2-pyridyl]-N-(2-phenylethyl)amine
      Reference Example D compound 19-10: N-[4-[2-butyl-4-(3-methylphenyl)-1,3-thiazol-5-yl]-2-pyridyl]-N-(3-phenylpropyl)amine
      Reference Example D compound 19-11: N-benzyl-N-[4-[2-(4-fluorophenyl)-4-(3-methylphenyl)-1,3-thiazol-5-yl]-2-pyridyl]amine
      Reference Example D compound 19-12: N-[4-[2-(4-fluorophenyl)-4-(3-methylphenyl)-1,3-thiazol-5-yl]-2-pyridyl]-N-(2-phenylethyl)amine
      Reference Example D compound 19-13: N-[4-[2-(4-fluorophenyl)-4-(3-methylphenyl)-1,3-thiazol-5-yl]-2-pyridyl]-N-(3-phenylpropyl)amine
      Reference Example D compound 19-14: N-benzyl-N-[4-[2-(2-chlorophenyl)-4-(3-methylphenyl)-1,3-thiazol-5-yl]-2-pyridyl]amine
      Reference Example D compound 19-15: N-[4-[2-(2-chlorophenyl)-4-(3-methylphenyl)-1,3-thiazol-5-yl]-2-pyridyl]-N-(2-phenylethyl)amine
      Reference Example D compound 19-16: N-[4-[2-(2-chlorophenyl)-4-(3-methylphenyl)-1,3-thiazol-5-yl]-2-pyridyl]-N-(3-phenylpropyl)amine
      Reference Example D compound 19-17: N-benzyl-N-[4-[4-(3-methylphenyl)-2-(4-methylthiophenyl)-1,3-thiazol-5-yl]-2-pyridyl]amine
      Reference Example D compound 19-18: N-[4-[4-(3-methylphenyl)-2-(4-methylthiophenyl)-1,3-thiazol-5-yl]-2-pyridyl]-N-(2-phenylethyl)amine
      Reference Example D compound 19-19: N-[4-[4-(3-methylphenyl)-2-(4-methylthiophenyl)-1,3-thiazol-5-yl]-2-pyridyl]-N-(3-phenylpropyl)amine
      Reference Example D compound 19-20: N-[4-[4-(3-methylphenyl)-2-(4-methylthiophenyl)-1,3-thiazol-5-yl]-2-pyridyl]-N-(2-naphthylmethyl)amine
   **Reference Example D 20:** N-[4-[4-(3-methylphenyl)-2-(4-methylsulfonylphenyl)-1,3-thiazol-5-yl]-2-pyridyl]benzamide
   **Reference Example D 21:**
      Reference Example D compound 21-1: N-[4-[4-(3-methylphenyl)-2-(4-methylsulfonylphenyl)-1,3-thiazol-5-yl]-2-pyridyl]phenylacetamide
      Reference Example D compound 21-2: N-[4-[4-(3-methylphenyl)-2-(4-methylsulfonylphenyl)-1,3-thiazol-5-yl]-2-pyridyl]-3-phenylpropionamide
      Reference Example D compound 21-3: N-[4-[4-(3-methylphenyl)-2-(4-methylsulfonylphenyl)-1,3-thiazol-5-yl]-2-pyridyl]-2-thiophenecarboxamide
      Reference Example D compound 21-4: N-[4-[4-(3-methylphenyl)-2-(4-methylsulfonylphenyl)-1,3-thiazol-5-yl]-2-pyridyl]-2-naphthamide
      Reference Example D compound 21-5: N-benzyl-N-[4-[4-(3-methylphenyl)-2-(4-methylsulfonylphenyl)-1,3-thiazol-5-yl]-2-pyridyl]amine
      Reference Example D compound 21-6: N-[4-[4-(3-methylphenyl)-2-(4-methylsulfonylphenyl)-1,3-thiazol-5-yl]-2-pyridyl]-N-(3-phenylpropyl)amine
      Reference Example D compound 21-7: N-[4-[4-(3-methylphenyl)-2-(4-methylsulfonylphenyl)-1,3-thiazol-5-yl]-2-pyridyl]-N-(2-naphthylmethyl)amine
   **Reference Example D 22:** N-[4-[2-amino-4-(3-methylphenyl)-1,3-thiazol-5-yl]-2-pyridyl]-N-benzylamine
   **Reference Example D 23:**
      Reference Example D compound 23-1: N-[4-[2-amino-4-(3-methylphenyl)-1,3-thiazol-5-yl]-2-pyridyl]-N-(4-methoxybenzyl)amine
      Reference Example D compound 23-2: N-[4-[2-amino-4-(3-methylphenyl)-1,3-thiazol-5-yl]-2-pyridyl]-N-(3-methoxybenzyl)amine
      Reference Example D compound 23-3: N-[4-[2-amino-4-(3-methylphenyl)-1,3-thiazol-5-yl]-2-pyridyl]-N-(2-methoxybenzyl)amine
      Reference Example D compound 23-4: N-[4-[2-amino-4-(3-methylphenyl)-1,3-thiazol-5-yl]-2-pyridyl]-N-(4-chlorobenzyl)amine
      Reference Example D compound 23-5: N-[4-[2-amino-4-(3-methylphenyl)-1,3-thiazol-5-yl]-2-pyridyl]-N-(3-chlorobenzyl)amine
      Reference Example D compound 23-6: (R)-N-[4-[2-amino-4-(3-methylphenyl)-1,3-thiazol-5-yl]-2-pyridyl]-N-(1-phenylethyl)amine
      Reference Example D compound 23-7: (S)-N-[4-[2-amino-4-(3-methylphenyl)-1,3-thiazol-5-yl]-2-pyridyl]-N-(1-phenylethyl)amine
      Reference Example D compound 23-8: N-[4-[2-amino-4-(3-methylphenyl)-1,3-thiazol-5-yl]-2-pyridyl]-N-benzyl-N-methylamine
   **Reference Example D 24:** N-[4-[2-amino-4-(3-methoxyphenyl)-1,3-thiazol-5-yl]-2-pyridyl]-N-benzylamine
   **Reference Example D 25:**
      Reference Example D compound 25-1: N-[4-[4-(3-methylphenyl)-2-(4-methylsulfonylphenyl)-1,3-thiazol-5-yl]-2-pyridyl]-N-(2-phenylethyl)amine
      Reference Example D compound 25-2: N-(4-fluorobenzyl)-N-[4-[4-(3-methylphenyl)-2-(4-methylsulfonylphenyl)-1,3-thiazol-5-yl]-2-pyridyl]amine
      Reference Example D compound 25-3: N-benzyl-N-methyl-N-[4-[4-(3-methylphenyl)-2-(4-methylsulfonylphenyl)-1,3-thiazol-5-yl]-2-pyridyl]amine
      Reference Example D compound 25-4: N-methyl-N-[4-[4-(3-methylphenyl)-2-(4-methylsulfonylphenyl)-1,3-thiazol-5-yl]-2-pyridyl]-N-(2-phenylethyl)amine
      Reference Example D compound 25-5: N-[4-[4-(3-methylphenyl)-2-(4-methylsulfonylphenyl)-1,3-thiazol-5-yl]-2-pyridyl]-N-(2-thienylmethyl)amine
   **Reference Example D 26:** 4-(3-methylphenyl)-2-(4-methylsulfonylphenyl)-5-(2-phenylthio-4-pyridyl)-1,3-thiazole
   **Reference Example D 27:** 5-(2-benzylthio-4-pyridyl)-4-(3-methylphenyl)-2-(4-methylsulfonylphenyl)-1,3-thiazole
   **Reference Example D 28:** 4-(3-methylphenyl)-2-(4-methylsulfonylphenyl)-5-(2-phenylsulfonyl-4-pyridyl)-1,3-thiazole

Compounds prepared in the above Reference Examples D 9 to 28 are shown in Tables 47 to 52.

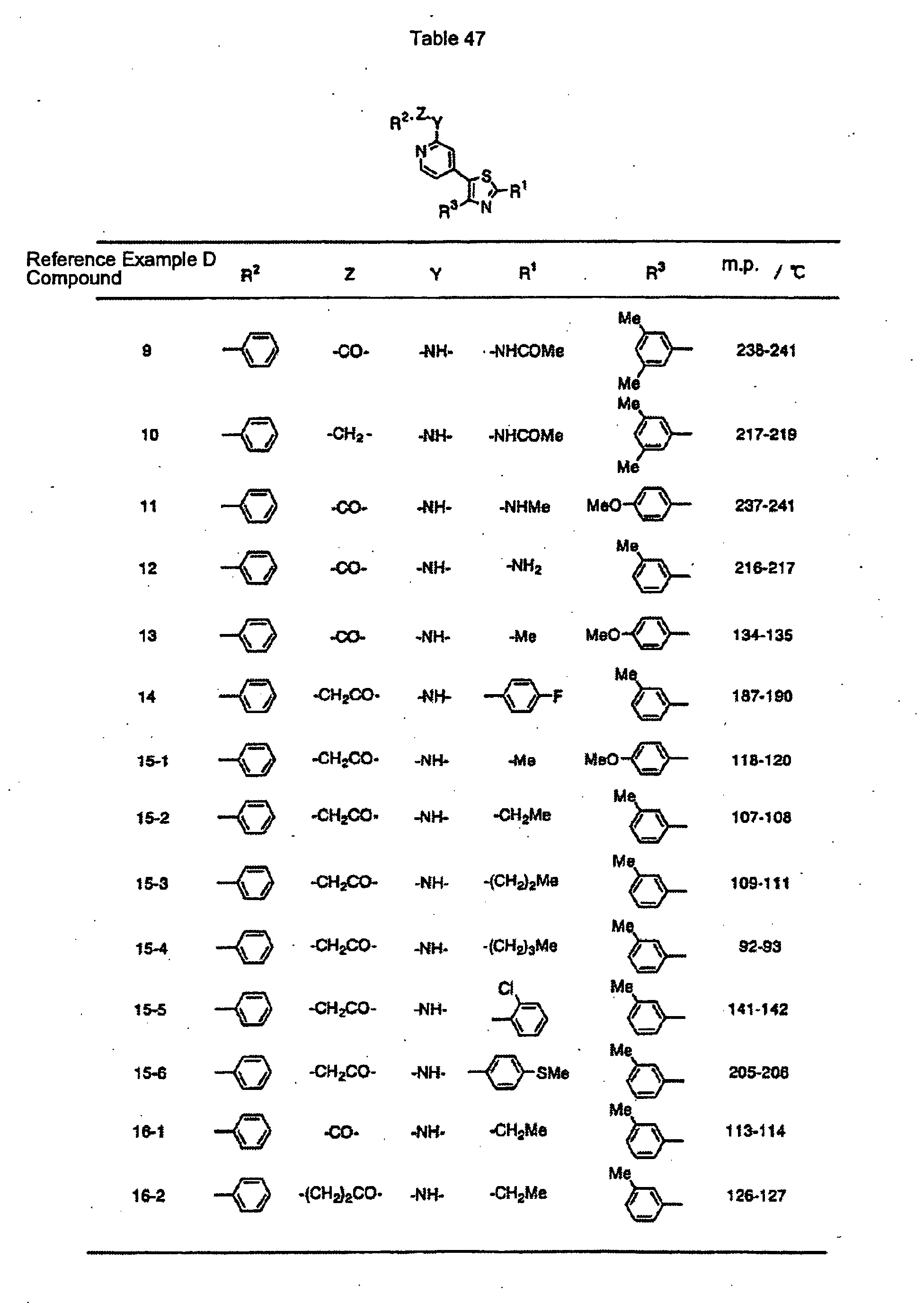

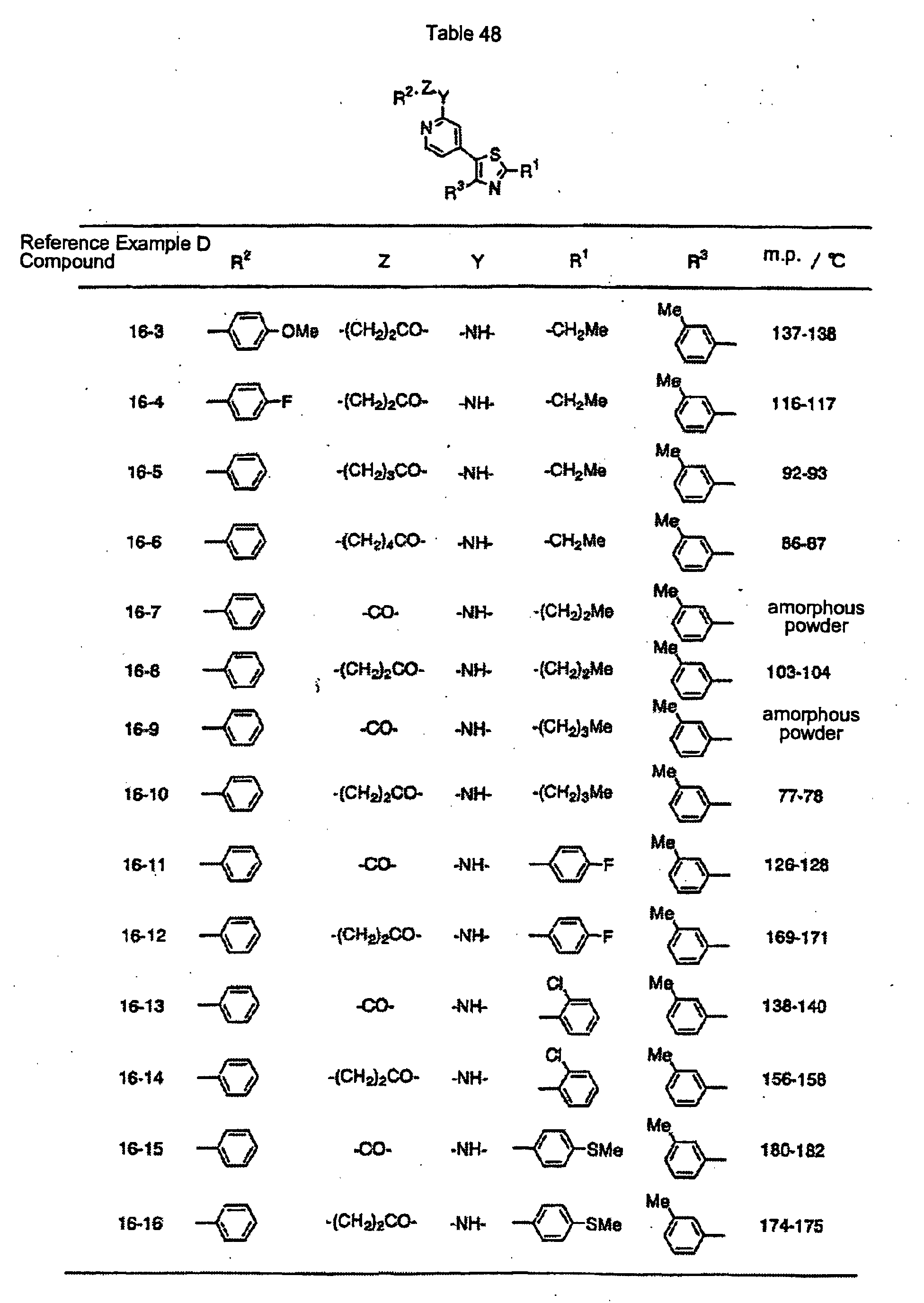

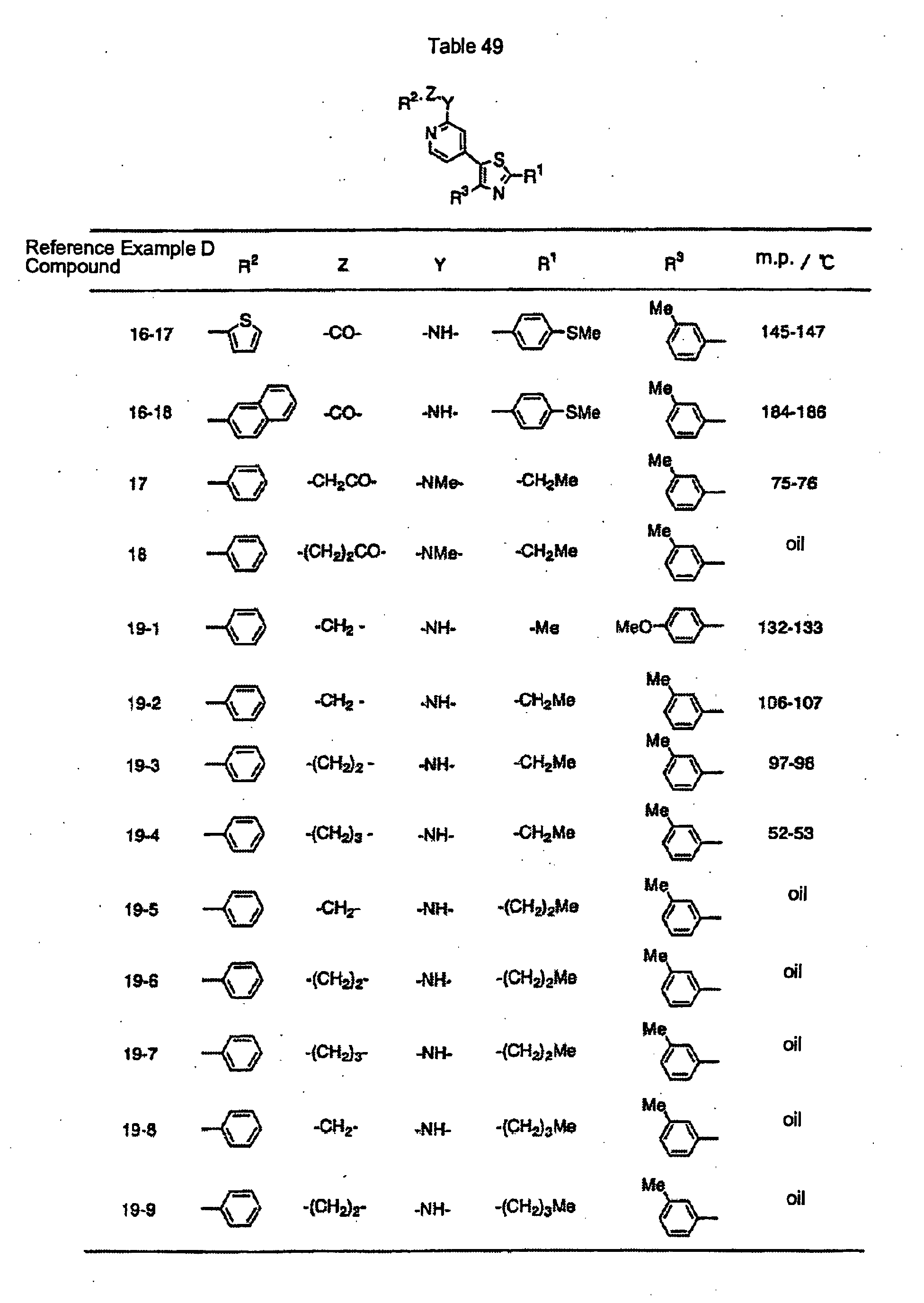

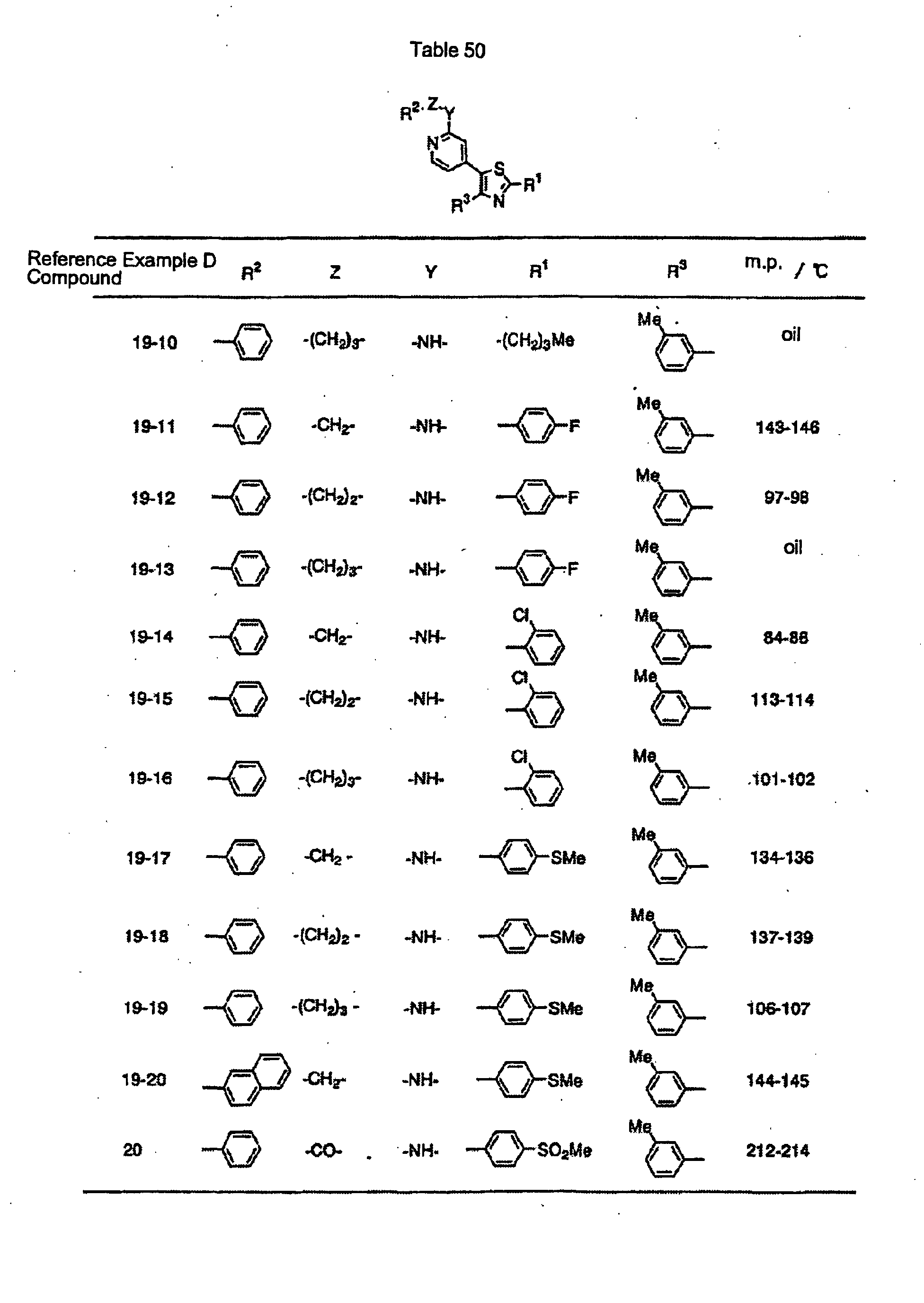

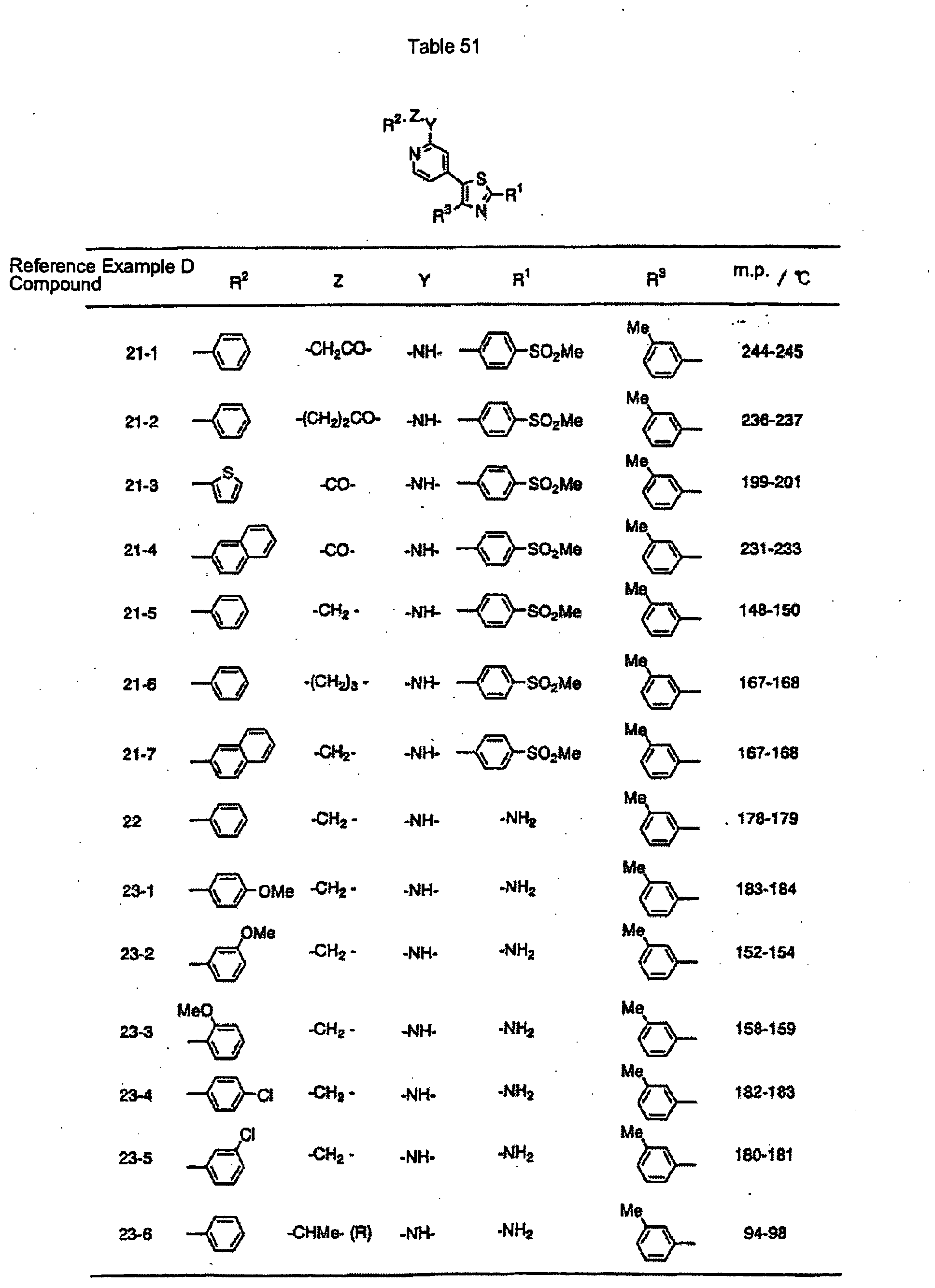

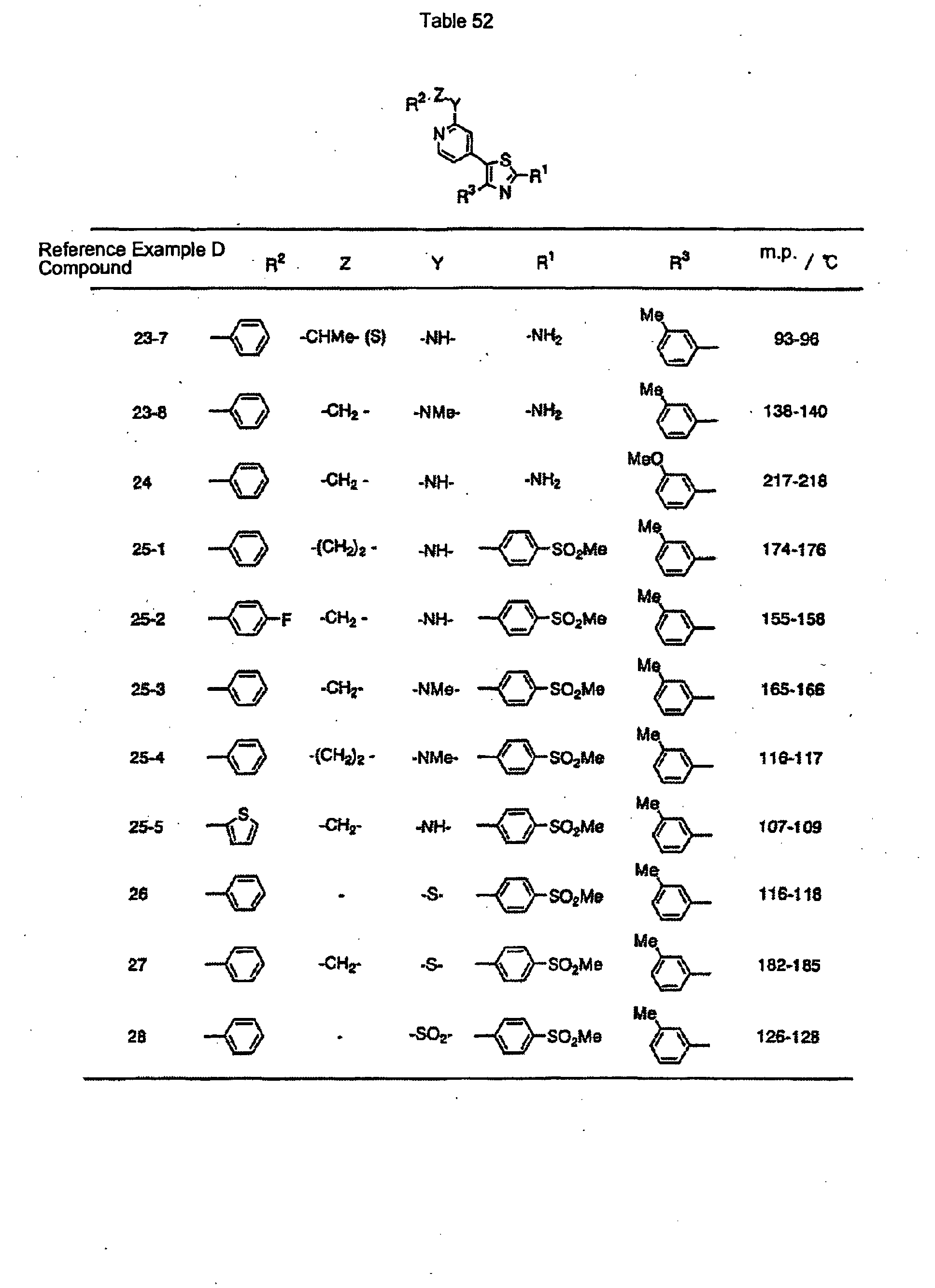

### Reference Example E 1:

| | |
|---|---|
| (1) Compound of Reference Example D 1 | 50 mg |
| (2) Lactose | 34 mg |
| (3) Corn starch | 10.6 mg |
| (4) Corn starch (pasty) | 5 mg |
| (5) Magnesium stearate | 0.4 mg |
| (6) Calcium carboxymethylcellulose | 20 mg |
| | Total 120 mg |

According to conventional methods, the above (1) to (6) were mixed, compressed with a compressing machine to obtain tablets.

### Reference Example E 2:

| | |
|---|---|
| (1) Reference Example D compound 16-1 | 10.0 mg |
| (2) Lactose | 60.0 mg |
| (3) Corn starch | 35.0 mg |
| (4) Gelatin | 3.0 mg |
| (5) Magnesium stearate | 2.0 mg |

10.0 mg of Reference Example D compound 16-1 and a mixture of 60.0 mg of lactose and 35.0 mg of corn starch were granulated by passing through a 1 mm mesh sieve using 0.03 ml of a 10% aqueous gelatin solution (3.0 mg as gelatin) and, thereafter, dried at 40°C and re-passed through a sieve. The granules thus obtained were mixed with 2.0 mg of magnesium stearate and compressed. The resulting core tablet is coated with a sugar coating of a suspension of sucrose, titanium dioxide, talc and arabic gum in water. The tablet coated with a coating is polished with beeswax to obtain a coated tablet.

### Reference Example E 3:

| | |
|---|---|
| (1) Reference Example D compound 16-1 | 10.0 mg |
| (2) Lactose | 70.0 mg |
| (3) Corn starch | 50.0 mg |
| (4) Soluble starch | 7.0 mg |
| (5) Magnesium stearate | 3.0 mg |

After 10.0 mg of Reference Example D compound 16-1 and 3.0 mg of magnesium stearate are granulated with 0.07 ml of an aqueous solution of soluble starch (7.0 mg as soluble starch), the granules are dried and mixed with 70.0 mg of lactose and 50.0 mg of corn starch. The mixture is compressed to obtain tablets.

### Reference Example E 4:

| | |
|---|---|
| (1) Reference Example D compound 18 | 5.0 mg |
| (2) Sodium chloride | 20.0 mg |
| (3) Distilled water | to total 2.0 ml |

5.0 mg of Reference Example D compound 18 and 20.0 mg of sodium chloride are dissolved in distilled water and water is added to total 2.0 ml. The solution is filtered and filled into a 2 ml of ampoule under sterile conditions. After the ampoule is sterilized, it is sealed to obtain a solution for injection.

### Reference Example F 1:

Genetic procedures were according to the methods described in Molecular Cloning, published by Cold Spring Harbor, Laboratory, 1989 or a method described in the attached protocol of the reagent.

### 1) Cloning of human adenosine A₃ receptor

Cloning of an adenosine A₃ receptor gene was performed from human brain cDNA by a PCR method. A PCR reaction was performed with a DNA thermal cycler 480 (Perkin Elmer) by using 1 ng of brain cDNA (Toyobo, QUICK-Clone cDNA) as a template, adding each 50 pmol of a primer set 5'-CGCCTCTAGACAAGATGCCCAACAACAGCACTGC-3' (SEQ ID NO:7) and 5'-CGGGGTCGACACTACTCAGAATTCTTCTCAATGC-3' (SEQ ID NO:8) made by reference to adenosine A₃ receptor gene base sequence reported by Salvatore et al. (Proc. Natl. Acad. Sci. U.S.A., 90:10365-10369, 1993) and employing Takara LA PCR Kit Ver.2 (Takara Shuzo) (reaction conditions: 35 cycles of 1 minute at 95°C, 1 minute at 66°C, 2 minutes at 75°C). The resulting PCR product was subjected to agarose gel electrophoresis and 1.0 kb of DNA fragment was recovered and, thereafter, an adenosine A₃ receptor gene was cloned using Original TA Cloning Kit (Funakoshi).

Next, the resulting plasmid was digested with a restriction enzyme XbaI (Takara Shuzo), treated with T4 DNA polymerase (Takara Shuzo) into end-blunted fragments and further digested with SalI (Takara Shuzo) to obtain adenosine A₃ receptor gene fragments.

### 2) Preparation of a plasmid for expressing of human adenosine A₃ receptor

A SRα promoter derived from pTB1411 described in JP-A 5-076385 was digested with BglII (Takara Shuzo), blunted, and ligated to EcoRI (Takara Shuzo)-digested pCI vector (Promega) with a DNA Ligation kit (Takara Shuzo) to make pCI-SRα. Next, this pCI-SRα was digested with ClaI (Takara Shuzo) and treated with T4 DNA polymerase (Takara Shuzo) to blunt-ended. On the other hand, after pGFP-C1 (Toyobo) was digested with Bsu36I (Daiichi Pure Chemicals), treated with T4 DNA polymerase (Takara Shuzo) to blunted end to obtain 1.63 kb of DNA fragment, and both were ligated with a DNA Ligation kit (Takara Shuzo) and competent cells of Escherichia coli JM109 were transformed to obtain the plasmid pMSRαneo.

Next, after pMSRαneo was digested with EcoRI (Takara Shuzo), treated with a T4 DNA polymerase (Takara Shuzo) to blunted end, and further digesting with SalI (Takara Shuzo) to obtain a 5.4 kb DNA fragment. The obtained DNA fragment and the fragments of adenosine A₃ receptor gene obtained in the above 1) were mixed, ligated with a DNA Ligation kit (Takara Shuzo) and competent cells of Escherichia coli JM109 (Takara Shuzo) were transformed to obtain the plasmid pA₃SR_{α}.

### 3) Introduction of a plasmid for expressing human adenosine A₃ receptor into CHO (dhfr-) cells and expression

CHO (dhfr-) cells obtained by culturing on Ham F12 medium (Nihonseiyaku) containing 10% bovine fetal serum (Lifetec Oriental) in a 750 ml tissue culture flask (Vecton Dickinson) were peeled with 0.5 g/L trypsin-0.2 g/L EDTA (Lifetec Oriental) and, thereafter, the cells were washed with PBS (Lifetec Oriental) and centrifuged (1000 rpm, 5 minutes), which was suspended in PBS.

Next, a DNA was introduced into cells using a gene pulser (BioRad) according to the following conditions. That is, 8×10⁶ cells and 10 µg of the plasmid pA₃SRα for expressing human adenosine A₃ receptor were added to 0.4 cm gapped cuvette and electroporation was performed with 0.8 ml volume, and under voltage 0.25 kV and capacitance 960 µF. Thereafter, the cells were transferred to Ham F12 medium containing 10% bovine fetal serum, cultured for 24 hours, the cells were peeled again and centrifuged, then, suspended in Ham F12 medium containing 10% bovine fetal serum to which Geneticin (Lifetec Oriental) had been added to 500 µg/ml, which was diluted to 10⁴ cells/ml to seed on a 96-well plate (Becton Dickinson) to obtain Geneticin-resistant strain.

Next, the resulting Geneticin-resistant strain was cultured on a 24 well-plate (Becton Dickinson) and, thereafter, an adenosine A₃ receptor expressing cell was selected among the resistant strains. That is, a reaction was conducted in an assay buffer I (HBSS (Wako Pure Chemicals) containing 0.1% BSA, 0.25 mM PMSF, 1µg/ml pepstatin and 20 µg/ml leupeptin) for 1 hour, washed with an assay buffer I, the radioactivity was measured with a γ-counter to select a cell to which a ligand is specifically bound, A₃AR/CHO strain.

### 4) Preparation of a cell membrane fraction of a cell for expressing adenosine A₃ receptor

After the A₃AR/CHO strain obtained in the above 3) was cultured in Ham F12 medium containing 10% bovine fetal serum for 2 days, the cells were peeled with 0.02% EDTA-containing PBS, the cells were recovered by centrifugation, suspended in an assay buffer II (50 mM Tris-hydrochloric acid (pH 7.5), 1mM EDTA, 10 mM magnesium chloride, 0.25 mM PMSF, 1 µg/mL pepstatin, 20 µg/ml leupeptin), and the cells were lysed by treating three times with a polytron homogenizer (Model PT-3000, KINEMATICA AG) at 20,000 rpm for 20 seconds. After the cells were ground, they were centrifuged at 20,000 rpm for 10 minutes to obtain the supernatant containing the membrane fraction. This supernatant was centrifuged with a supercentrifuge (Model L8-70M, rotor 70Ti, Beckmann) at 30,000 rpm for 1 hour to obtain the precipitates containing the membrane fraction.

Next, the precipitates were suspended in an assay buffer II containing 2 unit/ml adenosine deaminase (Boehringer Mannheim), treated at 30°C for 30 minutes and, thereafter, centrifuged again as described above to obtain the precipitates containing the membrane fraction.

### 5) Adenosine A₃ receptor binding test

On a 96 well-microplate, [³H]-NECA (Amersham) as a ligand was added to an assay buffer II containing the 100 µg/ml membrane fraction obtained in the above 4) and various concentrations of test compounds so that the concentration of the ligand was 10 nM, followed by reaction at room temperature for 1 hour. Then, the membrane fraction was transferred to unifilter GF/C (Packard) by filtering the reaction solution using Cell Harvester (Packard) and washed three times with 50 mM cooled Tris buffer (pH 7.5). After the filter was dried, Microscint 0 (Packard) was added to the filter, the radioactivity was measured with a TopCount (Packard) and the concentration (IC₅₀) of a test compound necessary for decreasing an amount of binding of [³H]-NECA to the membrane fraction by 50% was calculated with PRISM 2.01 (Graphpad Software).

As the result, the IC₅₀ value of the compound of Example 1 was 11.6 nM. It can be seen that Compound (I) is the excellent affinity for adenosine A₃ receptor.

### Reference Example F 2:

The genetic manipulations described below were according to the methods described in the book (Maniatis et al., Molecular Cloning, Cold Spring Harbor Laboratory, 1989) or a method described in the protocol attached to the reagent.

### (1) Cloning of human p38 MAP kinase gene and preparation of recombinant Baculovirus

Cloning of human p38 MAP kinase gene was performed by a PCR method using a primer set P38-U:5'-ACCACTCGAGATGGACTACAAGGACGACGATGACAAGTCTCAGGAGAGGCCCACGTTCTACC - 3' [SEQ ID NO:9] and PAG-L:5'-ACCCGGTACCACCAGGTGCTCAGGACTCCATCTCT-3' [SEQ ID NO:10] made by reference to the base sequence of p38 MAP kinase gene reported by Han et al. (Science 265 (5173), 808-811 (1994)) and employing kidney cDNA (Toyobo, QUICK-Clone cDNA) as a template.

A PCR reaction was performed by a Hot Start method using AmpliWax PCR Gem 100 (Takara Shuzo). As the lower mixed solution, 2 µL 10×LA PCR Buffer, 3 µL 2.5 mM dNTP solution, each 2.5 µL of 12.5 µM primer solution, and 10 µL sterile distilled water were mixed. As the upper mixed solution, 1 µL human cardiac cDNA (1 ng/mL) as a template, 3 µL 10×LA PCR Buffer, 1 µL 2.5 mM dNTP solution, 0.5 µL TaKaRa LA Taq DNA polymerase (Takara Shuzo), and 24.5 µL sterile distilled water were mixed. One AmpliWax PCR Gem 100 (Takara Shuzo) was added to the prepared lower mixed solution to treat at 70°C for 5 minutes and for 5 minutes in an ice and, thereafter, the upper mixed solution was added to prepare a reaction solution for PCR. A tube containing the reaction solution was set at a thermal cycler (Perkin Elmer), which was treated at 95°C for 2 minutes. Further, after repeating 35 times a cycle of 15 seconds at 95°C and 2 minutes at 68°C, treatment was performed at 72°C for 8 minutes. The resulting PCR product was subjected to agarose gel (1%) electrophoresis, 1.1 kb DNA fragment containing p38 MAP kinase gene was recovered from the gel and, thereafter, which was inserted into pT7Blue-T vector (Takara Shuzo) to make the plasmid pHP38.

The 4.8 kb XhoI-KpnI fragment of the plasmid pFASTBAC1 (CIBCOBRL) and the 1.1 kb XhoI-Kpn fragment of the above plasmid pHP38 were ligated to make the plasmid pFBHP38.

The plasmid pFBHP38 and BAC-TO-BAC Baculovirus Expression System (GIBCOBRL) were used to prepare the recombinant Baculovirus virusstock BAC-HP38.

### (2) Cloning of human MKK3 gene and preparation of recombinant Baculovirus

Cloning of human MKK3 gene was performed by a PCR method using a primer set MKK-U:5'-ACAAGAATTCATAACATATGGCTCATCATCATCATCATCATTCCAAGCCACCCGCACCCAA-3' [SEQ ID NO:11] and MKK-L: 5'-TCCCGTCTAGACTATGAGTCTTCTCCCAGGAT-3' [SEQ ID NO:12] made by reference to the base sequence of MKK3 gene reported by Derijard, B. et al., Science 267 (5198), 682-685 (1995) and using kidney cDNA (Toyobo, QUICK-Clone cDNA).

A PCR reaction was performed by a Hot Start method using AmpliWax PCR Gem 100 (Takara Shuzo). As the lower mixed solution, 2 µL 10×LA PCR Buffer, 3 µL 2.5 mM dNTP solution, each 2.5 µL of 12.5 µM primer solution, and 10 µL sterile distilled water were mixed. As the upper mixed solution, 1 µL human kidney cDNA (1 ng/mL), 3 µL 10×LA PCR Buffer, 1 µL 2.5 mM dNTP solution, 0.5 µL TaKaRa LA taq DNA polymerase (Takara Shuzo) and 24.5 µL sterile distilled water were mixed. One AmpliWax PCR Gem 100 (Takara Shuzo) was added to the prepared lower mixed solution to treat at 70°C for 5 minutes and for 5 minutes in ice and, thereafter, the upper mixed solution was added to prepare a reaction solution for PCR. A tube containing the reaction solution was set at a thermal cycler (Perkin Elmer), which was treated at 95°C for 2 minutes. Further, after repeating 35 times a cycle of 15 seconds at 95°C and 2 minutes at 68°C, treatment was performed at 72°C for 8 minutes. The resulting PCR product was subjected to agarose gel (1%) electrophoresis, 1.0 kb DNA fragment containing MKK3 gene was recovered from the gel and, thereafter, which was inserted into pT7Blue-T vector (Takara Shuzo) to make the plasmid pHMKK3.

In order to mutate MKK3 into a constitutive active form (from Ser to Glu at 189 position, from Thr to Glu at position 193), a primer set SER-U:5'-GGCTACTTGGTGGACGAGGTGGCCAAGGAGATGGATGCCGGCTGC-3' [SEQ ID NO:13] and SER-L:5'-GCAGCCGGCATCCATCTCCTTGGCCACCTCGTCCACCAAGTAGCC-3' [SEQ ID NO:14] was used to introduce a mutation by QuickChange Site-Directed Mutagenesis Kit (Stratagene), to obtain pcaMKK3.

4.8 kb EcoRI-XbaI fragment of the plasmid pFASTBAC1 (CIBCOBRL) and the 1.0 kb EcoRI-XbaI fragment of the above plasmid pcaMKK 3 were ligated to make the plasmid pFBcaMKK3.

The plasmid pFBcaMKK3 and BAC-TO-BAC Baculovirus Expression System (GIBCOBRL) were used to prepare the recombinant Baculovirus virusstock BAC-caMKK3.

### (3) Preparation of active form p38 MAP kinase

The Sf-21 cells were seeded on 100 ml Sf-900II SFM medium (GIBCOBRL) to 1×10⁶ cells/mL and cultured at 27°C for 24 hours. After each 0.2 mL of the virusstock BAC-HP38 of recombinant Baculovirus and BAC-caMKK3 were added, the culturing was further performed for 48 hours. After the cells were separated from the culturing solution by centrifugation (3000 rpm, 10 min), the cells were washed twice with PBS. After the cells were suspended in 10 ml Lysis buffer (25 mM HEPES (pH 7.5), 1% Triton X, 130 mM NaCl, 1 mM EDTA, 1 mM DTT, 25 mM β-glycerophosphate, 20 mM leupeptin, 1 mM APMSF, 1 mM Sodium orthovanadate), the cells were lysed by treating twice with a homogenizer (POLYTRON) at 20000 rpm for 2 minutes. By using Anti-FLAG M2 Affinity Gel (Eastman Chemical) from the supernatant obtained by centrifugation (40000 rpm, 45 minutes), active form p38 MAP kinase was purified.

### (4) Measurement of the p38 MAP kinase inhibitory activity

2.5 µL of a test compound dissolved in DMSO was added to 37.5 µL reaction solution (25 mM HEPES (pH 7.5), 10 mM Magnesium Acetate) containing 260 ng active form p38 MAP kinase and 1 µg Myelin Basic Protein, which was maintained at 30°C for 5 minutes. The reaction was initiated by adding 10 µL ATP solution (2.5 µM ATP, 0.1 µCi [g-³²P]ATP). After the reaction was performed at 30°C for 60 minutes, the reaction was stopped by adding 50 µL 20% TCA solution. After the reaction solution was allowed to stand at 0°C for 20 minutes, an acid insoluble fraction was transferred to GF/C filter (Packard Japan) using Cell Harvester (Packard Japan) and washed with 250 mM H₃PO₄. After drying at 45 °C for 60 minutes, 40 µM Microscint 0 (Packard Japan) was added and the radioactivity was measured with a TopCount (Packard Japan). The concentration (IC₅₀ value) necessary for inhibiting uptake of ³²P into an acid insoluble fraction by 50% was calculated with PRISM 2.01 (Graphpad Software).

The results are shown in Table 53.

**Table 53**

| Reference Example D No. | IC₅₀ (µM) |
|---|---|
| 1 | 0.43 |
| 2 | 0.063 |
| 3 | 0.023 |
| 4 | 0.020 |
| 5 | 0.029 |
| 6 | 0.023 |

From this, it can be seen that Compound (II) has the p38 MAP kinase inhibitory activity.

### Reference Example F 3:

### Measurement of inhibiting activity of TNF-α production

After THP-1 cells which had been cultured in PRMI 1640 medium (manufactured by Life Technologies, Inc.) containing 1% non-activated bovine fetal serum (manufactured by Life Technologies, Inc., U.S.A.) and 10 mM HEPES (pH 7.5) seeded on a 96-well plate to 1×10⁵ cells/well, 1 µL test compound dissolved in DMSO was added to there. After incubation at 37°C for 1 hour in a CO₂ incubator, LPS (Wako Pure Chemicals) was added to the final concentration 5 µg/mL. After cultured at 37°C for 4 hours in a CO₂ incubator, the supernatant was obtained by centrifugation. The concentration of TNF-α in the supernatant was measured with ELISA (R&D System, Quantikine Kit). The concentration (IC₅₀ value) necessary for inhibiting TNF-α production by 50% was calculated by PRIMS 2.01 (Graphpad Software).

The results are shown in Table 54.

**Table 54**

| Reference Example D No. | IC₅₀ (µM) |
|---|---|
| 3 | 0.026 |
| 4 | 0.014 |
| 5 | 0.020 |
| 6 | 0.140 |

From this, it can be seen that Compound (II) has the excellent inhibitory activity of TNF-α production.

### Reference Example G 1

### 1-bromo-3-ethylbenzene

To a solution of 3-ethylaniline (10.0 g, 82.5 mmol) in 50% sulfuric acid (43.6g) was added dropwise an aqueous solution (16.5 mL) of sodium nitrite (6.83 g, 99.0 mmol) over 30 minutes at 0°C. The resulting reaction mixture was stirred for 45 minutes at 0°C. This diazonium salt solution was added dropwise to a solution of copper (I) bromide (12.4g, 86.6mmol)in a 48% hydrobromic acid (82.5 mL) while heating gently under reflux. After the addition, the reaction mixture was heated to reflux for 30 minutes. The reaction mixture was cooled to room temperature, and extracted with ether. The extracts were sequentially washed with a 1N-aqueous sodium hydroxide solution and brine, and filtrated, dried, and concentrated. The residue was purified by silica gel column chromatography (hexane-ethyl acetate=20:1) to obtain the title compound (6.13g, yield 40%).
oil
¹H-NMR (CDCl₃) δ : 1.23 (3H, t, J= 7.5 Hz), 2.63 (2H, q, J= 7.5 Hz), 7.11-7.20 (2H, m), 7.28-7.38 (2H, m).

### Reference Example G 2

The following Reference Example G compound 2 was synthesized according to Reference Example G 1, using 3-(1-methylethyl)aniline instead of 3-ethylaniline.
Reference Example G compound 2: 1-bromo-3-(1-methylethyl)benzene
oil
¹H-NMR (CDCl₃) δ : 1.24 (6H, d, J= 7.0 Hz), 2.77-2.99 (1H, m), 7.03-7.16 (2H, m), 7.27-7.34 (1H, m), 7.37 (1H, s).

### Reference Example G 3

### 3-ethylbenzoic acid

Under an argon atmosphere, a solution of 1-bromo-3-ethylbenzene (5.1 g, 28 mmol) in tetrahydrofuran (45 mL) was added dropwise to a mixture of magnesium turnings (0.74 g, 31 mmol) in tetrahydrofuran (5.0 mL), and the mixture was stirred for 30 minutes under the same condition. The reaction mixture was added to crashed dry ice, and the mixture was stirred for 1 hour. To the reaction mixture was added 1N-hydrochloric acid, and extracted with ethyl acetate. The extracts were dried, filtrated and concentrated. The residue was purified by silica gel column chromatography (hexane-ethyl acetate=5:1) to obtain the title compound (3.87g, yield 93%).
oil
¹H-NMR (CDCl₃) δ : 1.28 (3H, t, J= 7.5 Hz), 2.73 (2H, q, J= 7.5 Hz), 7.34-7.50 (2H, m), 7.92-7.98 (2H, m).

### Reference Example G 4

The following Reference Example G compounds 4-1 and 4-2 were synthesized according to Reference Example G 3, using 1-bromo-3-(1-methylethyl)benzene, 1-bromo-4-fluoro-3-methylbenzene instead of 1-bromo-3-ethylbenzene.
Reference Example G compound 4-1: 3-(1-methylethyl)benzoic acid
   oil
   ¹H-NMR (CDCl₃) δ : 1.29 (6H, d, J= 7.0 Hz), 2.98-3.06 (1H, m), 7.38-7.54 (2H, m), 7.90-8.02 (2H, m).
Reference Example G compound 4-2: 4-fluoro-3-methylbenzoic acid
   m.p.: 165 - 167°C.

### Reference Example G 5

### 3-ethylbenzoyl chloride

3-Ethylbenzoic acid (9.40 g, 62.6 mmol) was added slowly to thionyl chloride (45 mL), and N,N-dimethylformamide (3 drops) was added dropwise. The resulting reaction mixture was heated under reflux for 2 hours under the same condition. The reaction mixture was concentrated, and used in the subsequent reaction without further purification.

### Reference Example G 6

The following Reference Example G compounds 6-1 to 6-4 were synthesized according to Reference Example G 5, using 3-(1-methylethyl)benzoic acid, 4-fluoro-3-methylbenzoic acid, 4-cyclohexylbenzoic acid and 3,5-dimethylbenzoic acid instead of 3-ethylbenzoic acid.
Reference Example G compound 6-1: 3-(1-methylethyl)benzoyl chloride
   This was used in the subsequent reaction without purification.
Reference Example G compound 6-2: 4-fluoro-3-methylbenzoyl chloride
   This was used in the subsequent reaction without purification.
Reference Example G compound 6-3: 4-cyclohexylbenzoyl chloride
   This was used in the subsequent reaction without purification.
Reference Example G compound 6-4: 3,5-dimethylbenzoyl chloride
   b.p. 82 - 85°C (933 Pa).

### Reference Example G 7

### N-(4-chlorobenzoyl)propyleneimine

A solution of propyleneimine (12 mL, 0.15 mol) in tetrahydrofuran (160 mL) was added to an 1 N-aqueous sodium hydroxide solution. To this mixture was added dropwise 4-chlorobenzoyl chloride (25 g, 0.14 mol) at 0°C. After completion of the addition, the mixture was further stirred for 30 minutes. The reaction mixture was extracted with ethyl acetate. The extract was dried, and the solvent was distilled off to obtain the title compound (25 g, yield 89%). oil
¹H-NMR (CDCl₃) δ : 1.39 (3H, d, J= 5.5 Hz), 2.15 (1H, d, J= 2.9 Hz), 2.51-2.66 (2H, m), 7.39-7.47 (2H, m), 7.93-8.01 (2H, m).

### Reference Example G 8

The following Reference Example G compounds 8-1 to 8-16 were synthesized according to Reference Example G 7, using 3-chlorobenzoyl chloride, 3-methylbenzoyl chloride, 3,5-dimethylbenzoyl chloride, 4-fluorobenzoyl chloride, benzoyl chloride, 3-bromobenzoyl chloride, 4-(methylthio)benzoyl chloride, 2-thiophenecarbonyl chloride, 3-propylbenzoyl chloride, 3-trifluoromethylbenzoyl chloride, 3-ethylbenzoyl chloride, 3-(1-methylethyl)benzoyl chloride, 4-fluoro-3-methylbenzoyl chloride, 3-fluorobenzoyl chloride, 3-methoxybenzoyl chloride and 4-methoxybenzoyl chloride, respectively, instead of 4-chlorobenzoyl chloride.
Reference Example G Compound 8-1: N-(3-chlorobenzoyl)propyleneimine
   oil
   ¹H-NMR (CDCl₃) δ : 1.40 (3H, d, J= 5.1 Hz), 2.17 (1H, d, J= 3.3 Hz), 2.53-2.68 (2H, m), 7.40 (1H, dd, J= 7.7, 8.1 Hz), 7.53 (1H, ddd, J= 1.5, 2.2, 8.1 Hz), 7.90 (1H, dt, J= 7.7, 1.5 Hz), 8.00 (1H, dd, J= 1.5, 2.2 Hz).
Reference Example G Compound 8-2: N-(3-methylbenzoyl)propyleneimine
   oil
   ¹H-NMR (CDCl₃) δ : 1.39 (3H, d, J= 5.5 Hz), 2.14 (1H, d, J= 3.3 Hz), 2.41 (3H, s), 2.51-2.66 (2H, m), 7.32-7.39 (2H, m), 7.79-7.87 (2H, m).
Reference Example G Compound 8-3: N-(3,5-dimethylbenzoyl)propyleneimine
   oil
   ¹H-NMR (CDCl₃) δ : 1.39 (3H, d, J= 5.5 Hz), 2.13 (1H, d, J= 3.7 Hz), 2.37 (6H, s), 2.47-2.62 (2H, m), 7.19 (1H, s), 7.64 (2H, s).
Reference Example G Compound 8-4: N-(4-fluorobenzoyl)propyleneimine
   oil
   ¹H-NMR (CDCl₃) δ : 1.39 (3H, d, J= 5.2 Hz), 2.14-2.15 (1H, m), 2.52-2.63 (2H, m), 7.08-7.19 (2H, m), 8.00-8.10 (2H, m).
Reference Example G Compound 8-5: N-benzoylpropyleneimine
   oil
   ¹H-NMR (CDCl₃) δ : 1.40 (3H, d, J= 6.0 Hz), 2.15 (1H, d, J=3.2 Hz), 2.52-2.67 (2H, m), 7.40-7.61 (3H, m), 7.98-8. 07 (2H, m).
Reference Example G Compound 8-6: N-(3-bromobenzoyl)propyleneimine
   oil
   ¹H-NMR (CDCl₃) δ : 1.40 (3H, d, J= 5.2 Hz), 2.16-2.18 (1H, m), 2.53-2.65 (2H, m), 7.34 (1H, t, J=7.9 Hz), 7.65-7.71 (1H, m), 7.95 (1H, d, J=7.9 Hz), 8.16 (1H, t, J=1.8 Hz).
Reference Example G Compound 8-7: N-[4-(methylthio)benzoyl)propyleneimine
   oil
   ¹H-NMR (CDCl₃) δ : 1.49 (3H, d, J= 6.0 Hz), 2.13 (1H, d, J=3.2 Hz), 2.49-2.60 (5H, m), 7.24-7.30 (2H, m), 7.90-7.96 (2H, m).
Reference Example G Compound 8-8: N-(2-thiophenecarbonyl)propyleneimine
   oil
   ¹H-NMR (CDCl₃) δ : 1.43 (3H, d, J= 5.2 Hz), 2.14 (1H, d, J=3.6 Hz), 2.56-2.72 (2H, m), 7.08-7.16 (1H, m), 7.53-7. 60 (1H, m) , 7.75-7.81 (1H, m).
Reference Example G Compound 8-9: N-(3-propylbenzoyl)propyleneimine
   oil
   ¹H-NMR (CDCl₃) δ : 0.95 (3H, t, J= 7.3 Hz), 1.40 (3H, d, J=4.8 Hz), 1.59-1.78 (2H, m), 2.14 (1H, d, J=2.8 Hz), 2.52-2.74 (4H, m), 7.34-7.43 (2H, m), 7.81-7.89 (2H, m).
Reference Example G Compound 8-10: N-(3-trifluoromethylbenzoyl)propyleneimine
   oil
   ¹H-NMR (CDCl₃) δ : 1.42 (3H, d, J= 5.5 Hz), 2.20 (1H, d, J=3.3 Hz), 2.56-2.67 (2H, m), 7.61 (1H, t, J=7.7 Hz), 7.81 (1H, d, J=7.7 Hz), 8.21 (1H, d, J=7.7 Hz), 8.30 (1H, s).
Reference Example G Compound 8-11: N-(3-ethylbenzoyl)propyleneimine
   oil
   ¹H-NMR (CDCl₃) δ : 1.27 (3H, t, J= 7.5 Hz), 1.40 (3H, d, J=5.5 Hz), 2.14 (1H, d, J=2.9 Hz), 2.52-2.61 (2H, m), 2.71 (2H, q, J=7.5 Hz), 7.32-7.41 (2H, m), 7.81-7.89 (2H, m).
Reference Example G Compound 8-12: N-[3-(1-methylethyl)benzoyl]propyleneimine
   oil
   ¹H-NMR (CDCl₃) δ : 1.29 (6H, t, J= 7.0 Hz), 1.40 (3H, d, J=5.9 Hz), 2.14 (1H, d, J=3.7 Hz), 2.51-2.64 (2H, m), 2.87-3.10 (1H, m), 7.33-7.46 (2H, m), 7.84 (1H, dt, J=7.0, 1.8 Hz). 7.91 (1H, s).
Reference Example G Compound 8-13: N-(4-fluoro-3-methylbenzoyl)propyleneimine
   oil
   ¹H-NMR (CDCl₃) δ : 1.39 (3H, d, J= 5.4 Hz), 2.14 (1H, d, J=3.4 Hz), 2.33 (3H, s), 2.51-2.61 (2H, m), 7.06 (1H, t, J=8.8 Hz), 7.81-7.90 (2H, m).
Reference Example G Compound 8-14: N-(3-fluorobenzoyl)propyleneimine
   oil
   ¹H-NMR (CDCl₃) δ : 1.40 (3H, d, J= 5.5 Hz), 2.16 (1H, d, J=3.3 Hz), 2.52-2.68 (2H, m), 7.25 (1H, ddd, J=1.1, 2.6, 8.4 Hz), 7.43 (1H, ddd, J=5.5, 7.7, 8.1 Hz), 7.69 (1H, ddd, J=1.5, 2.6, 8.1 Hz), 7.81 (1H, ddd, J=1.1, 1.5, 7.7 Hz).
Reference Example G Compound 8-15: N-(3-methoxybenzoyl)propyleneimine
   oil
   ¹H-NMR (CDCl₃) δ : 1.40 (3H, d, J= 5.9 Hz), 2.14 (1H, d, J=2.9 Hz), 2.52-2.65 (2H, m), 3.86 (3H, s), 7.10 (1H, ddd, J=1.1, 2.6, 8.4 Hz), 7.37 (1H, dd, J=8.4, 7.3 Hz), 7.55 (1H, dd, J=1.5, 2.6 Hz), 7.63 (1H, ddd, J=1.1, 1.5, 7.3 Hz).
Reference Example G Compound 8-16: N-(4-methoxyphenyl)propyleneimine
   oil
   ¹H-NMR (CDCl₃) δ : 1.39 (3H, d, J= 5.9 Hz), 2.11 (1H, d, J=3.3 Hz), 2.50-2.63 (2H, m), 3.87 (3H, s), 6.94 (2H, d, J=9.2 Hz), 8.00 (1H, d, J=9.2 Hz).

### Reference Example G 9

### 2-fluoro-4-methylpyridine

This was synthesized according to a method described in Journal of Medicinal Chemistry, 33, 1667-1675, 1990.
m.p.: 82 - 86°C (10 kPa).

### Reference Example G 10

### 2-phenylmethyloxy-4-methylpyridine

Sodium hydride (60% paraffin dispersion, 5.0 g, 120 mmol) was washed with hexane (5 mL) twice, and suspended in tetrahydrofuran (200 mL). To this suspension was added dropwise a solution of benzyl alcohol (14 g, 120 mol) in tetrahydrofuran (50 mL) at 0°C, and the mixture was allowed to warm to room temperature and stirred for 15 minutes. To this solution was added a solution of 2-bromo-4-methylpyridine (20 mL, 110 mol) in tetrahydrofuran (50 mL), and the mixture was heated to reflux for 14 hours. Water (200 mL) was added to the reaction mixture, and extracted with ethyl acetate. The extracted solution was dried, and the solvent was distilled off. The crude product was distilled under reduced pressure to obtain the title compound (13 g, yield 67%).
b.p. 116 - 118°C (400 Pa).
¹H-NMR (CDCl₃) δ : 2.30 (3H, s), 5.37 (2H, s), 6.63 (1H, s), 6.72 (1H, d, J= 5.1Hz), 7.29-7.50 (5H, m), 8.03 (1H, d, J= 5.1 Hz).

### Reference Example G 11

### 2-tert-butoxycarbonylamino-4-methylpyridine

It was synthesized according to a method described in Synthesis, pp. 877 to 882, 1996 or Journal of Organic Chemistry, 61, pp. 4810 to 4811, 1996.

### Reference Example G 12

### 2-(2-fluoro-4-pyridyl)-1-(3-methylphenyl)ethanone

Under an argon atmosphere, a solution of diisopropylamine (44mL, 0.31mol) in anhydrous tetrahydrofuran (300 mL) was cooled to -78°C, and to this was added dropwise a 1.6 M n-butyllithium hexane solution (190 mL, 0.31 mol) with stirring. After completion of the addition, the solution was stirred for 10 minutes, subsequently, a solution of 2-fluoro-4-methylpyridine (34.5 g, 0.31 mol) in anhydrous tetrahydrofuran (30 mL) was added. The reaction mixture was stirred for 30 minutes at -10°C. The reaction solution was cooled to -78°C, and a solution of N-(3-methylbenzoyl)propyleneimine (52 g, 0.30 mol) in anhydrous tetrahydrofuran (30 mL) was added dropwise. After completion of the addition, the mixture was stirred for 2 hours at room temperature. To the reaction mixture was added water (100 mL), and extracted with ethyl acetate. The extracted solution was washed with water, dried, then, the solvent was distilled off. The residue was re-crystallized from isopropyl ether to obtain the title compound (35 g, yield 52%).
m.p.: 66 - 67°C.

### Reference Example G 13

The following Reference Example G compounds 13-1 to 13-3 were synthesized according to Reference Example G 12, using N-(3-methoxybenzoyl)propyleneimine, N-(4-fluorobenzoyl)propyleneimine and N-(3-chlorobenzoyl)propyleneimine instead of N-(3-methylbenzoyl)propyleneimine.
Reference Example G compound 13-1: 2-(2-fluoro-4-pyridyl)-1-(3-methoxyphenyl)ethanone
   oil
   ¹H-NMR (CDCl₃) δ : 3.86 (3H, s), 4.31 (2H, s), 6.86 (1H, s), 7.03-7.19 (2H, m), 7.31-7.59 (3H, m), 8.18 (1H, d, J= 5.6 Hz).
Reference Example G compound 13-2: 1-(4-fluorophenyl)-2-(2-fluoro-4-pyridyl)ethanone
   m.p.: 100 - 101°C.
Reference Example G compound 13-3: 1-(3-chlorophenyl)-2-(2-fluoro-4-pyridyl)ethanone
   m.p.: 84 - 86°C.

### Reference Example G 14

### 1-(3-methylphenyl)-2-(2-methyl-4-pyridyl)ethanone

A solution of diisopropylamine (112 mL) in anhydrous tetrahydrofuran (760 mL) was cooled to -50°C, and to this was added dropwise a 1.6 M n-butyllithium hexane solution (500 mL) with stirring. After completion of the addition, the solution was stirred for 10 minutes, subsequently, a solution of 2,4-lutidine (87.9 mL) in anhydrous tetrahydrofuran (76 mL) was added dropwise at -30°C. The reaction mixture was stirred for 1 hour, then, a solution of N-(3-methylbenzoyl)propyleneimine (134 g) in anhydrous tetrahydrofuran (76 mL) was added dropwise at -78°C. After completion of the addition, the mixture was stirred for 2 hours at -78°C. The reaction mixture was allowed to warm to room temperature, to this was added water (800 mL), and extracted with ethyl acetate. The extracts were washed with water, dried, then, the solvent was distilled off. The resulted residue was crystallized from isopropyl ether-hexane to obtain the title compound (156 g, yield 91%).
m.p.: 56 - 57°C.

### Reference Example G 15

The following Reference Example G compounds 15-1 and 15-2 were synthesized according to Reference Example G 14, using N-(3,5-dimethylbenzoyl)propyleneimine and N-(4-fluorobenzoyl)propyleneimine instead of N-(3-methylbenzoyl)propyleneimine.
Reference Example G compound 15-1: 1-(3,5-dimethylphenyl)-2-(2-methyl-4-pyridyl)ethanone
   oil
   ¹H-NMR (CDCl₃) δ : 2.38 (6H, s), 2.54 (3H, s), 4.21 (2H, s), 6.98-7.10 (1H, m), 7.01 (1H, m), 7.06 (1H, s), 7.23 (1H, s), 7.60 (2H, s), 8.42-8.45 (1H, m).
Reference Example G compound 15-2: 2-(2-methyl-4-pyridyl)-1-(4-fluorophenyl)ethanone
   m.p.: 79 - 81°C.

### Reference Example G 16

The following Reference Example G compounds 16-1 and 16-2 were synthesized according to Reference Examples 14 and 15, using γ-choline instead of 2,4-lutidine.
Reference Example G compound 16-1: 2-(2,6-dimethyl-4-pyridyl)-1-(3-methylphenyl)ethanone
   m.p.: 46 - 48°C
Reference Example G compound 16-2: 1-(3,5-dimethylphenyl)-2-(2,6-dimethyl-4-pyridyl)ethanone
   m. p. 135 - 136°C.

### Reference Example G 17

### 2-(2-tert-butoxycarbonylamino-4-pyridyl)-1-(4-methoxyphenyl)ethanone

A solution of 2-tert-butoxycarbonylamino-4-methylpyridine (20 g, 97 mmol) in anhydrous tetrahydrofuran (300 mL) was cooled to -78°C, and to this was added dropwise a 1.6 M n-butyllithium hexane solution (140 mL, 0.23 mol) with stirring. After completion of the addition, the solution was stirred for 30 minutes at 0°C, then, the solution was cooled to -78°C. A solution of N-(4-methoxybenzoyl)propyleneimine (25 g, 0.13 mol) in anhydrous tetrahydrofuran (50 mL) was added dropwise. After completion of the addition, the reaction mixture was stirred for 2 hours at room temperature. To the reaction mixture was added water (100 mL) and isopropyl ether (300 mL), and the resulted crude crystal was filtrated. This crude crystal was recrystallized from tetrahydrofuran-hexane to obtain the title compound (23 g, yield: 69%).
m.p.: 187 - 190°C.

### Reference Example G 18

The following Reference Example G compounds 18-1 to 18-15 were synthesized according to Reference Example G 17, using N-(3-methylbenzoyl)propyleneimine, N-(3,5-dimethylbenzoyl)propyleneimine, N-(3-chlorobenzoyl)propyleneimine, N-benzoylpropyleneimine, N-(4-fluorobenzoyl)propyleneimine, N-[3-(trifluoromethyl)benzoyl]propyleneimine, N-(3-bromobenzoyl)propyleneimine, N-[4-(methylthio)benzoyl]propyleneimine, N-(2-thiophenecarbonyl)propyleneimine, N-(3-propylbenzoyl)propyleneimine, N-[3-(1-methylethyl)benzoyl]propyleneimine, N-(4-fluoro-3-methylbenzoyl)propyleneimine, N-(3-fluorobenzoyl)propyleneimine, N-(4-chlorobenzoyl)propyleneimine and N-(3-ethylbenzoyl)propyleneimine instead of N-(4-methoxybenzoyl)propyleneimine.
Reference Example G compound 18-1: 2-(2-tert-butoxycarbonylamino-4-pyridyl)-1-(3-methylphenyl)ethanone m.p.: 144 - 146°C.
Reference Example G compound 18-2: 2-(2-tert-butoxycarbonylamino-4-pyridyl)-1-(3,5-dimethylphenyl)ethanone
   m.p.: 133 - 136°C.
Reference Example G compound 18-3: 2-(2-tert-butoxycarbonylamino-4-pyridyl)-1-(3-chlorophenyl)ethanone
   m.p.: 152 - 153°C.
Reference Example G compound 18-4: 2-(2-tert-butoxycarbonylamino-4-pyridyl)-1-phenylethanone
   m.p.: 162 - 163°C.
Reference Example G compound 18-5: 2-(2-tert-butoxycarbonylamino-4-pyridyl)-1-(4-fluorophenyl)ethanone
   m.p.: 139 - 141°C.
Reference Example G compound 18-6: 2-(2-tert-butoxycarbonylamino-4-pyridyl)-1-[3-(trifluoromethyl)phenyl]ethanone
   m.p.: 149 - 150°C.
Reference Example G compound 18-7: 1-(3-bromophenyl)-2-(2-tert-butoxycarbonylamino-4-pyridyl)ethanone
   m.p.: 132 - 133°C.
Reference Example G compound 18-8: 2-(2-tert-butoxycarbonylamino-4-pyridyl)-1-[4-(methylthio)phenyl]ethanone
   m.p.: 177 - 178°C.
Reference Example G compound 18-9: 2-(2-tert-butoxycarbonylamino-4-pyridyl)-1-(2-thienyl)ethanone
   m.p.: 161 - 162°C.
Reference Example G compound 18-10: 2-(2-tert-butoxycarbonylamino-4-pyridyl)-1-(3-propylphenyl)ethanone
   m.p.: 110 - 111°C.
Reference Example G compound 18-11: 2-(2-tert-butoxycarbonylamino-4-pyridyl)-1-[(1-methylethyl)phenyl]ethanone
   m.p.: 176 - 177°C.
Reference Example G compound 18-12: 2-(2-tert-butoxycarbonylamino-4-pyridyl)-1-(4-fluoro-3-methylphenyl)ethanone
   m.p.: 143 - 144°C.
Reference Example G compound 18-13: 2-(2-tert-butoxycarbonylamino-4-pyridyl)-1-(3-fluorophenyl)ethanone
   m.p.: 164 - 165°C.
Reference Example G compound 18-14: 2-(2-tert-butoxycarbonylamino-4-pyridyl)-1-(4-chlorophenyl)ethanone
   m.p.: 155 - 156°C.
Reference Example G compound 18-15: 2-(2-tert-butoxycarbonylamino-4-pyridyl)-1-(3-ethylphenyl)ethanone
   m.p.: 122 - 123°C.

### Reference Example G 19

### 1-(3,5-dimethylphenyl)-2-(2-phenylmethyloxy-4-pyridyl)ethanone

A solution of diisopropylamine (9.6 mL, 69 mmol) in anhydrous tetrahydrofuran (60 mL) was cooled to -50°C, and to this was added dropwise a 1.6 M n-butyllithium hexane solution (43 mL, 69 mmol) with stirring. After completion of the addition, the solution was stirred for 10 minutes, subsequently, a solution of 2-phenylmethyloxy-4-methylpyridine (12 g, 62 mmol) in anhydrous tetrahydrofuran (12 mL) was dropped at -30°C. After stirring for 1 hour, a solution of N-(3,5-dimethylbenzoyl)propyleneimine (12 g, 62 mmol) in anhydrous tetrahydrofuran (12 mL) was added dropwise at -30°C. After completion of the addition, the mixture was allowed to warm to room temperature gradually, and stirred for 2 hours. Water (60 mL) was added to the reaction mixture, and extracted with ethyl acetate. The extracted solution was washed with water, dried, then, the solvent was distilled off. The residue was purified by silica gel column chromatography (hexane-ethyl acetate=5:1) to obtain the title compound (9.1 g, yield 44%).
oil
¹H-NMR (CDCl₃) δ : 2.37 (6H, s), 4.20 (2H, s), 5.37 (2H, s), 6.72 (1H, s), 6.81 (1H, d, J= 5.1 Hz), 7.22 (1H, s), 7.30-7.49 (5H, m), 7.59 (2H, s), 8.12 (1H, d, J= 5.1 Hz).

### Reference Example G 20

### 2-bromo-2-(2-tert-butoxycarbonylamino-4-pyridyl)-1-(4-methoxyphenyl)ethanone hydrobromide

To a solution of 2-(2-tert-butoxycarbonylamino-4-pyridyl)-1-(4-methoxyphenyl)ethanone (0.36 g, 1.1 mmol) in acetic acid (5 mL) was added bromine (0.058 mL, 1.1 mmol), and the mixture was stirred for 1 hour at room temperature. The reaction mixture was concentrated, and the residue was washed with isopropyl ether to obtain the title compound (0.44 g, yield 82%).
amorphous powder
¹H-NMR (CDCl₃) δ : 1.55 (6H, s), 3.92 (3H, s), 6.35 (1H, s), 6.99-7.03 (2H, m), 7.66 (1H, dd, J= 6.6, 1.8 Hz), 8.02-8.07 (2H, m), 8.20 (1H, d, J= 6.6 Hz), 8.70 (2H, d, J= 1.8 Hz), 11.02 (1H, br s).

### Reference Example G 21

The following Reference Example G compounds 21-1 to 21-4 were synthesized according to Reference Example G 20, using 2-(2-tert-butoxycarbonylamino-4-pyridyl)-1-(3-methylphenyl)ethanone, 2-(2-tert-butoxycarbonylamino-4-pyridyl)-1-(3,5-dimethylphenyl)ethanone, 1-(3,5-dimethylphenyl)-2-(2-phenylmethyloxy-4-pyridyl)ethanone and 1-(3-bromophenyl)-2-(2-tert-butoxycarbonylamino-4-pyridyl)ethanone, respectively, instead of 2-(2-tert-butoxycarbonylamino-4-pyridyl)-1-(4-methoxyphenyl)ethanone.
Reference Example G compound 21-1: 2-bromo-2-(2-tert-butoxycarbonylamino-4-pyridyl)-1-(3-methylphenyl)ethanone hydrobromide
   This was used in the subsequent reaction without purification.
Reference Example G compound 21-2: 2-bromo-2-(2-tert-butoxycarbonylamino-4-pyridyl)-1-(3,5-dimethylphenyl)ethanone hydrobromide
   This was used in the subsequent reaction without purification.
Reference Example G compound 21-3: 2-bromo-1-(3,5-dimethylphenyl)-2-(2-phenylmethyloxy-4-pyridyl)ethanone hydrobromide
   m.p.: 88 - 90°C.
Reference Example G compound 21-4: 2-bromo-1-(3-bromophenyl)-2-(2-tert-butoxycarbonylamino-4-pyridyl)ethanone hydrobromide
   This was used in the subsequent reaction without purification.

### Reference Example G 22

### 2-bromo-1-(3-methylphenyl)-2-(2-methyl-4-pyridyl)ethanone hydrobromide

1-(3-Methylphenyl)-2-(2-methyl-4-pyridyl)ethanone (150 g) was dissolved in acetic acid (450 mL), bromine (34.3 mL) was added to this, and the mixture was stirred for 3 hours at 70°C. The reaction solution was cooled by ice water, and the deposited crystal was filtrated off. The crystal was washed with ethyl acetate to obtain the title compound (168 g, yield 66%).
m.p.: 144 - 146°C.

### Reference Example G 23

The following Reference Example G compounds 23-1 to 23-22 were synthesized according to Reference Example G 22, using 2-(2-fluoro-4-pyridyl)-1-(3-methylphenyl)ethanone, 2-(2-fluoro-4-pyridyl)-1-(3-methoxyphenyl)ethanone, 2-(2-fluoro-4-pyridyl)-1-(4-fluorophenyl)ethanone, 2-(2-fluoro-4-pyridyl)-1-(3-chlorophenyl)ethanone, 1-(3,5-dimethylphenyl)-2-(2-methyl-4-pyridyl)ethanone, 2-(2-methyl-4-pyridyl)-1-(4-fluorophenyl)ethanone, 2-(2,6-dimethyl-4-pyridyl)-1-(3-methylphenyl)ethanone, 1-(3,5-dimethylphenyl)-2-(2,6-dimethyl-4-pyridyl)ethanone, 2-(2-tert-butoxycarbonylamino-4-pyridyl)-1-(3-methylphenyl)ethanone, 2-(2-tert-butoxycarbonylamino-4-pyridyl)-1-(3-chlorophenyl)ethanone, 2-(2-tert-butoxycarbonylamino-4-pyridyl)-1-phenylethanone, 2-(2-tert-butoxycarbonylamino-4-pyridyl)-1-(4-fluorophenyl)ethanone, 2-(2-tert-butoxycarbonylamino-4-pyridyl)-1-[3-(trifluoromethyl)phenyl]ethanone, 2-(2-tert-butoxycarbonylamino-4-pyridyl)-1-(3-bromophenyl)ethanone, 2-(2-tert-butoxycarbonylamino-4-pyridyl)-1-[4-(methylthio)phenyl]ethanone, 2-(2-tert-butoxycarbonylamino-4-pyridyl)-1-(2-thienyl)ethanone, 2-(2-tert-butoxycarbonylamino-4-pyridyl)-1-(3-propylphenyl)ethanone, 2-(2-tert-butoxycarbonylamino-4-pyridyl)-1-[(1-methylethyl)phenyl]ethanone, 2-(2-tert-butoxycarbonylamino-4-pyridyl)-1-(3-fluorophenyl)ethanone, 2-(2-tert-butoxycarbonylamino-4-pyridyl)-1-(4-chlorophenyl)ethanone, 2-(2-tert-butoxycarbonylamino-4-pyridyl)-1-(3-ethylphenyl)ethanone and 2-(2-tert-butoxycarbonylamino-4-pyridyl)-1-(4-fluoro-3-methylphenyl)ethanone, respectively, instead of 1-(3-methylphenyl)-2-(2-methyl-4-pyridyl)ethanone.
Reference Example G compound 23-1: 2-bromo-2-(2-fluoro-4-pyridyl)-1-(3-methylphenyl)ethanone hydrobromide
   This was used in the subsequent reaction without purification.
Reference Example G compound 23-2: 2-bromo-2-(2-fluoro-4-pyridyl)-1-(3-methoxyphenyl)ethanone hydrobromide
   This was used in the subsequent reaction without purification.
Reference Example G compound 23-3: 2-bromo-2-(2-fluoro-4-pyridyl)-1-(4-fluorophenyl)ethanone hydrobromide
   amorphous powder
   ¹H-NMR (DMSO-d₆) δ : 7.16 (1H, s), 7.37-7.54 (4H, m), 8.11-8.24 (2H, m), 8.30 (1H, d, J= 5.0 Hz).
Reference Example G compound 23-4: 2-bromo-1-(3-chlorophenyl)-2-(2-fluoro-4-pyridyl)ethanone hydrobromide amorphous powder
   ¹H-NMR (DMSO-d₆) δ : 7.19 (1H, s), 7.38 (1H, s), 7.52-7.56 (1H, m), 7.64 (1H, t, J=8.0 Hz), 7.77-7.82 (1H, m), 8.05-8.09 (1H, m), 8.16 (1H, t, J=1.8 Hz), 8.32 (1H, d, J=5.2 Hz), 10.23 (1H, br s).
Reference Example G compound 23-5: 2-bromo-1-(3,5-dimethylphenyl)-2-(2-methyl-4-pyridyl)ethanone hydrobromide
   This was used in the subsequent reaction without purification.
Reference Example G compound 23-6: 2-bromo-1-(4-fluorophenyl)-2-(2-methyl-4-pyridyl)ethanone hydrobromide amorphous powder
   ¹H-NMR (DMSO-d₆) δ : 3.02 (3H, s), 6.68 (1H, s), 7.23 (2H, t, J=8.4 Hz), 8.05 (1H, s), 8.10-8.22 (3H, m), 8.65 (1H, br s).
Reference Example G compound 23-7: 2-bromo-2-(2,6-dimethyl-4-pyridyl)-1-(3-methylphenyl)ethanone hydrobromide
   This was used in the subsequent reaction without purification.
Reference Example G compound 23-8: 2-bromo-1-(3,5-dimethylphenyl)-2-(2,6-dimethyl-4-pyridyl)ethanone hydrobromide
   m.p.: 208 - 212°C
Reference Example G compound 23-9: 2-(2-amino-4-pyridyl)-2-bromo-1-(3-methylphenyl)ethanone hydrobromide
   m.p.: 182 - 185°C
Reference Example G compound 23-10: 2-(2-amino-4-pyridyl)-2-bromo-1-(3-chlorophenyl)ethanone hydrobromide
   m.p.: 199 - 200°C
Reference Example G compound 23-11: 2-(2-amino-4-pyridyl)-2-bromo-1-phenylethanone hydrobromide
   m.p.: 155 - 156°C
Reference Example G compound 23-12: 2-(2-amino-4-pyridyl)-2-bromo-1-(4-fluorophenyl)ethanone hydrobromide
   m.p.: 171 - 172°C
Reference Example G compound 23-13: 2-(2-amino-4-pyridyl)-2-bromo-1-[3-(trifluoromethyl)phenyl]ethanone hydrobromide
   m.p.: 174 - 175°C
Reference Example G compound 23-14: 2-(2-amino-4-pyridyl)-2-bromo-1-(3-bromophenyl)ethanone hydrobromide
   This was used in the subsequent reaction without purification.
Reference Example G compound 23-15: 2-(2-amino-4-pyridyl)-2-bromo-1-[4-(methylthio)phenyl]ethanone hydrobromide
   amorphous powder
   ¹H-NMR (DMSO-d₆) δ : 6.96-7.09 (2H, m), 7.24 (1H, s), 7.32-7.43 (1H, m), 7.98 (1H, d, J=6.6 Hz), 8.12-8.36 (2H, m).
Reference Example G compound 23-16: 2-(2-amino-4-pyridyl)-2-bromo-1-(2-thienyl)ethanone hydrobromide
   amorphous powder
   ¹H-NMR (DMSO-d₆) δ : 2.57 (3H, s), 6.94-7.01 (1H, m), 7.14 (1H, s), 7.21 (1H, s), 7.38-7.46 (2H, m), 7.83-8.06 (3H, m), 8.21 (2H, br).
Reference Example G compound 23-17: 2-(2-amino-4-pyridyl)-2-bromo-1-(3-propylphenyl)ethanone hydrobromide
   amorphous powder
   ¹H-NMR (DMSO-d₆) δ : 0.90 (3H, t, J=7.3 Hz), 1.53-1.73 (2H, m), 2.65 (2H, t, J=7.5 Hz), 3.40 (2H, br s), 6.97 (1H, dd, J=1.8, 6.6 Hz), 7.13 (1H, s), 7.19 (1H, s), 7.46-7.59 (2H, m), 7.89-7.99 (3H, m), 8.14 (1H, br d, J=6.6 Hz).
Reference Example G compound 23-18: 2-(2-amino-4-pyridyl)-2-bromo-1-[3-(1-methylethyl)phenyl]ethanone hydrobromide amorphous powder
   ¹H-NMR (DMSO-d₆) δ : 1.24 (6H, d, J=6.6 Hz), 3.00 (1H, septet, J=6.6 Hz), 7.15 (1H, s), 7.17 (1H, s), 7.46-7.65 (2H, m), 7.88-7.98 (4H, m), 8.09 (1H, br s).
Reference Example G compound 23-19: 2-(2-amino-4-pyridyl)-2-bromo-1-(3-fluorophenyl)ethanone hydrobromide
   m.p.: 206 - 207°C
Reference Example G compound 23-20: 2-(2-amino-4-pyridyl)-2-bromo-1-(4-chlorophenyl)ethanone hydrobromide
   m.p.: 202 - 203°C
Reference Example G compound 23-21: 2-(2-amino-4-pyridyl)-2-bromo-1-(3-ethylphenyl)ethanone hydrobromide
   m.p.: 46 - 47°C
Reference Example G compound 23-22: 2-(2-amino-4-pyridyl)-2-bromo-1-(4-fluoro-3-methylphenyl)ethanone hydrobromide
   m.p.: 225 - 226°C

### Reference Example G 24

### 4-(methylthio)thiobenzamide

4-(Methylthio)benzonitrile (12 g, 80 mmol) was dissolved in a solution of 4 N hydrogen chloride in ethyl acetate (130 mL). To this solution was added dithiophosphoric acid 0,0-diethyl ester(15 mL, 88 mmol), and the mixture was stirred for 22 hours at room temperature. Water (100 mL) was added to the reaction mixture, and extracted with ethyl acetate. The insoluble material was filtrated off, then, the filtrate was washed with brine, dried, then, the solvent was distilled off. The residue was recrystallized from ethyl acetate to obtain the title compound (10 g, yield 67%).
m.p.: 176 - 178°C.

### Reference Example G 25

The following Reference Example G compounds 25-1 to 25-10 were synthesized according to Reference Example G 24, using 2-chlorobenzonitrile, 4-chlorobenzonitrile, 2-fluorobenzonitrile, 4-fluorobenzonitrile, 2,4-difluorobenzonitrile, butyronitrile, valeronitrile, 3-phenylpropionitrile, 4-phenylbutyronitrile, 1-methylpiperidine-4-carbonitrile, respectively, instead of 4-(methylthio)benzonitrile.
Reference Example G compound 25-1: 2-chlorothiobenzamide
   m.p.: 58 - 59°C
Reference Example G compound 25-2: 4-chlorothiobenzamide
   m.p.: 130 - 131°C
Reference Example G compound 25-3: 2-fluorothiobenzamide
   m.p.: 113 - 114°C
Reference Example G compound 25-4: 4-fluorothiobenzamide
   m.p.: 156 - 157°C
Reference Example G compound 25-5: 2,4-difluorothiobenzamide m.p.: 127 - 128°C
Reference Example G compound 25-6: thiobutyramide
   oil
   ¹H-NMR (CDCl₃) δ : 0.99 (3H, t, J= 7.6 Hz), 1.72-1.93 (2H, m), 2.64 (2H, t, J= 7.6 Hz), 7.02 (1H, br s), 7.77 (1H, br s).
Reference Example G compound 25-7: thiovaleramide
   oil
   ¹H-NMR (CDCl₃) δ : 0.94 (3H, t, J= 7.3 Hz), 1.31-1.49 (2H, m), 1.68-1.83 (2H, m), 2.67 (2H, t, J= 7.7 Hz), 6.92 (1H, br s), 7.73 (1H, br s).
Reference Example G compound 25-8: 3-phenyl(thiopropionamide)
   m.p.: 83 - 84°C
Reference Example G compound 25-9: 4-phenyl(thiobutyramide)
   m.p.: 60 - 61°C
Reference Example G compound 25-10: 1-methylpiperidine-4-carbothioamide
   m.p.: 216 - 220°C

### Reference Example G 26

### ethyl (4-phenyl-1-piperazinyl)carbothioylcarbamate

To a solution of ethyl isothiocyanatoformate (8.1 g, 62 mmol) in acetone (30 mL) was added 1-phenylpiperazine (10 g, 62 mmol), and the mixture was heated to reflux for 1 hour. The reaction mixture was concentrated, and the crude crystal was recrystallized from ethyl acetate to obtain the title compound (13 g, yield 73%).
m.p.: 134 - 135°C.

### Reference Example G 27

The following Reference Example G compound 27 was synthesized according to Reference Example G 26 using 1-methylpiperazine instead of 1-phenylpiperazine. Reference Example G compound 27: ethyl (4-methyl-1-piperazinyl)carbothioylcarbamate
m.p.: 155 - 157°C

### Reference Example G 28

### 4-phenyl-1-piperazinecarbothioamide

Ethyl (4-phenyl-1-piperazinyl)carbothioylcarbamate (13 g, 44 mmol) was added to conc. hydrochloric acid (44 mL), and the mixture was stirred for 2 hours at 80°C. The reaction mixture was made basic with an 8N-aqueous sodium hydroxide solution, and the crystal was collected by filtration. The crystal was washed with water, and dried to obtain the title compound (6.1 g, yield 63%).
m.p.: 178 - 179°C

### Reference Example G 29

The following Reference Example G compound 29 was synthesized according to Reference Example G 28 using ethyl (4-methyl-1-piperazinyl)carbothioylcarbamate instead of ethyl (4-phenyl-1-piperazinyl)carbothioylcarbamate.
Reference Example G compound 29: 4-methyl-1-piperazinecarbothioamide
m.p.: 173 - 175°C

### Reference Example G 30

### 3,3,3-trifluorothiopropionamide

To a solution of 3,3,3-trifluoropropionamide (2.00 g, 15.7 mmol) in anhydrous tetrahydrofuran (100 mL) was added a Lawesson's reagent (3.79 g, 9.37 mmol), and the mixture was heated under reflux for 2 hours. The mixture was cooled to room temperature, then, an aqueous saturated sodium hydrogen carbonate solution was added to the reaction mixture, and extracted with ethyl acetate. The extracts were dried, to distill off the solvent. The residue was purified by silica gel column chromatography (hexane-ethyl acetate=10:1 to 4:1) to give the title compound (1.85 g, yield: 82%).
oil
¹H-NMR (CDCl₃) δ : 3.61 (2H, q, J= 10.4 Hz), 6.70 - 8.00 (2H, m).

### Reference Example G 31

The following Reference Example G compounds 31-1 and 31-2 were synthesized according to Reference Example G 30, using ethyl 3-amino-3-oxopropanate and ethyl 2-amino-2-oxoacetate instead of 3,3,3-trifluoropropionamide.
Reference Example G compound 31-1: ethyl 3-amino-3-thioxopropanate
   oil
   ¹H-NMR (CDCl₃) δ : 1.31 (3H, t, J=7.1 Hz), 3.85 (2H, s), 4.22 (2H, q, J=7.1 Hz), 7.74 (1H, br s), 8.92 (1H, br s).
Reference Example G compound 31-2: ethyl 2-amino-2-thioxoacetate
   ¹H-NMR (CDCl₃) δ : 1.41 (3H, t, J=7.2 Hz), 4.38 (2H, q, J=7.2 Hz), 7.68 (1H, br s) , 8.24 (1H, br s).

### Reference Example G 32

The following Reference Example G compound 32 was synthesized according to Example 33 described later, using 4-(3-methylphenyl)-2-[4-(methylthio)phenyl]-5-(4-pyridyl)-1,3-thiazole instead of 4-(3,5-dimethylphenyl)-5-(2-methyl-4-pyridyl)-2-[4-(methylthio)phenyl]-1,3-thiazole.
Reference Example G compound 32: 4-[4-(3-methylphenyl)-2-(4-methylsulfonylphenyl)-1,3-thiazol-5-yl]pyridine N-oxide
m.p.: 196 - 197°C

### Reference Example G 33

### 1-tert-butoxycarbonylpiperidine-4-carboxamide

To a solution of piperidine-4-carboxamide (5.0 g, 39 mmol) in water (30 mL) was added di-tert-butyl dicarbonate (9.2 mL, 40 mmol) slowly, and then the reaction mixture was stirred at room temperature for 24 hours. The resulting mixture was extracted with ethyl acetate, and the extracts were washed with brine, dried, and concentrated in vacuo. The residue was crystallized from ethyl acetate to give the title compound (6.9 g, yield 78%).
m.p.: 163-165°C

### Reference Example G 34

The following Reference Example G compound 34 was synthesized according to Reference Example 30, using 1-tert-butoxycarbonylpiperidine-4-carboxamide instead of 3,3,3-trifluoropropionamide.
Reference Example G compound 34: 1-tert-butoxycarbonylpiperidine-4-carbothioamide
m.p.: 129-131°C

### Reference Example G 35

The following Reference Example G compound 35 was synthesized according to Reference Example 12, using N-(3-ethylbenzoyl)propyleneimine instead of N-(3-methylbenzoyl)propyleneimine
Reference Example G compound 35: 1-(3-ethylphenyl)-2-(2-fluoro-4-pyridyl)ethanone
m.p.: 59-60°C

### Reference Example G 36

The following Reference Example G compound 36 was synthesized according to Reference Example G 22, using 1-(3-ethylphenyl)-2-(2-fluoro-4-pyridyl)ethanone instead of 1-(3-methylphenyl)-2-(2-methyl-4-pyridyl)ethanone
Reference Example G compound 36: 2-bromo-1-(3-ethylphenyl)-2-(2-fluoro-4-pyridyl)ethanone hydrobromide
amorphous powder
¹H-NMR(DMSO-d₆) d: 1.22 (3H, t, J= 7.6Hz), 2.70 (2H, q, J= 7.6Hz), 7.19 (1H, s), 7.39 (1H, s), 7.45-7.58 (3H, m), 7.77 (1H, br s), 7.92-7.97 (2H, m), 8.30 (1H, d, J= 5.6Hz).

### Reference Example G 37

### ethyl 2,2-difluoropropionate

To ethyl pyruvate (3.0 g, 26 mmol) was added dropwise dimethylaminosulfur trifluoride (3.4 mL, 26 mmol) over 1 hour and the reaction mixture was stirred at 60°C for 4 hours. The resulting mixture was poured into ice-water and extracted with ethyl acetate. The extracts were washed with brine, dried, and concentrated under reduced pressure to give the title compound (1.2 g, yield 78%).
oil
¹H-NMR(CDCl₃) d: 1.36 (3H, t, J= 7.2Hz), 1.81 (3H, t, J= 19.0Hz), 4.33 (2H, q, J= 7.2Hz).

### Reference Example G 38

### 2,2-difluoropropionic acid

A solution of ethyl 2,2-difluoropropionate (1.2 g, 8.8 mmol) in ethanol (26 mL) was added to 2N aqueous sodium hydroxide (26 mL) and the resulting mixture was stirred at room temperature for 14 hours. The reaction mixture was acidified with 2N hydrochloric acid and extracted with ether. The extracts were dried, and concentrated under reduced pressure to give the title compound (0.90 g, yield 92%).
oil
¹H-NMR(CDCl₃) d: 1.85 (3H, t, J= 19.0Hz), 6.21 (1H, br s).

### Reference Example G 39

### 2,2-difluoropropionamide

To a solution of 2,2-difluoropropionic acid (7.8 g, 71 mmol) in tetrahydrofuran (80 mL) was added oxalyl chloride (6.6 mL, 78 mmol) at room temperature and then N,N-dimethylformamide (2drops) was added to the solution. The reaction mixture was stirred at room temperature for 2 hours. The resulting solution was added dropwise to 25% aqueous ammonia at 0°C over 15 minutes, and stirred for 1 hour. The reaction mixture was extracted with ethyl acetate. The extracts were dried, and concentrated under reduced pressure. The obtained crude mixture was crystallized from hexane to give the title compound (3.7 g, yield 49%).
m.p.: 70-71°C

### Reference Example G 40

The following Reference Example G compound 40 was synthesized according to Reference Example G 24, using (methylthio)acetonitrile instead of 4-(methylthio)benzonitrile. Reference Example G compound 40: (methylthio)thioacetamide m.p.: 66-67°C

### Reference Example G 41

The following Reference Example G compound 41-1 and 41-2 were synthesized according to Reference Example G 30, using 3-(methylthio)propionamide and 2,2-difluoropropionamide instead of 3,3,3-trifluoropropionamide.
Reference Example G compound 41-1: 3-(methylthio)thiopropionamide
   oil
   ¹H-NMR(CDCl₃) d: 2.17 (3H, s), 2.93 (4H, s), 7.52 (2H, br s).
Reference Example G compound 41-2: 2,2-difluorothiopropionamide
   oil
   ¹H-NMR(CDCl₃) d: 1.98 (3H, t, J= 18.5 HZ) , 7.56 (1H, br s), 7.72 (1H, br s).

### Reference Example G 42

### 2-amino-1-methyl-2-oxoethyl benzoate

To a solution of 2-hydroxypropionamide (10.8 g, 121 mmol) in pyridine (40 mL) was added benzoyl chloride (14.2 mL, 122 mmol) at 0°C and the reaction mixture was allowed to warm up to room temperature. The resulting mixture was stirred at room temperature for 3 hours and the solvent was removed under reduced pressure to give a residue. To the residue an aqueous sodium hydrogen carbonate solution was added and the mixture was extracted with ethyl acetate. The extracts were washed with an 1N hydrochloric acid twice and brine. The organic solution was dried, and concentrated under reduced pressure. The obtained crude crystal was recrystallized from ethyl acetate-hexane to give the title compound (17.9 g, yield 77%). m.p.: 116-117°C

### Reference Example G 43

### 2-amino-1-methyl-2-thioxoethyl benzoate

To a solution of 2-amino-1-methyl-2-oxoethyl benzoate (10.0 g, 52.0 mmol) in 1,2-dimethoxyethane (90 mL) was added Lawesson's reagent (11.2 g, 27.7 mmol) at 0°C and the reaction mixture was allowed to warm up to room temperature. The resulting mixture was stirred at room temperature for 24 hours and the precipitate was removed by filtration. The resulting solution was concentrated under reduced pressure to give a residue. The residue was dissolved in ethyl acetate and the solution was washed with brine. The organic solution was dried, and concentrated under reduced pressure to give a residue. The residue was purified by silica gel column chromatography (hexane-ethyl acetate=2:1) to obtain a crude crystal. The crude crystal was recrystallized from ethyl acetate-hexane to give the title compound (8.60 g, yield 79%).
m.p.: 100-101°C

### Reference Example H 1

### [5-(2-amino-4-pyridyl)-4-(4-methoxyphenyl)-1,3-thiazol-2-yl]amine

To a solution of 2-bromo-2-(2-tert-butoxycarbonylamino-4-pyridyl)-1-(4-methoxyphenyl)ethanone hydrobromide (synthesized from 2-(2-tert-butoxycarbonylamino-4-pyridyl)-1-(4-methoxyphenyl)ethanone (4.5 g, 13 mmol) according to the method described in Reference Example 20) in acetonitrile (40 mL) were added thiourea (1.1 g, 14 mmol) and triethylamine (1.9 mL, 14 mmol), and the mixture was stirred for 2 hours at 80°C. The reaction mixture was cooled to room temperature, then, concentrated. To the residue was added a saturated aqueous sodium hydrogen carbonate solution (200 mL), and the resulting solid was filtrated, and washed with water. To this solid was added 2N-hydrochloric acid (35 mL), and the mixture was stirred for 45 minutes at 100°C. The reaction mixture was cooled to room temperature, then, 8N-sodium hydroxide aqueous solution (10 mL) and aqueous sodium hydrogen carbonate solution (100 mL) were added. The resulted crude crystal was filtrated, and washed with water. This crude crystal was recrystallized from ethanol to obtain the title compound (2.7 g, yield 69%).
m.p.: 251 - 254°C

### Reference Example H 2

### [5-(2-tert-butoxycarbonylamino-4-pyridyl)-3-(4-methoxyphenyl)-1,3-thiazol-2-yl]amine

To a solution of 2-(2-tert-butoxycarbonylamino-4-pyridyl)-1-(4-methoxyphenyl)ethanone (6.1 g, 18 mmol) in acetic acid (100 mL) was added bromine (1.0 mL), and the mixture was stirred for 2 hours at room temperature. The reaction mixture was concentrated. The residue was dissolved in acetonitrile (100 mL), and to this solution were added thiourea (1.1 g, 14 mmol) and triethylamine (3.0 mL, 22 mmol), and the mixture was stirred at room temperature for 2 hours, then, concentrated. To the residue was added a saturated aqueous sodium hydrogen carbonate solution (50 mL), and the resulted solid was filtrated, washed with water, and recrystallized from ethanol to give the title compound (1.7 g, yield: 24%).
m.p.: 270°C or more (dec.)

### Reference Example H 3

### [5-(2-tert-butoxycarbonylamino)-4-pyridyl]-2-ethyl-4-(3-methylphenyl)-1,3-thiazole

A solution of 2-bromo-2-(2-tert-butoxycarbonylamino-4-pyridyl)-1-(3-methylphenyl)ethanone hydrobromide (synthesized from 2-(2-tert-butoxycarbonylamino-4-pyridyl)-1-(3-methylphenyl)ethanone (5.0 g, 24 mmol) according to the method described in Reference Example 21) and thiopropionamide (1.4 g, 16 mmol) in N,N-dimethylformamide (50 mL) was stirred for 14 hours at room temperature. To the reaction mixture was poured an aqueous sodium hydrogen carbonate solution, and extracted with ethyl acetate. The extracts were washed with water, then, dried and concentrated. The residue was purified by silica gel column chromatography (hexane-ethyl acetate=4:1) to obtain a crystal. This crystal was washed with hexane to obtain the title compound (2.43 g, yield 39%).
m.p.: 162 - 163°C

### Reference Example H 4

The following Reference Example H compounds 4-1 and 4-2 were synthesized according to Reference Example H 3, using thioacetamide and 4-(methylthio)thiobenzamide instead of thiopropionamide.
Reference Example H compound 4-1: 5-[2-(tert-butoxycarbonylamino)-4-pyridyl]-2-methyl-4-(3-methylphenyl)-1,3-thiazole
   This was used in the subsequent reaction without purification.
Reference Example H compound 4-2: [5-[2-(tert-butoxycarbonylamino)-4-pyridyl]-4-(3-methylphenyl)-2-[4-(methylthio)phenyl]-1,3-thiazole
   This was used in the subsequent reaction without purification.

### Reference Example H 5

The following Reference Example H compound 5 was synthesized according to Reference Example H 4, using 2-bromo-2-(2-tert-butoxycarbonylamino-4-pyridyl)-1-(4-methoxyphenyl)ethanone hydrobromide instead of 2-bromo-2-(2-tert-butoxycarbonylamino-4-pyridyl)-1-(3-methylphenyl)ethanone hydrobromide.
Reference Example H compound 5: 5-[2-(tert-butoxycarbonylamino)-4-pyridyl]-4-(4-methoxyphenyl)-2-methyl-1,3-thiazole.

This was used in the subsequent reaction without purification.

### Reference Example H 6

### [5-(2-fluoro-4-pyridyl)-4-(3-methylphenyl)-1,3-thiazol-2-yl]amine

To a mixture of 2-bromo-2-(2-fluoro-4-pyridyl)-1-(3-methylphenyl)ethanone hydrobromide and thiourea (3.03 g, 39.8 mmol) in acetonitrile (50 mL) was added triethylamine (5.2 mL, 37.3 mmol), and the mixture was stirred for 2 hours at 80°C. Aqueous sodium hydrogen carbonate solution was poured into the reaction mixture, and the deposited solid was collected by filtration. The resulted solid was washed with water, then, dried. The crude crystal was recrystallized from ethanol to obtain the title compound (3.67 g, yield 35%). m.p.: 214 - 218°C.

### Reference Example H 7

The following Reference Example H compounds 7-1 to 7-8 were synthesized according to Reference Example H 6, using 2-bromo-2-(2-fluoro-4-pyridyl)-1-(3-methoxyphenyl)ethanone hydrobromide, 2-bromo-1-(3-chlorophenyl)-2-(2-fluoro-4-pyridyl)ethanone hydrobromide, 2-bromo-1-(4-fluorophenyl)-2-(2-fluoro-4-pyridyl)ethanone hydrobromide, 2-bromo-1-(3-methylphenyl)-2-(2-methyl-4-pyridyl)ethanone hydrobromide, 2-bromo-1-(3,5-dimethylphenyl)-2-(2-methyl-4-pyridyl)ethanone hydrobromide, 2-bromo-1-(3,5-dimethylphenyl)-2-(2,6-dimethyl-4-pyridyl)ethanone hydrobromide and 2-bromo-1-(3,5-dimethylphenyl)-2-(2,6-dimethyl-4-pyridyl)ethanone hydrobromide, 2-bromo-1-(4-fluorophenyl)-2-(2-methyl-4-pyridyl)ethanone hydrobromide, respectively, instead of 2-bromo-2-(2-fluoro-4-pyridyl)-1-(3-methylphenyl)ethanone
hydrobromide.
Reference Example H compound 7-1: [5-(2-fluoro-4-pyridyl)-4-(3-methoxyphenyl)-1,3-thiazol-2-yl]amine
   m.p.: 190 - 191°C.
Reference Example H compound 7-2: 4-(3-chlorophenyl)-5-(2-fluoro-4-pyridyl)-1,3-thiazol-2-yl]amine
   m.p.: 227 - 228°C.
Reference Example H compound 7-3: [4-(4-fluorophenyl)-5-(2-fluoro-4-pyridyl)-1,3-thiazol-2-yl]amine
   m.p.: 243 - 245°C.
Reference Example H compound 7-4: [4-(3-methylphenyl)-5-(2-methyl-4-pyridyl)-1,3-thiazol-2-yl]amine
   m.p.: 205 - 206°C.
Reference Example H compound 7-5: [4-(3,5-dimethylphenyl)-5-(2-methyl-4-pyridyl)-1,3-thiazol-2-yl]amine
   m.p.: 219 - 220°C.
Reference Example H compound 7-6: [5-(2,6-dimethyl-4-pyridyl)-3-(3-methylphenyl)-1,3-thiazol-2-yl]amine
   m.p.: 214 - 216°C.
Reference Example H compound 7-7: [4-(3,5-dimethylphenyl)-5-(2,6-dimethyl-4-pyridyl)-1,3-thiazol-2-yl]amine
   m.p.: 256 - 258°C.
Reference Example H compound 7-8: [4-(4-fluorophenyl)-5-(2-methyl-4-pyridyl)-1,3-thiazol-2-yl]amine
   m.p.: 233 - 234°C.

### Reference Example H 8

The following Reference Example H compound 8 was synthesized according to Reference Example H 6, using N-methylthiourea instead of thiourea.
Reference Example H compound 8: N-methyl-[5-(2-fluoro-4-pyridyl)-4-(3-methylphenyl)-1,3-thiazol-2-yl]amine
m.p.: 186 - 187°C

### Reference Example H 9

The following Reference Example H compound 9 was synthesized according to Reference Example H 8, using 2-bromo-2-(2-methyl-4-pyridyl)-1-(3-methylphenyl)ethanone hydrobromide instead of 2-bromo-2-(2-fluoro-4-pyridyl)-1-(3-methylphenyl)ethanone hydrobromide.
Reference Example H compound 9: N-methyl-[4-(3-methylphenyl)-5-(2-methyl-4-pyridyl)-1,3-thiazol-2-yl]amine
m.p.: 164 - 165°C

### Reference Example H 10

The following example compound 10 was synthesized according to Example 9, using N,N-dimethylthiourea instead of N-methylthiourea.
Reference Example H compound 10: N,N-dimethyl-[4-(3-methylphenyl)-5-(2-methyl-4-pyridyl)-1,3-thiazol-2-yl]amine
m.p.: 77 - 79°C

### Reference Example H 11

### 2-ethyl-5-(2-fluoro-4-pyridyl)-4-(3-methylphenyl)-1,3-thiazole

A solution of 2-bromo-2-(2-fluoro-4-pyridyl)-1-(3-methylphenyl)ethanone hydrobromide (11 g, 29 mmol) and thiopropionamide (2.7 g, 30 mmol) in N,N-dimethylformamide (30 mL) was stirred for 14 hours at room temperature. Aqueous sodium hydrogen carbonate solution was added to the reaction mixture, and extracted with ethyl acetate. The extracts were washed with water, dried, then, the solvent was distilled off. The residue was purified by silica gel column chromatography (hexane-ethyl acetate= 4:1) to obtain the title compound (3.3 g, yield 38%).
oil
¹H-NMR (CDCl₃) δ : 1.64 (3H, t, J= 7.6 Hz), 2.34 (3H, s), 3.10 (2H, q, J= 7.6 Hz), 6.84-6.86 (1H, m), 7.05-7.09 (1H, m), 7.13-7.25 (3H, m), 7.37 (1H, s), 8.10 (1H, d, J= 5.6 Hz).

### Reference Example H 12

The following Reference Example H compound 12 was synthesized according to Reference Example H 11, using 2-bromo-1-(3-chlorophenyl)-2-(2-fluoro-4-pyridyl)ethanone hydrobromide instead of 2-bromo-2-(2-fluoro-4-pyridyl)-1-(3-methylphenyl)ethanone hydrobromide.
Reference Example H compound 12: 2-ethyl-4-(3-chlorophenyl)-5-(2-fluoro-4-pyridyl)-1,3-thiazole
m.p.: 102 - 103°C

### Reference Example H 13

The following Reference Example H compound 13 was synthesized according to Reference Example H 11, using 2-bromo-1-(3-methylphenyl)-2-(2-methyl-4-pyridyl)ethanone hydrobromide instead of 2-bromo-2-(2-fluoro-4-pyridyl)-1-(3-methylphenyl)ethanone hydrobromide.
Reference Example H compound 13: 2-ethyl-4-(3-methylphenyl)-5-(2-methyl-4-pyridyl)-1,3-thiazole
oil
¹H-NMR (CDCl₃) δ : 1.45 (3H, t, J= 7.6 Hz), 2.33 (3H, s), 2.51 (3H, s), 3.09 (2H, q, J= 7.6 Hz), 6.99 (1H, dd, J=1.2, 5.2 Hz), 7.13-7,30 (4H, m), 7.39 (1H, s), 8.38 (1H, d, J=5.2 Hz).

### Reference Example H 14

The following Reference Example H compounds 14-1 to 14-14 were synthesized according to Reference Example H 13, using 2-chlorothiobenzamide, 4-chlorothiobenzamide, 2-fluorothiobenzamide, 4-fluorothiobenzamide, 2,4-difluorothiobenzamide, thiobenzamide, phenyl(thioacetamide), 3-phenyl(thiopropionamide), 4-phenyl(thiobutyramide), thiovaleramide, thiobutyramide, ethyl 2-amino-2-thioxoacetate, 4-methyl-1-piperazinecarbothioamide and 1-methylpiperidine-4-carbothioamide, respectively, instead of thiopropionamide.
Reference Example H compound 14-1: 2-(2-chlorophenyl)-4-(3-methylphenyl)-5-(2-methyl-4-pyridyl)-1,3-thiazole
   m.p.: 83 - 84°C
Reference Example H compound 14-2: 2-(4-chlorophenyl)-4-(3-methylphenyl)-5-(2-methyl-4-pyridyl)-1,3-thiazole
   m.p.: 104 - 105°C
Reference Example H compound 14-3: 2-(2-fluorophenyl)-4-(3-methylphenyl)-5-(2-methyl-4-pyridyl)-1,3-thiazole
   m.p.: 73 - 74°C
Reference Example H compound 14-4: 2-(4-fluorophenyl)-4-(3-methylphenyl)-5-(2-methyl-4-pyridyl)-1,3-thiazole
   m.p.: 89 - 91°C
Reference Example H compound 14-5: 2-(2,4-difluorophenyl)-4-(3-methylphenyl)-5-(2-methyl-4-pyridyl)-1,3-thiazole
   m.p.: 90 - 91°C
Reference Example H compound 14-6: 4-(3-methylphenyl)-5-(2-methyl-4-pyridyl)-2-phenyl-1,3-thiazole
   m.p.: 79 - 80°C
Reference Example H compound 14-7: 4-(3-methylphenyl)-5-(2-methyl-4-pyridyl)-2-(phenylmethyl)-1,3-thiazole
   m.p.: 82 - 84°C
Reference Example H compound 14-8: 4-(3-methylphenyl)-5-(2-methyl-4-pyridyl)-2-(2-phenylethyl)-1,3-thiazole
   m.p.: 64 - 65°C
Reference Example H compound 14-9: 4-(3-methylphenyl)-5-(2-methyl-4-pyridyl)-2-(3-phenylpropyl)-1,3-thiazole
   oil
   ¹H-NMR (CDCl₃) δ : 2.12-2.27 (2H, m), 2.33 (3H, s), 2.50 (3H, s), 2.79 (2H, t, J=7.7 Hz), 3.08 (2H, t, J=7.9 Hz), 6.98 (1H, dd, J=1.4, 5.6 Hz), 7.10-7.35 (9H, m), 7.38 (1H, s), 8.38 (1H, d, J=5.6 Hz).
Reference Example H compound 14-10: 2-butyl-4-(3-methylphenyl)-5-(2-methyl-4-pyridyl)-1,3-thiazole
   oil
   ¹H-NMR (CDCl₃) δ : 0.99 (3H, t, J=7.1 Hz), 1.43-1.56 (2H, m), 1.76-1.91 (2H, m), 2.33 (3H, s), 2.50 (3H, s), 3.05 (2H, t, J=7.9 Hz), 6.99 (1H, d, J=5.4 Hz), 7.10-7.20 (4H, m), 7.38 (1H, s), 8.37 (1H, d, J=5.4 Hz).
Reference Example H compound 14-11: 4-(3-methylphenyl)-5-(2-methyl-4-pyridyl)-2-propyl-1,3-thiazole
   oil
   ¹H-NMR (CDCl₃) δ : 1.08 (3H, t, J=7.4 Hz), 1.79-2.00 (2H, m), 2.33 (3H, s), 2.50 (3H, s), 3.03 (2H, t, J=7.4 Hz), 6.99 (1H, d, J=5.3 Hz), 7.10-7.20 (4H, m), 7.39 (1H, s), 8.37 (1H, d, J=5.3 Hz).
Reference Example H compound 14-12: ethyl [4-(3-methylphenyl)-5-(2-methyl-4-pyridyl)-1,3-thiazol-2-yl]carboxylate
   m.p.: 97 - 98°C
Reference Example H compound 14-13: 4-(3-methylphenyl)-2-(4-methylpiperazin-1-yl)-5-(2-methyl-4-pyridyl)-1,3-thiazole m.p.: 115 - 116°C
Reference Example H compound 14-14: 4-(3-methylphenyl)-2-(1-methylpiperazin-4-yl)-5-(2-methyl-4-pyridyl)-1,3-thiazole m.p.: 127 - 130°C

### Reference Example H 15

The following Reference Example H compound 15 was synthesized according to Reference Example H 11, using 4-(methylthio)thiobenzamide, instead of thiopropionamide. Reference Example H compound 15: 5-(2-fluoro-4-pyridyl)-4-(3-methylphenyl)-2-[4-(methylthio)phenyl]-1,3-thiazole
m.p.: 97 - 100°C

### Reference Example H 16

The following Reference Example H compounds 16-1 to 16-6 were synthesized according to Reference Example H 15, using 2-bromo-1-(3-methylphenyl)-2-(2-methyl-4-pyridyl)ethanone hydrobromide, 2-bromo-1-(3,5-dimethylphenyl)-2-(2-methyl-4-pyridyl)ethanone hydrobromide, 2-bromo-1-(3,5-dimethylphenyl)-2-(2,6-dimethyl-4-pyridyl)ethanone hydrobromide, 2-bromo-1-(3,5-dimethylphenyl)-2-(2,6-dimethyl-4-pyridyl)ethanone hydrobromide, 2-bromo-1-(4-fluorophenyl)-2-(2-methyl-4-pyridyl)ethanone hydrobromide and 2-(2-amino-4-pyridyl)-2-bromo-1-(3-chlorophenyl)ethanone hydrobromide, respectively, instead of 2-bromo-2-(2-fluoro-4-pyridyl)-1-(3-methylphenyl)ethanone hydrobromide.
Reference Example H compound 16-1: 4-(3-methylphenyl)-5-(2-methyl-4-pyridyl)-2-[4-(methylthio)phenyl]-1,3-thiazole
   m.p.: 119 - 122°C
Reference Example H compound 16-2: 4-(3,5-dimethylphenyl)-5-(2-methyl-4-pyridyl)-2-[4-(methylthio)phenyl]-1,3-thiazole m.p.: 123 - 125°C
Reference Example H compound 16-3: 5-(2,6-dimethyl-4-pyridyl)-4-(3-methylphenyl)-2-[4-(methylthio)phenyl]-1,3-thiazole
   m.p.: 112 - 114°C
Reference Example H compound 16-4: 4-(3,5-dimethylphenyl)-5-(2,6-dimethyl-4-pyridyl)-2-[4-(methylthio)phenyl]-1,3-thiazole
   m.p.: 134 - 136°C
Reference Example H compound 16-5: 4-(4-fluorophenyl)-5-(2-methyl-4-pyridyl)-2-[4-(methylthio)phenyl]-1,3-thiazole
   m.p.: 99 - 100°C
Reference Example H compound 16-6: 4-[4-(3-chlorophenyl)-2-[4-(methylthio)phenyl-1,3-thiazol-5-yl]-2-pyridylamine
   m.p.: 183 - 184°C

### Reference Example H 17

### 4-[2-(2-chlorophenyl)-4-(3-methylphenyl)-1,3-thiazol-5-yl]-2-pyridylamine

A solution of 2-(2-amino-4-pyridyl)-2-bromo-1-(3-methylphenyl)ethanone hydrobromide (5.00 g, 12.3 mmol) and 2-chlorothiobenzamide (1.06 g, 11.9 mmol) in N,N-dimethylformamide (40 mL) was stirred for 14 hours at room temperature. Aqueous sodium hydrogen carbonate solution was poured into the reaction mixture, and extracted with ethyl acetate. The extracts were washed with water, dried, and concentrated. The residue was purified by silica gel column chromatography (hexane-ethyl acetate= 4:1 to 2:1) to obtain a crystal. This crystal was washed with isopropyl ether, to obtain the title compound (3.15 g, yield 81%).
m.p.: 175 - 177°C

### Reference Example H 18

The following Reference Example H compounds 18-1 to 18-5 were synthesized according to Reference Example H 17, using 4-fluorothiobenzamide, thiovaleramide, 3,3,3-trifluorothiopropionamide, thiobutyramide, ethyl 3-amino-3-thioxopropanate, respectively, instead of 2-chlorothiobenzamide.
Reference Example H compound 18-1: 4-[2-(4-fluorophenyl)-4-(3-methylphenyl)-1,3-thiazol-5-yl]-2-pyridylamine
   m.p.: 160 - 162°C
Reference Example H compound 18-2: 4-[2-butyl-4-(3-methylphenyl)-1,3-thiazol-5-yl]-2-pyridylamine oil
   ¹H-NMR (CDCl₃) δ : 0.98 (3H, t, J=7.3 Hz), 1.39-1.59 (2H, m), 1.76-1.92 (2H, m), 2.34 (3H, s), 3.04 (2H, t, J=7.4 Hz), 4.14 (2H, br s), 6.44 (1H, s), 6.56 (1H, dd, J=1.5, 5.4 Hz), 7.09-7.26 (3H, m), 7.41 (1H, s), 7.96 (1H, d, J=5.4 Hz).
Reference Example H compound 18-3: 4-[4-(3-methylphenyl)-2-(2,2,2-trifluoroethyl)-1,3-thiazol-5-yl]-2-pyridylamine
   m.p.: 131 - 132°C
Reference Example H compound 18-4: 4-[4-(3-methylphenyl)-2-propyl-1,3-thiazol-5-yl]-2-pyridylamine
   m.p.: 113 - 115°C
Reference Example H compound 18-5: Ethyl [5-(2-amino-4-pyridyl)-4-(3-methylphenyl)-1,3-thiazol-2-yl]acetate
   m.p.: 128 - 129°C

### Reference Example H 19

The following Reference Example H compound 19 was synthesized according to Reference Example H 17, using ethyl 2-amino-2-thioxoacetate instead of 2-chlorothiobenzamide. Reference Example H compound 19: ethyl [5-(2-amino-4-pyridyl-4-(3-methylphenyl)-1,3-thiazol-2-yl)carboxylate
m.p.: 147 - 148°C

### Reference Example H 20

The following Reference Example H compounds 20-1 to 20-12 were synthesized according to Reference Example H 19, using 2-(2-amino-4-pyridyl)-2-bromo-1-(3-chlorophenyl)ethanone hydrobromide, 2-(2-amino-4-pyridyl)-2-bromo-1-phenylethanone hydrobromide, 2-(2-amino-4-pyridyl)-2-bromo-1-(4-fluorophenyl)ethanone hydrobromide, 2-(2-amino-4-pyridyl)-2-bromo-1-[3-(trifluoromethyl)phenyl]ethanone hydrobromide, 2-(2-amino-4-pyridyl)-2-bromo-1-[4-(methylthio)phenyl]ethanone hydrobromide, 2-(2-amino-4-pyridyl)-2-bromo-1-(3-fluorophenyl)ethanone hydrobromide, 2-(2-amino-4-pyridyl)-2-bromo-1-(4-chlorophenyl)ethanone hydrobromide, 2-(2-amino-4-pyridyl)-2-bromo-1-(3-ethylphenyl)ethanone hydrobromide, 2-(2-amino-4-pyridyl)-2-bromo-1-(4-fluoro-3-methylphenyl)ethanone hydrobromide, 2-(2-amino-4-pyridyl)-2-bromo-1-[3-(1-methylethyl)phenyl]ethanone hydrobromide, 2-(2-amino-4-pyridyl)-2-bromo-1-(3-propylphenyl)ethanone hydrobromide, 2-(2-amino-4-pyridyl)-2-bromo-1-(2-thienyl)ethanone hydrobromide, respectively, instead of 2-(2-amino-4-pyridyl)-2-bromo-1-(3-methylphenyl)ethanone hydrobromide.
Reference Example H compound 20-1: 4-[2-ethyl-4-(3-chlorophenyl)-1,3-thiazol-5-yl]-2-pyridylamine
   m.p.: 132 - 133°C
Reference Example H compound 20-2: 4-(2-ethyl-4-phenyl-1,3-thiazol-5-yl)-2-pyridylamine
   m.p.: 158 - 159°C
Reference Example H compound 20-3: 4-[2-ethyl-4-(4-fluorophenyl)-1,3-thiazol-5-yl]-2-pyridylamine
   m.p.: 140 - 141°C
Reference Example H compound 20-4: 4-[2-ethyl-4-[3-(trifluoromethyl)phenyl]-1,3-thiazol-5-yl]-2-pyridylamine
   m.p.: 117 - 118°C
Reference Example H compound 20-5: 4-[2-ethyl-4-[4-(methylthio)phenyl]-1,3-thiazol-5-yl]-2-pyridylamine
   m.p.: 119 - 120°C
Reference Example H compound 20-6: 4-[2-ethyl-4-(3-fluorophenyl)-1,3-thiazol-5-yl]-2-pyridylamine
   m.p.: 153 - 154°C
Reference Example H compound 20-7: 4-[4-(4-chlorophenyl)-2-ethyl-1,3-thiazol-5-yl]-2-pyridylamine
   m.p.: 136 - 137°C
Reference Example H compound 20-8: 4-[2-ethyl-4-(3-ethylphenyl)-1,3-thiazol-5-yl]-2-pyridylamine
   m.p.: 128 - 129°C
Reference Example H compound 20-9: 4-[2-ethyl-4-(4-fluoro-3-methylphenyl)-1,3-thiazol-5-yl]-2-pyridylamine
   m.p.: 134 - 135°C
Reference Example H compound 20-10: 4-[2-ethyl-4-[3-(1-methylethyl)phenyl]-1,3-thiazol-5-yl]-2-pyridylamine
   m.p.: 80 - 81°C
Reference Example H compound 20-11: 4-[2-ethyl-4-(3-propylphenyl)-1,3-thiazol-5-yl]-2-pyridylamine
   m.p.: 72 - 74°C
Reference Example H compound 20-12: 4-[2-ethyl-4-(2-thienyl)-1,3-thiazol-5-yl]-2-pyridylamine
   m.p.: 159 - 160°C

### Reference Example H 21

The following Reference Example H compounds 21-1 and 21-2 were synthesized according to Reference Example H 18, using 2-(2-amino-4-pyridyl)-2-bromo-1-(3-chlorophenyl)ethanone hydrobromide instead of 2-(2-amino-4-pyridyl)-2-bromo-1-(3-methylphenyl)ethanone hydrobromide.
Reference Example H compound 21-1: 4-[4-(3-chlorophenyl)-2-propyl-1,3-thiazol-5-yl]-2-pyridylamine
   m.p.: 99 - 100°C
Reference Example H compound 21-2: Ethyl [5-(2-amino-4-pyridyl)-4-(3-chlorophenyl)-1,3-thiazol-2-yl]acetate
   m.p.: 154 - 155°C

### Reference Example H 22

### 4-[2-ethyl-4-(3-methylphenyl)-1,3-thiazol-5-yl]-2-pyridylamine

To 5-[2-(tert-butoxycarbonylamino)-4-pyridyl]-2-ethyl-4-(3-methylphenyl)-1,3-thiazole (synthesized from 2-(2-tert-butoxycarbonylamino-4-pyridyl)-1-(3-methylphenyl)ethanone (35 g, 170 mmol) according to the method described in Example 3) was added 2N-hydrochloric acid (200 mL), and the mixture was stirred for 1 hour at 100°C. The reaction mixture was cooled to room temperature, then, made alkaline with a 2N aqueous sodium hydrogen carbonate solution (200 mL) and aqueous sodium hydrogen carbonate solution. The resulted mixture was extracted by ethyl acetate, and the extracts were washed with water. This extracts were dried and concentrated. The residue was purified by silica gel column chromatography (hexane-ethyl acetate= 1:1) to obtain a crystal. This crystal was washed with isopropyl ether, to obtain the title compound (17 g, yield 55%).
m.p.: 144 - 146°C

### Reference Example H 23

The following Reference Example H compounds 23-1 to 23-3 were synthesized according to Reference Example H 22, using 5-[2-(tert-butoxycarbonylamino)-4-pyridyl]-2-methyl-4-(3-methylphenyl)-1,3-thiazole, 5-[2-(tert-butoxycarbonylamino)-4-pyridyl]-4-(3-methylphenyl)-2-[4-(methylthio)phenyl]-1,3-thiazole and 5-[2-(tert-butoxycarbonylamino)-4-pyridyl]-4-(4-methoxyphenyl)-2-methyl-1,3-thiazole, respectively, instead of 5-[2-(tert-butoxycarbonylamino)-4-pyridyl]-2-ethyl-4-(3-methylphenyl)-1,3-thiazole.
Reference Example H compound 23-1: 4-[2-methyl-4-(3-methylphenyl)-1,3-thiazol-5-yl]-2-pyridylamine
   m.p.: 152 - 153°C
Reference Example H compound 23-2: 4-[4-(3-methylphenyl)-2-[4-(methylthio)phenyl]-1,3-thiazol-5-yl]-2-pyridylamine
   m.p.: 181 - 183°C
Reference Example H compound 23-3: 4-[4-(4-methoxyphenyl)-2-methyl-1,3-thiazol-5-yl]-2-pyridylamine
   m.p.: 140 - 141°C

### Reference Example H 24

### [5-(2-amino-4-pyridyl)-4-(3-methylphenyl)-1,3-thiazol-2-yl]acetic acid

To a suspension of ethyl [5-(2-amino-4-pyridyl)-4-(3-methylphenyl)-1,3-thiazol-2-yl]acetate (7.00 g, 19.8 mmol) in ethanol (40 mL) was added a 1N aqueous sodium hydroxide solution (40 mL), and the mixture was stirred for 2 hours at the room temperature under the same condition, The reaction mixture was neutralized with 2N hydrochloric acid (20 mL), then, the produced solid was collected by filtration. The crude product was washed with water, and dried to obtain the title compound (6.10 g, yield: 95%).
m.p.: 132 - 133°C

### Reference Example H 25

The following Reference Example H compounds 25-1 to 25-3 were synthesized according to Reference Example H 24, using ethyl [5-(2-amino-4-pyridyl)-4-(3-methylphenyl)-1,3-thiazol-2-yl]carboxylate, ethyl [5-(2-methyl-4-pyridyl)-4-(3-methylphenyl)-1,3-thiazol-2-yl]carboxylate and ethyl [5-(2-amino-4-pyridyl)-4-(3-chlorophenyl)-1,3-thiazol-2-yl]acetate, respectively, instead of ethyl [5-(2-amino-4-pyridyl)-4-(3-methylphenyl)-1,3-thiazol-2-yl]acetate.
Reference Example H compound 25-1: 5-(2-amino-4-pyridyl)-4-(3-methylphenyl)-1,3-thiazole-2-carboxylic acid
   m.p.: 156 - 157°C
Reference Example H compound 25-2: 5-(2-methyl-4-pyridyl)-4-(3-methylphenyl)-1,3-thiazole-2-carboxylic acid
   m.p.: 135 - 136°C
Reference Example H compound 25-3: [5-(2-methyl-4-pyridyl)-4-(3-chlorophenyl)-1,3-thiazol-2-yl]acetic acid
   This was used in the subsequent reaction without purification.

### Reference Example H 26

### 4-(3-methylphenyl)-5-(2-methyl-4-pyridyl)-1,3-thiazole

4-(3-methylphenyl)-5-(2-methyl-4-pyridyl)-1,3-thiazole-2-carboxylic acid (0.20 g, 0.64 mmol) was stirred for 15 minutes at 150°C. It was cooled to room temperature, then, the crude product was purified by silica gel column chromatography (ethyl acetate) to obtain the title compound (0.17 g, yield 98%).
oil
¹H-NMR (CDCl₃) δ : 2.34 (3H, s), 2.53 (3H, s), 7.04 (1H, d, J=5.1 Hz), 7.16-7.24 (4H, m), 7.43 (1H, s), 8.42 (1H, d, J=5.1 Hz), 8.88 (1H, s).

### Reference Example H 27

The following Reference Example H compounds 27-1 and 27-2 were synthesized according to Reference Example H 26, using 5-(2-amino-4-pyridyl)-4-(3-methylphenyl)-1,3-thiazole-2-carboxylic acid and [5-(2-amino-4-pyridyl)-4-(3-chlorophenyl)-1,3-thiazol-2-yl]acetic acid, respectively, instead of 4-(3-methylphenyl)-5-(2-methyl-4-pyridyl)-1,3-thiazole-2-carboxylic acid.
Reference Example H compound 27-1: 4-[4-(3-methylphenyl)-1,3-thiazol-5-yl]-2-pyridylamine
   m.p.: 91 - 92°C
Reference Example H compound 27-2: 4-[4-(3-chlorophenyl)-2-methyl-1,3-thiazol-5-yl]-2-pyridylamine
   m.p.: 142 - 143°C

### Reference Example H 28

### N-[4-[2-ethyl-4-(3-methylphenyl)-1,3-thiazol-5-yl]-2-pyridyl]cyclohexanecarboxamide

To a solution of 4-[2-ethyl-4-(3-methylphenyl)-1,3-thiazol-5-yl]-2-pyridylamine (0.80 g, 2.7 mmol) in tetrahydrofuran (10 mL) were added cyclohexanecarbonyl chloride (0.40 mL, 3.0 mmol) and triethylamine (0.39 mL, 2.8 mmol) sequentially, and the mixture was stirred for 1 hour at room temperature. To the reaction mixture was added an aqueous sodium hydrogen carbonate solution, and extracted with ethyl acetate. The extracts were washed with an aqueous sodium hydrogen carbonate solution, then, dried and concentrated. The residue was purified by silica gel column chromatography (hexane-ethyl acetate=20:1 to 4:1) to obtain a crystal. This crystal was washed with hexane to obtain the title compound (0.83 g, yield 75%).
m.p.: 98 - 100°C

### Reference Example H 29

The following Reference Example H compounds 29-1 to 29-5 were synthesized according to Reference Example H 28, using cyclopentanecarbonyl chloride, acetyl chloride, 1-methylcyclohexanecarbonyl chloride, propionyl chloride and pivaloyl chloride instead of cyclohexanecarbonyl chloride. Reference Example H compound 29-1: N-[4-[2-ethyl-4-(3-methylphenyl)-1,3-thiazol-5-yl]-2-pyridyl]cyclopentanecarboxamide
m.p.: 123 - 125°C.
Reference Example H compound 29-2: N-[4-[2-ethyl-4-(3-methylphenyl)-1,3-thiazol-5-yl]-2-pyridyl]acetamide
   m.p.: 119 - 120°C.
Reference Example H compound 29-3: N-[4-[2-ethyl-4-(3-methylphenyl)-1,3-thiazol-5-yl]-2-pyridyl]-1-methylcyclohexanecarboxamide
   oil
   ¹H-NMR (CDCl₃) δ : 1.28 (3H, s), 1,30-1.75 (11H, m), 1.98-2.12 (2H, m), 2,33 (3H, s), 3.08 (2H, q, J=7.6 Hz), 6.79-6.85 (1H, m), 7.10-7.25 (3H, m), 7.38-7.42 (1H, m), 8.04-8.07 (2H, m), 8.40-8.43 (1H, m).
Reference Example H compound 29-4: N-[4-[2-ethyl-4-(3-methylphenyl)-1,3-thiazol-5-yl]-2-pyridyl]propionamide
   m.p.: 103 - 104°C
Reference Example H compound 29-5: N-[4-[2-ethyl-4-(3-methylphenyl)-1,3-thiazol-5-yl]-2-pyridyl]pivalamide
   oil
   ¹H-NMR (CDCl₃) δ : 1.34 (9H, s), 1.44 (3H, t, J=7.6 Hz), 2.33 (3H, s), 3.08 (2H, q, J=7.6 Hz), 6.79-6.84 (1H, m), 7.09-7.27 (3H, m), 7.36-7.39 (1H, m), 8.03-8.10 (2H, m), 8.38-8.42 (1H, m).

### Reference Example H 30

The following Reference Example H compounds 30-1 to 30-12 were synthesized according to Reference Example H 29, using 4-[4-(3-chlorophenyl)-2-methyl-1,3-thiazol-5-yl]-2-pyridylamine, 4-[4-(3-chlorophenyl)-2-ethyl-1,3-thiazol-5-yl]-2-pyridylamine, 4-[4-(3-chlorophenyl)-2-propyl-1,3-thiazol-5-yl]-2-pyridylamine, 4-[2-ethyl-4-(2-thienyl)-1,3-thiazol-5-yl]-2-pyridylamine and 4-[4-(3-chlorophenyl)-2-[4-(methylthio)phenyl]-1,3-thiazol-5-yl]-2-pyridylamine, respectively, instead of 4-[2-ethyl-4-(3-methylphenyl)-1,3-thiazol-5-yl]-2-pyridylamine.
Reference Example H compound 30-1: N-[4-[4-(3-chlorophenyl)-2-methyl-1,3-thiazol-5-yl]-2-pyridyl]acetamide
   m.p.: 112 - 115°C.
Reference Example H compound 30-2: N-[4-[4-(3-chlorophenyl)-2-ethyl-1,3-thiazol-5-yl]-2-pyridyl]acetamide
   m.p.: 149 - 150°C.
Reference Example H compound 30-3: N-[4-[4-(3-chlorophenyl)-2-propyl-1,3-thiazol-5-yl]-2-pyridyl]acetamide
   m.p.: 144 - 145°C.
Reference Example H compound 30-4: N-[4-[2-ethyl-4-(2-thienyl)-1,3-thiazol-5-yl]-2-pyridyl]acetamide
   m.p.: 154 - 155°C.
Reference Example H compound 30-5: N-[4-[4-(3-chlorophenyl)-2-[4-(methylthio)phenyl]-1,3-thiazol-5-yl]-2-pyridyl]acetamide
   m.p.: 207 - 208°C.
Reference Example H compound 30-6: N-[4-[4-(3-chlorophenyl)-2-ethyl-1,3-thiazol-5-yl]-2-pyridyl]-1-methylcyclohexanecarboxamide
   oil
   ¹H-NMR (CDCl₃) δ : 1.28 (3H, s), 1.35-1.82 (11H, m), 1.95-2.13 (2H, m), 3.08 (2H, q, J=7.8 Hz), 6.80-6.84 (1H, m), 7.19-7,37 (3H, m), 7.53-7.62 (1H, m), 8.07-8.12 (1H, m), 8.25-8.35 (1H, m), 8.40-8.43 (1H, m).
Reference Example H compound 30-7: N-[4-[4-(3-chlorophenyl)-2-methyl-1,3-thiazol-5-yl]-2-pyridyl]propionamide
   m.p.: 134 - 135°C.
Reference Example H compound 30-8: N-[4-[4-(3-chlorophenyl)-2-ethyl-1,3-thiazol-5-yl]-2-pyridyl]propionamide
   m.p.: 132 - 133°C.
Reference Example H compound 30-9: N-[4-[4-(3-chlorophenyl)-2-propyl-1,3-thiazol-5-yl)-2-pyridyl]propionamide
   m.p.: 103 - 104°C.
Reference Example H compound 30-10: N-[4-[2-ethyl-4-(2-thienyl)-1,3-thiazol-5-yl]-2-pyridyl]propionamide
   m.p.: 187 - 188°C.
Reference Example H compound 30-11: N-[4-[4-(3-chlorophenyl)-2-[4-(methylthio)phenyl]-1,3-thiazol-5-yl]-2-pyridyl]propionamide
   m.p.: 187 - 188°C.
Reference Example H compound 30-12: N-[4-[4-(3-chlorophenyl)-2-[4-(methylthio)phenyl]-1,3-thiazol-5-yl]-2-pyridyl]pivalamide
   m.p.: 119 - 120°C.

### Reference Example H 31

### N-(cyclohexylmethyl)-4-[2-ethyl-4-(3-methylphenyl)-1,3-thiazol-5-yl]-2-pyridylamine

To a solution of aluminum chloride (0.40 g, 3.0 mmol) in tetrahydrofuran (40 mL) was added lithium aluminum hydride (0.12 g, 3.0 mmol) at 0°C. To this solution was added dropwise a solution of N-[4-[2-ethyl-4-(3-methylphenyl)-1,3-thiazol-5-yl]-2-pyridyl]cyclohexanecarboxamine (0.40 g, 0.99 mmol) in tetrahydrofuran (10 mL), and the mixture was heated under reflux for 1 hour. The reaction mixture was cooled to room temperature, to this was added ice water, and extracted with ethyl acetate. The extracts were washed with an aqueous sodium hydrogen carbonate solution, then, dried and concentrated. The residue was purified by silica gel column chromatography (hexane-ethyl acetate=20:1 to 4:1) to obtain a crystal. This crystal was washed with hexane to obtain the title compound (0.27 g, yield 70%).
m.p.: 74 - 75°C

### Reference Example H 32

The following Reference Example H compound 32 was synthesized according to Reference Example H 31, using N-[4-[2-ethyl-4-(3-methylphenyl)-1,3-thiazol-5-yl]-2-pyridyl]cyclopentanecarboxamine instead of N-[4-[2-ethyl-4-(3-methylphenyl)-1,3-thiazol-5-yl]-2-pyridyl]cyclohexanecarboxamine.
Reference Example H compound 32: N-(cyclopentylmethyl)-4-[2-ethyl-4-(3-methylphenyl)-1,3-thiazol-5-yl]-2-pyridylamine
m.p.: 67 - 69°C.

### Reference Example H 33

### [4-(3-methylphenyl)-5-(2-pyperidino-4-pyridyl)-1,3-thiazol-2-yl]amine

5-(2-Fluoro-4-pyridyl)-4-(3-methylphenyl)-1,3-thiazol-2-yl]amine (0.70 g, 2.5 mmol) and piperidine (2.0 mL, 20 mmol) were stirred at 150°C for 3 hours. The reaction mixture was purified by silica gel column chromatography (hexane-ethyl acetate=1:1) to obtain the title compound (0.62 g, yield 72%).
m.p.: 181 - 182°C

### Reference Example H 34

The following Reference Example H compounds 34-1 to 34-3 were synthesized according to Reference Example H 33, using morpholine, cyclohexylamine and cyclopentylamine instead of piperidine.
Reference Example H compound 34-1: [4-(3-methylphenyl)-5-(2-morpholino-4-pyridyl)-1,3-thiazol-2-yl]amine
   m.p.: 188 - 189°C.
Reference Example H compound 34-2: [5-(2-cyclohexylamino-4-pyridyl)-4-(3-methylphenyl)-1,3-thiazol-2-yl]amine
   m.p.: 168 - 169°C.
Reference Example H compound 34-3: [5-(2-cyclopentylamino-4-pyridyl)-4-(3-methylphenyl)-1,3-thiazol-2-yl]amine
   m.p.: 169 - 170°C.

### Reference Example H 35

The following Reference Example H compounds 35-1 and 35-2 were synthesized according to Reference Example H 34, using 4-(3-chlorophenyl)-5-(2-fluoro-4-pyridyl)-1,3-thiazol-2-yl]amine and 5-(2-fluoro-4-pyridyl)-4-(3-methylphenyl)-2-(4-methylsulfonylphenyl)-1,3-thiazole instead of 5-(2-fluoro-4-pyridyl)-4-(3-methylphenyl)-1,3-thiazol-2-yl]amine.
Reference Example H compound 35-1: [4-(3-chlorophenyl)-5-(2-piperidino-4-pyridyl)-1,3-thiazol-2-yl]amine.
   m.p.: 206 - 208°C.
Reference Example H compound 35-2: 4-(3-methylphenyl)-2-(4-methylsulfonylphenyl)-5-(2-piperidino-1-pyridyl)-1,3-thiazole m.p.: 155 - 157°C.

### Reference Example H 36

The following Reference Example H compounds 36-1 to 36-11 were synthesized according to Reference Example H 34, using 5-(2-fluoro-4-pyridyl)-4-(4-fluorophenyl)-1,3-thiazol-2-yl]amine, N-methyl-[5-(2-fluoro-4-pyridyl)-4-(3-methylphenyl)-1,3-thiazol-2-yl]amine, 2-ethyl-5-(2-fluoro-4-pyridyl)-4-(3-methylphenyl)-1,3-thiazole, 5-(2-fluoro-4-pyridyl)-4-(3-methylphenyl)-2-(4-methylsulfonylphenyl)-1,3-thiazole and 4-(3-chlorophenyl)-2-ethyl-5-(2-fluoro-4-pyridyl)-1,3-thiazole, respectively, instead of 5-(2-fluoro-4-pyridyl)-4-(3-methylphenyl)-1,3-thiazol-2-yl]amine.
Reference Example H compound 36-1: [5-(2-cyclohexylamino-4-pyridyl)-4-(4-fluorophenyl)-1,3-thiazol-2-yl]amine
   m.p.: 194 - 195°C.
Reference Example H compound 36-2: N-methyl-[5-(2-cyclohexylamino-4-pyridyl)-4-(3-methylphenyl)-1,3-thiazol-2-yl]amine
   m.p.: 211 - 212°C.
Reference Example H compound 36-3: N-methyl-[5-(2-cyclopentylamino-4-pyridyl)-4-(3-methylphenyl)-1,3-thiazol-2-yl]amine
   m.p.: 170 - 172°C.
Reference Example H compound 36-4: N-cyclohexyl-4-[2-ethyl-4-(3-methylphenyl)-1,3-thiazol-5-yl]-2-pyridylamine
   m.p.: 110 - 112°C.
Reference Example H compound 36-5: N-cyclohexyl-4-[4-(3-methylphenyl)-2-(4-methylsulfonylphenyl)-1,3-thiazol-5-yl]-2-pyridylamine
   m.p.: 197 - 199°C.
Reference Example H compound 36-6: N-cyclopentyl-4-[2-ethyl-4-(3-methylphenyl)-1,3-thiazol-5-yl]-2-pyridylamine
   m.p.: 117 - 118°C.
Reference Example H compound 36-7: N-cyclopentyl-4-[4-(3-methylphenyl)-2-(4-methylsulfonylphenyl)-1,3-thiazol-5-yl]-2-pyridylamine
   m.p.: 154 - 156°C.
Reference Example H compound 36-8: 4-(3-methylphenyl)-2-(4-methylsulfonylphenyl)-5-(2-morpholino-4-pyridyl)-1,3-thiazole
   m.p.: 200 - 202°C.
Reference Example H compound 36-9: 2-ethyl-4-(3-methylphenyl)-5-(2-morpholino-4-pyridyl)-1,3-thiazole
   m.p.: 69 - 71°C.
Reference Example H compound 36-10: 4-[4-(3-chlorophenyl)-2-ethyl-1,3-thiazol-5-yl]-N-cyclohexyl-2-pyridylamine
   m.p.: 106 - 107°C.
Reference Example H compound 36-11: 4-[4-(3-chlorophenyl)-2-ethyl-1,3-thiazol-5-yl]-N-cyclopentyl-2-pyridylamine
   m.p.: 110 - 111°C.

### Reference Example H 37

The following Reference Example H compounds 37-1 to 37-5 were synthesized according to Reference Example H 36, using pyrrolidine, N-methylcyclohexylamine, (cyclohexylmethyl)amine and 1-methylpiperazine, respectively, instead of cyclohexylamine.
Reference Example H compound 37-1: 2-ethyl-4-(3-methylphenyl)-5-[2-(1-pyrrolidinyl)-4-pyridyl]-1,3-thiazole
   m.p.: 108 - 109°C.
Reference Example H compound 37-2: N-cyclohexyl-N-methyl-4-[4-(3-methylphenyl)-2-(4-methylsulfonylphenyl)-1,3-thiazol-5-yl]-2-pyridylamine
   m.p.: 173 - 174°C.
Reference Example H compound 37-3: N-cyclohexylmethyl-4-[4-(3-methylphenyl)-2-(4-methylsulfonylphenyl)-1,3-thiazol-5-yl]-2-pyridylamine
   m.p.: 157 - 159°C.
Reference Example H compound 37-4: 4-(3-methylphenyl)-2-(4-methylsulfonylphenyl)-5-[2-(1-pyrrolidinyl)-4-pyridyl]-1,3-thiazole
   m.p.: 199 - 201°C.
Reference Example H compound 37-5: 4-(3-methylphenyl)-5-[2-(4-methyl-1-piperazinyl)-4-pyridyl]-2-(4-methylsulfonylphenyl)-1,3-thiazole
   m.p.: 153 - 154°C.

### Reference Example H 38

### N-[5-(2-acetylamino-4-pyridyl)-4-(4-methoxyphenyl)-1,3-thiazol-2-yl]acetamide

To a solution of 4-[2-amino-4-(4-methoxyphenyl)-1,3-thiazol-5-yl]-2-pyridylamine (0.40 g, 1.4 mmol) and 4-dimethylaminopyridine (0.055 g, 0.45 mmol) in N,N-dimethylacetamide (10 mL) was added acetyl chloride (0.3 mL, 4.2 mmol), and the mixture was stirred for 14 hours at 70°C. An aqueous sodium hydrogen carbonate solution was poured into the reaction mixture, and extracted with ethyl acetate. The extracts were washed with brine, then, dried and concentrated. The crude crystal was recrystallized from ethanol to obtain the title compound (0.30 g, yield 58%).
m.p.: 262 - 264°C

### Reference Example H 39

### N-[4-(3-methylphenyl)-5-(2-methyl-4-pyridyl)-1,3-thiazol-2-yl]acetamide

To a solution of [4-(3-methylphenyl)-5-(2-methyl-4-pyridyl)-1,3-thiazol-2-yl]amine (0.50 g, 1.8 mmol) and 4-dimethylaminopyridine (0.061 g, 0.50 mmol) in N,N-dimethylacetamide (15 mL) was added acetyl chloride (0.19 mL, 2.7 mmol), and the mixture was stirred for 14 hours at 80°C. An aqueous sodium hydrogen carbonate solution was poured into the reaction mixture, and the deposited solid was filtrated. The resulted solid was washed with water, then, dried. The crude crystal was recrystallized from ethanol, to obtain the title compound (0.39 g, yield 67%).
m.p.: 230 - 231°C

### Reference Example H 40

The following Reference Example H compounds 40-1 to 40-3 were synthesized according to Reference Example H 39, using [4-(3,5-dimethylphenyl)-5-(2-methyl-4-pyridyl)-1,3-thiazol-2-yl]amine, [5-(2,6-dimethyl-4-pyridyl)-4-(3-methylphenyl)-1,3-thiazol-2-yl]amine and [4-(3,5-dimethylphenyl)-5-(2,6-dimethyl-4-pyridyl)-1,3-thiazol-2-yl]amine, respectively, instead of [4-(3-methylphenyl)-5-(2-methyl-4-pyridyl)-1,3-thiazol-2-yl]amine.
Reference Example H compound 40-1: N-[4-(3,5-dimethylphenyl)-5-(2-methyl-4-pyridyl)-1,3-thiazol-2-yl]acetamide
   m.p.: 236 - 237°C.
Reference Example H compound 40-2: N-[5-(2,6-dimethyl-4-pyridyl)-4-(3-methylphenyl)-1,3-thiazol-2-yl]acetamide
   m.p.: 185 - 187°C.
Reference Example H compound 40-3: N-[4-(3,5-dimethylphenyl)-5-(2,6-dimethyl-4-pyridyl)-1,3-thiazol-2-yl]acetamide
   m.p.: 266 - 267°C.

### Reference Example H 41

The following Reference Example H compound 41 was synthesized according to Reference Example H 39, using nicotinoyl chloride hydrochloride instead of acetyl chloride. Reference Example H compound 41: N-[4-(3-methylphenyl)-5-(2-methyl-4-pyridyl)-1,3-thiazol-2-yl]nicotinamide
m.p.: 175 - 178°C.

### Reference Example H 42

The following Reference Example H compounds 42-1 to 42-10 were synthesized according to Reference Example H 41, using [4-(3,5-dimethylphenyl)-5-(2-methyl-4-pyridyl)-1,3-thiazol-2-yl]amine, [4-(3,5-dimethylphenyl)-5-(2,6-dimethyl-4-pyridyl)-1,3-thiazol-2-yl]amine, [5-(2-cyclohexylamino-4-pyridyl)-4-(3-methylphenyl)-1,3-thiazol-2-yl]amine, [5-(2-cyclopentylamino-4-pyridyl)-4-(3-methylphenyl)-1,3-thiazol-2-yl]amine, [5-(2-cyclohexylamino-4-pyridyl)-4-(4-fluorophenyl)-1,3-thiazol-2-yl]amine, [5-(2-fluoro-4-pyridyl)-4-(3-methylphenyl)-1,3-thiazol-2-yl]amine, [4-(4-fluorophenyl)-5-(2-methyl-4-pyridyl)-1,3-thiazol-2-yl]amine, N-[5-(2-cyclohexylamino-4-pyridyl)-4-(3-methylphenyl)-1,3-thiazol-2-yl]-N-methylamine, N-[5-(2-cyclopentylamino-4-pyridyl)-4-(3-methylphenyl)-1,3-thiazol-2-yl]-N-methylamine and N-methyl-N-[5-(2-methyl-4-pyridyl)-4-(3-methylphenyl)-1,3-thiazol-2-yl]amine, respectively, instead of [4-(3-methylphenyl)-5-(2-methyl-4-pyridyl)-1,3-thiazol-2-yl]amine. Reference Example H compound 42-1: N-[4-(3,5-dimethylphenyl)-5-(2-methyl-4-pyridyl)-1,3-thiazol-2-yl]nicotinamide
m.p.: 203 - 206°C
Reference Example H compound 42-2: N-[4-(3,5-dimethylphenyl)-5-(2,6-dimethyl-4-pyridyl)-1,3-thiazol-2-yl]nicotinamide
   m.p.: 267 - 268°C
Reference Example H compound 42-3: N-[5-(2-cyclohexylamino-4-pyridyl)-4-(3-methylphenyl)-1,3-thiazol-2-yl]nicotinamide
   m.p.: 201 - 203°C
Reference Example H compound 42-4: N-[5-(2-cyclopentylamino-4-pyridyl)-4-(3-methylphenyl)-1,3-thiazol-2-yl]nicotinamide
   m.p.: 215 - 216°C
Reference Example H compound 42-5: N-[5-(2-cyclohexylamino-4-pyridyl)-4-(4-fluorophenyl)-1,3-thiazol-2-yl]nicotinamide
   m.p.: 136 - 138°C
Reference Example H compound 42-6: N-[5-(2-fluoro-4-pyridyl)-4-(3-methylphenyl)-1,3-thiazol-2-yl]nicotinamide
   m.p.: 229 - 231°C
Reference Example H compound 42-7: N-[4-(4-fluorophenyl)-5-(2-methyl-4-pyridyl)-1,3-thiazol-2-yl]nicotinamide
   m.p.: 261 - 262°C
Reference Example H compound 42-8: N-[5-(2-cyclohexylamino-4-pyridyl)-4-(3-methylphenyl)-1,3-thiazol-2-yl]-N-methylnicotinamide
   m.p.: 147 - 148°C
Reference Example H compound 42-9: N-[5-(2-cyclopentylamino-4-pyridyl)-4-(3-methylphenyl)-1,3-thiazol-2-yl]N-methylnicotinamide
   m.p.: 148 - 148°C
Reference Example H compound 42-10: N-methyl-N-[5-(2-methyl-4-pyridyl)-4-(3-methylphenyl)-1,3-thiazol-2-yl]nicotinamide
   oil
   ¹H-NMR (CDCl₃) δ : 2.35 (3H, s), 2.53 (3H, s), 3,78 (3H, s), 7.05 (1H, d, J=5.2 Hz), 7.06-7.30 (4H, m), 7.41 (1H, s), 7.49 (1H, dd, J=5.2, 7.0 Hz), 7.95 (1H, d, J=7,0 Hz), 8,40 (1H, d, J=5.2 Hz), 8.80 (1H, d, J=5.2 Hz), 8.88 (1H, s).

### Reference Example H 43

The following Reference Example H compound 43 was synthesized according to Reference Example H 39 using 6-chloro-3-pyridylcarbonyl chloride hydrochloride instead of acetyl chloride.
Reference Example H compound 43: 6-chloro-N-[4-(3-methylphenyl)-5-(2-methyl-4-pyridyl)-1,3-thiazol-2-yl]nicotinamide
m.p.: 228 - 230°C

### Reference Example H 44

The following Reference Example H compounds 44-1 to 44-4 were synthesized according to Reference Example H 43, using [5-(2-cyclohexylamino-4-pyridyl)-4-(3-methylphenyl)-1,3-thiazol-2-yl]amine, [5-(2-cyclopentylamino-4-pyridyl)-4-(3-methylphenyl)-1,3-thiazol-2-yl]amine, [4-(3,5-dimethylphenyl)-5-(2-methyl-4-pyridyl)-1,3-thiazol-2-yl]amine and N-methyl-[5-(2-cyclopentylamino-4-pyridyl)-4-(3-methylphenyl)-1,3-thiazol-2-yl]amine, respectively, instead of [4-(3-methylphenyl)-5-(2-methyl-4-pyridyl)-1,3-thiazol-2-yl]amine.
Reference Example H compound 44-1: 6-chloro-N-[5-(2-cyclohexylamino-4-pyridyl)-4-(3-methylphenyl)-1,3-thiazol-2-yl]nicotinamide
   m.p.: 255 - 256°C
Reference Example H compound 44-2: 6-chloro-N-[5-(2-cyclopentylamino-4-pyridyl)-4-(3-methylphenyl)-1,3-thiazol-2-yl]nicotinamide
   m.p.: 211 - 212°C
Reference Example H compound 44-3: 6-chloro-N-[4-(3,5-dimethylphenyl)-5-(2-methyl-4-pyridyl)-1,3-thiazol-2-yl]nicotinamide
   m.p.: 271 - 273°C
Reference Example H compound 44-4: 6-chloro-N-[5-(2-cyclohexylamino-4-pyridyl)-4-(3-methylphenyl)-1,3-thiazol-2-yl]-N-methylnicotinamide
   m.p.: 171 - 172°C

### Reference Example H 45

The following Reference Example H compound 45 was synthesized according to Reference Example H 39, using 6-methyl-3-pyridylcarbonyl chloride hydrochloride instead of acetyl chloride.
Reference Example H compound 45: 6-methyl-N-[4-(3-methylphenyl)-5-(2-methyl-4-pyridyl)-1,3-thiazol-2-yl]nicotinamide
m.p.: 233 - 234°C

### Reference Example H 46

The following Reference Example H compounds 46-1 to 46-4 were synthesized according to Reference Example H 45, using [5-(2-cyclohexylamino-4-pyridyl)-4-(3-methylphenyl)-1,3-thiazol-2-yl]amine, [5-(2-cyclopentylamino-4-pyridyl)-4-(3-methylphenyl)-1,3-thiazol-2-yl]amine, [4-(3,5-dimethylphenyl)-5-(2-methyl-4-pyridyl)-1,3-thiazol-2-yl]amine and N-methyl-[5-(2-cyclopentylamino-4-pyridyl)-4-(3-methylphenyl)-1,3-thiazol-2-yl]amine, respectively, instead of [4-(3-methylphenyl)-5-(2-methyl-4-pyridyl)-1,3-thiazol-2-yl]amine.
Reference Example H compound 46-1: N-[5-(2-cyclohexylamino-4-pyridyl)-4-(3-methylphenyl)-1,3-thiazol-2-yl]-6-methylnicotinamide
   m.p.: 242 - 243°C
Reference Example H compound 46-2: N-[5-(2-cyclopentylamino-4-pyridyl)-4-(3-methylphenyl)-1,3-thiazol-2-yl]-6-methylnicotinamide
   m.p.: 213 - 214°C
Reference Example H compound 46-3: N-[4-(3,5-dimethylphenyl)-5-(2-methyl-4-pyridyl)-1,3-thiazol-2-yl]-6-methylnicotinamide
   m.p.: 252 - 253°C
Reference Example H compound 46-4: N-[5-(2-cyclopentylamino-4-pyridyl)-4-(3-methylphenyl)-1,3-thiazol-2-yl]-N,6-dimethylnicotinamide
   m.p.: 176 - 177°C

### Reference Example H 47

The following Reference Example H compound 47 was synthesized according to Reference Example H 39, using 6-methoxy-3-pyridylcarbonyl chloride hydrochloride instead of acetyl chloride.
Reference Example H compound 47: 6-methoxy-N-[4-(3-methylphenyl)-5-(2-methyl-4-pyridyl)-1,3-thiazol-2-yl]nicotinamide
m.p.: 224 - 226°C

### Reference Example H 48

The following Reference Example H compounds 48-1 and 48-3 were synthesized according to Reference Example H 47, using [5-(2-cyclohexylamino-4-pyridyl)-4-(3-methylphenyl)-1,3-thiazol-2-yl]amine, [5-(2-cyclopentylamino-4-pyridyl)-4-(3-methylphenyl)-1,3-thiazol-2-yl]amine and [4-(3,5-dimethylphenyl)-5-(2-methyl-4-pyridyl)-1,3-thiazol-2-yl]amine, respectively, instead of [4-(3-methylphenyl)-5-(2-methyl-4-pyridyl)-1,3-thiazol-2-yl]amine.
Reference Example H compound 48-1: N-[5-(2-cyclohexylamino-4-pyridyl)-4-(3-methylphenyl)-1,3-thiazol-2-yl]-6-methoxynicotinamide
   m.p.: 191 - 192°C
Reference Example H compound 48-2: N-[5-(2-cyclopentylamino-4-pyridyl)-4-(3-methylphenyl)-1,3-thiazol-2-yl]-6-methoxynicotinamide
   m.p.: 219 - 221°C
Reference Example H compound 48-3: N-[4-(3,5-dimethylphenyl)-5-(2-methyl-4-pyridyl)-1,3-thiazol-2-yl]-6-methoxynicotinamide
   m.p.: 242 - 244°C

### Reference Example H 49

The following Reference Example H compound 49 was synthesized according to Reference Example H 39, using 2-methoxy-3-pyridylcarbonyl chloride hydrochloride instead of acetyl chloride.
Reference Example H compound 49: 2-methoxy-N-[4-(3-methylphenyl)-5-(2-methyl-4-pyridyl)-1,3-thiazol-2-yl]nicotinamide
m.p.: 169 - 170°C

### Reference Example H 50

The following Reference Example H compound 50 was synthesized according to Reference Example H 39, using [5-(2-tert-butoxycarbonylamino-4-pyridyl)-3-(4-methoxyphenyl)-1,3-thiazol-2-yl]amine instead of [4-(3-methylphenyl)-5-(2-methyl-1-pyridyl)-1,3-thiazol-2-yl]amine.
Reference Example H compound 50: N-[5-(2-amino-4-pyridyl)-4-(4-methoxyphenyl)-1,3-thiazol-2-yl]acetamide
m.p.: 247 - 250°C

### Reference Example H 51

The following Reference Example H compound 51 was synthesized according to Reference Example H 50, using benzoyl chloride instead of acetyl chloride.
Reference Example H compound 51: N-[5-(2-amino-4-pyridyl)-4-(4-methoxyphenyl)-1,3-thiazol-2-yl)benzamide
m.p.: 219 - 222°C

### Reference Example H 52

### N-[4-(2,6-dimethyl-4-pyridyl)-5-(3-methylphenyl)-1,3-thiazol-2-yl]-N'-phenylurea

To a solution of [4-(2,6-dimethyl-4-pyridyl)-5-(3-methylphenyl)-1,3-thiazol-2-yl]amine (0.50 g, 1.7 mmol) in N,N-dimethylacetamide (20 mL) was added phenyl isocyanate (0.28 mL, 2.6 mmol), and the mixture was stirred for 14 hours at 80°C. Aqueous sodium hydrogen carbonate was poured into the reaction mixture, and extracted with ethyl acetate. The extracts were washed with brine, then, dried to be concentrated. The residue was purified by silica gel column chromatography (hexane-ethyl acetate= 1:1). The resulted crude crystal was recrystallized from ethyl acetate-hexane to obtain the title compound (0.34 g, yield 48%).
m.p.: 173 - 174°C.

### Reference Example H 53

The following Reference Example H compounds 53-1 to 53-4 were synthesized according to Reference Example H 52, using [4-(3,5-dimethylphenyl)-5-(2,6-dimethyl-4-pyridyl)-1,3-thiazol-2-yl]amine, [5-(2-cyclohexylamino-4-pyridyl)-4-(3-methylphenyl)-1,3-thiazol-2-yl]amine, [5-(2-cyclopentylamino-4-pyridyl)-4-(3-methylphenyl)-1,3-thiazol-2-yl]amine and [4-(3-methylphenyl)-5-(2-methyl-4-pyridyl)-1,3-thiazol-2-yl]amine, respectively, instead of [4-(2,6-dimethyl-4-pyridyl)-5-(3-methylphenyl)-1,3-thiazol-2-yl]amine.
Reference Example H compound 53-1: N-[4-(3,5-dimethylphenyl)-5-(2,6-dimethyl-4-pyridyl)-1,3-thiazol-2-yl]-N'-phenylurea
   m.p.: 219 - 222°C.
Reference Example H compound 53-2: N-[5-(2-cyclohexylamino-4-pyridyl)-4-(3-methylphenyl)-1,3-thiazol-2-yl]-N'-phenylurea
   m.p.: 198 - 199°C.
Reference Example H compound 53-3: N-[5-(2-cyclopentylamino-4-pyridyl)-4-(3-methylphenyl)-1,3-thiazol-2-yl]-N'-phenylurea
   m.p.: 188 - 190°C.
Reference Example H compound 53-4: N-[4-(3-methylphenyl)-5-(2-methyl-4-pyridyl)-1,3-thiazol-2-yl]-N'-phenylurea
   m.p.: 168 - 169°C.

### Reference Example H 54

### 4-(3-methylphenyl)-5-(2-methyl-4-pyridyl)-2-(4-methylsulfinylphenyl)-1,3-thiazole

To a solution of 4-(3-methylphenyl)-5-(2-methyl-4-pyridyl)-2-[4-(methylthio)phenyl]-1,3-thiazole (0.55 g, 1.4 mmol) in acetic acid (15 mL) was added a solution of potassium persulfate (0.43 g, 1.6 mmol) in water (8 mL), and the mixture was stirred for 14 hours at room temperature. An aqueous sodium hydrogen carbonate solution was poured into the reaction mixture, and extracted with ethyl acetate. The extracts were washed with water, dried, then, the solvent was distilled off. The residue was purified by column chromatography (filler: Chromatorex NH DM1020 (trade name, manufactured by Fuji Silysia Chemical Ltd.), hexane-ethyl acetate= 1:4), and recrystallized from ethyl acetate-hexane to obtain the title compound (0.15 g, yield 26%).
m.p.: 128 - 130°C.

### Reference Example H 55

### 5-(2,6-dimethyl-4-pyridyl)-4-(3-methylphenyl)-2-(4-methylsulfinylphenyl)-1,3-thiazole

To a solution of 5-(2,6-dimethyl-4-pyridyl)-4-(3-methylphenyl)-2-[4-(methylthio)phenyl]-1,3-thiazole (0.41 g, 1.0 mmol) in N,N-dimethylformamide (15 mL) was added m-chloroperbenzoic acid (0.25 g, 1.0 mmol), and the mixture was stirred for 1 hour at room temperature. A 8N aqueous sodium hydroxide solution was poured into the reaction mixture, and extracted with ethyl acetate. The extracted solution was washed with brine, dried, then, the solvent was distilled off. The residue was purified by column chromatography (filler: Chromatorex NH DM1020 (trade name, manufactured by Fuji Silysia Chemical Ltd.), hexane-ethyl acetate= 1:2), to obtain the title compound (0.41 g, yield 97%).
m.p.: 133 - 134°C.

### Reference Example H 56

The following Reference Example H compounds 56-1 and 56-2 were synthesized according to Reference Example H 55, using 4-(3,5-dimethylphenyl)-5-(2-methyl-4-pyridyl)-2-[4-(methylthio)phenyl]-1,3-thiazole and 4-(3,5-dimethylphenyl)-5-(2,6-dimethyl-4-pyridyl)-2-[4-(methylthio)phenyl]-1,3-thiazole instead of 5-(2,6-dimethyl-4-pyridyl)-4-(3-methylphenyl)-2-[4-(methylthio)phenyl]-1,3-thiazole
Reference Example H compound 56-1: 4-(3,5-dimethylphenyl)-5-(2-methyl-4-pyridyl)-2-(4-methylsulfinylphenyl)-1,3-thiazole m.p.: 151 - 153°C.
Reference Example H compound 56-2: 4-(3,5-dimethylphenyl)-5-(2,6-dimethyl-4-pyridyl)-2-(4-methylsulfinylphenyl)-1,3-thiazole
   m.p.: 151 - 154°C.

### Reference Example H 57

### 4-(3-methylphenyl)-5-(2-methyl-4-pyridyl)-2-(4-methylsulfonylphenyl)-1,3-thiazole

To a solution of 4-(3-methylphenyl)-5-(2-methyl-4-pyridyl)-2-[4-(methylthio)phenyl]-1,3-thiazole (0.61 g, 1.6 mmol) in N,N-dimethylformamide (15 mL) was added m-chloroperbenzoic acid (0.87 g, 3.6 mmol), and the mixture was stirred for 30 minutes at room temperature. An aqueous sodium hydrogen carbonate solution was poured into the reaction mixture, and extracted with ethyl acetate. The extracts were washed with water, dried, then, the solvent was distilled off. The residue was purified by silica gel column chromatography (hexane-ethyl acetate= 1:1), and recrystallized from ethanol to obtain the title compound (0.39 g, yield 59%).
m.p.: 134 - 138°C.

### Reference Example H 58

The following Reference Example H compounds 58-1 to 58-8 were synthesized according to Reference Example H 57, using 4-(3,5-dimethylphenyl)-5-(2-methyl-4-pyridyl)-2-[4-(methylthio)phenyl]-1,3-thiazole, 5-(2,6-dimethyl-4-pyridyl)-4-(3-methylphenyl)-2-[4-(methylthio)phenyl]-1,3-thiazole, 4-(3,5-dimethylphenyl)-5-(2,6-dimethyl-4-pyridyl)-2-[4-(methylthio)phenyl]-1,3-thiazole, 5-(2-fluoro-4-pyridyl)-4-(3-methylphenyl)-2-[4-(methylthio)phenyl]-1,3-thiazole, 4-(4-fluoropheyl)-5-(2-methyl-4-pyridyl)-2-[4-(methylthio)phenyl]-1,3-thiazole, N-[4-[4-(3-chlorophenyl)-2-[4-(methylthio)phenyl]-1,3-thiazol-5-yl]-2-pyridyl]acetamide, N-[4-[4-(3-chlorophenyl)-2-[4-(methylthio)phenyl]-1,3-thiazol-5-yl]-2-pyridyl]propionamide and N-[4-[4-(3-chlorophenyl)-2-[4-(methylthio)phenyl]-1,3-thiazol-5-yl]-2-pyridyl]pivalamide, respectively, instead of 4-(3-methylphenyl)-5-(2-methyl-4-pyridyl)-2-[4-(methylthio)phenyl]-1,3-thiazole.
Reference Example H compound 58-1: 4-(3,5-dimethylphenyl)-5-(2-methyl-4-pyridyl)-2-(4-methylsulfonylphenyl)-1,3-thiazole
   m.p.: 196 - 197°C.
Reference Example H compound 58-2: 5-(2,6-dimethyl-4-pyridyl)-4-(3-methylphenyl)-2-(4-methylsulfonylphenyl)-1,3-thiazole
   m.p.: 182 - 184°C.
Reference Example H compound 58-3: 4-(3,5-dimethylphenyl)-5-(2,6-dimethyl-4-pyridyl)-2-(4-methylsulfonylphenyl)-1,3-thiazole
   m.p.: 217 - 220°C.
Reference Example H compound 58-4: 5-(2-fluoro-4-pyridyl)-4-(3-methylphenyl)-2-(4-methylsulfonylphenyl)-1,3-thiazole
   m.p.: 195 - 199°C.
Reference Example H compound 58-5: 4-(4-fluorophenyl)-5-(2-methyl-4-pyridyl)-2-(4-methylsulfonylphenyl)-1,3-thiazole
   m.p.: 190 - 192°C.
Reference Example H compound 58-6: N-[4-[4-(3-chlorophenyl)-2-(4-methylsulfonylphenyl)-1,3-thiazol-5-yl]-2-pyridyl]acetamide
   m.p.: 216 - 217°C.
Reference Example H compound 58-7: N-[4-[4-(3-chlorophenyl)-2-(4-methylsulfonylphenyl)-1,3-thiazol-5-yl]-2-pyridyl]propionamide
   m.p.: 224 - 225°C.
Reference Example H compound 58-8: N-[4-[4-(3-chlorophenyl)-2-(4-methyisulfonylphenyl)-1,3-thiazol-5-yl]-2-pyridyl]pivalamide
   m.p.: 122 - 123°C.

### Reference Example H 59

### 4-[4-(3,5-dimethylphenyl)-2-(4-methylsulfonylphenyl)-1,3-thiazol-5-yl]-2-methylpyridine N-oxide

To a solution of 4-[4-(3,5-dimethylphenyl)-5-(2-methyl-4-pyridyl)-2-[4-(methylthio)phenyl]-1,3-thiazole (0.60 g, 1.5 mmol) in N,N-dimethylformamide (20 mL) was added m-chloroperbenzoic acid (0.80 g, 3.2 mmol), and the mixture was stirred for 30 minutes at room temperature. m-Chloroperbenzoic acid (0.11 g, 0.45 mmol) was added to the reaction mixture, and the mixture was further stirred for 20 minutes at room temperature. m-Chloroperbenzoic acid (0.38 g, 1.5 mmol) was added to the reaction mixture, and the mixture was further stirred for 20 minutes at room temperature. An aqueous sodium hydrogen carbonate solution was poured, and extracted with ethyl acetate. The extracts were washed with water, dried, then, the solvent was distilled off. The residue was purified by silica gel column chromatography (hexane-ethyl acetate= 1:4), and recrystallized from ethanol to obtain the title compound (0.30 g, yield 44%).
m.p.: 255 - 256°C.

### Reference Example H 60

### N-[4-[4-(3-chlorophenyl)-2-(1-methylpiperidin-4-yl)-1,3-thiazol-5-yl)-2-pyridyl]propionamide dihydrochloride

To a solution of N-[4-[4-(3-chlorophenyl)-2-(4-piperidyl)-1,3-thiazol-5-yl]-2-pyridyl]propionamide (0.31 g, 0.72 mmol) in N,N-dimethylformamide (8 mL)were added potassium carbonate (0.11 g, 0.82 mmol) and methyl iodide (0.045 mL, 0.72 mmol) were added sequentially, and stirred at room temperature for 20 hours. Aqueous sodium hydrogen carbonate was added to the reaction mixture and extracted with ethyl acetate. The extracts were washed with brine, dried, and concentrated. The residue was purified by column chromatography (filler: Chromatorex NH DM1020 (trade name, manufactured by Fuji Silysia Chemical Ltd.), hexane-ethyl acetate = 1 : 1) and treated with 10% solution of hydrogen chloride in methanol to obtain hydrochloride. The crude crystalline was washed with ethyl acetate to give the title compound (0.12 g, yield 32%).
m.p.: 248-253°C

### Reference Example H 61

The following Reference Example H compound 61 was synthesized according to Reference Example H 53, using 2-chloroethyl isocyanate instead of phenyl isocyanate. Reference Example H compound 61: N-(2-chloroethyl)-N'-[5-(2-methyl-4-pyridyl)-4-(3-methylphenyl)-1,3-thiazol-2-yl]urea
m.p.: 149 - 151°C.

### Reference Example H 62

The following Reference Example H compound 62 was synthesized according to Reference Example H 39, using 2-chloroethyl chloroformate instead of acetyl chloride. Reference Example H compound 62: 2-chloroethyl [4-(3-methylphenyl)-5-(2-methyl-4-pyridyl)-1,3-thiazol-2-yl]carbamate
m.p.: 156 - 158°C.

### Reference Example H 63

### N-methoxy-N'-[4-(3-methylphenyl)-5-(2-methyl-4-pyridyl)-1,3-thiazol-2-yl]urea

To a solution of [4-(2-methyl-4-pyridyl)-3-(3-methylphenyl)-1,3-thiazol-2-yl]amine (0.50 g, 1.8 mmol) in N,N-dimethylacetamide (20 mL) was added 1,1-carbonyldiimidazole (0.43 g, 2.7 mmol), and the mixture was stirred for 3 hours at room temperature. A 0-methylhydroxylamine hydrochloride (0.22 g, 2,7 mmol) was added to the reaction mixture, and the mixture was stirred for 24 hours at room temperature. An aqueous sodium hydrogen carbonate solution was poured into the reaction mixture, and the produced solid was filtrated. This solid was washed with water, and dried. The crude product was purified by column chromatography (filler: Chromatorex NH DM1020 (trade name, manufactured by Fuji Silysia Chemical Ltd.), ethyl acetate). The resulted crystal was recrystallized from ethyl acetate to obtain the title compound (0.16 g, yield 25%).
m.p.: 194 - 195°C.

### Reference Example H 64

The following Reference Example H compound 64 was synthesized according to Reference Example H 63, using 0-phenylhydroxylamine hydrochloride instead of 0-methylhydroxylamine hydrochloride.
Reference Example H compound 64: N-[4-(3-methylphenyl)-5-(2-methyl-4-pyridyl)-1,3-thiazol-2-yl]-N'-phenyloxyurea
m.p.: 154 - 155°C

### Reference Example H 65

### 3-[4-(3-methylphenyl)-5-(2-methyl-4-pyridyl)-1,3-thiazol-5-yl]-oxazolidin-2-one

To a solution of 2-chloroethyl [4-(3-methylphenyl)-5-(2-methyl-4-pyridyl)-1,3-thiazol-2-yl]carbamate (0.30 g, 0.80 mmol) in N,N-dimethylformamide (3 mL) was added potassium tert-butoxide (0.12 g, 1.1 mmol), and the mixture was stirred at room temperature for 1 hour. An aqueous sodium hydrogen carbonate solution was poured into the reaction mixture, and extracted with ethyl acetate. The extracts were washed with water, dried, and concentrated. The residue was purified by silica gel column chromatography (hexane-ethyl acetate= 4:1) to obtain a crystal. This crystal was recrystallized from hexane-ethyl acetate, to obtain the title compound (0.20 g, yield 72%).
m.p.: 80 - 82°C

### Reference Example H 66

The following Reference Example H compound 66 was synthesized according to Reference Example H 65, using N-(2-chloroethyl)-N'-[5-(2-methyl-4-pyridyl)-4-(3-methylphenyl)-1,3-thiazol-2-yl]urea instead of 2-chloroethyl [4-(3-methylphenyl)-5-(2-methyl-4-pyridyl)-1,3-thiazol-2-yl]carbamate.
Reference Example H compound 66: 1-[4-(3-methylphenyl)-5-(2-methyl-4-pyridyl)-1,3-thiazol-2-yl]imidazolidin-2-one
m.p.: 200 - 201°C

### Reference Example H 67

### 1-[4-(3-methylphenyl)-5-(2-methyl-4-pyridyl)-1,3-thiazol-2-yl]-3-phenylimidazolidin-2-one

To a suspension of 1-[4-(3-methylphenyl)-5-(2-methyl-4-pyridyl)-1,3-thiazol-2-yl]-3-imidazolidin-2-one (0.42 g, 1.2 mmol), copper powder (0.23 g, 3.6 mmol), copper chloride (0.01 g, 0.12 mmol) and potassium acetate (0.23 g, 2.4 mmol) in N,N-dimethylacetamide (10 mL) was added bromobenzene (0.56 g, 3.6 mmol), and the mixture was stirred at 150°C for 4 hours. After filtration, water was added, and extracted with ethyl acetate. The extracts were washed with water, then, dried, and concentrated. The residue was purified by silica gel column chromatography (hexane-ethyl acetate= 4:1) to obtain crude crystal. This crude crystal was recrystallized from ethyl acetate, to obtain the title compound (0.18 g, yield 35%).
m.p.: 180 - 182°C

### Reference Example H 68

The following Reference Example H compounds 68-1 and 68-2 were synthesized according to Reference Example H 59, using 4-(3-methylphenyl)-5-(2-methyl-4-pyridyl)-2-[4-(methylthio)phenyl]-1,3-thiazole and 2-ethyl-4-(3-methylphenyl)-5-(2-methyl-4-pyridyl)-1,3-thiazole instead of 4-(3,5-dimethylphenyl)-5-(2-methyl-4-pyridyl)-2-[4-(methylthio)phenyl]-1,3-thiazole.
Reference Example H compound 68-1: 4-[4-(3-methylphenyl)-2-(4-methylsulfonylphenyl)-1,3-thiazol-5-yl]-2-methylpyridine N-oxide
   m.p.: 197 - 198°C
Reference Example H compound 68-2: [2-ethyl-4-(3-methylphenyl)-1,3-thiazol-5-yl]-2-methylpyridine N-oxide
   oil
   ¹H-NMR (CDCl₃) δ : 1.41 (3H, t, J=7.6 Hz), 2.34 (3H, s), 2.46 (3H, s), 3.09 (2H, q, J=7.6 Hz), 7.01 (1H, dd, J=2.2, 7.0 Hz), 7.12-7.24 (4H, m), 8.10 (1H, d, J=2.0 Hz).

### Reference Example H 69

### 5-(2-chloro-4-pyridyl)-2-ethyl-4-(3-methylphenyl)-1,3-thiazole

A solution of 4-[2-ethyl-4-(3-methylphenyl)-1,3-thiazol-5-yl]pyridine N-oxide (1.00 g, 3.37 mmol) in phosphorus oxychloride (6.5 mL) was stirred at 100°C for 2 hours. The reaction solution was cooled, and poured into a saturated aqueous sodium hydrogen carbonate solution, and extracted with ethyl acetate. The extracts were washed with brine, then, dried, and concentrated. The residue was purified by silica gel column chromatography (hexane-ethyl acetate= 2:1) to obtain the title compound (0.90 g, yield 81%).
oil
¹H-NMR (CDCl₃) δ : 1.42 (3H, t, J=7.8 Hz), 2.35 (3H, s), 3.10 (2H, q, J=7.8 Hz), 7.09 (1H, dd, J=1.4, 5.2 Hz), 7.12-7.30 (4H, m), 7.37 (1H, s), 8.22-8.27 (1H, m).

### Reference Example H 70

A solution of 4-[2-ethyl-4-(3-methylphenyl)-1,3-thiazol-5-yl]pyridine N-oxide (1.0 g, 3.2 mmol) in phosphorus oxychloride (8 mL) was stirred at 100°C for 1 hour. The reaction solution was cooled, and poured into a saturated aqueous sodium hydrogen carbonate solution, and extracted with ethyl acetate. The extracts were washed with brine, then, dried, and concentrated. The residue was purified by silica gel column chromatography (hexane-ethyl acetate= 10:1 to 2:1) to 5-(2-chloro-6-methyl-4-pyridyl)-2-ethyl-4-(3-methylphenyl)-1,3-thiazole (0.60 g, yield 57%) and 5-(2-chloromethyl-4-pyridyl)-2-ethyl-4-(3-methylphenyl)-1,3-thiazole (0.20 g, yield 19%).
Reference Example H compound 70-1: 5-(2-chloro-6-methyl-4-pyridyl)-2-ethyl-4-(3-methylphenyl)-1,3-thiazole
   oil
   ¹H-NMR (CDCl₃) δ : 1.45 (3H, t, J=7.8 Hz), 2.35 (3H, s), 2.45 (3H, s), 3.09 (2H, q, J=7.8 Hz), 6.98 (1H, s), 7.06 (1H, s), 7.12-7.24 (3H, m), 7.38 (1H, s).
Reference Example H compound 70-2: 5-(2-chloromethyl-4-pyridyl)-2-ethyl-4-(3-methylphenyl)-1,3-thiazole
   oil
   ¹H-NMR (CDCl₃) δ : 1.46 (3H, t, J=7.6 Hz), 2.33 (3H, s), 3.10 (2H, q, J=7.6 Hz), 4.59 (2H, s), 7.10-7.23 (4H, m), 7.35-7.40 (2H, m), 8.42-8.47 (1H, m).

### Reference Example H 71

### 5-(2-cyanomethyl-4-pyridyl)-2-ethyl-4-(3-methylphenyl)-1,3-thiazole

A suspension of 5-(2-chloromethyl-4-pyridyl)-2-ethyl-4-(3-methylphenyl)-1,3-thiazole (0.40 g, 1.2 mmol), potassium cyanide (0.095 g, 1.5 mmol), 18-crown-6 (0.14 g, 0.51 mmol) in acetonitrile (5 mL) was heated under reflux for 6 hours. After cooling, an aqueous potassium carbonate solution was added, and extracted with ethyl acetate. The extracts were washed with brine, then, dried, and concentrated. The residue was purified by silica gel column chromatography (hexane-ethyl acetate= 2:1). The resulting crystal was washed with hexane-isopropyl ether to obtain the title compound (0.19 g, 48%).
m.p.: 68 - 69°C

### Reference Example H 72

### 4-[4-(3-methylphenyl)-2-(4-methylsulfonylphenyl)-1,3-thiazol-5-yl]-2-pyridylmethanol

To a solution of 4-[4-(3-methylphenyl)-2-(4-methylsulfonylphenyl)-1,3-thiazol-5-yl]pyridine N-oxide (0.43 g, 1.0 mmol) in methylene chloride (10 mL) was added trimethyloxonium tetrafluoroborate (0.17 g, 1.2 mmol), and the mixture was stirred for 2 hours. The reaction mixture was concentrated under reduced pressure, and methanol (15 mL) was added to the residue. To the mixture was added a solution of ammonium persulfate (0.05 g, 0.22 mmol) in water (1 mL) under reflux, and the mixture was heated to reflux for 30 minutes. A solution of ammonium persulfate (0.03 g, 0.11 mmol) in water (1 mL) was added to the reaction mixture, and the mixture was further heated to reflux for 13 hours. The reaction mixture was cooled to room temperature, then, concentrated under reduced pressure. To the residue was added an aqueous sodium hydrogen carbonate solution, and extracted with ethyl acetate. The extracts were washed with brine, dried over magnesium sulfate, and filtrated and concentrated under reduced pressure. The residue was purified by column chromatography (filler: Chromatorex NH DM1020 (trade name, manufactured by Fuji Silysia Chemical Ltd.), ethyl acetate) to obtain the title compound (0.26 g, yield 61%).
m.p.: 172 - 173°C

### Reference Example H 73

### 2-(4-methylsulfonylphenyl)-4-(3-methylphenyl)-5-[2-(1-pyrrolidinylmethyl)-4-pyridyl]-1,3-thiazole dihydrochloride

To a solution of 4-[4-(3-methylphenyl)-2-(4-methylsulfonylphenyl)-1,3-thiazol-5-yl]-2-pyridylmethanol (0.43 g, 1.0 mmol) in tetrahydrofuran (20 mL) was added thionyl chloride (0.08 mL, 1.0 mmol), and the mixture was stirred for 20 minutes. The reaction mixture was concentrated under reduced pressure, and the residue was added to a suspension of pyrrolidine (0.09 mL, 1.1 mmol) and potassium carbonate (0.36 g, 2.6 mmol) in N,N-dimethylformamide (10 mL), and the mixture was stirred for 27 hours at room temperature. To the mixture was added an aqueous sodium hydrogen carbonate solution, and extracted with ethyl acetate. The extracts were washed with brine, dried over magnesium sulfate, and filtrated and concentrated under reduced pressure. The residue was purified by column chromatography (filler: Chromatorex NH DM1020 (trade name, manufactured by Fuji Silysia Chemical Ltd.), hexane-ethyl acetate=1:2). The resulted oil was dissolved in methanol (10 mL), and to this was added a 10% hydrogen chloride-methanol solution (5 mL), and the mixture was stirred for 1 hour at room temperature. The reaction mixture was concentrated under reduced pressure, and the resulting solid was washed with ethanol, to obtain the title compound (yield 17%). amorphous powder
¹H-NMR (CDCl₃) δ : 1.90-2.10 (4H, m), 2.33 (3H, s), 3.31 (3H, s), 4.10-4.40 (4H, m), 4.53 (2H, s), 7.28 (3H, s), 7.43-7.47 (2H, m), 7.67 (1H, s), 8.12 (2H, d, J=8.0 Hz), 8.30 (2H, d, J=8.0 Hz), 8.68 (1H, d, J=5.2 Hz).

### Reference Example H 74

### 2-ethyl-4-(3-methylphenyl)-5-[2-(1-pyrrolidinylmethyl)-4-pyridyl]-1,3-thiazole dihydrochloride

To a solution of 5-(2-chloromethyl-4-pyridyl)-2-ethyl-4-(3-methylphenyl)-1,3-thiazole (0.20 g, 0.61 mmol) in pyrrolidine (0.5 mL) was stirred for 1 hour at 80°C. After cooling, to this was added an aqueous potassium carbonate solution, and extracted with ethyl acetate. The extracts were washed with brine, then, dried and concentrated. The residue was purified by alumina column chromatography (hexane-ethyl acetate=2:1). The resulting oil was made into a hydrochloride by using a solution of 4N-hydrogen chloride in ethyl acetate, and recrystallized from ethanol-isopropyl ether, to obtain the title compound (0.23 g, 85%).
m.p.: 146 - 151°C

### Reference Example H 75

To a solution of 2-bromo-1-(3-bromophenyl)-2-[2-(tert-butoxycarbonylamino)-4-pyridyl]ethanone hydrobromide (22 g, 51 mmol) in N,N,-dimethylformamide (100 mL) was added thiopropionamide (4.3 g, 49 mmol), and the mixture was stirred for 2 hours at room temperature. Into the reaction mixture was poured an aqueous sodium hydrogen carbonate solution, and extracted with ethyl acetate. The extracts were washed with water, then, dried and concentrated. The residue was purified by silica gel column chromatography (hexane-ethyl acetate=4:1), then, recrystallized from hexane-ethyl acetate to obtain 4-(3-bromophenyl)-5-[2-(tert-butoxycarbonylamino)-4-pyridyl]-2-ethyl-1,3-thiazole (6.0 g, yield 27%) and 5-(2-amino-4-pyridyl)-4-(3-bromophenyl)-2-ethyl-1,3-thiazole (1.4 g, yield 8%).
Reference Example H compound 75-1: 4-(3-bromophenyl)-5-[2-(tert-butoxycarbonylamino)-4-pyridyl]-2-ethyl-1,3-thiazole m.p.: 172 - 174°C
Reference Example H compound 75-2: 4-[4-(3-bromophenyl)-2-ethyl-1,3-thiazol-5-yl]-2-pyridylamine
   m.p.: 132 - 134°C

### Reference Example H 76

### 4-[4-(3-cyanophenyl)-2-ethyl-1,3-thiazol-5-yl]-2-pyridylamine

To a solution of 4-(3-bromophenyl)-5-[2-(tert-butoxycarbonylamino)-4-pyridyl]-2-ethyl-1,3-thiazole (0.5 g, 1.1 mmol) in N,N-dimethylformamide (10 mL) was added copper cyanide (0.25 g, 1.6 mmol), and the mixture was stirred for 20 hours at 150°C under argon atmosphere. Into the reaction mixture was poured ammonia water, and the deposit was removed, then, extracted with ethyl acetate. The extracts were washed with water, then, dried and concentrated. The residue was purified by silica gel column chromatography (hexane-ethyl acetate=4:1) to obtain a crystal. This crystal was washed with hexane-ethyl acetate to obtain the title compound (0.19 g, yield 57%).
m.p.: 178 - 179°C

### Reference Example H 77

### 3-[5-(2-amino-4-pyridyl)-2-ethyl-1,3-thiazol-4-yl]benzoic acid

To a solution of 5-(2-amino-4-pyridyl)-4-(3-cyanophenyl)-2-ethyl-1,3-thiazole (0,50 g, 1.6 mmol) in acetic acid (5 mL) was added 50% sulfuric acid (2.0 mL), and the mixture was stirred for 6 hours at 110°C. The reaction mixture was basified with aqueous sodium hydroxide solution and washed with ethyl acetate. The aqueous phase was neutralized with hydrochloric acid, and the deposited crystal was washed with water and ethyl ether to obtain the title compound (0.45 g, yield 84%).
m.p.: 273 - 274°C

### Reference Example H 78

### methyl 3-[5-(2-amino-4-pyridyl)-2-ethyl-1,3-thiazol-4-yl]benzoate

To a solution of 3-[5-(2-amino-4-pyridyl)-4-(3-cyanophenyl)-2-ethyl-1,3-thiazol-4-yl]benzoic acid (0.3 g, 1.0 mmol) in methanol (10 mL) was added concentrated sulfuric acid (0.1 mL) and the mixture was stirred for 5 hours at 70°C. The reaction mixture was basified with sodium hydroxide solution and extracted with ethyl acetate. The extracts were washed with water, then, dried and concentrated. The residue was washed with hexane-ethyl acetate to obtain the title compound (0.29 g, yield 85%).
m.p.: 173 - 174°C

### Reference Example H 79

To a solution of 4-(3-bromophenyl)-5-[2-(tert-butoxycarbonylamino)-4-pyridyl]-2-ethyl-1,3-thiazole (1.0 g, 2.2 mmol) in tetrahydrofuran (20 mL) was added dropwise a 1.5 M n-butyllithium hexane solution (2.9 mL, 4.3 mmol), and the mixture was stirred for 15 minutes. The reaction mixture was poured onto dry ice, and extracted with ethyl acetate-tetrahydrofuran. The extracts were washed with an aqueous sodium hydroxide solution, then, dried and concentrated. The residue was recrystallized from hexane-ethyl acetate to obtain 5-[2-(tert-butoxycarboylamino)-4-pyridyl]-2-ethyl-4-phenyl-1,3-thiazole (0.29 g, yield 35%). The aqueous layer was made acidic with hydrochloric acid, then, extracted with ethyl acetate-tetrahydrofuran. The extracts were washed with water, then, dried and concentrated. The residue was washed with ethyl acetate to obtain [5-[2-(tert-butoxycarboylamino)-4-pyridyl]-2-ethyl-1,3-thiazol-4-yl]benzoic acid (0.21 g, yield 23%).
Reference Example H compound 79-1: 5-[2-(tert-butoxycarbonylamino)-4-pyridyl]-2-ethyl-4-phenyl-1,3-thiazole
   m.p.: 154 - 155°C
Reference Example H compound 79-2: 3-[5-[2-(tert-butoxycarbonylamino)-4-pyridyl]-2-ethyl-1,3-thiazol-4-yl]benzoic acid
   ¹H-NMR (CDCl₃) δ : 1.37 (3H, t, J=7.5 Hz), 1.45 (9H, s) , 3.08 (2H, q, J=7.5 Hz), 6.83 (1H, dd, J=1.4, 5.0 Hz), 7,34-7.37 (2H, m), 7.42-7.49 (2H, m), 7.86 (1H, s), 8.16 (1H, d, J=5.0 Hz), 9.91 (1H, s).

### Reference Example H 80

### 4-[2-(1-tert-butoxycarbonylpiperidin-4-yl)-4-(3-chlorophenyl)-1,3-thiazol-5-yl]-2-pyridylamine

A solution of 2-(2-amino-4-pyridyl)-2-bromo-1-(3-chlorophenyl)ethanone hydrobromide (2.74 g, 8.36 mmol) and 1-tert-butoxycarbonylpiperidine-4-carbothioamide in N,N-dimethylformamide (50 mL) was stirred at room temperature for 3 hours. Aqueous sodium hydrogen carbonate was added to the reaction mixture and extracted with ethyl acetate. The extracts were washed with water, dried, and concentrated. The residue was purified by silica gel column chromatography (ethyl acetate) and then purified by column chromatography (filler: Chromatorex NH DM1020 (trade name, manufactured by Fuji Silysia Chemical Ltd.), ethyl acetate). The obtained crude crystalline was recrystallized from ethyl acetate-hexane to give the title compound (1.94 g, yield 50%).
m.p.: 143-145°C

### Reference Example H 81

### N-[4-[2-(1-tert-butoxycarbonylpiperidin-4-yl)-4-(3-chlorophenyl)-1,3-thiazol-5-yl]-2-pyridyl]acetamide

To a solution of [4-[2-(1-tert-butoxycarbonylpiperidin-4-yl)-4-(3-chlorophenyl)-1,3-thiazol-5-yl]-2-pyridylamine (1.60 g, 3.40 mmol) in tetrahydrofuran (20 mL) were added acetyl chloride (0.25 mL, 3.52 mmol) and triethylamine (0.50 mL, 3.58 mmol) at 0°C sequentially, and the resulting mixture was stirred at room temperature for 3 hours. Aqueous sodium hydrogen carbonate was added to the reaction mixture and extracted with ethyl acetate. The extracts were washed with brine, dried, and concentrated. The residue was purified by column chromatography (filler: Chromatorex NH DM1020 (trade name, manufactured by Fuji Silysia Chemical Ltd.), hexane-ethyl acetate = 1 : 1) to give the title compound (1.79 g, yield 98%).
amorphous powder
¹H-NMR(CDCl₃) d: 1.49 (9H, s), 1.68-1.88 (2H, m), 2.13-2.21 (5H, m), 2.91 (2H, br t, J= 12.0 Hz), 3.12-3.25 (1H, m). 4.20-4.27 (2H, m), 6.87 (1H, dd, J= 1.8, 5.4Hz), 7.18-7.35 (3H, m), 7.56 (1H, t, J= 1.8 Hz), 8.15 (1H, d, J= 5.4 Hz), 8.27-8.33 (2H, m).

### Reference Example H 82

The following Reference Example H compounds 82-1 to 82-12 were synthesized according to Reference Example H 81, using 4-(2-ethyl-4-phenyl-1,3-thiazol-5-yl)-2-pyridylamine, 4-[2-ethyl-4-(4-fluorophenyl)-1,3-thiazol-5-yl]-2-pyridylamine, 4-[2-ethyl-4-(3-trifluoromethylphenyl)-1,3-thiazol-5-yl]-2-pyridylamine, 4-[2-ethyl-4-(4-fluoro-3-methylphenyl)-1,3-thiazol-5-yl]-2-pyridylamine, 4-[2-ethyl-4-(3-fluorophenyl)-1,3-thiazol-5-yl]-2-pyridylamine, 4-[2-ethyl-4-(4-chlorophenyl)-1,3-thiazol-5-yl]-2-pyridylamine, 4-[2-ethyl-4-(3-ethylphenyl)-1,3-thiazol-5-yl]-2-pyridylamine, 4-[2-ethyl-4-[3-(1-methylethyl)phenyl]-1,3-thiazol-5-yl]-2-pyridylamine, 4-[2-ethyl-4-(3-propylphenyl)-1,3-thiazol-5-yl]-2-pyridylamine, 4-[2-methyl-4-(3-methylphenyl)-1,3-thiazol-5-yl]-2-pyridylamine, 4-[4-(3-methylphenyl)-1,3-thiazol-5-yl]-2-pyridylamine and 4-[4-(3-methylphenyl)-2-[4-(methylthio)phenyl]-1,3-thiazol-5-yl]-2-pyridylamine, respectively, instead of 4-[2-(1-tert-butoxycarbonylpiperidin-4-yl)-4-(3-chlorophenyl)-1,3-thiazol-5-yl]-2-pyridylamine.
Reference Example H compound 82-1: N-[4-(2-ethyl-4-phenyl-1,3-thiazol-5-yl)-2-pyridyl]acetamide
   m.p.: 175-176°C
Reference Example H compound 82-2: N-[4-[2-ethyl-4-(4-fluorophenyl)-1,3-thiazol-5-yl]-2-pyridyl]acetamide
   m.p.: 190-191°C
Reference Example H compound 82-3: N-[4-[2-ethyl-4-(3-trifluoromethylphenyl)-1,3-thiazol-5-yl]-2-pyridyl]acetamide
   m.p.: 146-147°C
Reference Example H compound 82-4: N-[4-[2-ethyl-4-(4-fluoro-3-methylphenyl)-1,3-thiazol-5-yl]-2-pyridyl]acetamide
   m.p.: 142-143°C
Reference Example H compound 82-5: N-[4-[2-ethyl-4-(3-fluorophenyl)-1,3-thiazol-5-yl]-2-pyridyl]acetamide
   m.p.: 141-142°C
Reference Example H compound 82-6: N-[4-[4-(4-chlorophenyl)-2-ethyl-1,3-thiazol-5-yl]-2-pyridyl]acetamide
   m.p.: 190-191°C
Reference Example H compound 82-7: N-[4-[2-ethyl-4-(3-ethylphenyl)-1,3-thiazol-5-yl]-2-pyridyl]acetamide
   m.p.: 112-113°C
Reference Example H compound 82-8: N-[4-[2-ethyl-4-[3-(1-methylethyl)phenyl]-1,3-thiazol-5-yl]-2-pyridyl]acetamide m.p.: 116-117°C
Reference Example H compound 82-9: N-[4-[2-ethyl-4-(3-propylphenyl)-1,3-thiazol-5-yl]-2-pyridyl]acetamide
   m.p.: 121-122°C
Reference Example H compound 82-10: N-[4-[2-methyl-4-(3-methylphenyl)-1,3-thiazol-5-yl]-2-pyridyl]acetamide
   m.p. : 162-163°C
Reference Example H compound 82-11: N-[4-[4-(3-methylphenyl)-1,3-thiazol-5-yl]-2-pyridyl]acetamide
   m.p.: 149-150°C
Reference Example H compound 82-12: N-[4-[4-(3-methylphenyl)-2-[4-(methylthio)phenyl]-1,3-thiazol-5-yl]-2-pyridyl]acetamide m.p.: 181-182°C

### Reference Example H 83

The following Reference Example H compound 83 was synthesized according to Reference Example H 81, using propionyl chloride instead of acetyl chloride.
Reference Example H compound 83: N-[4-[2-(1-tert-butoxycarbonylpiperidin-4-yl)-4-(3-chlorophenyl)-1,3-thiazol-5-yl]-2-pyridyl]propionamide
amorphous powder
¹H-NMR(CDCl₃) d: 1.25 (3H, t, J= 7.5 Hz), 1.49 (9H, s), 1.66-1.89 (2H, m), 2.08-2.22 (2H, m), 2.44 (2H, q, J= 7.5 Hz), 2.82-3.00 (2H, m), 3.11-3.24 (1H, m). 4.18-4.30 (2H, m), 6.84 (1H, dd, J= 1.8, 5.0 Hz), 7.19-7.36 (3H, m), 7.56 (1H, t, J= 3.2 Hz), 8.13 (1H, d, J= 5.0 Hz), 8.18 (1H, br s), 8.33 (1H, s).

### Reference Example H 84

The following Reference Example H compounds 84-1 to 84-9 were synthesized according to Reference Example H 83, using 4-(2-ethyl-4-phenyl-1,3-thiazol-5-yl)-2-pyridylamine, 4-[2-ethyl-4-(4-fluorophenyl)-1,3-thiazol-5-yl]-2-pyridylamine, 4-[2-ethyl-4-(3-trifluoromethylphenyl)-1,3-thiazol-5-yl]-2-pyridylamine, 4-[2-ethyl-4-(4-fluoro-3-methylphenyl)-1,3-thiazol-5-yl]-2-pyridylamine, 4-[2-ethyl-4-(3-fluorophenyl)-1,3-thiazol-5-yl]-2-pyridylamine, 4-[2-ethyl-4-(4-chlorophenyl)-1,3-thiazol-5-yl]-2-pyridylamine, 4-[2-ethyl-4-[3-(1-methylethyl)phenyl]-1,3-thiazol-5-yl]-2-pyridylamine, 4-[2-ethyl-4-(3-propylphenyl)-1,3-thiazol-5-yl]-2-pyridylamine and 4-[4-(3-methylphenyl)-2-[4-(methylthio)phenyl]-1,3-thiazol-5-yl]-2-pyridylamine, respectively, instead of 4-[2-(1-tert-butoxycarbonylpiperidin-4-yl)-4-(3-chlorophenyl)-1,3-thiazol-5-yl]-2-pyridylamine.
Reference Example H compound 84-1: N-[4-(2-ethyl-4-phenyl-1,3-thiazol-5-yl)-2-pyridyl]propionamide
   m.p.: 139-140°C
Reference Example H compound 84-2: N-[4-[2-ethyl-4-(4-fluorophenyl)-1,3-thiazol-5-yl]-2-pyridyl]propionamide
   m.p.: 156-157°C
Reference Example H compound 84-3: N-[4-[2-ethyl-4-(3-trifluoromethylphenyl)-1,3-thiazol-5-yl]-2-pyridyl]propionamide
   m.p.: 126-127°C
Reference Example H compound 84-4: N-[4-[2-ethyl-4-(4-fluoro-3-methylphenyl)-1,3-thiazol-5-yl]-2-pyridyl]propionamide
   m.p.: 105-107°C
Reference Example H compound 84-5: N-[4-[2-ethyl-4-(3-fluorophenyl)-1,3-thiazol-5-yl]-2-pyridyl]propionamide
   m.p.: 121-122°C
Reference Example H compound 84-6: N-[4-[4-(4-chlorophenyl)-2-ethyl-1,3-thiazol-5-yl]-2-pyridyl]propionamide.
   m.p.: 152-153°C
Reference Example H compound 84-7: N-[4-[2-ethyl-4-[3-(1-methylethyl)phenyl]-1,3-thiazol-5-yl]-2-pyridyl]propionamide
   m.p.: 93-94°C
Reference Example H compound 84-8: N-[4-[2-ethyl-4-(3-propylphenyl)-1,3-thiazol-5-yl]-2-pyridyl]propionamide
   m.p.: 124-125°C
Reference Example H compound 84-9: N-[4-[4-(3-methylphenyl)-2-[4-(methylthio)phenyl]-1,3-thiazol-5-yl]-2-pyridyl]propionamide
   m.p.: 171-172°C

### Reference Example H 85

The following Reference Example H compound 85 was synthesized according to Reference Example H 28, using butyryl chloride instead of cyclohexanecarbonyl chloride.
Reference Example H compound 85: N-[4-[2-ethyl-4-(3-methylphenyl)-1,3-thiazol-5-yl]-2-pyridyl]butyramide
m.p.: 88-89°C

### Reference Example H 86

The following Reference Example H compound 86.was synthesized according to Reference Example H 85, using 4-[4-(3-chlorophenyl)-2-ethyl-1,3-thiazol-5-yl]-2-pyridylamine instead of 4-[2-ethyl-4-(3-methylphenyl)-1,3-thiazol-5-yl]-2-pyridylamine.
Reference Example H compound 86: N-[4-[4-(3-chlorophenyl)-2-ethyl-1,3-thiazol-5-yl]-2-pyridyl]butyramide
m.p.: 119-120°C

### Reference Example H 87

The following Reference Example H compounds 87-1 and 87-2 were synthesized according to Reference Example H 85, using valeryl chloride and hexanoyl chloride, respectively, instead of butyryl chloride.
Reference Example H compound 87-1: N-[4-[2-ethyl-4-(3-methylphenyl)-1,3-thiazol-5-yl]-2-pyridyl]valeramide
   m.p.: 81-82°C
Reference Example H compound 87-2: N-[4-[2-ethyl-4-(3-methylphenyl)-1,3-thiazol-5-yl]-2-pyridyl]hexanamide
   m.p.: 84-85°C

### Reference Example H 88

The following Reference Example H compounds 88-1 and 88-2 were synthesized according to Reference Example H 86, using valeryl chloride and hexanoyl chloride, respectively, instead of butyryl chloride.
Reference Example H compound 88-1: N-[4-[4-(3-chlorophenyl)-2-ethyl-1,3-thiazol-5-yl]-2-pyridyl]valeramide
   m.p.: 109-110°C
Reference Example H compound 88-2: N-[4-[4-(3-chlorophenyl)-2-ethyl-1,3-thiazol-5-yl]-2-pyridyl]hexanamide
   m.p.: 114-115°C

### Reference Example H 89

The following Reference Example H compound 89 was synthesized according to Reference Example H 28, using cyclopentylacetyl chloride instead of cyclohexanecarbonyl chloride.
Reference Example H compound 89: N-[4-[2-ethyl-4-(3-methylphenyl)-1,3-thiazol-5-yl]-2-pyridyl]cyclopentylacetamide
m.p.: 85-86°C

### Reference Example H 90

The following Reference Example H compounds 90-1 and 90-2 were synthesized according to Reference Example H 89, using 4-[4-(3-chlorophenyl)-2-ethyl-1,3-thiazol-5-yl]-2-pyridylamine and 4-[4-(4-chlorophenyl)-2-ethyl-1,3-thiazol-5-yl]-2-pyridylamine, respectively, instead of 4-[2-ethyl-4-(3-methylphenyl)-1,3-thiazol-5-yl]-2-pyridylamine.
Reference Example H compound 90-1: N-[4-[4-(3-chlorophenyl)-2-ethyl-1,3-thiazol-5-yl]-2-pyridyl]cyclopentylacetamide
   m.p.: 121-122°C
Reference Example H compound 90-2: N-[4-[4-(4-chlorophenyl)-2-ethyl-1,3-thiazol-5-yl]-2-pyridyl]cyclopentylacetamide
   m.p.: 149-150°C

### Reference Example H 91

### N-[4-[4-(3-chlorophenyl)-2-(4-piperidyl)-1,3-thiazol-5-yl]-2-pyridyl]acetamide dihydrochloride

To a solution of N-[4-[2-(1-tert-butoxycarbonylpiperidin-4-yl)-4-(3-chlorophenyl)-1,3-thiazol-5-yl]-2-pyridylacetamide (1.44 g, 2.81 mmol) in methanol (10 mL) was added 2N-hydrochloric acid (4 mL) and stirred at 80°C for an hour. The solvent was removed under reduced pressure and the residue was recrystallized from methanol to give the title compound (0.87 g, yield 64%).
m.p.: 193-195°C

### Reference Example H 92

The following Reference Example H compound 92 was synthesized according to Reference Example H 91, using N-[4-[2-(1-tert-butoxycarbonylpiperidin-4-yl)-4-(3-chlorophenyl)-1,3-thiazol-5-yl]-2-pyridyl]propionamide instead of N-[4-[2-(1-tert-butoxycarbonylpiperidin-4-yl)-4-(3-chlorophenyl)-1,3-thiazol-5-yl]-2-pyridyl]acetamide.
Reference Example H compound 92: N-[4-[4-(3-chlorophenyl)-2-(4-piperidyl)-1,3-thiazol-5-yl]-2-pyridyl]propionamide dihydrochloride
m.p.: 202-203°C

### Reference Example H 93

### N-[4-[2-(1-acetylpiperidin-4-yl)-4-(3-chlorophenyl)-1,3-thiazol-5-yl]-2-pyridyl]acetamide

To a suspension of N-[4-[4-(3-chlorophenyl)-2-(4-piperidyl)-1,3-thiazol-5-yl]-2-pyridyl]acetamide dihydrochloride (0.41 g, 0.84 mmol) in tetrahydrofuran (20 mL) were added acetyl chloride (0.13 mL, 1.8 mmol) and triethylamine (0.50 mL, 3.6 mmol) sequentially, and the resulting mixture was stirred at room temperature for 30 minutes. Aqueous sodium hydrogen carbonate was added to the reaction mixture and extracted with ethyl acetate. The extracts were washed with water, dried, and concentrated. The residue was recrystallized from ethyl acetate-hexane to give the title compound (0.24 g, yield 62%).
m.p.: 160-161°C

### Reference Example H 94

The following Reference Example H compound 94 was synthesized according to Reference Example H 93, using N-[4-[4-(3-chlorophenyl)-2-(4-piperidyl)-1,3-thiazol-5-yl]-2-pyridyl]propionamide dihydrochloride instead of N-[4-[4-(3-chlorophenyl)-2-(4-piperidyl)-1,3-thiazol-5-yl]-2-pyridyl]acetamide dihydrochloride.
Reference Example H compound 94: N-[4-[2-(1-acetylpiperidin-4-yl)-4-(3-chlorophenyl)-1,3-thiazol-5-yl]-2-pyridyl]propionamide
m.p.: 174-175°C

### Reference Example H 95

The following Reference Example H compound 95 was synthesized according to Reference Example H 11, using 2-bromo-1-(3-ethylphenyl)-2-(2-fluoro-4-pyridyl)ethanone hydrobromide instead of 2-bromo-2-(2-fluoro-4-pyridyl)-1-(3-methylphenyl)ethanone hydrobromide.
Reference Example H compound 95: 2-ethyl-4-(3-ethylphenyl)-5-(2-fluoro-4-pyridyl)-1,3-thiazole
oil
¹H-NMR(CDCl₃) d: 1.18 (3H, t, J= 7.6 Hz), 1.46 (3H, t, J= 7.6 Hz), 2.62 (2H, q, J= 7.6 Hz), 3.10 (2H, q, J= 7.6 Hz), 6.86 (1H, t, J= 1.4 Hz), 7.07 (1H, dt, J = 1.4, 5.2 Hz), 7.16-7.30 (3H, m), 7.33 (1H, s), 8.10 (1H, d, J= 5.2 Hz).

### Reference Example H 96

### N-cyclopentyl-4-[2-ethyl-4-(3-ethylphenyl)-1,3-thiazol-5-yl]-2-pyridyl]amine

2-ethyl-4-(3-ethylphenyl)-5-(2-fluoro-4-pyridyl)-1,3-thiazole (0.51 g, 1.6 mmol) and cyclopentylamine (1.6 mL, 16 mmol) were stirred at 140°C for 12 hours. Aqueous sodium hydrogen carbonate was added to the reaction mixture and extracted with ethyl acetate. The extracts were washed with aqueous sodium hydrogen carbonate and brine, in order, dried, and concentrated. The residue was purified by silica gel column chromatography (hexane-ethyl acetate = 4 : 1). The obtained crude crystalline was recrystallized from isopropyl ether to give the title compound (0.40 g, yield 66%).
m.p.: 77-79°C

### Reference Example H 97

The following Reference Example H compound 97 was synthesized according to Reference Example H 96, using cyclohexylamine instead of cyclopentylamine.
Reference Example H compound 97: N-cyclohexyl-4-[2-ethyl-4-(3-ethylphenyl)-1,3-thiazol-5-yl]-2-pyridyl]amine
m.p.: 115-116°C

### Reference Example H 98

The following Reference Example H compounds 98-1 and 98-2 were synthesized according to Reference Example H 57, using N-[4-[4-(3-methylphenyl)-2-[4-(methylthio)phenyl]-1,3-thiazol-5-yl]-2-pyridyl]acetamide and N-[4-[4-(3-methylphenyl)-2-[4-(methylthio)phenyl]-1,3-thiazol-5-yl]-2-pyridyl]propionamide, respectively, instead of 4-(3-methylphenyl)-5-(2-methyl-4-pyridyl)-2-[4-(methylthio)phenyl]-1,3-thiazole.
Reference Example H compound 98-1: N-[4-[4-(3-methylphenyl)-2-(4-methylsulfonylphenyl)-1,3-thiazol-5-yl]-2-pyridyl]acetamide
   m.p.: 222-223°C
Reference Example H compound 98-2: N-[4-[4-(3-methylphenyl)-2-(4-methylsulfonylphenyl)-1,3-thiazol-5-yl]-2-pyridyl]propionamide
   m.p.: 238-239°C

### Reference Example H 99

The following Reference Example H compounds 99-1 and 99-2 were synthesized according to Reference Example H 17, using 3-(methylthio)thiopropionamide and 2-amino-1-methyl-2-thioxoethyl benzoate instead of 2-chlorothiobenzamide.
Reference Example H compound 99-1: 4-[4-(3-methylphenyl)-2-[2-(methylthio)ethyl]-1,3-thiazol-5-yl]-2-pyridylamine
   m.p.: 98-99°C
Reference Example H compound 99-2: 1-[5-(2-amino-4-pyridyl)-4-(3-methylphenyl)-1,3-thiazol-2-yl]ethyl benzoate
   m.p.: 89-91°C

### Reference Example H 100

The following Reference Example H compounds 100-1 and 100-2 were synthesized according to Reference Example H 99, using 2-(2-amino-4-pyridyl)-2-bromo-1-(3-chlorophenyl)ethanone hydrobromide instead of 2-(2-amino-4-pyridyl)-2-bromo-1-(3-methylphenyl)ethanone hydrobromide.
Reference Example H compound 100-1: 4-[4-(3-chlorophenyl)-2-[2-(methylthio)ethyl]-1,3-thiazol-5-yl]-2-pyridylamine
   m.p.: 96-97°C
Reference Example H compound 100-2: 1-[5-(2-amino-4-pyridyl)-4-(3-chlorophenyl)-1,3-thiazol-2-yl]ethyl benzoate
   amorphous powder
   ¹H-NMR(CDCl₃) d: 1.89 (3H, d, J= 6.4Hz), 4.50 (2H, br s), 6.38-6.47 (2H, m), 6.56 (1H, dd, J= 1.4, 5.6 Hz), 7.23-7.38 (3H, m), 7.45-7.53 (2H, m), 7.58-7.66 (2H, m), 8.01 (1H, d, J= 5.6Hz), 8.11-8.16 (2H, m).

### Reference Example H 101

The following Reference Example H compounds 101-1 and 101-2 were synthesized according to Reference Example H 100, using (methylthio)thioacetamide and 2,2-difluorothiopropionamide instead of 3-(methylthio)thiopropionamide.
Reference Example H compound 101-1: 4-[4-(3-chlorophenyl)-2-(methylthio)methyl-1,3-thiazol-5-yl]-2-pyridylamine
   m.p.: 111-112°C
Reference Example H compound 101-2: 4-[4-(3-chlorophenyl)-2-(1,1-difluoroethyl)-1,3-thiazol-5-yl]-2-pyridylamine
   m.p.: 131-132°C

### Reference Example H 102

The following Reference Example H compounds 102-1 to 102-6 were synthesized according to Reference Example H 83, using 4-[4-(3-methylphenyl)-2-[2-(methylthio)ethyl]-1,3-thiazol-5-yl]-2-pyridylamine, 4-[4-(3-chlorophenyl)-2-[2-(methylthio)ethyl]-1,3-thiazol-5-yl]-2-pyridylamine, 4-[4-(3-chlorophenyl)-2-(methylthio)methyl-1,3-thiazol-5-yl]-2-pyridylamine, 4-[4-(3-chlorophenyl)-2-(1,1-difluoroethyl)-1,3-thiazol-5-yl]-2-pyridylamine, 1-[5-(2-amino-4-pyridyl)-4-(3-methylphenyl)-1,3-thiazol-2-yl]ethyl benzoate and 1-[5-(2-amino-4-pyridyl)-4-(3-chlorophenyl)-1,3-thiazol-2-yl]ethyl benzoate respectively, instead of 4-[2-(1-tert-butoxycarbonylpiperidin-4-yl)-4-(3-chlorophenyl)-1,3-thiazol-5-yl]-2-pyridylamine.
Reference Example H compound 102-1: N-[4-[4-(3-methylphenyl)-2-[2-(methylthio)ethyl]-1,3-thiazol-5-yl]-2-pyridyl]propionamide
   m.p.: 85-86°C
Reference Example H compound 102-2: N-[4-[4-(3-chlorophenyl)-2-[2-(methylthio)ethyl]-1,3-thiazol-5-yl]-2-pyridyl]propionamide
   m.p.: 91-92°C
Reference Example H compound 102-3: N-[4-[4-(3-chlorophenyl)-2-(methylthio)methyl-1,3-thiazol-5-yl]-2-pyridyl]propionamide
   m.p.: 118-119°C
Reference Example H compound 102-4: N-[4-[4-(3-chlorophenyl)-2-(1,1-difluoroethyl)-1,3-thiazol-5-yl]-2-pyridyl]propionamide
   m.p.: 141-142°C
Reference Example H compound 102-5: 1-[4-(3-methylphenyl)-5-(2-propionylamino-4-pyridyl)-1,3-thiazol-2-yl]ethyl benzoate
   m.p.: 102-103°C
Reference Example H compound 102-6: 1-[4-(3-chlorophenyl)-5-(2-propionylamino-4-pyridyl)-1,3-thiazol-2-yl]ethyl benzoate
   m.p.: 124-127°C

### Reference Example H 103

The following Reference Example H compounds 103-1 and 103-2 were synthesized according to Reference Example H 81, using 1-[5-(2-amino-4-pyridyl)-4-(3-chlorophenyl)-1,3-thiazol-2-yl]ethyl benzoate and ethyl [5-(2-amino-4-pyridyl)-4-(3-chlorophenyl)-1,3-thiazol-2-yl]acetate instead of [4-[2-(1-tert-butoxycarbonylpiperidin-4-yl)-4-(3-chlorophenyl)-1,3-thiazol-5-yl]-2-pyridylamine.
Reference Example H compound 103-1: 1-[5-(2-acetylamino-4-pyridyl)-4-(3-chlorophenyl)-1,3-thiazol-2-yl]ethyl benzoate
   m.p.: 152-154°C
Reference Example H compound 103-2: ethyl [5-(2-acetylamino-4-pyridyl)-4-(3-chlorophenyl)-1,3-thiazol-2-yl)acetate
   m.p.: 99-100°C

### Reference Example H 104

### N-[4-[2-(1-hydroxyethyl)-4-(3-methylphenyl)-1,3-thiazol-5-yl]-2-pyridyl]propionamide

A 1N aqueous sodium hydroxide solution was added dropwise to a solution of 1-[4-(3-methylphenyl)-5-(2-propionylamino-4-pyridyl)-1,3-thiazol-2-yl]ethyl benzoate (1.63 g, 3.46 mmol) in methanol (5 mL) and tetrahydrofuran (20 mL) at 0°C and the reaction mixture was allowed to warm up to room temperature. The resulting mixture was stirred at room temperature for 30 minutes and the solvent was removed under reduced pressure. The residue was treated with aqueous sodium hydrogen carbonate solution and extracted with ethyl acetate. The extracts were washed with aqueous sodium hydrogen carbonate solution and brine. The resulting solution was dried, and concentrated under reduced pressure. The obtained crude crystal was recrystallized from ethyl acetate-hexane to give the title compound (1.12 g, yield 89%).
m.p.: 115-116°C

### Reference Example H 105

The following Reference Example H compound 105 was synthesized according to Reference Example H 104, using 1-[4-(3-chlorophenyl)-5-(2-propionylamino-4-pyridyl)-1,3-thiazol-2-yl]ethyl benzoate instead of 1-[4-(3-methylphenyl)-5-(2-propionylamino-4-pyridyl)-1,3-thiazol-2-yl]ethyl benzoate. Reference Example H compound 105: N-[4-[4-(3-chlorophenyl)-2-(1-hydroxyethyl)-1,3-thiazol-5-yl]-2-pyridyl]propionamide
m.p.: 131-132°C

### Reference Example H 106

### N-[4-[2-acetyl-4-(3-methylphenyl)-1,3-thiazol-5-yl]-2-pyridyl]propionamide

A solution of dimethylsulfoxide (0.30 mL, 4.2 mmol) in dichloromethane (1.0 mL) was added to a solution of oxalyl chloride (0.11 mL, 1.26 mmol) at -78°C and the resulting mixture was stirred for 15 minutes. A solution of N-[4-[2-(1-hydroxyethyl)-4-(3-methylphenyl)-1,3-thiazol-5-yl]-2-pyridyl]propionamide (0.38 g, 1.0 mmol) in dichloromethane (1.5 mL) was added to the mixture and the resulting mixture was stirred for 45 minutes. The reaction mixture was allowed to warm up to -50°C and triethylamine (0.72 mL, 5.17 mmol) was added dropwise to the mixture. The resulting mixture was stirred for 30 minutes and poured into aqueous sodium hydrogen carbonate. The mixture was extracted with ethyl acetate and the extracts were washed with aqueous sodium hydrogen carbonate solution and brine. The resulting solution was dried, and concentrated under reduced pressure. The residue was purified by silica gel column chromatography (hexane-ethyl acetate=2:1) to obtain a crude crystal. The obtained crude crystal was recrystallized from isopropyl ether to give the title compound (0.22 g, yield 58%).
m.p.: 121-123°C

### Reference Example H 107

The following Reference Example H compound 107 was synthesized according to Reference Example H 106, using N-[4-[4-(3-chlorophenyl)-2-(1-hydroxyethyl)-1,3-thiazol-5-yl]-2-pyridyl]propionamide instead of N-[4-[2-(1-hydroxyethyl)-4-(3-methylphenyl)-1,3-thiazol-5-yl]-2-pyridyl]propionamide. Reference Example H compound 107: N-[4-[2-acetyl-4-(3-chlorophenyl)-1,3-thiazol-5-yl]-2-pyridyl]propionamide
m.p.: 115-117°C

### Reference Example H 108

### N-ethyl-N'-[4-[2-ethyl-4-(3-methylphenyl)-1,3-thiazol-5-yl]-2-pyridyl]urea

To a solution of 4-[2-ethyl-4-(3-methylphenyl)-1,3-thiazol-5-yl]-2-pyridylamine (0.50 g, 1.7 mmol) in N,N-dimethylacetamide (10 mL) was added ethyl isocyanate (0.20 mL, 2.5 mmol) and the reaction mixture was stirred at 80°C for 20 hours. The reaction mixture was poured into aqueous sodium hydrogen carbonate and extracted with ethyl acetate. The extracts were washed with aqueous sodium hydrogen carbonate solution and brine. The resulting solution was dried, and concentrated under reduced pressure. The obtained crude crystal was recrystallized from ethyl acetate-hexane to give the title compound (0.29 g, yield 47%).
m.p.: 160-162°C

### Reference Example H 109

The following Reference Example H compound 109 was synthesized according to Reference Example H 108, using 4-[4-(3-chlorophenyl)-2-ethyl-1,3-thiazol-5-yl]-2-pyridylamine instead of 4-[2-ethyl-4-(3-methylphenyl)-1,3-thiazol-5-yl]-2-pyridylamine.
Reference Example H compound 109: N-[4-[4-(3-chlorophenyl)-2-ethyl-1,3-thiazol-5-yl]-2-pyridyl]-N'-ethyl-urea
m.p.: 177-180°C

### Reference Example H 110

The following Reference Example H compound 110 was synthesized according to Reference Example H 81, using 1-[5-(2-amino-4-pyridyl)-4-(3-methylphenyl)-1,3-thiazol-2-yl]ethyl benzoate instead of [4-[2-(1-tert-butoxycarbonylpiperidin-4-yl)-4-(3-chlorophenyl)-1,3-thiazol-5-yl]-2-pyridylamine. Reference Example H compound 110: 1-[5-(2-acetylamino-4-pyridyl)-4-(3-methylphenyl)-1,3-thiazol-2-yl]ethyl benzoate m.p.: 110-113°C

### Reference Example H 111

The following Reference Example H compounds 111-1 and 111-2 were synthesized according to Reference Example H 104, using 1-[5-(2-acetylamino-4-pyridyl)-4-(3-methylphenyl)-1,3-thiazol-2-yl]ethyl benzoate and 1-[5-(2-acetylamino-4-pyridyl)-4-(3-chlorophenyl)-1,3-thiazol-2-yl]ethyl benzoate instead of 1-[4-(3-methylphenyl)-5-(2-propionylamino-4-pyridyl)-1,3-thiazol-2-yl]ethyl benzoate.
Reference Example H compound 111-1: N-[4-[2-(1-hydroxyethyl)-4-(3-methylphenyl)- 1,3-thiazol-5-yl]-2-pyridyl]acetamide
   m.p.: 99-102°C
Reference Example H compound 111-2: N-[4-[4-(3-chlorophenyl)-2-(1-hydroxyethyl)-1,3-thiazol-5-yl]-2-pyridyl]acetamide
   m.p.: 142-145°C

### Reference Example H 112

The following Reference Example H compound 112 was synthesized according to Reference Example H 106, using N-[4-[4-(3-chlorophenyl)-2-(1-hydroxyethyl)-1,3-thiazol-5-yl]-2-pyridyl]acetamide instead of N-[4-[2-(1-hydroxyethyl)-4-(3-methylphenyl)-1,3-thiazol-5-yl]-2-pyridyl]propionamide. Reference Example H compound 112: N-[4-[2-acetyl-4-(3-chlorophenyl)-1,3-thiazol-5-yl]-2-pyridyl]acetamide
m.p.: 180-183 °C

### Reference Example H 113

The following Reference Example H compounds 113-1 and 113-2 were synthesized according to Reference Example H 101, using 2-(2-amino-4-pyridyl)-2-bromo-1-(3-methylphenyl)ethanone hydrobromide instead of 2-(2-amino-4-pyridyl)-2-bromo-1-(3-chlorophenyl)ethanone hydrobromide.
Reference Example H compound 113-1: 4-[4-(3-methylphenyl)-2-(methylthio)methyl-1,3-thiazol-5-yl]-2-pyridylamine
   m.p.: 113-114°C
Reference Example H compound 113-2: 4-[2-(1,1-difluoroethyl)-4-(3-methylphenyl)-1,3-thiazol-5-yl]-2-pyridylamine
   m.p.: 140-141°C

The compounds produced in Reference Examples H 1 to 113 are shown in Tables 55 to 75.

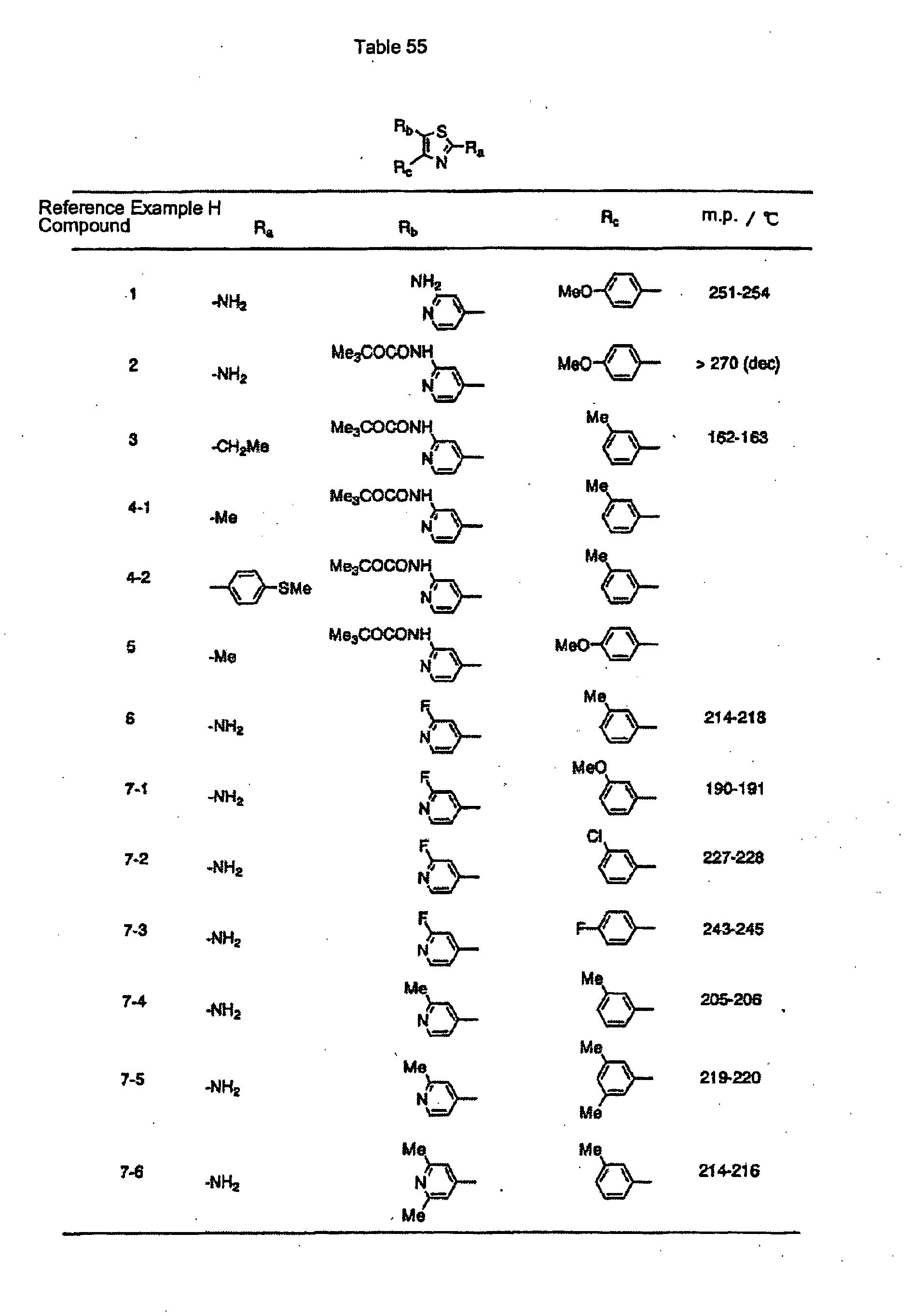

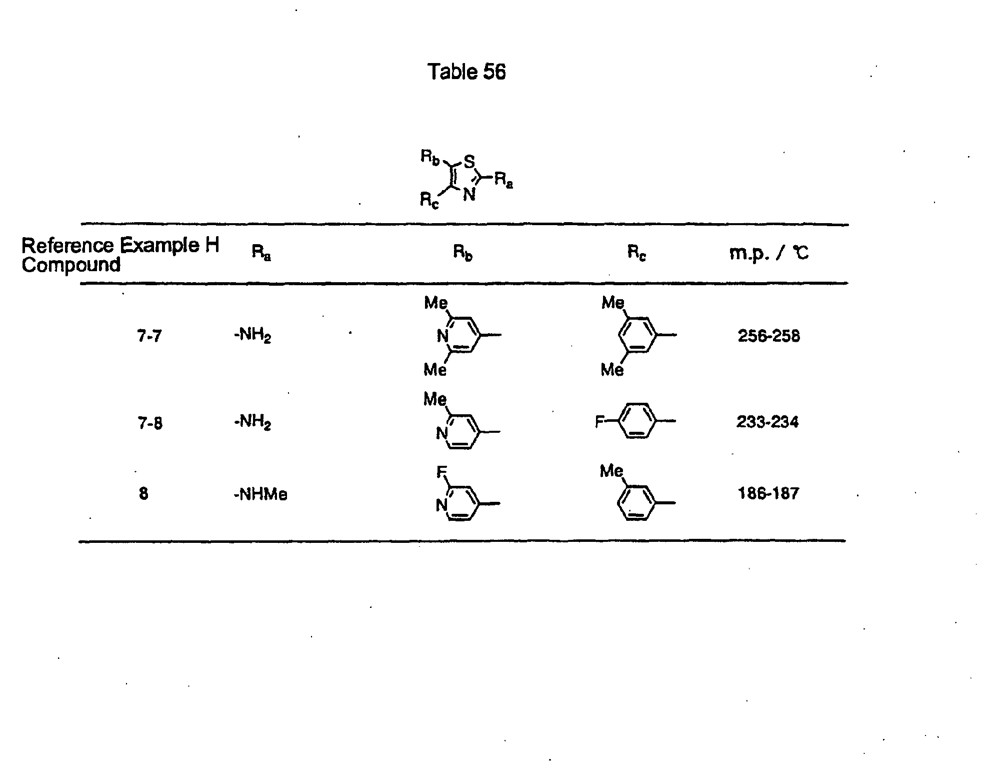

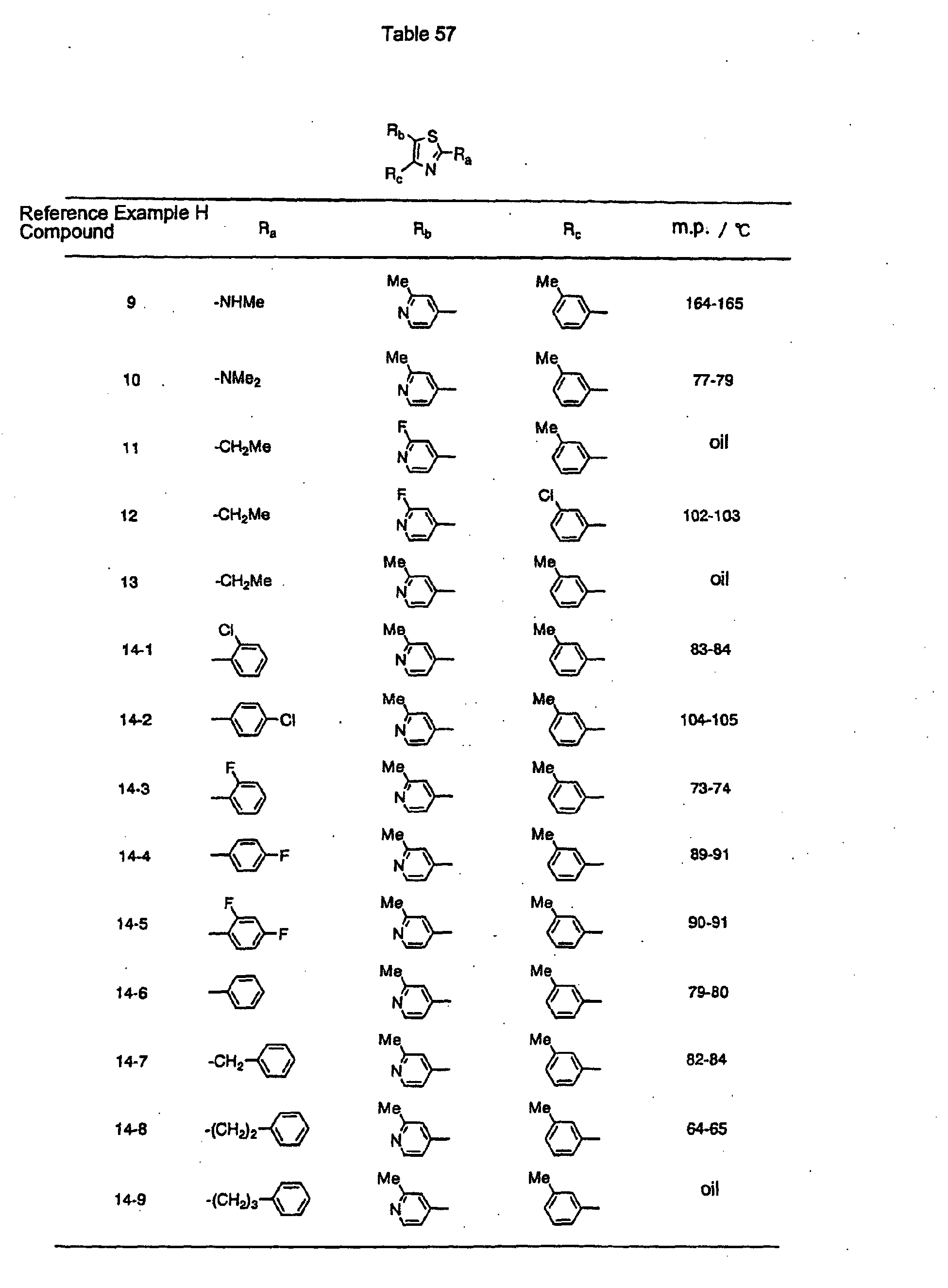

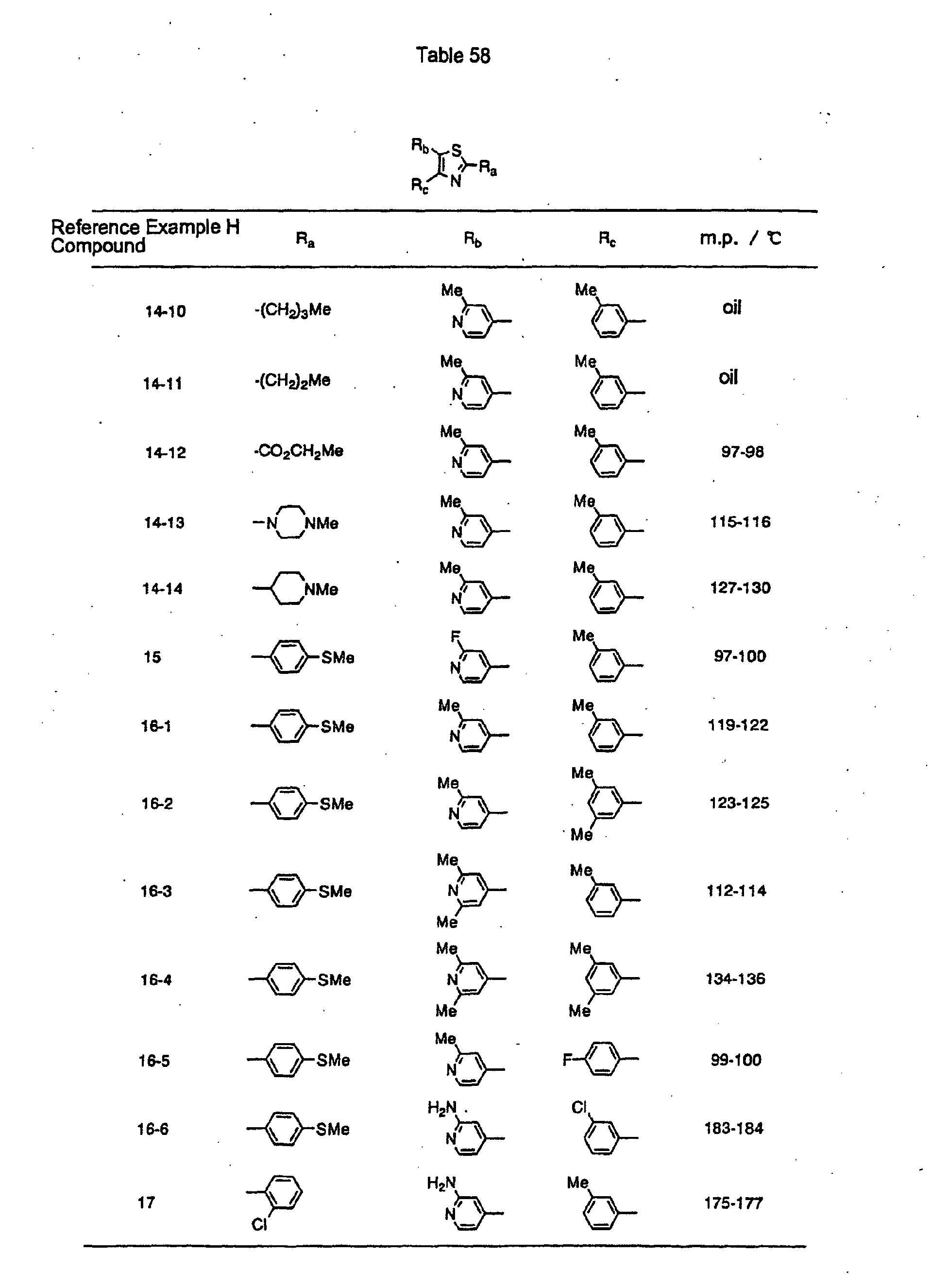

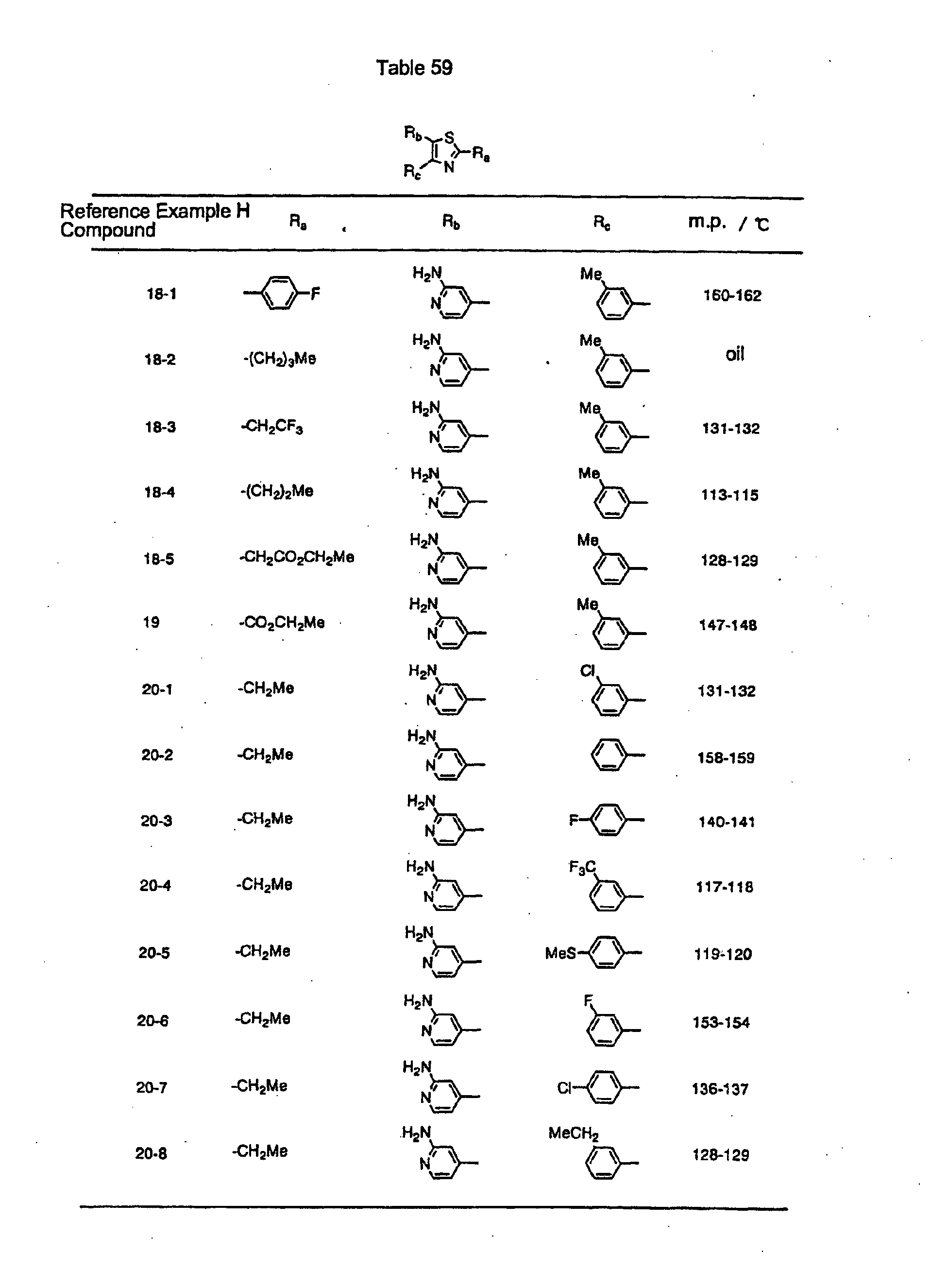

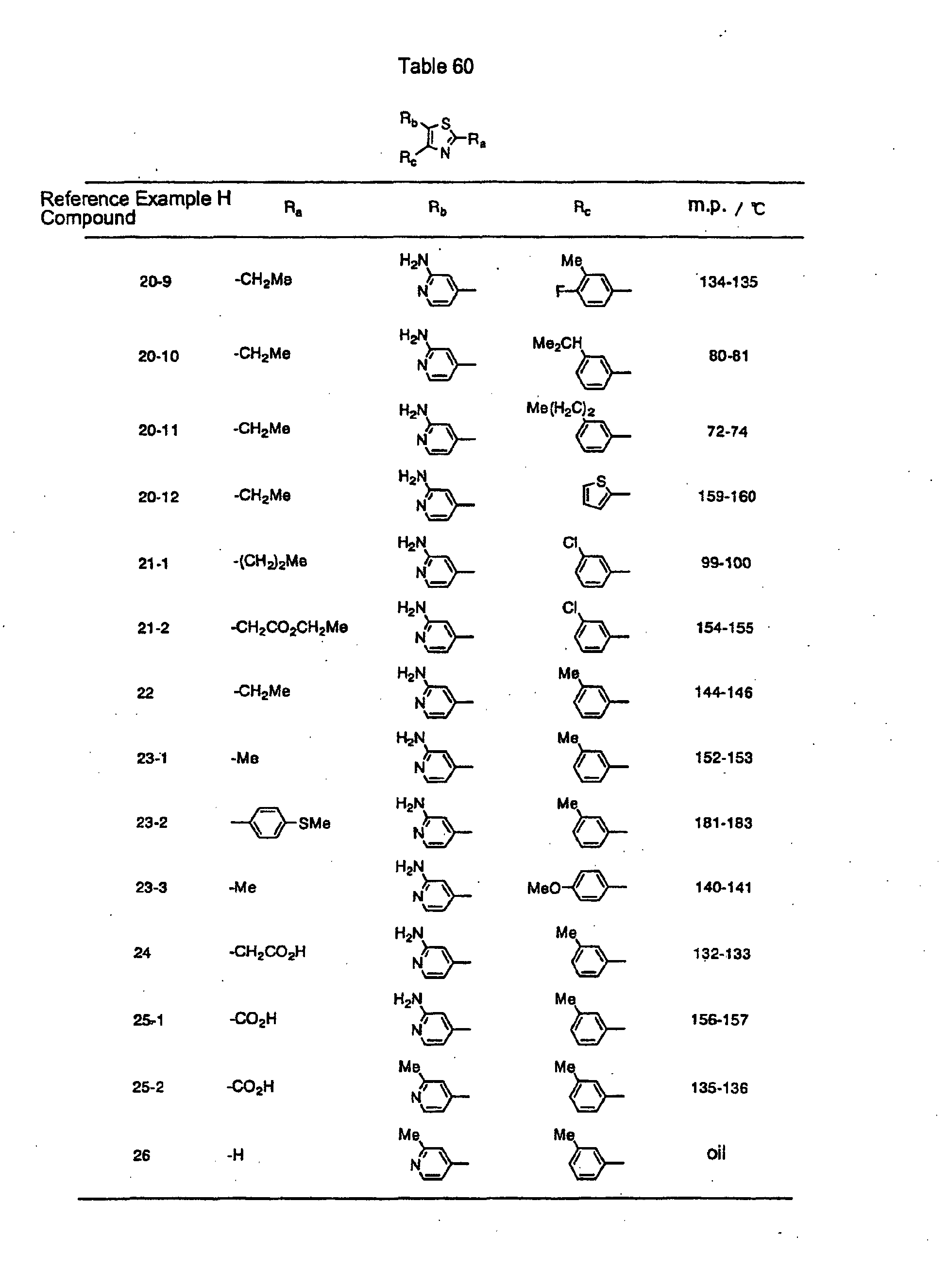

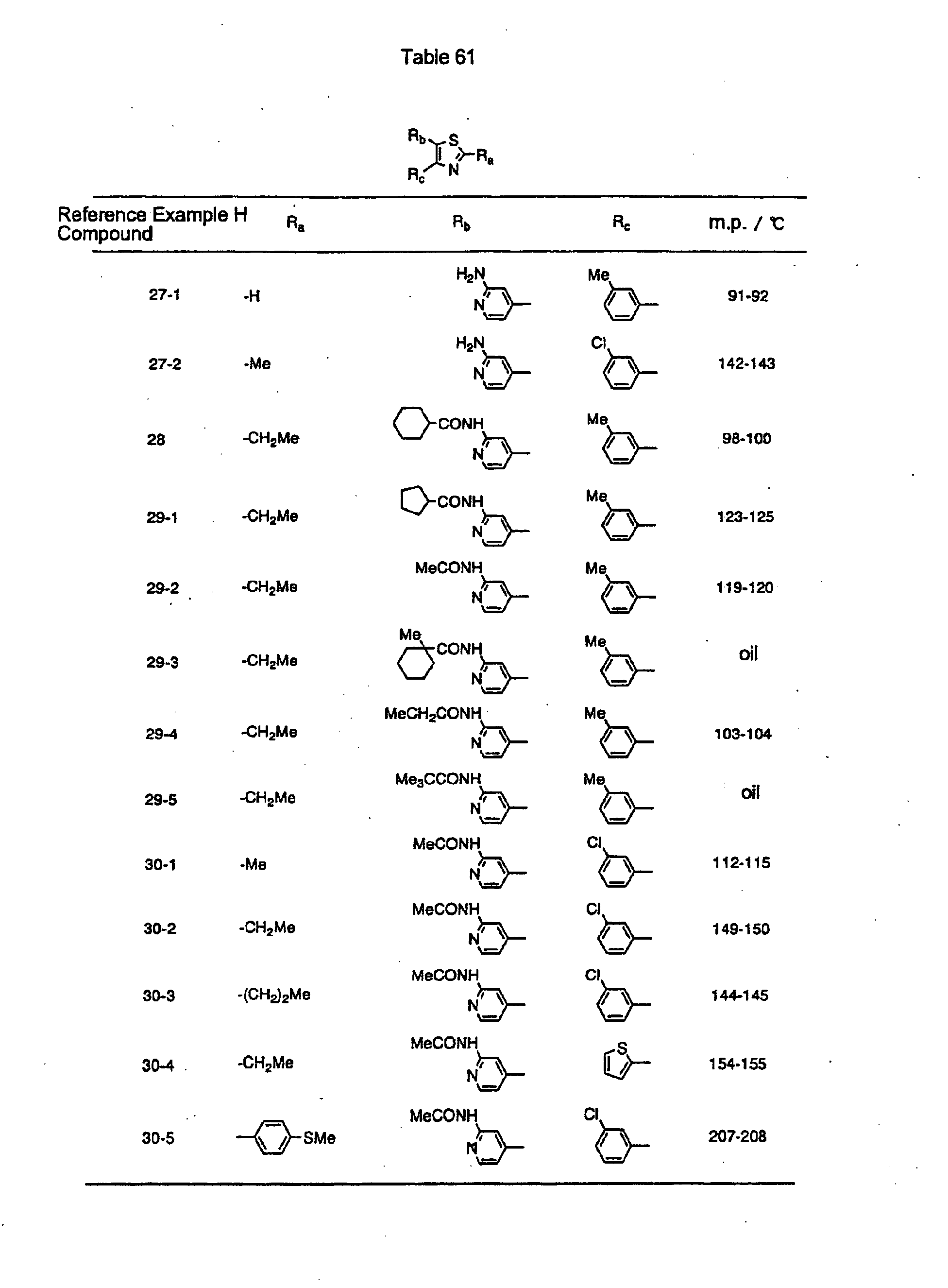

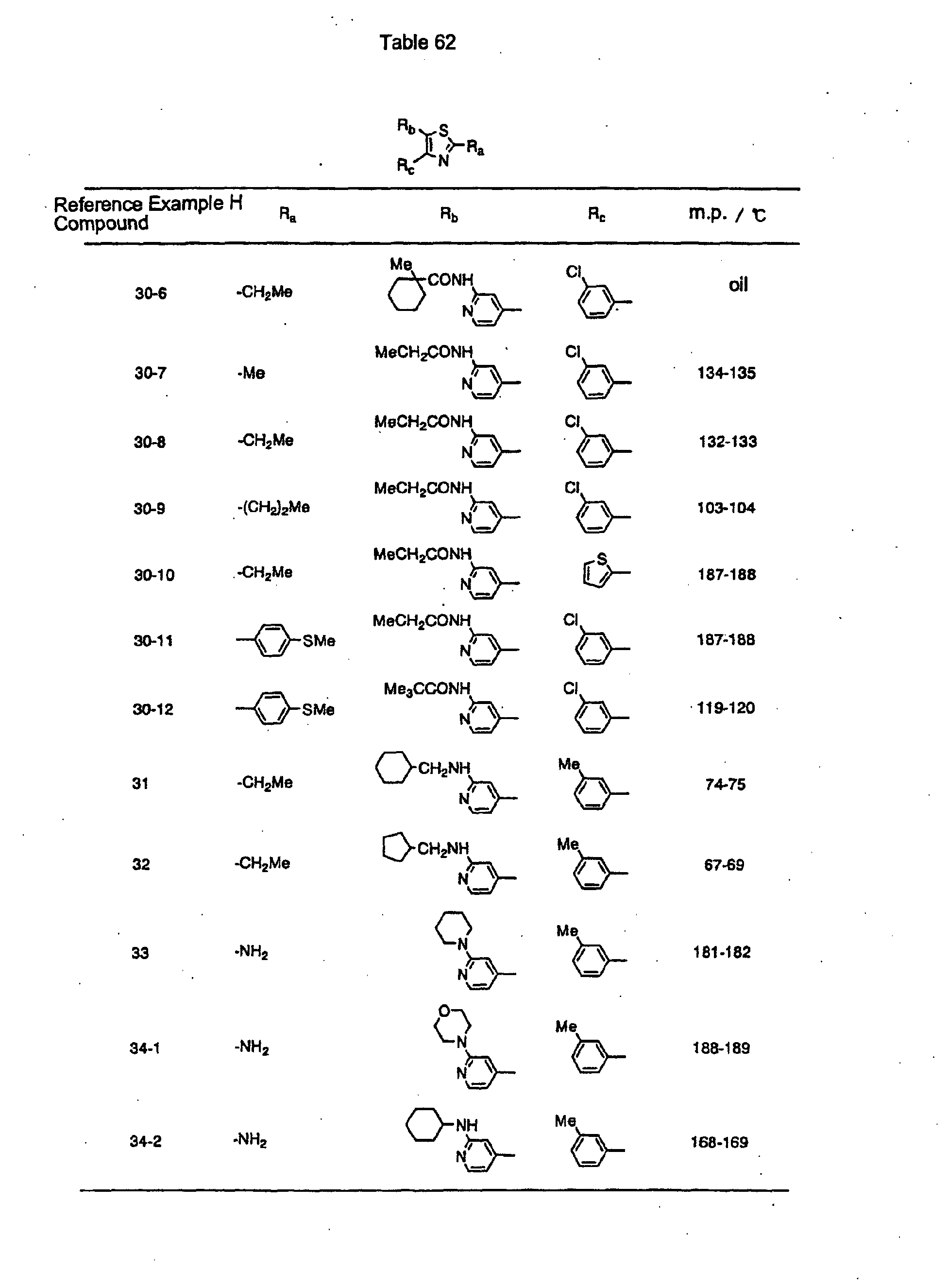

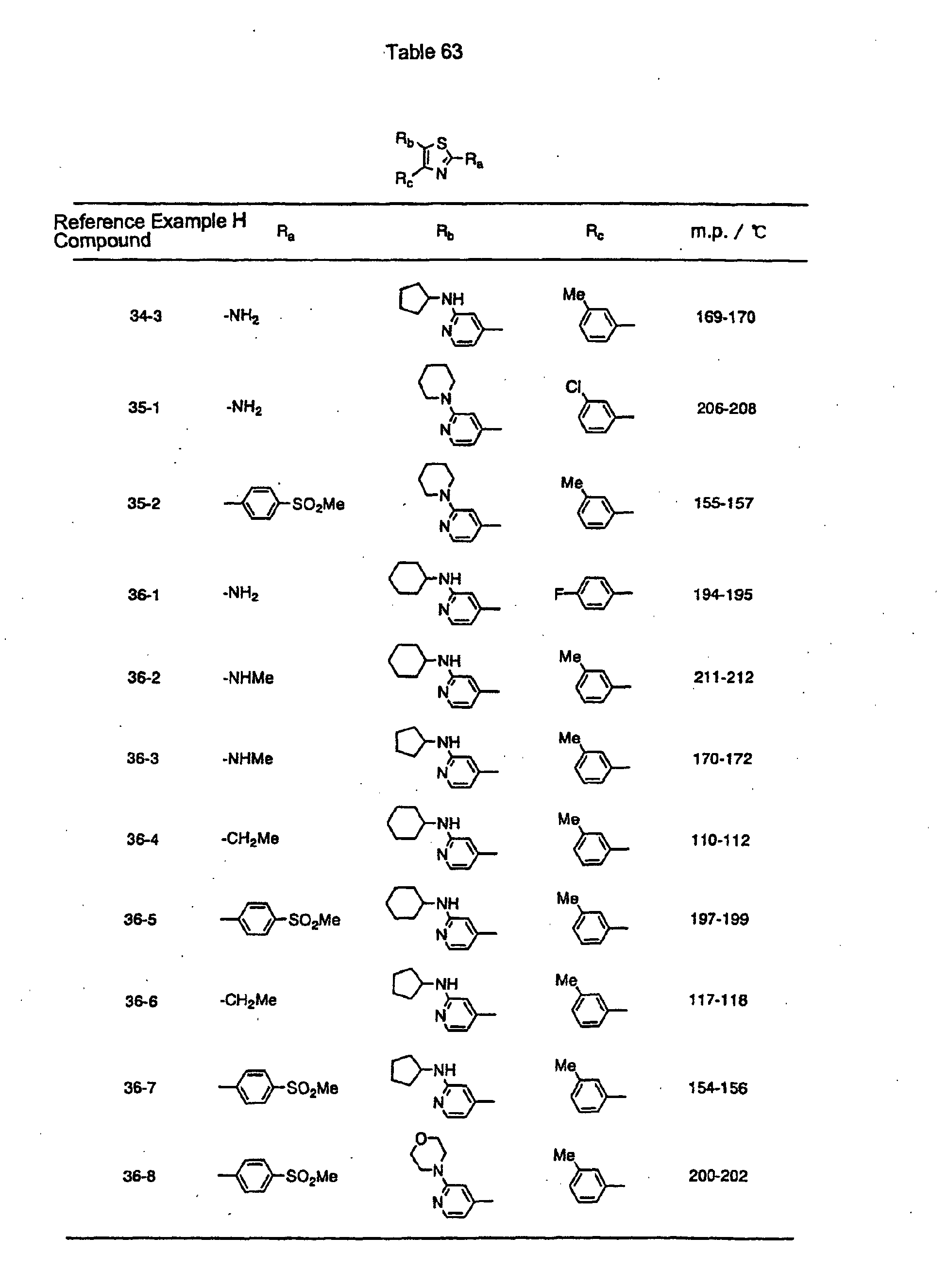

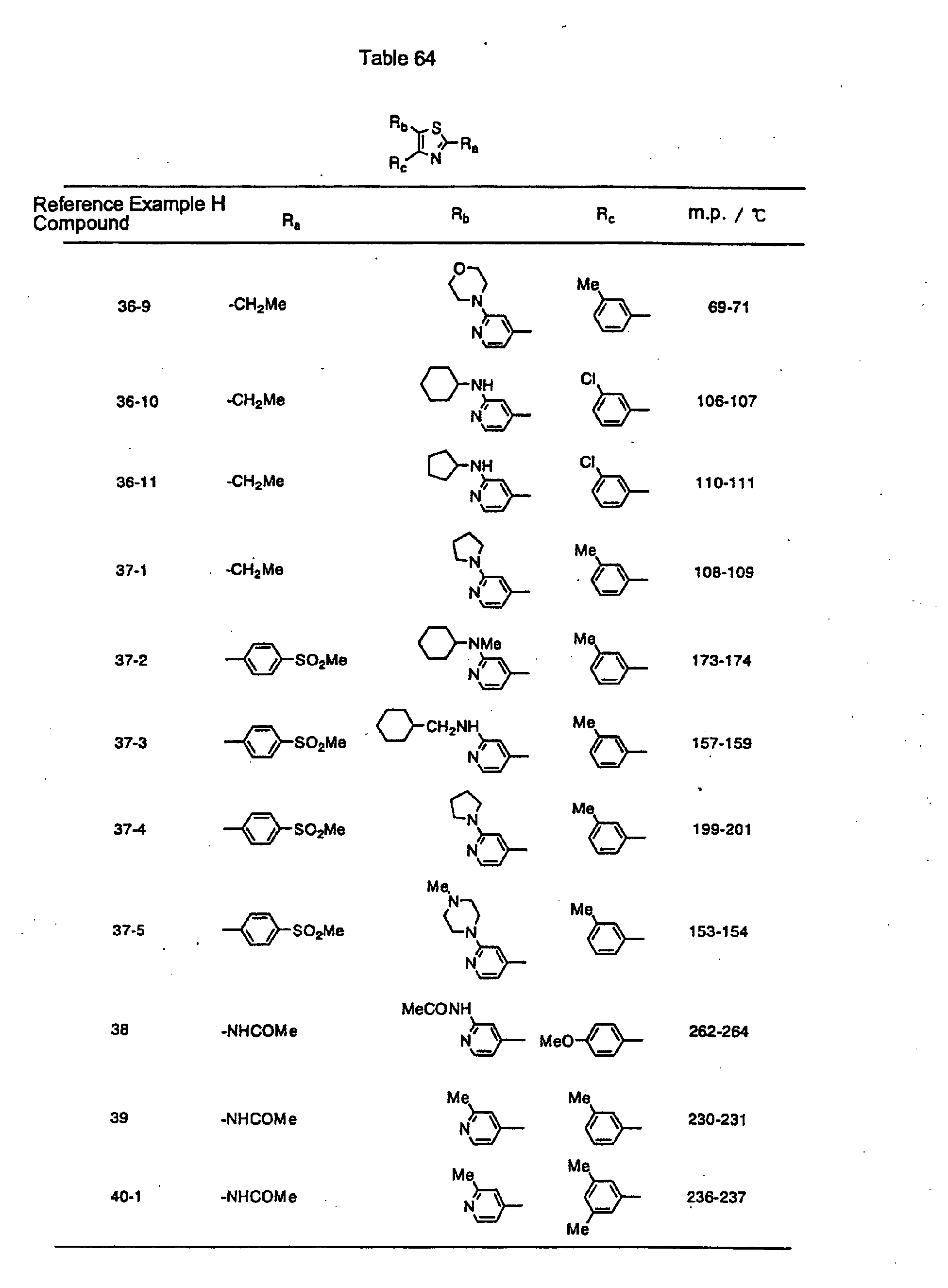

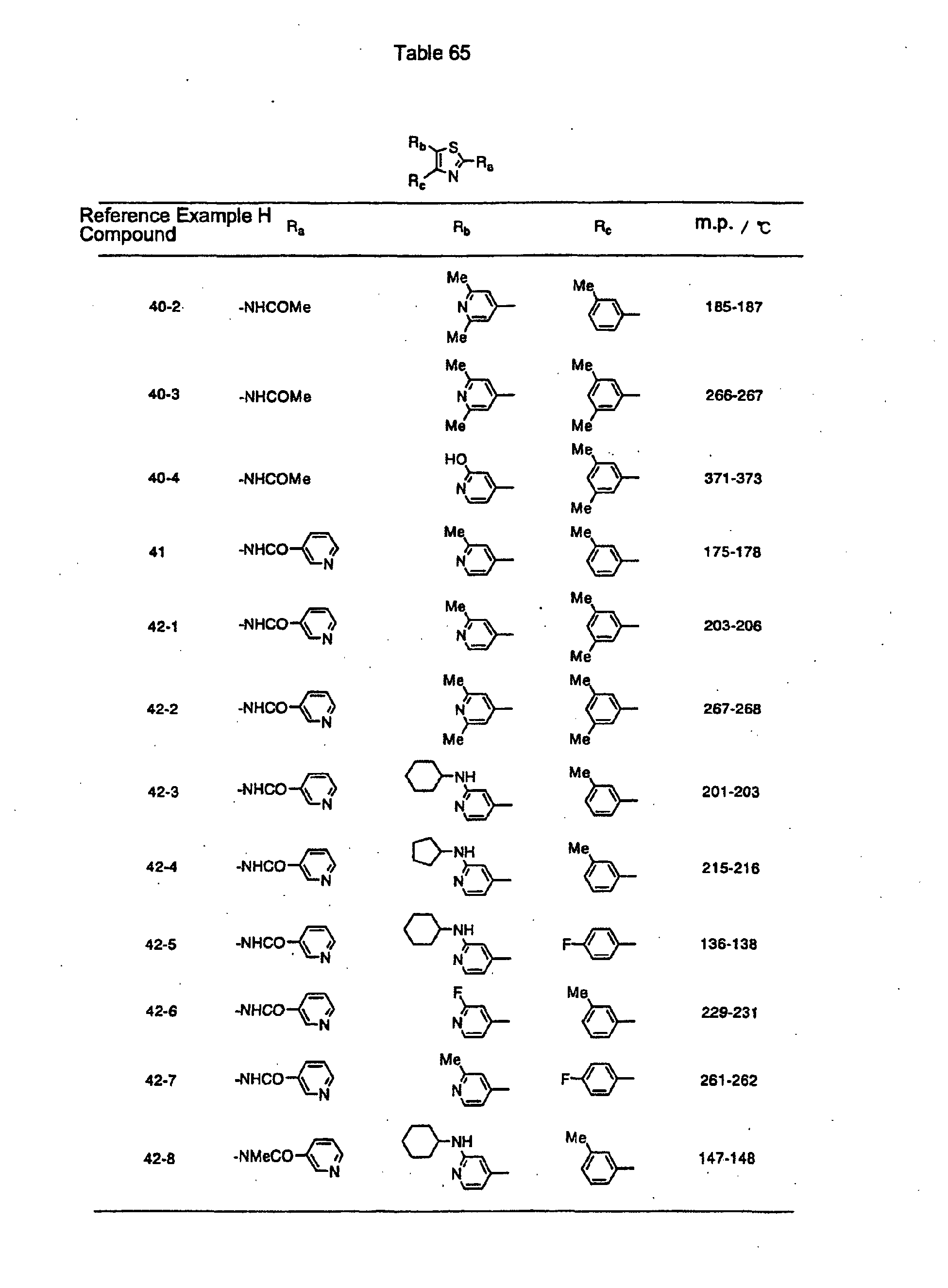

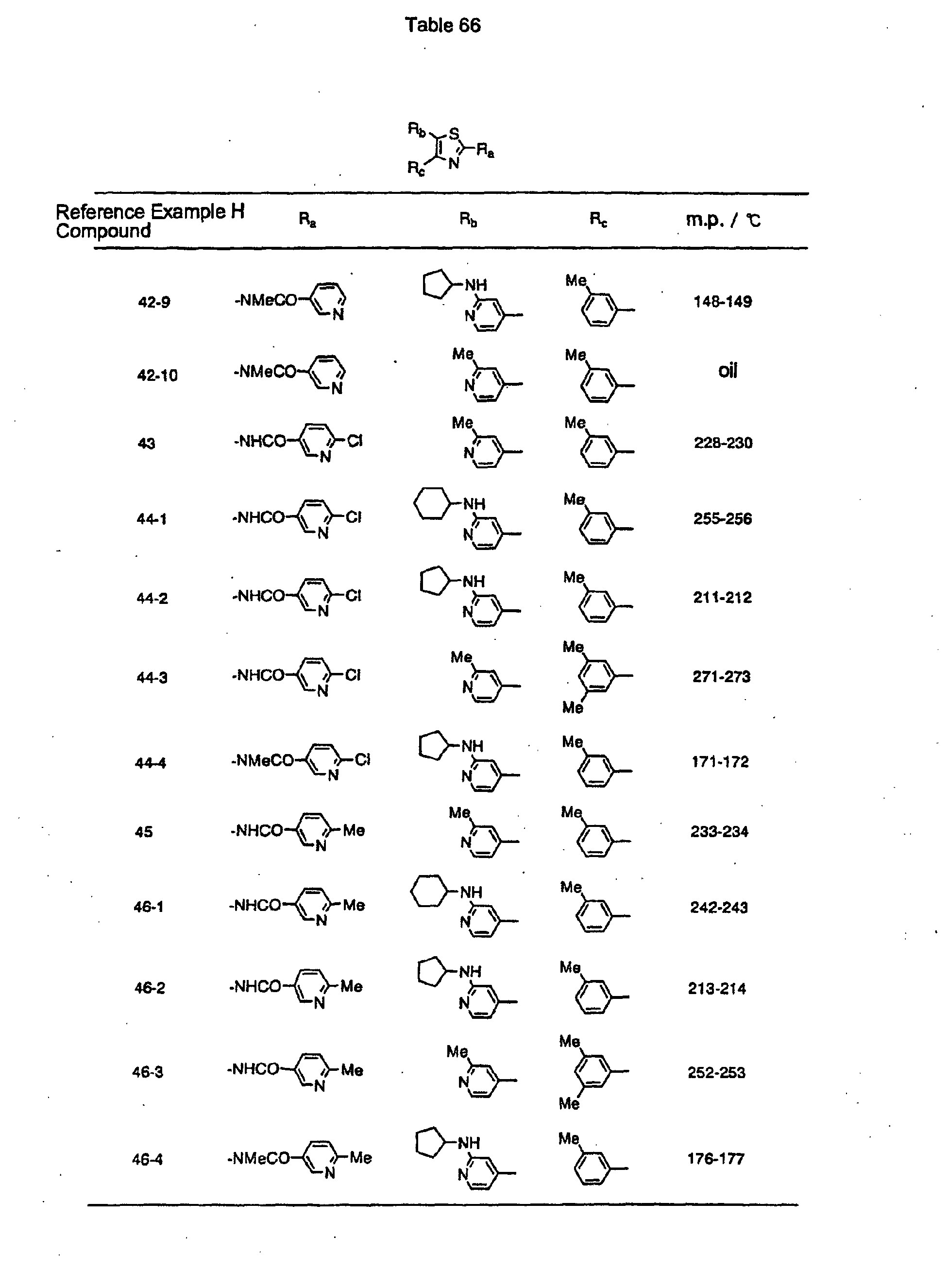

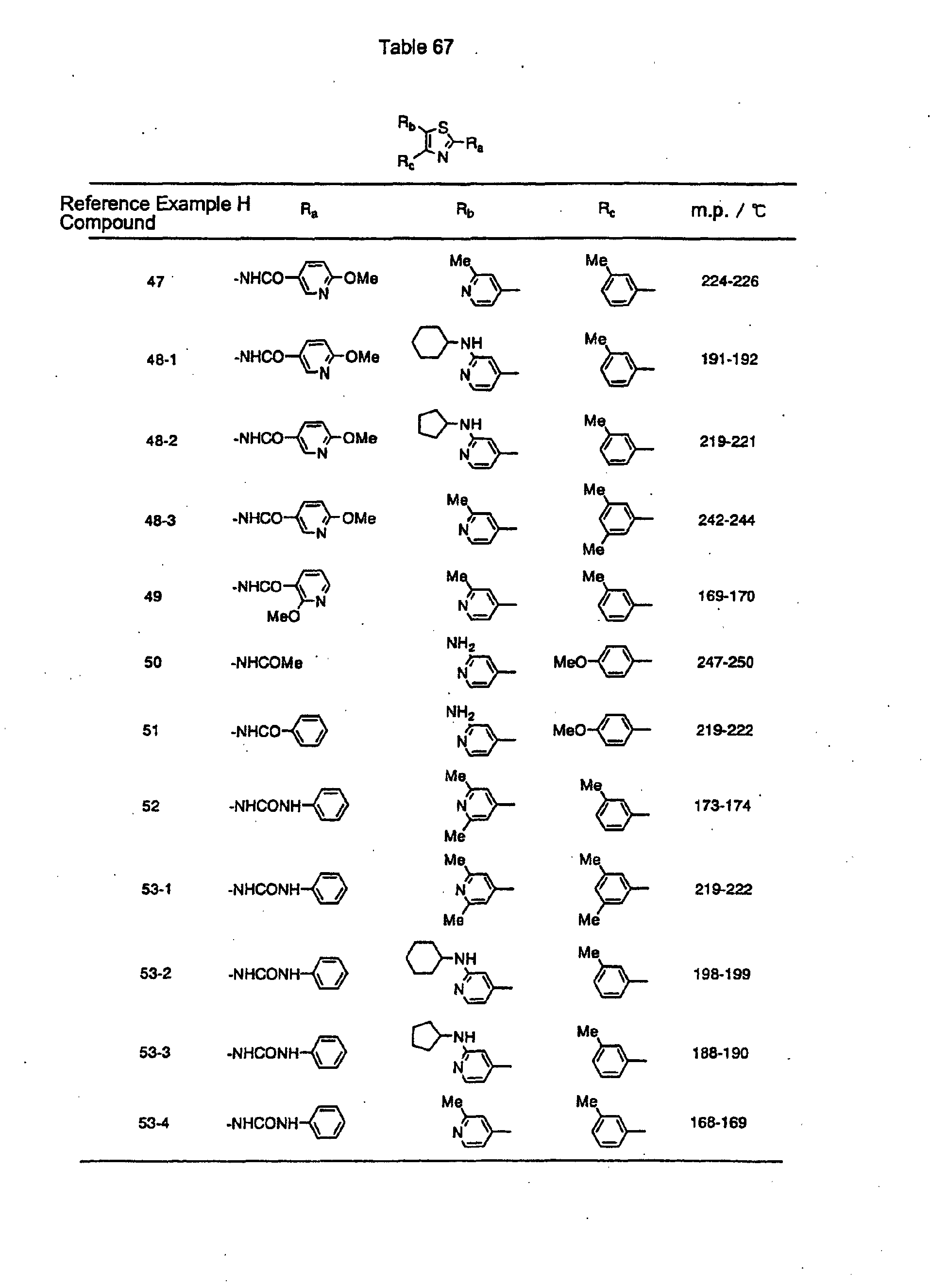

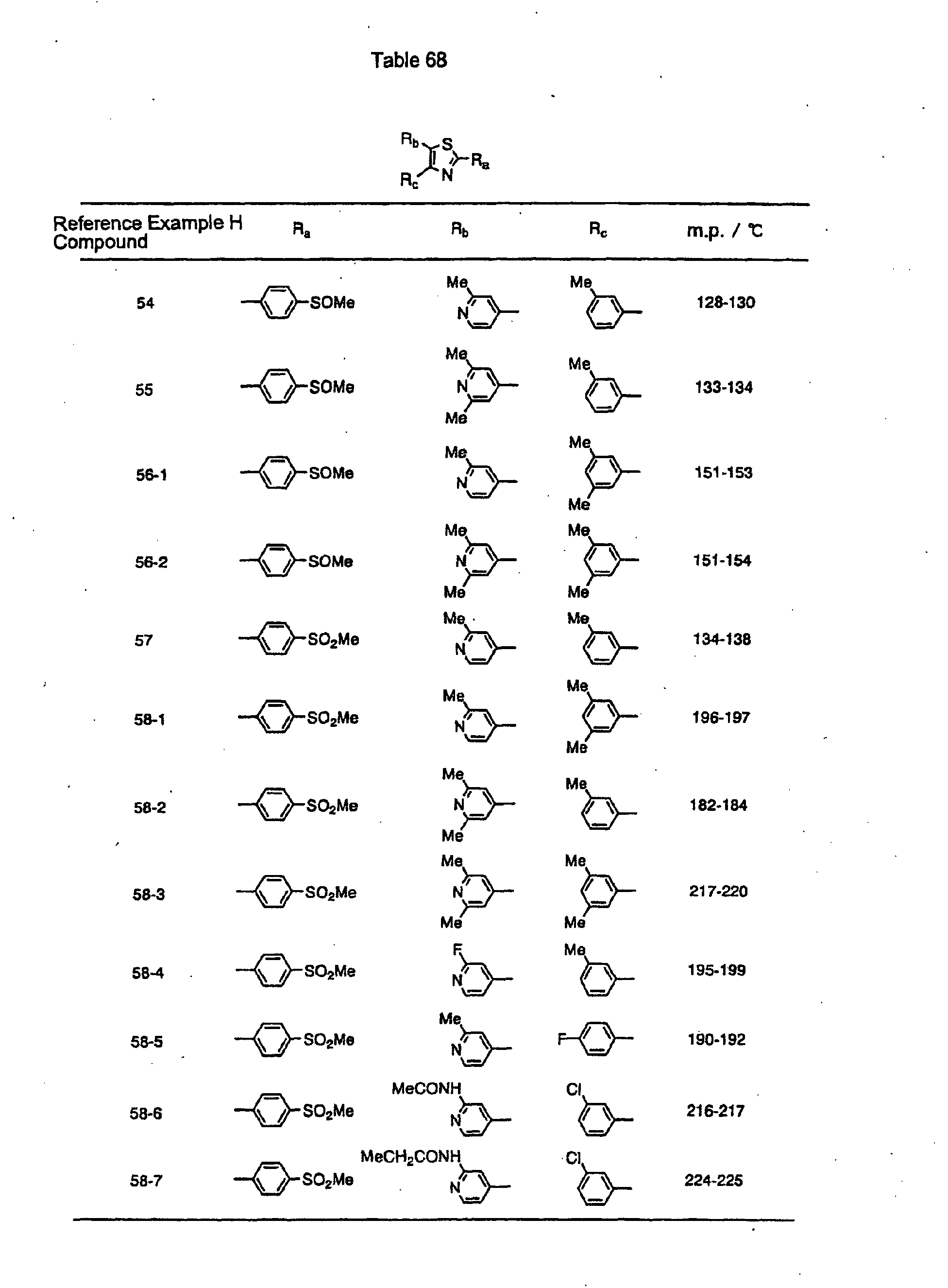

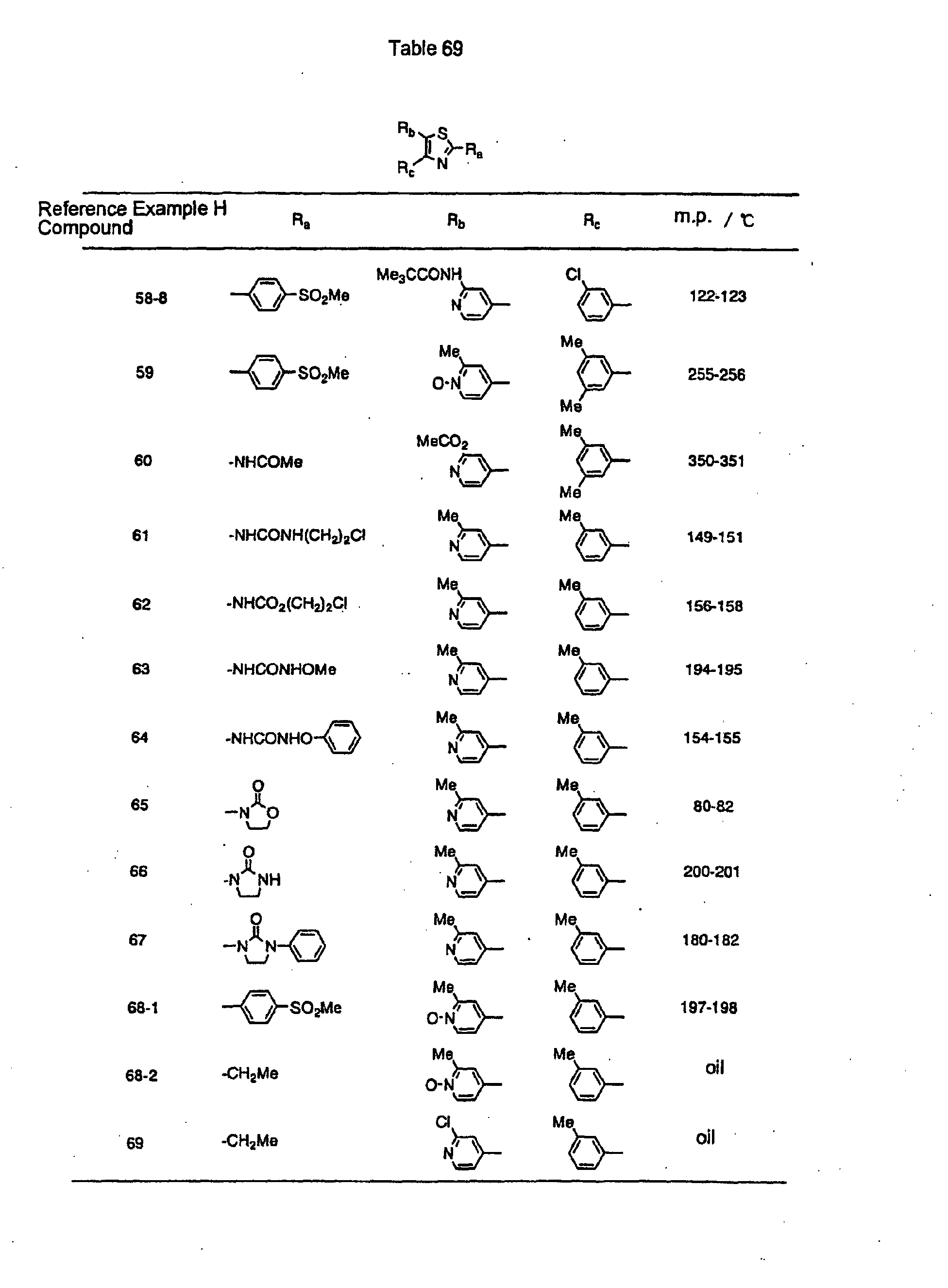

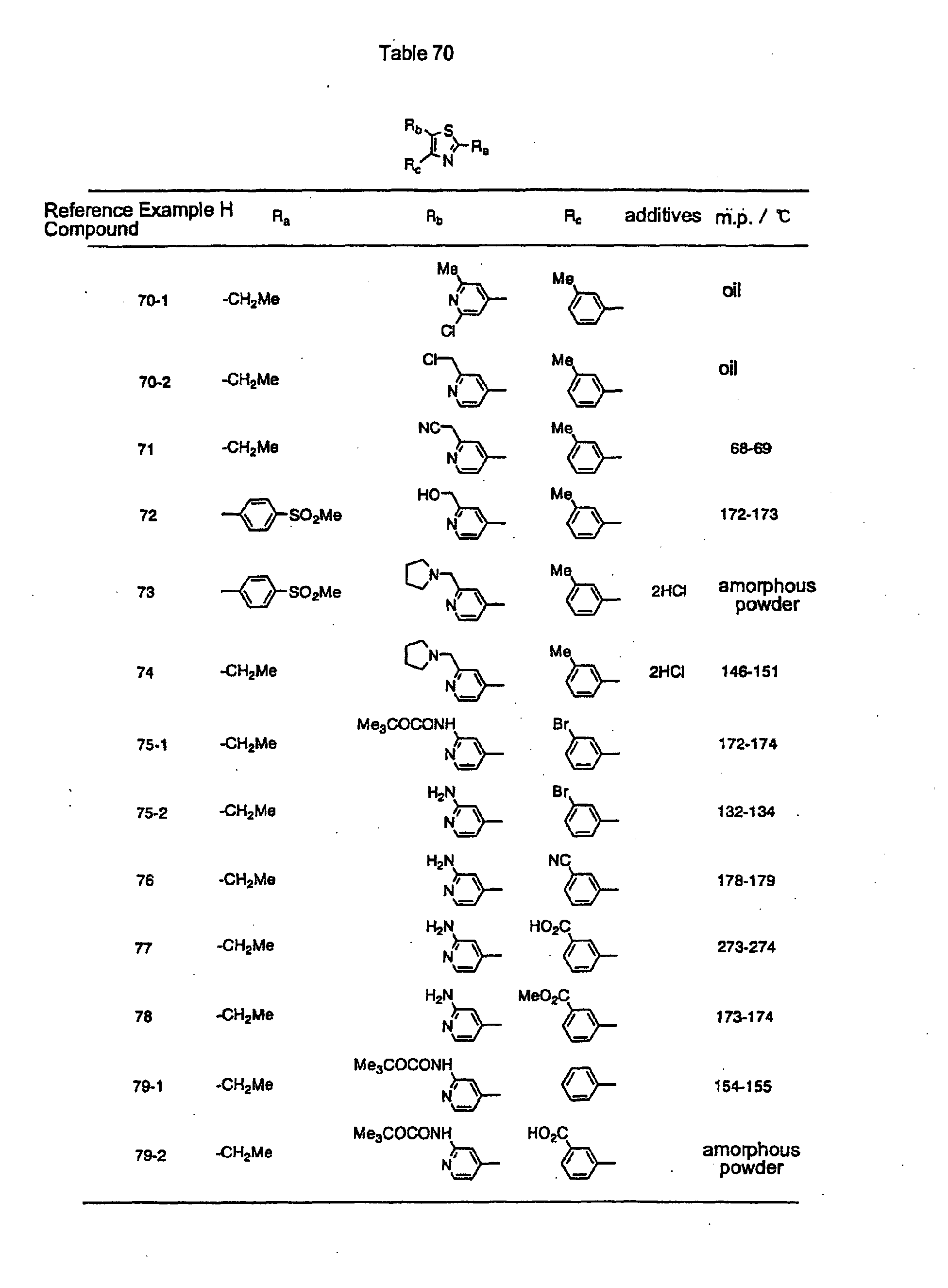

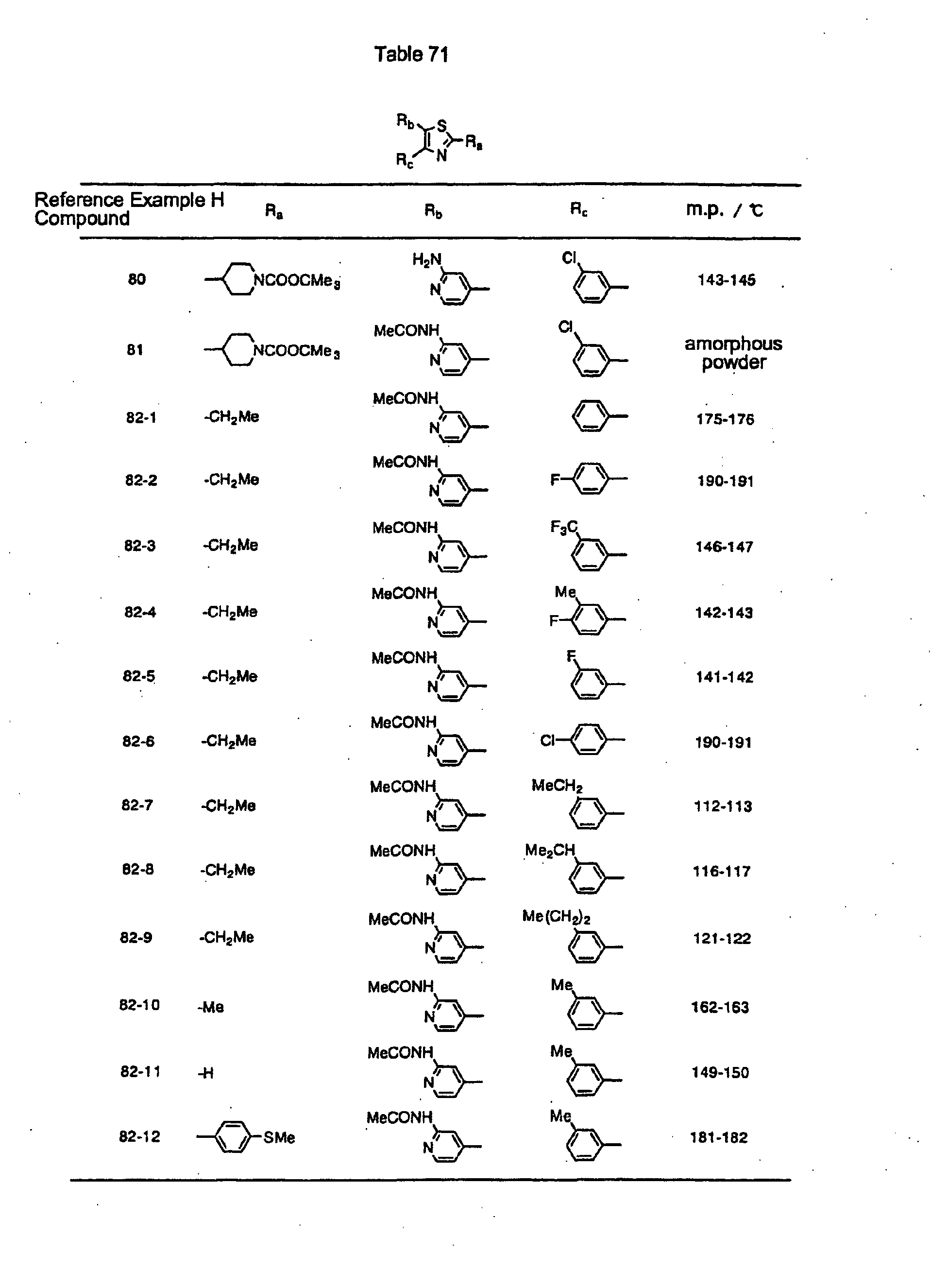

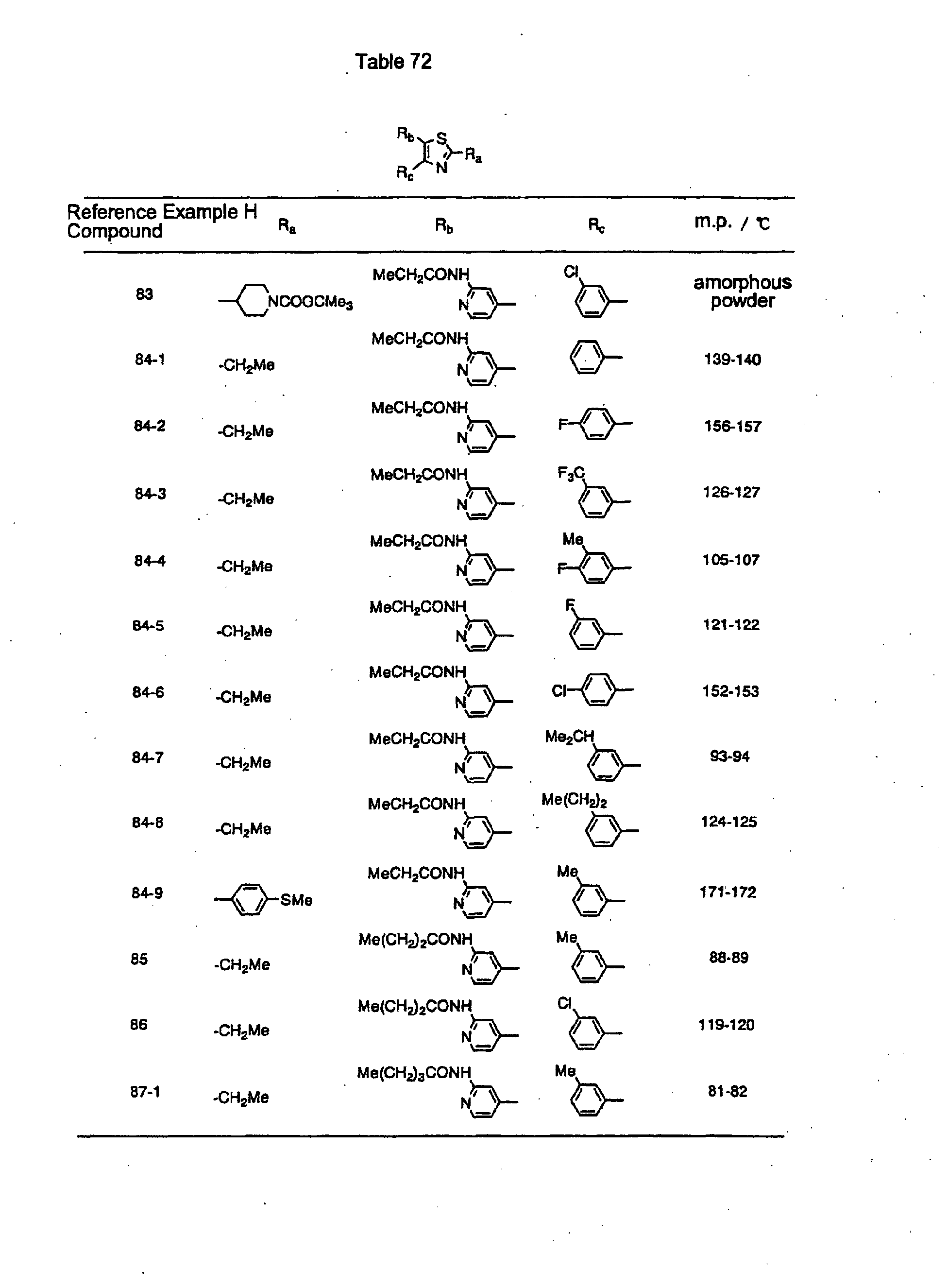

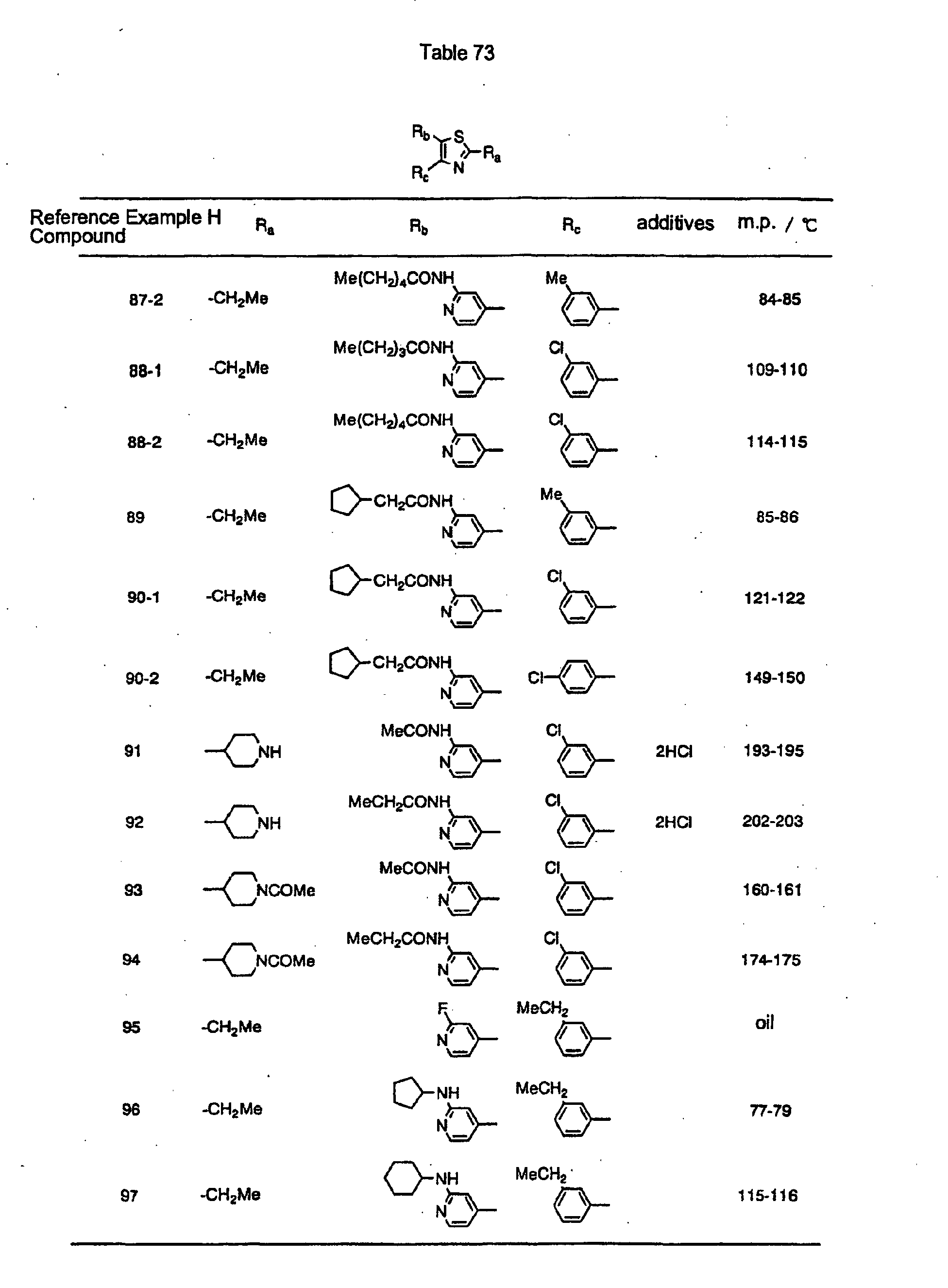

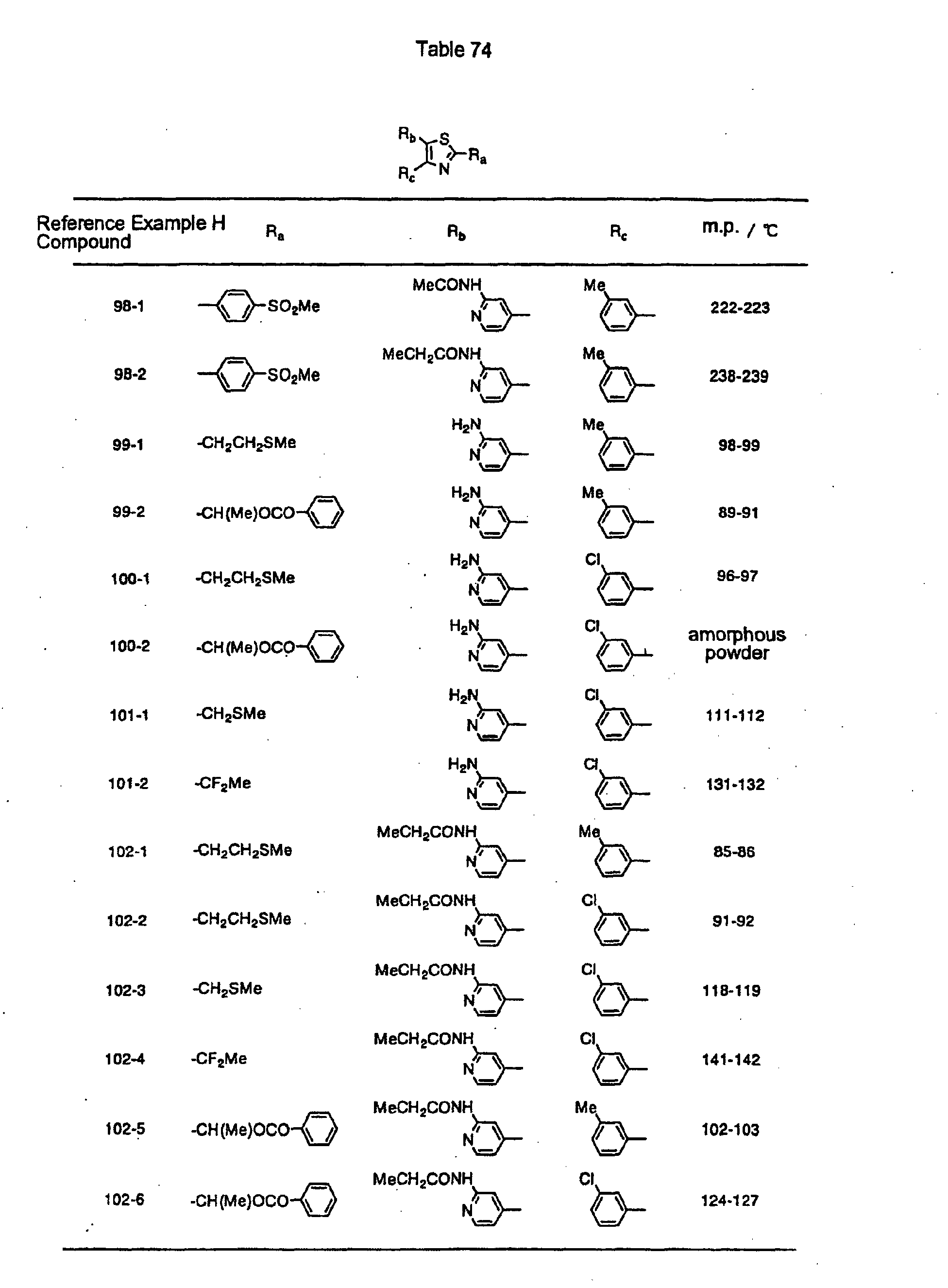

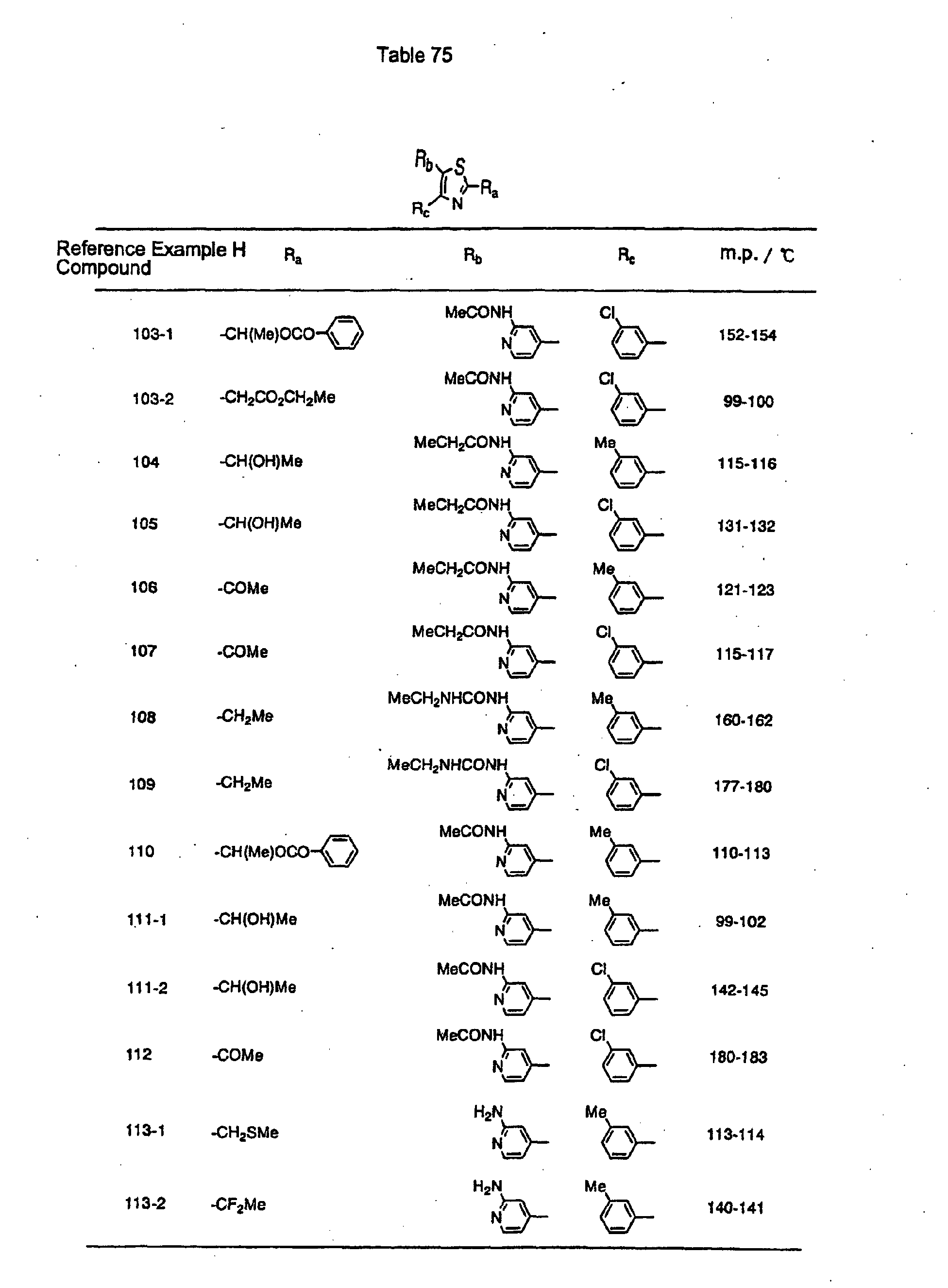

### Reference Example I 1

| | |
|---|---|
| (1) Reference Example H compound 29-2 | 10.0 mg |
| (2) Lactose | 60.0 mg |
| (3) Corn starch | 35.0 mg |
| (4) Gelatin | 3.0 mg |
| (5) Magnesium stearate | 2.0 mg |

10.0 mg of Reference Example H compound 29-2, 60.0 mg of lactose and 35.0 mg of Corn starch are granulated through a 1mm mesh sieve using 0.03 ml of a 10% gelatin aqueous solution (3.0 g in terms of gelatin), then, dried at 40°C and sieved again. Thus obtained granules are mixed with 2.0 mg of magnesium stearate and compressed. The resulted core tablets are coated with sugar coating made from a water suspension of sucrose, titanium dioxide, talc and Arabic gum. The coated tables are endowed with gloss by bees wax to give coated tablets.

### Reference Example I 2

| | |
|---|---|
| (1) Reference Example H compound 29-2 | 10.0 mg |
| (2) Lactose | 70.0 mg |
| (3) Corn starch | 50.0 mg |
| (4) Soluble starch | 7.0 mg |
| (5) Magnesium stearate | 3.0 mg |

10.0 mg of Reference Example H compound 29-2 and 3.0 mg of magnesium stearate are granulated with 0.07 ml of an aqueous solution of soluble starch (7.0 mg in terms of soluble starch), then, dried, and mixed with 70.0 mg of lactose and 50.0 mg of corn starch. The mixture is compressed to obtain tablets.

### Reference Example I 3

| | |
|---|---|
| (1) Reference Example H compound 29-2 | 5.0 mg |
| (2) Sodium Chloride | 20.0 mg |
| (3) Distilled water amount to give total amount of | 2 ml |

5.0 mg of Reference Example H compound 29-2 and 20.0 mg of sodium chloride are dissolved in distilled water, and to this was added water to give a total amount of 2.0 ml. The solution is filtrated, and a 2 ml ampoule is filled with the filtrate under sterile condition. The ampoule is disinfected, then, sealed to give an injection solution.

### Reference Example J 1

| | |
|---|---|
| (1) Rofecoxiv | 5.0 mg |
| (2) Sodium chloride | 20.0 mg |
| (3) Distilled water amount to give a total amount of | 2 ml |

5.0 mg of lofecoxiv and 20.0 mg of sodium chloride are dissolved in distilled water, and to this is added water to give a total amount of 2.0 ml. The solution is filtrated, and a 2 ml ampoule is filled with the filtrate under sterile condition. The ampoule is disinfected, then, sealed to give an injection solution.

### Reference Example J 2

| | |
|---|---|
| (1) Rofecoxiv | 50 mg |
| (2) Lactose | 34 mg |
| (3) Corn starch | 10.6 mg |
| (4) Corn starch (paste) | 5 mg |
| (5) Magnesium stearate | 0.4 mg |
| (6) Calcium carboxymethylcellulose | 20 mg |
| Total | 120 mg |

The above-described components (1) to (6) are mixed according to a normal method, and tabletted by a tabletting machine to obtain tablets.

### Reference Example I 4

Any of preparations of Reference Examples I 1 to 3 and any of preparations of Reference Examples J 1 and 2 are combined.

### Reference Example K 1

Genetic manipulation methods described below are based on methods described in Maniatis et al., Molecular Cloning, ColdSpring Harbor Laboratory, 1989, and the appended reagent protocol.
(1) Cloning of human p38 MAP kinase gene and preparation of recombinant Baculovirus
   Cloning of human p38 MAP kinase gene was conducted by a PCR method using primer set p38-U: 5'-ACCACTCGAGATGGACTACAAGGACGACGATGACAAGTCTCAGGAGAGGCCCACGTTCTAC C-3' [SEQ ID No. 1] and PAG-L: 5'-ACCCGGTACCACCAGGTGCTCAGGACTCCATCTCT-3' [SEQ ID No. 2] synthesized referring to the nucleotide sequence of p38 MAP kinase gene of Han et al., Science 265 (5173), 808-811 (1994), utilizing kidney cDNA (QUICK-Clone cDNA, manufactured by Toyobo Co., Ltd.) as a template.
   A PCR reaction was performed according to Hot Start method using AmpliWax PCR Gem 100 (Takara Shuzo Co., Ltd.). For preparing lower layer mixed liquid, 2 µL of 10xLA PCR Buffer, 3 µL of 2.5 mM dNTP solution, each 2.5 µL of 12.5 µM primer solution, and 10 µL of sterile distilled water were mixed. For preparing upper layer mixed liquid, 1 µL of human heart cDNA (1 ng/mL) as a template, 3 µL of 10xLA PCR Buffer, 1 µL of 2.5 mM dNTP solution, 0.5 µL of TaKaRa LA Taq DNA polymerase (Takara Shuzo Co., Ltd.) and 24.5 µL of sterile distilled water were mixed. To the prepared lower mixed liquid was added one AmpliWax PCR Gem 100 (Takara Shuzo Co., Ltd.), treated for 5 minutes at 70°C and 5 minutes in ice, then, the upper mixed liquid was added, to prepare a reaction solution for PCR. A tube filled with the reaction solution was set on Thermal Cycler (Perkin Elmer), then, treated for 2 minutes at 95°C. Further, a cycle including 15 seconds at 95°C and 2 minutes at 68°C was repeated 35 times, then, treated for 8 minutes at 72°C. The resulted PCR product was subjected to agarose gel (1%) electrophoresis, a 1.1 kb DNA fragment containing a p38 MAP kinase gene was recovered from the gel, then, pT7Blue-T vector (Takara Shuzo Co., Ltd.) was inserted to prepare a plasmid pHP38.
   4.8 kb XhoI-KpnI fragment of plasmid pFASTBAC1 (CIBCOBRL) and 1.1 kb XhoI-Kpn fragment of the above-mentioned plasmid pHP38 were ligated to construct plasmid pFBHP38.
   Plasmid pFBHP38 and BAC-TO-BAC Baculovirus Expression System (GIBCOBRL) were used to prepare virus stock BAC-HP38 of recombinant baculovirus.
(2) Cloning of human MKK3 gene and preparation of recombinant baculovirus
   Cloning of human MKK3 gene was conducted by a PCR method using primer set MKK-U: 5'-ACAAGAATTCATAACATATGGCTCATCATCATCATCATCATTCCAAGCCACCCGCACCCAA -3' [SEQ ID No. 3] and MKK-L: 5'-TCCCGTCTAGACTATGAGTCTTCTCCCAGGAT-3' [SEQ ID No. 4] synthesized referring to the nucleotide sequence of MKK3 gene of Derijard, B. et al., Science 267 (5198), 682-685 (1995), utilizing kidney cDNA (QUICK-Clone cDNA, manufactured by Toyobo Co., Ltd.) as a template.
   A PCR reaction was performed according to Hot Start method using AmpliWax PCR Gem 100 (Takara Shuzo Co., Ltd.). For preparing lower layer mixed liquid, 2 µL of 10xLA PCR Buffer, 3 µL of 2.5 mM dNTP solution, each 2.5 µL of 12.5 µM primer solution, and 10 µL of sterile distilled water were mixed. For preparing upper layer mixed liquid, 1 µL of human kidney cDNA (1 ng/mL) as a template, 3 µL of 10xLA PCR Buffer, 1 µL of 2.5 mM dNTP solution, 0.5 µL of TaKaRa LA Taq DNA polymerase (Takara Shuzo Co., Ltd.) and 24.5 µL of sterile distilled water were mixed. To the prepared lower mixed liquid was added one AmpliWax PCR Gem 100 (Takara Shuzo Co., Ltd.), treated for 5 minutes at 70°C and 5 minutes in ice, then, the upper mixed liquid was added, to prepare a reaction solution for PCR. A tube filled with the reaction solution was set on Thermal Cycler (Perkin Elmer), then, treated for 2 minutes at 95°C. Further, a cycle including 15 seconds at 95°C and 2 minutes at 68°C was repeated 35 times, then, treated for 8 minutes at 72°C. The resulted PCR product was subjected to agarose gel (1%) electrophoresis, a 1.0 kb DNA fragment containing a MKK3 gene was recovered from the gel, then, pT7Blue-T vector (Takara Shuzo Co., Ltd.) was inserted to prepare a plasmid pHMKK3.
   For converting MKK3 into constitutively active type
   (Ser at 189 is converted into Glu, and Thr at 193 is converted into Glu), mutation was introduced by QuikChange Site-Directed Mutagenesis Kit (Stratagene) using primer set SER-U: 5'-GGCTACTTGGTGGACGAGGTGGCCAAGGAGATGGATGCCGGCTGC-3' [SEQ ID No. 5] and SER-L: 5'-GCAGCCGGCATCCATCTCCTTGGCCACCTCGTCCACCAAGTAGCC-3' [SEQ ID No. 6], to obtain pcaMKK3.
   4.8 kb EcoRI-XbaI fragment of plasmid pFASTBAC1 (CIBCOBRL) and 1.0 kb EcoRI-XbaI fragment of the above-mentioned plasmid pcaMKK3 were ligated to construct plasmid pFBcaMKK3.
   Plasmid pFBcaMKK3 and BAC-TO-BAC Baculovirus Expression System (GIBCOBRL) were used to prepare virus stock BAC-caMKK3 of recombinant baculovirus.
(3) Preparation of active type p38 MAP kinase
   Sf-21 cells were inoculated on 100 mL Sf-900II SF medium (GIBCOBRL) to give 1x10⁶cells/mL, then, cultured for 24 hours at 27°C. Each 0.2 mL of virus stocks BAC-HP38 and BAC-caMKK3 of the recombinant baculovirus were added, then, cultured for further 48 hours. Cells were separated from the culture solution by centrifugal separation (3000 rpm, 10 min.), then, washed with PBS twice. The cells were suspended in 10 mL of Lysis buffer (25mM HEPES (pH7.5), 1% TritonX, 130mM NaCl, 1mM EDTA, 1mM DTT, 25mM β-glycerophosphate, 20mM leupeptin, 1mM APMSF, 1mM Sodium orthovanadate), then, treatment by Homogenizer (POLYTRON) for 2 minutes at 20000 rpm was performed twice to lyse the cells. Active type p38 MAP kinase was purified from the supernatant obtained by centrifugal separation (40000 rpm, 45 minutes), by using Anti-FLAG M2 Affinity Gel (Eastman Chemical).
(4) To 37.5 µL of a reaction solution (25 mM HEPES (pH 7.5), 10 mM Magnesium Acetate) containing 260 ng of active type p38 MAP kinase and 1 µg of Myelin Basic Protein was added 2.5 µL of a sample compound dissolved in DMSO, then, the mixture was kept at 30°C for 5 minutes. The reaction was initiated by adding 10 µL of an ATP solution (2.5 µM ATP, 0.1 µCi[γ-³²P] ATP). After reaction for 60 minutes at 30°C, the reaction was terminated by adding 50 µL of a 20% TCA solution. The reaction solution was left for 20 minutes at 0°C, then, acid insoluble fraction was transferred to GF/C filter (Packard Japan) using Cell Harvester (Packard Japan), and washed with 250 mM H₃PO₄. After drying for 60 minutes at 45°C, 40 µL of Microscint 0 (Packard Japan) was added, and radiation activity was measured by Top Counter (Packard Japan). The concentration (IC₅₀ value) of a sample compound necessary for inhibiting 50% of incorporation into the acid insoluble fraction of ³²P was calculated by PRISM 2.01 (GraphPad Software).

The results are shown in Table 76 below.

**Table 76**

| Reference Example H Compound No. | IC₅₀ (µM) |
|---|---|
| 30-2 | 0.010 |
| 34-2 | 0.0099 |
| 36-10 | 0.0011 |
| 46-4 | 0.017 |
| 58-7 | 0.0084 |

### Reference Example K 2

### Measurement of inhibitory activity of TNF-α production

THP-1 cells cultured in a PRMI 1640 medium (Life Technologies, Inc., USA) containing 1% inactivated fetal bovine serum (Life Technologies, Inc.) and 10mM HEPES (pH 7.5) were inoculated on a 96 well plate to give 1x10⁵ cells/well, then, 1 µL of a sample compound dissolved in DMSO was added. After incubation for 1 hour at 37°C in a CO₂ incubator, LPS (Wako Pure Chemical Industries Ltd.) was added to give a final concentration of 5 µg/mL. After incubation for 4 hours at 37°C in a CO₂ incubator, the supernatant was obtained by centrifugal separation. The TNF-α concentration in the supernatant was measured by ELISA (R&D Systems, Quantikine Kit). The concentration (IC₅₀ value) of a sample compound necessary for inhibiting 50% of TNF-α production was calculated by PRISM 2.01 (GraphPad Software).

The results are shown in Table 77 below.

**Table 77**

| Reference Example H Compound No. | IC₅₀ (µM) |
|---|---|
| 30-2 | 0.12 |
| 34-2 | 0.002 |
| 36-10 | 0.055 |
| 46-4 | 0.082 |
| 58-7 | 0.021 |

From the above-described results, it is known that compounds of the present invention have excellent inhibitory activity of p38 MAP kinase and TNF-α production.

### Reference Example L 1

### 1-bromo-3-ethylbenzene

To an aqueous 50% sulfuric acid solution (43.6 g) containing 3-ethylaniline (10.0 g, 82.5 mmol) was added dropwise an aqueous solution (16.5 mL) of sodium nitrite (6.83 g, 99.0 mmol) at 0°C over 30 min. The obtained reaction mixture was stirred at 0°C for 45 min. A solution of this diazonium salt was added by small portions to a 48% hydrobromic acid solution (82.5 mL) containing copper(I) bromide (12.4 g, 86.6 mmol) with heating under gentle reflux. After the addition, the reaction mixture was heated under reflux for 30 min. The reaction mixture was cooled to room temperature and extracted with ethyl ether. The extract was washed successively with 1N aqueous sodium hydroxide solution and saturated brine, filtered, dried and concentrated. The residue was purified by silica gel column chromatography (hexane-ethyl acetate = 20 : 1) to give the title compound (6.13 g, yield 40%).
oil
¹H-NMR(CDCl₃)δ: 1.23 (3H, t, J= 7.5Hz), 2.63 (2H, q, J= 7.5Hz), 7.11-7.20 (2H, m), 7.28-7.38 (2H, m).

### Reference Example L 2

In accordance with Reference Example L 1 and using 3-(1-methylethyl)aniline instead of 3-ethylaniline, the following Reference Example L compound 2 was synthesized.
Reference Example L compound 2: 1-bromo-3-(1-methylethyl)benzene
oil
¹H-NMR(CDCl₃)δ: 1.24 (6H, d, J= 7.0Hz), 2.77-2.99 (1H, m), 7.03-7.16 (2H, m), 7.27-7.34 (1H, m), 7.37 (1H, s).

### Reference Example L 3

### 3-ethylbenzoic acid

Under an argon atmosphere, a solution of 1-bromo-3-ethylbenzene (5.1 g, 28 mmol) in tetrahydrofuran (45 mL) was added dropwise to a mixture of magnesium turnings (0.74 g, 31 mmol) in tetrahydrofuran (5.0 mL), and the mixture was stirred for 30 min. The reaction mixture was added to crushed dry ice and stirred for 1 hr. 1N Hydrochloric acid was added to the reaction mixture and the mixture was extracted with ethyl acetate. The extract was dried, filtered and concentrated. The residue was purified by silica gel column chromatography (hexane-ethyl acetate = 5 : 1) to give the title compound (3.87 g, yield 93%).
oil
¹H-NMR(CDCl₃)δ: 1.28 (3H, t, J= 7.5Hz), 2.73 (2H, q, J= 7.5Hz), 7.34-7.50 (2H, m), 7.92-7.98 (2H, m).

### Reference Example L 4

In accordance with Reference Example L 3 and using 1-bromo-3-(1-methylethyl)benzene and 1-bromo-4-fluoro-3-methylbenzene instead of 1-bromo-3-ethylbenzene, the following Reference Example L compounds 4-1 and 4-2 were synthesized respectively.
Reference Example L compound 4-1: 3-(1-methylethyl)benzoic acid
   oil
   ¹H-NMR(CDCl₃)δ: 1.29 (6H, d, J= 7.0Hz), 2.98-3.06 (1H, m), 7.38-7.54 (2H, m), 7.90-8.02 (2H, m).
Reference Example L compound 4-2: 4-fluoro-3-methylbenzoic acid
   m.p.: 165-167°C.

### Reference Example L 5

### 3-ethylbenzoyl chloride

3-Ethylbenzoic acid (9.40 g, 62.6 mmol) was slowly added to thionyl chloride (45 mL) at 0°C and N,N-dimethylformamide (3 drops) was added dropwise. The obtained reaction mixture was heated under reflux for 2 hrs. The reaction mixture was concentrated and used for the next reaction without purification.

### Reference Example L 6

In accordance with Reference Example L 5 and using 3-(1-methylethyl)benzoic acid and 4-fluoro-3-methylbenzoic acid instead of 3-ethylbenzoic acid, the following Reference Example L compounds 6-1 and 6-2 were synthesized respectively.
Reference Example L compound 6-1: 3-(1-methylethyl)benzoyl chloride
   Used for the next reaction without purification.
Reference Example L compound 6-2: 4-fluoro-3-methylbenzoyl chloride
   Used for the next reaction without purification.

### Reference Example L 7

### N-(4-chlorobenzoyl)propylenimine

A solution of propyleneimine (12 mL, 0.15 mol) in tetrahydrofuran (160 mL) was added to 1N aqueous sodium hydroxide solution. To this mixture was added dropwise 4-chlorobenzoyl chloride (25 g, 0.14 mol) at 0°C. After the completion of the dropwise addition, the mixture was further stirred for 30 min. The reaction mixture was extracted with ethyl acetate. The extract was dried and the solvent was evaporated to give the title compound (25 g, yield 89%).
oil
¹H-NMR(CDCl₃)δ: 1.39 (3H, d, J= 5.5Hz), 2.15 (1H, d, J= 2.9Hz), 2.51-2.66 (2H, m), 7.39-7.47 (2H, m), 7.93-8.01 (2H, m).

### Reference Example L 8

In accordance with Reference Example L 7 and using 3-chlorobenzoyl chloride, 3-methylbenzoyl chloride, 3-ethylbenzoyl chloride, 3-(1-methylethyl)benzoyl chloride, 4-fluoro-3-methylbenzoyl chloride and 3-fluorobenzoyl chloride instead of 4-chlorobenzoyl chloride, the following Reference Example L compounds 8-1 to 8-6 were synthesized respectively.
Reference Example L compound 8-1: N-(3-chlorobenzoyl)propylenimine
   oil
   ¹H-NMR(CDCl₃)δ: 1.40 (3H, d, J= 5.1Hz), 2.17 (1H, d, J= 3.3Hz), 2.53-2.68 (2H, m), 7.40 (1H, dd, J= 7.7, 8.1Hz), 7.53 (1H, ddd, J= 1.5, 2.2, 8.1Hz), 7.90 (1H, dt, J= 7.7, 1.5Hz), 8.00 (1H, dd, J= 1.5, 2.2Hz).
Reference Example L compound 8-2: N-(3-methylbenzoyl)propylenimine
   oil
   ¹H-NMR(CDCl₃)δ: 1.39 (3H, d, J= 5.5Hz), 2.14 (1H, d, J= 3.3Hz), 2.41 (3H, s), 2.51-2.66 (2H, m), 7.32-7.39 (2H, m), 7.79-7.87
   (2H, m).
Reference Example L compound 8-3: N-(3-ethylbenzoyl)propylenimine
   oil
   ¹H-NMR(CDCl₃)δ: 1.27 (3H, t, J= 7.5Hz), 1.40 (3H, d, J= 5.5Hz), 2.14 (1H, d, J= 2.9Hz), 2.52-2.61 (2H, m), 2.71 (2H, q, J= 7.5Hz), 7.32-7.41 (2H, m), 7.81-7.89 (2H, m).
Reference Example L compound 8-4: N-[3-(1-methylethyl)benzoyl]propylenimine
   oil
   ¹H-NMR(CDCl₃)δ: 1.29 (6H, d, J= 7.0Hz), 1.40 (3H, d, J= 5.9Hz), 2.14 (1H, d, J= 3.7Hz), 2.51-2.64 (2H, m), 2.87-3.10 (1H, m), 7.33-7.46 (2H, m), 7.84 (1H, dt, J= 7.0, 1.8Hz), 7.91 (1H, s).
Reference Example L compound 8-5: N-(4-fluoro-3-methylbenzoyl)propylenimine
   oil
   ¹H-NMR(CDCl₃)δ: 1.39 (3H, d, J= 5.4Hz), 2.14 (1H, d, J= 3.4Hz), 2.33 (s, 3H), 2.51-2.61 (2H, m), 7.06 (1H, t, J= 8.8Hz), 7.81-7.90 (2H, m).
Reference Example L compound 8-6: N-(3-fluorobenzoyl)propylenimine
   oil
   ¹H-NMR(CDCl₃)δ: 1.40 (3H, d, J= 5.5Hz), 2.16 (1H, d, J= 3.3Hz), 2.52-2.68 (2H, m), 7.25 (1H, ddd, J= 1.1, 2.6, 8.4Hz), 7.43 (1H, ddd, J= 5.5, 7.7, 8.1Hz), 7.69 (1H, ddd, J= 1.5, 2.6, 8.1Hz), 7.81 (1H, ddd, J= 1.1, 1.5, 7.7Hz).

### Reference Example L 9

### 2-fluoro-4-methylpyridine

The title compound was synthesized according to the method described in Journal of Medicinal Chemistry, vol. 33, pp. 1667-1675 (1990).
b.p.: 82-86°C (10kPa).

### Reference Example L 10

### 2-tert-butoxycarbonylamino-4-methylpyridine

The title compound was synthesized according to the method described in Synthesis, pp. 877-882 (1996) or Journal of Organic Chemistry, vol. 61, pp. 4810-4811 (1996).

### Reference Example L 11

### 2-(2-fluoro-4-pyridyl)-1-(3-methylphenyl)ethanone

Under an argon atmosphere, a solution of diisopropylamine (44 mL, 0.31 mol) in anhydrous tetrahydrofuran (300 mL) was cooled to -78°C and, with stirring, a solution of 1.6M n-butyl lithium in hexane (190 mL, 0.31 mol) was added dropwise. After the completion of the dropwise addition, the mixture was stirred for 10 min. and a solution of 2-fluoro-4-methylpyridine (34.5 g, 0.31 mol) in anhydrous tetrahydrofuran (30 mL) was added. The reaction mixture was stirred at -10°C for 30 min. The reaction solution was cooled to -78°C and a solution of N-(3-methylbenzoyl)propyleneimine (52 g, 0.30 mol) in anhydrous tetrahydrofuran (30 mL) was added dropwise. After the completion of the dropwise addition, the mixture was stirred at room temperature for 2 hrs. Water (100 mL) was added to the reaction mixture and the mixture was extracted with ethyl acetate. The extract was washed with water, dried and the solvent was evaporated. The residue was recrystallized from isopropyl ether to give the title compound (35 g, yield 52%).
m.p.: 66-67°C.

### Reference Example L 12

In accordance with Reference Example L 11 and using N-(3-chlorobenzoyl)propyleneimine instead of N-(3-methylbenzoyl)propyleneimine, the following Reference Example compound 12 was synthesized.
Reference Example L compound 12: 1-(3-chlorophenyl)-2-(2-fluoro-4-pyridyl)ethanone
m.p.: 84-86°C.

### Reference Example L 13

### 2-(2-tert-butoxycarbonylamino-4-pyridyl)-1-(3-methylphenyl)ethanone

A solution of 2-tert-butoxycarbonylamino-4-methylpyridine (146 g, 0.700 mol) in anhydrous tetrahydrofuran (1.30 L) was cooled to -78°C and, with stirring, a solution of 1.6 M n-butyl lithium in hexane (875 mL, 1.40 mol) was added dropwise. After the completion of the dropwise addition, the mixture was stirred at 0°C for 30 min. and cooled to -78°C. A solution of N-(3-methylbenzoyl)propyleneimine (123 g, 0.700 mol) in anhydrous tetrahydrofuran (130 mL) was added dropwise. After the completion of the dropwise addition, the mixture was stirred at -78°C for 1 hr., warmed to room temperature and stirred for 1 hr. Saturated brine (1.30 L) was added to the reaction mixture and the organic layer was separated. The aqueous layer was extracted with ethyl acetate and the combined organic layer was dried and concentrated. The crude crystals were recrystallized from ethyl acetate to give the title compound (185 g, yield 81%).
m.p.: 144-146°C.

### Reference Example L 14

In accordance with Reference Example 13 and using N-(3-chlorobenzoyl)propylenimine, N-[3-(1-methylethyl)benzoyl]propylenimine, N-(4-fluoro-3-methylbenzoyl)propylenimine, N-(3-fluorobenzoyl)propylenimine, N-(4-chlorobenzoyl)propylenimine and N-(3-ethylbenzoyl)propylenimine instead of N-(3-methylbenzoyl)propylenimine, the following Reference Example compounds 14-1 to 14-6 were synthesized respectively.
Reference Example compound 14-1: 2-(2-tert-butoxycarbonylamino-4-pyridyl)-1-(3-chlorophenyl)ethanone
   m.p.: 152-153°C.
Reference Example compound 14-2: 2-(2-tert-butoxycarbonylamino-4-pyridyl)-1-[3-(1-methylethyl)phenyl]ethanone
   m.p.: 176-177°C.
Reference Example compound 14-3: 2-(2-tert-butoxycarbonylamino-4-pyridyl)-1-(4-fluoro-3-methylphenyl)ethanone
   m.p.: 143-144°C.
Reference Example compound 14-4: 2-(2-tert-butoxycarbonylamino-4-pyridyl)-1-(3-fluorophenyl)ethanone
   m.p.: 164-165°C.
Reference Example compound 14-5: 2-(2-tert-butoxycarbonylamino-4-pyridyl)-1-(4-chlorophenyl)ethanone
   m.p.: 155-156°C.
Reference Example compound 14-6: 2-(2-tert-butoxycarbonylamino-4-pyridyl)-1-(3-ethylphenyl)ethanone
   m.p.: 122-123°C.

### Reference Example L 15

### 2-(2-amino-4-pyridyl)-1-(3-methylphenyl)ethanone

2-(2-tert-Butoxycarbonylamino-4-pyridyl)-1-(3-methylphenyl)ethanone (50.0 g, 0.153 mol) was added to 2N-hydrochloric acid (260 mL) and the mixture was stirred at 100°C for 2 hrs. The reaction mixture was neutralized with aqueous sodium hydroxide solution and extracted with ethyl acetate. The extract was dried and concentrated. The crude crystals were washed with isopropyl ether to give the title compound (29.1 g, yield 84%).
m.p.: 119-120°C.

### Reference Example L 16

In accordance with Reference Example L 15 and using 2-(2-tert-butoxycarbonylamino-4-pyridyl)-1-(3-chlorophenyl)ethanone instead of 2-(2-tert-butoxycarbonylamino-4-pyridyl)-1-(3-methylphenyl)ethanone, the following Reference Example compound 16 was synthesized.
Reference Example L compound 16: 2-(2-amino-4-pyridyl)-1-(3-chlorophenyl)ethanone
m.p.: 173-175°C.

### Reference Example L 17

### N-[4-[2-(3-methylphenyl)-2-oxoethyl]-2-pyridyl]benzamide

To a solution of 2-(2-amino-4-pyridyl)-1-(3-methylphenyl)ethanone (24.0 g, 0.106 mol) in acetonitrile (500 mL) was added benzoyl chloride (27.0 mL, 0.233 mol) at 0°C. Triethylamine (35.6 mL, 0.256 mol) was added dropwise to the obtained mixture and the mixture was stirred at room temperature for 4 hrs. Water was added to the reaction mixture and the precipitated solids were collected by filtration. The aqueous layer was extracted with ethyl acetate and the extract was washed with aqueous sodium hydrogen carbonate solution. The extract was dried and concentrated. The obtained residue and the solid were dissolved in a mixture of tetrahydrofuran (450 mL) and methanol (110 mL) and 1N aqueous sodium hydroxide solution (256 mL) was added. The reaction mixture was stirred for 2 hrs. and concentrated. The residue was extracted with ethyl acetate, dried and concentrated. The residue was purified by silica gel column chromatography (hexane-ethyl acetate = 2 : 1), and the obtained oil was crystallized from ethyl ether to give the title compound (19.5 g, yield 56%).
m.p.: 67-69°C.

### Reference Example L 18

In accordance with Reference Example L 17 and using 2-(2-amino-4-pyridyl)-1-(3-chlorophenyl)ethanone instead of 2-(2-amino-4-pyridyl)-1-(3-methylphenyl)ethanone, the following Reference Example L compound 18 was synthesized.
Reference Example L compound 18: N-[4-[2-(3-chlorophenyl)-2-oxoethyl]-2-pyridyl]benzamide
m.p.: 121-123°C.

### Reference Example L 19

### 2-(2-amino-4-pyridyl)-2-bromo-1-(3-methylphenyl)ethanone hydrobromide

To a solution of 2-(2-tert-butoxycarbonylamino-4-pyridyl)-1-(3-methylphenyl)ethanone (185 g, 0.566 mol) in acetic acid (400 mL) was added bromine (29.2 mL, 0.566 mol) and the mixture was stirred at 80°C for 2 hrs. The reaction mixture was concentrated and the residue was crystallized from acetonitrile-ethyl acetate to give the title compound (171 g, yield 78%).
m.p.: 182-185°C.

### Reference Example L 20

In accordance with Reference Example L 19 and using 2-(2-fluoro-4-pyridyl)-1-(3-methylphenyl)ethanone, 1-(3-chlorophenyl)-2-(2-fluoro-4-pyridyl)ethanone, 2-(2-tert-butoxycarbonylamino-4-pyridyl)-1-(3-chlorophenyl)ethanone, 2-(2-tert-butoxycarbonylamino-4-pyridyl)-1-[3-(1-methylethyl)phenyl]ethanone, 2-(2-tert-butoxycarbonylamino-4-pyridyl)-1-(3-fluorophenyl)ethanone, 2-(2-tert-butoxycarbonylamino-4-pyridyl)-1-(4-chlorophenyl)ethanone, 2-(2-tert-butoxycarbonylamino-4-pyridyl)-1-(3-ethylphenyl)ethanone and 2-(2-tert-butoxycarbonylamino-4-pyridyl)-1-(4-fluoro-3-methylphenyl)ethanone instead of 2-(2-tert-butoxycarbonylamino-4-pyridyl)-1-(3-methylphenyl)ethanone, the following Reference Example L compounds 20-1 to 20-8 were synthesized respectively.
Reference Example L compound 20-1: 2-bromo-2-(2-fluoro-4-pyridyl)-1-(3-methylphenyl)ethanone hydrobromide
   Used for the next reaction without purification.
Reference Example L compound 20-2: 2-bromo-1-(3-chlorophenyl)-2-(2-fluoro-4-pyridyl)ethanone hydrobromide
   amorphous powder
   ¹H-NMR(DMSO-d₆)δ: 7.19 (1H, s), 7.38 (1H, s), 7.52-7.56 (1H, m), 7.64 (1H, t, J= 8.0Hz), 7.77-7.82 (1H, m), 8.05-8.09 (1H, m), 8.16 (1H, t, J= 1.8Hz), 8.32 (1H, d, J= 5.2Hz), 10.23 (1H, br s).
Reference Example L compound 20-3: 2-(2-amino-4-pyridyl)-2-bromo-1-(3-chlorophenyl)ethanone hydrobromide
   m.p.: 199-200°C.
Reference Example L compound 20-4: 2-(2-amino-4-pyridyl)-2-bromo-1-[3-(1-methylethyl)phenyl]ethanone hydrobromide amorphous powder
   ¹H-NMR(DMSO-d₆) δ: 1.24 (6H, d, J= 6.6Hz), 3.00 (1H, septet, J= 6.6Hz), 7.15 (1H, s), 7.17 (1H, s), 7.46-7.65 (2H, m), 7.88-7.98 (4H, m), 8.09 (1H, br s).
Reference Example L compound 20-5: 2-(2-amino-4-pyridyl)-2-bromo-1-(3-fluorophenyl)ethanone hydrobromide
   m.p.: 206-207°C.
Reference Example L compound 20-6: 2-(2-amino-4-pyridyl)-2-bromo-1-(4-chlorophenyl)ethanone hydrobromide
   m.p.: 202-203°C.
Reference Example L compound 20-7: 2-(2-amino-4-pyridyl)-2-bromo-1-(3-ethylphenyl)ethanone hydrobromide
   m.p.: 46-47°C.
Reference Example L compound 20-8: 2-(2-amino-4-pyridyl)-2-bromo-1-(4-fluoro-3-methylphenyl)ethanone hydrobromide
   m.p.: 225-226°C.

### Reference Example L 21

### N-[4-[1-bromo-2-(3-methylphenyl)-2-oxoethyl]-2-pyridyl]benzamide hydrobromide

To a solution of N-[4-[2-(3-methylphenyl)-2-oxoethyl]-2-pyridyl]benzamide (19.0 g, 57.5 mmol) in acetic acid (60 mL) was added dropwise bromine (3.0 mL, 57.5 mmol) at room temperature over 1 hr., and the reaction mixture was stirred for 2 hrs. The precipitated crude crystals were collected by filtration and washed with ethyl acetate to give the title compound (25.4 g, yield 90%).
m.p.: 203-206°C.

### Reference Example L 22

In accordance with Reference Example L 21 and using N-[4-[2-(3-chlorophenyl)-2-oxoethyl]-2-pyridyl]benzamide instead of N-[4-[2-(3-methylphenyl)-2-oxoethyl]-2-pyridyl]benzamide, the following Reference Example L compound 22 was synthesized. Reference Example L compound 22: N-[4-[1-bromo-2-(3-chlorophenyl)-2-oxoethyl]-2-pyridyl]benzamide hydrobromide
m.p.: 212-213°C.

### Reference Example L 23

### thiobutylamide

Butyronitrile (10.0 g, 145 mol) was dissolved in a 4N hydrogen chloride in ethyl acetate solution (100 mL). To this solution was added O,O-diethyl phosphorodithioate (26.7 mL, 0.160 mol), and the mixture was stirred at room temperature for 22 hrs. Water (100 mL) was added to the reaction mixture, and the mixture was extracted with ethyl acetate. The filtrate was washed with saturated brine and aqueous sodium hydrogen carbonate solution, dried, and the solvent was evaporated. The residue was purified by silica gel column chromatography (hexane-ethyl acetate = 1 : 1) to give the title compound (6.68 g, yield 45%).
oil
¹H-NMR(CDCl₃)δ: 0.99 (3H, t, J= 7.6 Hz), 1.72-1.93 (2H, m), 2.64 (2H, t, J= 7.6Hz), 7.02 (1H, br s), 7.77 (1H, br s).

### Reference Example L 24

In accordance with Reference Example L 23 and using 1-methylpiperidine-4-carbonitrile instead of butyronitrile, the following Reference Example L compound 24 was synthesized. Reference Example L compound 24: 1-methylpiperidine-4-carbothioamide
m.p.: 216-220°C.

### Reference Example L 25

### [5-(2-fluoro-4-pyridyl)-4-(3-methylphenyl)-1,3-thiazol-2-yl]amine

To a mixture of 2-bromo-2-(2-fluoro-4-pyridyl)-1-(3-methylphenyl)ethanone hydrobromide [synthesized according to Reference Example L 19 using 2-(2-fluoro-4-pyridyl)-1-(3-methylphenyl)ethanone (8.46 g, 36.9 mmol) instead of 2-(2-tert-butoxycarbonylamino-4-pyridyl)-1-(3-methylphenyl)ethanone] and thiourea (3.03 g, 39.8 mmol) in acetonitrile (50 mL) was added triethylamine (5.2 mL, 37.3 mmol), and the mixture was stirred at 80°C for 2 hrs. Aqueous sodium hydrogen carbonate solution was poured into the reaction mixture and the precipitated solids were collected by filtration. The obtained solid was washed with water and dried. Crude crystals were recrystallized from ethanol to give the title compound (3.67 g, yield 35%).
m.p.: 214-218°C.

### Reference Example L 26

### 2-ethyl-5-(2-fluoro-4-pyridyl)-4-(3-methylphenyl)-1,3-thiazole

A solution of 2-bromo-2-(2-fluoro-4-pyridyl)-1-(3-methylphenyl)ethanone hydrobromide (11 g, 29 mmol) and thiopropionamide (2.7 g, 30 mmol) in N,N-dimethylformamide (30 mL) was stirred at room temperature for 14 hrs. Aqueous sodium hydrogen carbonate solution was poured into the reaction mixture, and the mixture was extracted with ethyl acetate. The extract was washed with water, dried and the solvent was evaporated. The residue was purified by silica gel column chromatography (hexane-ethyl acetate = 4 : 1) to give the title compound (3.3 g, yield 38%).
oil
¹H-NMR(CDCl₃)δ: 1.64 (3H, t, J= 7.6Hz), 2.34 (3H, s), 3.10 (2H, q, J= 7.6Hz), 6.84-6.86 (1H, m), 7.05-7.09 (1H, m), 7.13-7.25 (3H, m), 7.37 (1H, s), 8.10 (1H, d, J= 5.6Hz).

### Reference Example L 27

In accordance with Reference Example L 26 and using 2-bromo-2-(2-fluoro-4-pyridyl)-1-(3-chlorophenyl)ethanone hydrobromide instead of 2-bromo-2-(2-fluoro-4-pyridyl)-1-(3-methylphenyl)ethanone hydrobromide, the following Reference Example L compound 27 was synthesized.
Reference Example L compound 27: 2-ethyl-5-(2-fluoro-4-pyridyl)-4-(3-chlorophenyl)-1,3-thiazole
m.p.: 102-103°C.

### Reference Example L 28

### 4-[2-ethyl-4-(3-methylphenyl)-1,3-thiazol-5-yl]-2-pyridylamine

A solution of 2-(2-amino-4-pyridyl)-2-bromo-1-(3-methylphenyl)ethanone hydrobromide (125 g, 0.323 mol) and thiopropionamide (28g, 0.314 mol) in N,N-dimethylformamide (1200 mL) was stirred at room temperature for 14 hrs. The solvent was evaporated under reduced pressure. Aqueous sodium hydrogen carbonate solution was poured into the residue and the mixture was extracted with ethyl acetate. The extract was washed with saturated aqueous sodium hydrogen carbonate solution, dried and concentrated. Crude crystals were washed with hexane-ethyl acetate = 1 : 1 to give the title compound (76.0 g, yield 82%).
m.p.: 144-146°C.

### Reference Example L 29

In accordance with Reference Example L 28 and using thiobutylamide instead of thiopropionamide, the following Reference Example L compound 29 was synthesized.
Reference Example L compound 29: 4-[4-(3-methylphenyl)-2-propyl-1,3-thiazol-5-yl]-2-pyridylamine
m.p.: 113-115°C.

### Reference Example L 30

In accordance with Reference Example L 28 and using 2-(2-amino-4-pyridyl)-2-bromo-1-(3-fluorophenyl)ethanone hydrobromide, 2-(2-amino-4-pyridyl)-2-bromo-1-(4-chlorophenyl)ethanone hydrobromide, 2-(2-amino-4-pyridyl)-2-bromo-1-(3-ethylphenyl)ethanone hydrobromide, 2-(2-amino-4-pyridyl)-2-bromo-1-(4-fluoro-3-methylphenyl)ethanone hydrobromide and 2-(2-amino-4-pyridyl)-2-bromo-1-[3-(1-methylethyl)phenyl]ethanone hydrobromide instead of 2-(2-amino-4-pyridyl)-2-bromo-1-(3-methylphenyl)ethanone hydrobromide, the following Reference Example L compounds 30-1 to 30-5 were synthesized respectively.
Reference Example L compound 30-1: 4-[2-ethyl-4-(3-fluorophenyl)-1,3-thiazol-5-yl]-2-pyridylamine
   m.p.: 153-154°C.
Reference Example L compound 30-2: 4-[4-(4-chlorophenyl)-2-ethyl-1,3-thiazol-5-yl]-2-pyridylamine
   m.p.: 136-137°C.
Reference Example L compound 30-3: 4-[2-ethyl-4-(3-ethylphenyl)-1,3-thiazol-5-yl]-2-pyridylamine
   m.p.: 128-129°C.
Reference Example L compound 30-4: 4-[2-ethyl-4-(4-fluoro-3-methylphenyl)-1,3-thiazol-5-yl]-2-pyridylamine
   m.p.: 134-135°C.
Reference Example L compound 30-5: 4-[2-ethyl-4-[3-(1-methylethyl)phenyl]-1,3-thiazol-5-yl]-2-pyridylamine
   m.p.: 80-81°C.

### Reference Example L 31

### [5-(2-benzylamino-4-pyridyl)-4-(3-methylphenyl)-1,3-thiazol-2-yl]amine

A mixture of [5-(2-fluoro-4-pyridyl)-4-(3-methylphenyl)-1,3-thiazol-2-yl]amine (0.29 g, 1.0 mmol) and benzylamine (1.2 mL, 11 mmol) was stirred at 150°C for 3 hrs. The reaction mixture was cooled to room temperature, saturated aqueous sodium hydrogen carbonate solution was added and the mixture was extracted with ethyl acetate. The extract was washed with water, dried and concentrated. The residue was purified by silica gel column chromatography (hexane-ethyl acetate = 1 : 1) to give the title compound (0.16 g, yield 41%).
m.p.: 178-179°C.

### Reference Example L 32

In accordance with Reference Example L 31 and using cyclohexylamine and cyclopentylamine instead of benzylamine, the following Reference Example L compounds 32-1 and 32-2 were synthesized respectively.
Reference Example L compound 32-1: [5-(2-cyclohexylamino-4-pyridyl)-4-(3-methylphenyl)-1,3-thiazol-2-yl]amine
   m.p.: 168-169°C.
Reference Example L compound 32-2: [5-(2-cyclopentylamino-4-pyridyl)-4-(3-methylphenyl)-1,3-thiazol-2-yl]amine
   m.p.: 169-170°C.

### Reference Example L 33

### N-cyclopentyl-4-[2-ethyl-4-(3-methylphenyl)-1,3-thiazol-5-yl]-2-pyridylamine

A mixture of 2-ethyl-5-(2-fluoro-4-pyridyl)-4-(3-methylphenyl)-1,3-thiazole (0.48 g, 1.6 mmol) and cyclopentylamine (1.6 mL, 16 mmol) was heated under reflux for 14 hrs. The reaction mixture was cooled to room temperature, saturated aqueous sodium hydrogen carbonate solution was added, and the mixture was extracted with ethyl acetate. The extract was washed with water, dried and concentrated. The obtained crude crystals were recrystallized from ethyl acetate to give the title compound (0.19 g, yield 33%).
m.p.: 117-118°C.

### Reference Example L 34

### 4-[4-(3-chlorophenyl)-2-ethyl-1,3-thiazol-5-yl]-N-[(1S)-1-phenylethyl]-2-pyridylamine hydrochloride

A mixture of 4-(3-chlorophenyl)-2-ethyl-5-(2-fluoro-4-pyridyl)-1,3-thiazole (0.35 g, 1.1 mmol) and (S)-1-phenylethylamine (1.4 mL, 11 mmol) was stirred at 150°C for 16 hrs. The reaction mixture was cooled to room temperature, saturated aqueous sodium hydrogen carbonate solution was added and the mixture was extracted with ethyl acetate. The extract was washed with water, dried and concentrated. The obtained oil was treated with 10% hydrogen chloride-methanol to give the title compound (0.27 g, yield 56%).
m.p.: 165-166°C.

### Reference Example L 35

### N-[4-[2-ethyl-4-(3-methylphenyl)-1,3-thiazol-5-yl]-2-pyridyl]phenylacetamide

To a solution of 4-[2-ethyl-4-(3-methylphenyl)-1,3-thiazol-5-yl]-2-pyridylamine (0.80 g, 2.7 mmol) in tetrahydrofuran (8 mL) was added phenylacetyl chloride (0.47 mL, 3.0 mmol), and triethylamine (0.41 mL, 3.0 mmol) was added to the obtained mixture. The reaction mixture was stirred at room temperature for 2 hrs. Saturated aqueous sodium hydrogen carbonate solution was added and the mixture was extracted with ethyl acetate. The extract was washed with saturated brine, dried and concentrated. The residue was purified by silica gel column chromatography (hexane-ethyl acetate = 20 : 1 - 4 : 1) and crystallized from isopropyl ether to give the title compound (0.75 g, yield 67%).
m.p.: 107-108°C.

### Reference Example L 36

In accordance with Reference Example L 35 and using propionyl chloride instead of phenylacetyl chloride, the following Reference Example L compound 36 was synthesized. Reference Example L compound 36: N-[4-[2-ethyl-4-(3-methylphenyl)-1,3-thiazol-5-yl]-2-pyridyl]propionamide
m.p.: 103-104°C.

### Reference Example L 37

In accordance with Reference Example L 35 and using 4-[4-(3-methylphenyl)-2-propyl-1,3-thiazol-5-yl]-2-pyridylamine, 4-[4-(4-chlorophenyl)-2-ethyl-1,3-thiazol-5-yl]-2-pyridylamine, 4-[2-ethyl-4-(3-fluorophenyl)-1,3-thiazol-5-yl]-2-pyridylamine, 4-[2-ethyl-4-(3-ethylphenyl)-1,3-thiazol-5-yl]-2-pyridylamine and 4-[2-ethyl-4-[3-(1-methylethyl)phenyl]-1,3-thiazol-5-yl]-2-pyridylamine instead of 4-[2-ethyl-4-(3-methylphenyl)-1,3-thiazol-5-yl]-2-pyridylamine, the following Reference Example L compounds 37-1 to 37-5 were synthesized respectively.
Reference Example L compound 37-1: N-[4-[2-propyl-4-(3-methylphenyl)-1,3-thiazol-5-yl]-2-pyridyl]phenylacetamide
   m.p.: 109-111°C.
Reference Example L compound 37-2: N-[4-[4-(4-chlorophenyl)-2-ethyl-1,3-thiazol-5-yl]-2-pyridyl]phenylacetamide
   m.p.: 150-151°C.
Reference Example L compound 37-3: N-[4-[2-ethyl-4-(3-fluorophenyl)-1,3-thiazol-5-yl]-2-pyridyl]phenylacetamide
   m.p.: 113-114°C.
Reference Example L compound 37-4: N-[4-[2-ethyl-4-(3-ethylphenyl)-1,3-thiazol-5-yl]-2-pyridyl]phenylacetamide
   m.p. : 155-156°C.
Reference Example L compound 37-5: N-[4-[2-ethyl-4-[3-(1-methylethyl)phenyl]-1,3-thiazol-5-yl]-2-pyridyl]phenylacetamide
   m.p.: 112-113°C.

### Reference Example L 38

### N-[4-[2-ethyl-4-(3-methylphenyl)-1,3-thiazol-5-yl]-2-pyridyl]-2-thiophenecarboxamide

To a solution of 4-[2-ethyl-4-(3-methylphenyl)-1,3-thiazol-5-yl]-2-pyridylamine (0.50 g, 1.7 mmol) in tetrahydrofuran (10 mL) was added 2-thiophenecarbonyl chloride (0.36 mL, 3.4 mmol), and triethylamine (0.52 mL, 3.7 mmol) was added to the obtained mixture. The reaction mixture was stirred at room temperature for 10 min. Saturated aqueous sodium hydrogen carbonate solution was added and the mixture was extracted with ethyl acetate. The extract was washed with saturated brine, dried and concentrated. The residue was dissolved in conc. hydrochloric acid (5 mL) and the mixture was stirred at 40°C for 14 hrs. The reaction mixture was neutralized with aqueous sodium hydroxide solution and extracted with ethyl acetate. The extract was dried and concentrated. The residue was purified by silica gel column chromatography (hexane-ethyl acetate = 4 : 1) and crystallized from isopropyl ether-hexane to give the title compound (0.56 g, yield 82%).
m.p.: 102-103°C.

### Reference Example L 39

In accordance with Reference Example L 38 and using 3-thiophenecarbonyl chloride, 4-methoxybenzoyl chloride, 4-methylbenzoyl chloride, 3-fluorobenzoyl chloride, 4-fluorobenzoyl chloride and 3,5-dichlorobenzoyl chloride instead of 2-thiophenecarbonyl chloride, the following Reference Example L compounds 39-1 to 39-6 were synthesized respectively.
Reference Example L compound 39-1: N-[4-[2-ethyl-4-(3-methylphenyl)-1,3-thiazol-5-yl]-2-pyridyl]-3-thiophenecarboxamide
   m.p.: 99-101°C.
Reference Example L compound 39-2: N-[4-[2-ethyl-4-(3-methylphenyl)-1,3-thiazol-5-yl]-2-pyridyl]-4-methoxybenzamide
   m.p.: 124-125°C.
Reference Example L compound 39-3: N-[4-[2-ethyl-4-(3-methylphenyl)-1,3-thiazol-5-yl]-2-pyridyl]-4-methylbenzamide
   m.p.: 105-106°C.
Reference Example L compound 39-4: N-[4-[2-ethyl-4-(3-methylphenyl)-1,3-thiazol-5-yl]-2-pyridyl]-3-fluorobenzamide
   m.p.: 101-102°C.
Reference Example L compound 39-5: N-[4-[2-ethyl-4-(3-methylphenyl)-1,3-thiazol-5-yl]-2-pyridyl]-4-fluorobenzamide
   m.p.: 110-111°C.
Reference Example L compound 39-6: 3,5-dichloro-N-[4-[2-ethyl-4-(3-methylphenyl)-1,3-thiazol-5-yl]-2-pyridyl]benzamide
   m.p.: 77-78°C.

### Reference Example L 40

In accordance with Reference Example L 38 and using benzoyl chloride instead of 2-thiophenecarbonyl chloride, and 4-[4-(4-chlorophenyl)-2-ethyl-1,3-thiazol-5-yl]-2-pyridylamine and 4-[2-ethyl-4-(4-fluoro-3-methylphenyl)-1,3-thiazol-5-yl]-2-pyridylamine instead of 4-[2-ethyl-4 -(3-methylphenyl)-1,3-thiazol-5-yl]-2-pyridylamine, the following Reference Example L compounds 40-1 and 40-2 were synthesized respectively.
Reference Example L compound 40-1: N-[4-[4-(4-chlorophenyl)-2-ethyl-1,3-thiazol-5-yl]-2-pyridyl]benzamide
   m.p.: 138-139°C.
Reference Example L compound 40-2: N-[4-[2-ethyl-4-(4-fluoro-3-methylphenyl)-1,3-thiazol-5-yl]-2-pyridyl]benzamide
   m.p.: 108-109°C.

### Reference Example L 41

### N-[5-(2-benzylamino-4-pyridyl)-4-(3-methylphenyl)-1,3-thiazol-2-yl]nicotinamide

To a solution of [5-(2-benzylamino-4-pyridyl)-4-(3-methylphenyl)-1,3-thiazol-2-yl]amine (0.52 g, 1.4 mmol) and 4-dimethylaminopyridine (0.051g, 0.42 mmol) in N,N-dimethylacetamide (10 mL) was added nicotinoyl chloride hydrochloride (0.37 g, 2.1 mmol), and the mixture was stirred at 80°C for 14 hrs. Into the reaction mixture was poured aqueous sodium hydrogen carbonate solution and the mixture was extracted with ethyl acetate. The extract was washed with saturated brine, dried and concentrated. The resulting crystals were crystallized from isopropyl ether to give the title compound (0.28 g, yield 59%).
m.p.: 220-222°C.

### Reference Example L 42

### 6-chloro-N-[5-(2-cyclopentylamino-4-pyridyl)-4-(3-methylphenyl)-1,3-thiazol-2-yl]nicotinamide

To a solution of [5-(2-cyclopentylamino-4-pyridyl)-4-(3-methylphenyl)-1,3-thiazol-2-yl]amine (0.50 g, 1.4 mmol) and 4-dimethylaminopyridine (0.052 g, 0.43 mmol) in N,N-dimethylacetamide (10 mL) was added 6-chloronicotinoyl chloride hydrochloride (0.46 g, 2.1 mmol), and the mixture was stirred at 80°C for 14 hrs. Into the reaction mixture was poured aqueous sodium hydrogen carbonate solution and the mixture was extracted with ethyl acetate. The extract was washed with saturated brine, dried and concentrated. The residue was purified by silica gel column chromatography (hexane-ethyl acetate = 4 : 1) and the resulting crystals were recrystallized from ethanol to give the title compound (0.30 g, yield 42%).
m.p.: 211-212°C.

### Reference Example L 43

In accordance with Reference Example L 42 and using [5-(2-cyclohexylamino-4-pyridyl)-4-(3-methylphenyl)-1,3-thiazol-2-yl]amine instead of [5-(2-cyclopentylamino-4-pyridyl)-4-(3-methylphenyl)-1,3-thiazol-2-yl]amine, the following Reference Example L compound 43 was synthesized.
Reference Example L compound 43: 6-chloro-N-[5-(2-cyclohexylamino-4-pyridyl)-4-(3-methylphenyl)-1,3-thiazol-2-yl]nicotinamide
m.p.: 255-256°C.

### Reference Example L 44

### N-[4-[4-(3-methylphenyl)-2-(1-methylpiperidin-4-yl)-1,3-thiazol-5-yl]-2-pyridyl]benzamide

A solution of N-[4-[1-bromo-2-(3-methylphenyl)-2-oxoethyl]-2-pyridyl]benzamide hydrobromide (0.60 g, 1.2 mmol) and 1-methylpiperidine-4-carbothioamide (0.19 g, 1.18 mmol) in N,N-dimethylformamide (20 mL) was stirred at room temperature for 14 hrs. Into the reaction mixture was poured aqueous sodium hydrogen carbonate solution and the mixture was extracted with ethyl acetate. The extract was washed with saturated aqueous sodium hydrogen carbonate solution, dried and concentrated. The residue was purified by column chromatography (packing: Chromatorex NH DM1020 (product name, manufactured by Fuji Silysia Chemical Ltd.), hexane-ethyl acetate = 2 : 1) and the resulting crystals were recrystallized from ethyl acetate to give the title compound (0.26 g, yield 46%).
m.p.: 151-152°C.

### Reference Example L 45

In accordance with Reference Example L 44 and using N-[4-[1-bromo-2-(3-chlorophenyl)-2-oxoethyl]-2-pyridyl]benzamide hydrobromide instead of N-[4-[1-bromo-2-(3-methylphenyl)-2-oxoethyl]-2-pyridyl]benzamide hydrobromide, the following Reference Example L compound 45 was synthesized. Reference Example L compound 45: N-[4-[4-(3-chlorophenyl)-2-(1-methylpiperidin-4-yl)-1,3-thiazol-5-yl]-2-pyridyl]benzamide m.p.: 125-127°C.

The compounds produced in Reference Examples L 32-45 are shown in Tables 78 and 79.

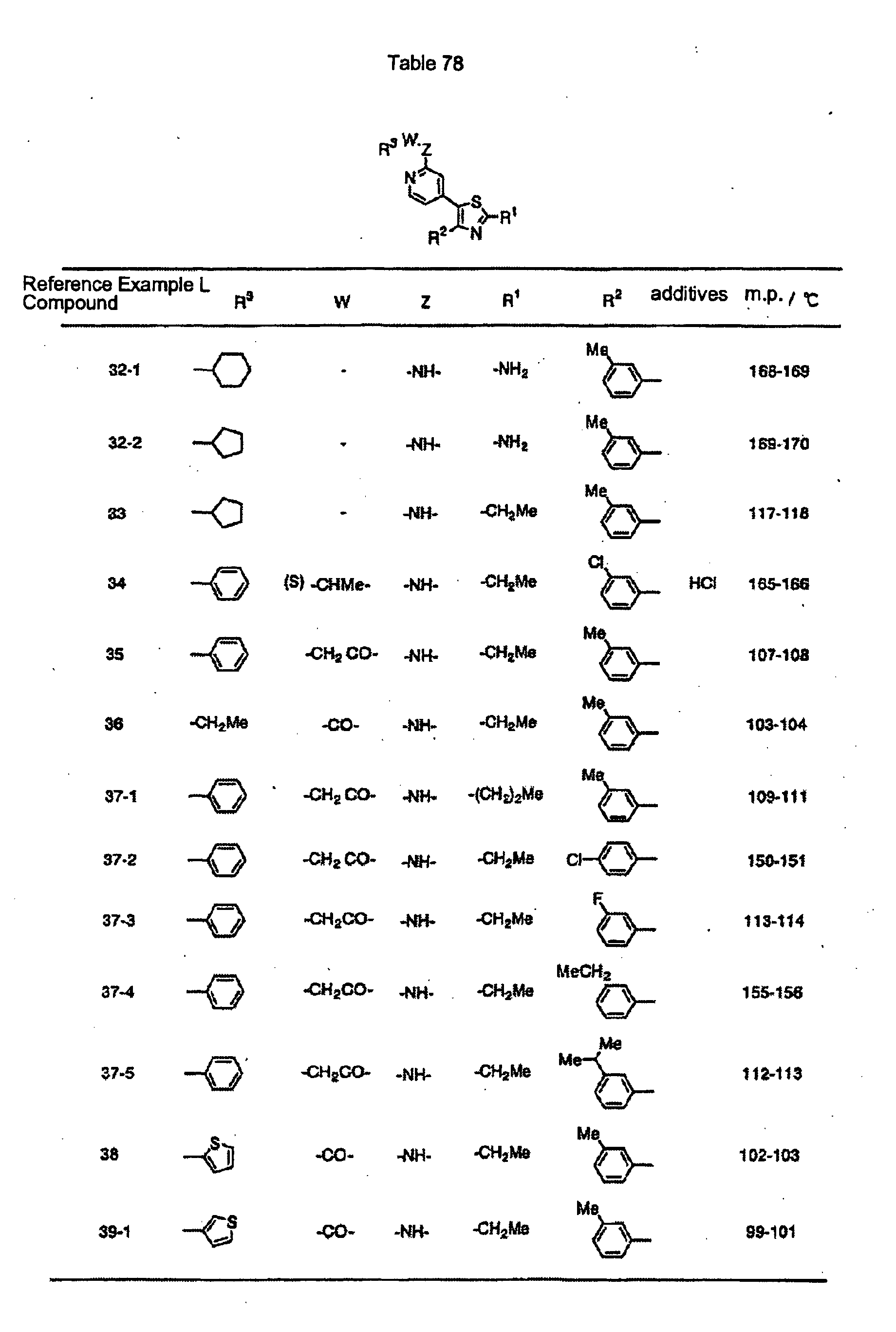

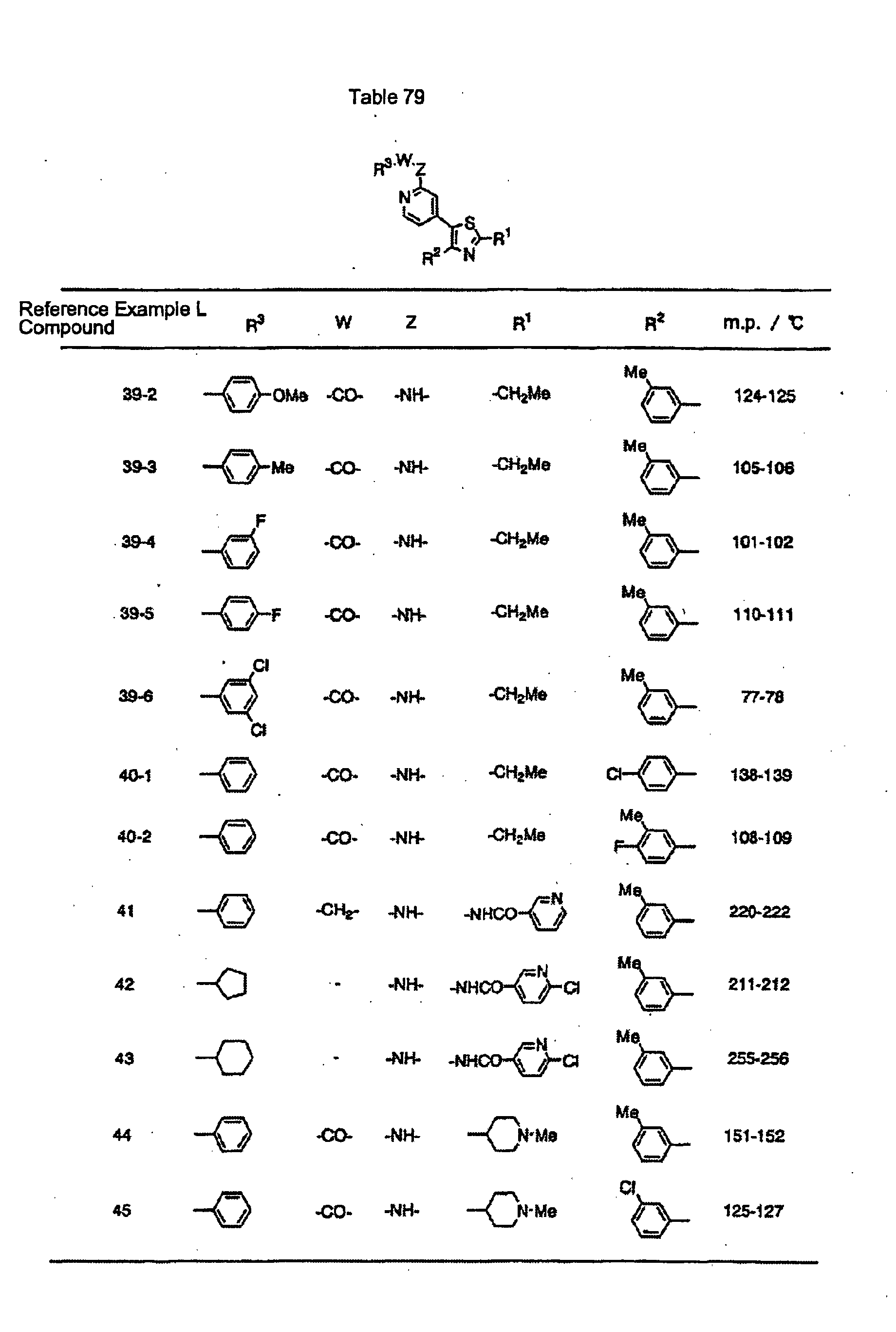

### Reference Example L 46

In accordance with Reference Example L 7 and using benzoyl chloride, 3-methoxybenzoyl chloride, 4-methylbenzoyl chloride, 3-bromobenzoyl chloride, 2-thiophenecarbonyl chloride and 4-fluorobenzoyl chloride instead of 4-chlorobenzoyl chloride, the following Reference Example L compounds 46-1 to 46-6 were synthesized respectively.
Reference Example L compound 46-1: N-benzoylpropylenimine
   oil
   ¹H-NMR (CDCl₃)δ: 1.40 (3H, d, J= 6.0 Hz), 2.15 (1H, d, J=3.2 Hz), 2.52-2.67 (2H, m), 7.40-7.61 (3H, m), 7.98-8.07 (2H, m).
Reference Example L compound 46-2: N-(3-methoxybenzoyl)propylenimine
   oil
   ¹H-NMR (CDCl₃)δ: 1.40 (3H, d, J= 5.9 Hz), 2.14 (1H, d, J=2.9 Hz), 2.52-2.65 (2H, m), 3.86 (3H, s), 7.10 (1H, ddd, J=1.1, 2.6, 8.4 Hz), 7.37 (1H, dd, J=8.4, 7.3 Hz), 7.55 (1H, dd, J=1.5, 2.6 Hz), 7.63 (1H, ddd, J=1.1, 1.5, 7.3 Hz).
Reference Example L compound 46-3: N-(4-methylbenzoyl)propylenimine
   oil
   ¹H-NMR (CDCl₃)δ: 1.39 (3H, d, J= 5.5 Hz), 2.12 (1H, d, J= 2.9 Hz), 2.42 (3H, s), 2.50-2.62 (2H, m), 7.25 (2H, d, J= 8.1 Hz), 7.92 (2H, d, J= 8.1 Hz).
Reference Example L compound 46-4: N-(3-bromobenzoyl)propylenimine
   oil
   ¹H-NMR(CDCl₃)δ: 1.40 (3H, d, J= 5.2Hz), 2.16-2.18 (1H, m), 2.53-2.65 (2H, m), 7.34 (1H, t, J= 7.9Hz), 7.65-7.71 (1H, m), 7.95 (1H, d, J= 7.9Hz), 8.16 (1H, t, J= 1.8Hz).
Reference Example L compound 46-5: N-(2-thiophenecarbonyl)propylenimine
   oil
   ¹H-NMR(CDCl₃)δ: 1.43 (3H, d, J= 5.2Hz), 2. 14 (1H, d, J= 3. 6Hz) , 2.56-2.72 (2H, m), 7.08-7.16 (1H, m), 7.53-7.60 (1H, m), 7.75-7.81 (1H, m).
Reference Example L compound 46-6: N-(4-fluorobenzoyl)propylenimine
   oil
   ¹H-NMR(CDCl₃)δ: 1.39 (3H, d, J= 5.2Hz), 2.14-2.15 (1H, m), 2.52-2.63 (2H, m), 7.08-7.19 (2H, m), 8.00-8.10 (2H, m).

### Reference Example L 47

In accordance with Reference Example L 11 and using N-(4-fluorobenzoyl)propylenimine instead of N-(3-methylbenzoyl)propylenimine, the following Reference Example L compound 47 was synthesized.
Reference Example L compound 47: 1-(4-fluorophenyl)-2-(2-fluoro-4-pyridyl)ethanone
m.p.: 100-101°C.

### Reference Example L 48

In accordance with Reference Example L 13 and using N-benzoylpropylenimine, N-(4-fluorobenzoyl)propylenimine, N-(3-bromobenzoyl)propylenimine, N-(2-thiophenecarbonyl)propylenimine, N-(3-methoxybenzoyl)propylenimine and N-(4-methylbenzoyl)propylenimine instead of N-(3-methylbenzoyl)propylenimine, the following Reference Example L compounds 48-1 to 48-6 were synthesized respectively.
Reference Example L compound 48-1: 2-(2-tert-butoxycarbonylamino-4-pyridyl)-1-phenylethanone
   m.p.: 162-163°C.
Reference Example L compound 48-2: 2-(2-tert-butoxycarbonylamino-4-pyridyl)-1-(4-fluorophenyl)ethanone
   m.p.: 139-141°C.
Reference Example L compound 48-3: 1-(3-bromophenyl)-2-(2-tert-butoxycarbonylamino-4-pyridyl)ethanone
   m.p. : 132-133°C.
Reference Example L compound 48-4: 2-(2-tert-butoxycarbonylamino-4-pyridyl)-1-(2-thienyl)ethanone
   m.p.: 161-162°C.
Reference Example L compound 48-5: 2-(2-tert-butoxycarbonylamino-4-pyridyl)-1-(3-methoxyphenyl)ethanone
   m.p.: 99-100°C.
Reference Example L compound 48-6: 2-(2-tert-butoxycarbonylamino-4-pyridyl)-1-(4-methylphenyl)ethanone
   m.p.: 137-138°C.

### Reference Example L 49

In accordance with Reference Example L 19 and using 1-(4-fluorophenyl)-2-(2-fluoro-4-pyridyl)ethanone, 1-(3-bromophenyl)-2-(2-tert-butoxycarbonylamino-4-pyridyl)ethanone, 2-(2-tert-butoxycarbonylamino-4-pyridyl)-1-(4-fluorophenyl)ethanone, 2-(2-tert-butoxycarbonylamino-4-pyridyl)-1-(4-methylphenyl)ethanone, 2-(2-tert-butoxycarbonylamino-4-pyridyl)-1-phenylethanone, 2-(2-tert-butoxycarbonylamino-4-pyridyl)-1-(2-thienyl)ethanone and 2-(2-tert-butoxycarbonylamino-4-pyridyl)-1-(3-methoxyphenyl)ethanone instead of 2-(2-tert-butoxycarbonylamino-4-pyridyl)-1-(3-methylphenyl)ethanone, the following Reference Example L compounds 49-1 to 49-7 were synthesized respectively.
Reference Example L compound 49-1: 2-bromo-1-(4-fluorophenyl)-2-(2-fluoro-4-pyridyl)ethanone hydrobromide
   amorphous powder
   ¹H-NMR(DMSO-d₆)δ: 7.16 (1H, s), 7.37-7.54 (4H, m), 8.11-8.24 (2H, m), 8.30 (1H, d, J= 5.0Hz).
Reference Example L compound 49-2: 2-(2-amino-4-pyridyl)-2-bromo-1-(3-bromophenyl)ethanone hydrobromide
   Used for the next reaction without purification.
Reference Example L compound 49-3: 2-(2-amino-4-pyridyl)-2-bromo-1-(4-fluorophenyl)ethanone hydrobromide
   m.p.: 171-172°C.
Reference Example L compound 49-4: 2-(2-amino-4-pyridyl)-2-bromo-1-(4-methylphenyl)ethanone hydrobromide
   m.p.: 200-201°C.
Reference Example L compound 49-5: 2-(2-amino-4-pyridyl)-2-bromo-1-phenylethanone hydrobromide
   m.p.: 155-156°C.
Reference Example L compound 49-6: 2-(2-amino-4-pyridyl)-2-bromo-1-(2-thienyl)ethanone hydrobromide
   amorphous powder
   ¹H-NMR(DMSO-d₆)δ: 6.96-7.09 (2H, m), 7.24 (1H, s), 7.32-7.43 (1H, m), 7.98 (1H, d, J= 6.6 Hz), 8.12-8.36 (2H, m).
Reference Example L compound 49-7: 2-(2-amino-4-pyridyl)-2-bromo-1-(3-methoxyphenyl)ethanone hydrobromide
   m.p. : 205-206°C.

### Reference Example L 50

In accordance with Reference Example L 23 and using 4-(methylthio)benzonitrile, valeronitrile and ethylcyanoacetate instead of butyronitrile, the following Reference Example L compounds 50-1 to 50-3 were synthesized respectively.
Reference Example L compound 50-1: 4-(methylthio)thiobenzamide m.p.: 176-178°C.
Reference Example L compound 50-2: thiovaleramide
   oil
   ¹H-NMR(CDCl₃)δ: 0.94 (3H, t, J= 7.3Hz) , 1.31-1.49 (2H, m), 1.68-1.83 (2H, m), 2.67 (2H, t, J= 7.7Hz), 6.92 (1H, br s), 7.73 (1H, br s).
Reference Example L compound 50-3: ethyl 3-amino-3-thioxopropanate
   oil
   ¹H-NMR(CDCl₃)δ: 1.31 (3H, t, J= 7.1Hz), 3.85 (2H, s), 4.22 (2H, q, J= 7.1Hz), 7.74 (1H, br s), 8.92 (1H, br s).

### Reference Example L 51

### ethyl 2-amino-2-thioxoacetate

To a solution of ethyl oxamate (3.21 g, 27.4 mmol) in anhydrous tetrahydrofuran (100 mL) was added Lawesson's reagent (6.10 g, 15.1 mmol), and the mixture was heated under reflux for 2 hrs. The mixture was cooled to room temperature, and saturated aqueous sodium hydrogen carbonate solution was added to the reaction mixture. The mixture was extracted with ethyl acetate. The extract was dried and the solvent was evaporated. The residue was purified by silica gel column chromatography (hexane-ethyl acetate = 4 : 1-2 : 1) and crystallized from isopropyl ether to give the title compound (2.92 g, yield 80%).
m.p.: 60-62°C.

### Reference Example L 52

In accordance with Reference Example L 25 and using 2-bromo-1-(4-fluorophenyl)-2-(2-fluoro-4-pyridyl)ethanone hydrobromide instead of 2-bromo-2-(2-fluoro-4-pyridyl)-1-(3-methylphenyl)ethanone hydrobromide, the following Reference Example L compound 52 was synthesized.
Reference Example L compound 52: [4-(4-fluorophenyl)-5-(2-fluoro-4-pyridyl)-1,3-thiazol-2-yl]amine
m.p.: 243-245°C.

### Reference Example L 53

In accordance with Reference Example L 25 and using N-methylthiourea instead of thiourea, the following Reference Example L compound 53 was synthesized.
Reference Example L compound 53: N-methyl-[5-(2-fluoro-4-pyridyl)-4-(3-methylphenyl)-1,3-thiazol-2-yl]amine
m.p.: 186-187°C.

### Reference Example L 54

In accordance with Reference Example L 28 and using thiovaleramide, ethyl 2-amino-2-thioxoacetate and ethyl 3-amino-3-thioxopropanate instead of thiopropionamide, the following Reference Example L compounds 54-1 to 54-3 were synthesized respectively.
Reference Example L compound 54-1: 4-[2-butyl-4-(3-methylphenyl)-1,3-thiazol-5-yl]-2-pyridylamine
   oil
   ¹H-NMR(CDCl₃)δ: 0.98 (3H, t, J= 7.3Hz), 1.39-1.59 (2H, m), 1.76-1.92 (2H, m), 2.34 (3H, s), 3.04 (2H, t, J= 7.4Hz), 4.14 (2H, br s), 6.44 (1H, s), 6.56 (1H, dd, J= 1.5, 5.4Hz), 7.09-7.26 (3H, m), 7.41 (1H, s), 7.96 (1H, d, J= 5.4Hz).
Reference Example L compound 54-2: ethyl [5-(2-amino-4-pyridyl)-4-(3-methylphenyl)-1,3-thiazol-2-yl]carboxylate
   m.p.: 147-148°C.
Reference Example L compound 54-3: ethyl [5-(2-amino-4-pyridyl)-4-(3-methylphenyl)-1,3-thiazol-2-yl]acetate
   m.p.: 128-129°C.

### Reference Example L 55

In accordance with Reference Example L 28 and using 2-(2-amino-4-pyridyl)-2-bromo-1-(3-chlorophenyl)ethanone hydrobromide, 2-(2-amino-4-pyridyl)-2-bromo-1-(3-bromophenyl)ethanone hydrobromide, 2-(2-amino-4-pyridyl)-2-bromo-1-(4-fluorophenyl)ethanone hydrobromide, 2-(2-amino-4-pyridyl)-2-bromo-1-(4-methylphenyl)ethanone hydrobromide, 2-(2-amino-4-pyridyl)-2-bromo-1-phenylethanone hydrobromide, 2-(2-amino-4-pyridyl)-2-bromo-1-(2-thienyl)ethanone hydrobromide and 2-(2-amino-4-pyridyl)-2-bromo-1-(3-methoxyphenyl)ethanone hydrobromide instead of 2-(2-amino-4-pyridyl)-2-bromo-1-(3-methylphenyl)ethanone hydrobromide, the following Reference Example L compounds 55-1 to 55-7 were synthesized respectively.
Reference Example L compound 55-1: 4-[4-(3-chlorophenyl)-2-ethyl-1,3-thiazol-5-yl]-2-pyridylamine
   m.p.: 132-133°C.
Reference Example L compound 55-2: 4-[4-(3-bromophenyl)-2-ethyl-1,3-thiazol-5-yl]-2-pyridylamine
   m.p.: 132-134°C.
Reference Example L compound 55-3: 4-[2-ethyl-4-(4-fluorophenyl)-1,3-thiazol-5-yl]-2-pyridylamine
   m.p.: 140-141°C.
Reference Example L compound 55-4: 4-[2-ethyl-4-(4-methylphenyl)-1,3-thiazol-5-yl]-2-pyridylamine
   m.p.: 126-127°C.
Reference Example L compound 55-5: 4-(2-ethyl-4-phenyl-1,3-thiazol-5-yl)-2-pyridylamine
   m.p.: 158-159°C.
Reference Example L compound 55-6: 4-[2-ethyl-4-(2-thienyl)-1,3-thiazol-5-yl]-2-pyridylamine
   m.p.: 159-160°C.
Reference Example L compound 55-7: 4-[2-ethyl-4-(3-methoxyphenyl)-1,3-thiazol-5-yl]-2-pyridylamine m.p.: 130-131°C.

### Reference Example L 56

In accordance with Reference Example L 29 and using 2-(2-amino-4-pyridyl)-2-bromo-1-(3-chlorophenyl)ethanone hydrobromide instead of 2-(2-amino-4-pyridyl)-2-bromo-1-(3-methylphenyl)ethanone hydrobromide, the following Reference Example L compound 56 was synthesized.
Reference Example L compound 56: 4-[4-(3-chlorophenyl)-2-propyl-1,3-thiazol-5-yl]-2-pyridylamine
m.p.: 99-100°C.

### Reference Example L 57

In accordance with Reference Example L 56 and using 4-(methylthio)thiobenzamide and ethyl 3-amino-3-thioxopropanate instead of thiobutylamide, the following Reference Example L compounds 57-1 and 57-2 were synthesized respectively.
Reference Example L compound 57-1: 4-[4-(3-chlorophenyl)-2-[4-(methylthio)phenyl]-1,3-thiazol-5-yl]-2-pyridylamine
   m.p. : 183-184°C.
Reference Example L compound 57-2: ethyl [5-(2-amino-4-pyridyl)-4-(3-chlorophenyl)-1,3-thiazol-2-yl]acetate
   m.p.: 154-155°C.

### Reference Example L 58

### [5-(2-amino-4-pyridyl)-4-(3-methylphenyl)-1,3-thiazol-2-yl]acetic acid

To a suspension of ethyl [5-(2-amino-4-pyridyl)-4-(3-methylphenyl)-1,3-thiazol-2-yl]acetate (7.00 g, 19.8 mmol) in ethanol (40 mL) was added 1N aqueous sodium hydroxide solution (40 mL), and the mixture was stirred at room temperature for 2 hrs. The reaction mixture was neutralized with 2N hydrochloric acid (20 mL) and the resulting solid was collected by filtration. The crude product was washed with water and dried to give the title compound (6.10 g, yield 95%).
m.p.: 132-133°C.

### Reference Example L 59

In accordance with Reference Example L 58 and using ethyl [5-(2-amino-4-pyridyl)-4-(3-methylphenyl)-1,3-thiazol-2-yl]carboxylate and ethyl [5-(2-amino-4-pyridyl)-4-(3-chlorophenyl)-1,3-thiazol-2-yl]acetate instead of ethyl [5-(2-amino-4-pyridyl)-4-(3-methylphenyl)-1,3-thiazol-2-yl]acetate, the following Reference Example L compounds 59-1 and 59-2 were synthesized respectively.
Reference Example L compound 59-1: 5-(2-amino-4-pyridyl)-4-(3-methylphenyl)-1,3-thiazole-2-carboxylic acid
   m.p.: 135-136°C.
Reference Example L compound 59-2: [5-(2-amino-4-pyridyl)-4-(3-chlorophenyl)-1,3-thiazol-2-yl]acetic acid
   Used for the next reaction without isolation.

### Reference Example L 60

### 4-[2-methyl-4-(3-methylphenyl)-1,3-thiazol-5-yl]-2-pyridylamine

[5-(2-Amino-4-pyridyl)-4-(3-methylphenyl)-1,3-thiazol-2-yl]acetic acid (5.0 g, 15 mmol) was stirred at 150°C for 15 min and cooled to room temperature. The crude product was purified by silica gel column chromatography (ethyl acetate) and subjected to recrystallization from ethyl acetate to give the title compound (4.0 g, yield 93%).
m.p.: 152-153°C.

### Reference Example L 61

In accordance with Reference Example L 60 and using 5-(2-amino-4-pyridyl)-4-(3-methylphenyl)-1,3-thiazole-2-carboxylic acid and [5-(2-amino-4-pyridyl)-4-(3-chlorophenyl)-1,3-thiazol-2-yl]acetic acid instead of [5-(2-amino-4-pyridyl)-4-(3-methylphenyl)-1,3-thiazol-2-yl]acetic acid, the following Reference Example L compounds 61-1 and 61-2 were synthesized respectively.
Reference Example L compound 61-1: 4-[4-(3-methylphenyl)-1,3-thiazol-5-yl]-2-pyridylamine
   m.p.: 91-92°C.
Reference Example L compound 61-2: 4-[4-(3-chlorophenyl)-2-methyl-1,3-thiazol-5-yl]-2-pyridylamine
   m.p.: 142-143°C.

### Reference Example L 62

In accordance with Reference Example L 33 and using N-methyl-[5-(2-fluoro-4-pyridyl)-4-(3-methylphenyl)-1,3-thiazol-2-yl]amine instead of 2-ethyl-5-(2-fluoro-4-pyridyl)-4-(3-methylphenyl)-1,3-thiazole, the following Reference Example L compound 62 was synthesized.
Reference Example L compound 62: N-methyl-[5-(2-cyclopentylamino-4-pyridyl)-4-(3-methylphenyl)-1,3-thiazol-2-yl]amine
m.p.: 170-172°C.

### Reference Example L 63

In accordance with Reference Example L 62 and using cyclohexylamine instead of cyclopentylamine, the following Reference Example L compound 63 was synthesized.
Reference Example L compound 63: N-methyl-[5-(2-cyclohexylamino-4-pyridyl)-4-(3-methylphenyl)-1,3-thiazol-2-yl]amine
m.p.: 211-212°C.

### Reference Example L 64

In accordance with Reference Example L 63 and using [4-(4-fluorophenyl)-5-(2-fluoro-4-pyridyl)-1,3-thiazol-2-yl]amine, 2-ethyl-5-(2-fluoro-4-pyridyl)-4-(3-methylphenyl)-1,3-thiazole and 4-(3-chlorophenyl)-2-ethyl-5-(2-fluoro-4-pyridyl)-1,3-thiazole instead of N-methyl-[5-(2-fluoro-4-pyridyl)-4-(3-methylphenyl)-1,3-thiazol-2-yl]amine, the following Reference Example L compounds 64-1 to 64-3 were synthesized respectively.
Reference Example L compound 64-1: [5-(2-cyclohexylamino-4-pyridyl)-4-(4-fluorophenyl)-1,3-thiazol-2-yl]amine
   m.p.: 194-195°C.
Reference Example L compound 64-2: N-cyclohexyl-4-[2-ethyl-4-(3-methylphenyl)-1,3-thiazol-5-yl]-2-pyridylamine
   m.p.: 110-112°C.
Reference Example L compound 64-3: N-cyclohexyl-4-[4-(3-chlorophenyl)-2-ethyl-1,3-thiazol-5-yl]-2-pyridylamine
   m.p.: 106-107°C.

### Reference Example L 65

In accordance with Reference Example L 41 and using N-cyclopentyl-4-[2-ethyl-4-(3-methylphenyl)-1,3-thiazol-5-yl]-2-pyridylamine, N-cyclohexyl-4-[2-ethyl-4-(3-methylphenyl)-1,3-thiazol-5-yl]-2-pyridylamine, N-methyl-[5-(2-cyclopentylamino-4-pyridyl)-4-(3-methylphenyl)-1,3-thiazol-2-yl]amine and N-methyl-[5-(2-cyclohexylamino-4-pyridyl)-4-(3-methylphenyl)-1,3-thiazol-2-yl]amine instead of [5-(2-benzylamino-4-pyridyl)-4-(3-methylphenyl)-1,3-thiazol-2-yl]amine, the following Reference Example L compounds 65-1 to 65-4 were synthesized respectively.
Reference Example L compound 65-1: N-[5-(2-cyclopentylamino-4-pyridyl)-4-(3-methylphenyl)-1,3-thiazol-2-yl]nicotinamide
   m.p.: 201-203°C.
Reference Example L compound 65-2: N-[5-(2-cyclohexylamino-4-pyridyl)-4-(3-methylphenyl)-1,3-thiazol-2-yl]nicotinamide
   m.p.: 215-216°C.
Reference Example L compound 65-3: N-methyl-N-[5-(2-cyclopentylamino-4-pyridyl)-4-(3-methylphenyl)-1,3-thiazol-2-yl]nicotinamide
   m.p.: 135-136°C.
Reference Example L compound 65-4: N-methyl-N-[5-(2-cyclohexylamino-4-pyridyl)-4-(3-methylphenyl)-1,3-thiazol-2-yl]nicotinamide
   m.p.: 148-149°C.

### Reference Example L 66

In accordance with Reference Example L 42 and using 6-methylnicotinoyl chloride hydrochloride and 6-methoxynicotinoyl chloride hydrochloride instead of 6-chloronicotinoyl chloride hydrochloride, the following Reference Example L compounds 66-1 and 66-2 were synthesized respectively.
Reference Example L compound 66-1: N-[5-(2-cyclopentylamino-4-pyridyl)-4-(3-methylphenyl)-1,3-thiazol-2-yl]-6-methylnicotinamide
   m.p.: 213-214°C.
Reference Example L compound 66-2: N-[5-(2-cyclopentylamino-4-pyridyl)-4-(3-methylphenyl)-1,3-thiazol-2-yl]-6-methoxynicotinamide
   m.p.: 219-221°C.

### Reference Example L 67

In accordance with Reference Example L 66 and using [5-(2-cyclohexylamino-4-pyridyl)-4-(3-methylphenyl)-1,3-thiazol-2-yl]amine and N-methyl-[5-(2-cyclopentylamino-4-pyridyl)-4-(3-methylphenyl)-1,3-thiazol-2-yl]amine instead of [5-(2-cyclopentylamino-4-pyridyl)-4-(3-methylphenyl)-1,3-thiazol-2-yl]amine, the following Reference Example L compounds 67-1 to 67-3 were synthesized respectively.
Reference Example L compound 67-1: N-[5-(2-cyclohexylamino-4-pyridyl)-4-(3-methylphenyl)-1,3-thiazol-2-yl]-6-methylnicotinamide
   m.p.: 242-243°C.
Reference Example L compound 67-2: N-[5-(2-cyclopentylamino-4-pyridyl)-4-(3-methylphenyl)-1,3-thiazol-2-yl]-N,6-dimethylnicotinamide
   m.p.: 176-177°C.
Reference Example L compound 67-3: N-[5-(2-cyclohexylamino-4-pyridyl)-4-(3-methylphenyl)-1,3-thiazol-2-yl]-6-methoxynicotinamide
   m.p.: 191-192°C.

### Reference Example L 68

In accordance with Reference Example L 35 and using 4-[4-(3-bromophenyl)-2-ethyl-1,3-thiazol-5-yl]-2-pyridylamine, 4-[4-(3-chlorophenyl)-2-ethyl-1,3-thiazol-5-yl]-2-pyridylamine, 4-[4-(3-chlorophenyl)-2-methyl-1,3-thiazol-5-yl]-2-pyridylamine, 4-[2-butyl-4-(3-methylphenyl)-1,3-thiazol-5-yl]-2-pyridylamine, 4-[2-methyl-4-(3-methylphenyl)-1,3-thiazol-5-yl]-2-pyridylamine, 4-[4-(3-methylphenyl)-1,3-thiazol-5-yl]-2-pyridylamine, 4-[2-ethyl-4-(4-fluorophenyl)-1,3-thiazol-5-yl]-2-pyridylamine, 4-[2-ethyl-4-(4-methylphenyl)-1,3-thiazol-5-yl]-2-pyridylamine, 4-(2-ethyl-4-phenyl-1,3-thiazol-5-yl)-2-pyridylamine and 4-[2-ethyl-4-(3-methoxyphenyl)-1,3-thiazol-5-yl]-2-pyridylamine instead of 4-[2-ethyl-4-(3-methylphenyl)-1,3-thiazol-5-yl]-2-pyridylamine, the following Reference Example L compounds 68-1 to 68-10 were synthesized respectively.
Reference Example L compound 68-1: N-[4-[4-(3-bromophenyl)-2-ethyl-1,3-thiazol-5-yl]-2-pyridyl]phenylacetamide
   m.p.: 97-99°C.
Reference Example L compound 68-2: N-[4-[4-(3-chlorophenyl)-2-ethyl-1,3-thiazol-5-yl]-2-pyridyl]phenylacetamide
   m.p.: 111-112°C.
Reference Example L compound 68-3: N-[4-[4-(3-chlorophenyl)-2-methyl-1,3-thiazol-5-yl]-2-pyridyl]phenylacetamide
   m.p.: 99-101°C.
Reference Example L compound 68-4: N-[4-[2-butyl-4-(3-methylphenyl]-1,3-thiazol-5-yl]-2-pyridyl]phenylacetamide
   m.p.: 92-93°C.
Reference Example L compound 68-5: N-[4-[2-methyl-4-(3-methylphenyl)-1,3-thiazol-5-yl]-2-pyridyl]phenylacetamide
   m.p.: 114-115°C.
Reference Example L compound 68-6: N-[4-[4-(3-methylphenyl)-1,3-thiazol-5-yl]-2-pyridyl]phenylacetamide
   m.p.: 135-136°C.
Reference Example L compound 68-7: N-[4-[2-ethyl-4-(4-fluorophenyl)-1,3-thiazol-5-yl]-2-pyridyl]phenylacetamide
   m.p.: 178-179°C.
Reference Example L compound 68-8: N-[4-[2-ethyl-4-(4-methylphenyl)-1,3-thiazol-5-yl]-2-pyridyl]phenylacetamide
   m.p.: 128-129°C.
Reference Example L compound 68-9: N-[4-(2-ethyl-4-phenyl-1,3-thiazol-5-yl)-2-pyridyl]phenylacetamide
   m.p.: 162-163°C.
Reference Example L compound 68-10: N-[4-[2-ethyl-4-(3-methoxyphenyl)-1,3-thiazol-5-yl]-2-pyridyl]phenylacetamide
   m.p.: 128-129°C.

### Reference Example L 69

In accordance with Reference Example L 36 and using 4-[4-(3-chlorophenyl)-2-ethyl-1,3-thiazol-5-yl]-2-pyridylamine, 4-[4-(3-chlorophenyl)-2-methyl-1,3-thiazol-5-yl]-2-pyridylamine, 4-[4-(3-chlorophenyl)-2-propyl-1,3-thiazol-5-yl]-2-pyridylamine, 4-[4-(3-chlorophenyl)-2-[4-(methylthio)phenyl]-1,3-thiazol-5-yl]-2-pyridylamine and 4-[2-ethyl-4-(2-thienyl)-1,3-thiazol-5-yl]-2-pyridylamine instead of 4-[2-ethyl-4-(3-methylphenyl)-1,3-thiazol-5-yl]-2-pyridylamine, the following Reference Example L compounds 69-1 to 69-5 were synthesized respectively.
Reference Example L compound 69-1: N-[4-[4-(3-chlorophenyl)-2-ethyl-1,3-thiazol-5-yl]-2-pyridyl]propionamide
   m.p.: 132-133°C.
Reference Example L compound 69-2: N-[4-[4-(3-chlorophenyl)-2-methyl-1,3-thiazol-5-yl]-2-pyridyl]propionamide
   m.p.: 134-135°C.
Reference Example L compound 69-3: N-[4-[4-(3-chlorophenyl)-2-propyl-1,3-thiazol-5-yl]-2-pyridyl]propionamide
   m.p. : 103-104°C.
Reference Example L compound 69-4: N-[4-[4-(3-chlorophenyl)-2-[4-(methylthio)phenyl]-1,3-thiazol-5-yl]-2-pyridyl]propionamide
   m.p.: 187-188°C.
Reference Example L compound 69-5: N-[4-[2-ethyl-4-(2-thienyl)-1,3-thiazol-5-yl]-2-pyridyl]propionamide
   m.p.: 187-188°C.

### Reference Example L 70

In accordance with Reference Example L 69 and using acetyl chloride, benzoyl chloride and pivaloyl chloride instead of propionyl chloride, the following Reference Example L compounds 70-1 to 70-5 were synthesized respectively.
Reference Example L compound 70-1: N-[4-[4-(3-chlorophenyl)-2-ethyl-1,3-thiazol-5-yl]-2-pyridyl]acetamide
   m.p.: 149-150°C.
Reference Example L compound 70-2: N-[4-[4-(3-chlorophenyl)-2-propyl-1,3-thiazol-5-yl]-2-pyridyl]acetamide
   m.p.: 144-145°C.
Reference Example L compound 70-3: N-[4-[4-(3-chlorophenyl)-2-[4-(methylthio)phenyl]-1,3-thiazol-5-yl]-2-pyridyl]acetamide
   m.p.: 207-208°C.
Reference Example L compound 70-4: N-[4-[2-ethyl-4-(2-thienyl)-1,3-thiazol-5-yl]-2-pyridyl]benzamide
   m.p.: 116-117°C.
Reference Example L compound 70-5: N-[4-[4-(3-chlorophenyl)-2-[4-(methylthio)phenyl]-1,3-thiazol-5-yl]-2-pyridyl]pivalamide
   m.p.: 119-120°C.

### Reference Example L 71

### N-[4-[4-(3-chlorophenyl)-2-(4-methylsulfonylphenyl)-1,3-thiazol-5-yl]-2-pyridyl]acetamide

To a solution of N-[4-[4-(3-chlorophenyl)-2-[4-(methylthio)phenyl]-1,3-thiazol-5-yl]-2-pyridyl]acetamide (0.30 g, 0.66 mmol) in N,N-dimethylformamide (10 mL) was added 70% m-chloroperbenzoic acid (0.34 g, 1.4 mmol) and the mixture was stirred at room temperature for 2 hrs. Into the reaction mixture was poured aqueous sodium hydrogen carbonate solution and the mixture was extracted with ethyl acetate. The extract was washed with water, dried and the solvent was evaporated. The residue was purified by silica gel column chromatography (ethyl acetate) and washed with ethyl acetate-isopropyl ether to give the title compound (0.18 g, yield 55%).
m.p.: 216-217°C.

### Reference Example L 72

In accordance with Reference Example L 71 and using N-[4-[4-(3-chlorophenyl)-2-[4-(methylthio)phenyl]-1,3-thiazol-5-yl]-2-pyridyl]propionamide instead of N-[4-[4-(3-chlorophenyl)-2-[4-(methylthio)phenyl]-1,3-thiazol-5-yl]-2-pyridyl]acetamide, the following Reference Example L compound 72 was synthesized.
Reference Example L compound 72: N-[4-[4-(3-chlorophenyl)-2-(4-methylsulfonylphenyl)-1,3-thiazol-5-yl]-2-pyridyl]propionamide
m.p.: 224-225°C.

### Reference Example L 73

### 4-(3-chlorophenyl)-2-ethyl-5-[2-(phenylmethylthio)-4-pyridyl]-1,3-thiazole

Sodium hydride (0.24 g, 6.0 mmol) was washed twice with hexane and suspended in N,N-dimethylformamide (10 mL). Phenylmethylthiol (0.58 mL, 4.9 mmol) was added to the suspension at 0°C and the mixture was stirred at the same temperature for 1 hr. To the obtained solution was added a solution of 4-(3-chlorophenyl)-2-ethyl-5-(2-fluoro-4-pyridyl)-1,3-thiazole (0.78 g, 2.5 mmol) in N,N-dimethylformamide (6 mL) at the same temperature and the mixture was further stirred at room temperature for 1 hr. To the reaction mixture was added 8N aqueous sodium hydroxide solution (5 mL) and the mixture was extracted with isopropyl ether. The extract was washed with saturated aqueous sodium hydrogen carbonate solution and saturated brine, dried and the solvent was evaporated. The residue was purified by silica gel column chromatography (hexane-ethyl acetate = 9 : 1) to give the title compound (0.56 g, yield 54%).
oil
¹H-NMR(CDCl₃)δ: 1.44 (3H, t, J= 7.6Hz) , 3.07 (2H, q, J= 7.6 Hz), 4.39 (2H, s), 6.84-6.87 (1H, m), 7.10-7.11 (1H, m), 7.18-7.41 (8H, m), 7.58-7.60 (1H, m), 8.34-8.37 (1H, m).

### Reference Example L 74

### N-[5-(2-cyclopentylamino-4-pyridyl)-4-(3-methylphenyl)-1,3-thiazol-2-yl]-N'-phenylurea

To a solution of [5-(2-cyclopentylamino-4-pyridyl)-4-(3-methylphenyl)-1,3-thiazol-2-yl]amine (0.43 g, 1.2 mmol) in N,N-dimethylacetamide (10 mL) was added phenyl isocyanate (0.19 mL, 1.8 mmol) and the mixture was stirred at 80°C for 1 hr. Into the reaction mixture was poured aqueous sodium hydrogen carbonate solution and the mixture was extracted with ethyl acetate. The extract was washed with saturated brine, dried and concentrated. The residue was purified by silica gel column chromatography (hexane-ethyl acetate = 1 : 1). The obtained crude crystals were recrystallized from ethyl acetate to give the title compound (0.23 g, yield 39%).
m.p.: 198-199°C.

The compounds produced in Reference Examples L 62-74 are shown in Tables 80 to 82.

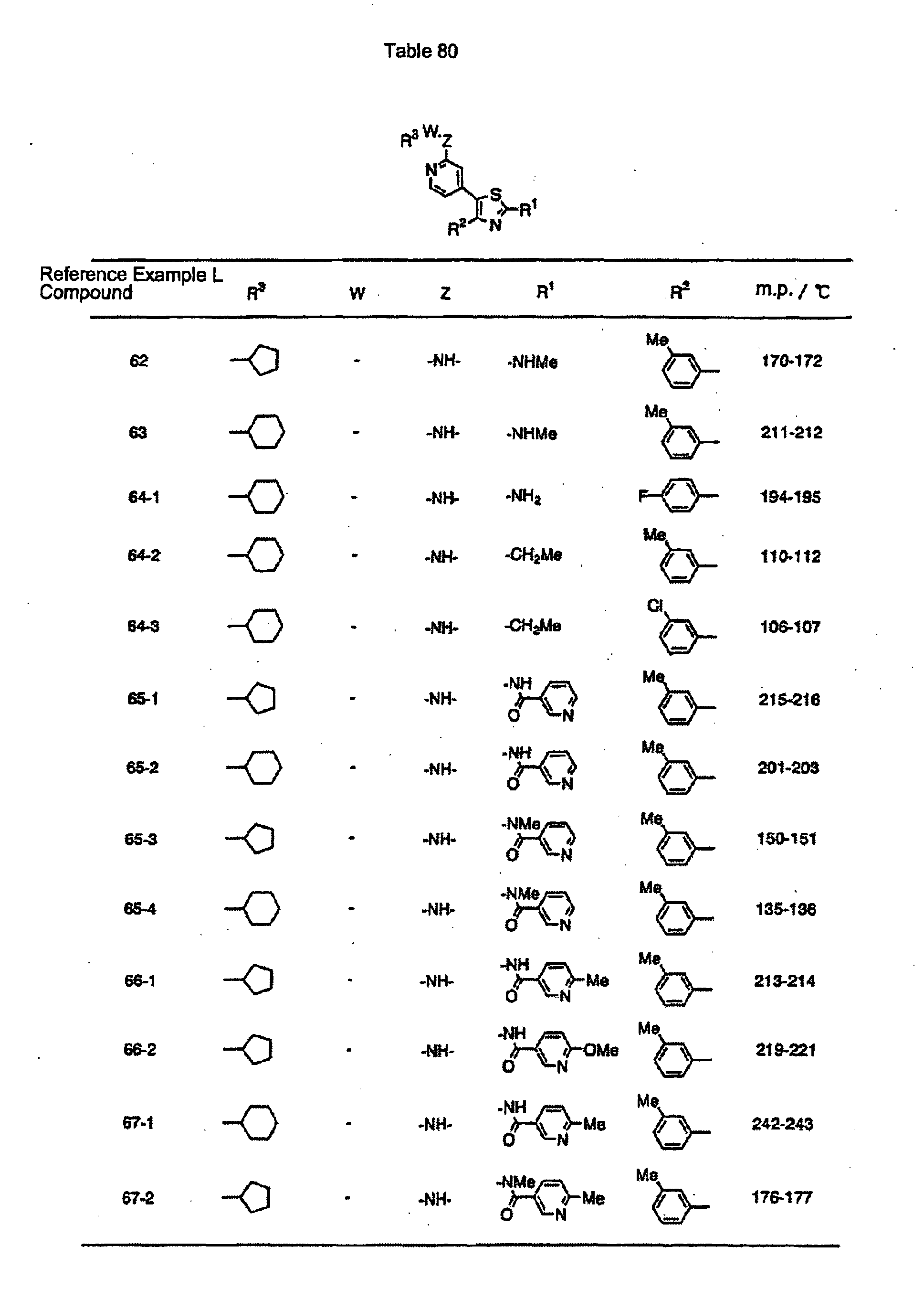

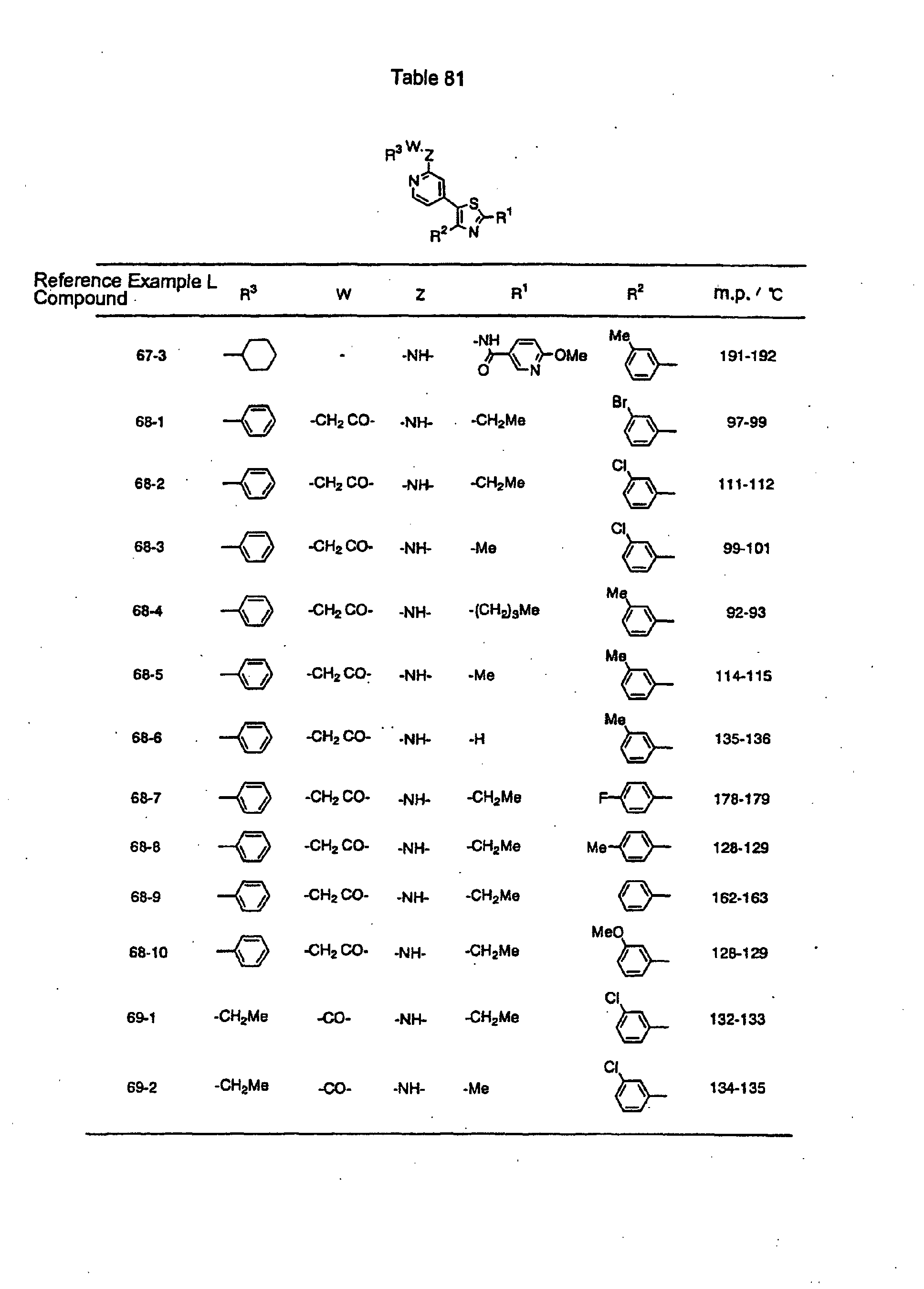

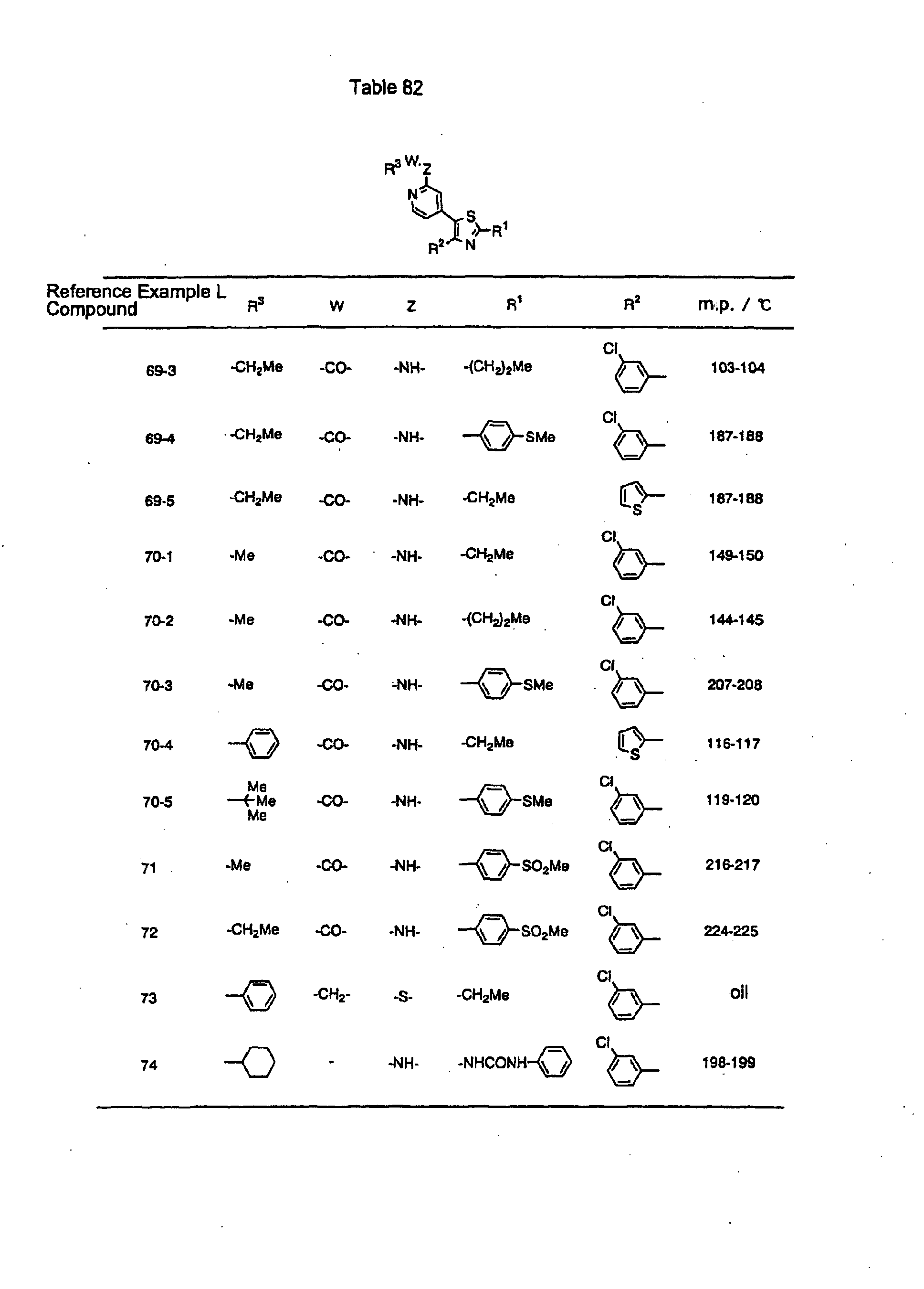

### Reference Example L 75

### ethyl 2-acetyl-3-(dimethylamino)acrylate

Ethyl acetoacetate (79 mL, 0.62 mol) was added to N,N-dimethylformamide dimethylacetal (100 mL, 0.68 mol) and the mixture was heated under reflux for 1 hr. The excess amount of acetal was evaporated under reduced pressure and the residue was subjected to distillation under reduced pressure to give the title compound (85 g, yield 74%).
b.p.: 125-130°C (400Pa)
¹H-NMR(CDCl₃)δ: 1.33 (3H, t, J= 7.1 Hz), 2.33 (3H, s), 3.04 (6H, br s), 4.23 (2H, q, J= 7.1 Hz), 7.68 (1H, s).

### Reference Example L 76

### ethyl 2,4-dimethyl-5-pyrimidinecarboxate

Acetamidine hydrochloride was added to a 10% sodium ethoxide-ethanol solution (410 mL, 0.53 mol) at room temperature. Then, ethyl 2-acetyl-3-(dimethylamino)acrylate (98 g, 0.53 mol) was added to the mixture, and the mixture was heated under reflux for 24 hrs. Ethanol was evaporated under reduced pressure. Water was added to the residue and the mixture was extracted with ethyl acetate. The extract was washed with water, dried and the solvent was evaporated. The residue was distilled under reduced pressure to give the title compound (73 g, yield 76%).
b.p.: 93-98°C (130Pa)
¹H-NMR(CDCl₃)δ: 1.42 (3H, t, J= 7.1 Hz) , 2.75 (3H, s), 2.80 (3H, s), 4.41 (2H, q, J= 7.1 Hz), 9.05 (1H, s).

### Reference Example L 77

### 2,4-dimethyl-5-pyrimidinecarboxylic acid

A solution of potassium hydroxide (67 g, 1.0 mol) in 95% ethanol (300 mL) was added to a solution of ethyl 2,4-dimethyl-5-pyrimidinecarboxate (73 g, 0.40 mol) in 95% ethanol (100 mL) and the mixture was heated under reflux for 5 hrs. Ethanol was evaporated under reduced pressure, and the residue was dissolved in water, and the aqueous solution was acidified with conc. hydrochloric acid. Precipitated solids were collected by filtration, washed with water and dried to give the title compound (36 g, yield 58%). ¹H-NMR(CDCl₃)δ: 2.63 (3H, s), 2.69 (3H, s), 8.97 (1H, s).

### Reference Example L 78

### 2,4-dimethylpyrimidine

2,4-Dimethyl-5-pyrimidinecarboxylic acid was heated at 160°C for 4 hrs. The reaction mixture was distilled under atmospheric pressure to give the title compound (17 g, yield 49%).
b.p.: 152-153°C.
¹H-NMR(CDCl₃)δ: 2.50 (3H, s), 2.70 (3H, s), 6.98 (1H, d, J= 5.1Hz), 8.49 (1H, d, J= 5.1Hz).

### Reference Example L 79

### tert-butyl 4-methyl-2-pyrimidinylcarbamate

Di(tert-butyl) dicarbamate (12 mL, 50 mmol) was added dropwise to a solution of 4-methyl-2-pyrimidinylamine (5.0 g, 46 mmol) in tert-butanol over 1 hr. and the solution was stirred at room temperature for 14 hrs. The solvent was evaporated under reduced pressure and the obtained residue was purified by silica gel column chromatography (hexane-ethyl acetate = 1 : 1). Crystallization from isopropyl ether-hexane gave the title compound (6.7 g, yield 70%).
m.p.: 87-88°C.

### Reference Example L 80

### 1-(3-methylphenyl)-2-(4-pyrimidinyl)ethenol

A solution of diisopropylamine (16 mL, 0.12 mol) in anhydrous tetrahydrofuran (100 mL) was cooled to -50°C and, with stirring, a 1.6 M n-butyl lithium in hexane solution (73 mL, 0.117 mol) was added dropwise. After the completion of the dropwise addition, the mixture was stirred for 10 min. Then, a solution of 4-methylpyrimidine (10 g, 0.11 mol) in anhydrous tetrahydrofuran (10 mL) was added dropwise at -30°C. The mixture was stirred for 0.5 hrs. and the reaction mixture was cooled to -78°C. A solution of N-(3-methylbenzoyl)propyleneimine (19 g, 0.11 mol) in anhydrous tetrahydrofuran (10 mL) was added dropwise. After the completion of the dropwise addition, the mixture was stirred at -78°C for 2 hrs. The reaction mixture was heated to room temperature, and water (100 mL) was added. The mixture was extracted with ethyl acetate. The extract was washed with water, dried and the solvent was evaporated. The obtained residue was purified by silica gel column chromatography (hexane-ethyl acetate = 7 : 3). Crystallization from isopropyl ether gave the title compound (11 g, yield 49%).
m.p.: 66-67°C.

### Reference Example L 81

In accordance with Reference Example L 80 and using 2,4-dimethylpyrimidine and tert-butyl 4-methyl-2-pyrimidinylcarbamate instead of 4-methylpyrimidine, the following Reference Example L compounds 81-1 and 81-2 were synthesized respectively.
Reference Example L compound 81-1: 1-(3-methylphenyl)-2-(2-methyl-4-pyrimidinyl)ethenol
   m.p.: 88-89°C.
Reference Example L compound 81-2: tert-butyl 4-[2-hydroxy-2-(3-methylphenyl)ethenyl]-2-pyrimidinylcarbamate
   m.p.: 194-195°C.

### Reference Example M 1

| | |
|---|---|
| (1) compound of Reference Example L 35 | 50 mg |
| (2) lactose | 34 mg |
| (3) corn starch | 10.6 mg |
| (4) corn starch (paste) | 5 mg |
| (5) magnesium stearate | 0.4 mg |
| (6) carboxymethylcellulose calcium | 20 mg |
| total | 120 mg |

The above-mentioned (1)-(6) were mixed according to a conventional method and the mixture was punched out by a tableting machine to give tablets.

### Reference Example M 2

| | |
|---|---|
| (1) compound of Reference Example L 35 | 10.0 mg |
| (2) lactose | 60.0 mg |
| (3) corn starch | 35.0 mg |
| (4) gelatin | 3.0 mg |
| (5) magnesium stearate | 2.0 mg |

A mixture of the compound of Reference Example L 35 (10.0 mg), lactose (60.0 mg) and corn starch (35.0 mg) was granulated using a 10% aqueous gelatin solution (0.03 ml, 3.0 mg as gelatin) and passed through a 1 mm mesh sieve. The granules were dried at 40°C and passed through the sieve again. The thus-obtained granules were mixed with magnesium stearate (2.0 mg) and compressed. The obtained core tablets were coated with a sugar coating of an aqueous suspension of sucrose, titanium dioxide, talc and gum arabic. The coated tablet were polished with bee's wax to give coated tablets.

### Reference Example M 3

| | |
|---|---|
| (1) compound of Reference Example L 35 | 10.0 mg |
| (2) lactose | 70.0 mg |
| (3) corn starch | 50.0 mg |
| (4) soluble starch | 7.0 mg |
| (5) magnesium stearate | 3.0 mg |

The compound of Reference Example L 35 (10.0 mg) and magnesium stearate (3.0 mg) were granulated using an aqueous soluble starch solution (0.07 ml, 7.0 mg as soluble starch), and the granules were dried and mixed with lactose (70.0 mg) and corn starch (50.0 mg). The mixture was compressed to give tablets.

### Reference Example M 4

| | |
|---|---|
| (1) compound of Reference Example L 35 | 5.0 mg |
| (2) sodium chloride | 20.0 mg |
| (3) distilled water to make total amount | 2.0 ml |

The compound of Reference Example L 35 (5.0 mg) and sodium chloride (20.0 mg) were dissolved in distilled water, and water was added to the solution to make the total amount 2.0 ml. The solution was filtered and filled in a 2 ml ampoule under aseptic conditions. The ampoule was sterilized and sealed to give a solution for injection.

### (Reference Formulation Example M 1) concomitant drug

| | |
|---|---|
| (1) Rofecoxib | 5.0 mg |
| (2) sodium chloride | 20.0 mg |
| (3) distilled water | to make total amount 2.0 ml |

Rofecoxib (5.0 mg) and sodium chloride (20.0 mg) are dissolved in distilled water and water is added to the solution to make the total amount 2.0 ml. The solution is filtered and filled in a 2 ml ampoule under aseptic conditions. The ampoule is sterilized and sealed to give a solution for injection.

### (Reference Formulation Example 2) concomitant drug

| | |
|---|---|
| (1) Rofecoxib | 50 mg |
| (2) lactose | 34 mg |
| (3) corn starch | 10.6 mg |
| (4) corn starch (paste) | 5 mg |
| (5) magnesium stearate | 0.4 mg |
| (6) carboxymethylcellulose calcium | 20 mg |
| total | 120 mg |

The above-mentioned (1)-(6) were mixed according to a conventional method and the mixture was punched out by a tableting machine to give tablets.

### Reference Example M 5

Any preparation prepared in Reference Examples M 1-4 and a preparation of Reference Formulation Example M 1 or M 2 are combined.

### Reference Example M 6

### [4-(3-methylphenyl)-5-(4-pyrimidinyl)-1,3-thiazol-2-yl]amine

To a solution (70 mL) of 1-(3-methylphenyl)-2-(4-pyrimidinyl)ethenol (3.0 g, 14 mmol) and sodium acetate (2.32 g, 28.26 mmol) in acetic acid was added dropwise a solution (70 mL) of bromine (0.72 mL, 14 mmol) in acetic acid at room temperature over 30 min. The mixture was stirred at room temperature for 2 hrs. Acetic acid was evaporated under reduced pressure, and aqueous sodium hydrogen carbonate solution was added to the residue. The mixture was extracted with ethyl acetate and the extract was dried and concentrated. The residue was dissolved in N,N-dimethylformamide (15 mL), and thiourea (1.1 g, 16 mmol) was added to the solution. The reaction mixture was stirred at room temperature for 14 hrs. Aqueous sodium hydrogen carbonate solution was added, and the precipitated solids were collected by filtration. The solids were washed with water, dried and subjected to recrystallization from ethyl acetate to give the title compound (3.4 g, yield 89%).
m.p. : 241-242°C.

### Reference Example M 7

In accordance with Reference Example M 6 and using 1-(3-methylphenyl)-2-(2-methyl-4-pyrimidinyl)ethenol and tert-butyl 4-[2-hydroxy-2-(3-methylphenyl)ethenyl]-2-pyrimidinylcarbamate instead of 1-(3-methylphenyl)-2-(4-pyrimidinyl)ethenol, the following Reference Example M compounds 7-1 and 7-2 were synthesized.
Reference Example M compound 7-1: [4-(3-methylphenyl)-5-(2-methyl-4-pyrimidinyl)-1,3-thiazol-2-yl]amine
   m.p.: 185-186°C.
Reference Example M compound 7-2: tert-butyl 4-[2-amino-4-(3-methylphenyl)-1,3-thiazol-5-yl]-2-pyrimidinylcarbamate
   m.p.: 262-264°C.

### Reference Example M 8

### methyl 4-[[[4-(3-methylphenyl)-5-(4-pyrimidinyl)-1,3-thiazol-2-yl]amino]carbonyl]benzoate

Methyl 4-chloroformylbenzoate (1.1 g, 5.6 mmol) was added to a solution of [4-(3-methylphenyl)-5-(4-pyrimidinyl)-1,3-thiazol-2-yl]amine and 4-dimethylaminopyridine (0.14 g, 1.1 mmol) in N,N-dimethylacetamide (10 mL), and the mixture was stirred at 70°C for 14 hrs. Aqueous sodium hydrogen carbonate solution was added to the reaction mixture and the precipitated solids were collected by filtration. Crude crystals were washed with water, dried and recrystallized from pyridine to give the title compound (1.0 g, yield 65%).
m.p.: 339-341°C.

### Reference Example M 9

### 4-[[[4-(3-methylphenyl)-5-(4-pyrimidinyl)-1,3-thiazol-2-yl]amino]carbonyl]benzoic acid

To a suspension of methyl 4-[[[4-(3-methylphenyl)-5-(4-pyrimidinyl)-1,3-thiazol-2-yl]amino]carbonyl]benzoate (0.50 g, 1.2 mmol) in ethanol (10 mL) was added 2N aqueous sodium hydroxide solution (1.2 mL), and the mixture was stirred at room temperature for 2 hrs. The reaction mixture was acidified with 2N hydrochloric acid and the precipitated solids were collected by filtration. Crude crystals were washed with water and dried to give the title compound (0.40 g, yield 82%).
m.p.: 380-381°C.

### Reference Example M 10

### N-[4-(3-methylphenyl)-5-(2-methyl-4-pyrimidinyl)-1,3-thiazol-2-yl]acetamide

To a solution of [4-(3-methylphenyl)-5-(2-methyl-4-pyrimidinyl)-1,3-thiazol-2-yl]amine (0.50 g, 1.8 mmol) and 4-dimethylaminopyridine (0.065 g, 0.53 mmol) in N,N-dimethylacetamide (10 mL) was added acetyl chloride (0.19 mL, 2.7 mmol), and the mixture was stirred at 80°C for 14 hrs. Into the reaction mixture was poured aqueous sodium hydrogen carbonate solution and the mixture was extracted with ethyl acetate. The extract was washed with saturated brine, dried and concentrated. The resulting crystals were recrystallized from ethyl acetate to give the title compound (0.35 g, yield 61%).
m.p.: 230-231°C.

### Reference Example M 11

### N-[5-(2-methyl-4-pyrimidinyl)-4-(3-methylphenyl)-1,3-thiazol-2-yl]-N'-phenylurea

To a solution of [5-(2-methyl-4-pyrimidinyl)-4-(3-methylphenyl)-1,3-thiazol-2-yl]amine (0.50 g, 1.8 mmol) in N,N-dimethylacetamide (10 mL) was added phenylisocyanate (0.29 mL, 2.7 mmol), and the mixture was stirred at 80°C for 2 hrs. Into the reaction mixture was poured aqueous sodium hydrogen carbonate solution, and the mixture was extracted with ethyl acetate. The extract was washed with saturated brine, dried and concentrated. The resulting crystals were recrystallized from ethyl acetate-hexane to give the title compound (0.55 g, yield 78%).
m.p.: 141-142°C.

### Reference Example M 12

### N-[4-(3-methylphenyl)-5-[2-(phenylacetylamino)-4-pyrimidinyl]-1,3-thiazol-2-yl]phenylacetamide

To a solution of tert-butyl 4-[2-amino-4-(3-methylphenyl)-1,3-thiazol-5-yl]-2-pyrimidinylcarbamate (0.50 g, 1.3 mmol) in N,N-dimethylacetamide (5 mL) was added phenylacetylchloride (0.52 mL, 3.9 mmol) and the mixture was stirred at 80°C for 14 hrs. Into the reaction mixture was poured aqueous sodium hydrogen carbonate solution, and the mixture was extracted with ethyl acetate. The extract was washed with saturated brine, dried and concentrated. The obtained residue was purified by silica gel column chromatography (hexane-ethyl acetate = 7 : 3), and the obtained oil was recrystallized from ethyl ether to give the title compound (0.09 g, yield 13%).
m.p.: 110-113°C.

The compounds prepared in Reference Examples M 6-12 are shown in Table 83.

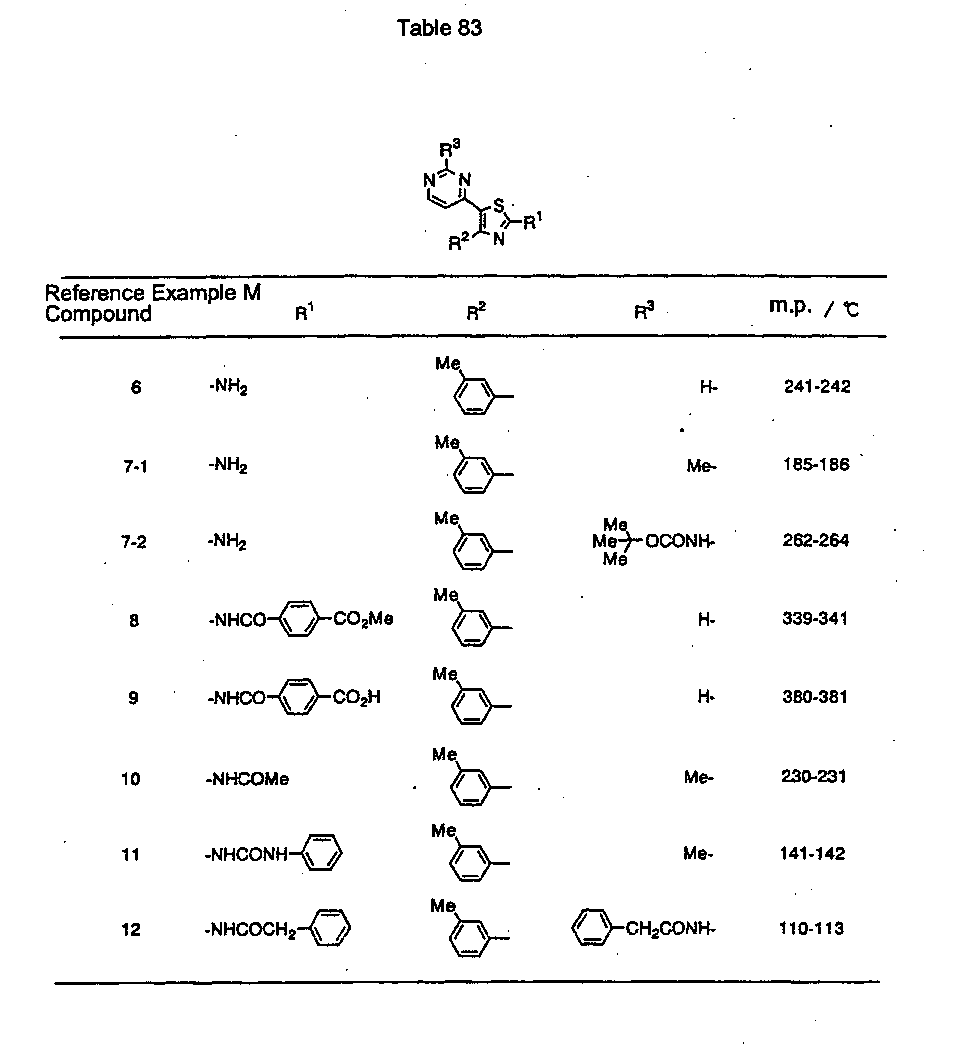

### Reference Example N 1 Measurement of TNF-α production inhibitory activity

After THP-1 cells which had been cultured on RPMI 1640 medium (GibcoBRL) containing 1% inactivated fetal bovine serum and 10 mM HEPES (pH 7.5) were seeded on a 96-well plate to 1×10⁵ cells/well, 1 mL test compound dissolved in DMSO was added. After incubation at 37°C for 1 hour in a CO₂ incubator, LPS (Wako Pure Chemical Industries, Ltd.) was added to the final concentration 5 mg/mL. After cultured at 37°C for 4 hours in a CO₂ incubator, the supernatant was obtained by centrifugation. The concentration of TNF-α in the supernatant was measured by ELISA kit (DIACLONE). The concentration of the test compound necessary for inhibiting TNF-α production by 50% (IC₅₀ value) was calculated using PRISM 2.01 (Graphpad Software). The results are shown in Table 84.

**Table 84**

| Reference Example L Compound No. | IC₅₀ (µM) |
|---|---|
| 29-1 | 0.0020 |
| 32 | 0.10 |
| 34-1 | 0.057 |
| 39 | 0.0059 |
| 40 | 0.015 |

From the above results, it is clear that the Compound of the present invention has an excellent inhibitory activity against TNF-α production.

### Example 1 test using rat yeast-induced pain model animal

Male Sprague-Dawley rats (4-week-old, Clear Japan) were used. A 20% yeast physiological saline solution (0.1 ml) was subcutaneously injected into right hind pedal sole to induce inflammation in the right hind pedal sole. One hour later, 0.5% aqueous methyl cellulose or Reference Example D compound 16-1 suspended in 0.5% aqueous methyl cellulose was orally administered (0.5 ml/100 g body weight). After 2 hours from sample administration, pain threshold value was measured based on false escape reaction using a pressure device. A group contained 6 rats.

As a result, Reference Example D compound 16-1 showed a dose-dependent antinociceptive effect and the minimum effective dose was 10 mg/kg.

### Example 2 Inhibitory action of osteoclast formation

Bone marrow cells were collected from the tibia of 8-week-old male ddY mouse and suspended in αMEM medium containing 10% FBS and sown in a 24-well plate at a density of 10⁶ cells/0.5 ml/well. After static culture overnight, the medium was changed to the above-mentioned medium containing 10⁻ ⁸ M 1α, 25-(OH)₂D₃ or Reference Example D compound 16-1 at 0.01, 0.1, 1 or 10 µM, respectively and further cultured for 6 days. After the completion of the culture, the cells were fixed and subjected to staining of tartrateresistant acid phosphatase (TRAP), which is a differentiation index of osteoclast, and TRAP positive multinucleated cells with three or more nuclei were counted as osteoclasts with a microscope.

As a result, osteoclasts of about 200 cells/well were formed from bone marrow cells, and the number decreased in a dose-dependent manner by the addition of Reference Example D compound 16-1 (Table 85). The action was found significantly from 0.1 µM, and formation of osteoclast was barely observed at 10 µM. The IC₅₀ value of Reference Example D compound 16-1 against formation of osteoclast was 0.3 µM.

**Table 85**

| Reference Example D 16-1 concentration (µM) | Osteoclast count (/well) |
|---|---|
| 0 | 202.5±9.3 |
| 0.01 | 183.8±2.9 |
| 0.1 | 140.8±7.6* |
| 1 | 72.4±4.4* |
| 10 | 0.6±0.4* |

| | |
|---|---|
| *: p≤0.025 vs vehicle control by one-tailed Williams test average value ± standard deviation (N=4-5) | |

From the results, it was found that Reference Example D compound 16-1 had a superior inhibitory effect on osteoclast formation in the mouse bone marrow cells culture system.

### Example 3 Measurement of CYP3A4 inhibitory activity

10 pmol/mL CYP3A4, 100 µM testosterone, 1/10 volume of the reaction mixture of NADPH generating system and test substance (10 µM) were added to a 50 mM phosphate buffer (pH 7.4) and control microsome was added to adjust the final concentration of protein to 1.0 mg/mL. The reaction mixture was prepared and reacted at 37°C for 30 min. (amount of total reaction solution 100 µL). The reaction was quenched by the addition of 100 µL of acetonitrile. After stirring, 100 µL of distilled water was added and the mixture was further stirred. A supernatant obtained by centrifugation (15000 rpm) for 10 min. was injected to HPLC by 80 µL and concentration of 6-β-hydroxytestosterone produced by the reaction was measured.

For preparation of the NADPH generating system, 1 volume of 50 mM NADP (β-nicotinamide adenine dinucleotide oxidized type), 1 volume of 0.5 M glucose-6-phosphate, 5 volumes of 0.1 M magnesium chloride, 1 volume of 150 unit/mL glucose-6-phosphate dehydrase and 2 volumes of distilled water were mixed. The results are shown in Table 86.

**Table 86**

| Reference Example compound No. | inhibitory rate (%) at 10 µM |
|---|---|
| Reference Example A 44-24 | >60% |
| Reference Example A 46-8 | <30% |
| Reference Example D 19-4 | <30% |
| Reference Example H 11 | <30% |
| Reference Example H 13 | <30% |
| Reference Example H 22 | <30% |
| Reference Example H 29-2 | <30% |
| Reference Example H 69 | <30% |

From the results, it was found that a compound containing a pyridyl group wherein a substituent has been introduced into the α-position of nitrogen atom of the pyridyl group, and a compound containing a pyridyl group and an aromatic hydrocarbon group wherein a polar group has been introduced into the aromatic hydrocarbon group showed reduced P450 inhibitory action.

### Industrial Applicability

The p38 MAP kinase inhibitor and/or the TNF-α production inhibitor are useful as an agent for prophylaxis or treatment of pain, an agent for suppressing activation of osteoclast, and an inhibitor of osteoclast formation.

This application is based on patent application Nos. 2001-175224 and 2001-175273 filed in Japan, the contents of which are hereby incorporated by reference.

## Claims

1. An agent for the prophylaxis or treatment of pain and/or suppression of activation and/or inhibition of formation of osteoclast, which contains a p38 MAP kinase inhibitor and/or a TNF-α production inhibitor.

2. The agent of claim 1 for the prophylaxis or treatment of pain, which contains a p38 MAP kinase inhibitor and/or a TNF-α production inhibitor.

3. The agent of claim 1 for the suppression of activation and/or inhibition of formation of osteoclast, which contains a p38 MAP kinase inhibitor and/or a TNF-α production inhibitor.

4. The agent of claim 1, wherein the p38 MAP kinase inhibitor and/or the TNF-α production inhibitor are/is a 1,3-thiazole compound substituted at the 5-position by a pyridyl group optionally having substituents, or a salt thereof or a prodrug thereof.

5. The agent of claim 1, wherein the p38 MAP kinase inhibitor and/or the TNF-α production inhibitor are/is a compound represented by the formula: 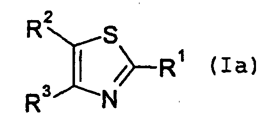 wherein
R¹ represents a hydrogen atom, a hydrocarbon group optionally having substituents, a heterocyclic group optionally having substituents, an amino group optionally having substituents or an acyl group;
R² represents a pyridyl group optionally having substituents; and
R³ represents an aromatic group optionally having substituents, a salt thereof or a prodrug thereof.

6. The agent of claim 1, wherein the p38 MAP kinase inhibitor and/or the TNF-α production inhibitor are/is an optionally N-oxidized compound represented by the formula: 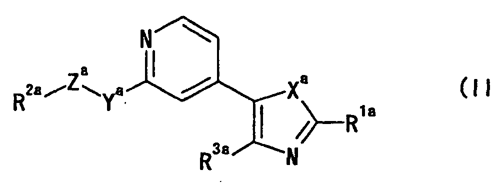 wherein
R^{1a} represents a hydrogen atom, a hydrocarbon group optionally having substituents, a heterocyclic group optionally having substituents, an amino group optionally having substituents or an acyl group;
R^{2a} represents an aromatic group optionally having substituents;
R^{3a} represents a hydrogen atom, a pyridyl group optionally having substituents or an aromatic hydrocarbon group optionally having substituents;
X^{a} represents an oxygen atom or an optionally oxidized sulfur atom;
Y^{a} represents a bond, an oxygen atom, an optionally oxidized sulfur atom or a group represented by the formula: NR^{4a} (wherein R^{4a} represents a hydrogen atom, a hydrocarbon group optionally having substituents or an acyl group); and
Z^{a} represents a bond or a divalent acyclic hydrocarbon group optionally having substituents,
or a salt thereof, or a prodrug thereof.

7. The agent of claim 1, wherein the p38 MAP kinase inhibitor and/or the TNF-α production inhibitor are/is a compound represented by the formula: 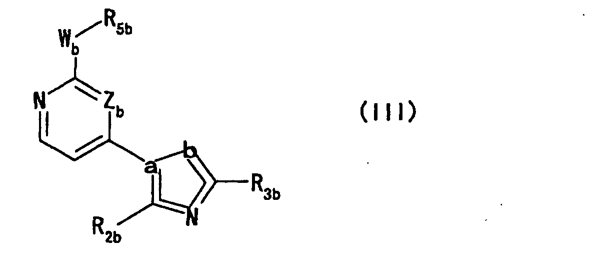 wherein
a is N or C;
b is CH when a is N, or O when a is C;
= denotes a single or a double bond dependent upon whether the azole ring is an imidazole ring or an oxazole ring;
Z_{b} is N or CH;
W_{b} is -NR_{6b}-Y_{b}-, -0- or -S-,
where R_{6b} is a hydrogen atom, C₁₋₄ alkyl group, C₃₋₈ cycloalkyl group, C₃₋₈ cycloalkyl-C₁₋₃ alkyl group, C₆₋₁₈ aryl group, C₃₋₁₈ heteroaryl group, C₇₋₁₉ aralkyl group or C₄₋₁₉ heteroaralkyl group, and -Y_{b}- is C₁₋₄ alkylene group or a bond;
R_{2b} is phenyl group, optionally substituted by one or more substituents selected from the group consisting of a halogen atom, trifluoromethyl, cyano, amido, thioamido, carboxylate, thiocarboxylate, C₁₋₄ alkoxy, C₁₋₄ alkyl, amino, and mono- or di-C₁₋₄ alkylamino;
R_{3b} is a hydrogen atom, a halogen atom, C₁₋₁₀ alkyl group, C₂₋₄ alkenyl group, C₃₋₁₀ cycloalkyl group, C₃₋₁₈ heterocycloalkyl group, C₆₋₁₈ aryl group, C₃₋₁₈ heteroaryl group or -CH=N-NH-C(NH)NH₂ (wherein C₁₋₁₀ alkyl group, C₂₋₄ alkenyl group, C₃₋₁₀ cycloalkyl group, C₃₋₁₈ heterocycloalkyl group, C₆₋₁₈ aryl group, C₃₋₁₈ heteroaryl group and -CH=N-NH-C(NH)NH₂ are each optionally substituted by 1 to 4 substituents selected from the group consisting of C₁₋₄ alkyl optionally substituted by hydroxy, halogen atom, halo-substituted-C₁₋₄ alkyl, hydroxy, C₁₋₄ alkoxy, C₁₋₄ alkylthio, carboxy, carbonyl optionally substituted by C₁₋₆ alkyl or C₁₋₆ alkoxy, amino, mono- or di-C₁₋₄ alkylamino and 5- to 7- membered N-heterocyclic group optionally further containing heteroatom(s)); and
R_{5b} is C₆₋₁₈ aryl group, C₃₋₁₈ heteroaryl group or C₃₋₁₂ cycloalkyl group each of which is optionally substituted by 1 to 4 substituents selected from the group consisting of C₁₋₄ alkyl, halogen, halo-substitued-C₁₋₄ alkyl, hydroxy, C₁₋₄ alkoxy, C₁₋₄ alkylthio, amino, mono- or di-C₁₋₄ alkylamino and 5- to 7-membered N-heterocyclic group optionally further containing heteroatom(s),
or a salt thereof or a prodrug thereof.

8. The agent of claim 1, wherein the p38 MAP kinase inhibitor and/or the TNF-α production inhibitor is a 1,3-thiazole compound substituted at the 5-position by a 4-pyridyl group having substituents free of aromatic group, or a salt thereof or a prodrug thereof.

9. The agent of [8], wherein the 1,3-thiazole compound is a compound represented by the formula: 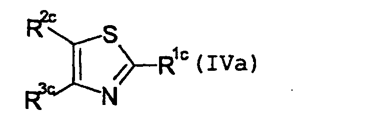 wherein
R^{1c} is a hydrogen atom, a hydrocarbon group optionally having substituents, a heterocyclic group optionally having substituents, an amino group optionally having substituents or an acyl group;
R^{2c} is a 4-pyridyl group having substituents free of aromatic group; and
R^{3c} is an aromatic group optionally having substituents,
or a salt thereof.

10. The agent of claim 1, wherein the p38 MAP kinase inhibitor and/or the TNF-α production inhibitor is a 1,3-thiazole compound substituted at the 5-position by a pyridyl group having substituents free of aromatic group at a position next to a nitrogen atom of the pyridyl group, or a salt thereof, or a prodrug thereof.

11. The agent of claim 10, wherein the 1,3-thiazole compound is a compound represented by the formula: 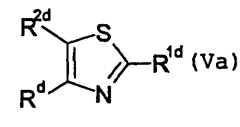 wherein
R^{1d} is a hydrogen atom, a hydrocarbon group optionally having substituents, a heterocyclic group optionally having substituents, an amino group optionally having substituents or an acyl group;
R^{2d} is a pyridyl group having substituents free of aromatic group at a position next to a nitrogen atom of the pyridyl group; and
R^{3d} is an aromatic group optionally having substituents;
or a salt thereof.

12. The agent of claim 1, wherein the p38 MAP kinase inhibitor and/or the TNF-α production inhibitor are/is a 1,3-thiazole compound substituted at the 5-position by a 4-pyridyl group having substituents free of aromatic group at a position next to a nitrogen atom of the 4-pyridyl group, or a salt thereof or a prodrug thereof.

13. The agent of claim 1, wherein the p38 MAP kinase inhibitor and/or the TNF-α production inhibitor are/is
N-[5-(2-benzoylamino-4-pyridyl)-4-(3,5-dimethylphenyl)-1,3-thiazol-2-yl]acetamide,
N-[5-(2-benzylamino-4-pyridyl)-4-(3,5-dimethylphenyl)-1,3-thiazol-2-yl]acetamide,
N-[4-[4-(4-methoxyphenyl)-2-methyl-1,3-thiazol-5-yl]-2-pyridyl]benzamide,
N-[4-[2-(4-fluorophenyl)-4-(3-methylphenyl)-1,3-thiazol-5-yl]-2-pyridyl]phenylacetamide,
N-[4-[2-ethyl-4-(3-methylphenyl)-1,3-thiazol-5-yl]-2-pyridyl]phenylacetamide,
N-[4-[4-(3-methylphenyl)-2-propyl-1,3-thiazol-5-yl]-2-pyridyl]phenylacetamide,
N-[4-[2-butyl-4-(3-methylphenyl)-1,3-thiazol-5-yl]-2-pyridyl]phenylacetamide,
N-[4-[4-(3-methylphenyl)-2-(4-methylthiophenyl)-1,3-thiazol-5-yl]-2-pyridyl]phenylacetamide,
N-[4-[2-ethyl-4-(3-methylphenyl)-1,3-thiazol-5-yl]-2-pyridyl]benzamide,
N-[4-[2-ethyl-4-(3-methylphenyl)-1,3-thiazol-5-yl]-2-pyridyl]-3-phenylpropionamide,
N-[4-[2-ethyl-4-(3-methylphenyl)-1,3-thiazol-5-yl]-2-pyridyl]-3-(4-methoxyphenyl)propionamide,
N-[4-[2-ethyl-4-(3-methylphenyl)-1,3-thiazol-5-yl]-2-pyridyl]-4-phenylbutyramide,
N-[4-[4-(3-methylphenyl)-2-propyl-1,3-thiazol-5-yl]-2-pyridyl]benzamide,
N-[4-[4-(3-methylphenyl)-2-propyl-1,3-thiazol-5-yl]-2-pyridyl]-3-phenylpropionamide,
N-[4-[2-butyl-4-(3-methylphenyl)-1,3-thiazol-5-yl]-2-pyridyl]benzamide,
N-[4-[2-butyl-4-(3-methylphenyl)-1,3-thiazol-5-yl]-2-pyridyl]-3-phenylpropionamide,
N-[4-[2-(4-fluorophenyl)-4-(3-methylphenyl)-1,3-thiazol-5-yl]-2-pyridyl]benzamide,
N-[4-[2-(4-fluorophenyl)-4-(3-methylphenyl)-1,3-thiazol-5-yl]-2-pyridyl]-3-phenylpropionamide,
N-[4-[4-(3-methylphenyl)-2-(4-methylthiophenyl)-1,3-thiazol-5-yl]-2-pyridyl]benzamide,
N-[4-[4-(3-methylphenyl)-2-(4-methylthiophenyl)-1,3-thiazol-5-yl]-2-pyridyl]-3-phenylpropionamide,
N-benzyl-N-[4-[2-ethyl-4-(3-methylphenyl)-1,3-thiazol-5-yl]-2-pyridyl]amine,
N-[4-[2-ethyl-4-(3-methylphenyl)-1,3-thiazol-5-yl]-2-pyridyl]-N-(2-phenylethyl)amine,
N-[4-[2-ethyl-4-(3-methylphenyl)-1,3-thiazol-5-yl]-2-pyridyl]-N-(3-phenylpropyl)amine,
N-benzyl-N-[4-[4-(3-methylphenyl)-2-propyl-1,3-thiazol-5-yl]-2-pyridyl]amine,
N-[4-[4-(3-methylphenyl)-2-propyl-1,3-thiazol-5-yl]-2-pyridyl]-N-(2-phenylethyl)amine,
N-[4-[4-(3-methylphenyl)-2-propyl-1,3-thiazol-5-yl]-2-pyridyl]-N-(3-phenylpropyl)amine,
N-benzyl-N-[4-[2-butyl-4-(3-methylphenyl)-1,3-thiazol-5-yl]-2-pyridyl]amine,
N-[4-[2-butyl-4-(3-methylphenyl)-1,3-thiazol-5-yl]-2-pyridyl]-N-(2-phenylethyl)amine,
N-[4-[2-butyl-4-(3-methylphenyl)-1,3-thiazol-5-yl]-2-pyridyl]-N-(3-phenylpropyl)amine,
N-benzyl-N-[4-[4-(3-methylphenyl)-2-(4-methylthiophenyl)-1,3-thiazol-5-yl]-2-pyridyl]amine,
N-[4-[4-(3-methylphenyl)-2-(4-methylthiophenyl)-1,3-thiazol-5-yl]-2-pyridyl]-N-(2-phenylethyl)amine,
N-[4-[4-(3-methylphenyl)-2-(4-methylthiophenyl)-1,3-thiazol-5-yl]-2-pyridyl]-N-(3-phenylpropyl)amine,
N-[4-[4-(3-methylphenyl)-2-(4-methylsulfonylphenyl)-1,3-thiazol-5-yl]-2-pyridyl]benzamide,
N-[4-[4-(3-methylphenyl)-2-(4-methylsulfonylphenyl)-1,3-thiazol-5-yl]-2-pyridyl]phenylacetamide,
N-[4-[4-(3-methylphenyl)-2-(4-methylsulfonylphenyl)-1,3-thiazol-5-yl]-2-pyridyl]-3-phenylpropionamide,
N-benzyl-N-[4-[4-(3-methylphenyl)-2-(4-methylsulfonylphenyl)-1,3-thiazol-5-yl]-2-pyridyl]amine,
N-[4-[4-(3-methylphenyl)-2-(4-methylsulfonylphenyl)-1,3-thiazol-5-yl]-2-pyridyl]-N-(3-phenylpropyl)amine,
N-[4-[4-(3-methylphenyl)-2-(4-methylsulfonylphenyl)-1,3-thiazol-5-yl]-2-pyridyl]-N-(2-phenylethyl)amine,
N-(4-fluorobenzyl)-N-[4-[4-(3-methylphenyl)-2-(4-methylsulfonylphenyl)-1,3-thiazol-5-yl]-2-pyridyl]amine,
(S)-N-[4-(3-methylphenyl)-5-(2-(1-phenylethylamino)-4-pyridyl)-1,3-thiazol-2-yl]nicotinamide,
(R)-N-[4-(3-methylphenyl)-5-(2-(1-phenylethylamino)-4-pyridyl)-1,3-thiazol-2-yl]nicotinamide,
(S)-N-[4-(3-methylphenyl)-5-(2-(1-phenylethylamino)-4-pyridyl)-1,3-thiazol-2-yl]-2-methylnicotinamide,
(R)-N-[4-(3-methylphenyl)-5-(2-(1-phenylethylamino)-4-pyridyl)-1,3-thiazol-2-yl]-2-methylnicotinamide,
(S)-N-[4-(3-methylphenyl)-5-(2-(1-phenylethylamino)-4-pyridyl)-1,3-thiazol-2-yl]-2-chloronicotinamide,
(R)-N-[4-(3-methylphenyl)-5-(2-(1-phenylethylamino)-4-pyridyl)-1,3-thiazol-2-yl]-2-chloronicotinamide,
(S)-N-[4-(3-methylphenyl)-5-(2-(1-phenylethylamino)-4-pyridyl)-1,3-thiazol-2-yl]-2-methoxynicotinamide,
(R)-N-[4-(3-methylphenyl)-5-(2-(1-phenylethylamino)-4-pyridyl)-1,3-thiazol-2-yl]-2-methoxynicotinamide,
N-[5-(2-benzylamino-4-pyridyl)-4-(3-methylphenyl)-1,3-thiazol-2-yl]nicotinamide,
N-[5-(2-benzylamino-4-pyridyl)-4-(3-methylphenyl)-1,3-thiazol-2-yl]-2-methoxynicotinamide,
N-[5-(2-benzylamino-4-pyridyl)-4-(3-methylphenyl)-1,3-thiazol-2-yl]-2-chloronicotinamide,
N-[5-(2-benzylamino-4-pyridyl)-4-(3-methylphenyl)-1,3-thiazol-2-yl]-2-methylnicotinamide,
N-[5-(2-benzoylamino-4-pyridyl)-4-(3-methylphenyl)-1,3-thiazol-2-yl]nicotinamide,
N-[5-(2-benzoylamino-4-pyridyl)-4-(3-methylphenyl)-1,3-thiazol-2-yl]-2-methylnicotinamide,
N-[5-(2-benzoylamino-4-pyridyl)-4-(3-methylphenyl)-1,3-thiazol-2-yl]-2-chloronicotinamide,
N-[5-(2-benzoylamino-4-pyridyl)-4-(3-methylphenyl)-1,3-thiazol-2-yl]-2-methoxynicotinamide,
(S)-N-(1-phenylethyl)-4-[2-ethyl-4-(3-methylphenyl)-1,3-thiazol-5-yl]-2-pyridylamine,
(R)-N-(1-phenylethyl)-4-[2-ethyl-4-(3-methylphenyl)-1,3-thiazol-5-yl]-2-pyridylamine,
(S)-N-(1-phenylethyl)-4-[4-(3-methylphenyl)-2-propyl-1,3-thiazol-5-yl]-2-pyridylamine,
(R)-N-(1-phenylethyl)-4-[4-(3-methylphenyl)-2-propyl-1,3-thiazol-5-yl]-2-pyridylamine,
(S)-N-(1-phenylethyl)-4-[2-butyl-4-(3-methylphenyl)-1,3-thiazol-5-yl]-2-pyridylamine,
(R)-N-(1-phenylethyl)-4-[2-butyl-4-(3-methylphenyl)-1,3-thiazol-5-yl]-2-pyridylamine,
(S)-N-(1-phenylethyl)-4-[4-(3-methylphenyl)-2-(4-methylthiophenyl)-1,3-thiazol-5-yl]-2-pyridylamine,
(R)-N-(1-phenylethyl)-4-[4-(3-methylphenyl)-2-(4-methylthiophenyl)-1,3-thiazol-5-yl]-2-pyridylamine,
(S)-N-(1-phenylethyl)-4-[4-(3-methylphenyl)-2-(4-methylsulfonylphenyl)-1,3-thiazol-5-yl]-2-pyridylamine,
(R)-N-(1-phenylethyl)-4-[4-(3-methylphenyl)-2-(4-methylsulfonylphenyl)-1,3-thiazol-5-yl]-2-pyridylamine,
(S)-N-(1-phenylethyl)-4-[2-(4-fluorophenyl)-4-(3-methylphenyl)-1,3-thiazol-5-yl]-2-pyridylamine,
(R)-N-(1-phenylethyl)-4-[2-(4-fluorophenyl)-4-(3-methylphenyl)-1,3-thiazol-5-yl]-2-pyridylamine, or a salt thereof.

14. The agent of claim 3, which is an agent for the prophylaxis or treatment of (1) postmonopausal or senile primary osteoporosis, (2) secondary osteoporosis caused by inflammation, blood system malignant disease, endocrine disorder or administration of pharmaceutical agent, (3) bone or joint tissue destruction or deforming associated with bone metastasis of tumor or rheumatism, (4) Paget's disease or (5) hypercalcemia.

15. A method for the prophylaxis or treatment of pain, which comprises administering an effective amount of p38 MAP kinase inhibitor and/or the TNF-α production inhibitor to a mammal.

16. A method for the suppression of activation and/or inhibition of formation of osteoclast, which comprises administering an effective amount of p38 MAP kinase inhibitor and/or the TNF-α production inhibitor to a mammal.

17. A method for the prophylaxis or treatment of (1) postmonopausal or senile primary osteoporosis, (2) secondary osteoporosis caused by inflammation, blood system malignant disease, endocrine disorder or administration of pharmaceutical agent, (3) bone or joint tissue destruction or deforming associated with bone metastasis of tumor or rheumatism, (4) Paget's disease or (5) hypercalcemia, which comprises administering an effective amount of p38 MAP kinase inhibitor and/or the TNF-α production inhibitor to a mammal.

18. Use of a p38 MAP kinase inhibitor and/or a TNF-α production inhibitor for the production of an agent for the prophylaxis or treatment of pain.

19. Use of a p38 MAP kinase inhibitor and/or a TNF-α production inhibitor for the production of an agent for the suppression of activation and/or inhibition of formation of osteoclast.

20. Use of a p38 MAP kinase inhibitor and/or a TNF-α production inhibitor for the production of an agent for the prophylaxis or treatment of (1) postmonopausal or senile primary osteoporosis, (2) secondary osteoporosis caused by inflammation, blood system malignant disease, endocrine disorder or administration of pharmaceutical agent, (3) bone or joint tissue destruction or deforming associated with bone metastasis of tumor or rheumatism, (4) Paget's disease or (5) hypercalcemia.

21. A method for reducing a P450 inhibitory action of a compound containing a pyridyl group or a salt thereof, which comprises introducing a substituent into the α-position of a nitrogen atom of the pyridyl group of the compound or a salt thereof.

22. A method for reducing a P450 inhibitory action of a compound containing a pyridyl group and an aromatic hydrocarbon group, or a salt thereof, which comprises introducing a polar group into the aromatic hydrocarbon group of the compound or a salt thereof.

23. The method of claim 22, further comprising introducing a substituent into the α-position of a nitrogen atom of the pyridyl group.

24. The method of claim 21 or 22, wherein the P450 is CYP2C9, CYP2D6 or CYP3A4.

25. The method of claim 21 or 23, wherein the substituent is 1 to 3 selected from
(i) halogen atom,
(ii) C₁₋₆ alkyl group, C₂₋₆ alkenyl group, C₂₋₆ alkynyl group, C₃₋₆ cycloalkyl group, C₆₋₁₄ aryl group and C₇₋₁₆ aralkyl group [these groups may have 1 to 5 substituents selected from a group consisting of oxo, halogen atom, C₁₋₃ alkylenedioxy, nitro, cyano, optionally halogenated C₁₋₆ alkyl, optionally halogenated C₂₋₆ alkenyl, carboxy C₂₋₆ alkenyl, optionally halogenated C₂₋₆ alkynyl, optionally halogenated C₃₋₆ cycloalkyl, C₆₋₁₄ aryl, optionally halogenated C₁₋₈ alkoxy, C₁₋₆ alkoxy-carbonyl-C₁₋₆ alkoxy, hydroxy, C₆₋₁₄ aryloxy, C₇₋₁₆ aralkyloxy, mercapto, optionally halogenated C₁₋₆ alkylthio, C₆₋₁₄ arylthio, C₇₋₁₆ aralkylthio, amino, mono-C₁₋₆ alkylamino, mono-C₆₋₁₄ arylamino, di-C₁₋₆ alkylamino, di-C₆₋₁₄ arylamino, formyl, carboxy, C₁₋₆ alkyl-carbonyl, C₃₋₆ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl, carbamoyl, thiocarbamoyl, mono-C₁₋₆ alkyl-carbamoyl, di-C₁₋₆ alkyl-carbamoyl, C₆₋₁₄ aryl-carbamoyl, 5- or 6-membered heterocyclic carbamoyl, C₁₋₆ alkylsulfonyl, C₆₋₁₄ arylsulfonyl, C₁₋₆ alkylsulfinyl, C₆₋₁₄ arylsulfinyl, formylamino, C₁₋₆ alkyl-carbonylamino, C₆₋₁₄ aryl-carbonylamino, C₁₋₆ alkoxy-carbonylamino, C₁₋₆ alkylsulfonylamino, C₆₋₁₄ arylsulfonylamino, C₁₋₆ alkyl-carbonyloxy, C₆₋₁₄ aryl-carbonyloxy, C₁₋₆ alkoxy-carbonyloxy, mono-C₁₋₆ alkyl-carbamoyloxy, di-C₁₋₆ alkyl-carbamoyloxy, C₆₋₁₄ aryl-carbamoyloxy, nicotinoyloxy, 5- to 7-membered saturated cyclic amino containingl to 4 of 1 or 2 kinds of hetero atoms selected from nitrogen atom, sulfur atom and oxygen atom, besides one nitrogen atom and carbon atom (this cyclic amino may have substituents selected from the group consisting of C₁₋₆ alkyl, C₆₋₁₄ aryl, C₁₋₆ alkyl-carbonyl, 5- to 10-membered aromatic heterocyclic group and oxo), and 5-to 10-membered aromatic heterocyclic group, containingl to 4 of 1 or 2 kinds of hetero atoms selected from nitrogen atom, sulfur atom and oxygen atom, besides carbon atom, sulfo, sulfamoyl, sulfinamoyl and sulfenamoyl (substituent group A)],
(iii) 5- to 14-membered heterocyclic group containingl to 4 of 1 or 2 kinds of hetero atoms selected from nitrogen atom, sulfur atom and oxygen atom, besides carbon atom, which may have 1 to 3 substituents selected from substituent group A,
(iv) acyl group represented by the formula: -(C=O)-R⁵, -(C=O)-OR⁵, -(C=O)-NR⁵R⁶, -(C=S)-NHR⁵ or -SO₂-R⁷
wherein R⁵ is (1) hydrogen atom, (2) C₁₋₆ alkyl group, C₂₋₆ alkenyl group, C₂₋₆ alkynyl group, C₃₋₆ cycloalkyl group, C₆₋₁₄ aryl group or C₇₋₁₆ aralkyl group, which may have 1 to 3 substituents selected from substituent group A or (3) 5- to 14-membered heterocyclic group containing1 to 4 of 1 or 2 kinds of hetero atoms selected from nitrogen atom, sulfur atom and oxygen atom, besides carbon atom, which may have 1 to 3 substituents selected from substituent group A, R⁶ is hydrogen atom or C₁₋₆ alkyl group, and R⁷ is (1) C₁₋₆ alkyl group, C₂₋₆ alkenyl group, C₂₋₆ alkynyl group, C₃₋₆ cycloalkyl group, C₆₋₁₄ aryl group or C₇₋₁₆ aralkyl group, which may have 1 to 3 substituents selected from substituent group A or (3) 5- to 14-membered heterocyclic group containing 1 to 4 of 1 or 2 kinds of hetero atoms selected from nitrogen atom, sulfur atom and oxygen atom, besides carbon atom, which may have 1 to 3 substituents selected from substituent group A,
(v) amino group (this amino group may have 1 or 2 substituents selected from (1) C₁₋₆ alkyl group, C₂₋₆ alkenyl group, C₂₋₆ alkynyl group, C₃₋₆ cycloalkyl group, C₆₋₁₄ aryl group or C₇₋₁₆ aralkyl group, which may have 1 to 3 substituents selected from substituent group A, (2) 5- to 14-membered heterocyclic group containing 1 to 4 of 1 or 2 kinds of hetero atoms selected from nitrogen atom, sulfur atom and oxygen atom, besides carbon atom, which may have 1 to 3 substituents selected from substituent group A, and (3) acyl group shown by the above-mentioned (iv)),
(vi) 5- to 7-membered non-aromatic cyclic amino group containingl to 4 of 1 or 2 kinds of hetero atoms selected from nitrogen atom, sulfur atom and oxygen atom, besides one nitrogen atom and carbon atom, (this cyclic amino group may have 1 to 3 substituents selected from C₁₋₆ alkyl, C₆₋₁₄ aryl, C₁₋ ₆ alkyl-carbonyl, 5- to 10-membered aromatic heterocyclic group and oxo), and
(vii) C₁₋₆ alkoxy group, C₆₋₁₄ aryloxy group and C₇₋₁₆ aralkyloxy group, which may have 1 to 3 substituents selected from substituent group A.

26. The method of claim 22, wherein the polar group is 1 to 3 selected from (1) halogen atom, (2) hydroxy, (3) amino optionally having 1 or 2 substituents selected from a substituent selected from substituent group A and acyl shown by the above-mentioned (iv), (4) nitro, (5) carboxy, (6) formyl, (7) C₁₋₆ alkoxy optionally having 1 to 3 substituents selected from substituent group A, (8) C₁₋₆ alkoxy-carbonyl optionally having 1 to 3 substituents selected from substituent group A, (9) cyano and (10) C₁₋₆ alkyl or C₆₋₁₄ aryl having 1 to 3 groups from the above-mentioned (1)-(9) as substituents.


SEQUENCE LISTING

<110> Takeda Chemical Industries, Ltd.

<120> Pharmaceutical Composition

<130> 2915WOOP

<150> JP 2001-175224

<151> 2001-6-11

<150> JP 2001-175273

<151> 2001-6-11

<160> 20

<210> 1

<211> 62

<212> DNA

<213> Artificial Sequence

<400> 1

accactcgag atggactaca aggacgacga tgacaagtct caggagaggc ccacgttcta 60

cc 62

<210> 2

<211> 35

<212> DNA

<213> Artificial Sequence

<400> 2

acccggtacc accaggtgct caggactcca tctct 35

<210> 3

<211> 61

<212> DNA

<213> Artificial Sequence

<400> 3

acaagaattc ataacatatg gctcatcatc atcatcatca ttccaagcca cccgcaccca 60

a 61

<210> 4

<211> 32

<212> DNA

<213> Artificial Sequence

<400> 4

tcccgtctag actatgagtc ttctcccagg at 32

<210> 5

<211> 45

<212> DNA

<213> Artificial Sequence

<400> 5

ggctacttgg tggacgaggt ggccaaggag atggatgccg gctgc 45

<210> 6

<211> 45

<212> DNA

<213> Artificial Sequence

<400> 6

gcagccggca tccatctcct tggccacctc gtccaccaag tagcc 45

<210> 7

<211> 34

<212> DNA

<213> Artificial Sequence

<400> 7

cgcctctaga caagatgccc aacaacagca ctgc 34

<210> 8

<211> 34

<212> DNA

<213> Artificial Sequence

<400> 8

cggggtcgac actactcaga attcttctca atgc                                    34

<210> 9

<211> 62

<212> DNA

<213> Artificial Sequence

<400> 9

accactcgag atggactaca aggacgacga tgacaagtct caggagaggc ccacgttcta    60

cc                                                                       62

<210> 10

<211> 35

<212> DNA

<213> Artificial Sequence

<400> 10

acccggtacc accaggtgct caggactcca tctct                                   35

<210> 11

<211> 61

<212> DNA

<213> Artificial Sequence

<400> 11

acaagaattc ataacatatg gctcatcatc atcatcatca ttccaagcca cccgcaccca    60

a                                                                        61

<210> 12

<211> 32

<212> DNA

<213> Artificial Sequence

<400> 12

tcccgtctag actatgagtc ttctcccagg at                                    32

<210> 13

<211> 45

<212> DNA

<213> Artificial Sequence

<400> 13

ggctacttgg tggacgaggt ggccaaggag atggatgccg gctgc                      45

<210> 14

<211> 45

<212> DNA

<213> Artificial Sequence

<400> 14

gcagccggca tccatctcct tggccacctc gtccaccaag tagcc                      45

<210> 15

<211> 62

<212> DNA

<213> Artificial Sequence

<400> 15

accactcgag atggactaca aggacgacga tgacaagtct caggagaggc ccacgttcta    60

cc                                                                     62

<210> 16

<211> 35

<212> DNA

<213> Artificial Sequence

<400> 16

acccggtacc accaggtgct caggactcca tctct 35

<210> 17

<211> 61

<212> DNA

<213> Artificial Sequence

<400> 17

acaagaattc ataacatatg gctcatcatc atcatcatca ttccaagcca cccgcaccca 60

a 61

<210> 18

<211> 32

<212> DNA

<213> Artificial Sequence

<400> 18

tcccgtctag actatgagtc ttctcccagg at 32

<210> 19

<211> 45

<212> DNA

<213> Artificial Sequence

<400> 19

ggctacttgg tggacgaggt ggccaaggag atggatgccg gctgc 45

<210> 20

<211> 45

<212> DNA

<213> Artificial Sequence

<400> 20

gcagccggca tccatctcct tggccacctc gtccaccaag tagcc 45